(19) Europäisches Patentamt
European Patent Office
Office européen des brevets

(11) EP 4 692 091 A1

(12) EUROPEAN PATENT APPLICATION
published in accordance with Art. 153(4) EPC

(43) Date of publication:
**11.02.2026 Bulletin 2026/07**

(21) Application number: **24784396.4**

(22) Date of filing: **03.04.2024**

(51) International Patent Classification (IPC):
***C07D 491/22*** (2006.01) ***C07D 495/22*** (2006.01)
***C07K 16/28*** (2006.01) ***A61P 35/00*** (2006.01)
***A61K 31/4745*** (2006.01) ***A61K 47/68*** (2017.01)
***A61K 31/48*** (2006.01) ***A61K 39/395*** (2006.01)

(52) Cooperative Patent Classification (CPC):
**A61K 31/4745; A61K 31/48; A61K 39/395; A61K 47/68; A61P 35/00; C07D 491/22; C07D 495/22; C07K 16/28**

(86) International application number:
**PCT/CN2024/085983**

(87) International publication number:
**WO 2024/208314 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.04.2023 CN 202310367781**
**21.06.2023 CN 202310743291**
**28.07.2023 CN 202310941713**
**12.09.2023 CN 202311173712**
**21.11.2023 CN 202311558824**
**25.12.2023 CN 202311802330**

(71) Applicant: **Changchun Genescience Pharmaceutical Co., Ltd.**
**Changchun, Jilin 130012 (CN)**

(72) Inventors:
- **YANG, Fanglong**
**Changchun, Jilin 130012 (CN)**
- **HUANG, Yue**
**Changchun, Jilin 130012 (CN)**
- **HE, Weiming**
**Changchun, Jilin 130012 (CN)**
- **DAI, Kaiqin**
**Changchun, Jilin 130012 (CN)**
- **WANG, Siqin**
**Changchun, Jilin 130012 (CN)**
- **JIN, Lei**
**Changchun, Jilin 130012 (CN)**

(74) Representative: **Gille Hrabal Partnerschaftsgesellschaft mbB Patentanwälte**
**Brucknerstraße 20**
**40593 Düsseldorf (DE)**

Remarks:
The complete document including Reference Table(s) and the Sequence Listing(s) can be downloaded from the EPO website

# (54) CAMPTOTHECIN DERIVATIVE, AND PHARMACEUTICAL COMPOSITION, PREPARATION METHOD THEREFOR AND USE THEREOF

(57) The present invention relates to a camptothecin derivative represented by general formula I, and a pharmaceutical composition, a preparation method therefor and a use thereof. The camptothecin derivative has good tumor inhibitory activity, and can be used for treating or preventing tumors, and for preparing drugs for treating or preventing tumor diseases.

EP 4 692 091 A1

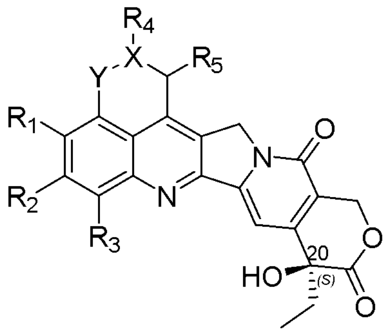

I

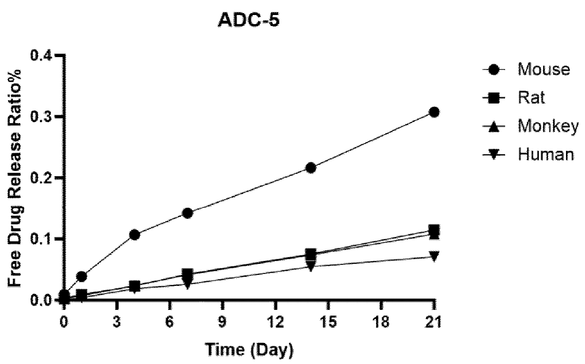
ADC-5
Free Drug Release Ratio%
0.0
0.1
0.2
0.3
0.4
0
3
6
9
12
15
18
21
Time (Day)
Mouse
Rat
Monkey
Human

FIG. 1

## Description

### CROSS-REFERENCE TO RELATED APPLICATIONS

The present application claims priority to application No. 202310367781.7 filed April 7, 2023, application No. 202310743291.2 filed June 21, 2023, application No. 202310941713.7 filed July 28, 2023, application No. 202311173712.9 filed September 12, 2023, application No. 202311558824.6 filed November 21, 2023, and application No. 202311802330.8 filed December 25, 2023, which are all titled "CAMPTOTHECIN DERIVATIVE, AND PHARMACEUTICAL COMPOSITION, PREPARATION METHOD THEREFOR AND USE THEREOF".

### TECHNICAL FIELD

The present invention belongs to the field of medicine, and specifically relates to a camptothecin derivative, and a pharmaceutical composition, a preparation method therefor, and use thereof.

### BACKGROUND

In the field of tumor treatment, an antibody-drug conjugates (ADC) is composed of a monoclonal antibody drug targeting a specific antigen conjugated to a small molecule cytotoxic drug through a linker. It has both the powerful killing effect of traditional small molecule chemotherapy and the tumor targeting of an antibody drug.

Camptothecin (CPT) is a natural product isolated from *Camptotheca acuminata,* a plant of nyssaceae. Camptothecin is a pentacyclic compound composed of a quinoline ring AB, a pyrrole ring C, a pyridone ring D, and α-hydroxylactone ring E, in which the 20-C is in S-configuration (see the structural formula below). Due to its excellent anticancer activity, it was put into clinical use in the early 1970s. Later, severe drug side effects such as diarrhea and hemorrhagic cystitis occurred clinically, and the clinical trial was terminated.

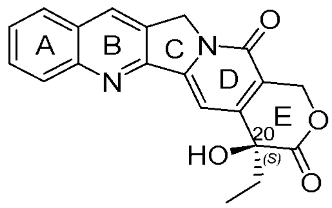

Camptothecin (CPT) structure

Research data shows that camptothecin can form a ternary complex with DNA topoisomerase I in cells, thereby inhibiting DNA despiralization, blocking DNA replication, and resulting in cell death *(*Cancer Res. 1989, 49, 6365). Camptothecin and a derivative thereof have very strong antitumor activity in an in vivo animal model such as lung cancer, breast cancer, colorectal cancer, and ovarian cancer *(*Nature Review Cancer. 2006, 6, 789). At present, a plurality of camptothecin drugs have been approved for marketing for tumor treatment (Med Res. Rev. 2015, 35, 753). Irinotecan is a medicament for treating colorectal cancer; topotecan is used for treating ovarian cancer; and belotecan is used for treating ovarian cancer and small cell lung cancer. The camptothecin derivative further comprises, e.g., exatecan, rubitecan, diflomotecan, lurtotecan, gimatecan, simimtecan, chimmitecan, or elomotecan. The camptothecin drugs or derivatives often have hematotoxicity caused by myelosuppression, such as leukopenia, thrombocytopenia, anemia, or neutropenia, as well as gastrointestinal side effects, such as nausea, emesis, or diarrhea. As found through clinical studies, measures for improving the safety and efficacy of camptothecin compounds comprise, e.g., increasing water solubility, improving pharmacokinetic properties thereof, enhancing activity, reducing dosage, or forming antibody-drug conjugates using conjugates thereof and antibodies. Therefore, research and development of camptothecin compounds and conjugates thereof with novel structures and capable of enhancing efficacy and improving safety issues still has very high clinical demand and application value.

### SUMMARY OF THE INVENTION

The present invention provides a compound represented by formula I, a racemate, a stereoisomer, a tautomer, an isotope-labeled counterpart, a solvate, a polymorph, a pharmaceutically acceptable salt or a prodrug compound thereof:

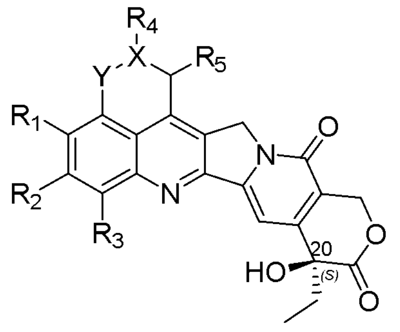

I

wherein $R_1$, $R_2$, and $R_3$ are the same or different and each independently selected from H, OH, CN, halogen, $C_{1-10}$ alkyl, $C_{2-10}$ alkenyl, $C_{2-10}$ alkynyl, $C_{1-10}$ alkoxy, halo-$C_{1-10}$ alkyl, halo-$C_{1-10}$ alkoxy, cyano-$C_{1-10}$ alkyl, cyano-$C_{1-10}$ alkoxy, or $C_{3-10}$ cycloalkyl;

$R_4$ is selected from H or

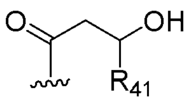

; $R_{41}$ is selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl;

$R_5$ is selected from H,

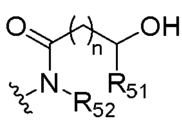 , 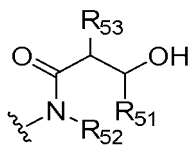 , or 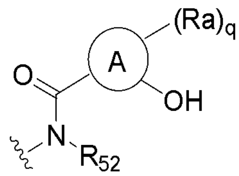 ;

$R_{51}$ and $R_{52}$ are the same or different and each independently selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl; $R_{53}$ is selected from $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl; ring A is selected from $C_{3-8}$ cycloalkyl or 3-8-membered heterocyclyl; Ra is selected from H, hydroxyl, CN, halogen, $C_{1-6}$ alkyl, or $C_{1-6}$ haloalkyl; n is selected from 0, 1, or 2; and q is selected from 0, 1, or 2;

X is selected from CH or N;

Y is selected from $-(CH_2)_m-$, or $-(CH_2)_m-O-(CH_2)_p-$;

m is an integer selected from 0-6; and

p is an integer selected from 0-6.

According to some embodiments, $R_1$ is selected from H, OH, CN, halogen, $C_{1-6}$ alkyl, $C_{2-6}$ alkenyl, $C_{2-6}$ alkynyl, $C_{3-6}$ cycloalkyl, or halo-$C_{1-6}$ alkoxy.

According to some embodiments, $R_1$ is selected from H, OH, Br, methyl, difluoromethoxy, 2,2,2-trifluoroethoxy, vinyl, cyclopropyl, or ethynyl.

According to some embodiments, $R_2$ is selected from H, halogen, CN, or $C_{1-6}$ alkyl.

According to some embodiments, $R_2$ is selected from H or F.

According to some embodiments, $R_3$ is selected from H or $C_{1-6}$ alkyl.

According to some embodiments, $R_3$ is H.

According to some embodiments, $R_4$ is selected from H or

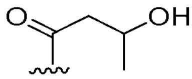;

for example,

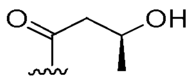 or 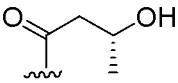.

I

According to some embodiments, $R_4$ is selected from H or

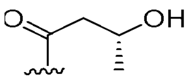.

According to some embodiments, X-$R_4$ is

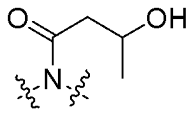

, and is preferably

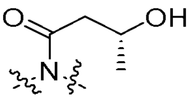.

According to some embodiments, X-$R_4$ is $-CH_2-$.

According to some embodiments, $R_5$ is selected from H,

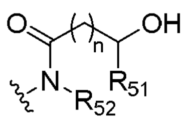, 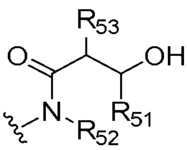,

or

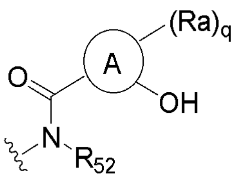;

; wherein $R_{51}$ is selected from H, methyl, ethyl, isopropyl, or cyclopropyl; $R_{52}$ is selected from H or methyl; $R_{53}$ is selected from methyl; ring A is selected from $C_{3-6}$ cycloalkyl; Ra is selected from H, hydroxyl, CN, halogen, $C_{1-6}$ alkyl, or $C_{1-6}$ haloalkyl; n is selected from 0 or 1; and q is selected from 0 or 1.

According to some embodiments, ring A is selected from a cyclobutane ring.

According to some embodiments, $R_5$ is selected from H,

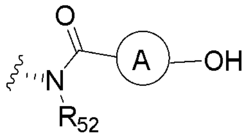,

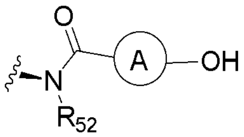, 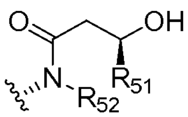, 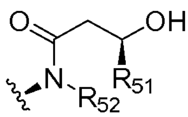, 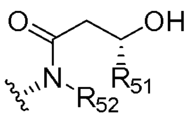, 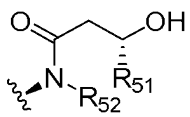,

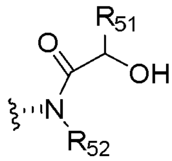, 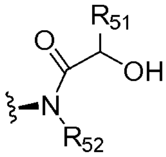, 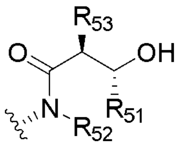, or 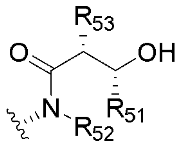.

According to some embodiments, $R_{51}$ is selected from H, methyl, ethyl, isopropyl, or cyclopropyl; $R_{52}$ is selected from H or methyl; $R_{53}$ is selected from methyl; and ring A is selected from a cyclobutane ring.

According to some embodiments, $R_5$ is selected from H,

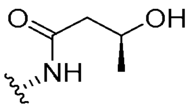, 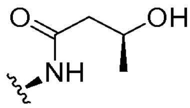,

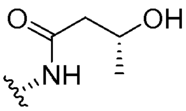, 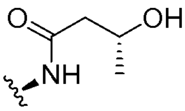, 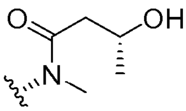, 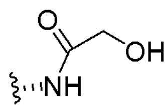, 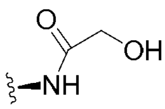, 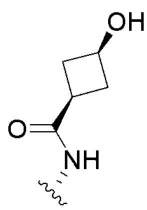,

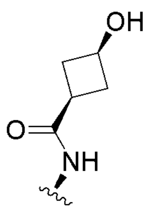, 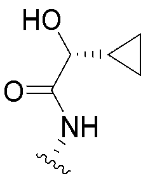, 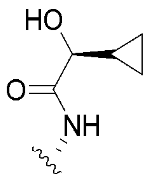, 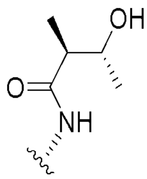, 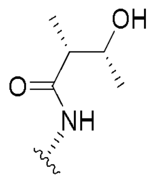, 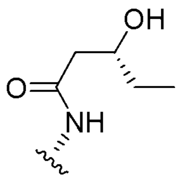, 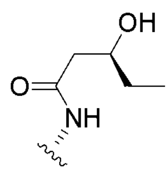,

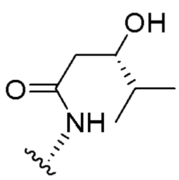, 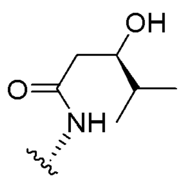, 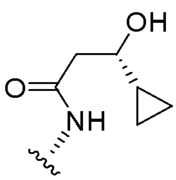, or 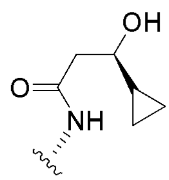.

According to some embodiments, X is selected from CH or N; and when X is CH, $R_4$ is H; or when X is N, $R_5$ is H.

According to some embodiments, m is selected from 0, 1, or 2.

According to some embodiments, p is selected from 0, 1, or 2.

According to some embodiments, Y is selected from $-CH_2-$, $-CH_2-CH_2-$ or $-CH_2-O-$.

According to some embodiments, the compound represented by formula I has a structure as shown below:

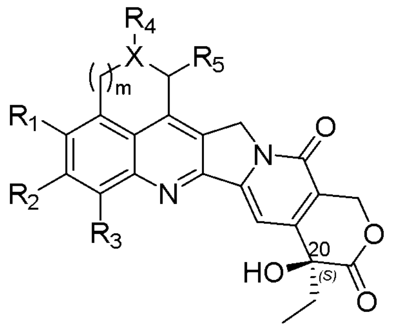

I-1

wherein $R_1$, $R_2$, $R_3$, $R_4$, $R_5$, X, and m are each independently as defined herein.

According to some embodiments, the compound represented by formula I has a structure as shown below:

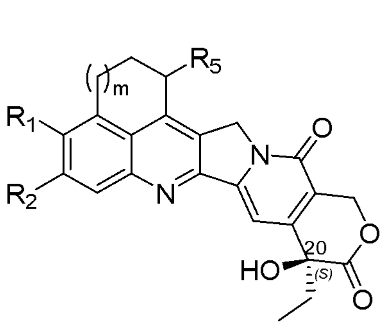

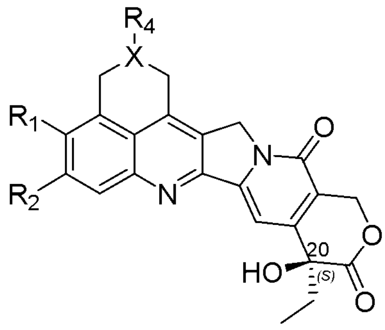

;

I-a

I-b

wherein $R_1$, $R_2$, $R_4$, $R_5$, X, and m are each independently as defined herein.

According to some embodiments, the compound represented by formula I has a structure as shown below:

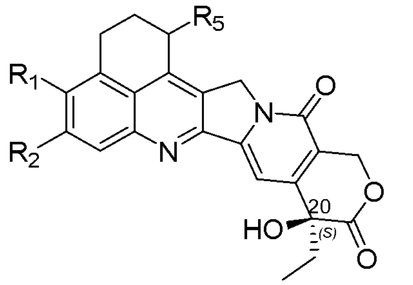

wherein $R_1$, $R_2$, and $R_5$ are each independently as defined herein.

According to some embodiments, the compound represented by formula I has a structure as shown below:

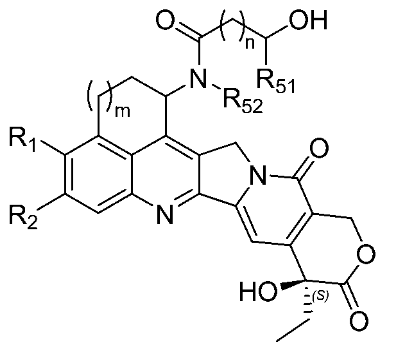

III-a

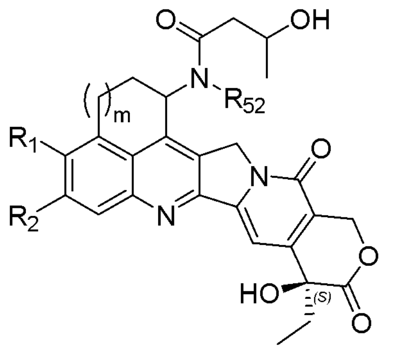

III-b

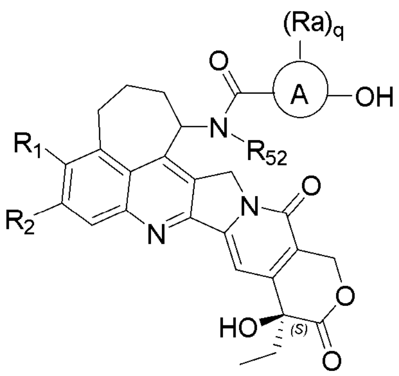

III-c

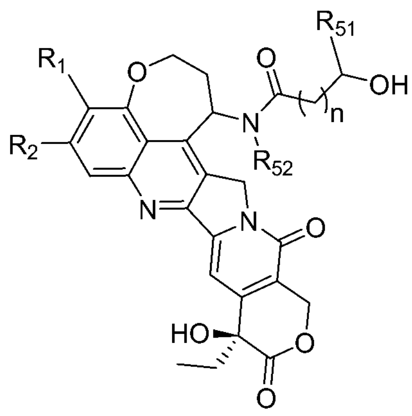

III-d

wherein $R_1$, $R_2$, $R_{51}$, $R_{52}$, ring A, Ra, m, n, and q are each independently as defined herein.

According to some embodiments of the present invention, the compound of formula I has a structure as shown below:

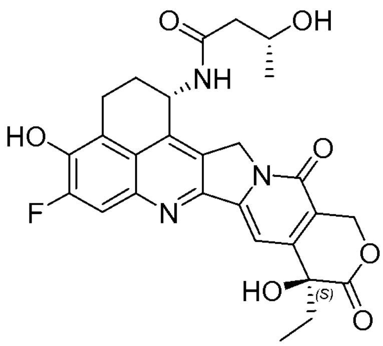

,

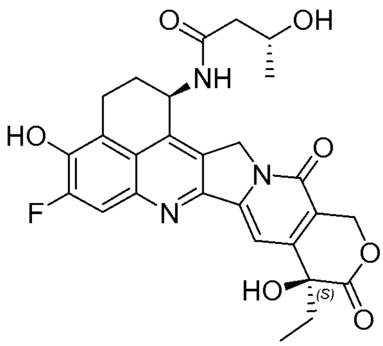

,

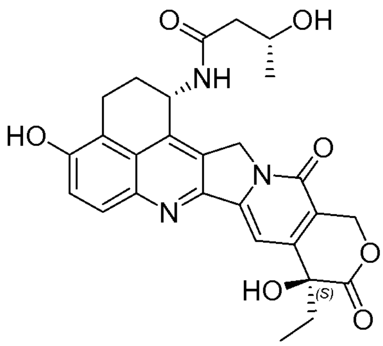

,

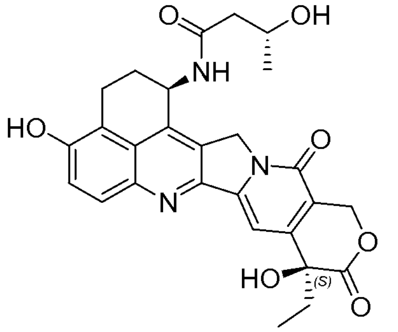,

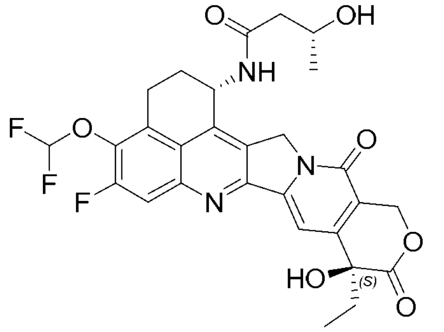,

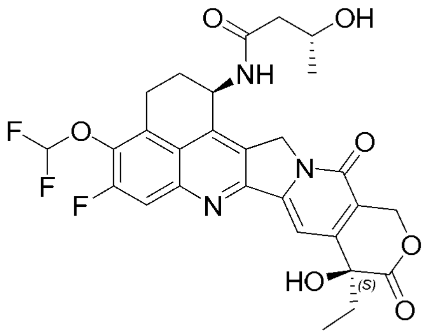,

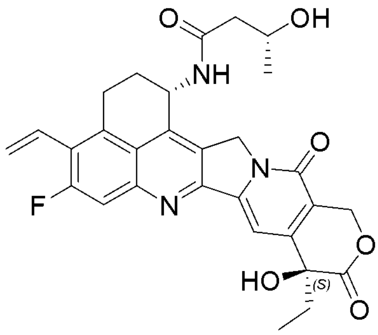,

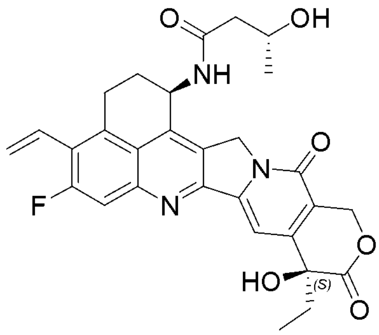,

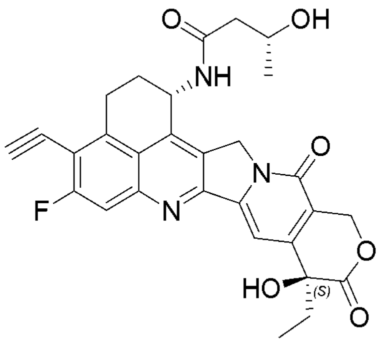,

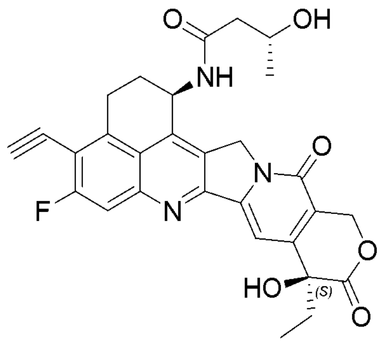,

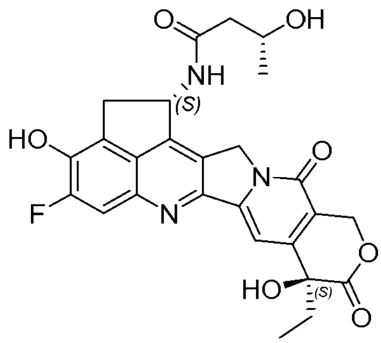,

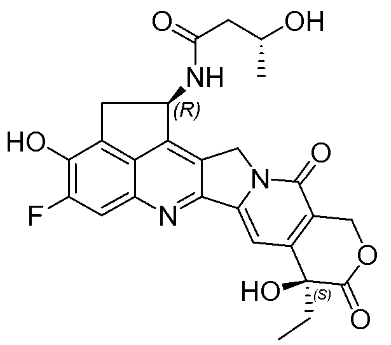,

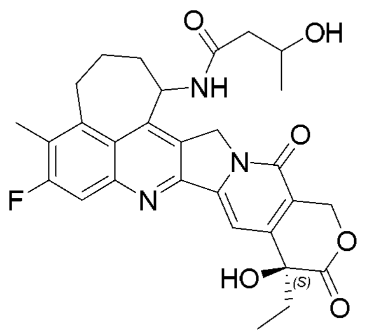,

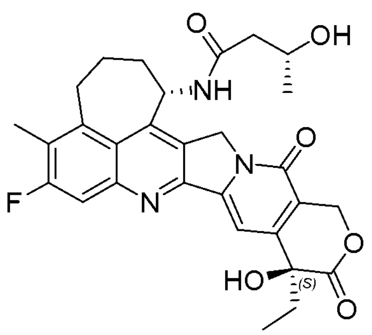,

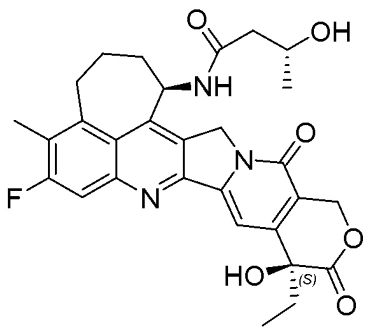
,
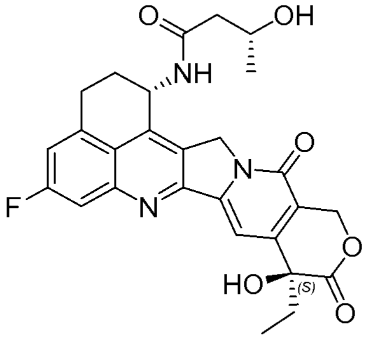
,
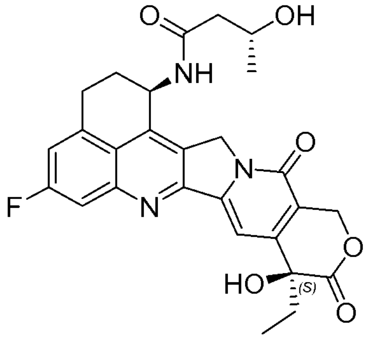
,

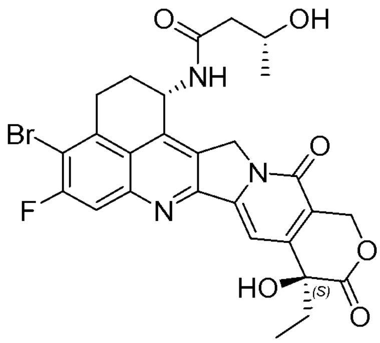
,
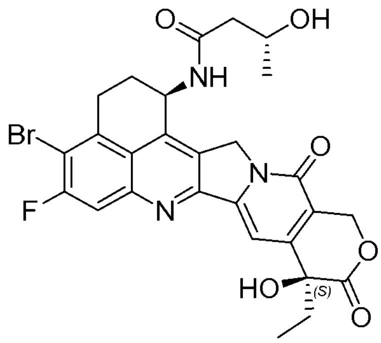
,
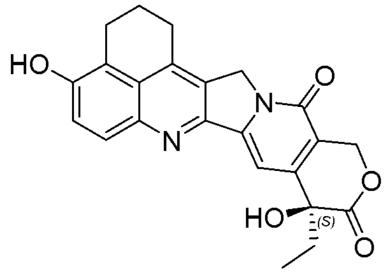
,

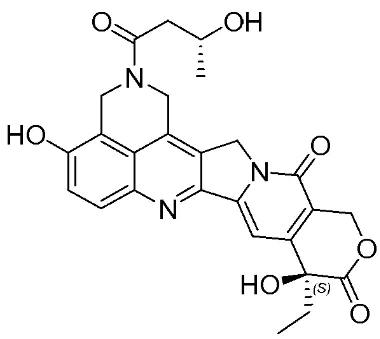
,
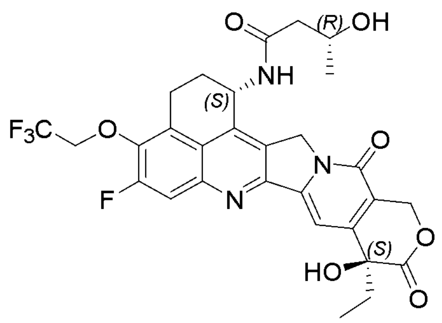
,

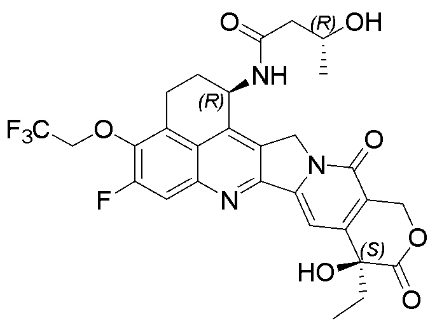
,
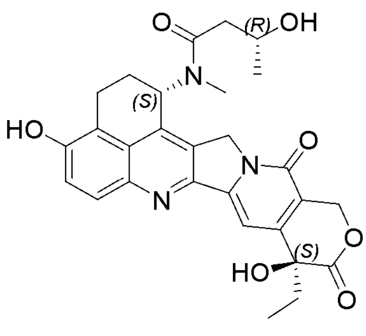
,
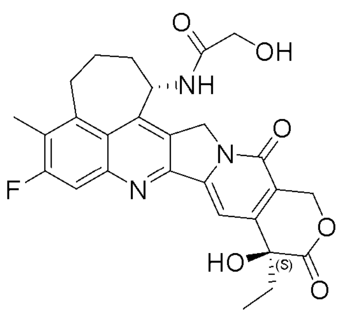
,

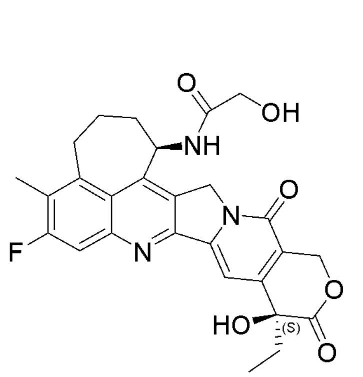,
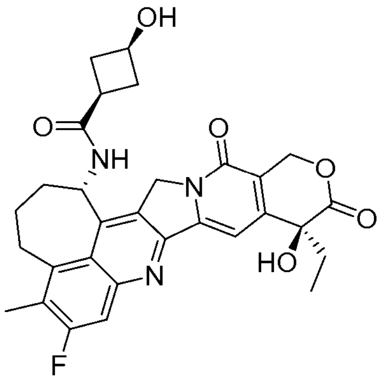,
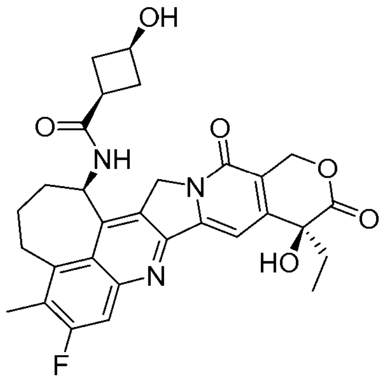,

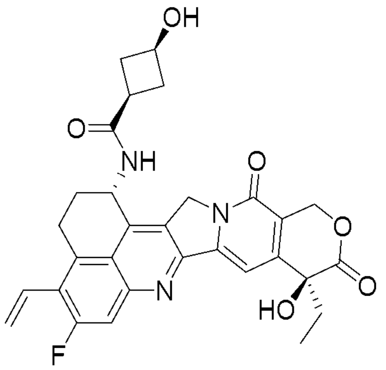,
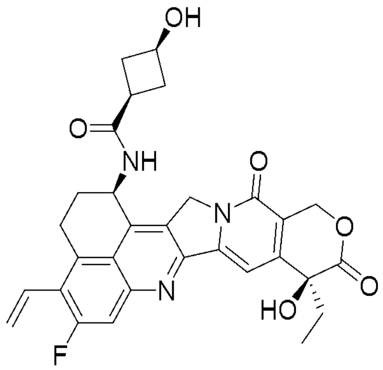,
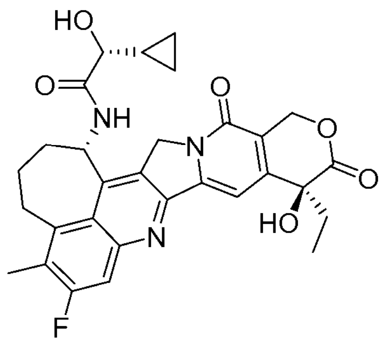,

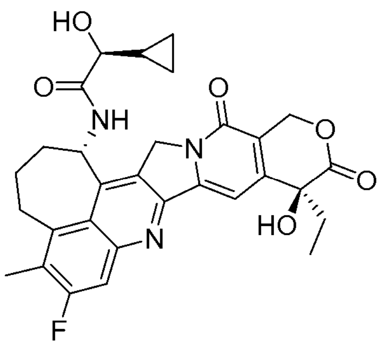,
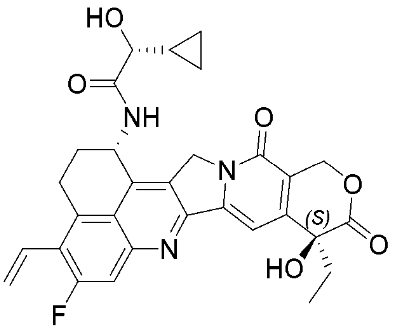,
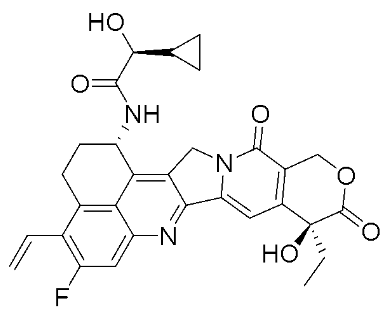,

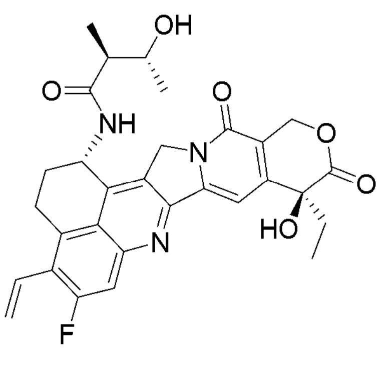,
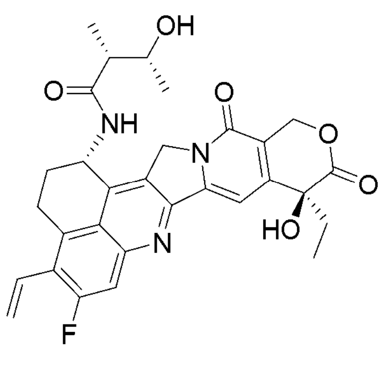,
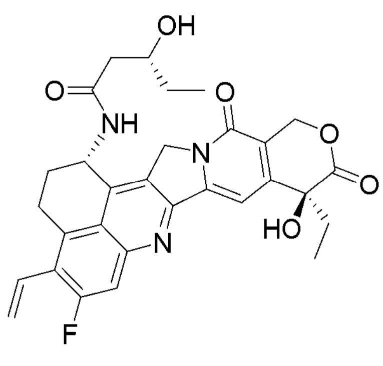,

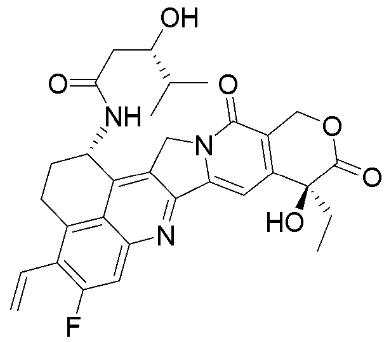,
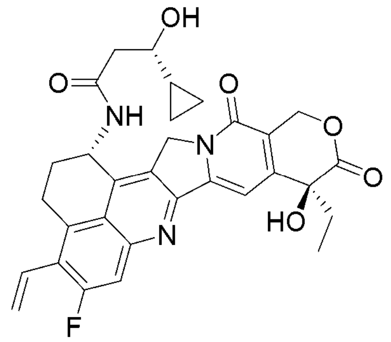,
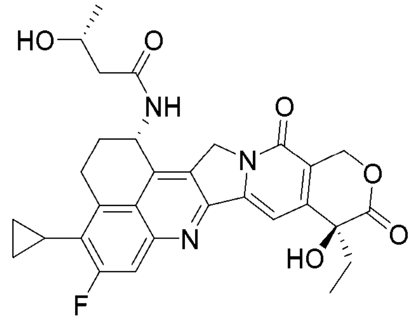,

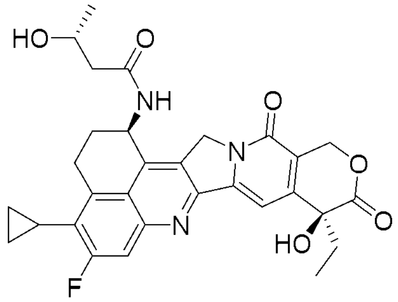,
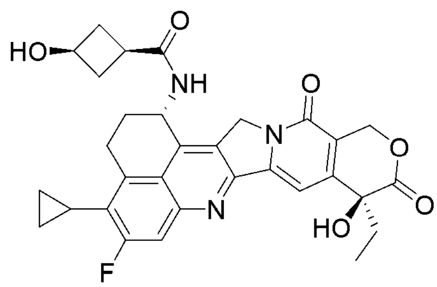,

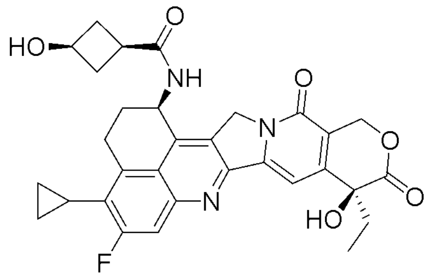,
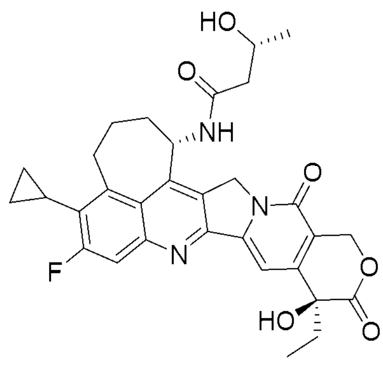,
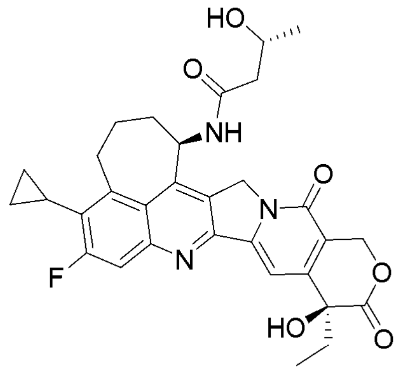,

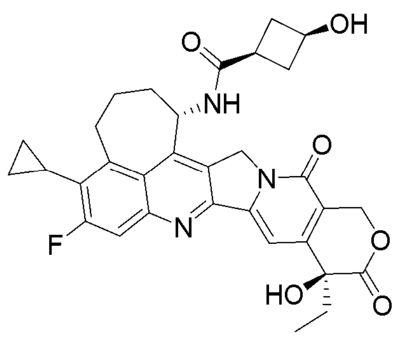,
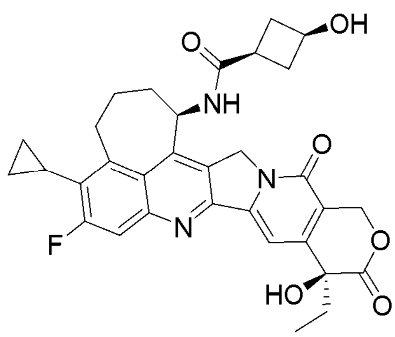,
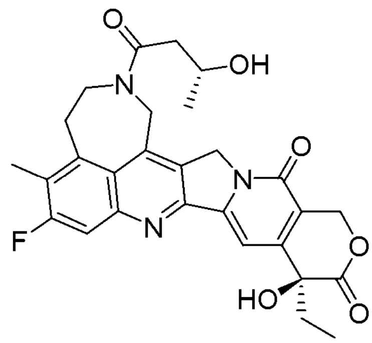,

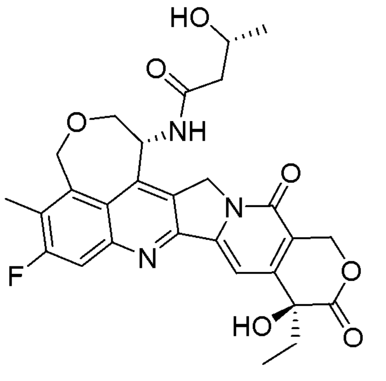,
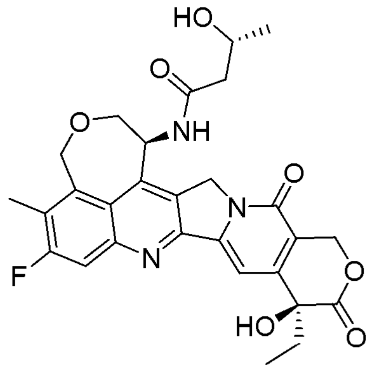,
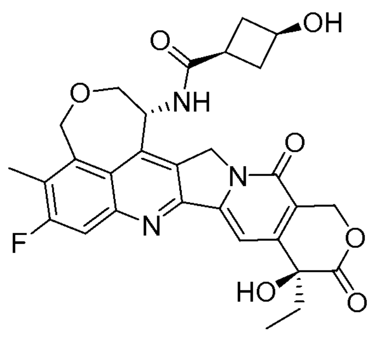,

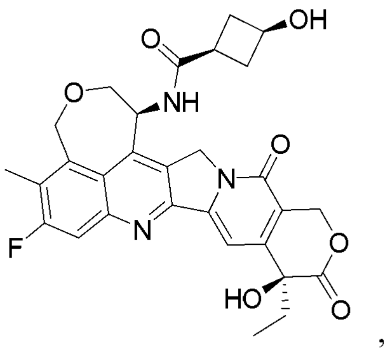,
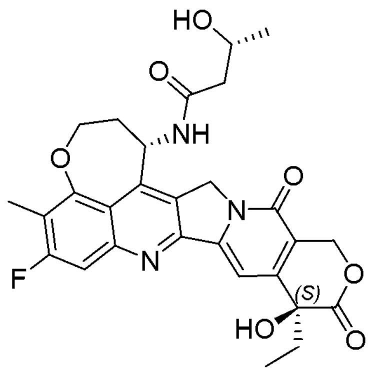,
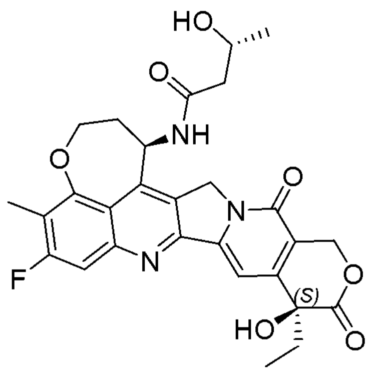,

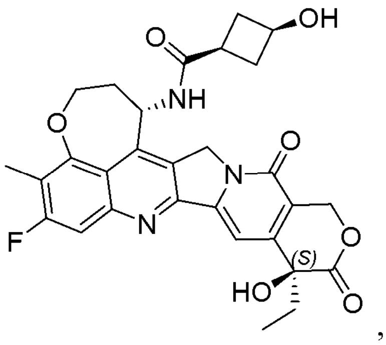,
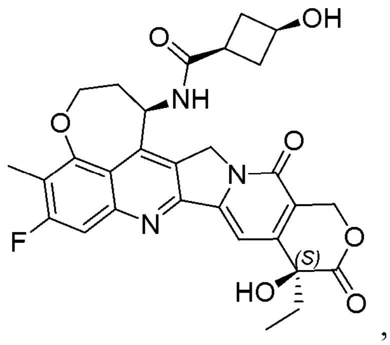,
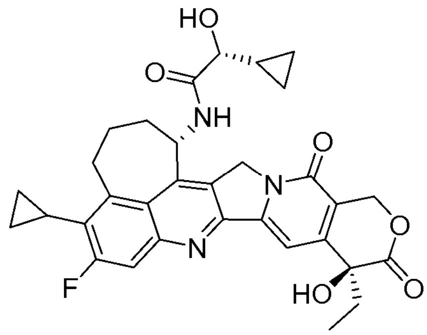,

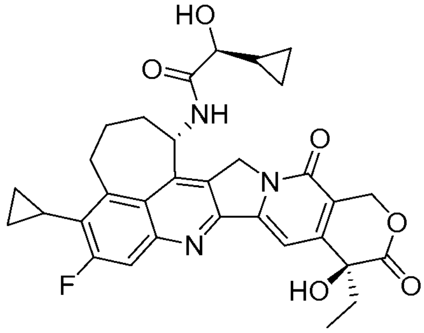,
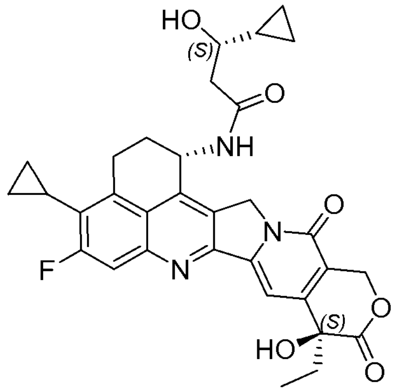,

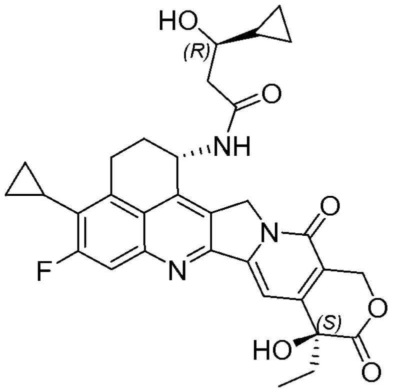, 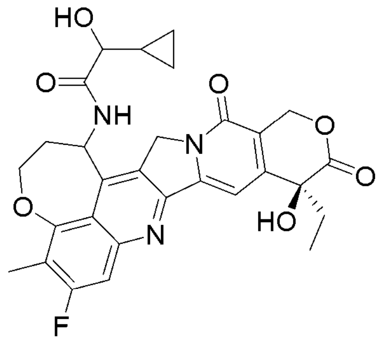, 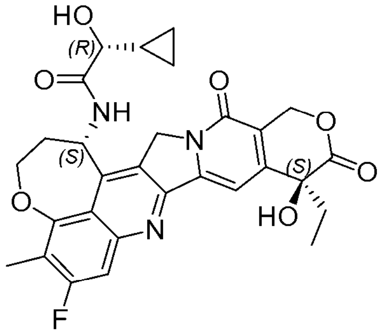,

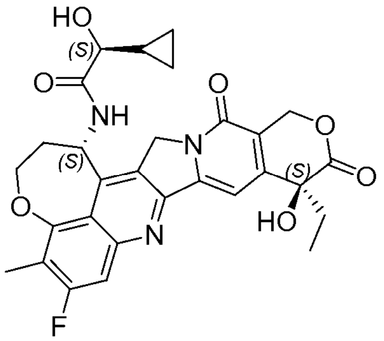, 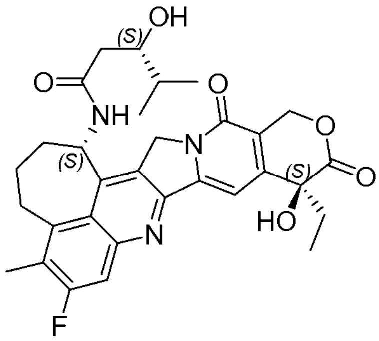, 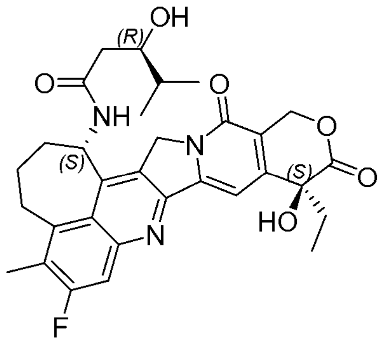,

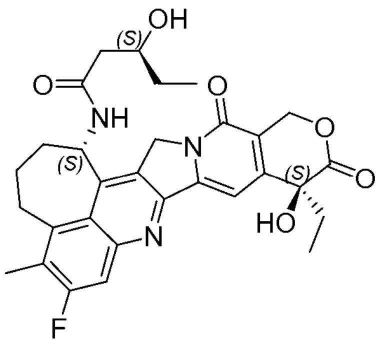, 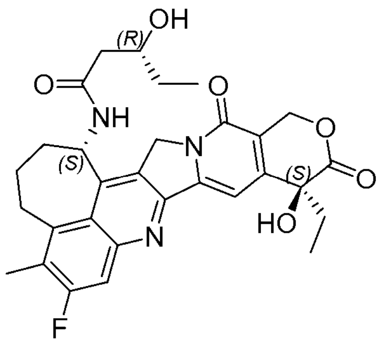, 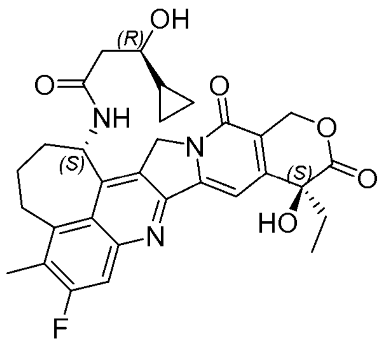,

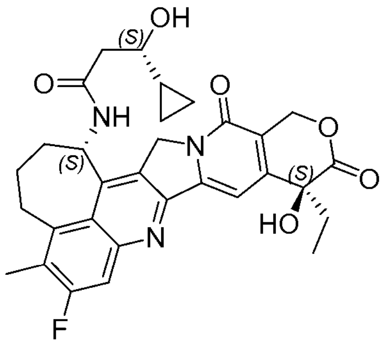, or 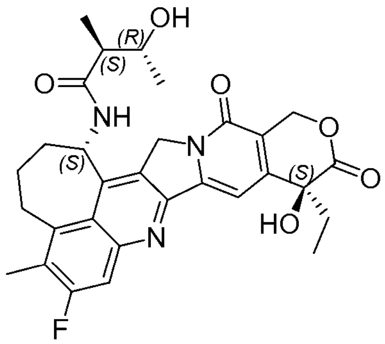.

The present invention further provides a compound represented by formula V, a racemate, a stereoisomer, a tautomer, an isotope-labeled counterpart, a solvate, a polymorph, a pharmaceutically acceptable salt or a prodrug compound thereof:

$M\text{-}L_1\text{-}L_2\text{-}D$ (Formula V)

wherein M is a linker site linking to an antibody or an antigen-binding fragment thereof;

$L_1$ is a peptide residue; preferably, it is selected from glycine-glycine-phenylalanine-glycine (GGFG), glutamic acid-valine-citrulline (EVC), valine-citrulline (VC), aspartic acid-valine-citrulline (DVC), glutamic acid-glycine-glycine-phenylalanine-glycine (EGGFG), aspartic acid-glycine-glycine-phenylalanine-glycine (DGGFG);

$L_2$ is a linking group between the peptide residue and D; preferably, it is selected from a chemical bond, -NH-$C_{1-6}$ alkyl-,

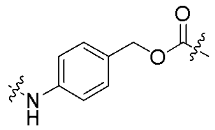 or 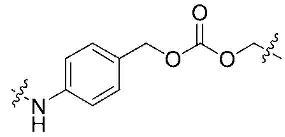 ;

preferably, $L_2$ is selected from -NH-$CH_2$-; and

D is a structural fragment of a biologically active molecule.

According to some embodiments, D is selected from a dehydrogenated structure of the compound represented by formula I herein.

According to some embodiments, D has a structure as shown below:

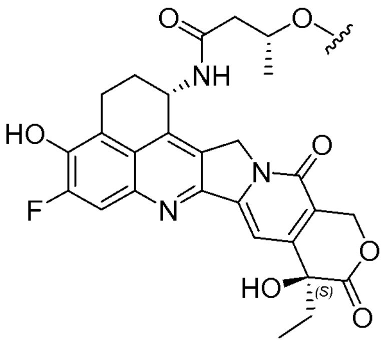 , 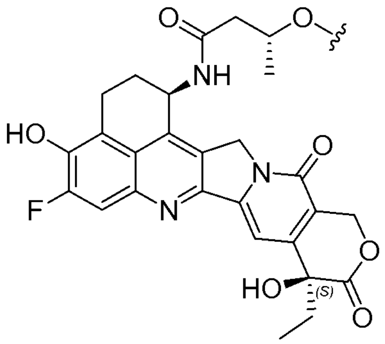 , 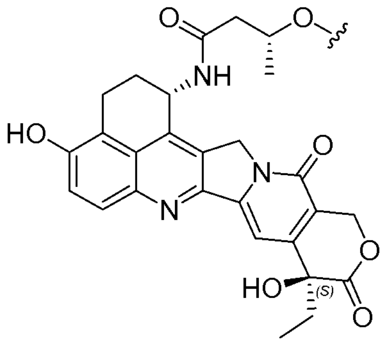 ,

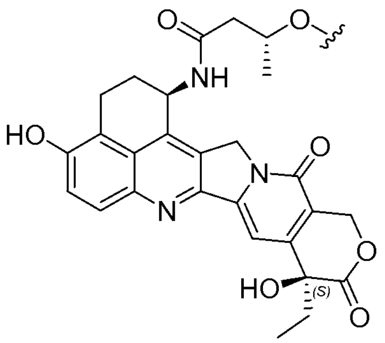 , 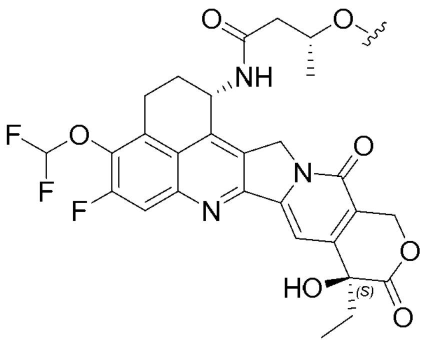 ,

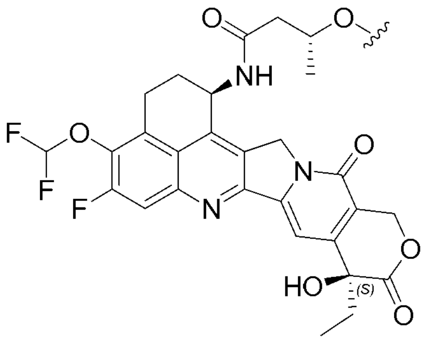 , 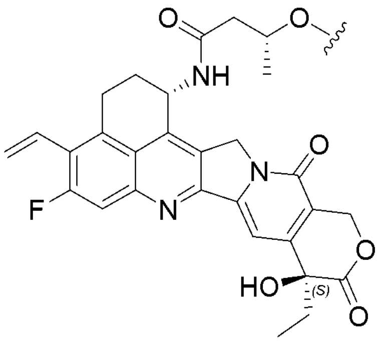 , 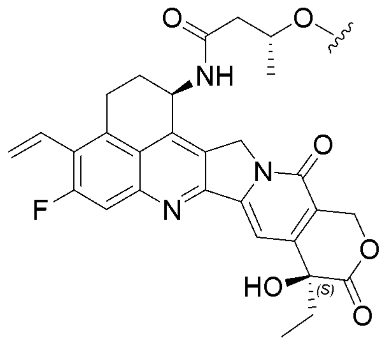 ,

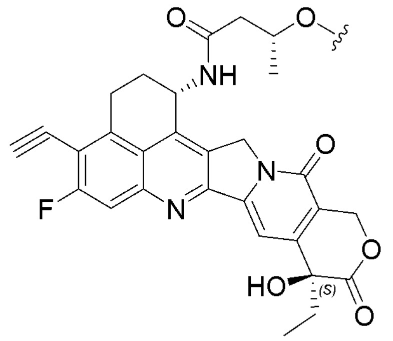 , 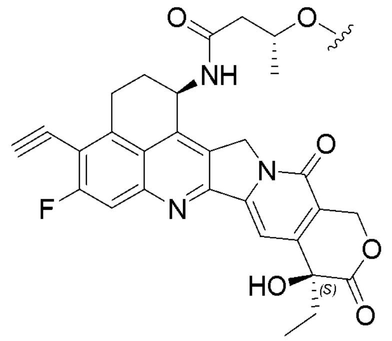 , 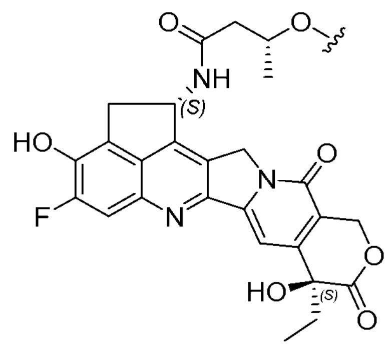 ,

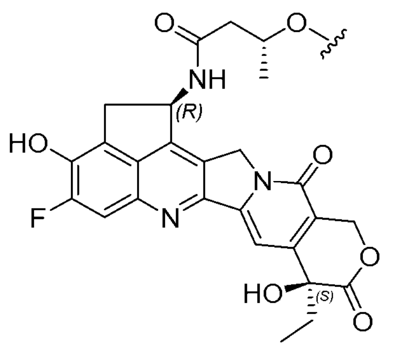 , 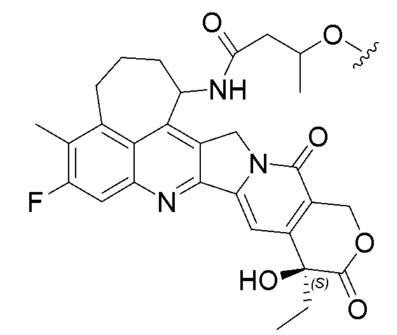 , 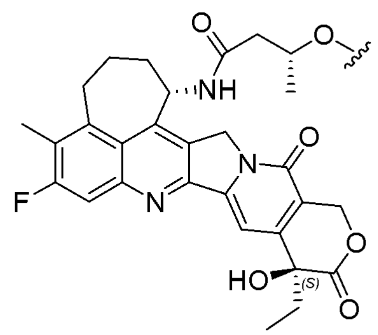 ,

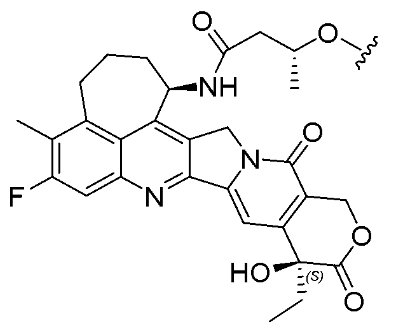 , 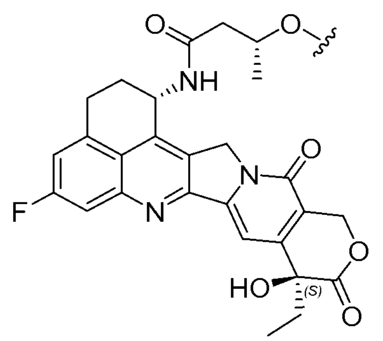 , 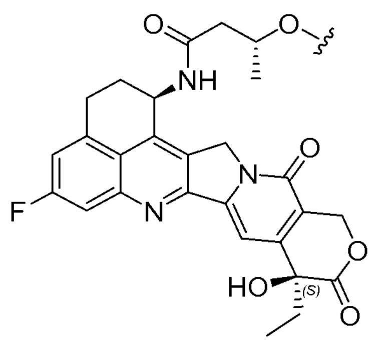 ,

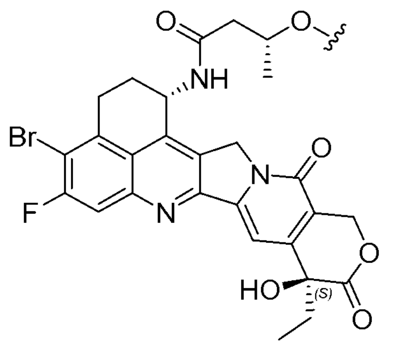 , 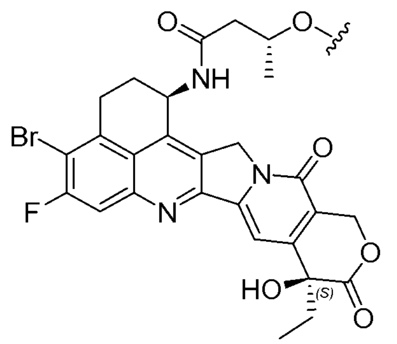 , 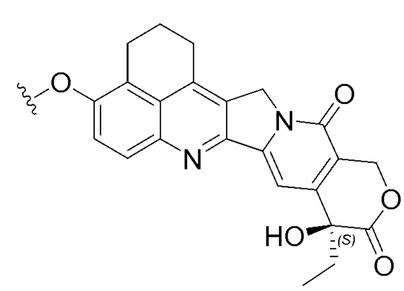 ,

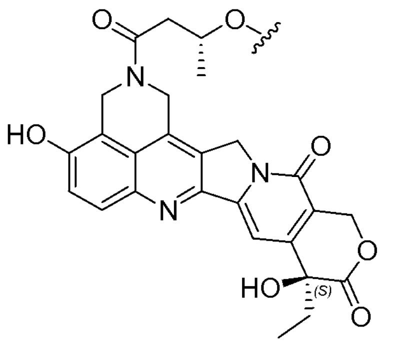
,
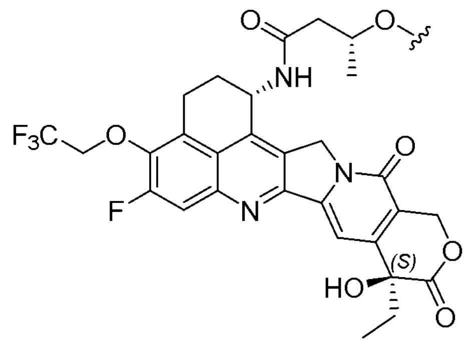
,

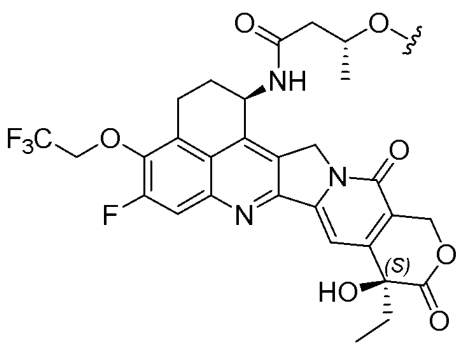
,
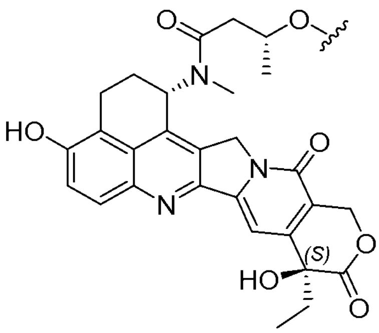
,
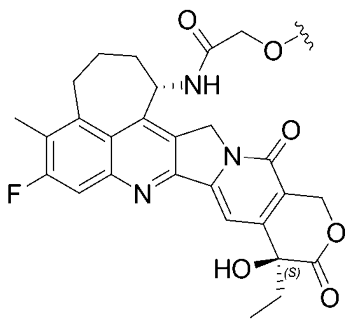
,

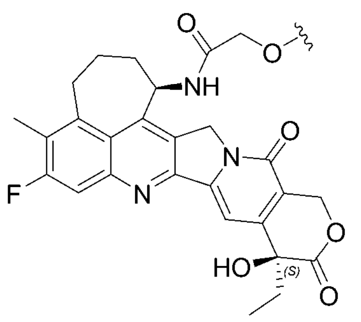
,
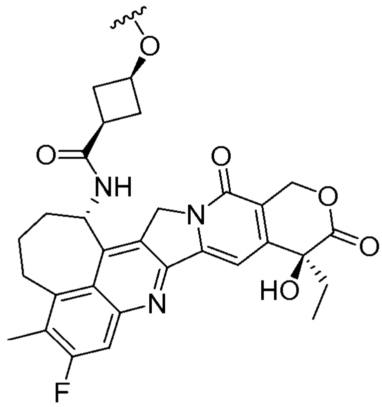
,
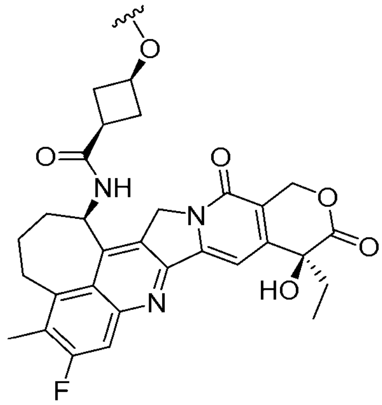
,

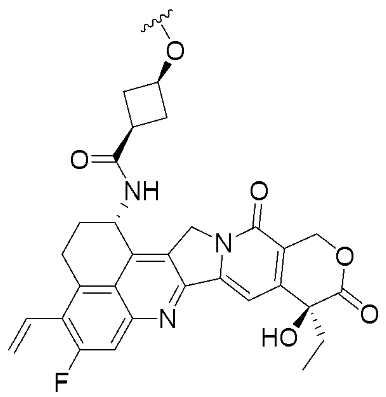
,
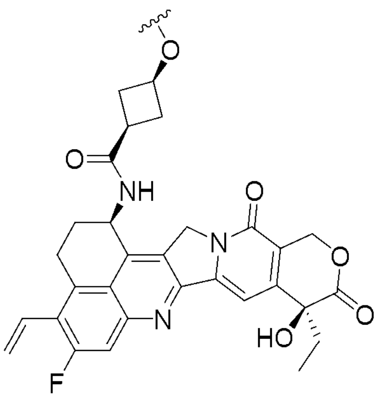
,
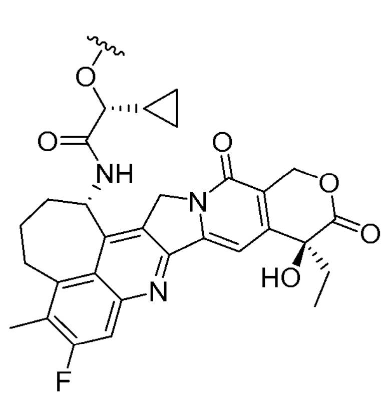
,

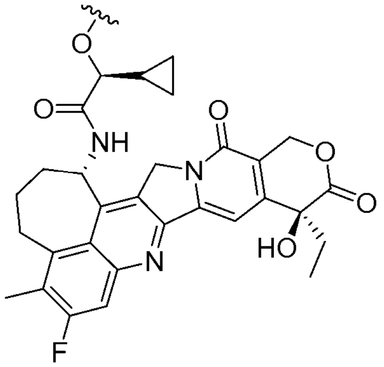,
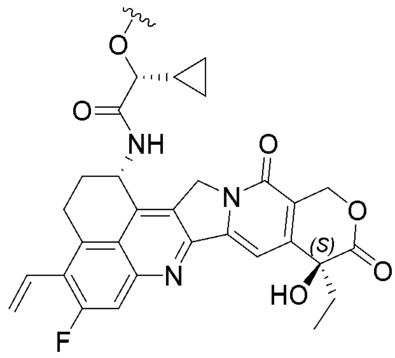,
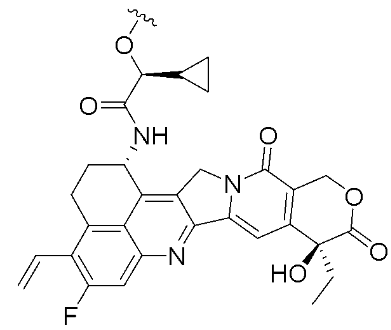,

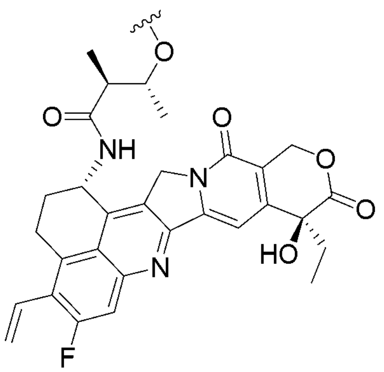,
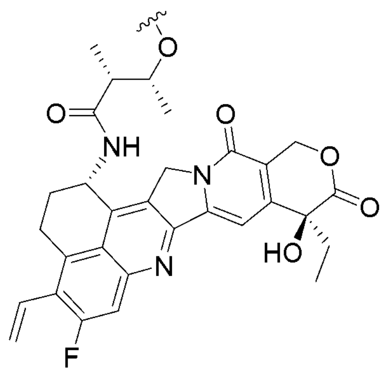,
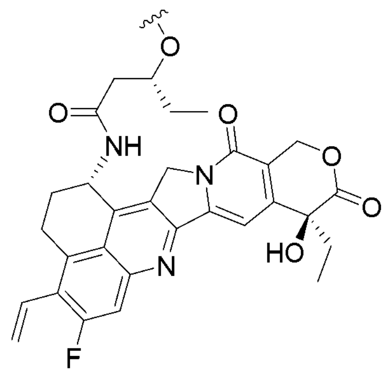,

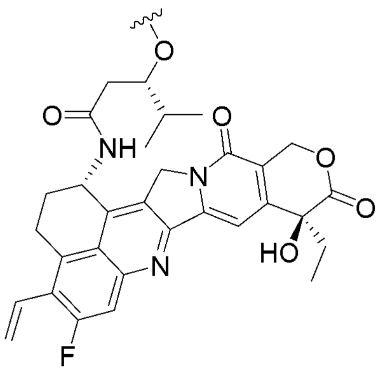,
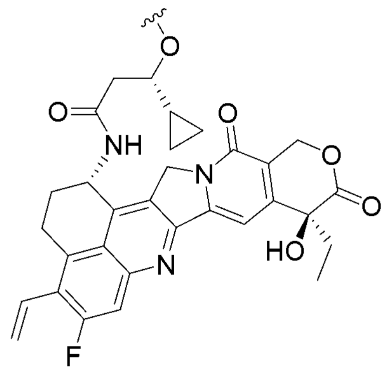,
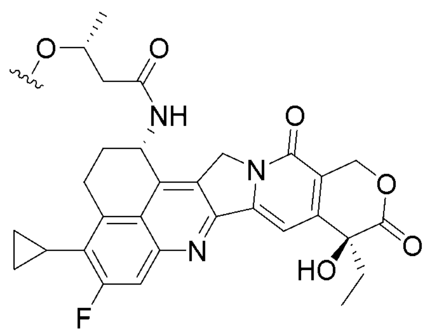,

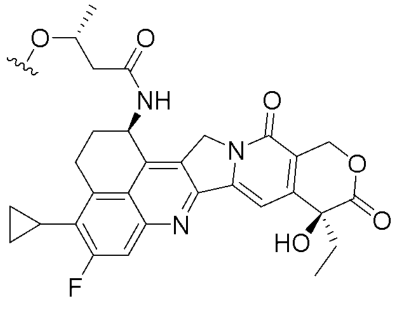,
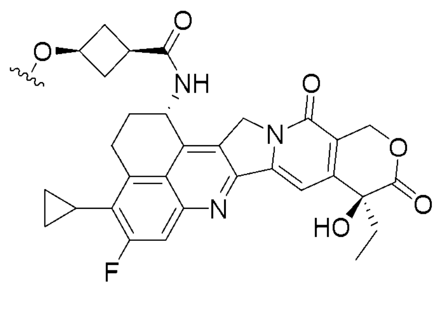,

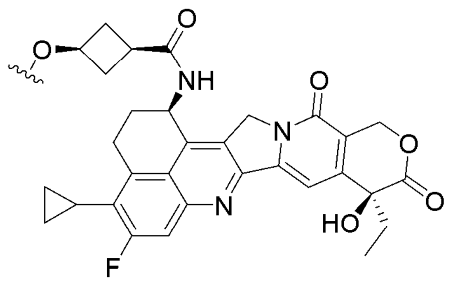
,
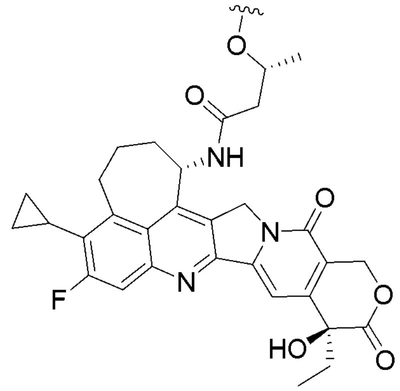
,
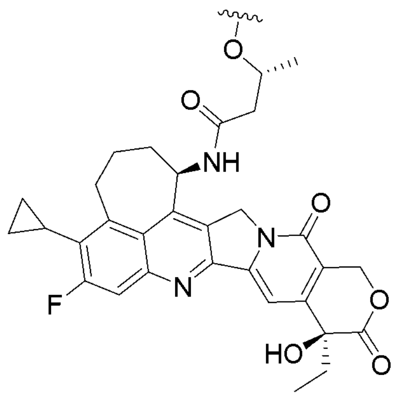
,
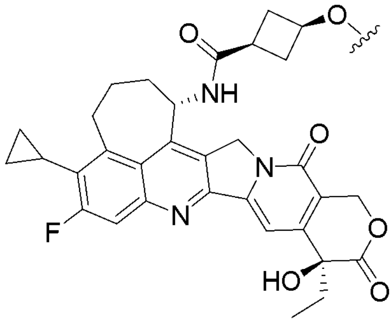
,
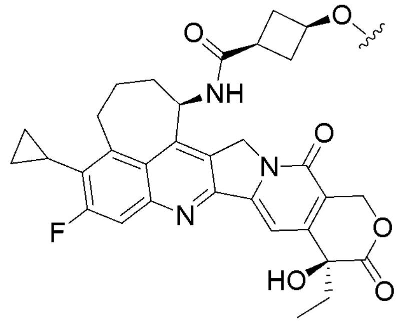
,
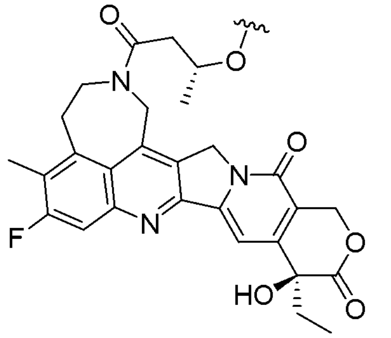
,
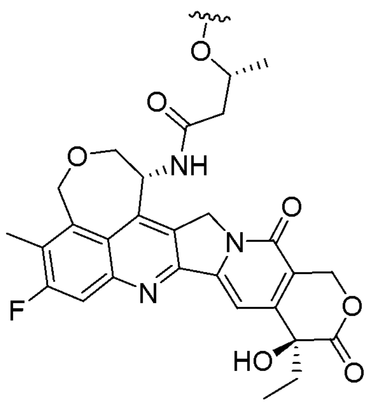
,
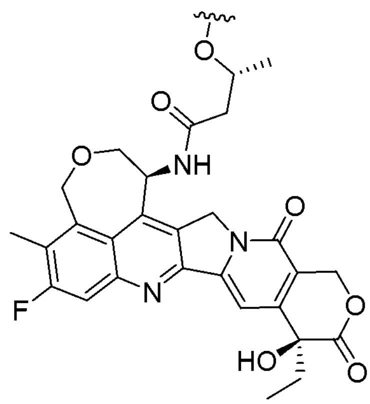
,
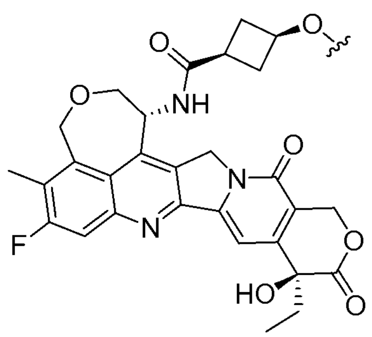
,
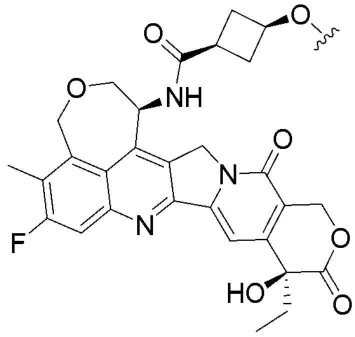
,
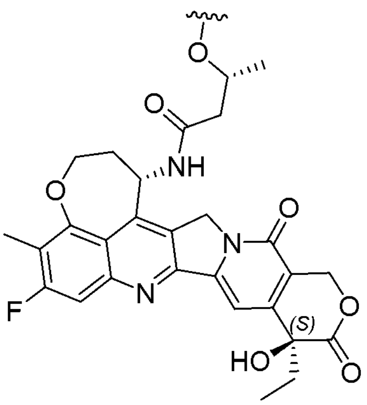
,

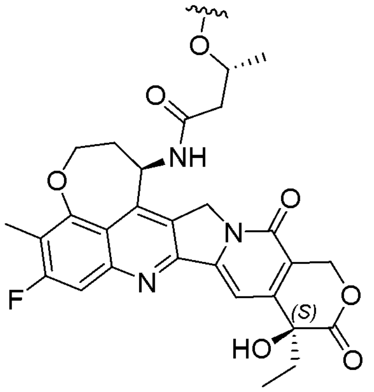,
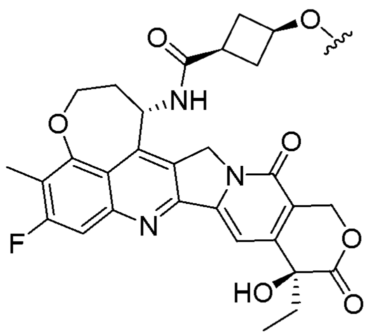,
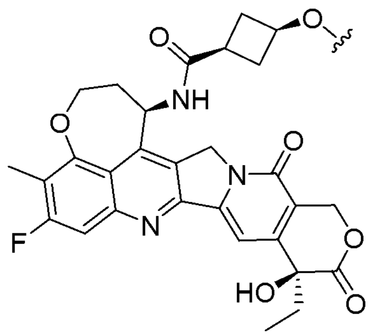,

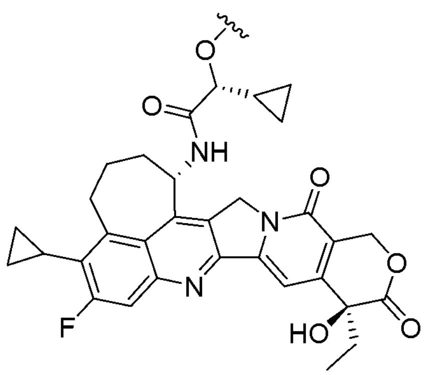,
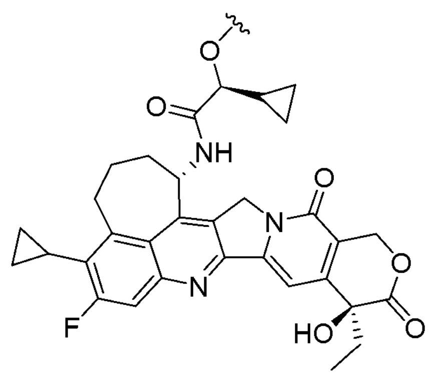,

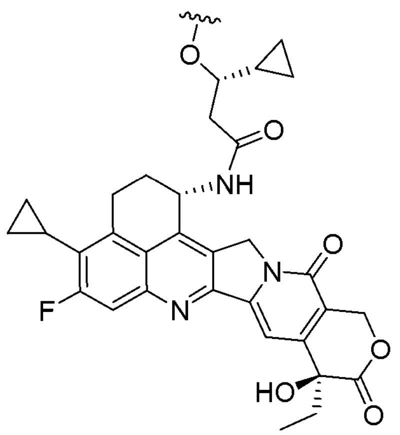,
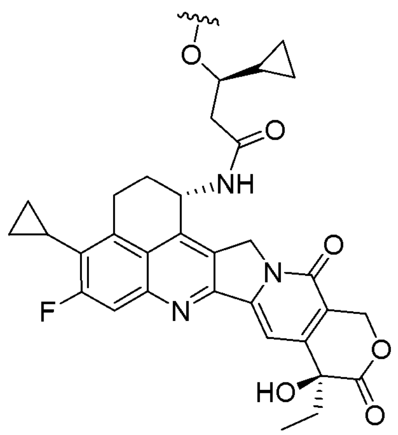,
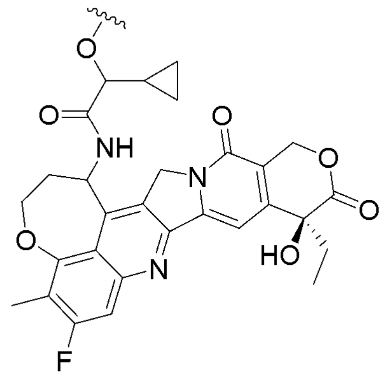,

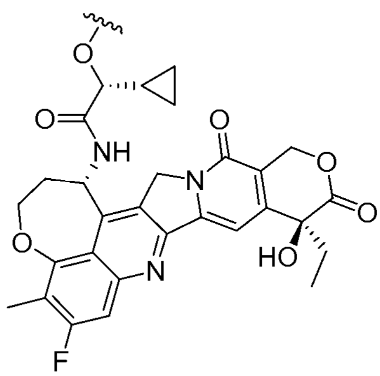,
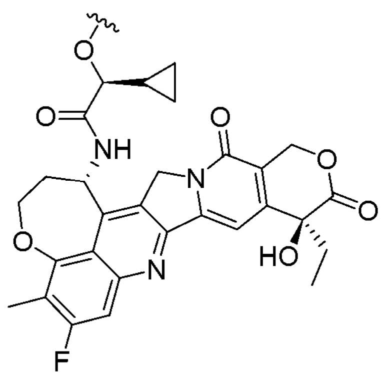,
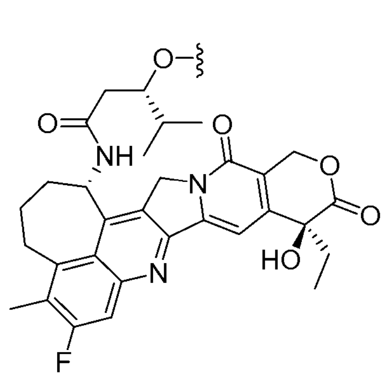,

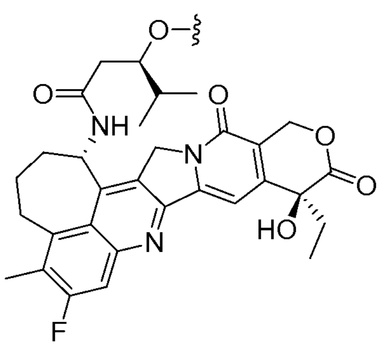, 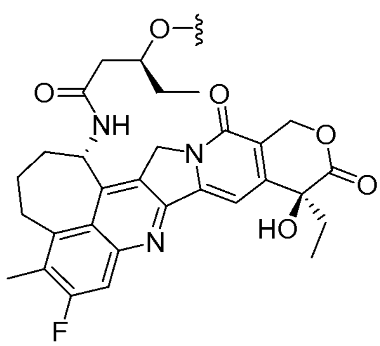, 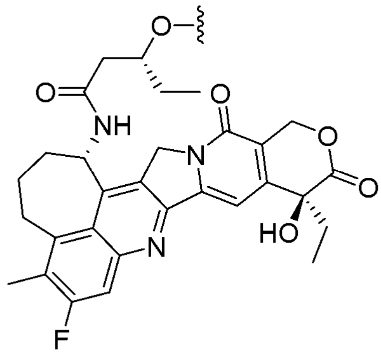,

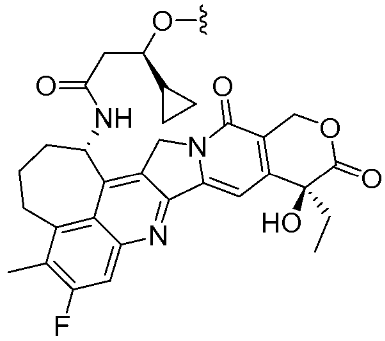, 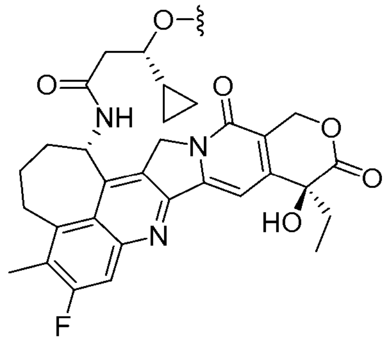, or 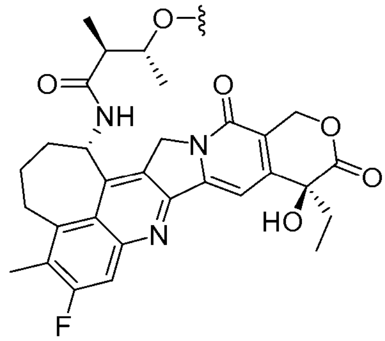.

According to some embodiments, M is selected from the following structure:

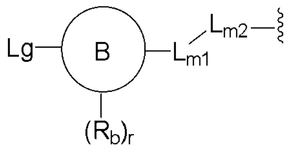

Lg is absent or is a leaving group, and the leaving group is selected from halogen, sulfone, trifluoromethanesulfonyl, and methanesulfonyl;

ring B is selected from a 5-14-membered heteroaromatic ring, or a 3-14-membered heterocyclic ring;

each $R_b$ is the same or different and each independently selected from the following groups: halogen, cyano, oxo (=O), $C_{1-6}$ alkyl, halo-$C_{1-6}$ alkyl, hydroxy-$C_{1-6}$ alkyl, $C_{1-6}$ alkoxy, $C_{3-8}$ cycloalkyl, 3-8-membered heterocyclyl, $C_{1-6}$ alkyl-O-$C_{1-6}$ alkyl-, or $C_{1-6}$ alkyl-(5-6-membered) heteroaryl;

$L_{m1}$ is absent, or is selected from the following groups which are unsubstituted or optionally substituted with one, two or more $R_{m1}$: a $C_{6-14}$ aryl, 5-14-membered heteroaryl, 3-14-membered heterocyclyl; each $R_{m1}$ is the same or different and each independently selected from H, halogen, cyano, $C_{1-6}$ alkyl or HOOC-$C_{1-3}$ alkylene;

$L_{m2}$ is selected from the following groups which are unsubstituted or optionally substituted with one, two or more $R_{m2}$: $-(CH_2)_s-(C=O)-$, or $-C{\equiv}C-(CH_2)_t-(C=O)-$; each $R_{m2}$ is the same or different and each independently selected from H, halogen, cyano, $C_{1-6}$ alkyl or -$C_{1-6}$ alkylene-COOH, wherein the alkylene is optionally interrupted by one, two or more of the following groups: O, or NH;

r is an integer selected from 0-6;

s is an integer selected from 0-6; and

t is an integer selected from 0-6.

According to some embodiments, ring B is selected from a 5-6-membered N-containing heteroaromatic ring, or a 3-6 membered N-containing heterocyclic ring.

According to some embodiments, ring B is selected from a pyrimidine ring, a pyridine ring, a triazine ring (such as

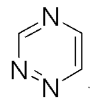

), or

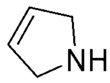.

According to some embodiments, each $R_b$ is the same or different and each independently selected from cyano, oxo (=O), methoxy, cyclopropyl, trifluoromethyl,

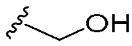, 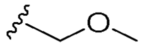,

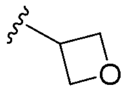 or 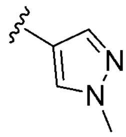.

According to some embodiments, $L_{m1}$ is absent or is selected from the following groups which are unsubstituted or optionally substituted with one, two or more $R_{m1}$: phenyl, piperidinyl or piperazinyl.

According to some embodiments, $R_{m1}$ is selected from $-CH_2COOH$.

According to some embodiments, $L_{m1}$ is selected from

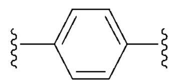, 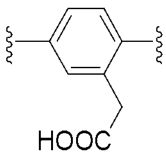,

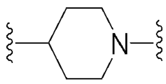 or 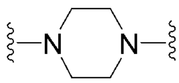.

According to some embodiments, $L_{m2}$ is selected from the following groups which are unsubstituted or optionally substituted with one, two, or more $R_{m2}$: $-CH_2-(C=O)-$, $-(CH_2)_2-(C=O)-$, $-(CH_2)_5-(C=O)-$, or $-C \equiv C-(CH_2)_3-(C=O)-$.

According to some embodiments, each $R_{m2}$ is the same or different and each independently selected from $-C_{1-3}$ alkylene-COOH, and the alkylene is optionally interrupted by O or NH.

According to some embodiments, each $R_{m2}$ is the same or different and each independently selected from

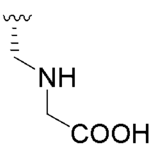, 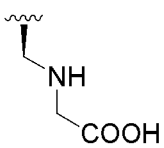, 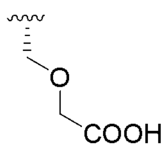, or 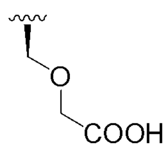.

According to some embodiments, M is selected from

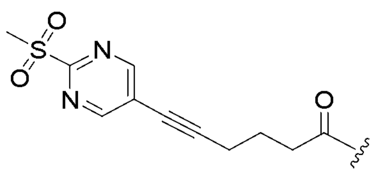,

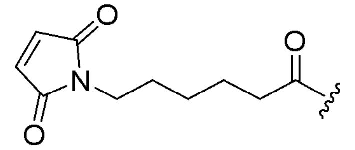,
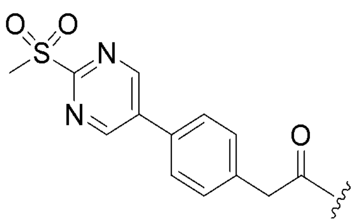,
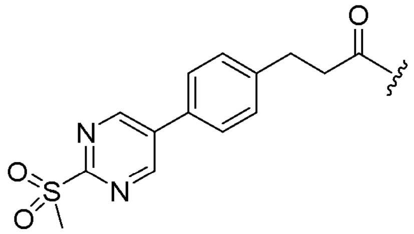,

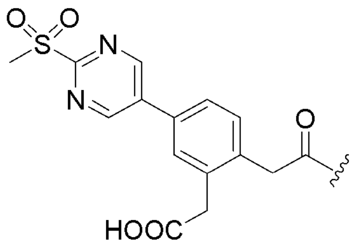,
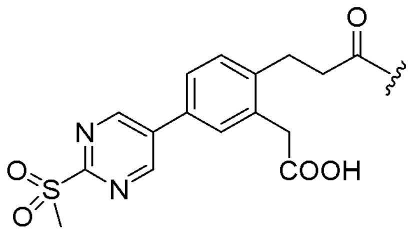,
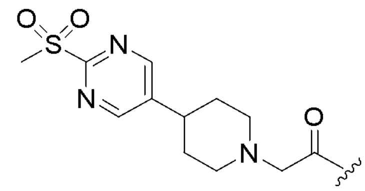,

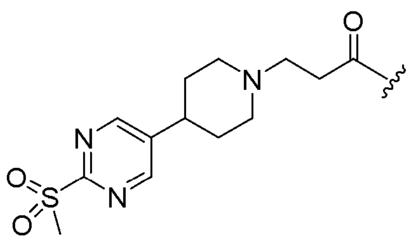,
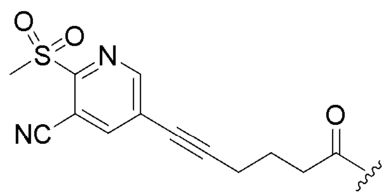,
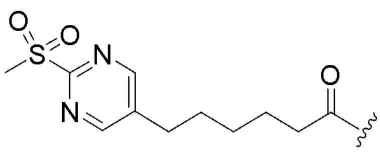,

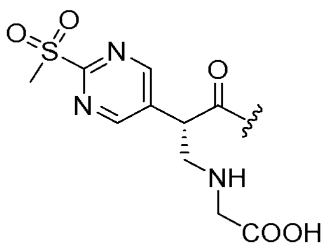,
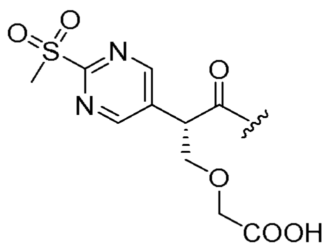,
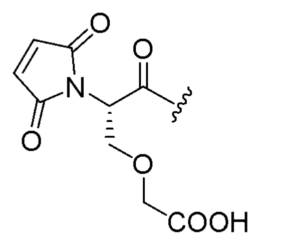,

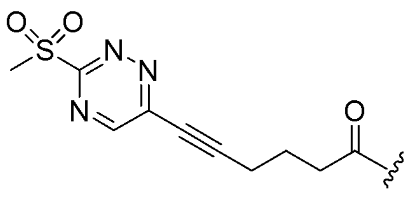,
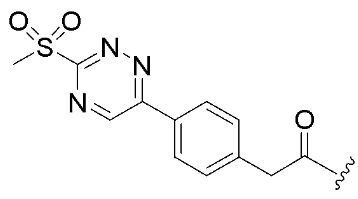,
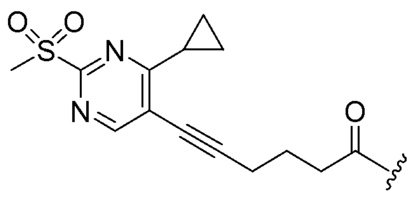,

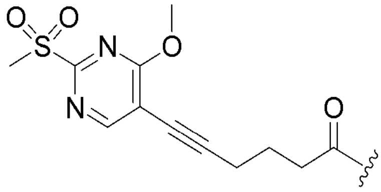,
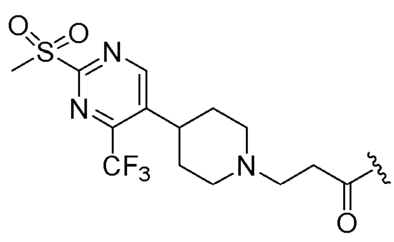,
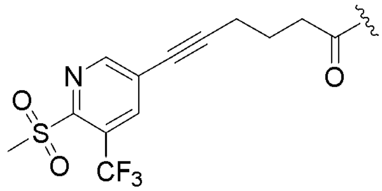,

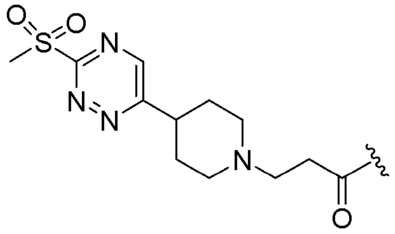 , 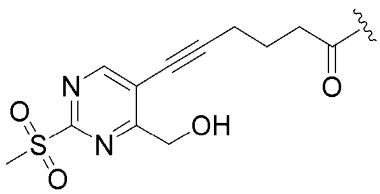 , 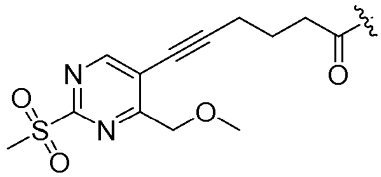 ,

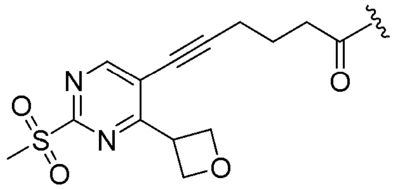 , 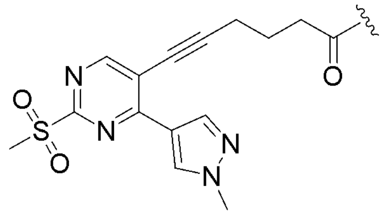 , 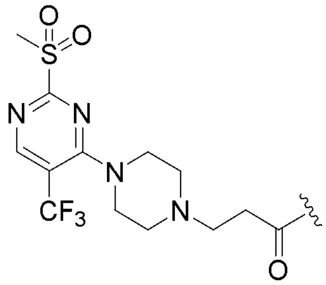 ,

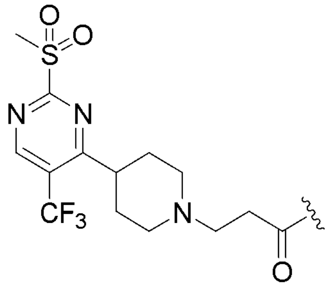 , 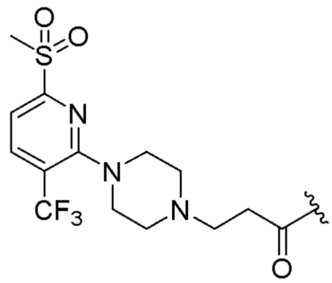 , 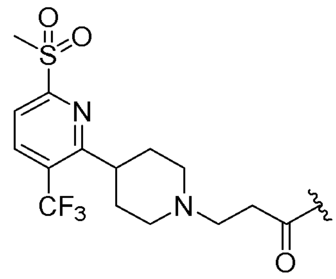 ,

or

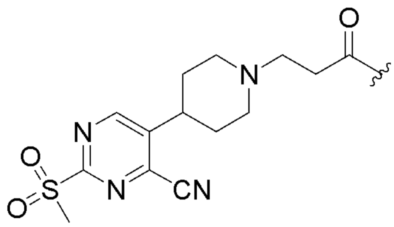 .

According to some embodiments, $L_1$ is glycine-glycine-phenylalanine-glycine (GGFG), i.e.

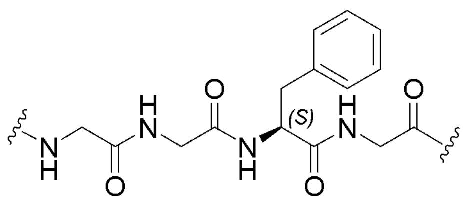

GGFG .

According to some embodiments, $L_2$ is -NH-$CH_2$.

According to some embodiments, M-$L_1$- $L_2$ is selected from the following groups:

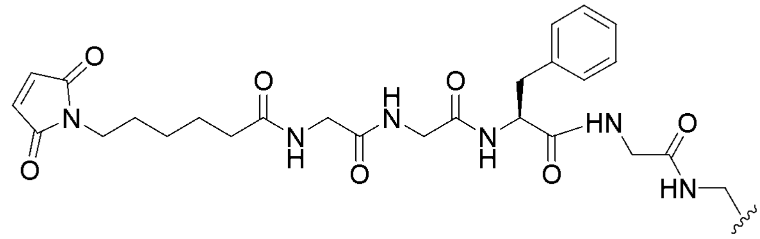

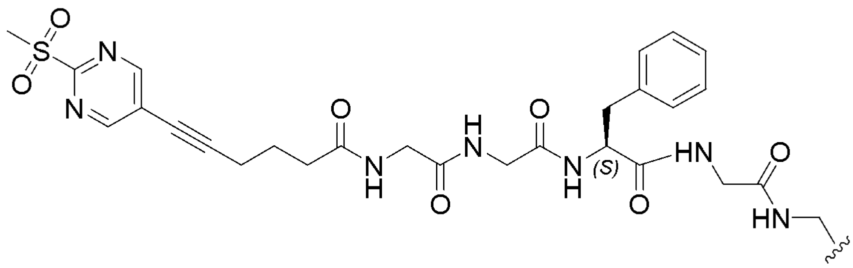

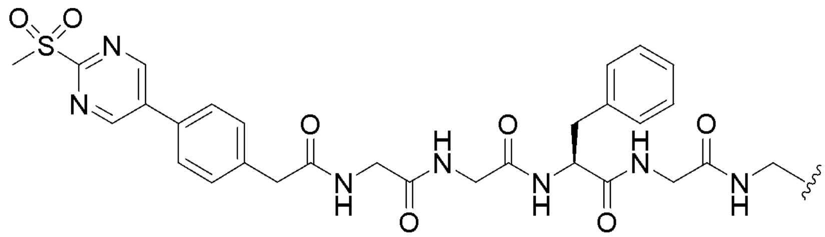

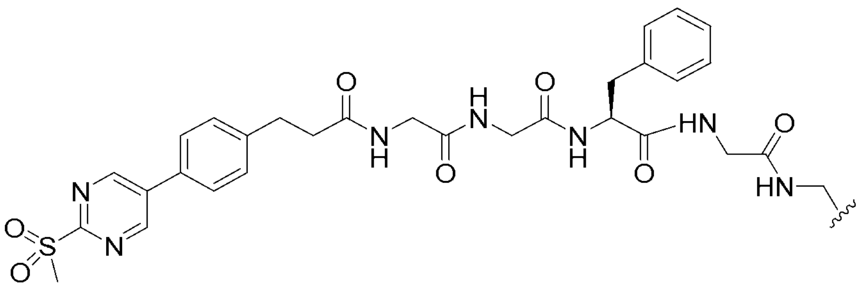

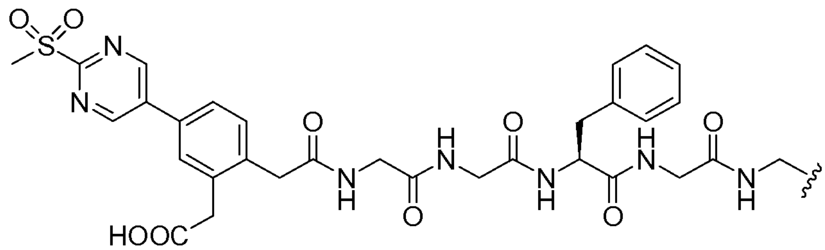

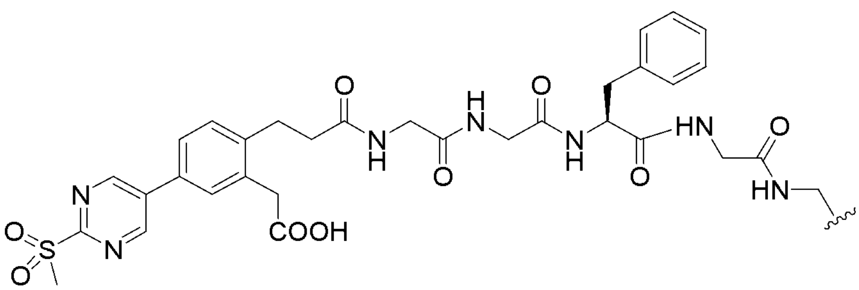

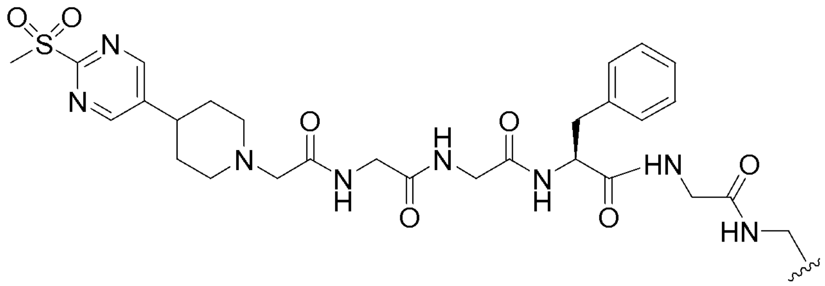

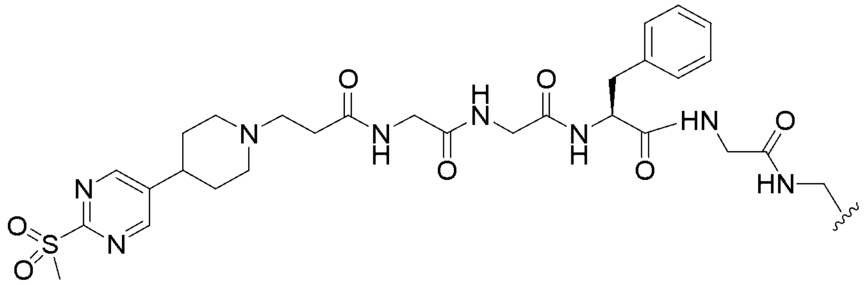

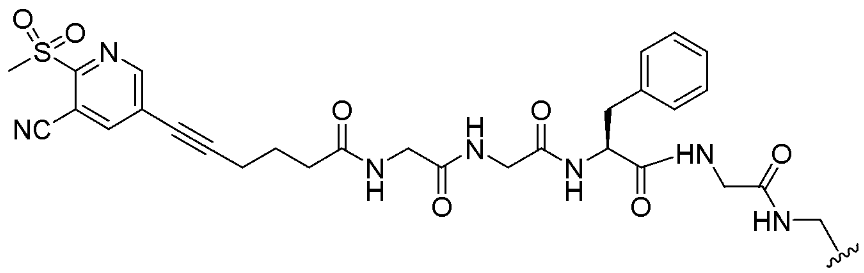

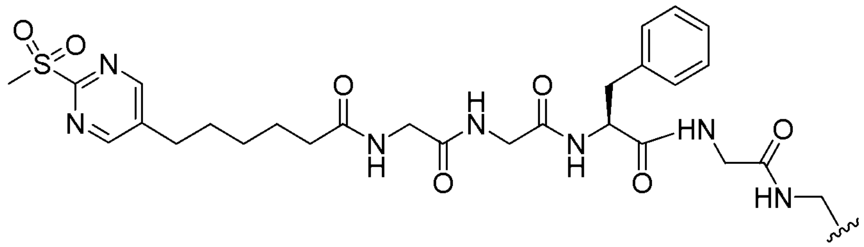

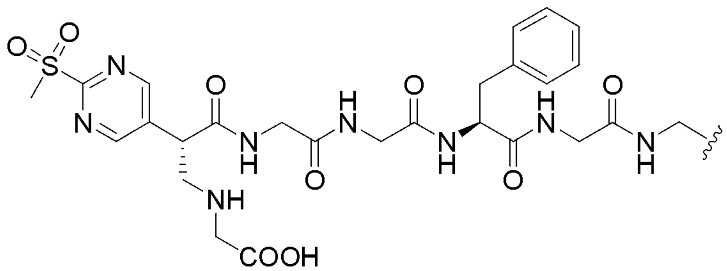

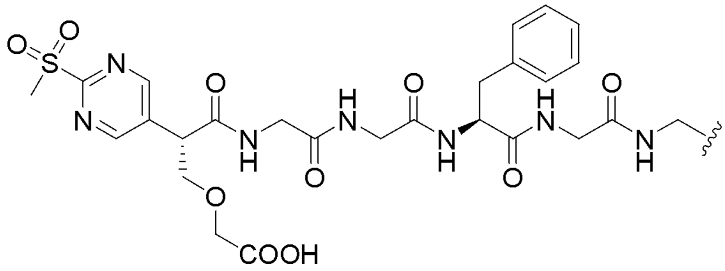

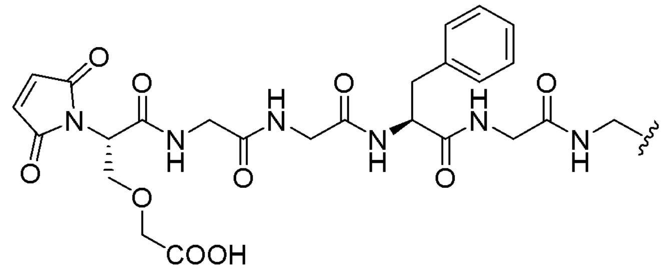

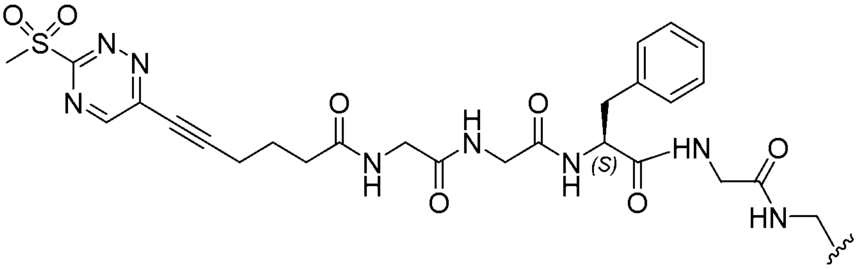

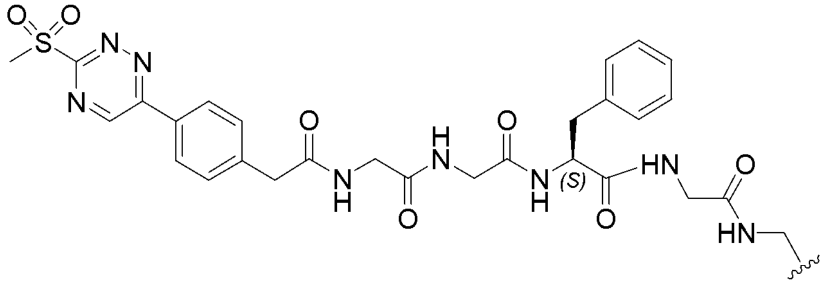

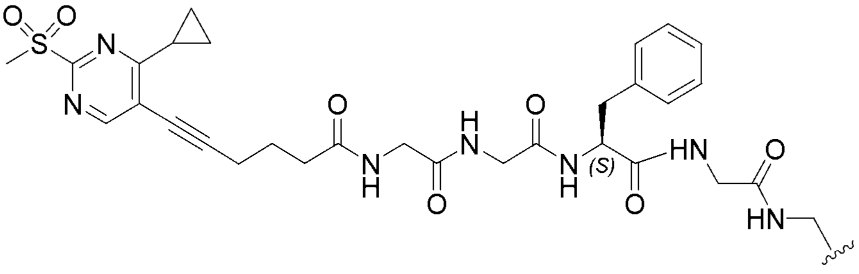

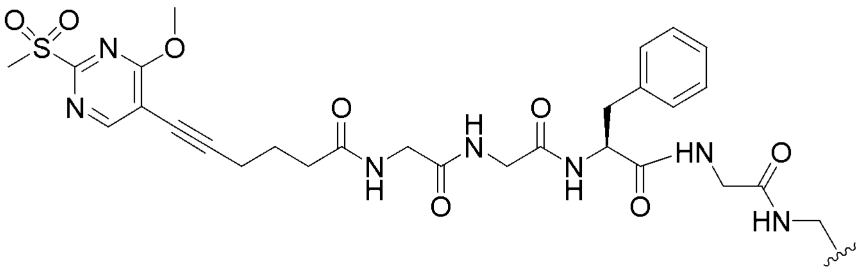

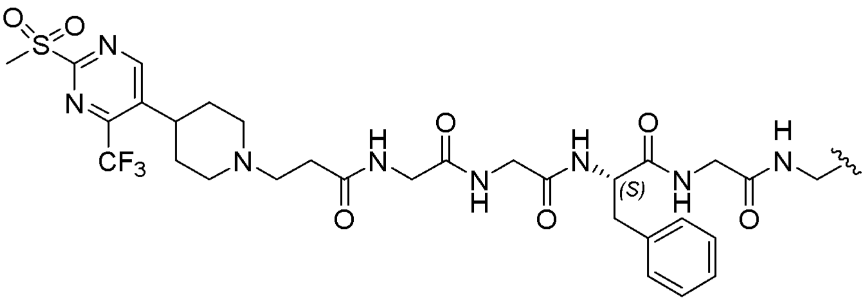

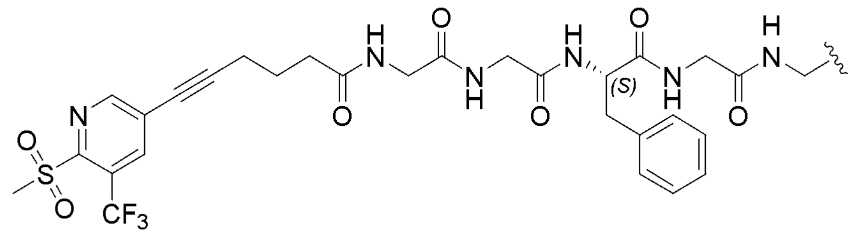

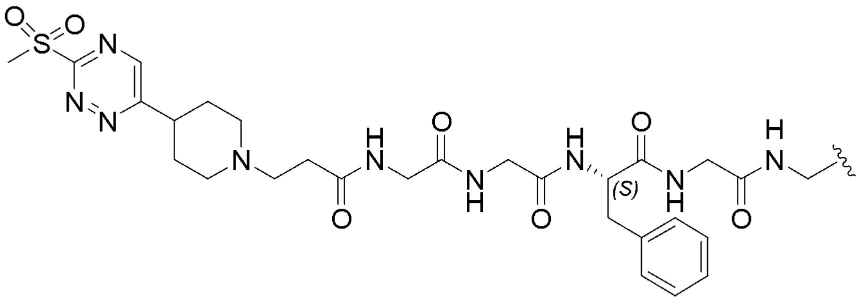

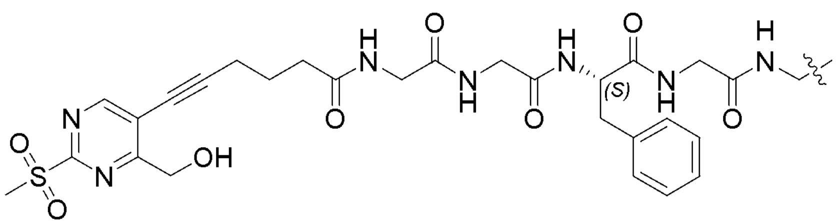

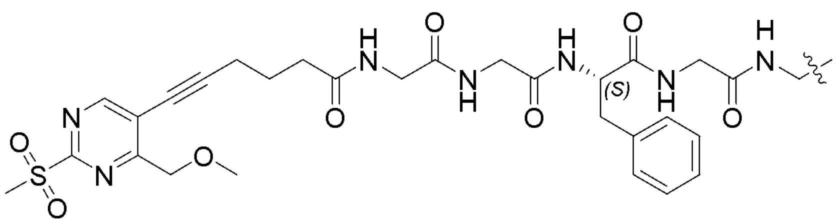

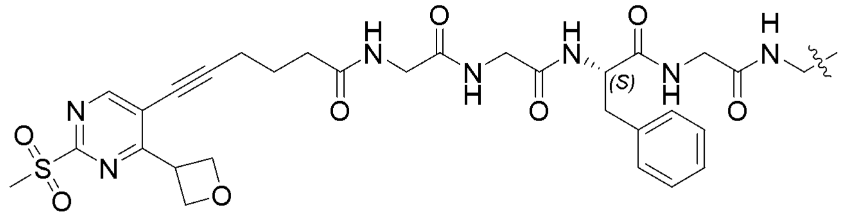

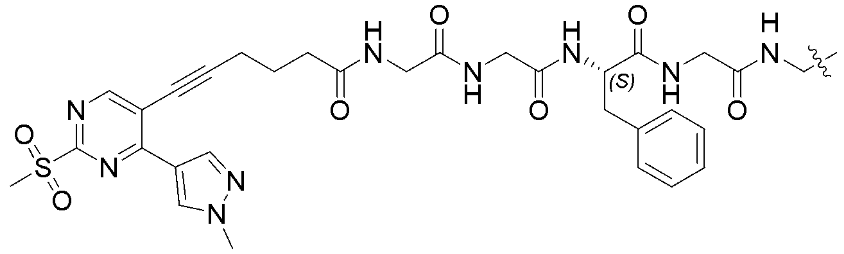

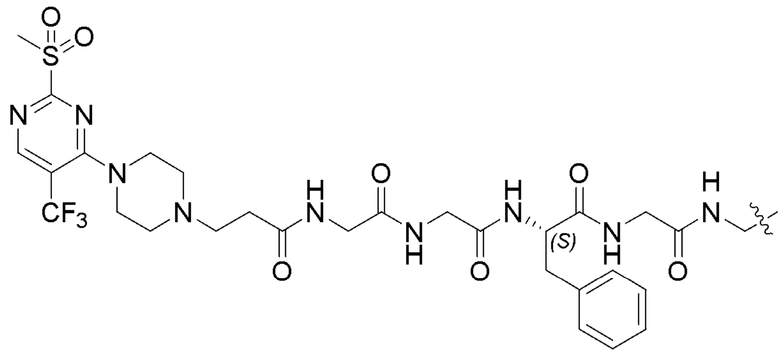

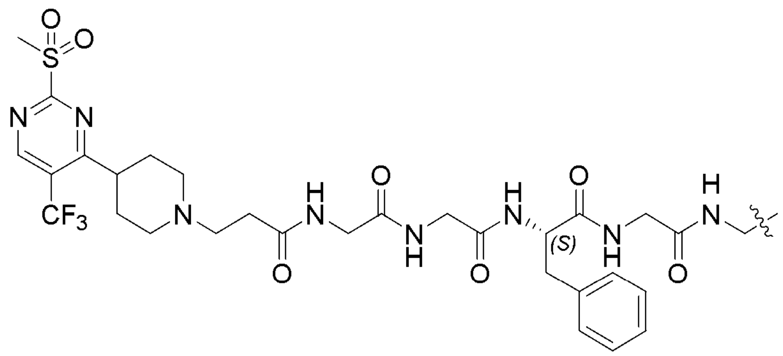

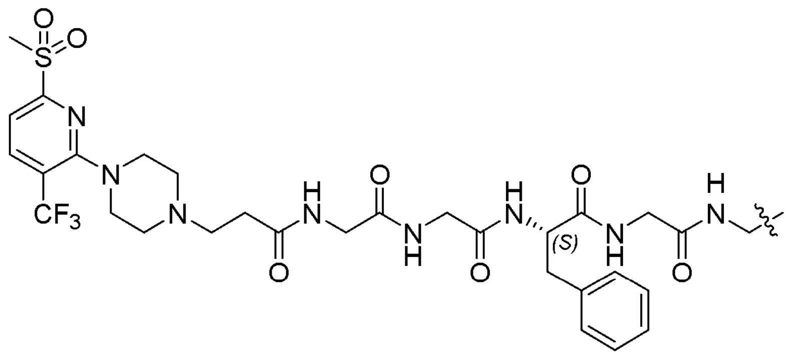

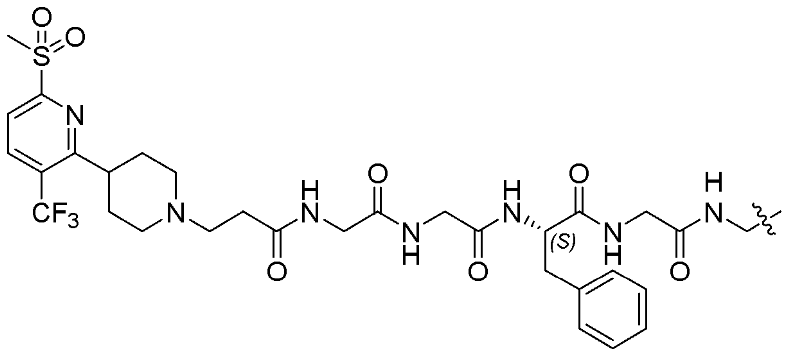

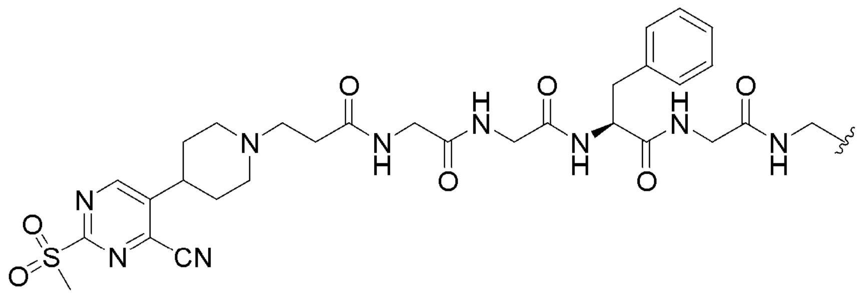

.

According to some embodiments, the compound represented by formula V is selected from the following structures:

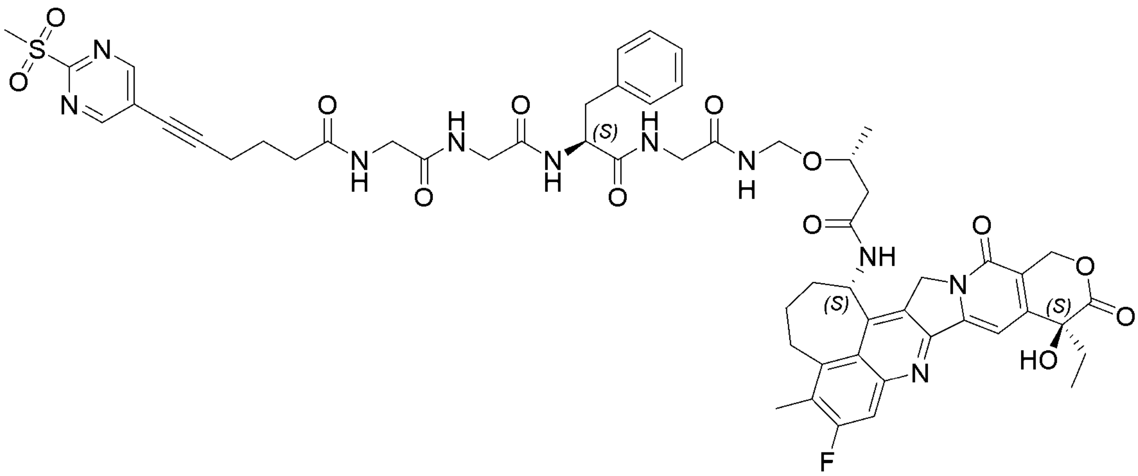

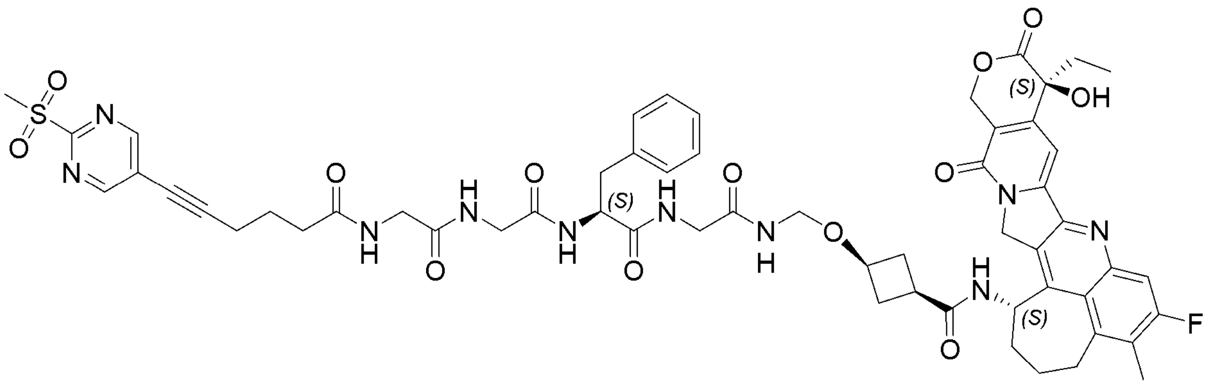

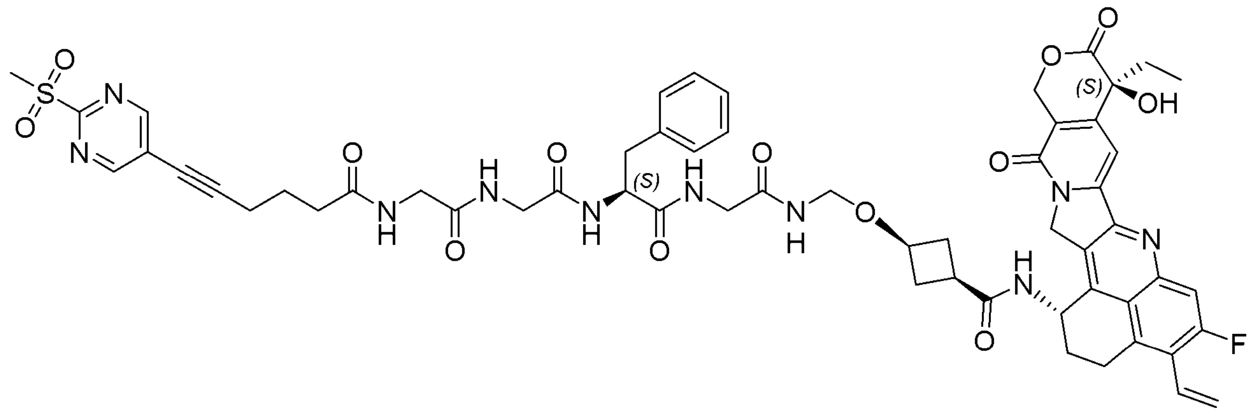

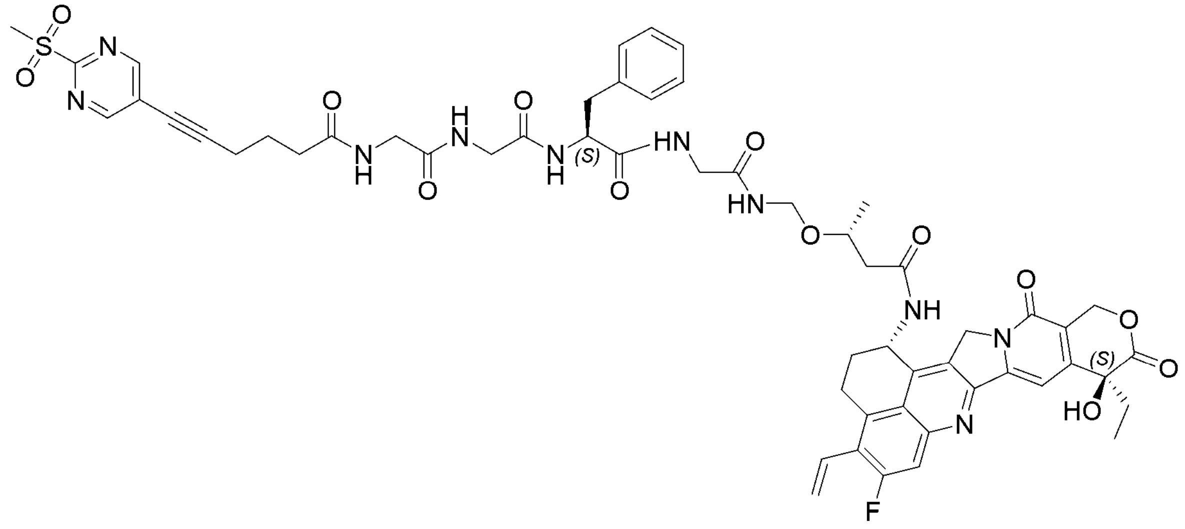

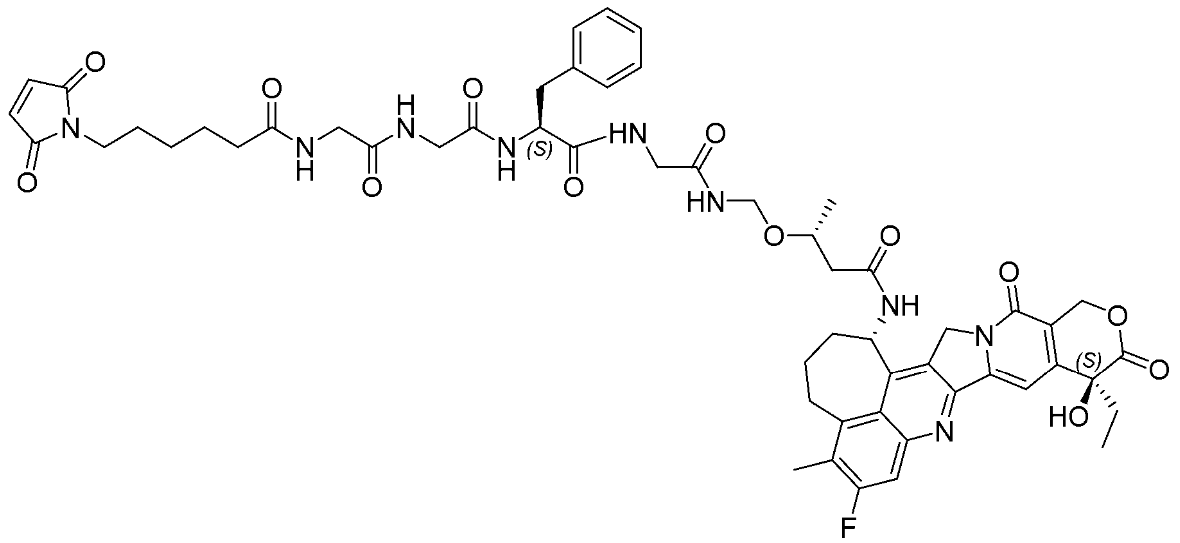

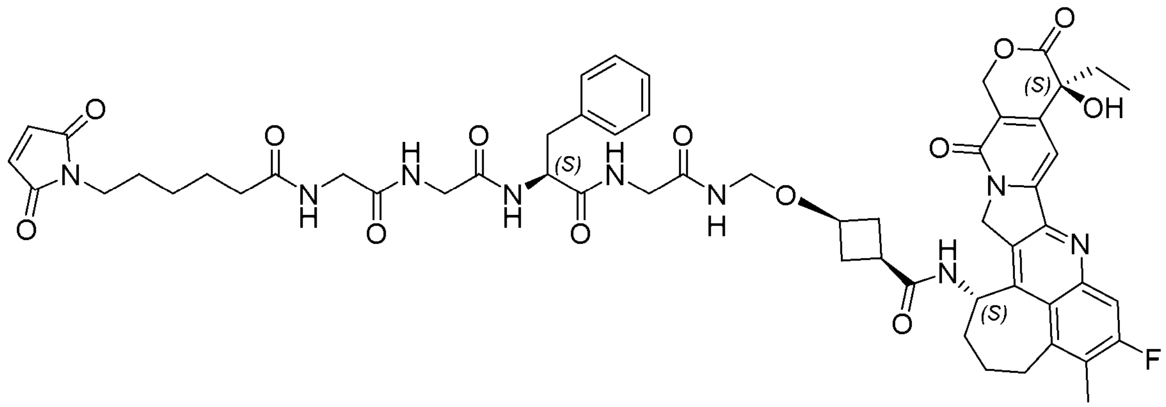

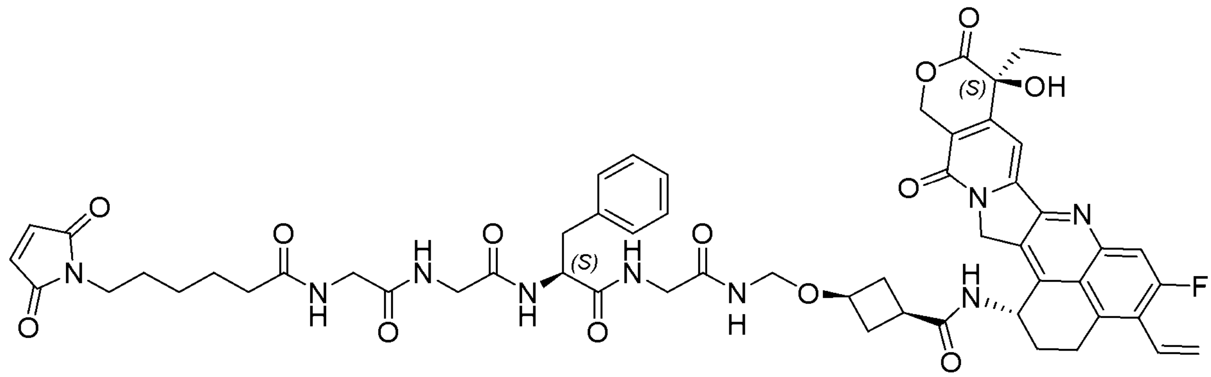

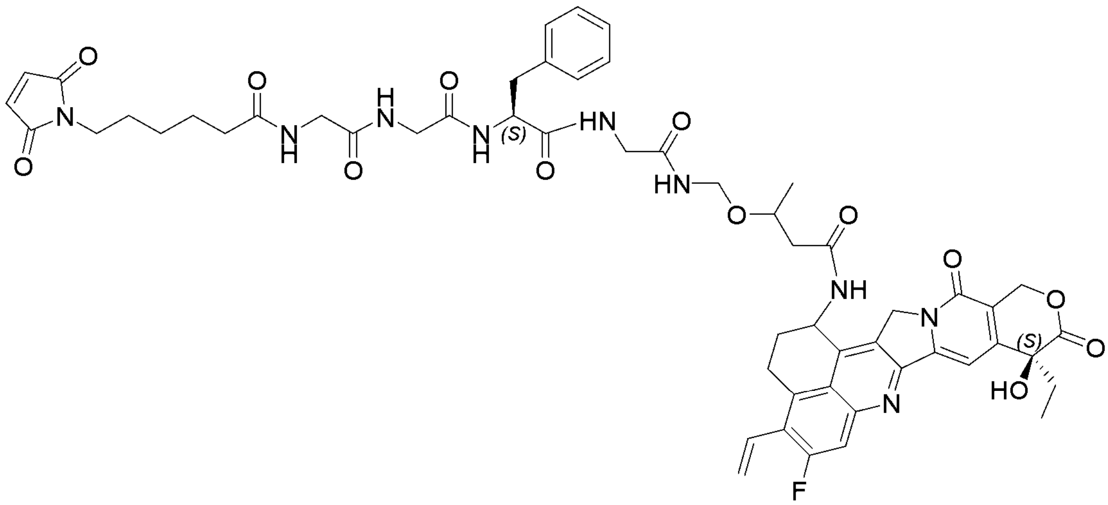

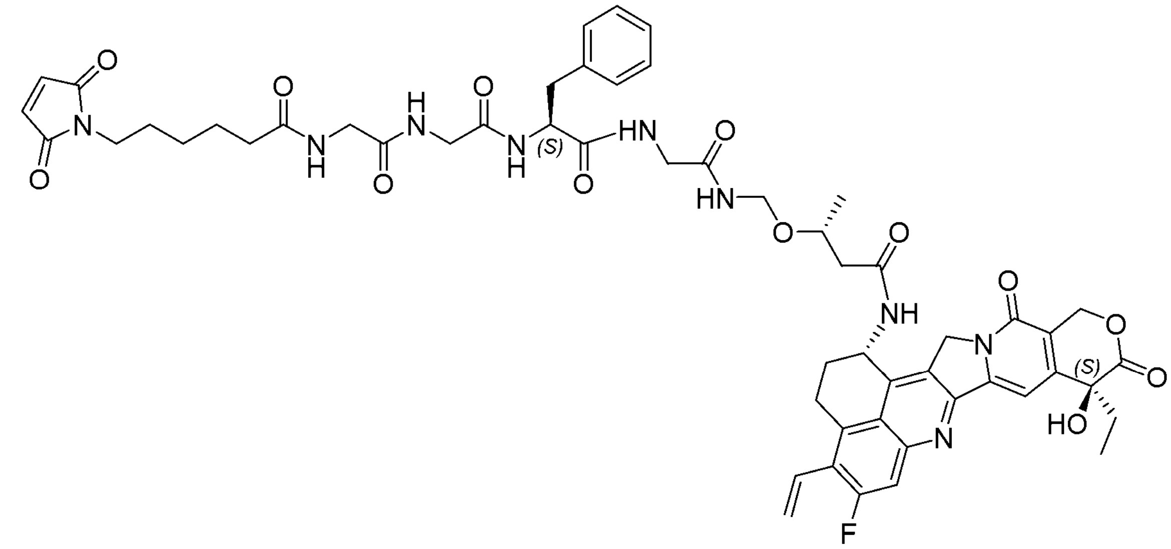

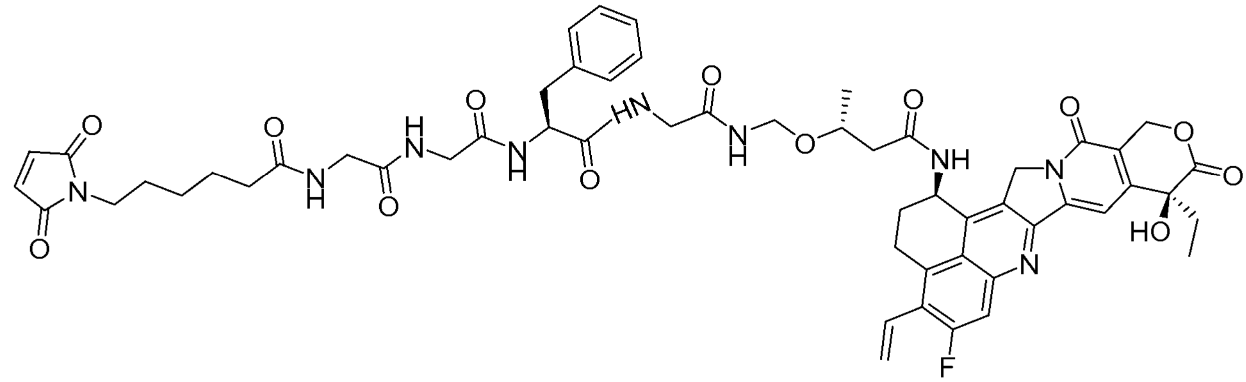

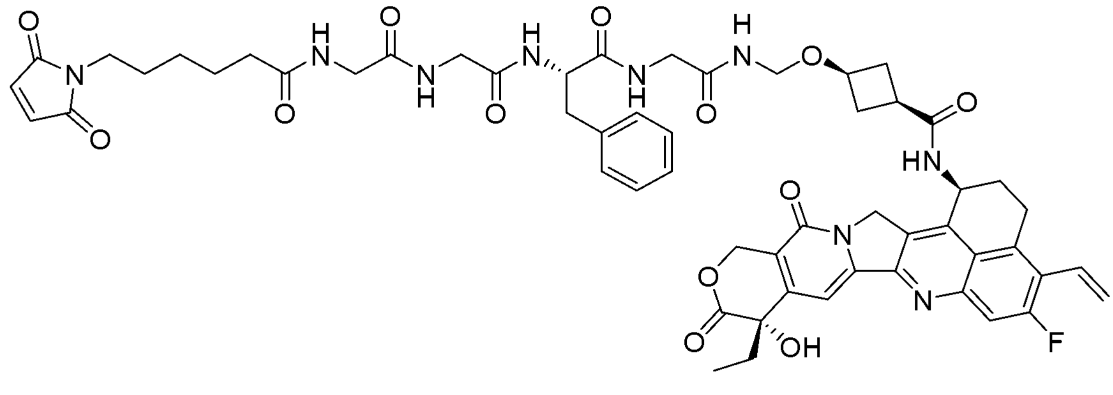

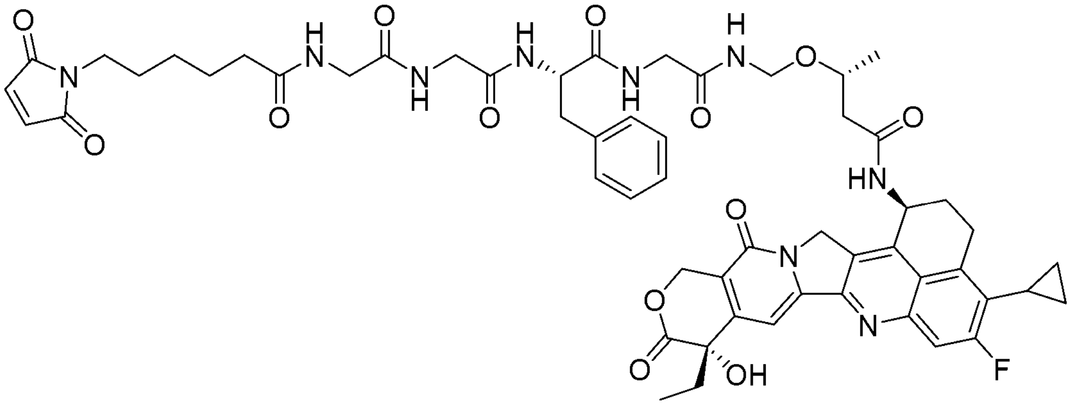

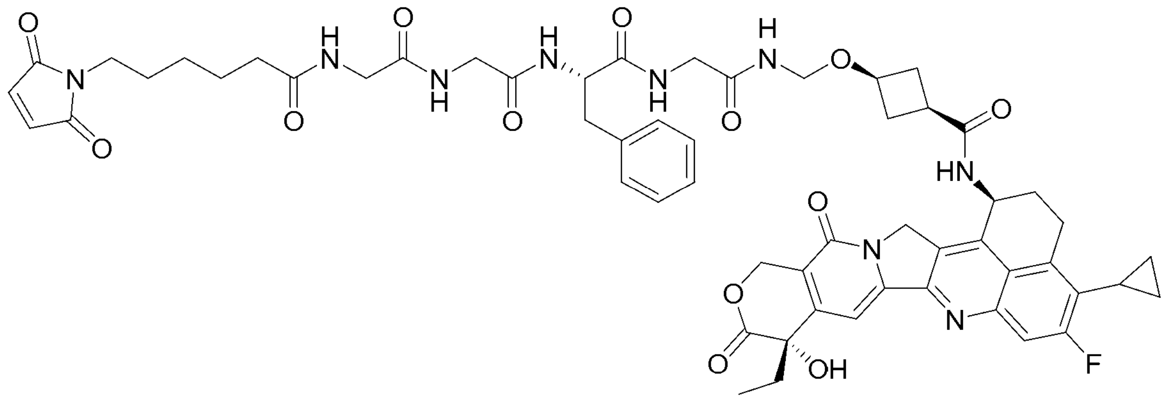

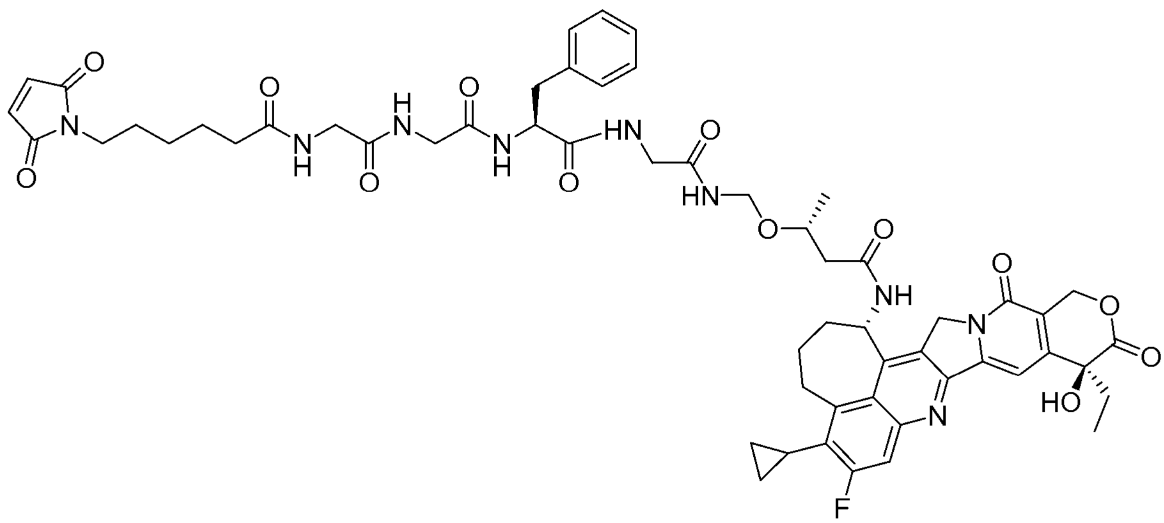

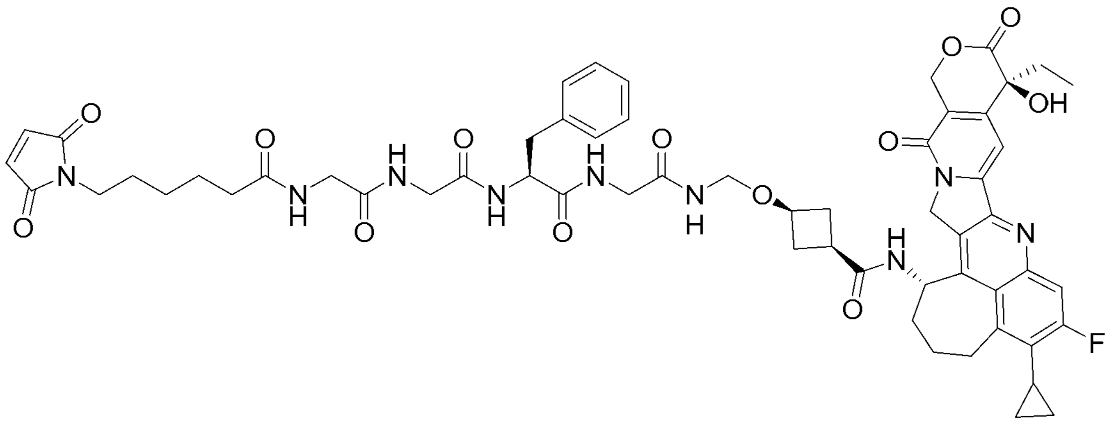

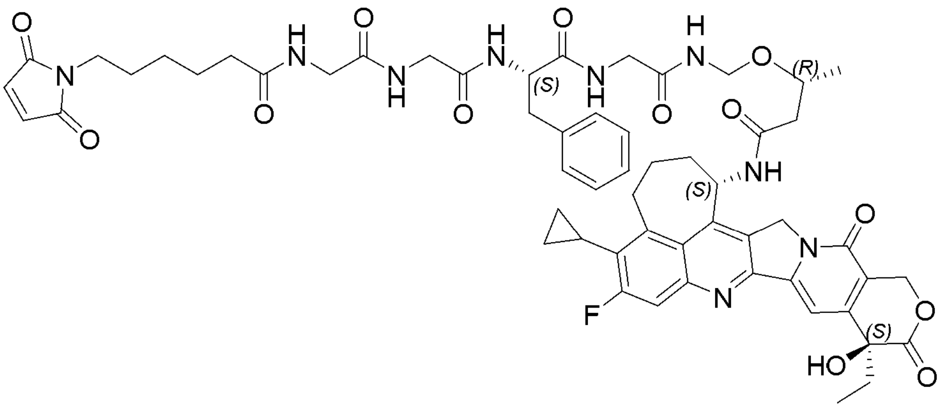

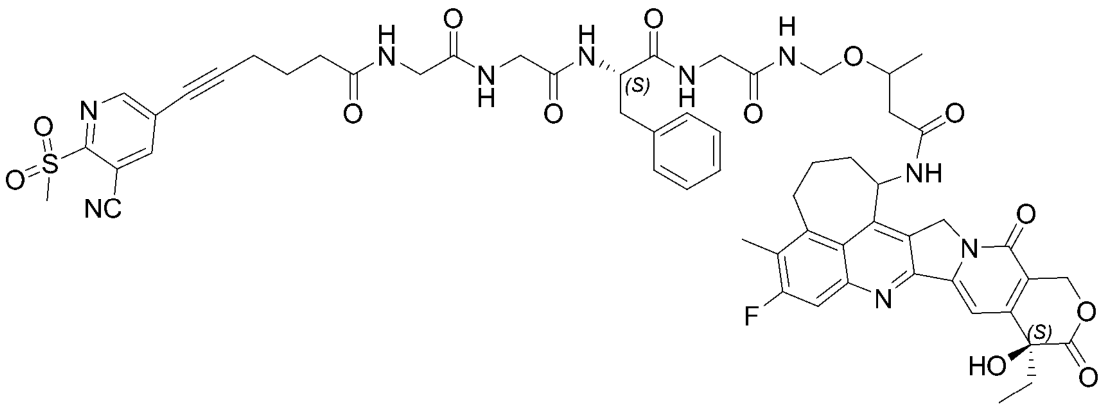

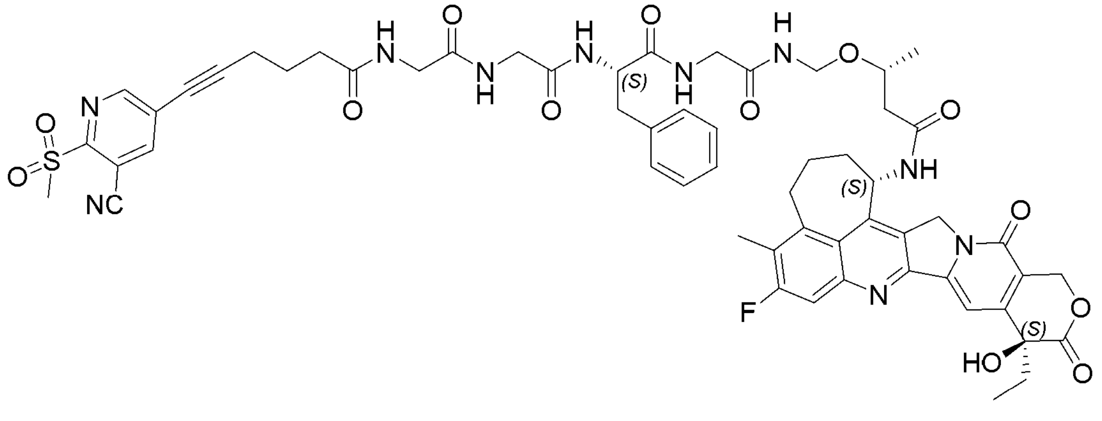

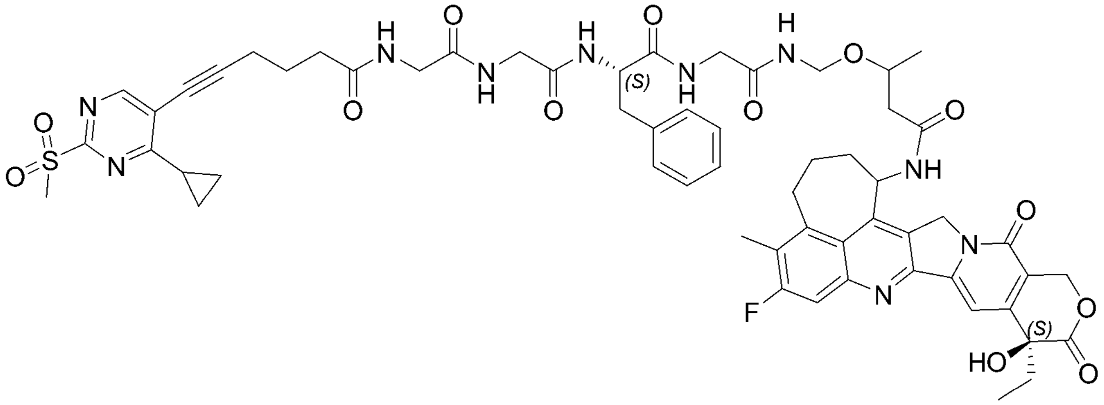

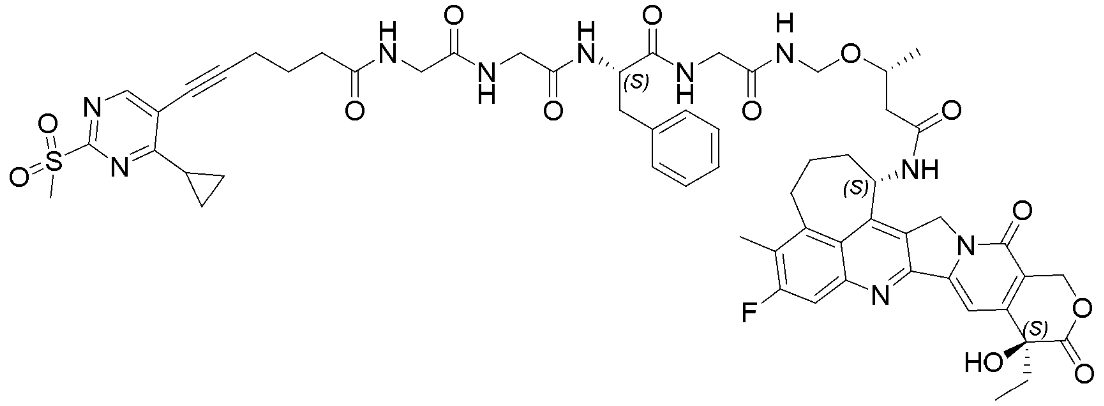

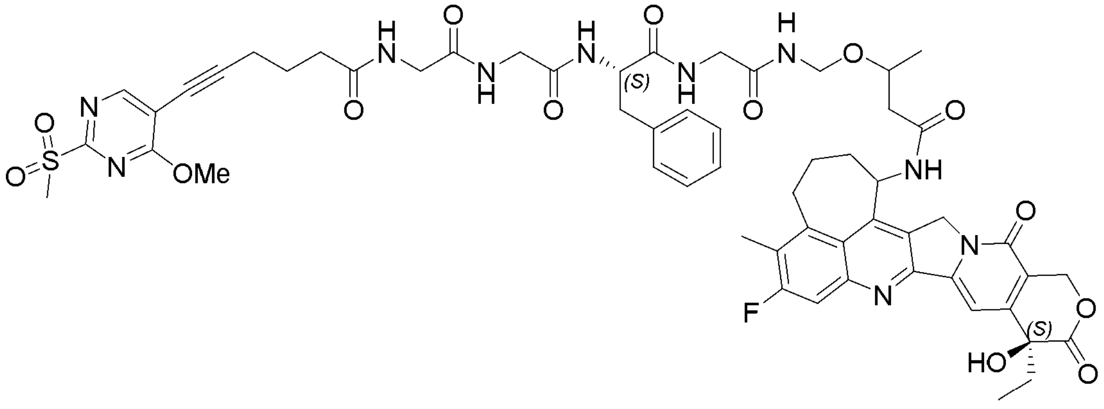

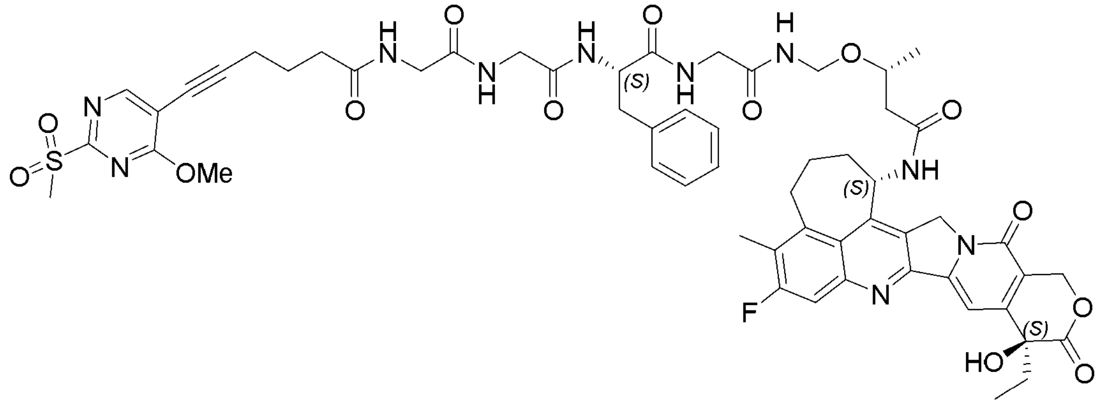

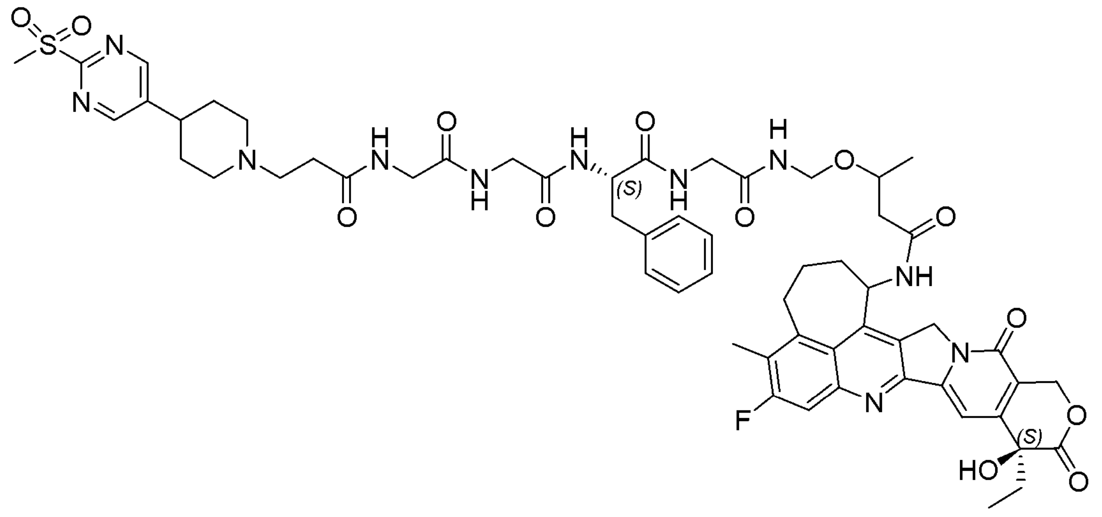

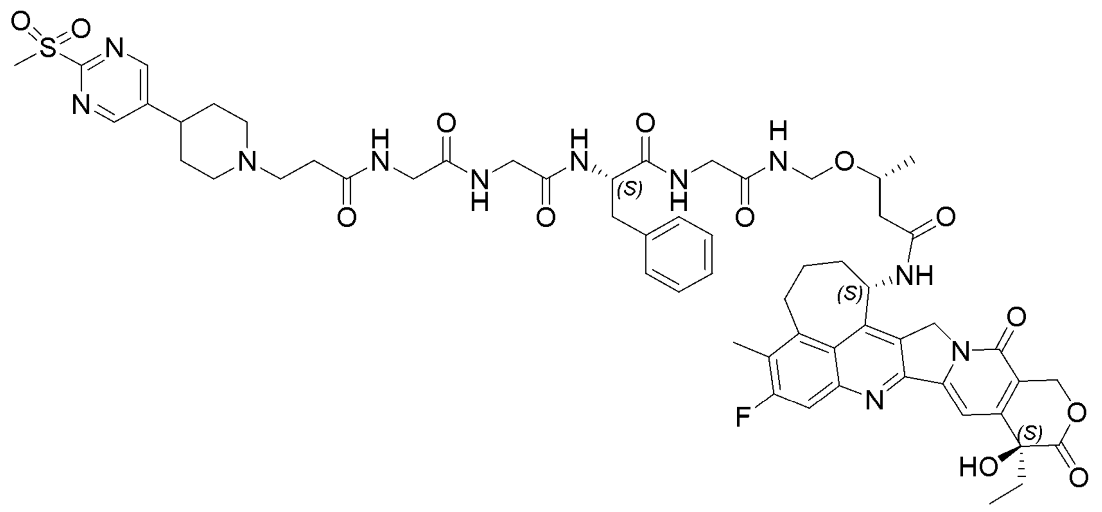

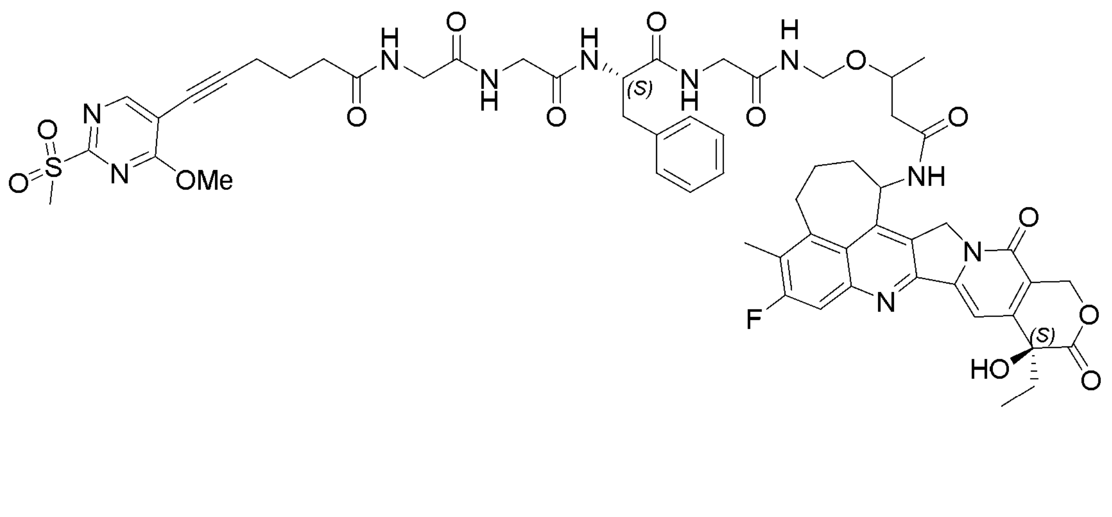

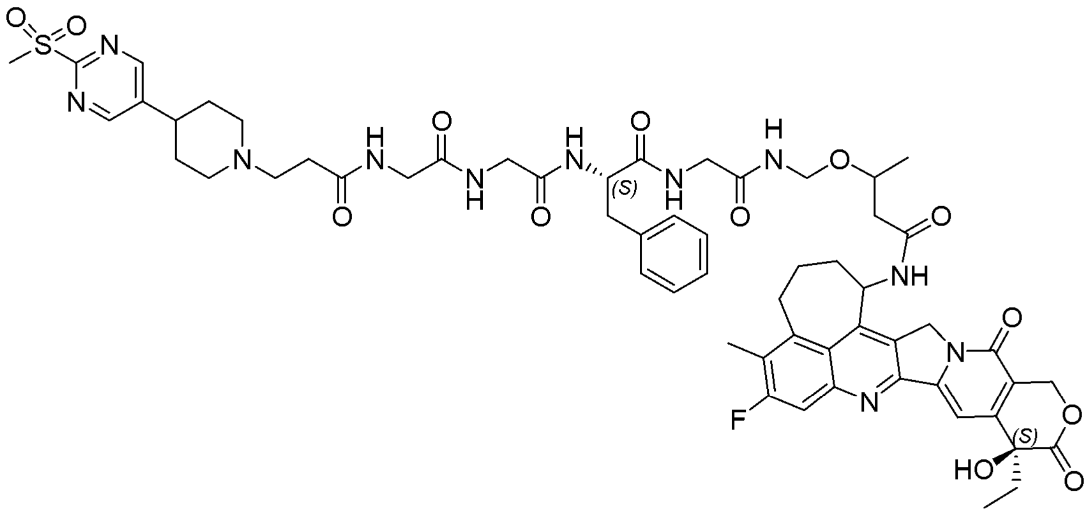

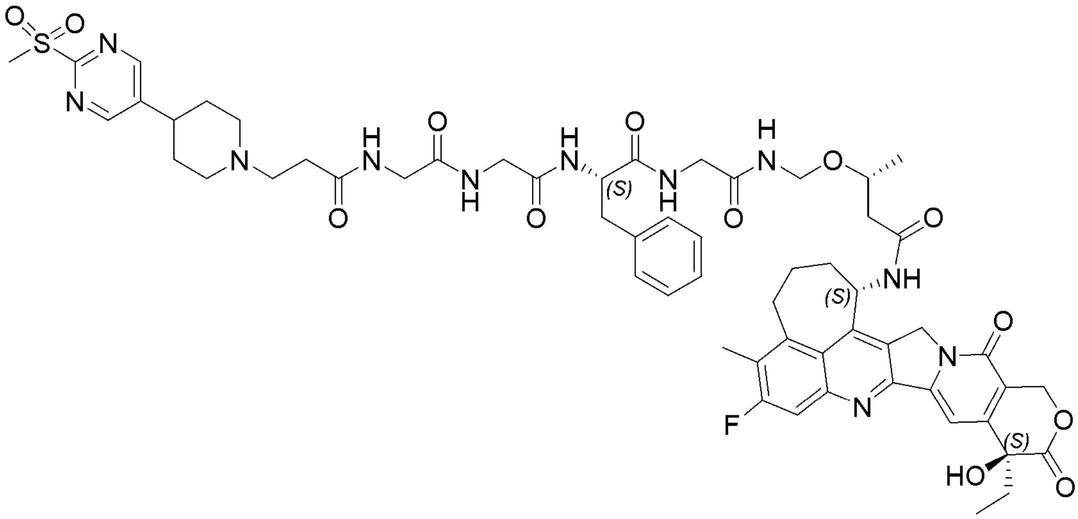

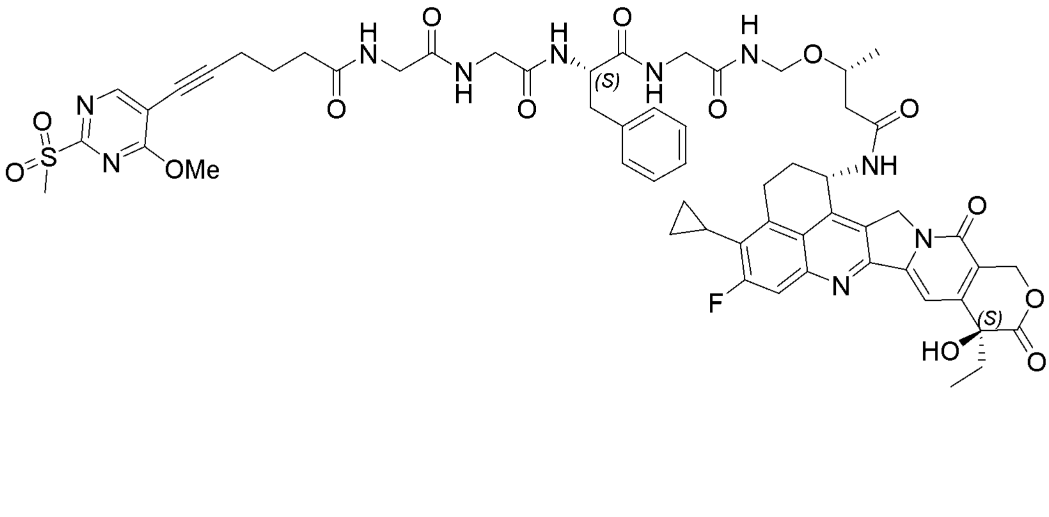

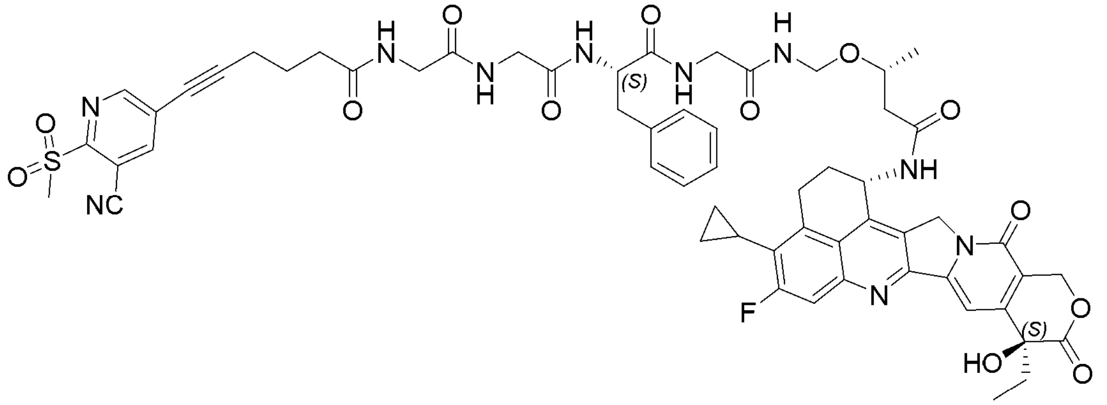

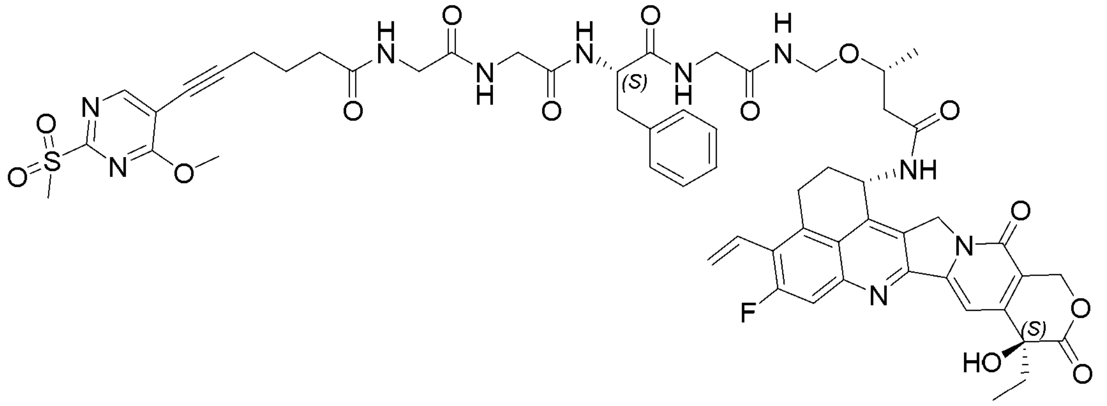

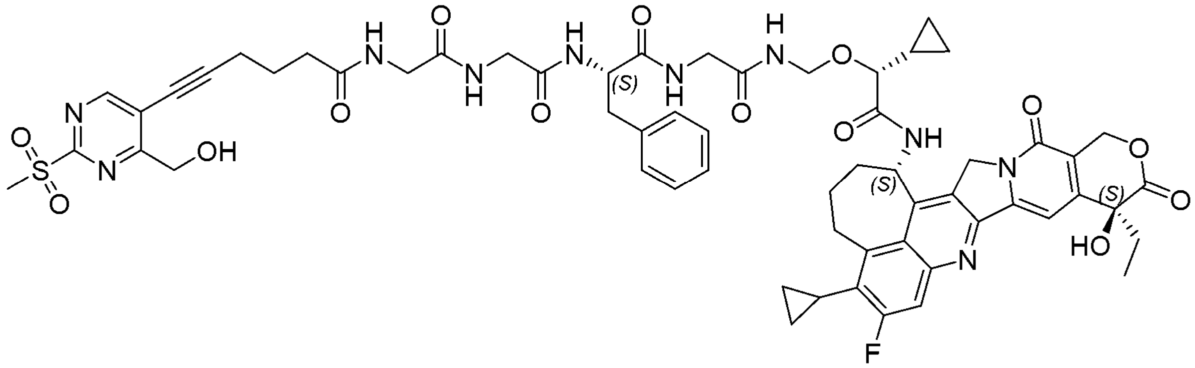

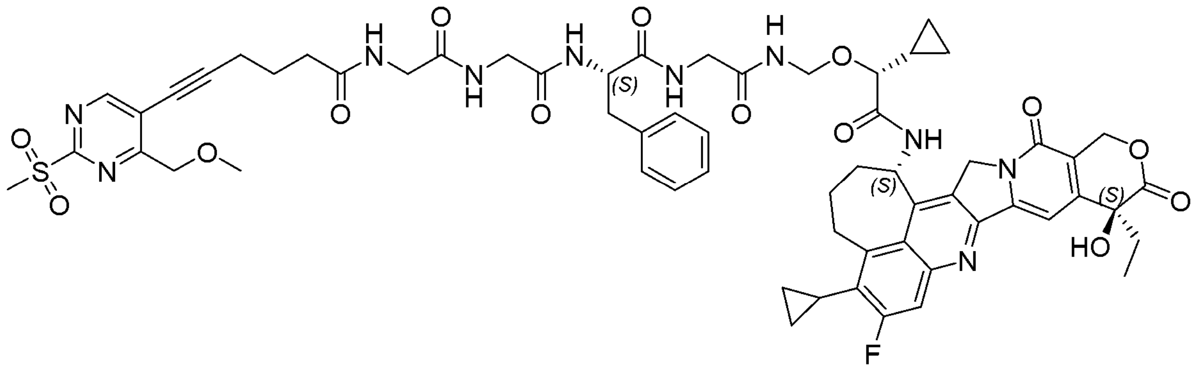

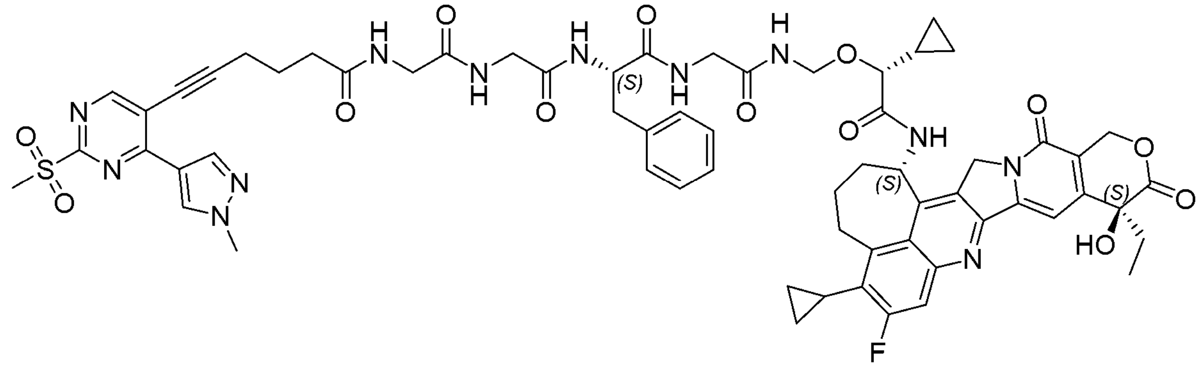

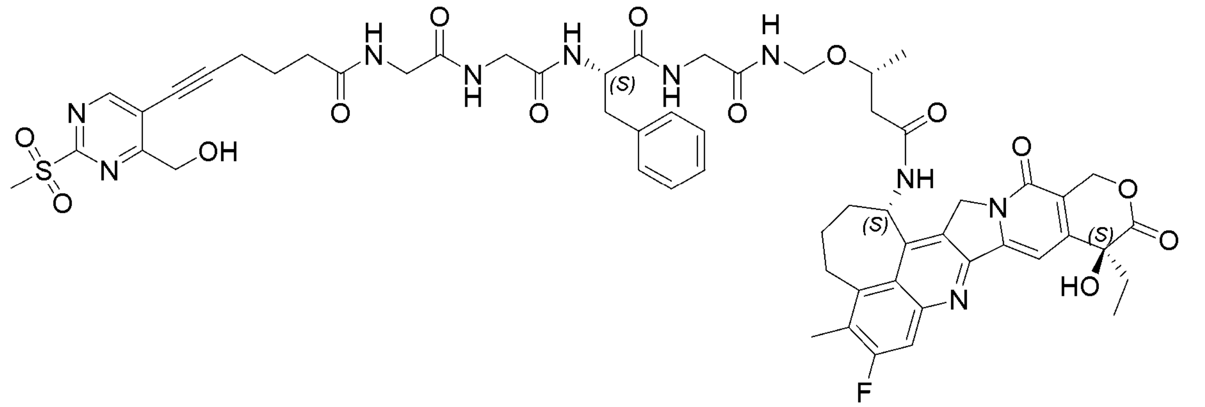

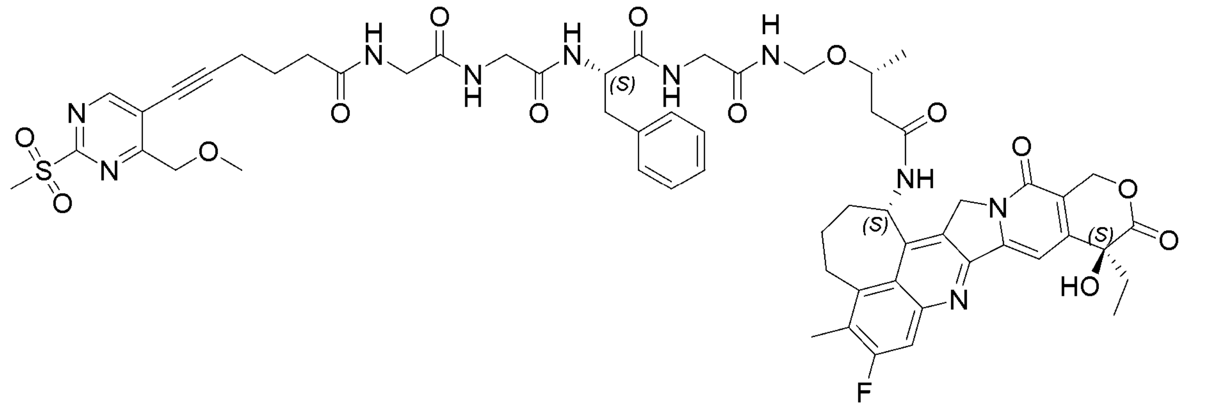

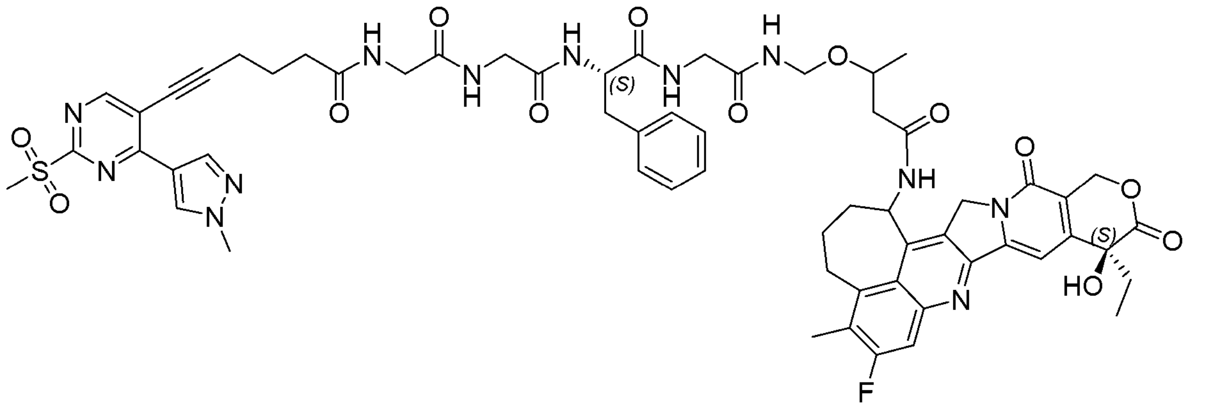

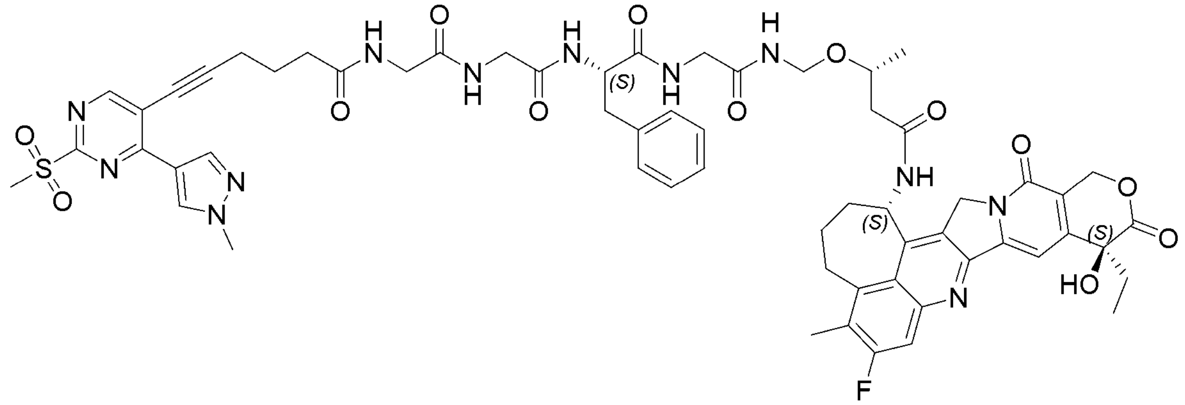

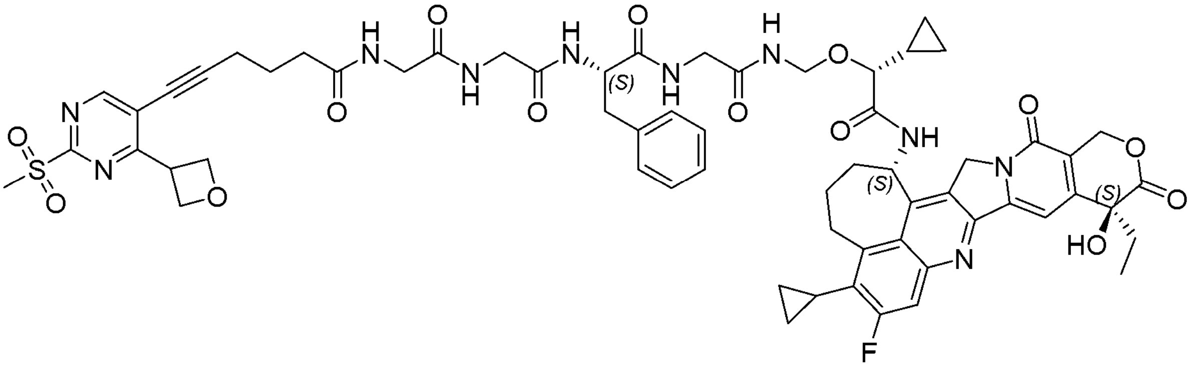

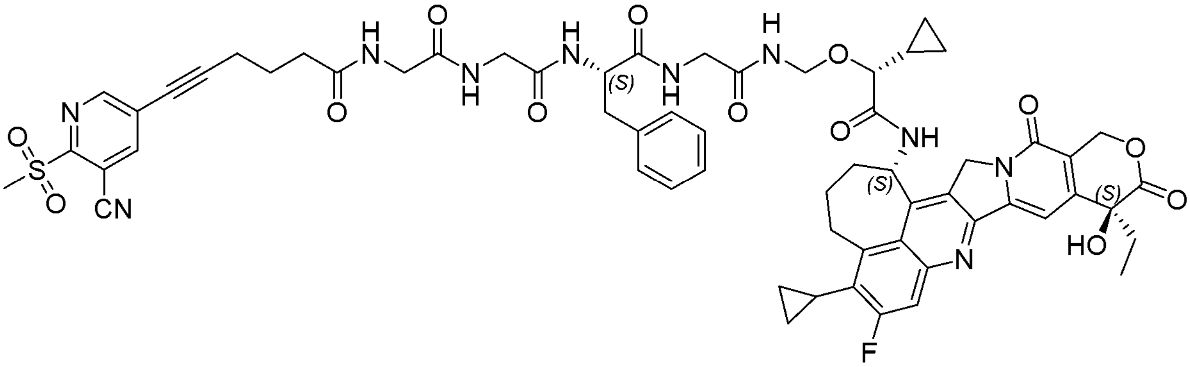

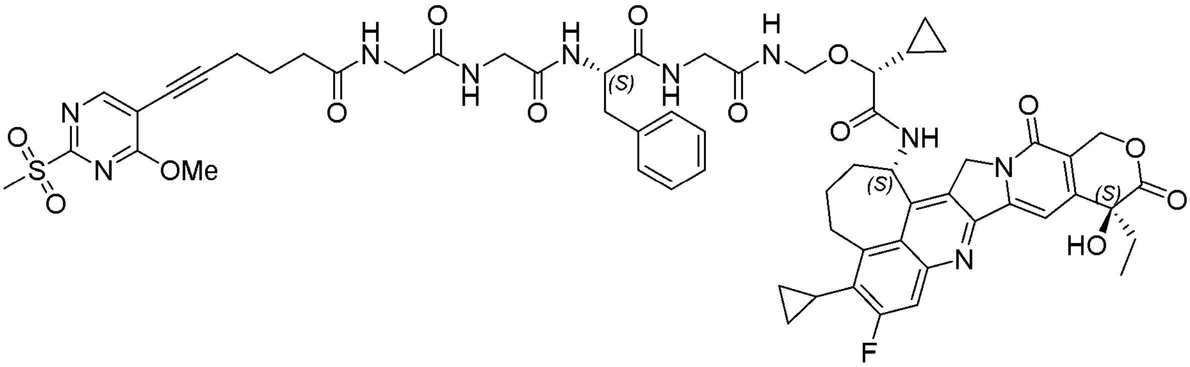

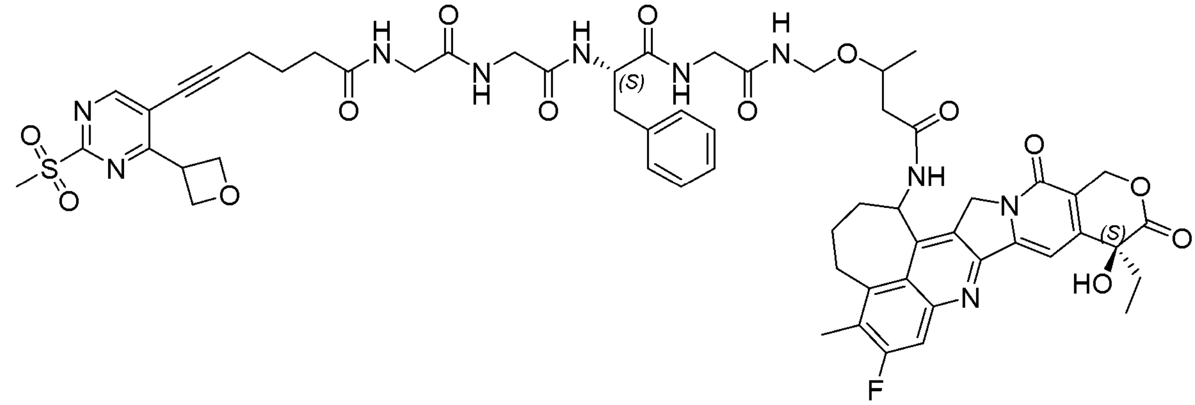

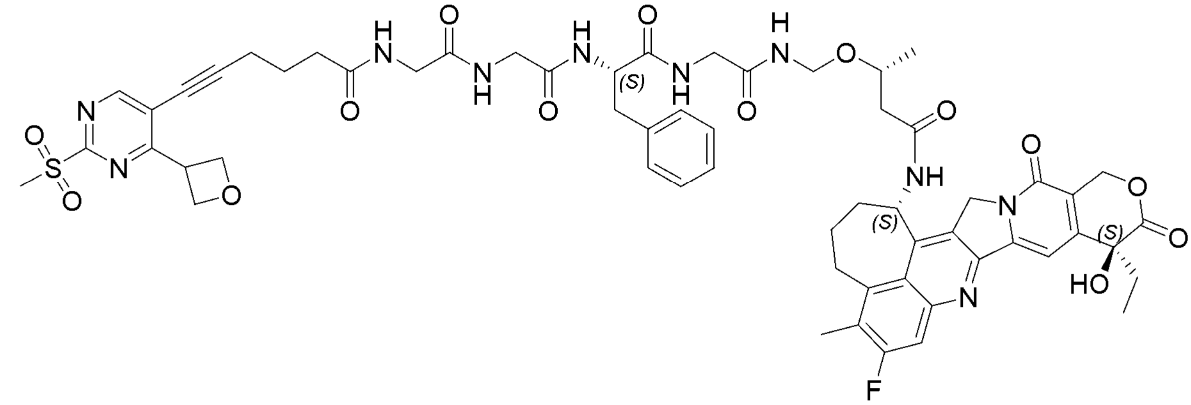

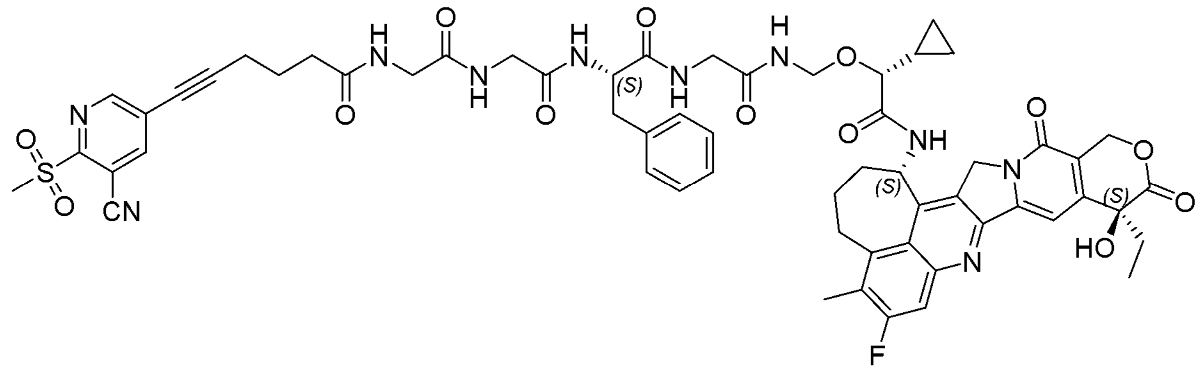

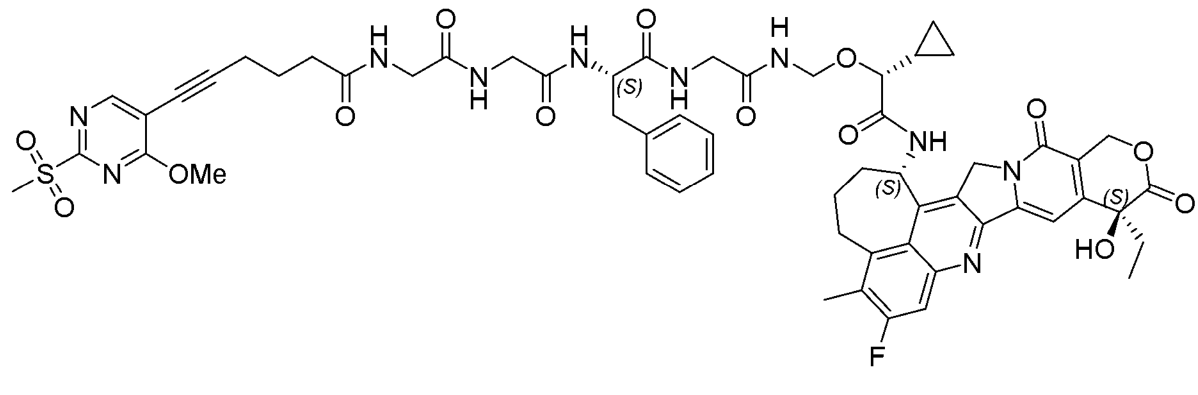

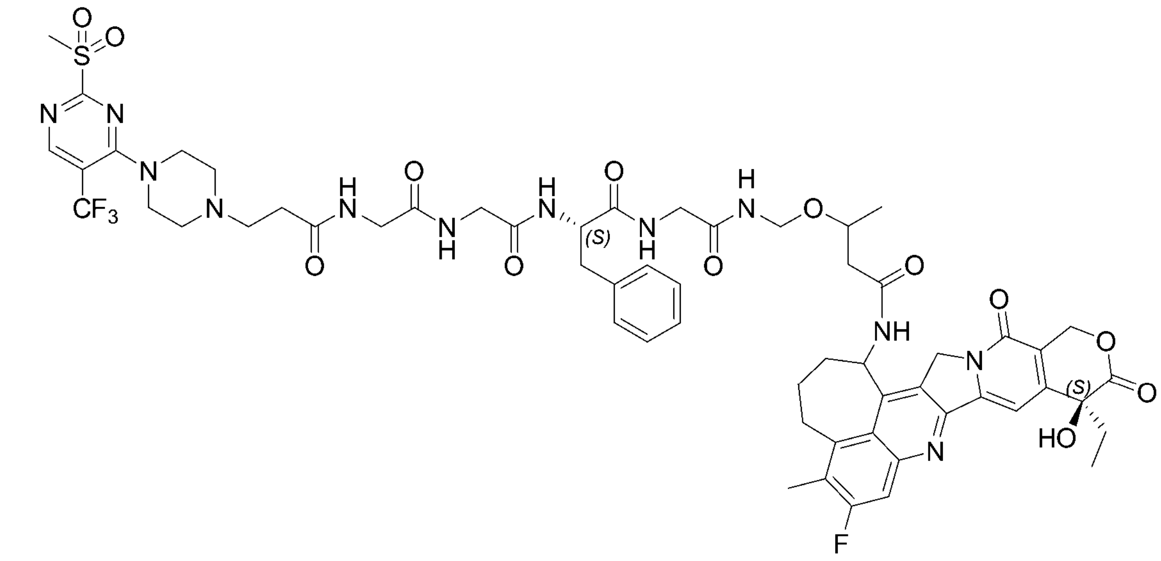

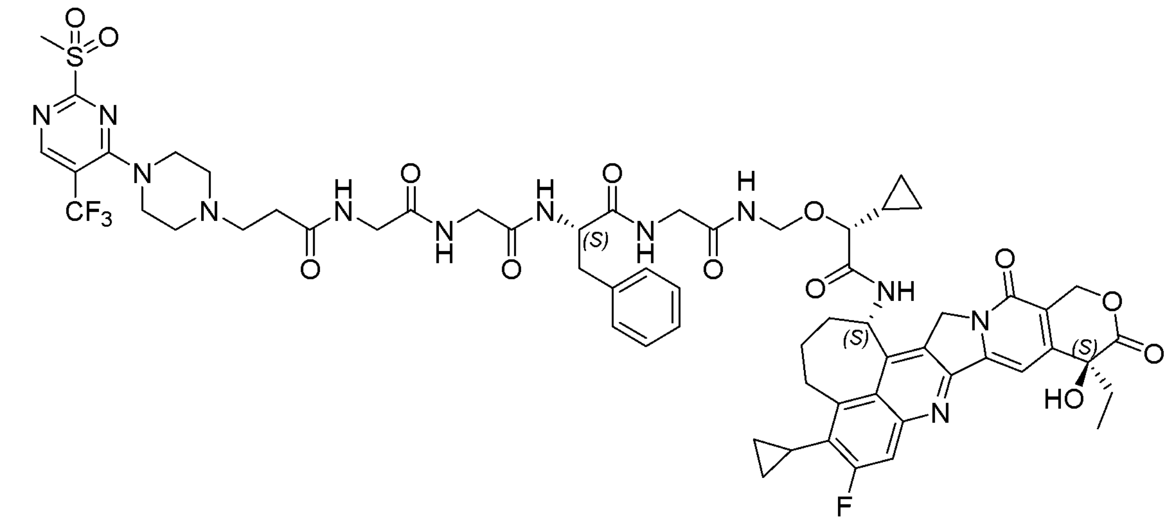

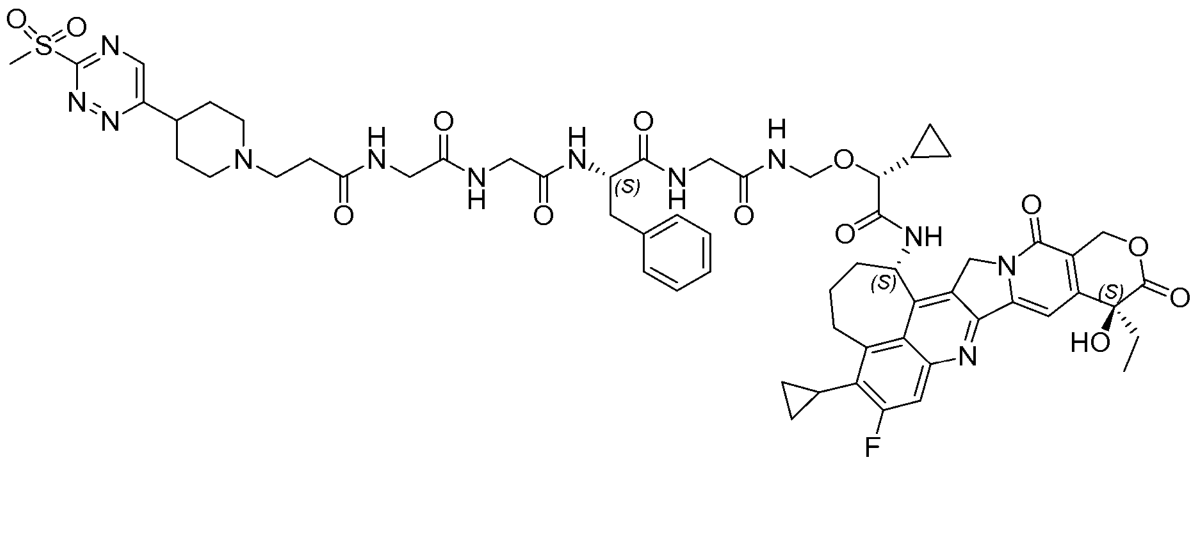

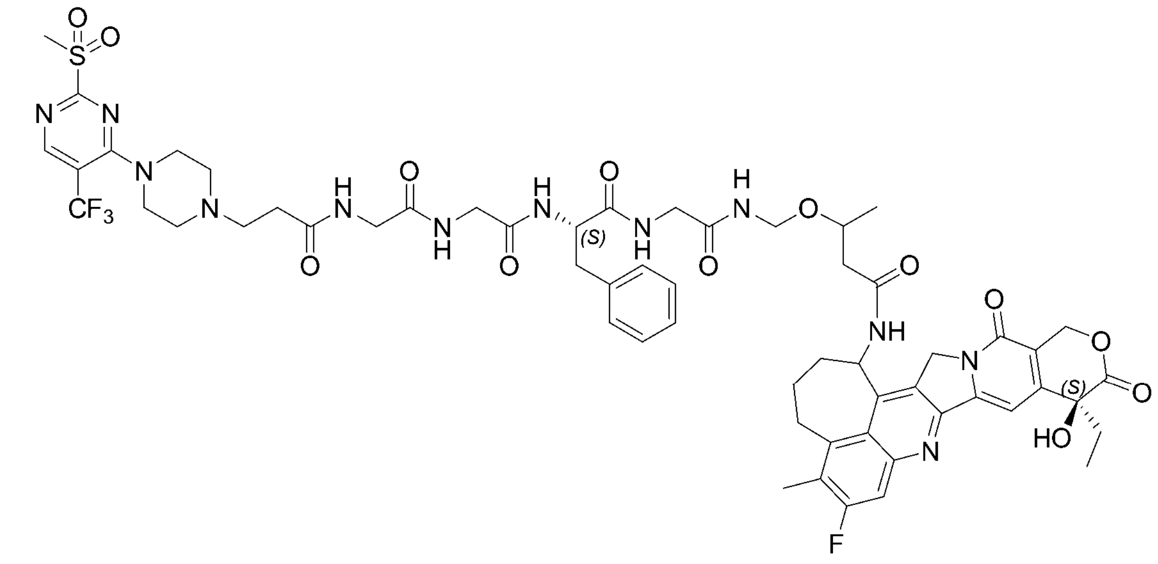

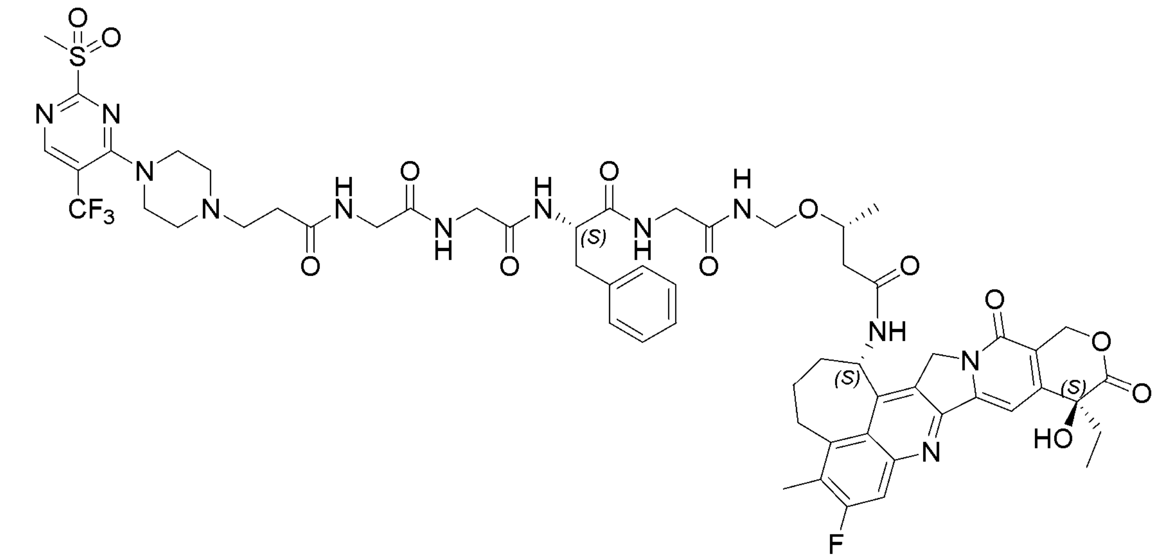

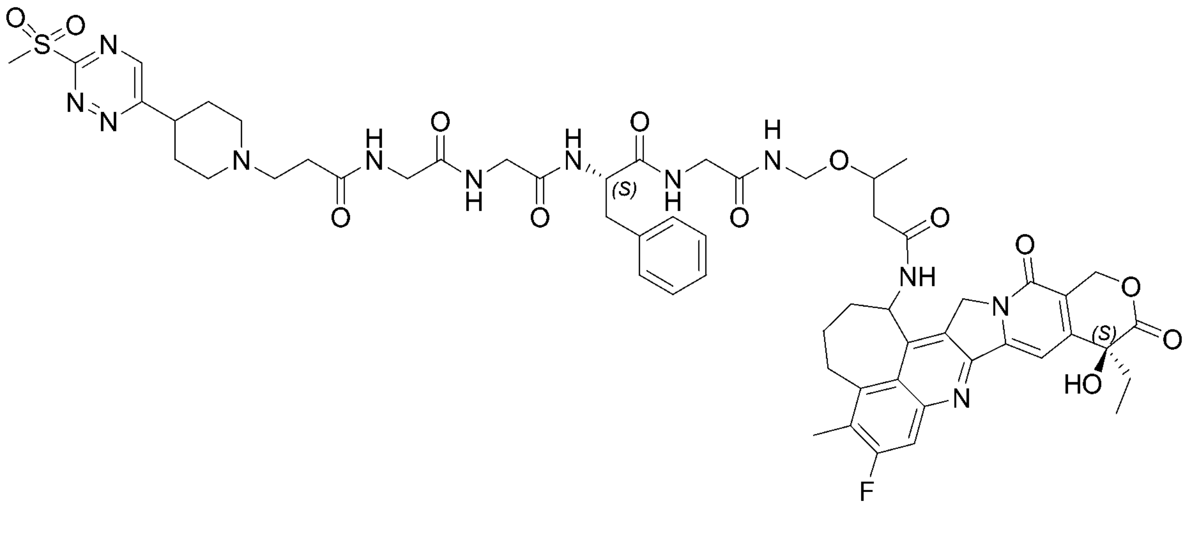

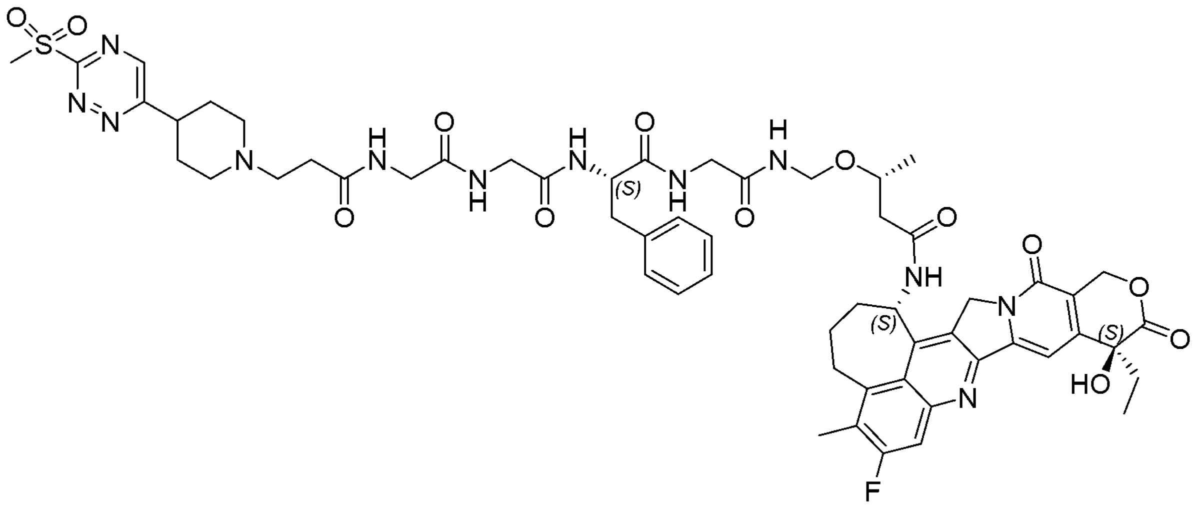

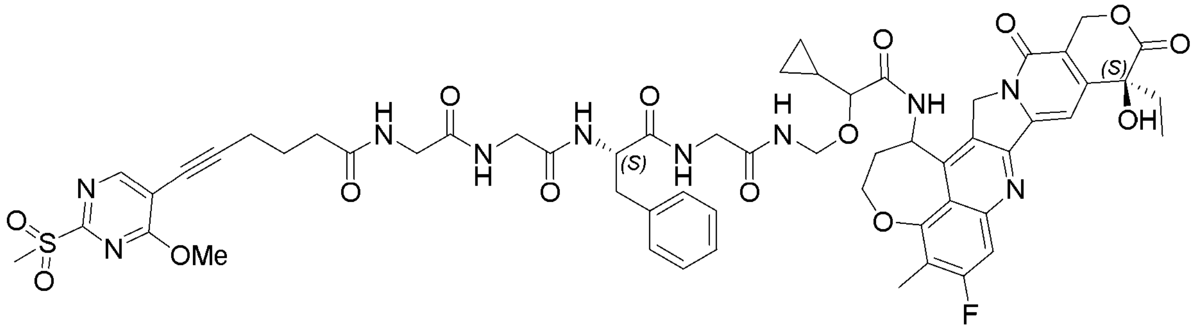

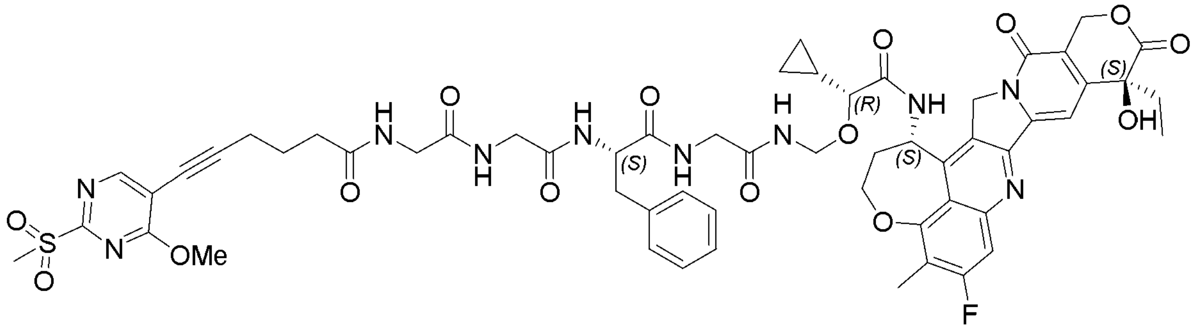

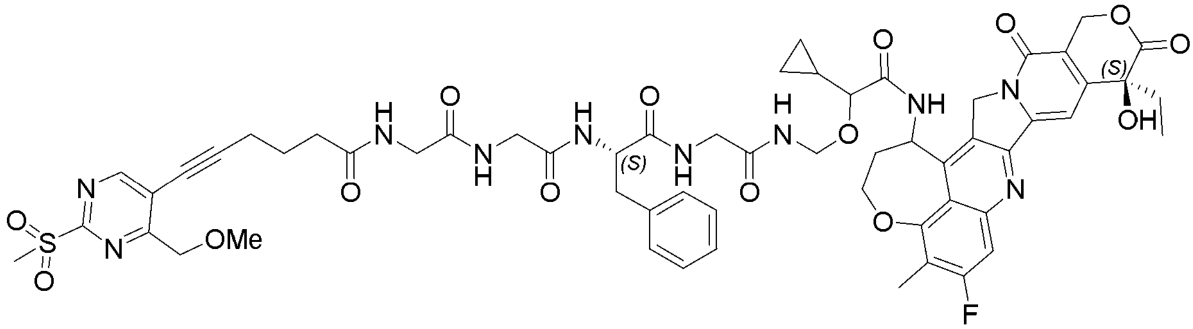

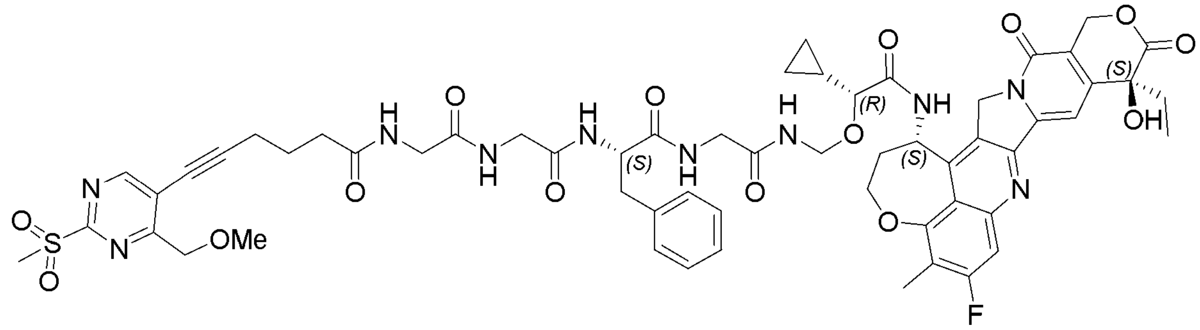

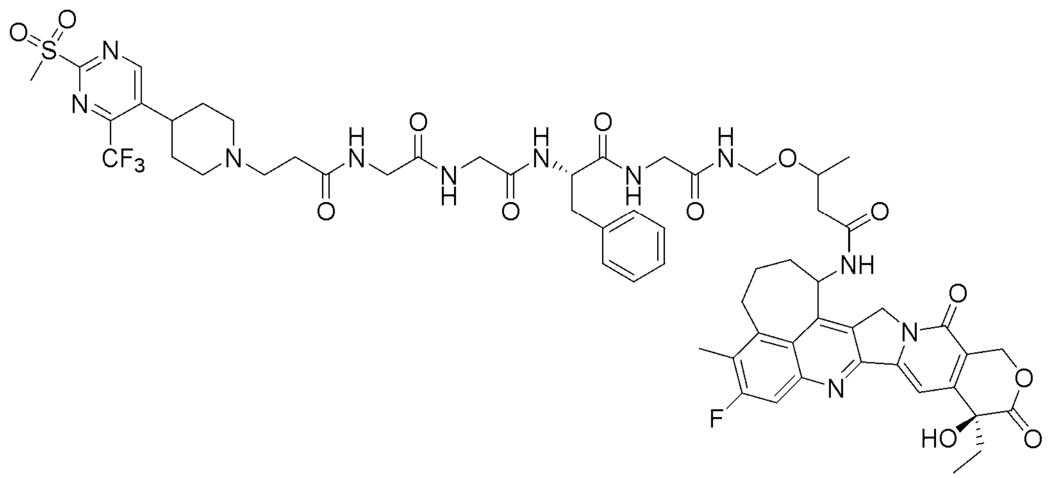

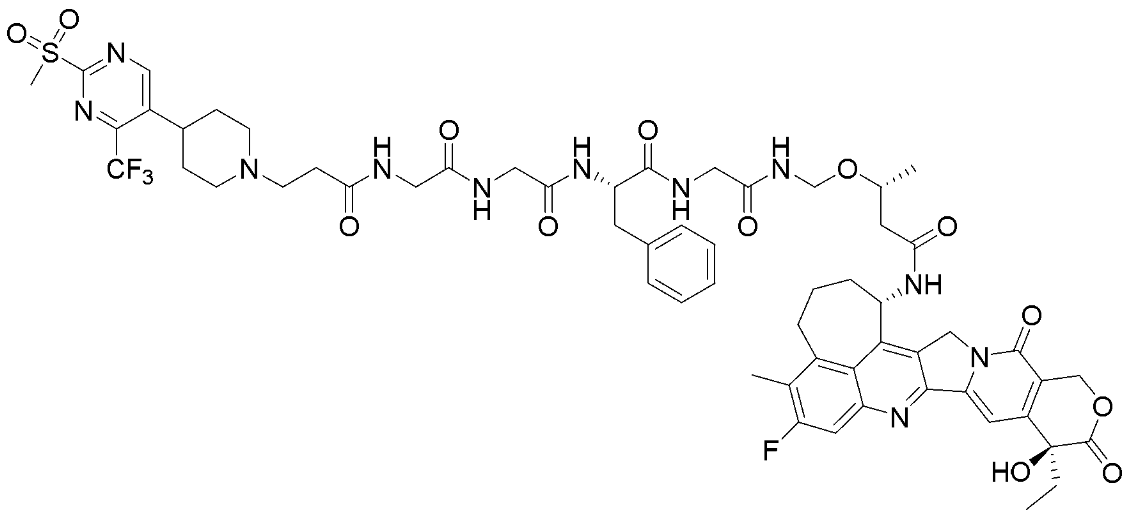

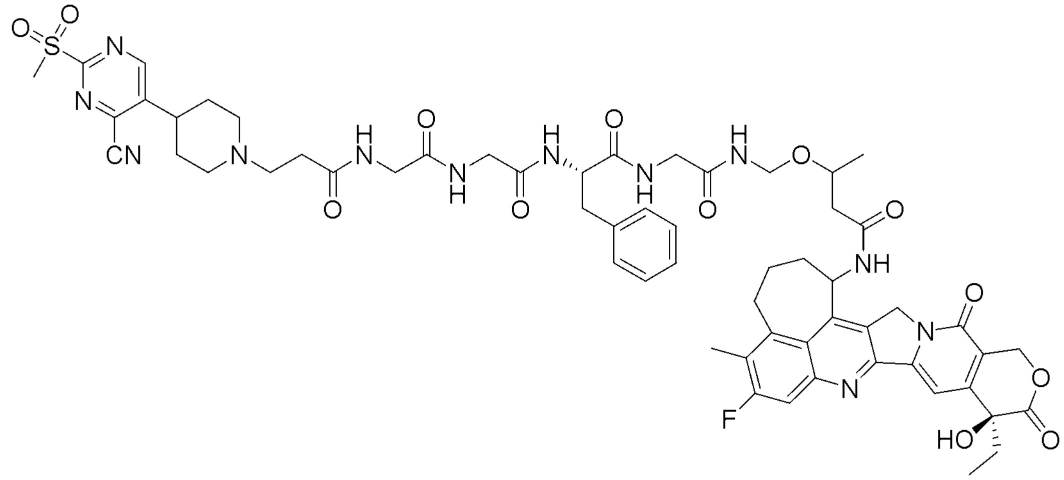

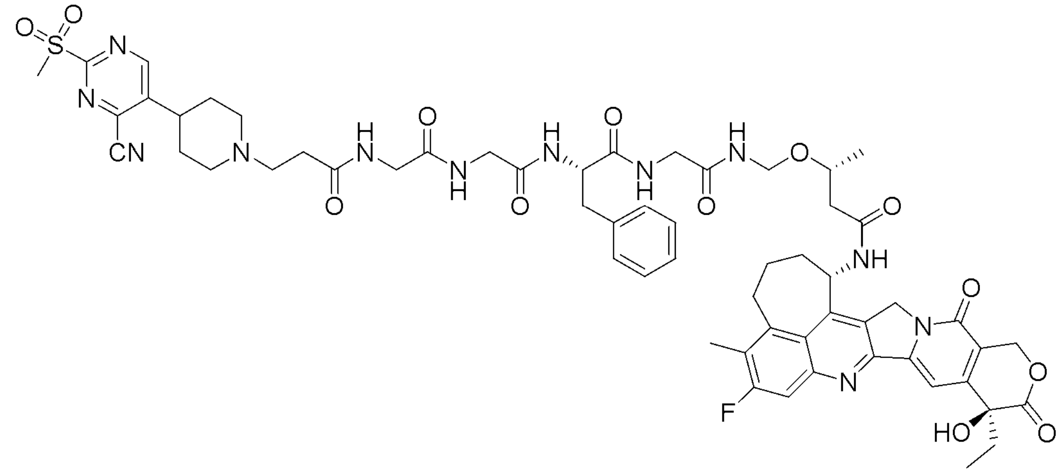

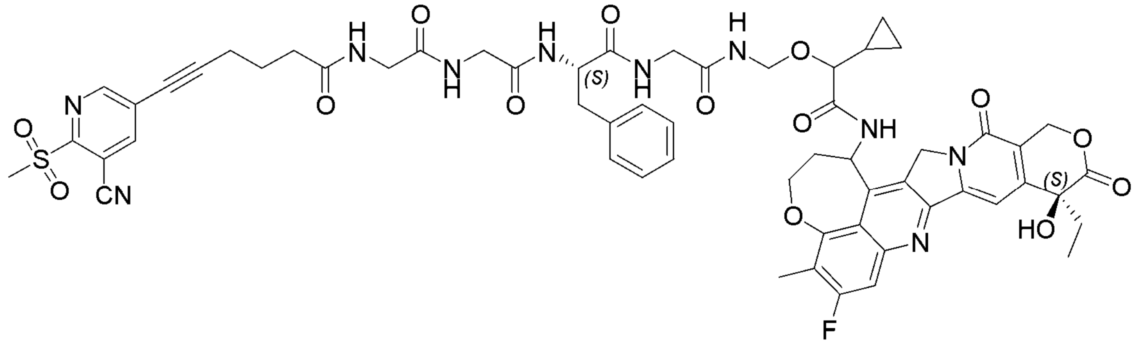

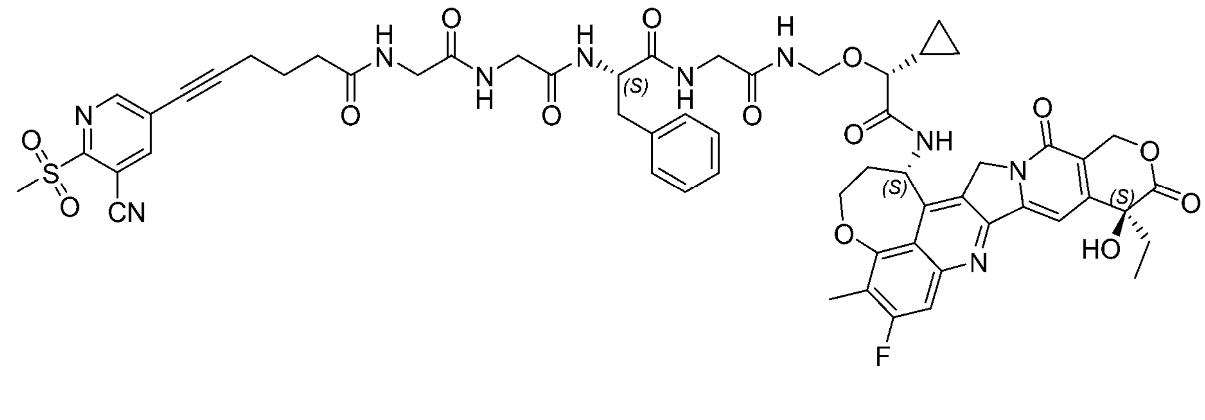

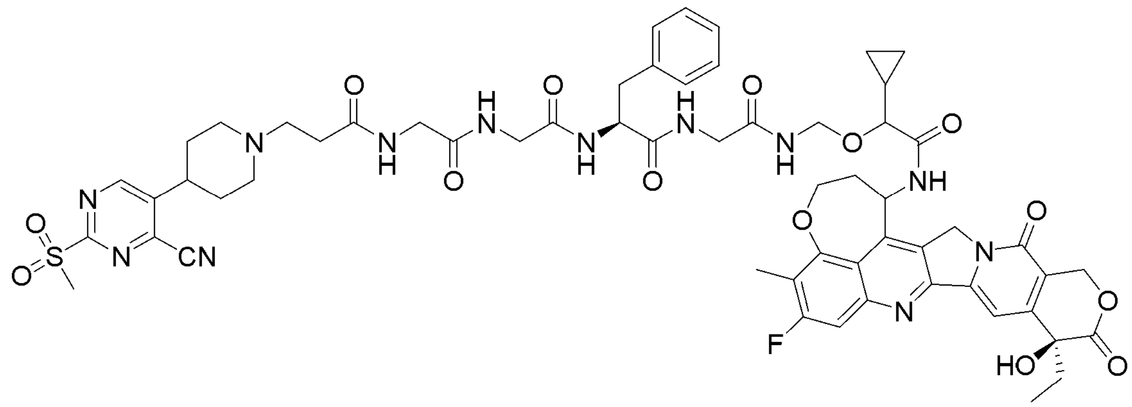

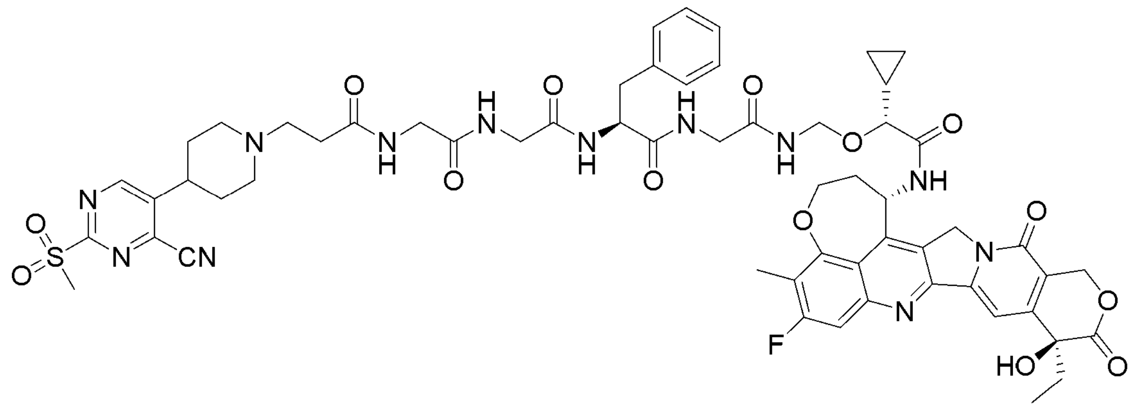

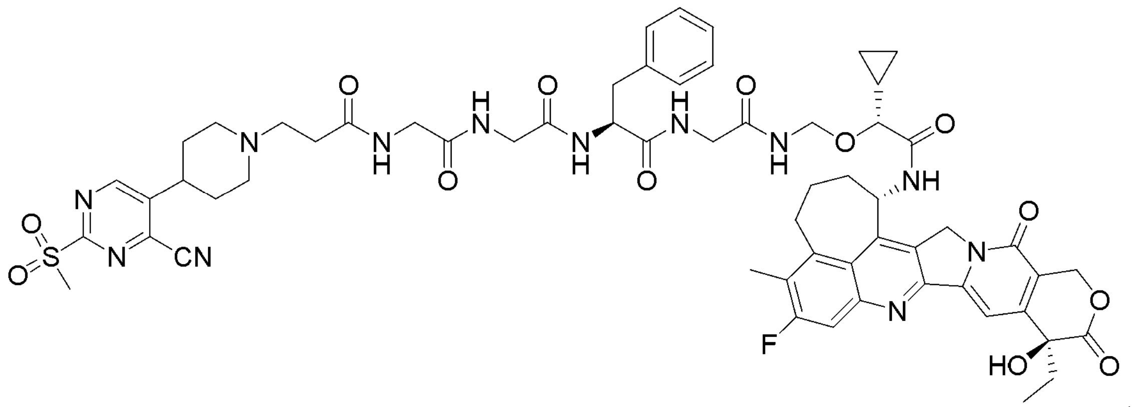

.

The present invention further provides an antibody-drug conjugate represented by formula VI,

Ab-[L-D]$_\beta$ (formula VI)

wherein Ab is an antibody or an antigen-binding fragment thereof, D is as defined in the text of the present application, L is a linker linking the Ab to the D, and β is an integer or a decimal selected from 1-10.

According to some embodiments, Ab is an antibody or an antigen-binding fragment, andthe antigen-binding fragment is selected from Fab, Fab', (Fab')$_2$, Fd, Fv, disulfide-linked Fv, scFv, di-scFv, (scFv)$_2$, a diabody, and a single domain antibody (sdAb); and/or the antibody is a murine antibody, a humanized antibody, a chimeric antibody, a bispecific antibody, or a multispecific antibody.

According to some embodiments, Ab is an anti-HER2 antibody or an antigen-binding fragment thereof, for example, Ab is trastuzumab or an antigen-binding fragment thereof.

According to some embodiments, β is selected from an integer or decimal between 4 and 9 (e.g., 7, 7.71, 7.84, 7.92, 7.94, 7.97, 7.98, 7.99, 8, 8.02, 8.06, or 8.14).

According to some embodiments, L is selected from M'-$L_1$- $L_2$, wherein M' is a linker site linking to an antibody or

an antigen-binding fragment thereof, formed by conjugating M defined in text of the present application with an antibody or an antigen-binding fragment thereof, and $L_1$ and $L_2$ are as defined in the text of the present application.

According to some embodiments, M' is selected from

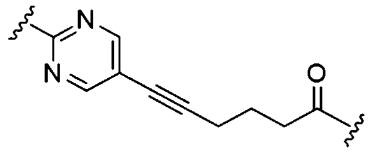,

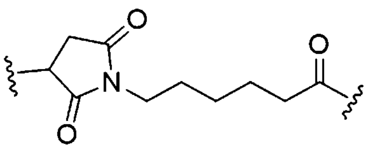, 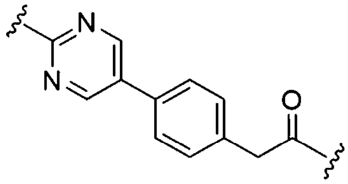, 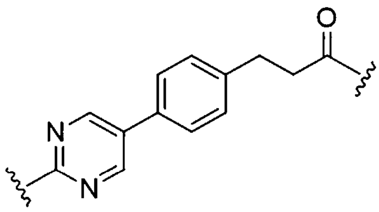,

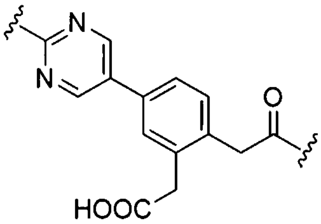, 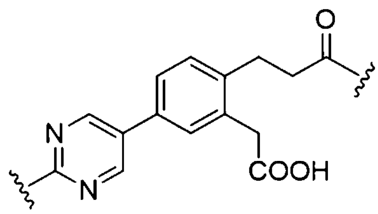, 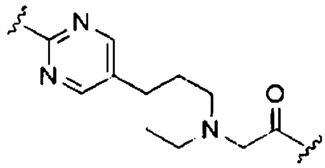,

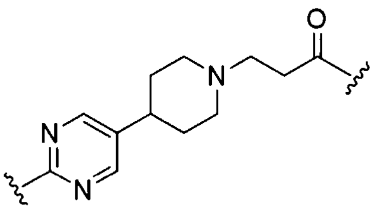, 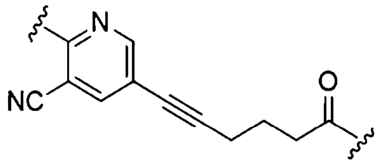, 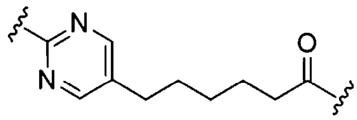,

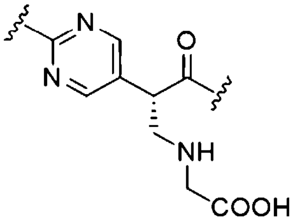, 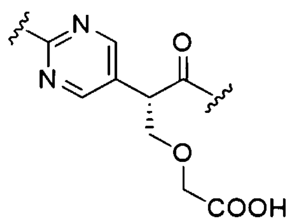, 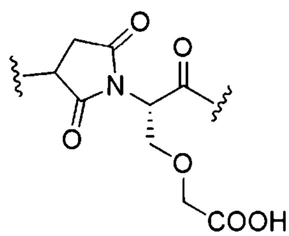,

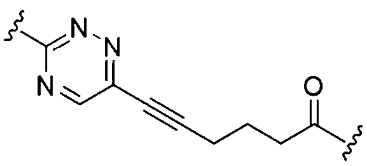, 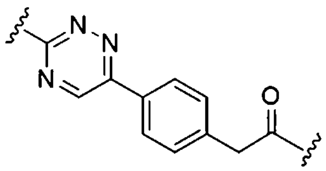, 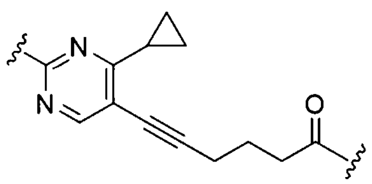,

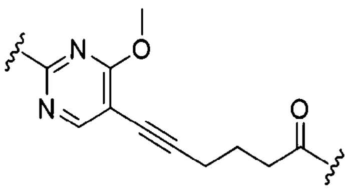 , 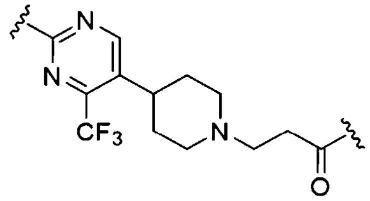 , 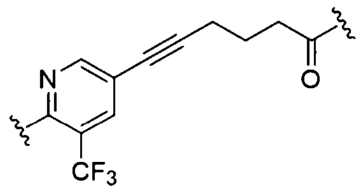 ,

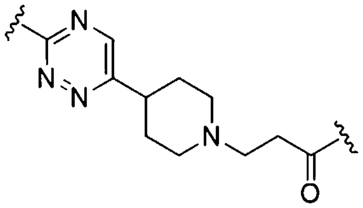 , 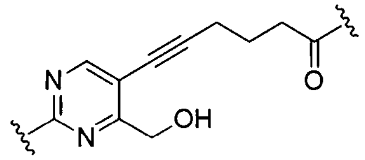 , 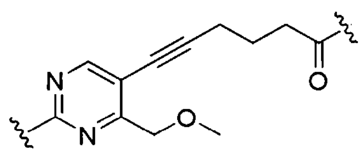 ,

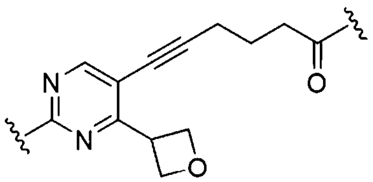 , 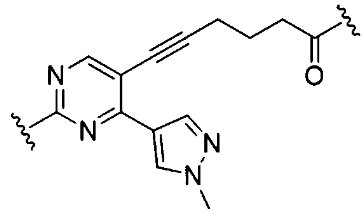 , 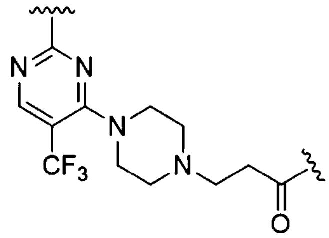 ,

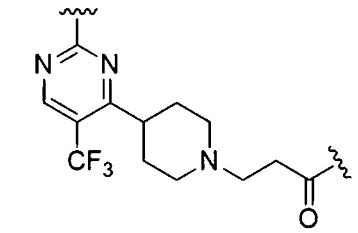 , 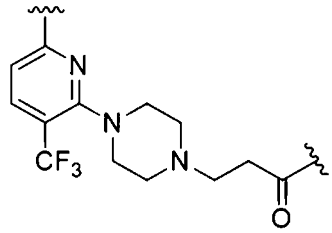 , 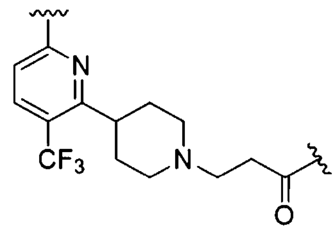 ,

or

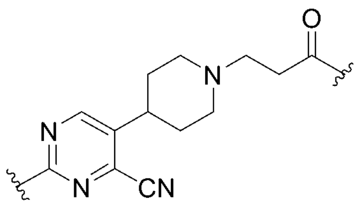 .

Preferably, the carbonyl linking position in M' is linked to $L_1$, and the linking position on the heterocyclic or heteroaromatic ring is linked to Ab.

According to some embodiments, L is selected from:

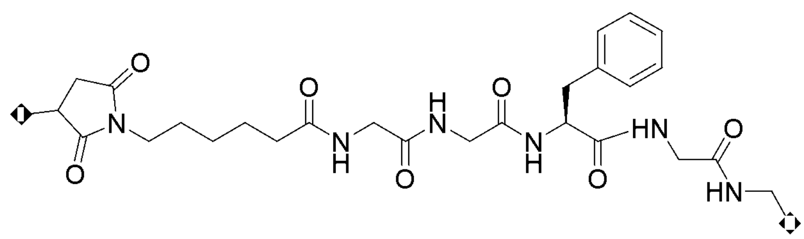

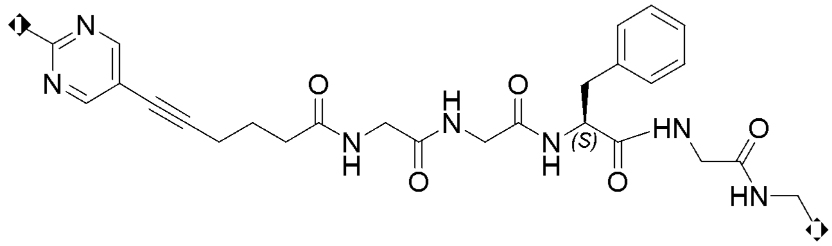

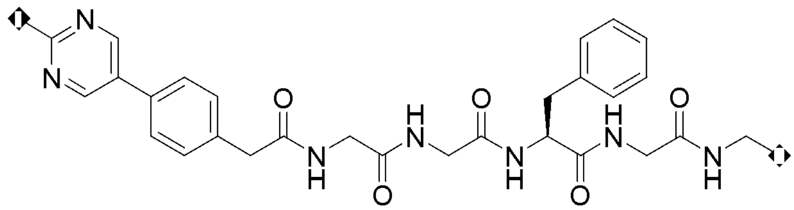

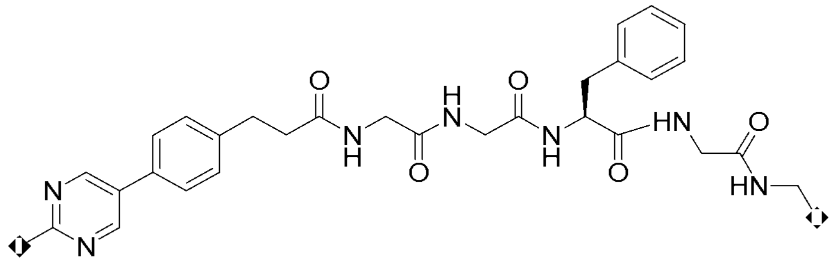

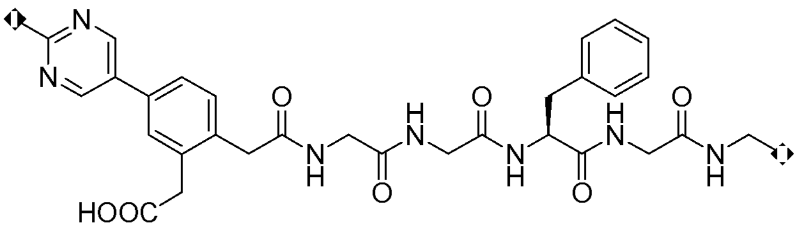

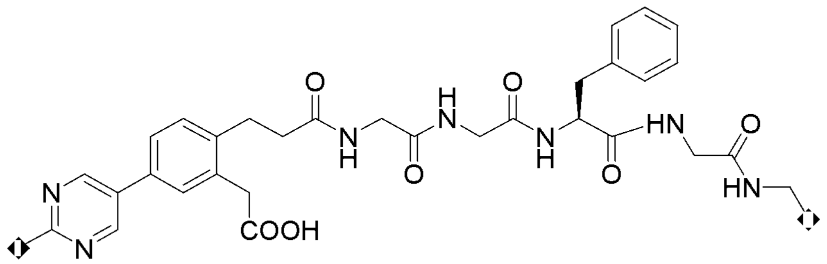

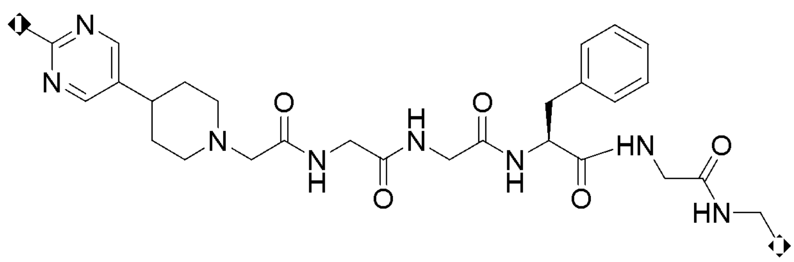

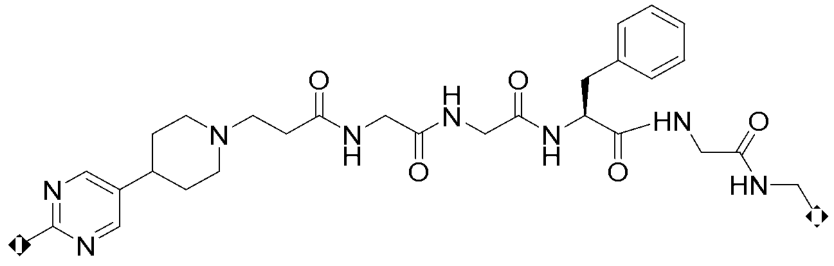

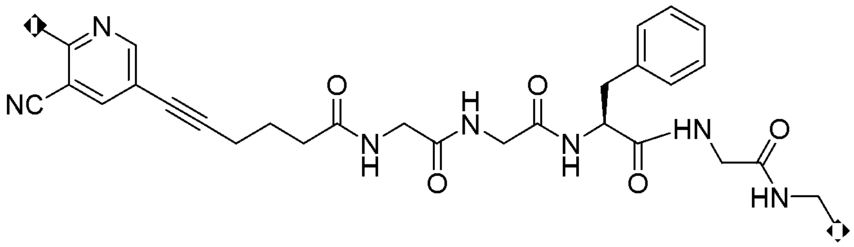

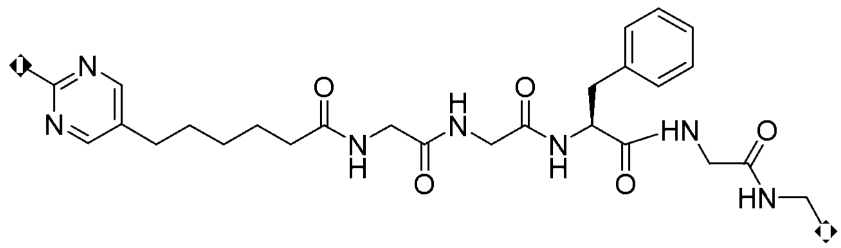

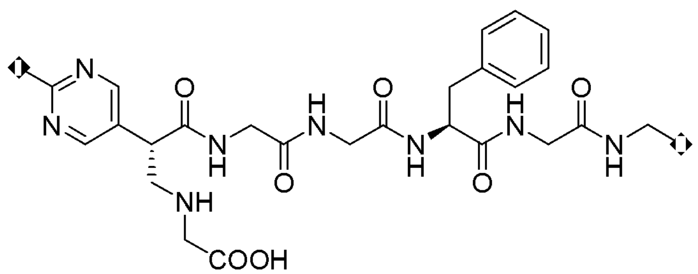

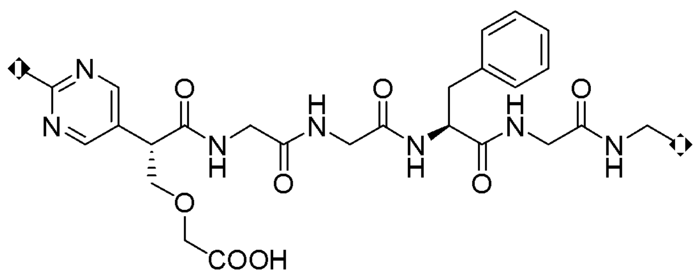

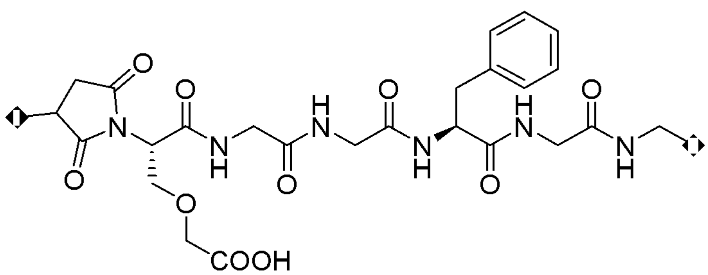

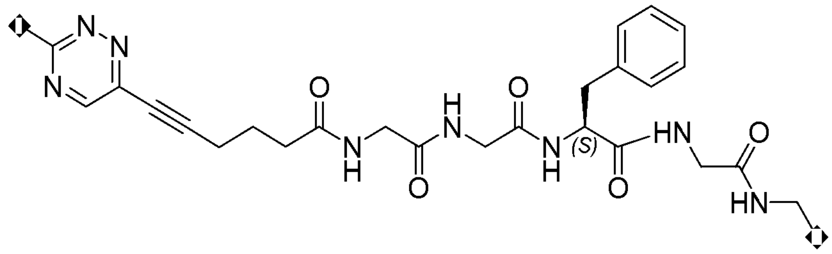

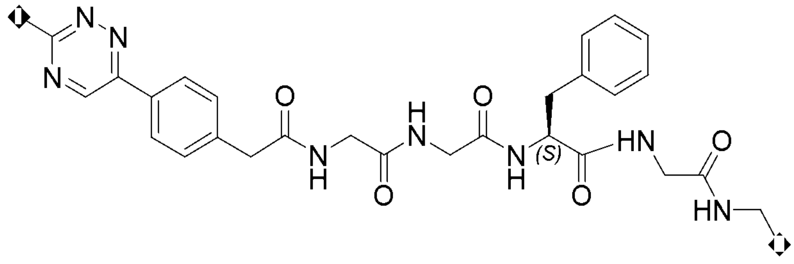

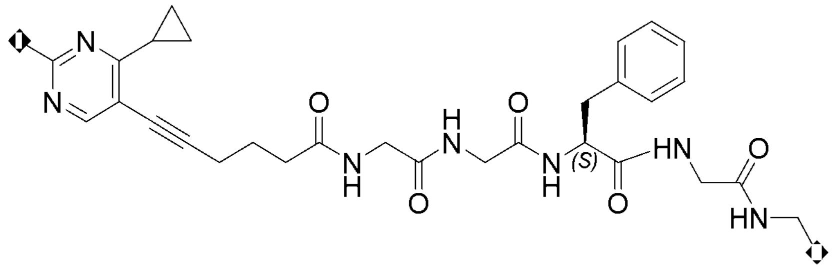

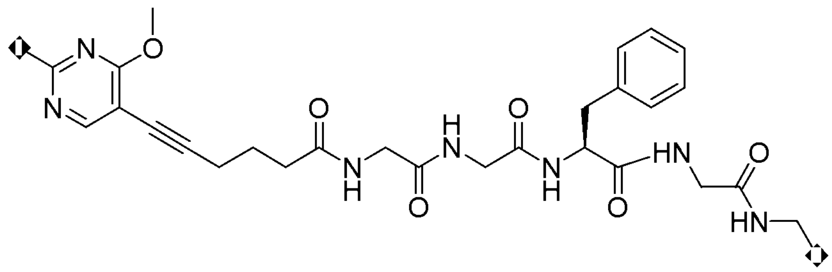

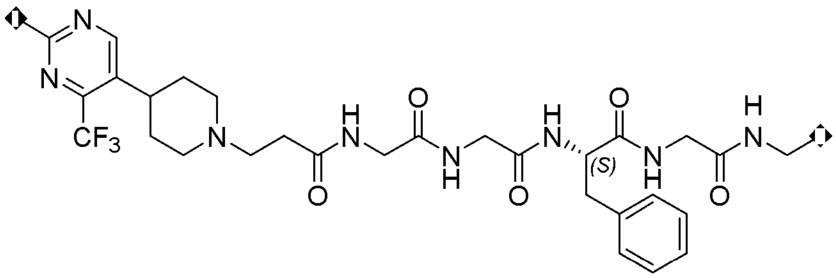

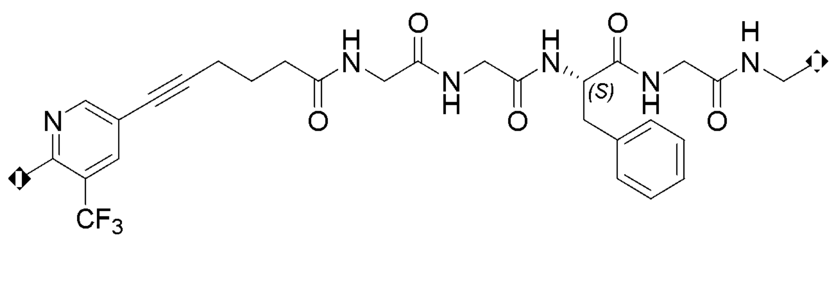

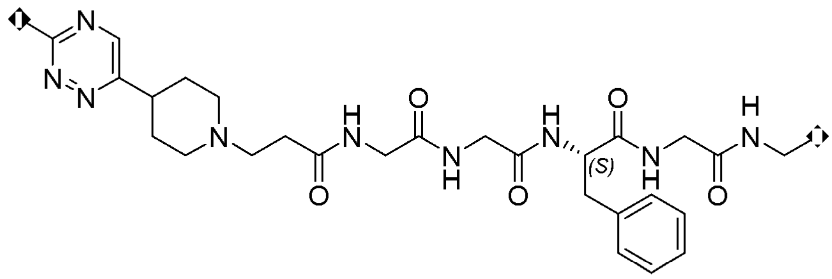

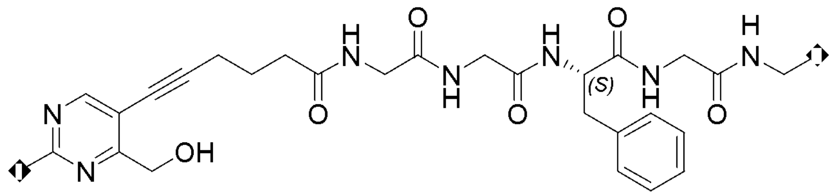

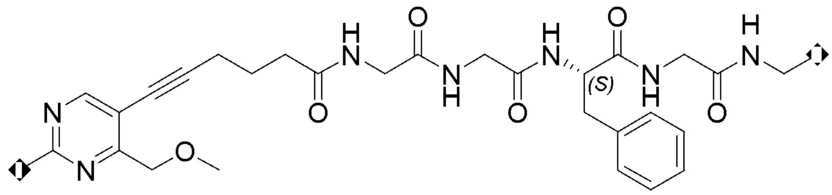

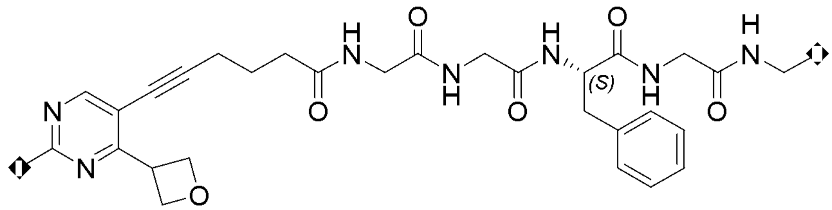

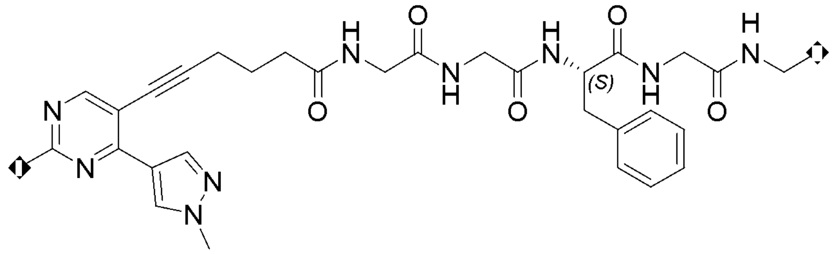

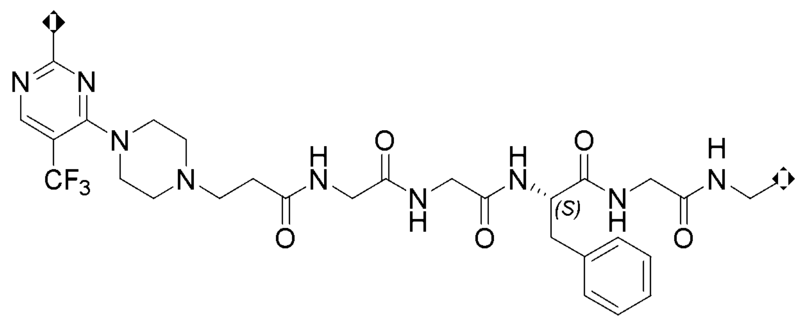

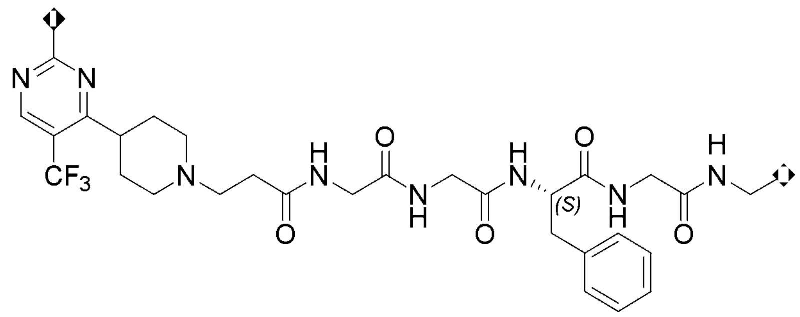

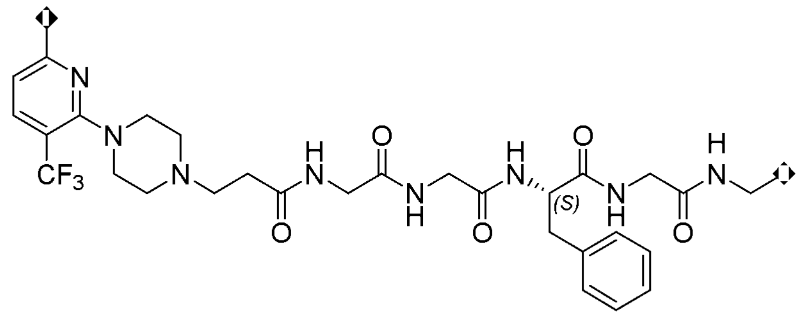

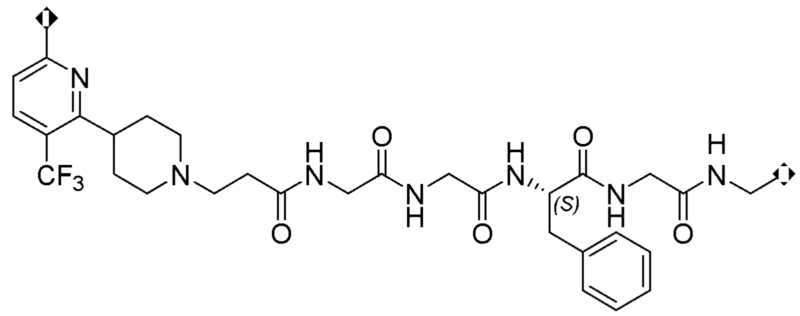

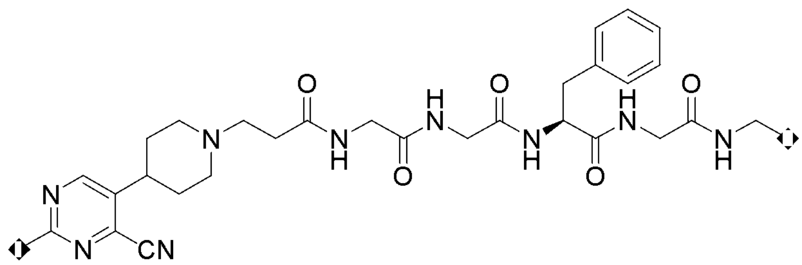

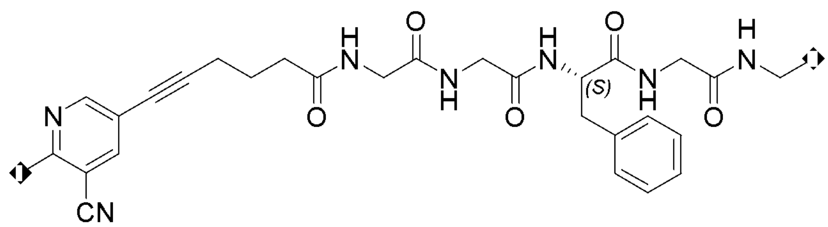

,

wherein position 1 is linked to Ab and position 2 is linked to D.

According to some embodiments, L-D is selected from:

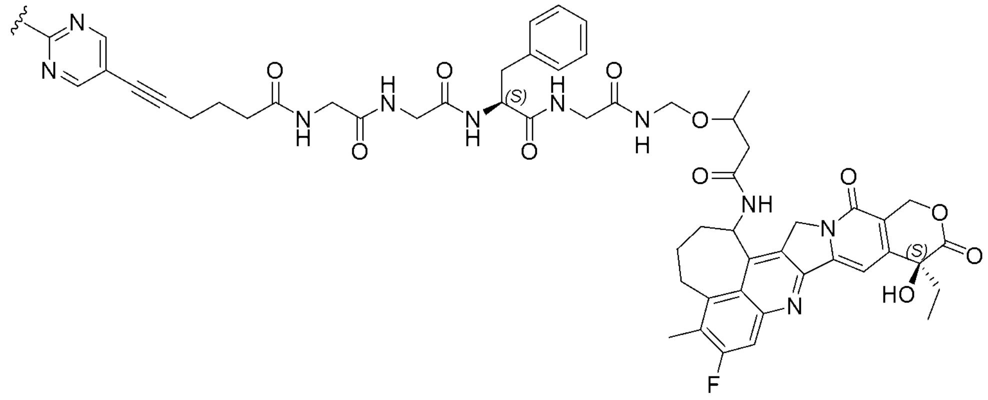

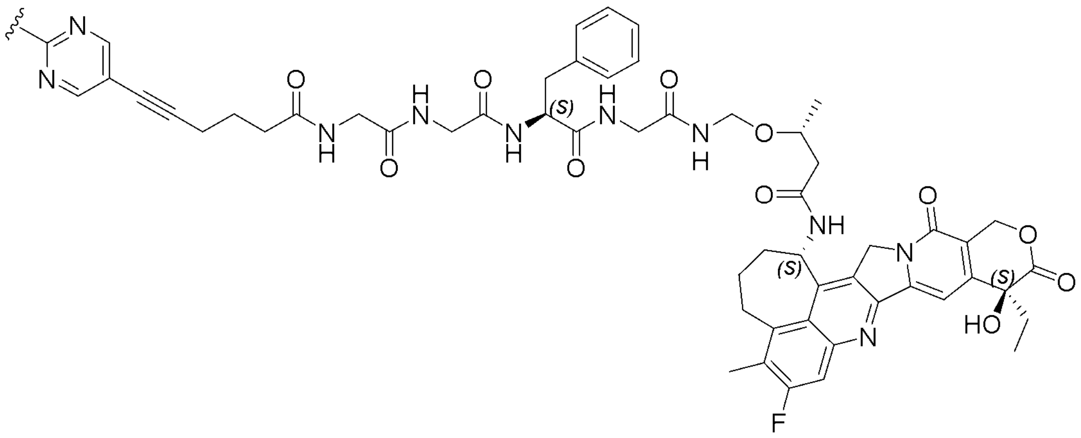

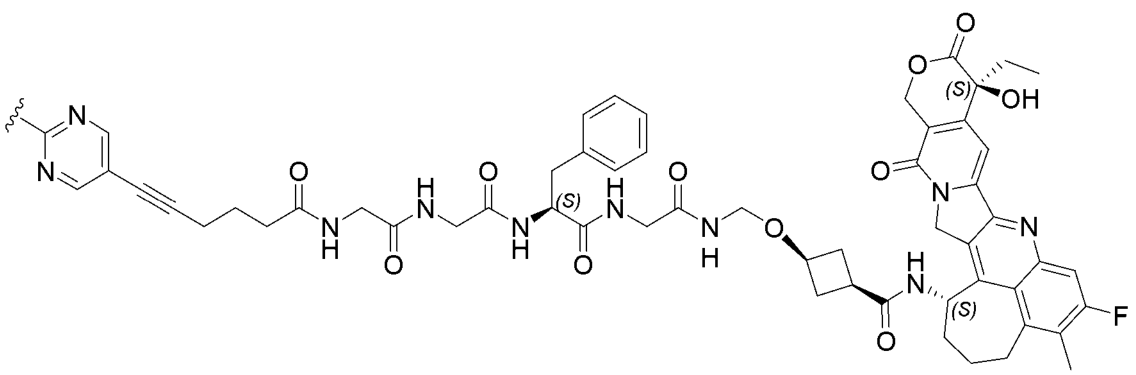

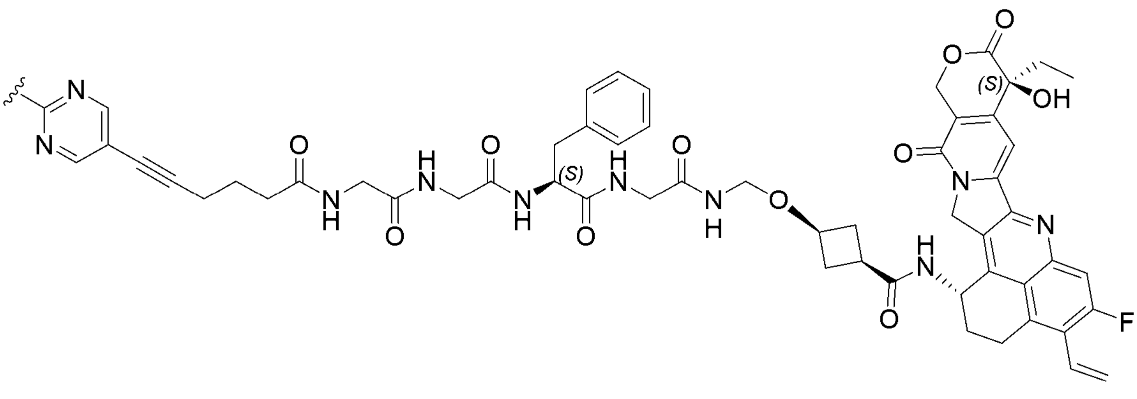

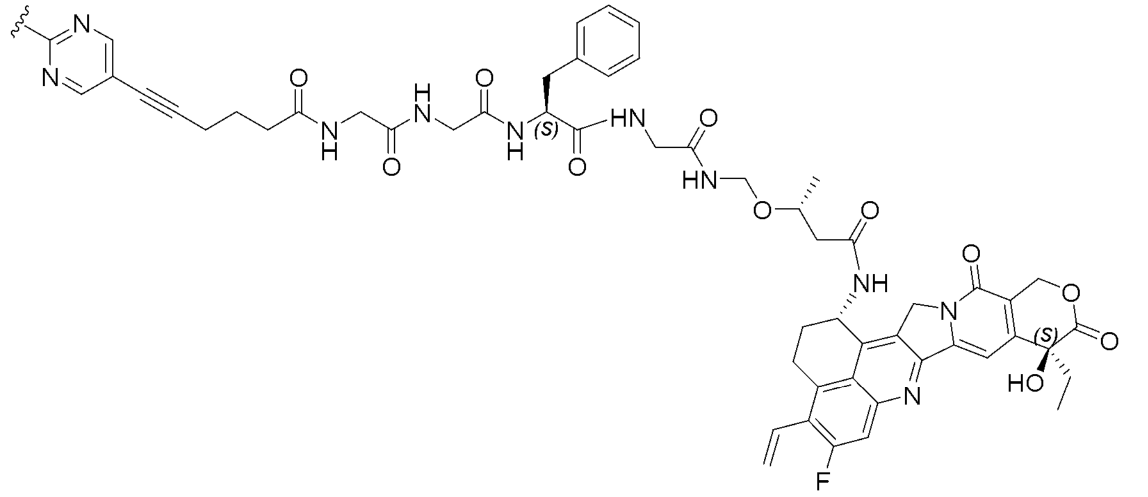

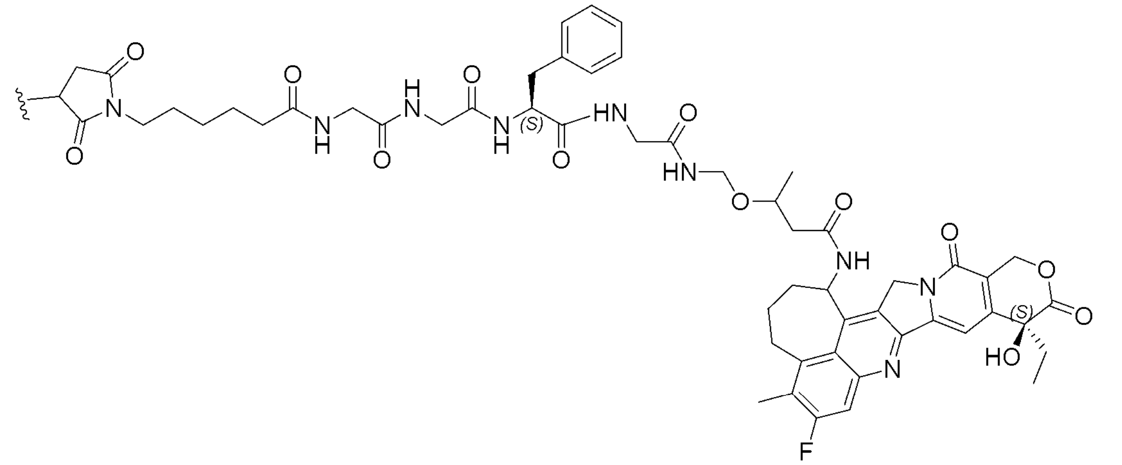

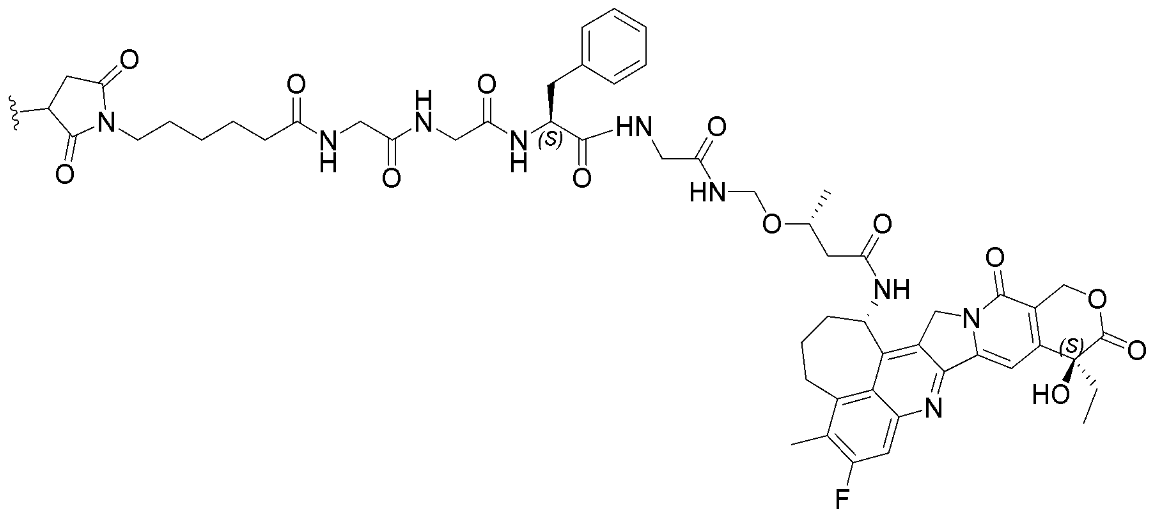

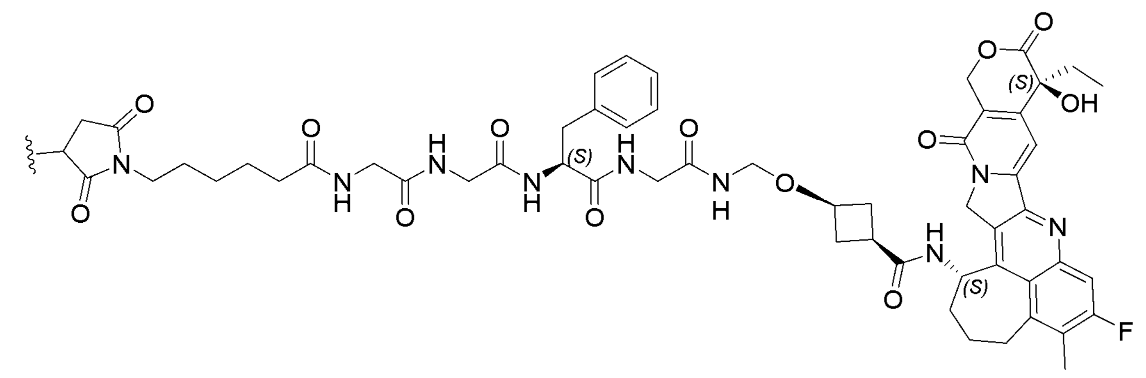

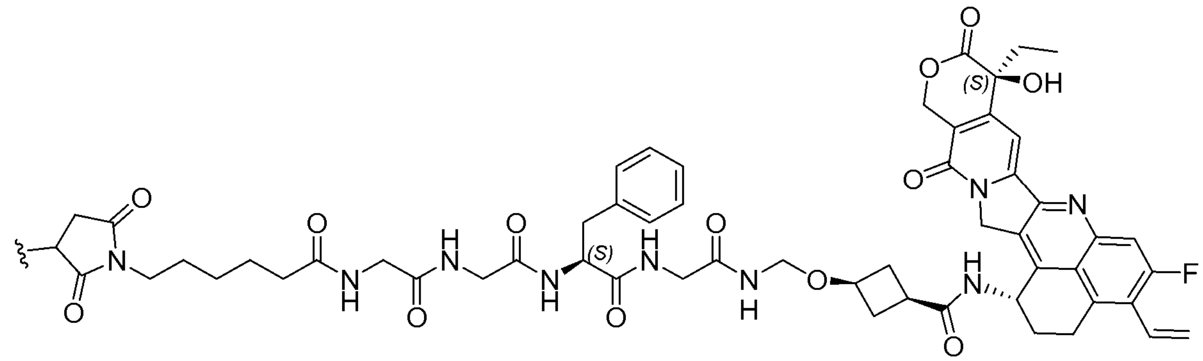

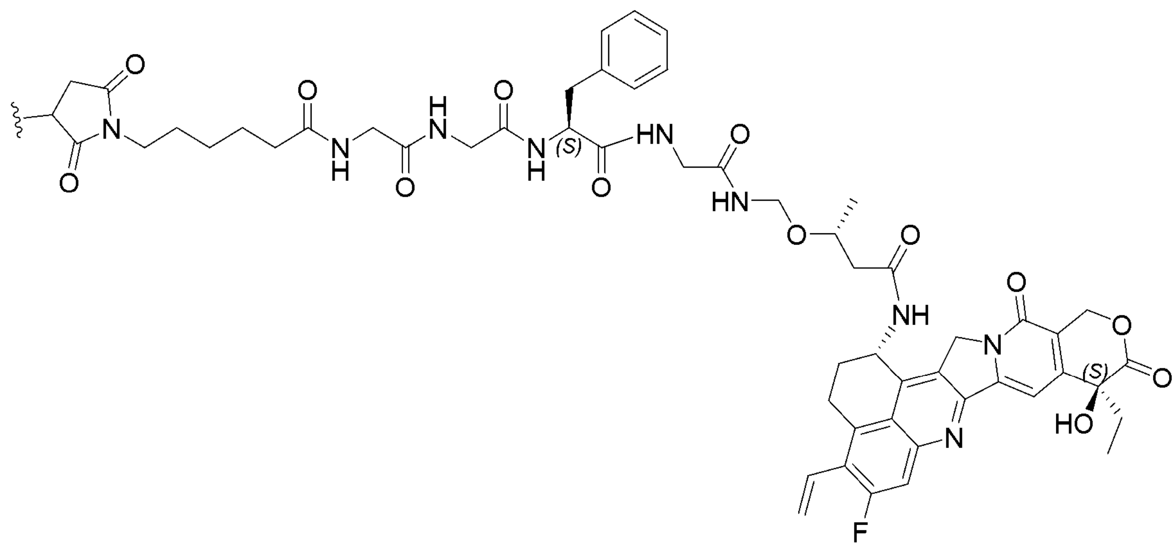

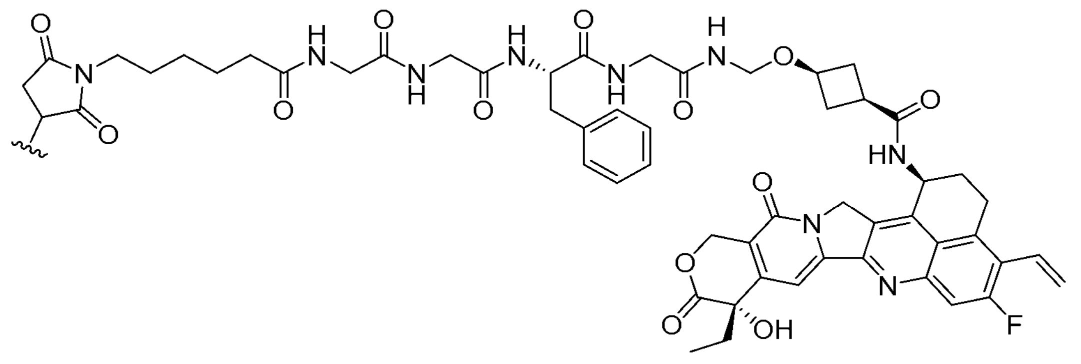

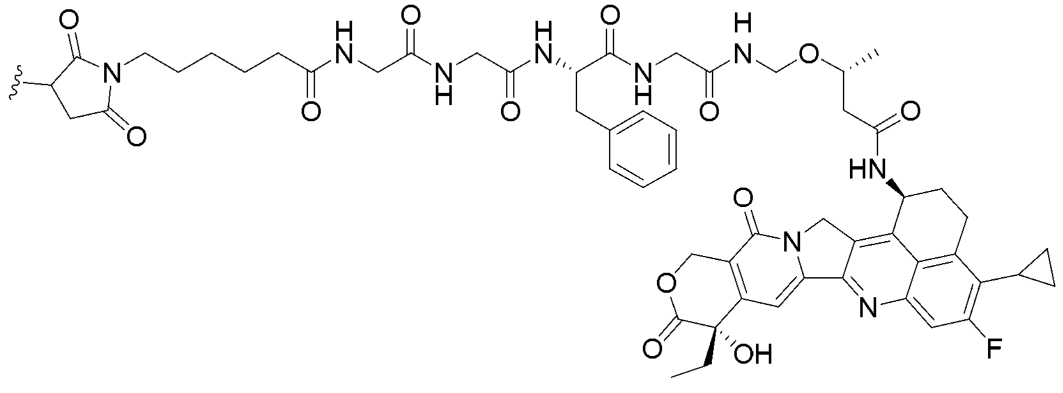

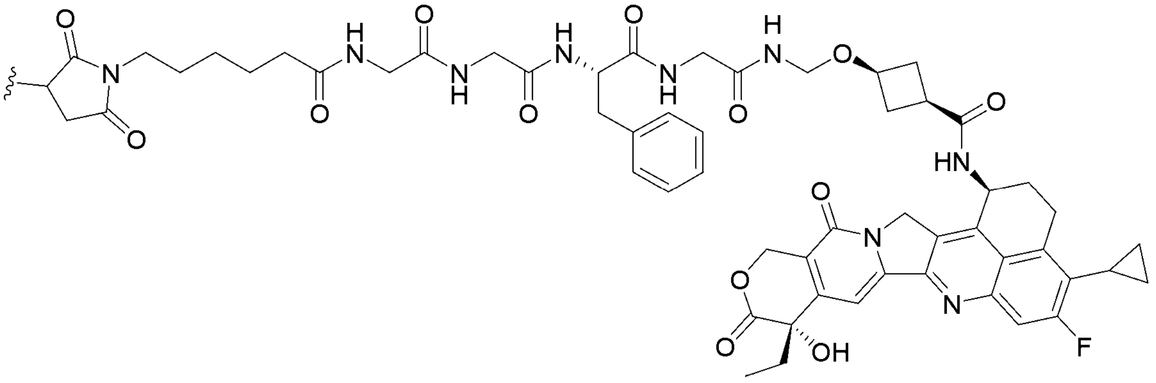

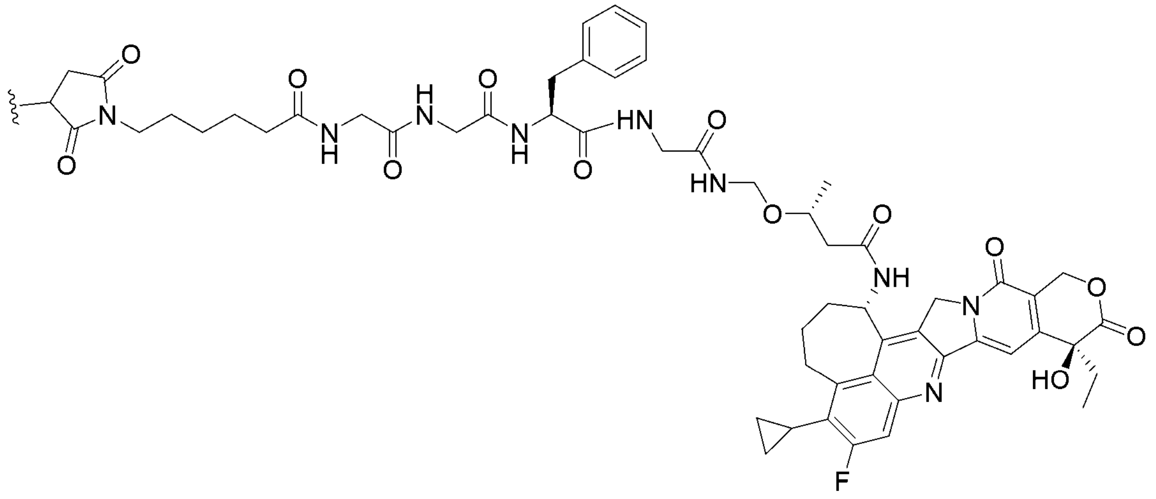

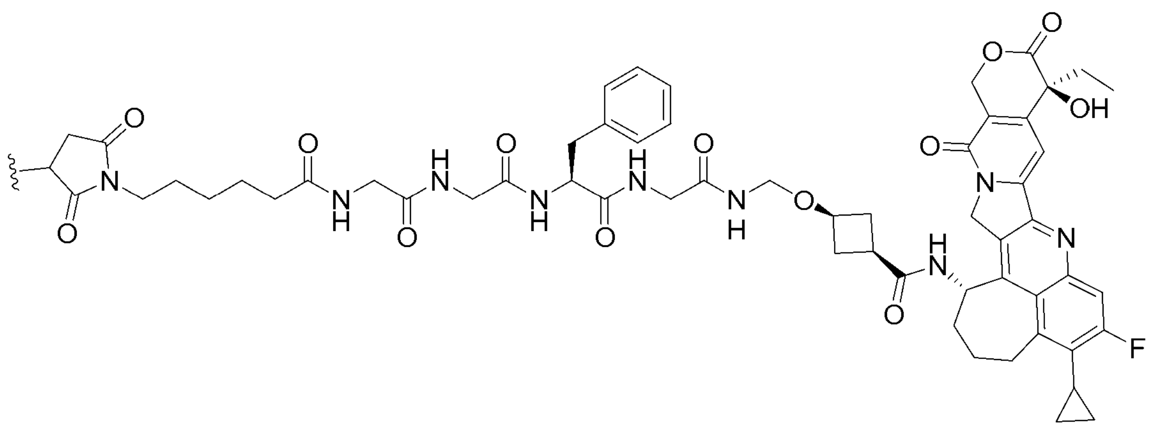

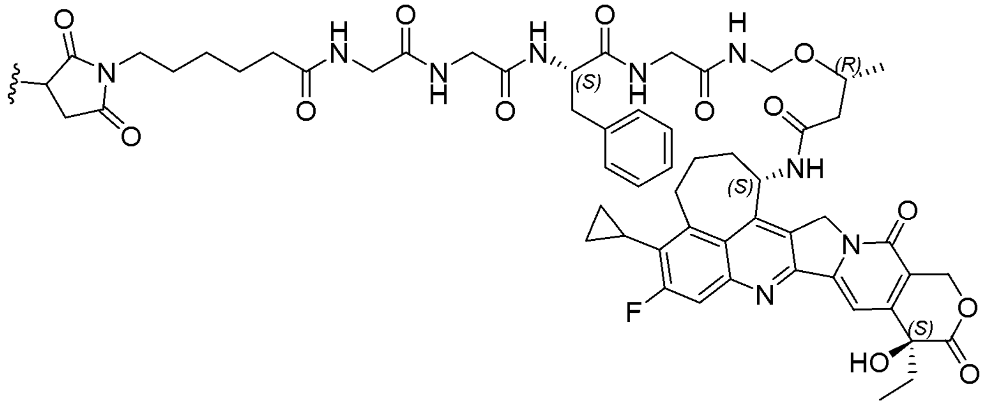

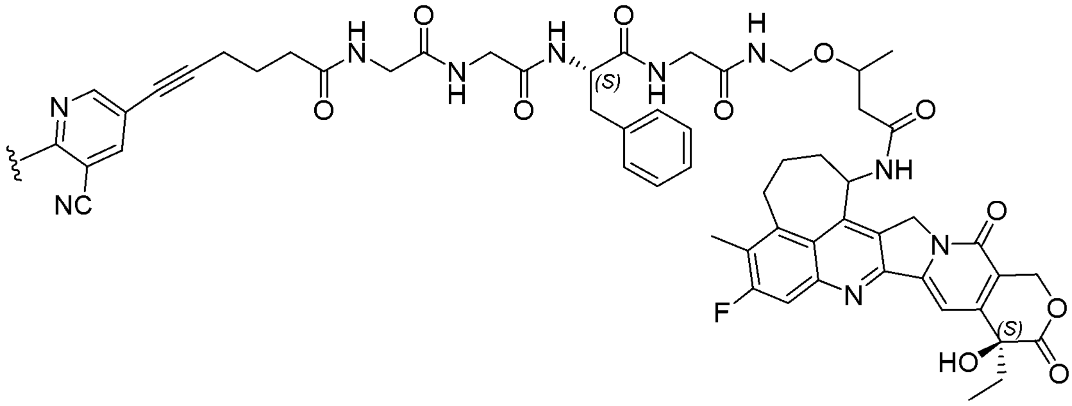

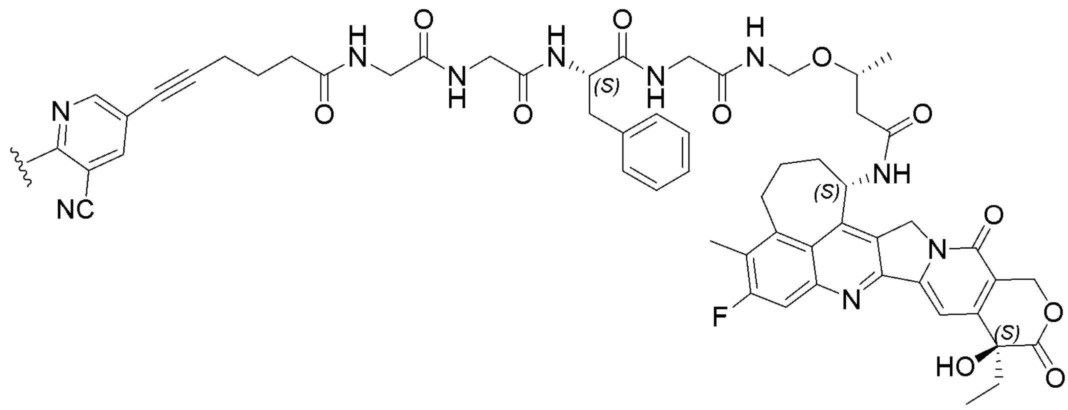

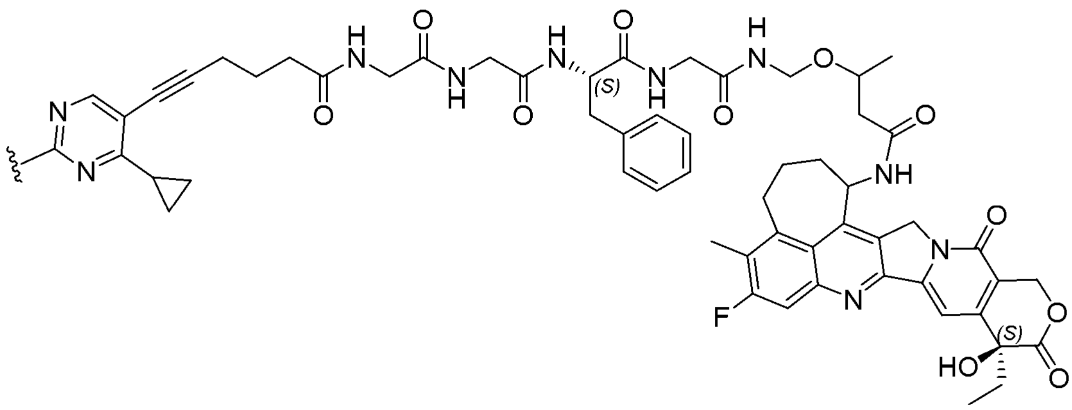

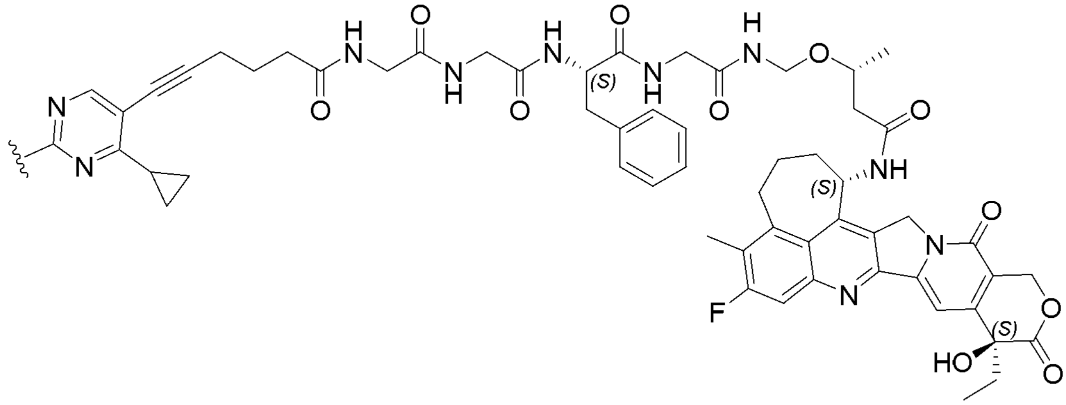

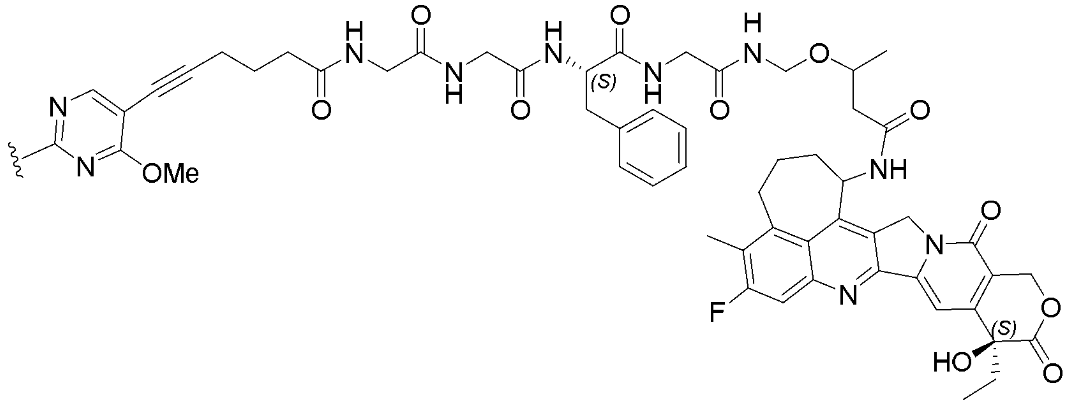

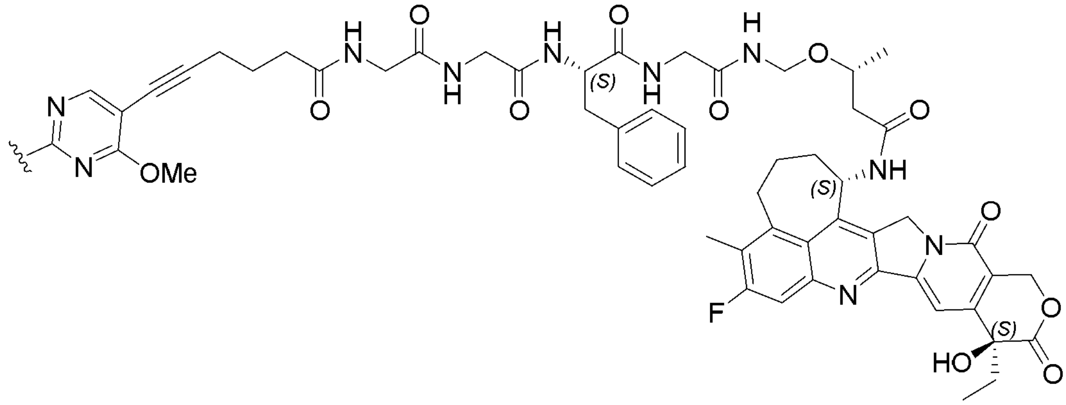

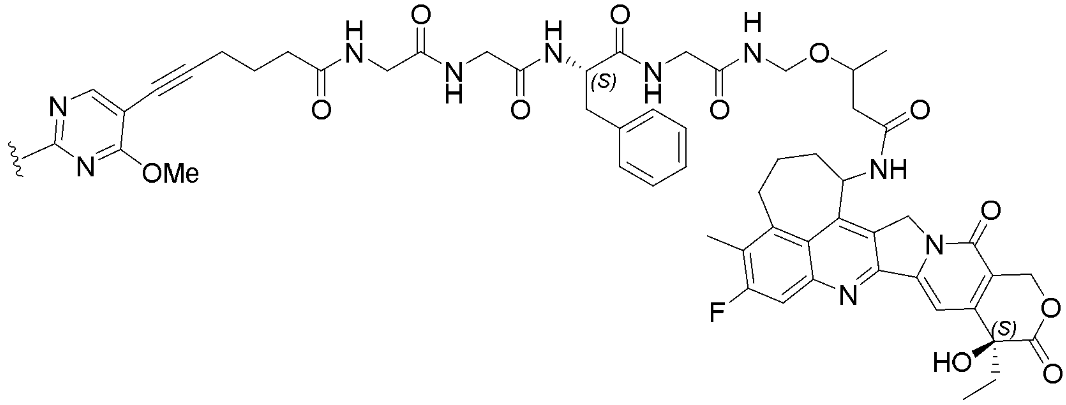

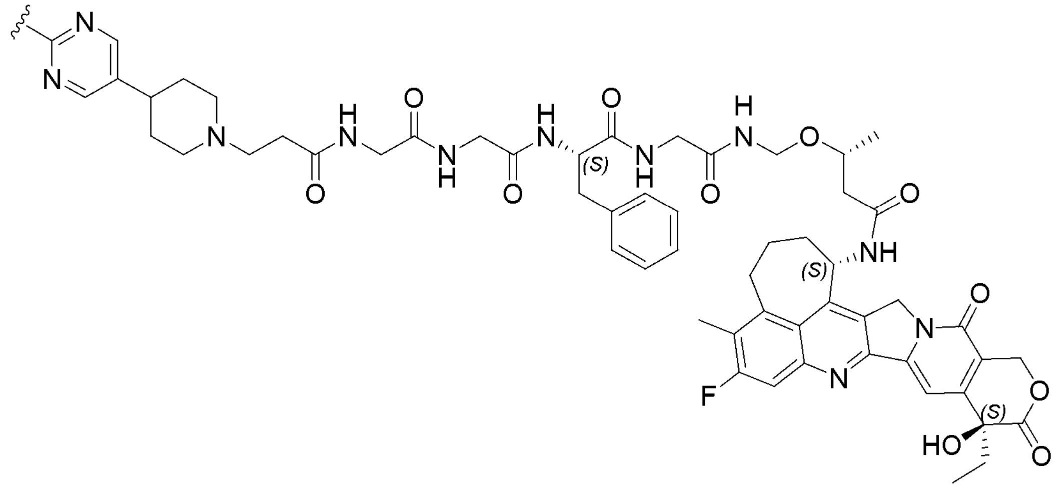

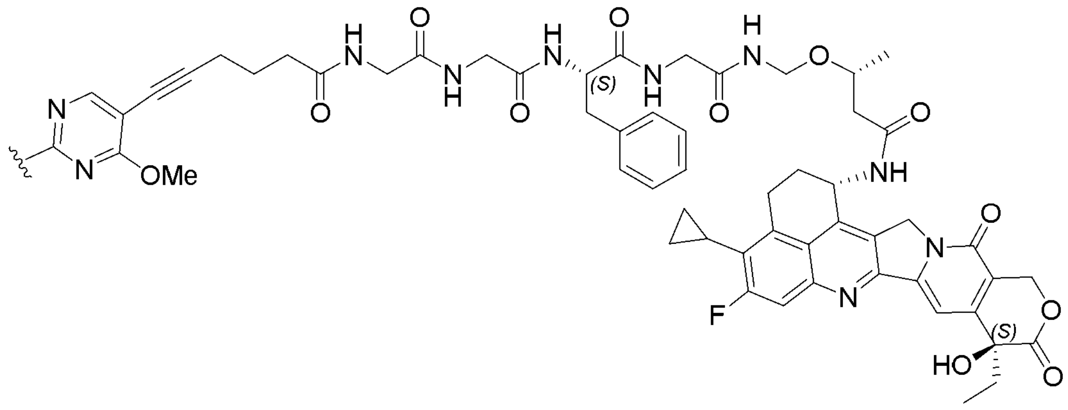

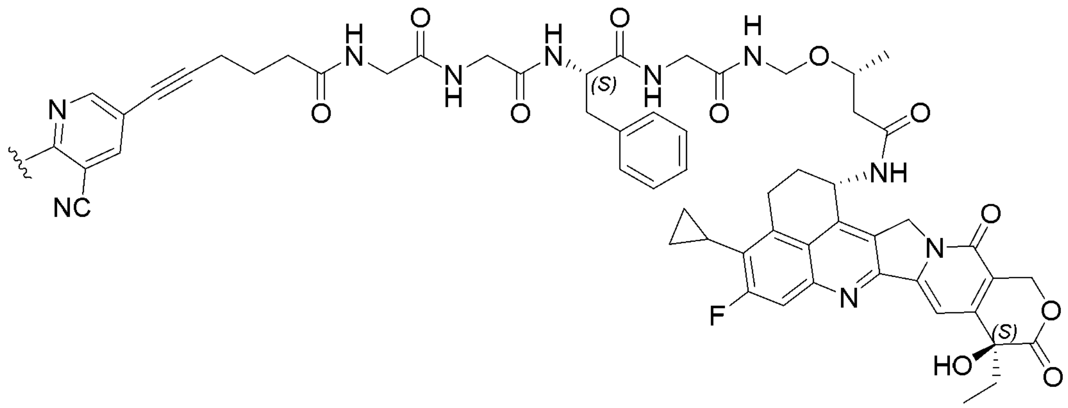

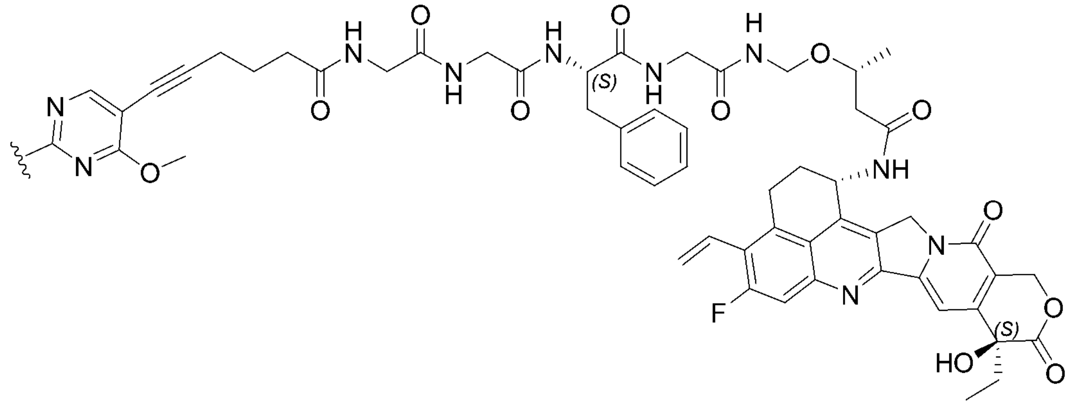

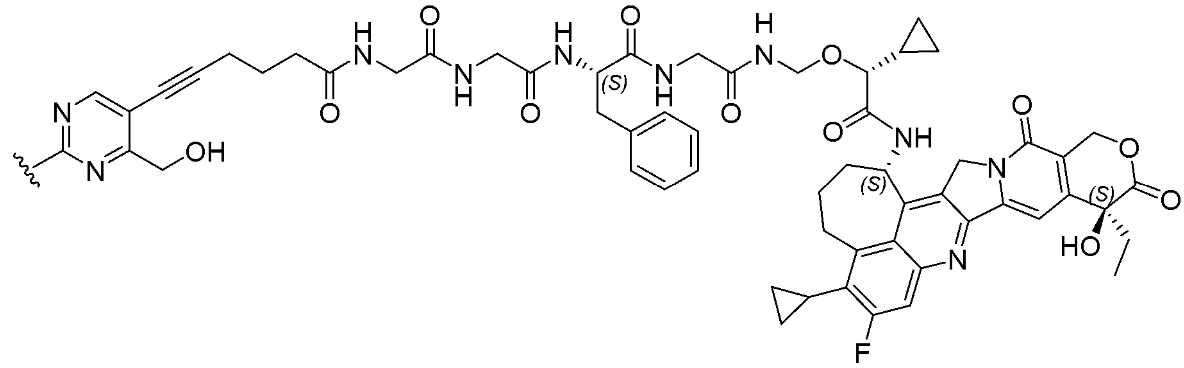

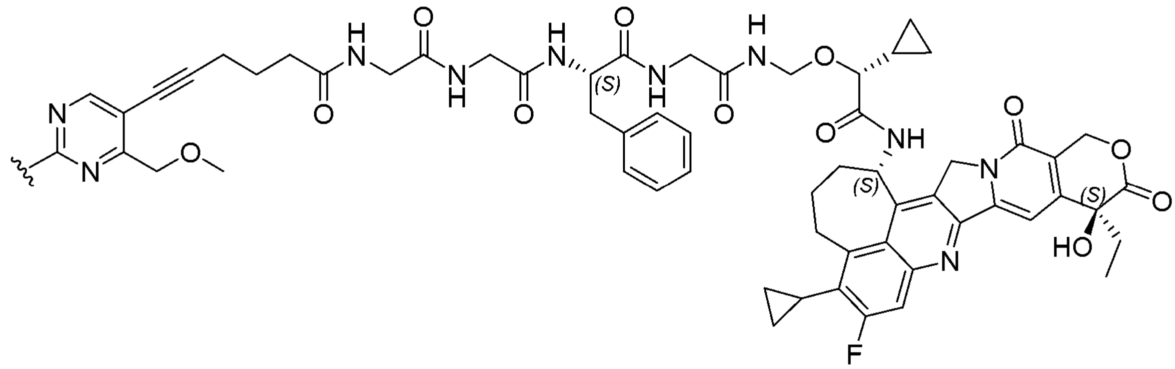

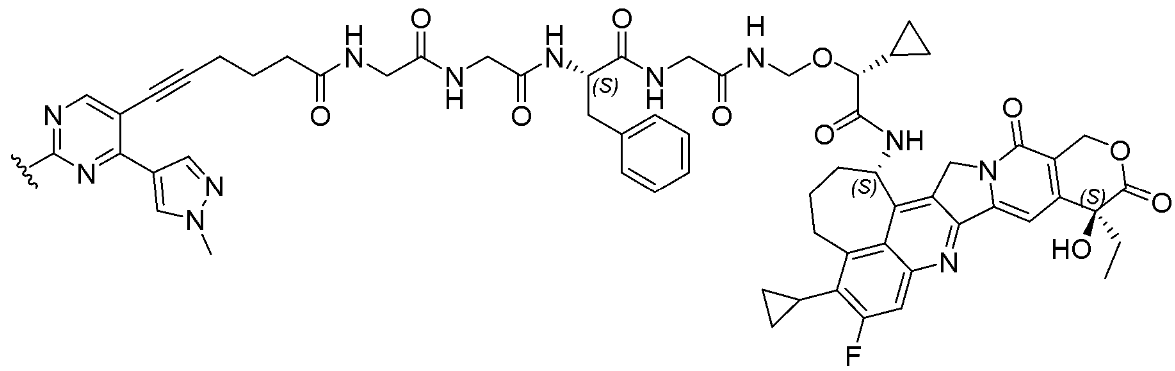

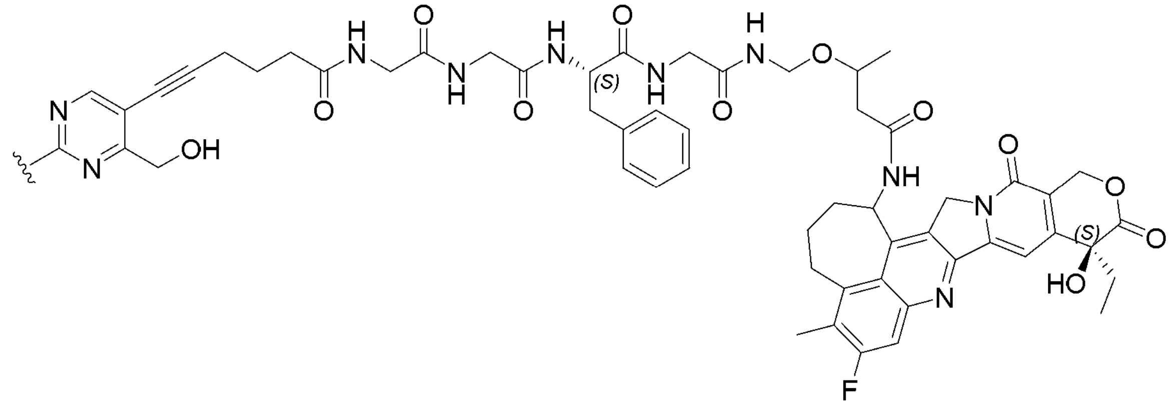

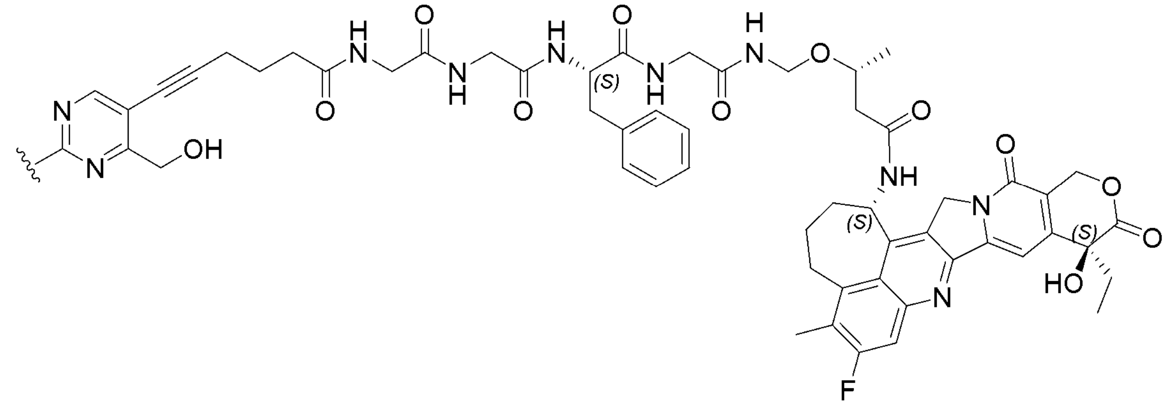

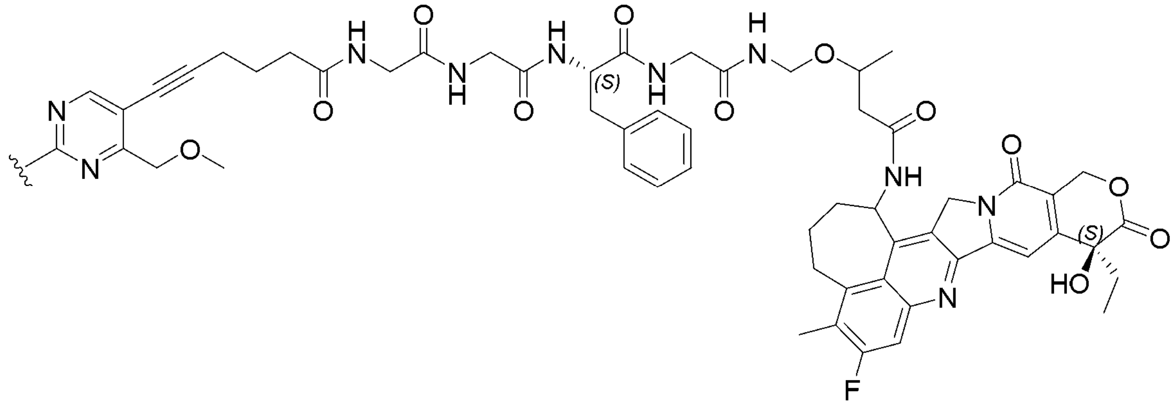

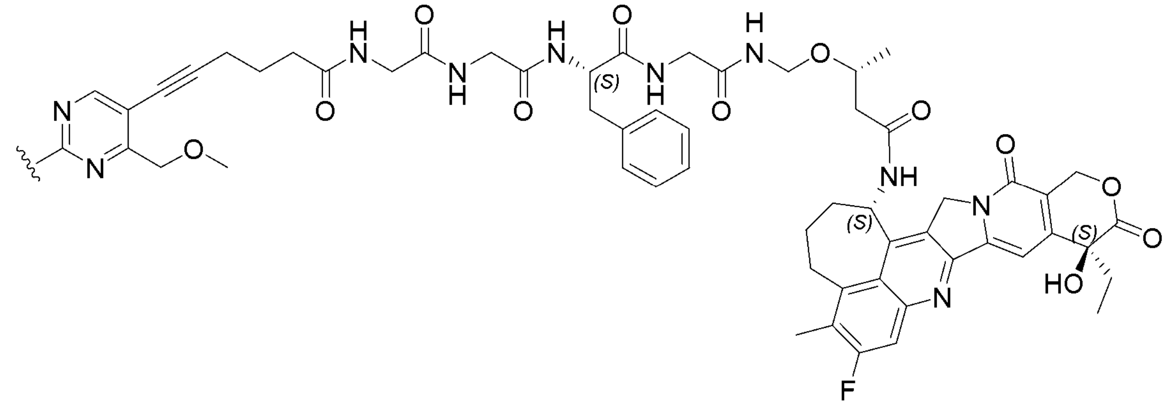

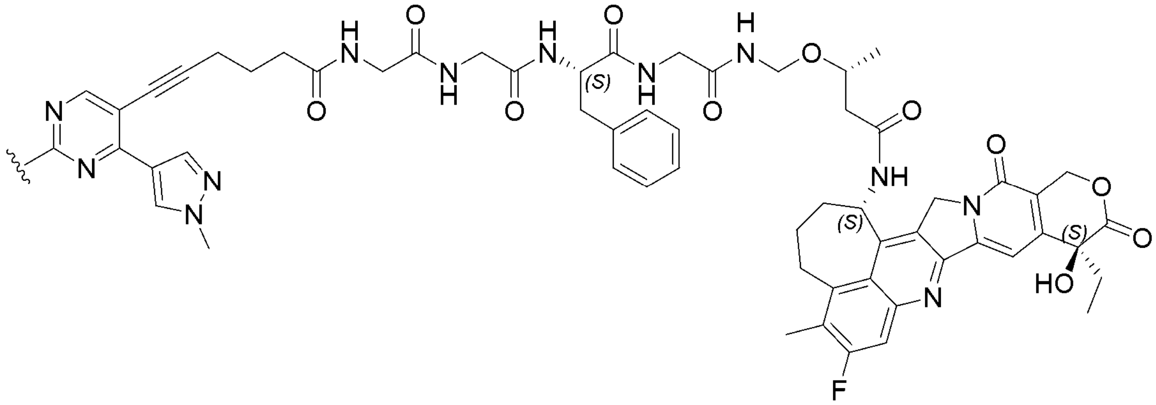

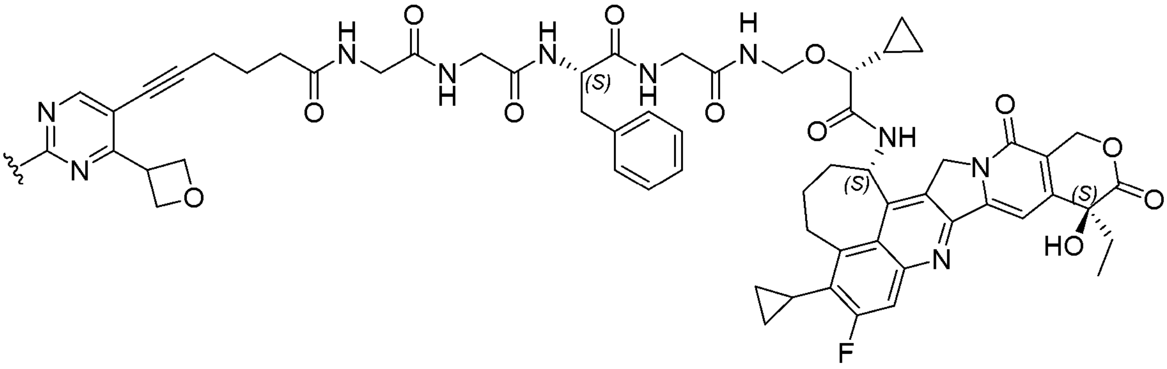

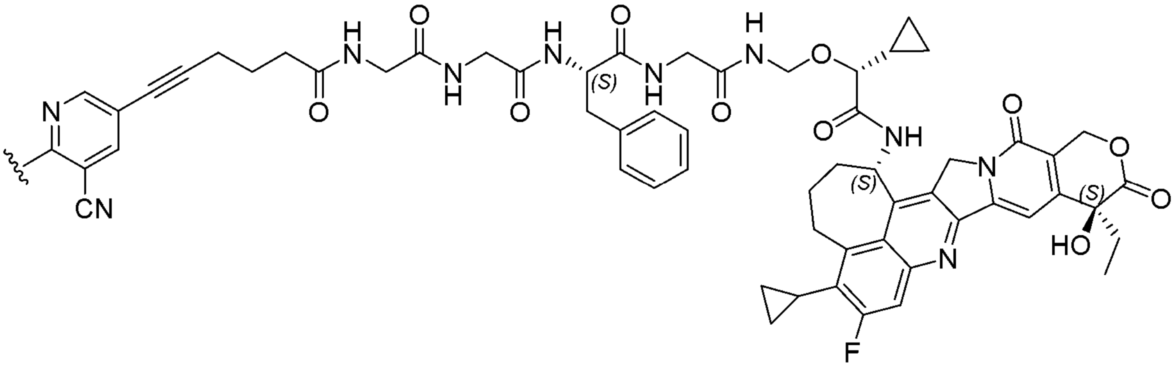

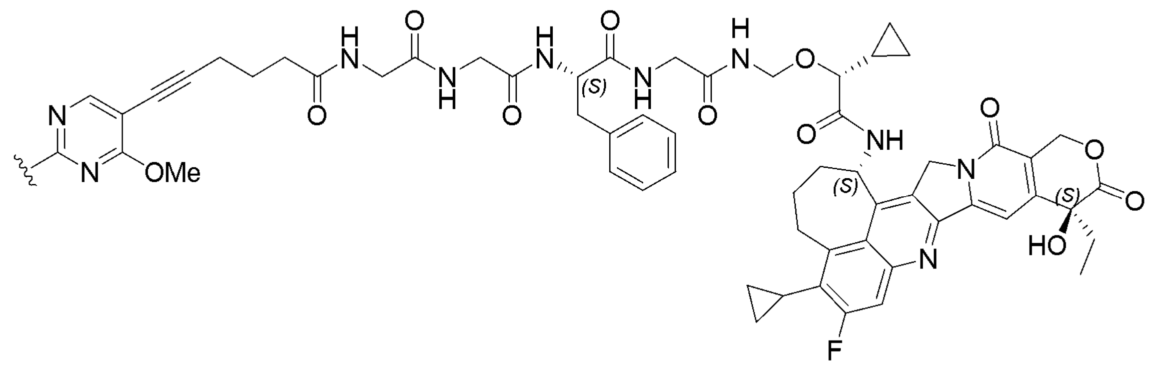

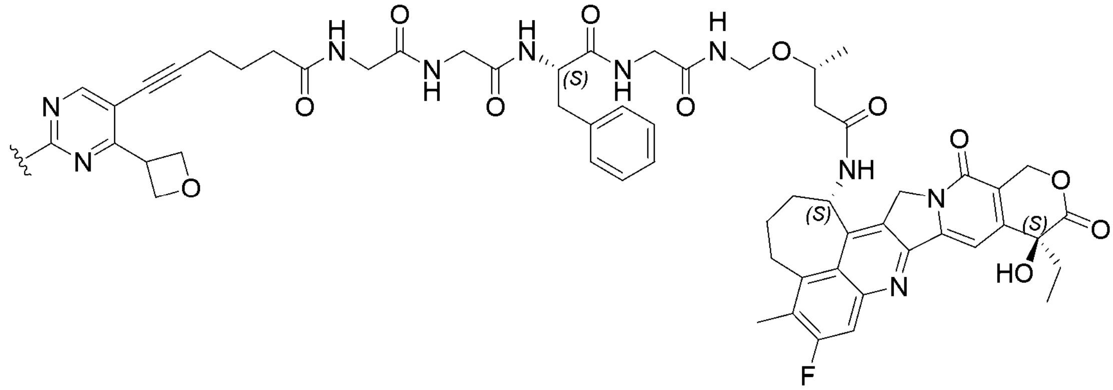

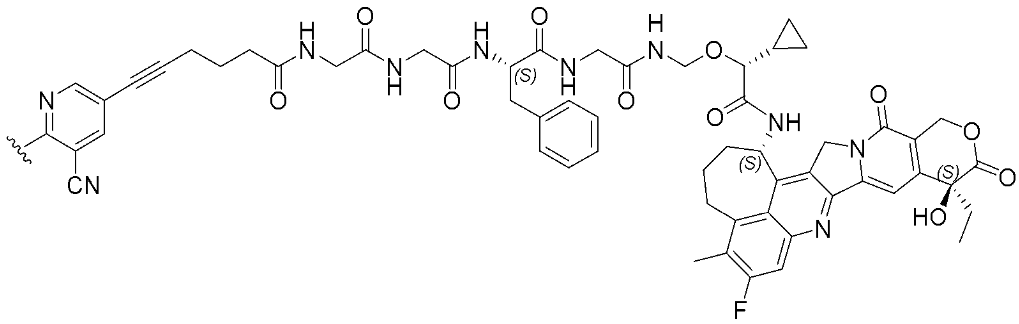

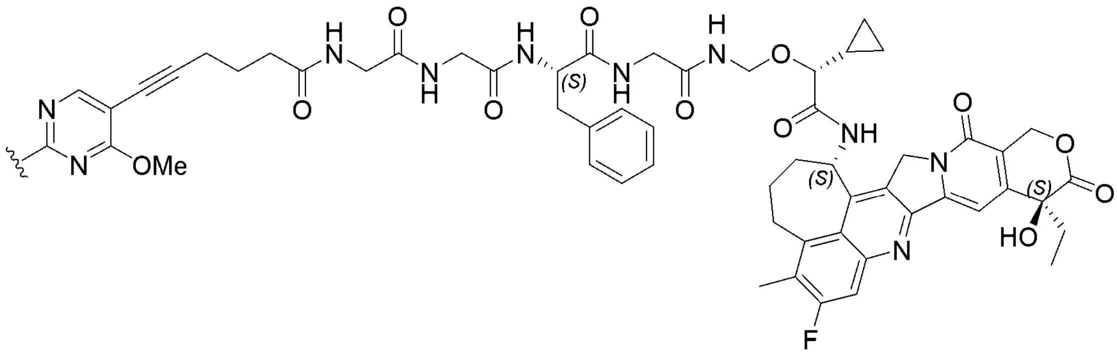

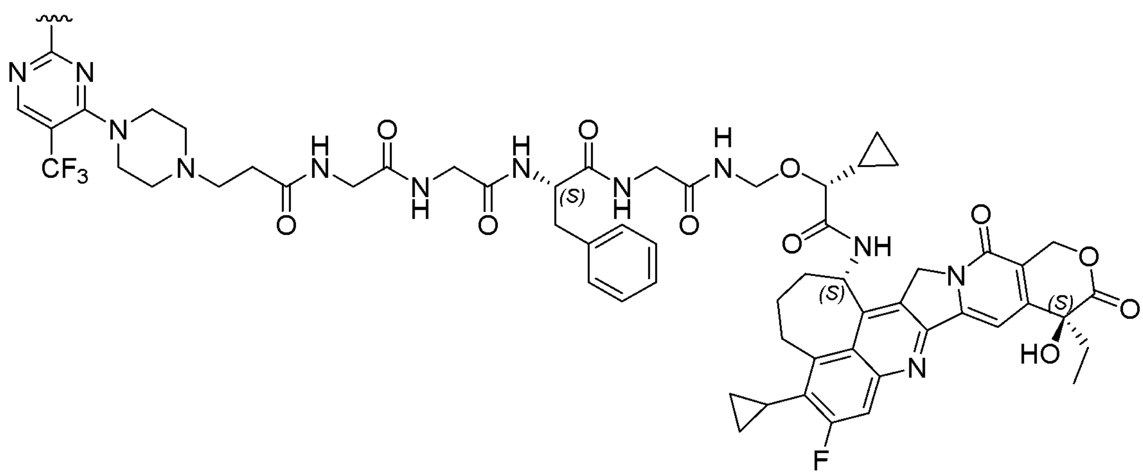

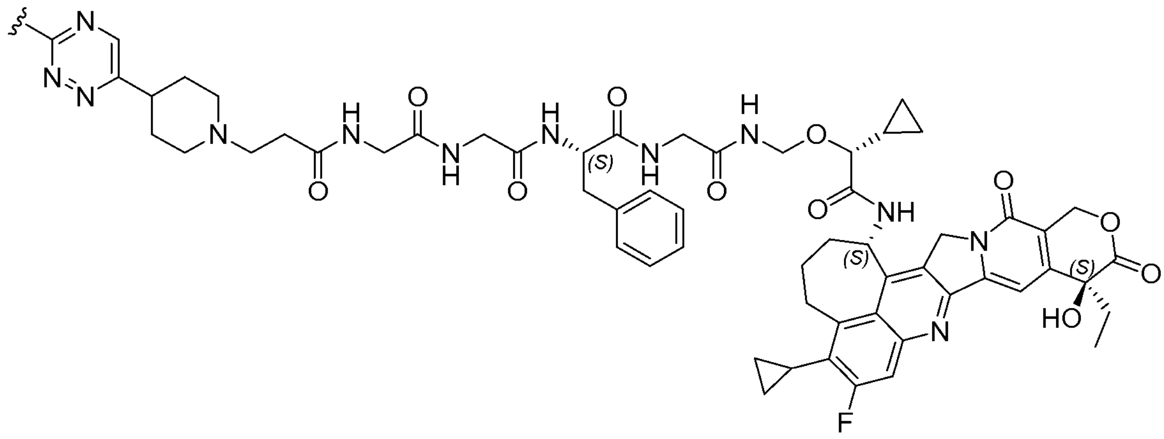

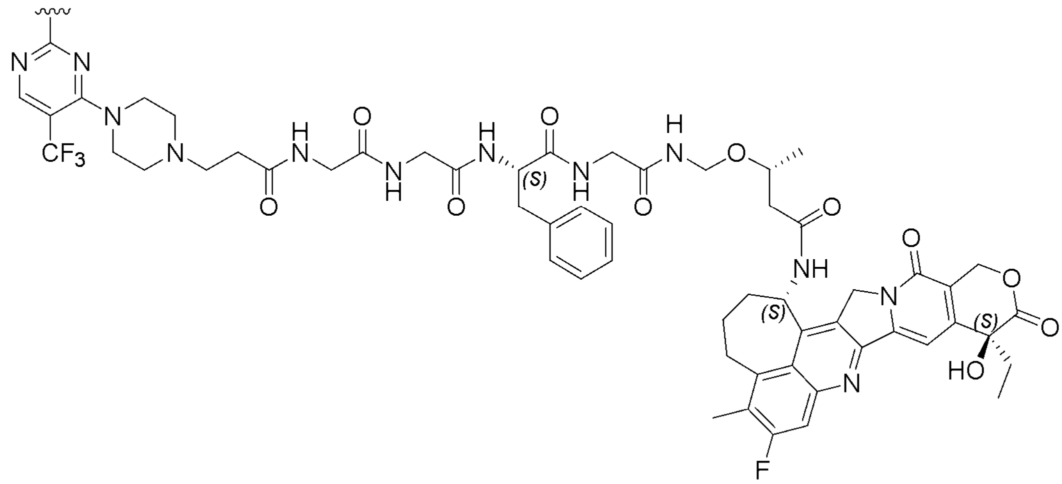

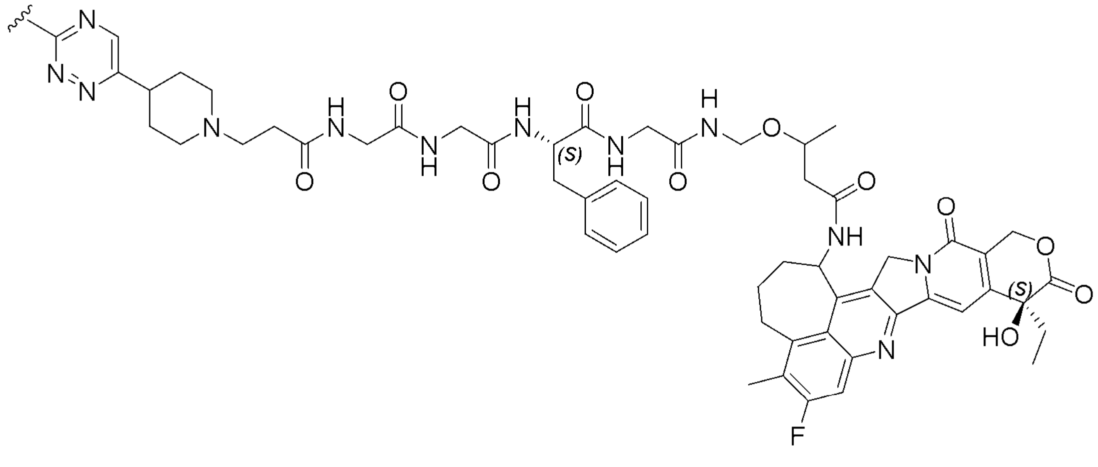

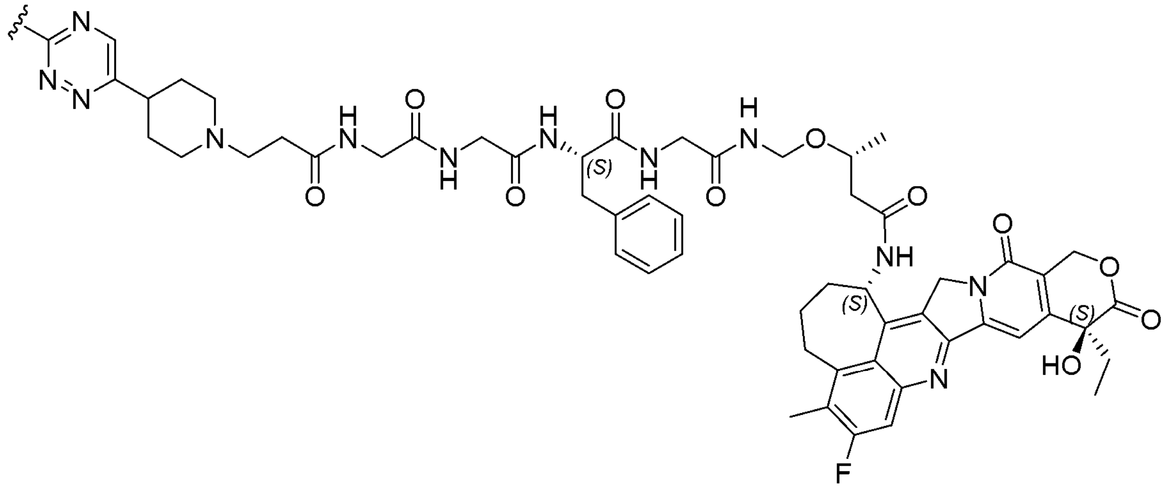

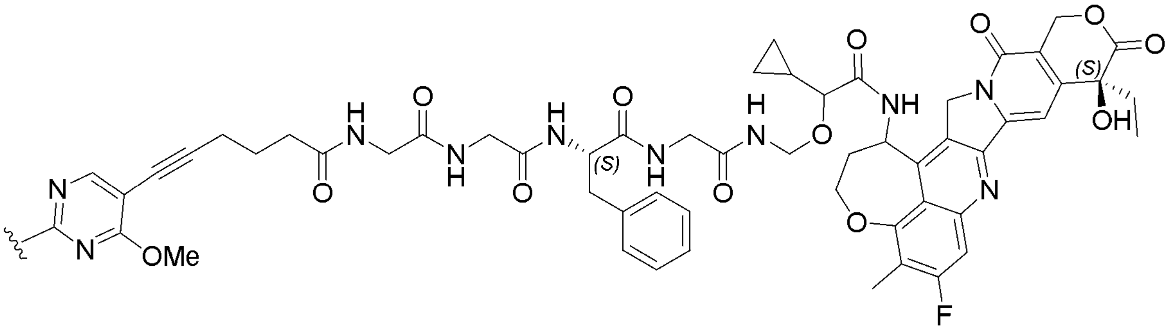

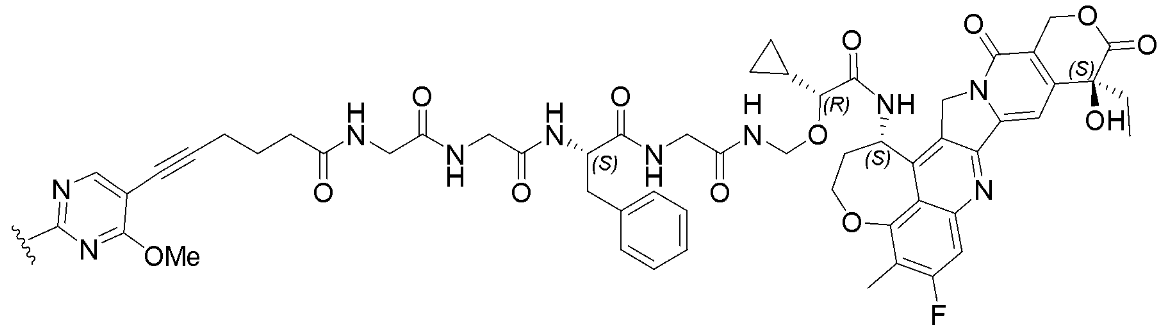

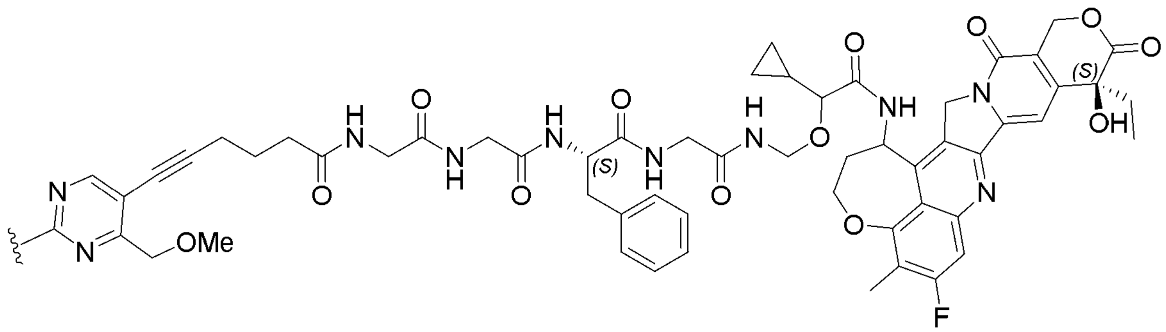

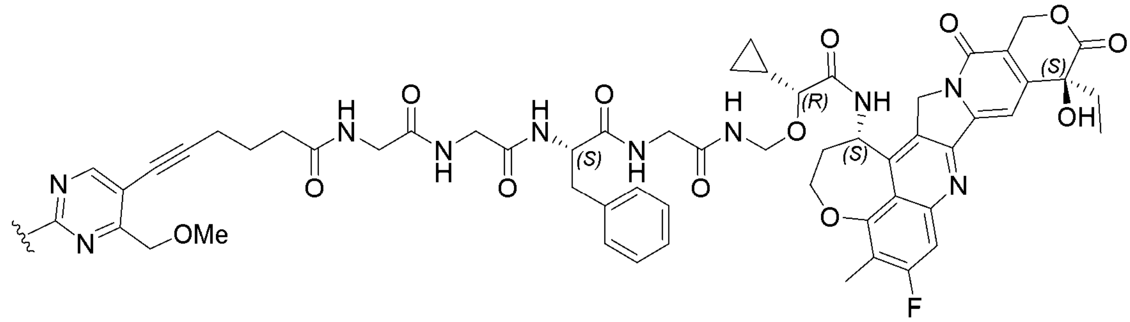

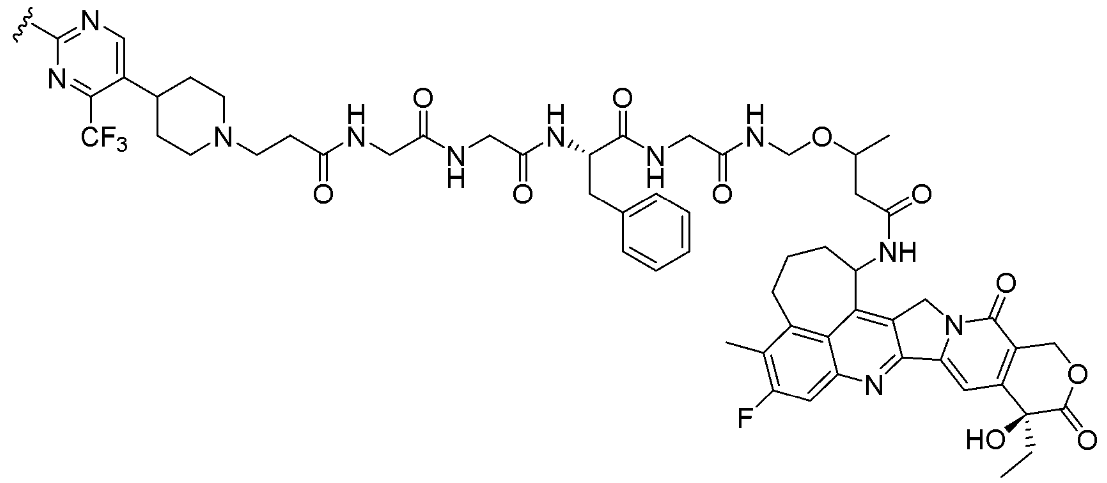

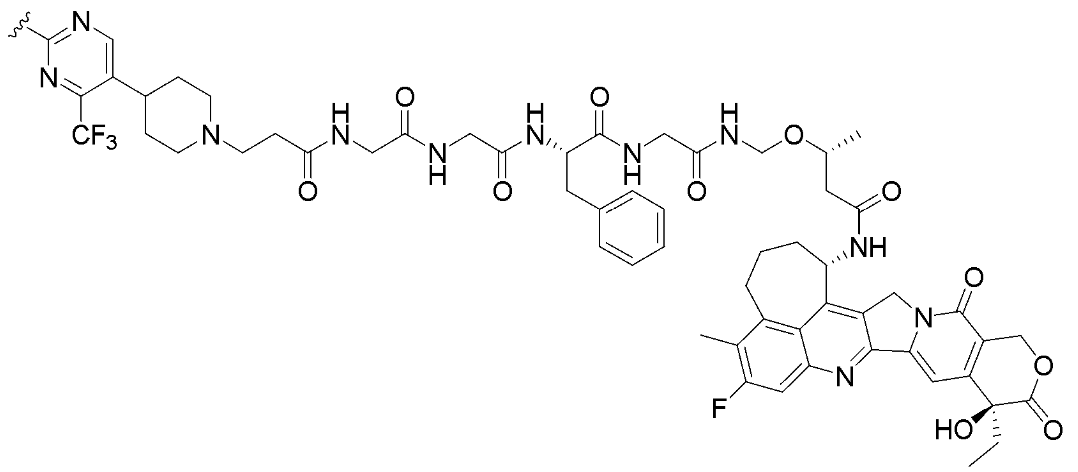

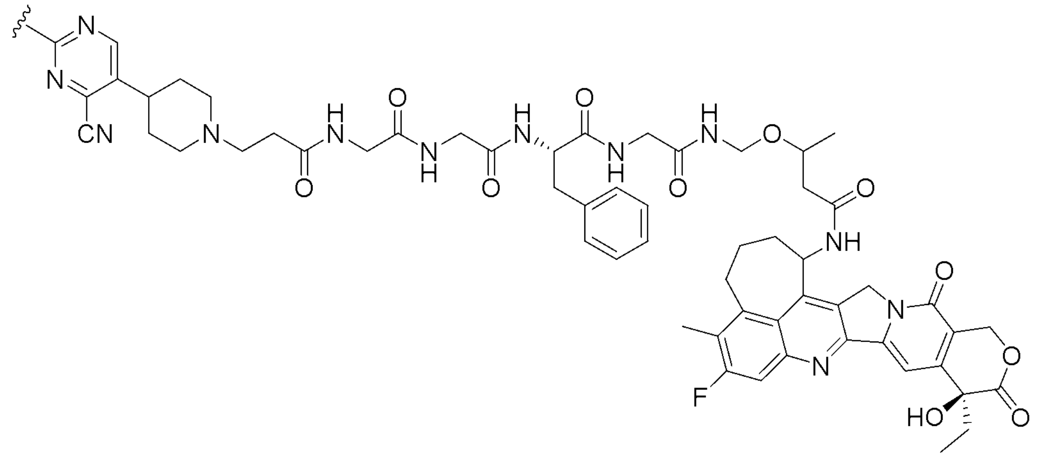

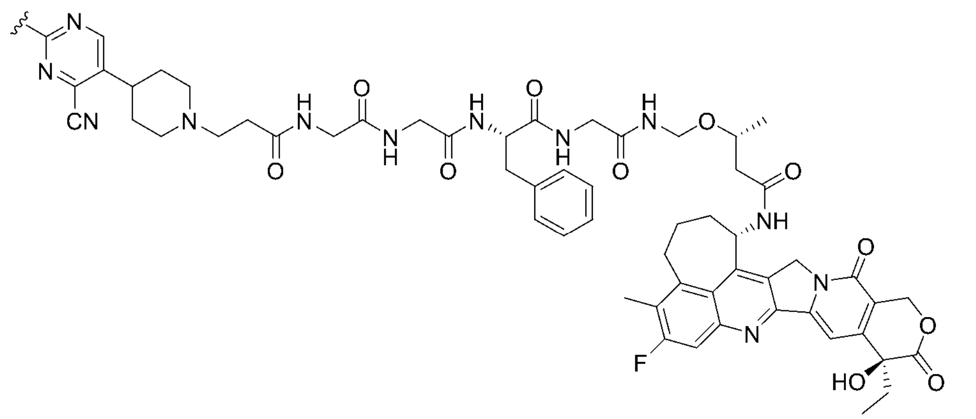

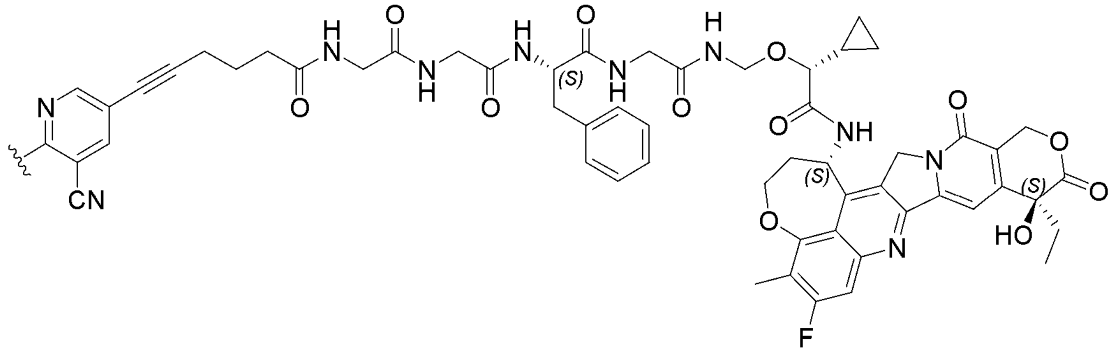

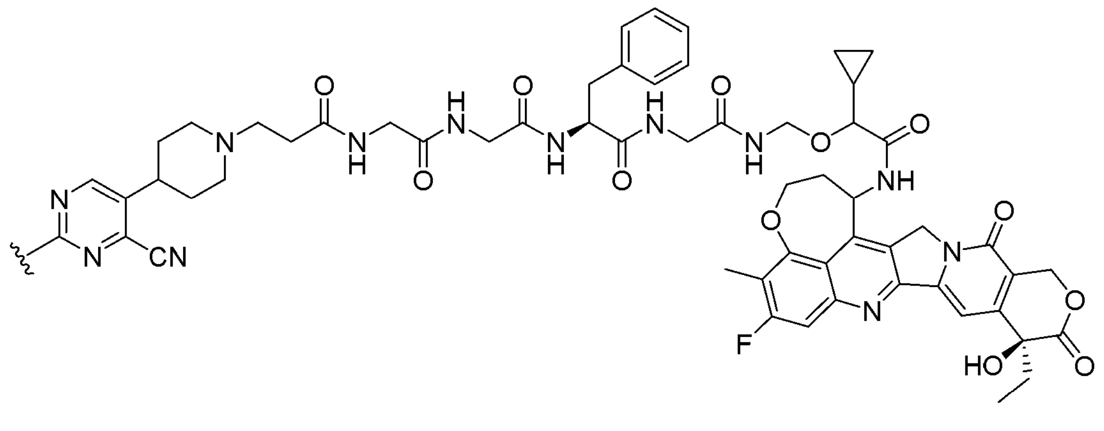

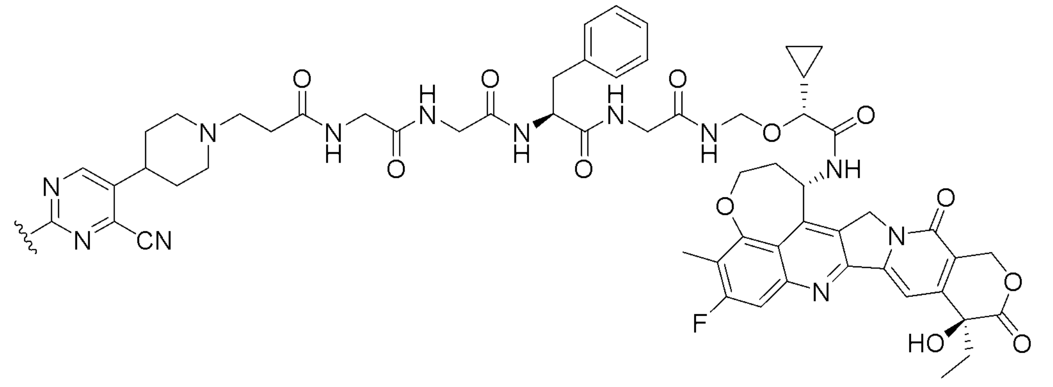

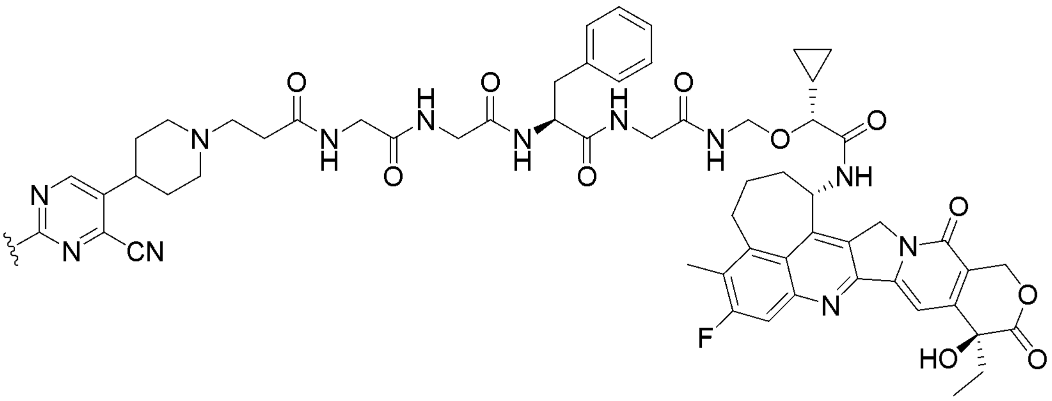

.

According to some embodiments, the antibody-drug conjugate represented by formula VI is selected from the following structures:

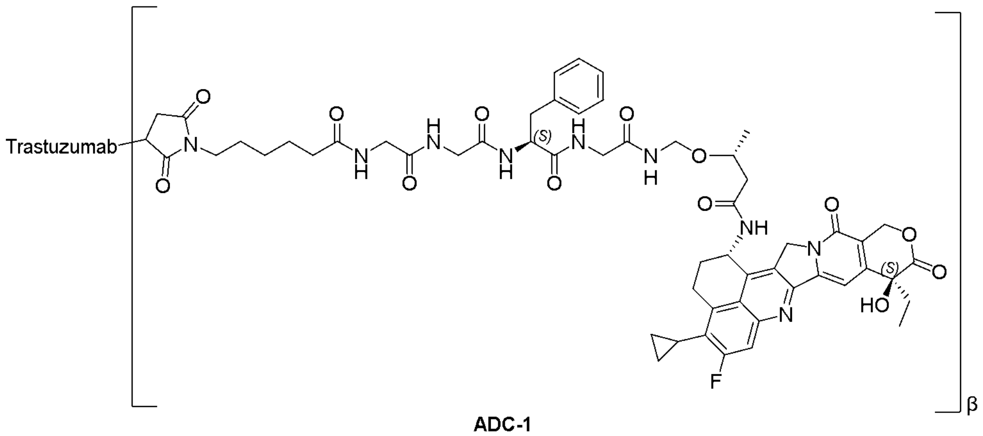

**ADC-1**

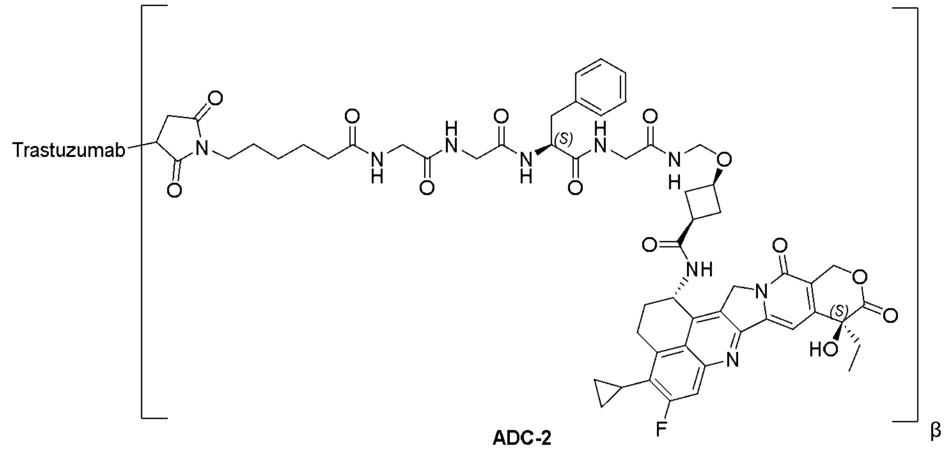

ADC-2

,

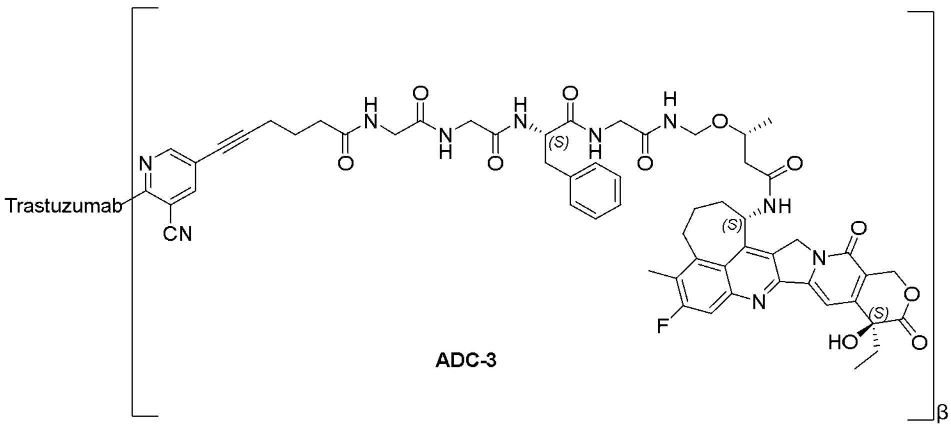

ADC-3

,

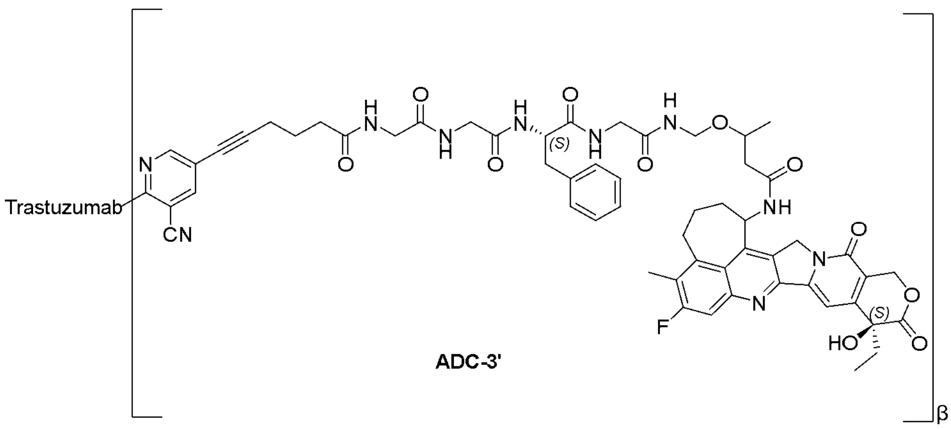

ADC-3'

,

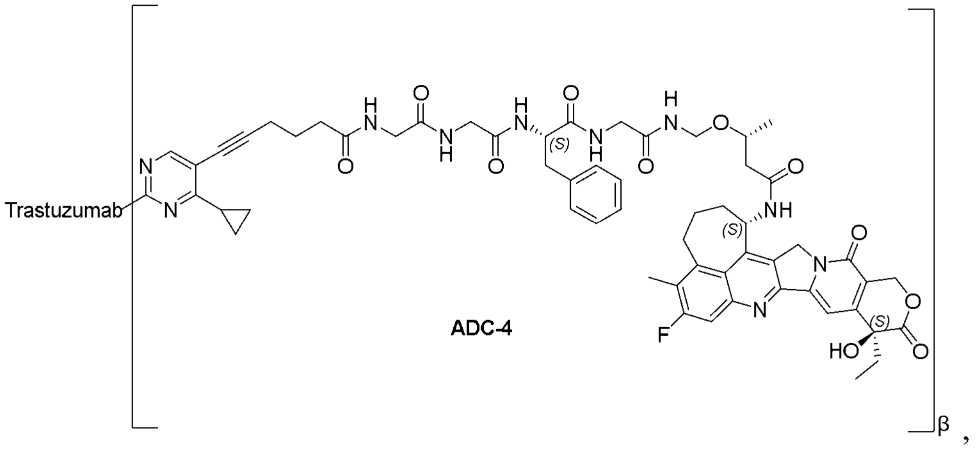

ADC-4 ,

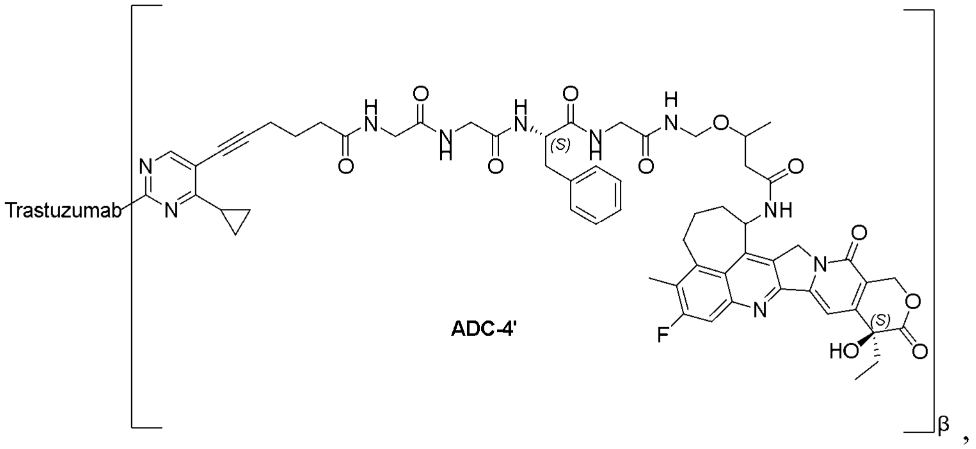

ADC-4' ,

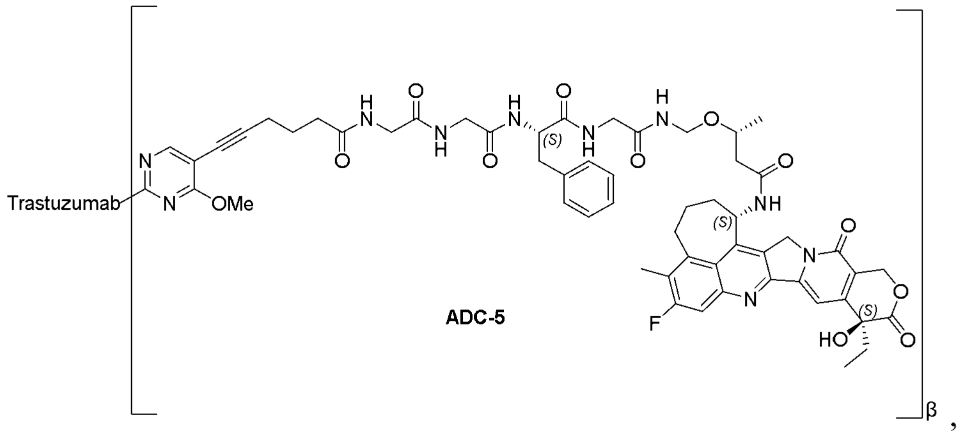

ADC-5 ,

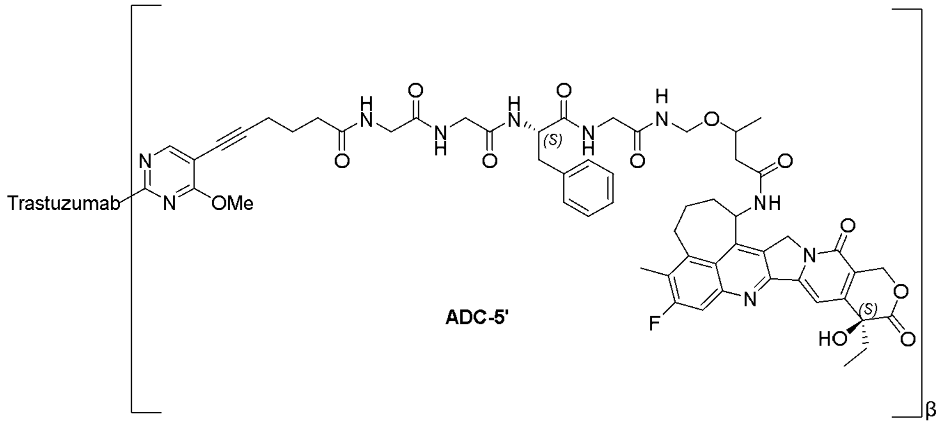

ADC-5',

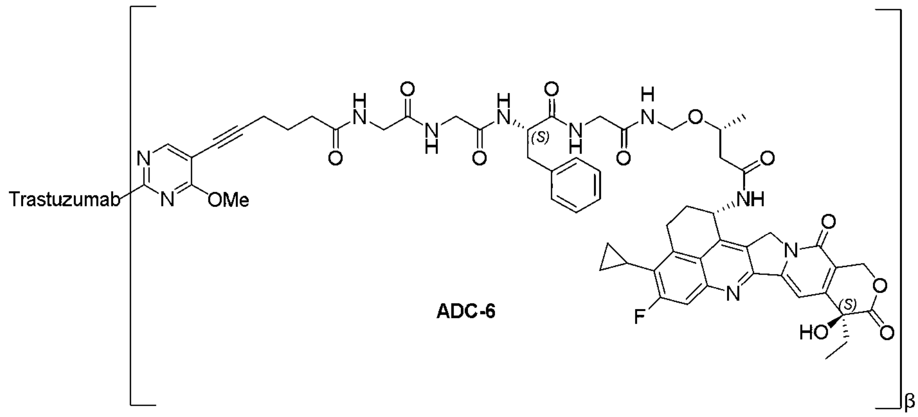

ADC-6,

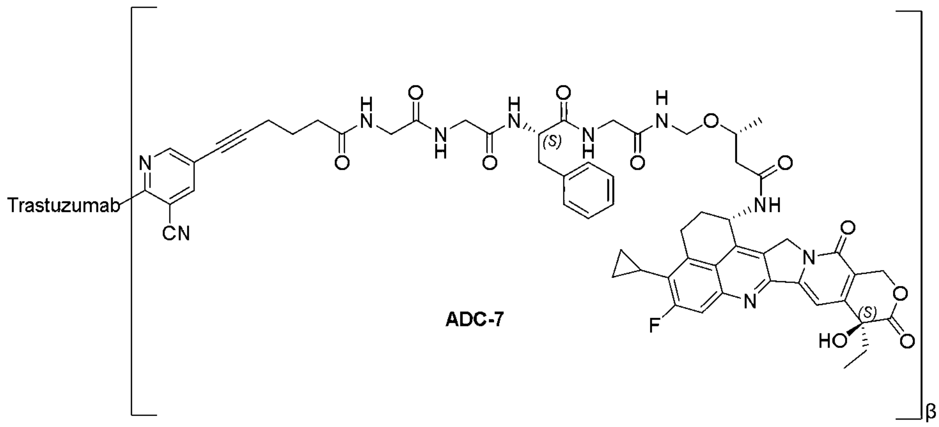

ADC-7,

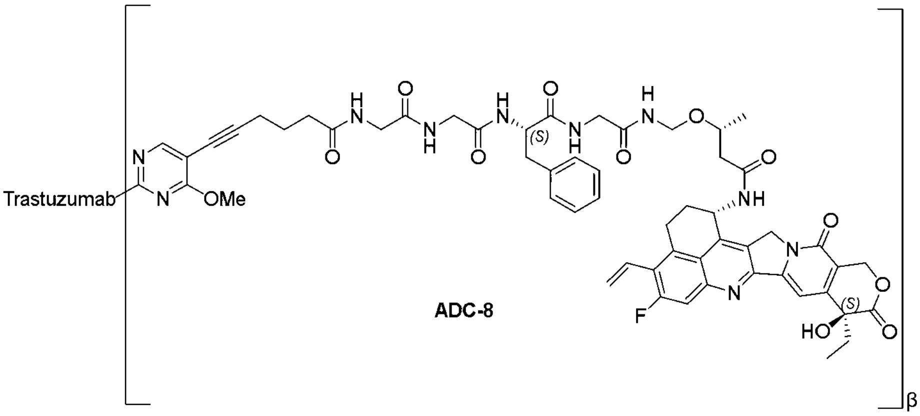

ADC-8

,

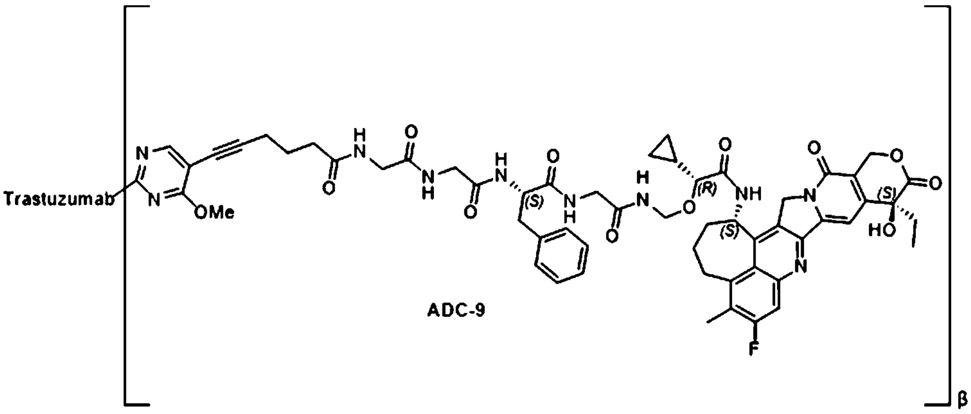

ADC-9

,

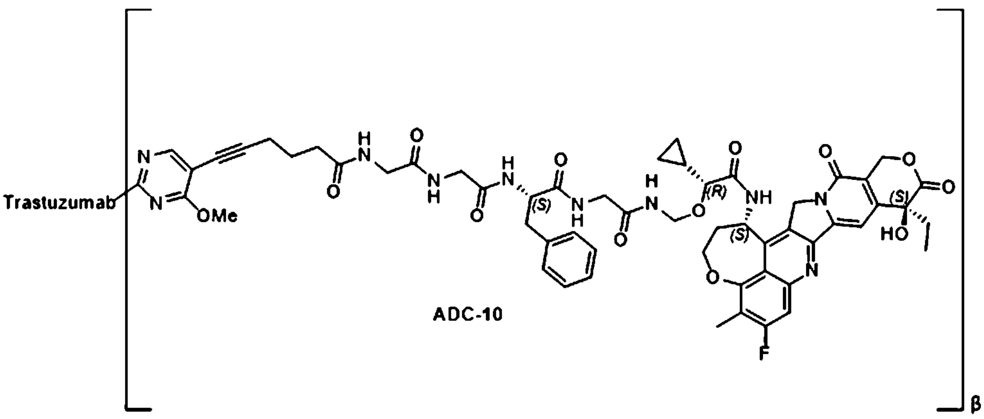

ADC-10

,

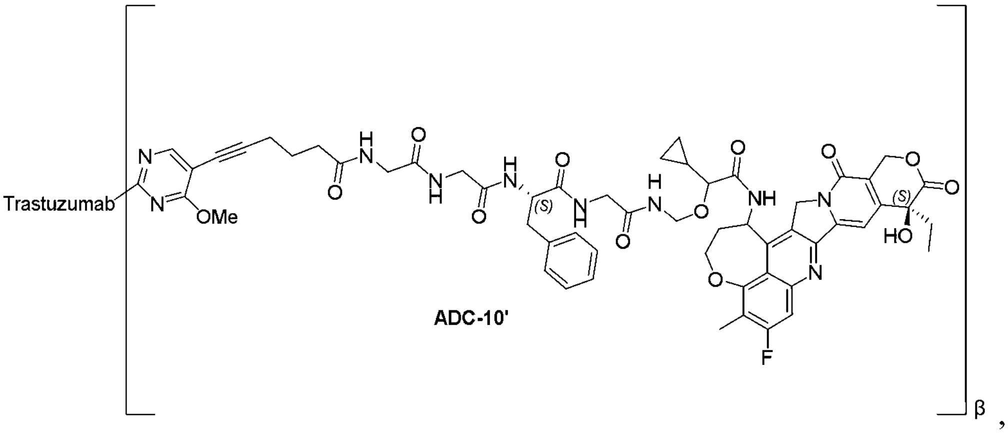

ADC-10',

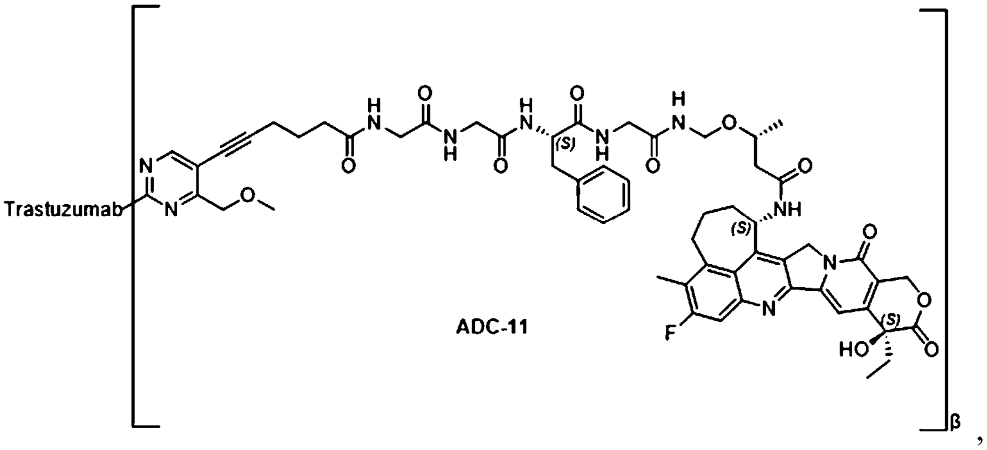

ADC-11,

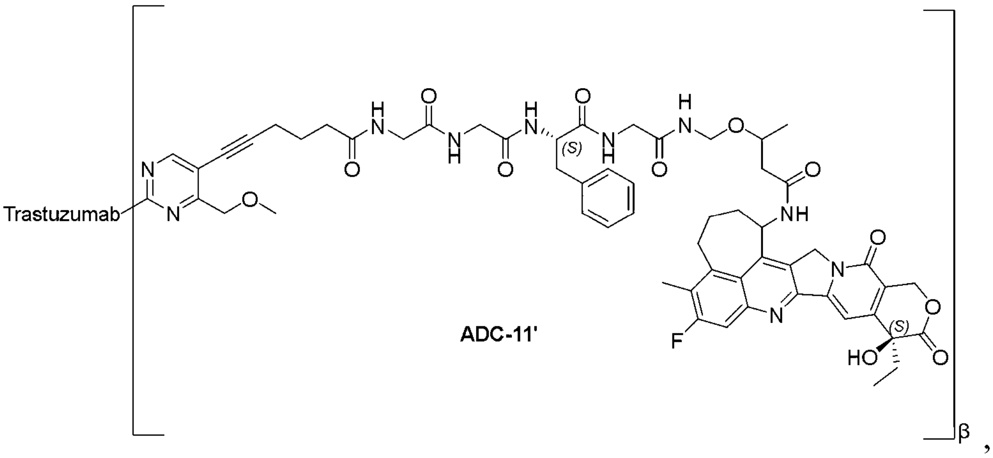

ADC-11',

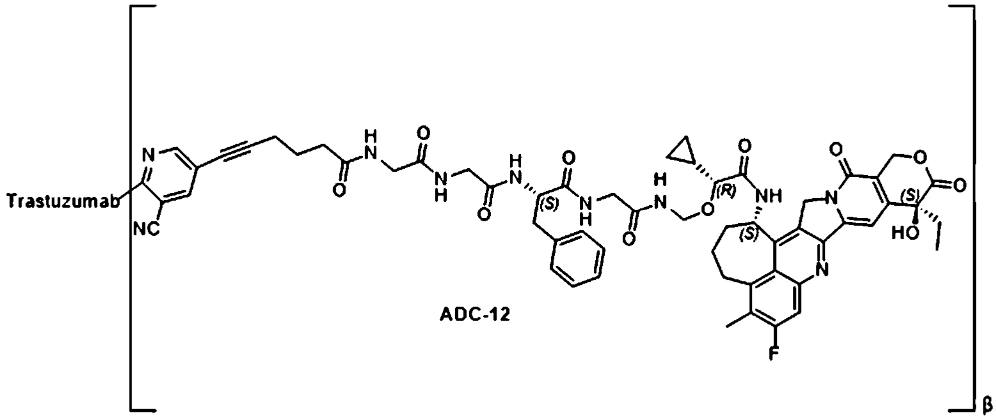

ADC-12

,

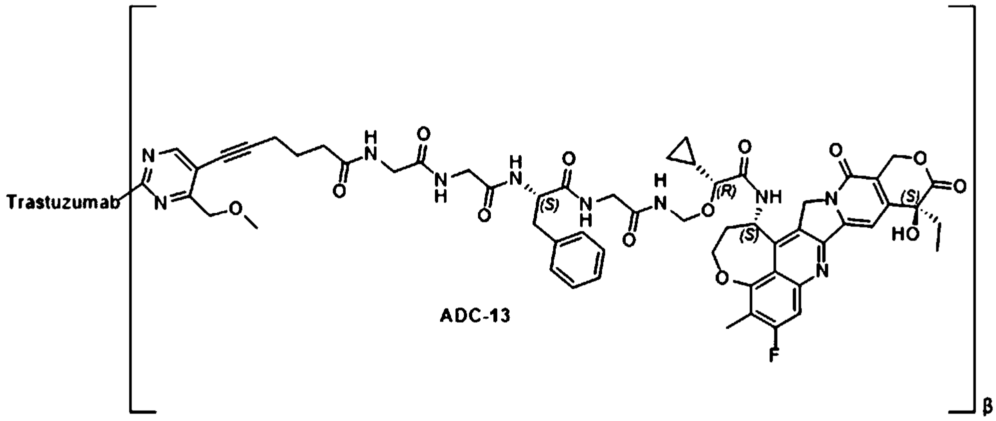

ADC-13

,

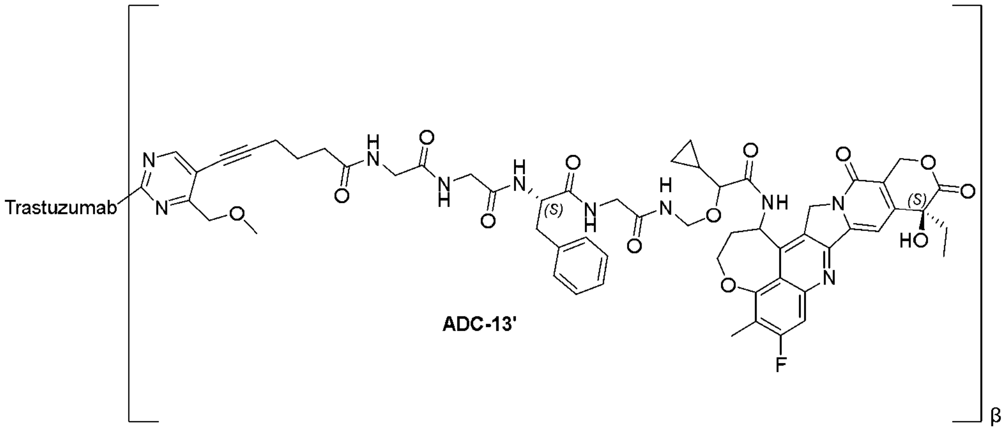

ADC-13'

,

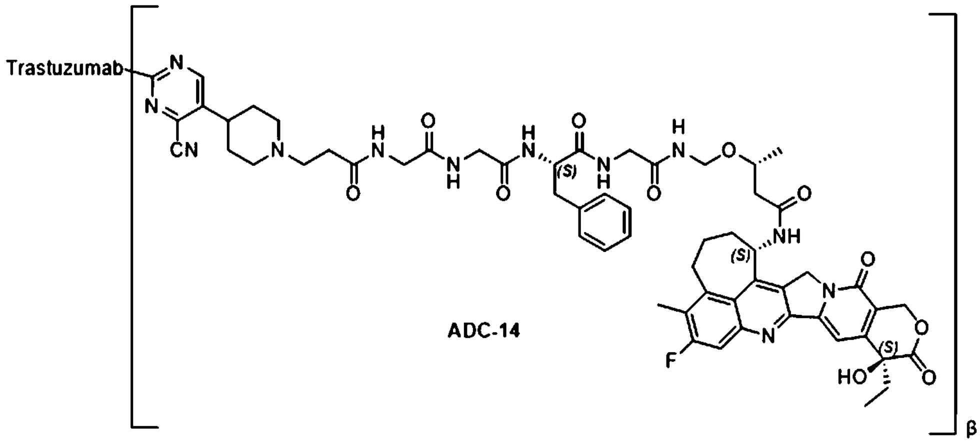

ADC-14 ,

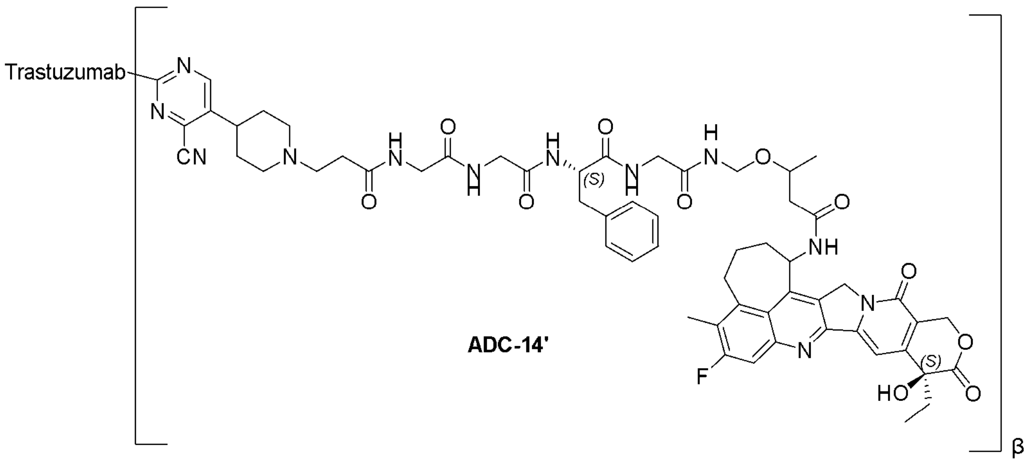

ADC-14' ,

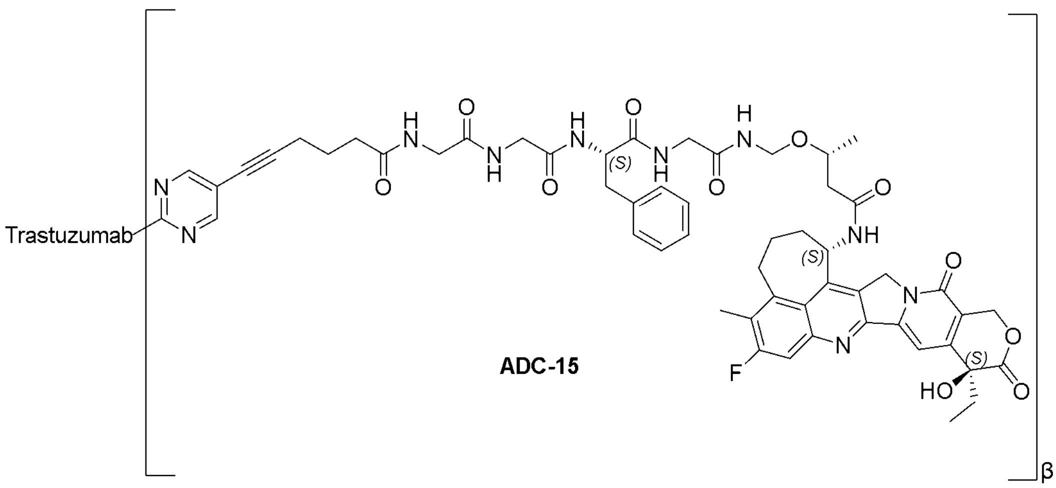

ADC-15 ,

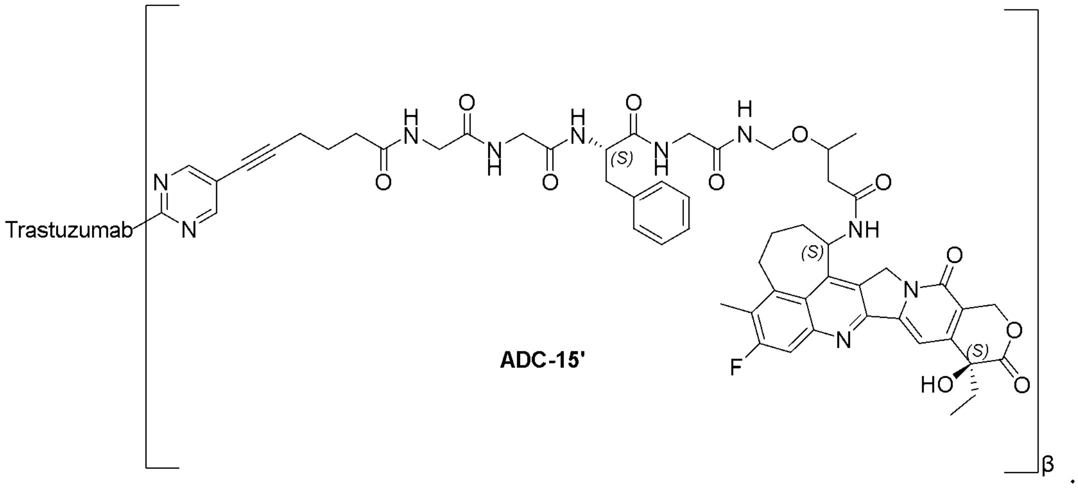

ADC-15'

The present invention further provides a method for preparing the compound of formula I, comprising Scheme 1 or Scheme 2 below:

Scheme 1: comprising steps of:

(1) removing a protecting group $PG_4$ from compound I-41 to give compound I-42; and
(2) allowing the compound I-42 to react with compound I-43 to give the compound represented by formula I;

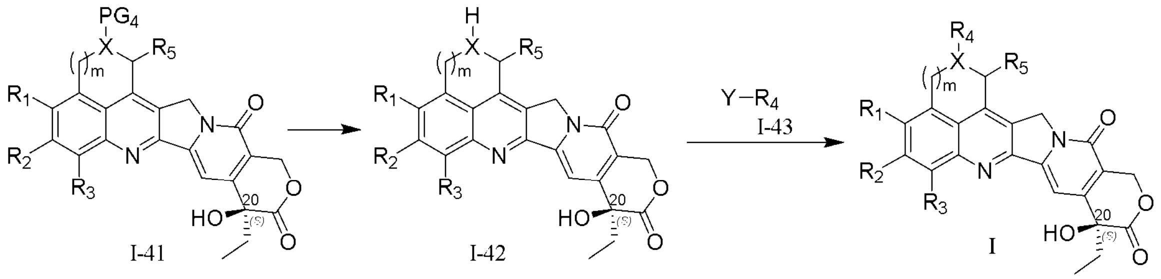


I-41 I-42 I

wherein $R_1$, $R_2$, $R_3$, $R_4$, $R_5$, X, and m are as defined herein; Y is selected from a leaving group, for example, OH, Cl, Br, or I; and $PG_4$ is selected from an amino protecting group, for example, Fmoc, Boc, Bn, or Cbz;

Scheme 2: comprising steps of:

(1) removing a protecting group $PG_5$ from compound I-51 to give compound I-52; and
(2) allowing the compound I-52 to react with compound I-53 to give the compound represented by formula I;

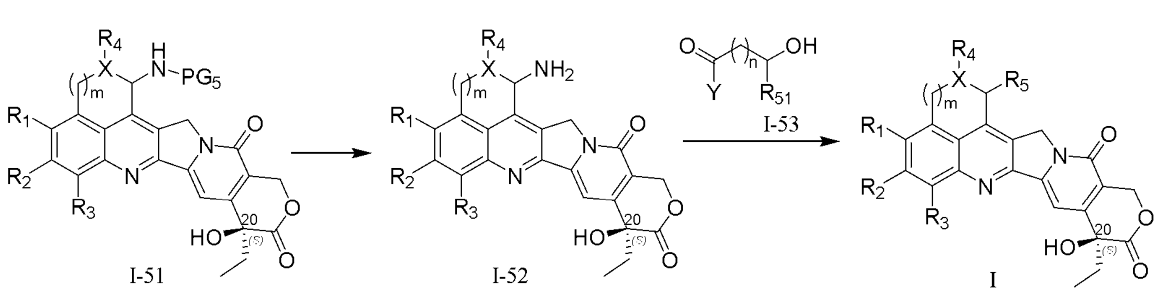


I-51 I-52 I

wherein $R_1$, $R_2$, $R_3$, $R_4$, $R_5$, $R_{51}$, X, m, and n are as defined herein; Y is selected from a leaving group, for example, OH, Cl, Br, or I; and $PG_5$ is selected from an amino protecting group, for example, Fmoc, Boc, Bn, or Cbz.

The present invention further provides a pharmaceutical composition, comprising a therapeutically effective amount of at least one of the compound represented by formula I or formula V, the racemate, the stereoisomer, the tautomer, the isotope-labeled counterpart, the solvate, the polymorph, the pharmaceutically acceptable salt or the prodrug

compound thereof.

The present invention further provides a pharmaceutical composition, comprising a therapeutically effective amount of the antibody-drug conjugate represented by formula VI.

According to embodiments of the present invention, the pharmaceutical composition further comprises one or more pharmaceutically acceptable excipients.

According to embodiments of the present invention, the pharmaceutical composition may further comprise one or more additional therapeutic agents.

The present invention further provides a method for treating tumor diseases, which comprises administering to a patient a prophylactically or therapeutically effective amount of at least one of a compound represented by formula I or formula V, a racemate, a stereoisomer, a tautomer, an isotope-labeled counterpart, a solvate, a polymorph, a pharmaceutically acceptable salt or a prodrug compound thereof.

The present invention further provides a method for treating tumor diseases, comprising administering to a patient a prophylactically or therapeutically effective amount of the above pharmaceutical composition.

The tumor disease is selected from breast cancer, gastric cancer, lung cancer, colorectal cancer, large intestine cancer, ovarian cancer, liver cancer, kidney cancer, esophageal cancer, cervical cancer, bladder cancer, pancreatic cancer, prostate cancer, nasopharyngeal carcinoma, melanoma, or leukemia.

In some embodiments, the patient comprises a mammal, and is preferably a human.

The present invention further provides at least one of a compound represented by formula I or formula V, or an antibody-drug conjugate represented by formula VI, a racemate, a stereoisomer, a tautomer, an isotope-labeled counterpart, a solvate, a polymorph, a pharmaceutically acceptable salt or a prodrug compound thereof, or a pharmaceutical composition thereof for treating tumor diseases.

The present invention further provides use of at least one of a compound represented by formula I or formula V or an antibody-drug conjugate represented by formula VI, a racemate, a stereoisomer, a tautomer, an isotope-labeled counterpart, a solvate, a polymorph, a pharmaceutically acceptable salt or a prodrug compound thereof, or the above pharmaceutical composition in the manufacture of a topoisomerase I inhibitor and/or in the manufacture of a medicament for preventing or treating a disease or condition associated with topoisomerase I.

In some embodiments, the disease or condition is tumor, comprising breast cancer, gastric cancer, lung cancer, colorectal cancer, large intestine cancer, ovarian cancer, liver cancer, kidney cancer, esophageal cancer, cervical cancer, bladder cancer, pancreatic cancer, prostate cancer, nasopharyngeal carcinoma, melanoma, or leukemia.

The present invention further provides a compound represented by formula I', a racemate, a stereoisomer, a tautomer, an isotope-labeled counterpart, a solvate, a polymorph, a pharmaceutically acceptable salt or a prodrug compound thereof:

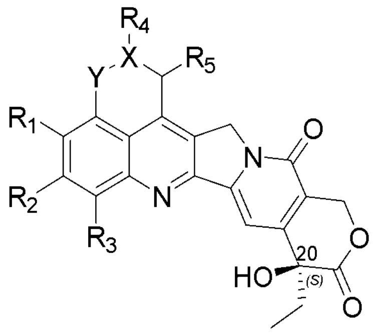

I'

wherein $R_1$, $R_2$, and $R_3$ are the same or different, and each independently selected from H, OH, CN, halogen, $C_{1-10}$ alkyl, $C_{2-10}$ alkenyl, $C_{2-10}$ alkynyl, $C_{1-10}$ alkoxy, halo-$C_{1-10}$ alkyl, halo-$C_{1-10}$ alkoxy, cyano-$C_{1-10}$ alkyl, cyano-$C_{1-10}$ alkoxy, or $C_{3-10}$ cycloalkyl;

$R_4$ is selected from H or

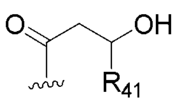

;

$R_{41}$ is selected from H, $C_{1-6}$ alkyl, $C_{1-6}$ haloalkyl, $C_{1-6}$ alkyl-NH-, ($C_{1-6}$ alkyl)$_2$N-, $C_{1-6}$ alkyl-NH-$C_{1-6}$ alkyl, ($C_{1-6}$

alkyl)$_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl;
$R_5$ is selected from H,

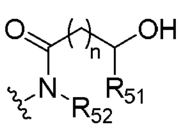
,
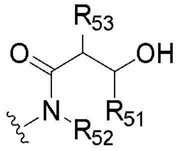
, or
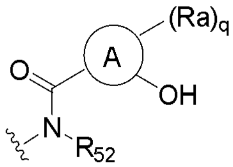
;

$R_{51}$ and $R_{52}$ are the same or different and each independently selected from H, $C_{1-6}$ alkyl, $C_{1-6}$ haloalkyl, $C_{1-6}$ alkyl-NH-, ($C_{1-6}$ alkyl)$_2$N-, $C_{1-6}$ alkyl-NH-$C_{1-6}$ alkyl, ($C_{1-6}$ alkyl)$_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl; $R_{53}$ is selected from $C_{1-6}$ alkyl, $C_{1-6}$ haloalkyl, $C_{1-6}$ alkyl-NH-, ($C_{1-6}$ alkyl)$_2$N-, $C_{1-6}$ alkyl-NH-$C_{1-6}$ alkyl, ($C_{1-6}$ alkyl)$_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$aryl, and 5-14-membered heteroaryl; ring A is selected from $C_{3-8}$ cycloalkyl or $C_{3-8}$ heterocyclyl; Ra is selected from H, hydroxyl, CN, halogen, $C_{1-6}$ alkyl, or $C_{1-6}$ haloalkyl; n is selected from 0, 1, or 2; and q is selected from 0, 1, or 2;
X is selected from CH or N;
Y is selected from $-(CH_2)_m-O-(CH_2)_p-$;
m is an integer selected from 0-6; and
p is an integer selected from 0-6.

According to some embodiments, $R_1$ is selected from H, OH, CN, halogen, $C_{1-6}$ alkyl, $C_{2-6}$ alkenyl, $C_{2-6}$ alkynyl, $C_{3-6}$ cycloalkyl, or halo-$C_{1-6}$ alkoxy;

preferably, $R_1$ is selected from H, OH, Br, methyl, difluoromethoxy, 2,2,2-trifluoroethoxy, vinyl, cyclopropyl, or ethynyl;
preferably, $R_2$ is selected from H, halogen, CN, or $C_{1-6}$ alkyl;
preferably, $R_2$ is selected from H or F;
preferably, $R_3$ is selected from H or $C_{1-6}$ alkyl; and
preferably, $R_3$ is H.

According to some embodiments, $R_4$ is selected from H or

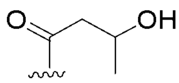
;

for example,

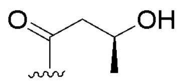
or
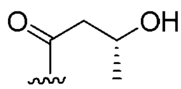
;

preferably, $R_4$ is selected from H or

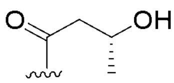
;

preferably, X-$R_4$ is

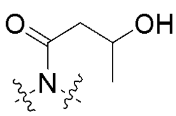
,

and is preferably

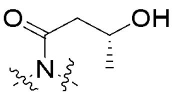

;

preferably, X-$R_4$ is $-CH_2-$; and
preferably, Y is selected from $-CH_2-O-$.

According to some embodiments, $R_5$ is selected from H,

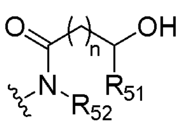 , 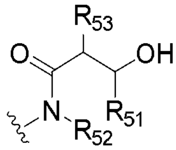 ,

or

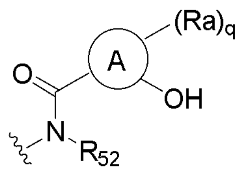

;

wherein $R_{51}$ is selected from H, methyl, ethyl, isopropyl, or cyclopropyl; $R_{52}$ is selected from H or methyl; $R_{53}$ is selected from methyl; ring A is selected from $C_{3-6}$ cycloalkyl; Ra is selected from H, hydroxyl, CN, halogen, $C_{1-6}$ alkyl, or $C_{1-6}$ haloalkyl; n is selected from 0 or 1; and q is selected from 0 or 1;

preferably, ring A is selected from a cyclobutane ring;

preferably, $R_5$ is selected from H,

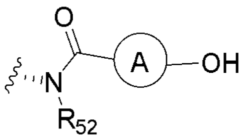 , 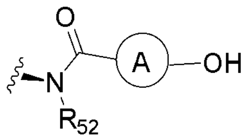 , 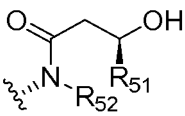 ,

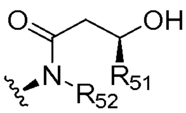 , 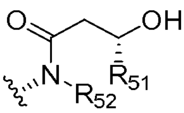 , 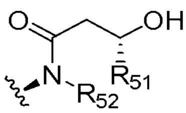 , 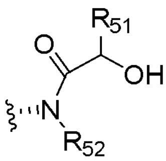 , 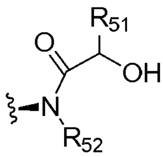 , 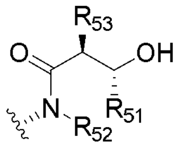 ,

or

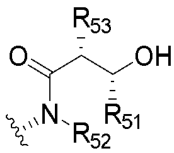

;

preferably, $R_{51}$ is selected from H, methyl, ethyl, isopropyl, or cyclopropyl; $R_{52}$ is selected from H or methyl; $R_{53}$ is selected from methyl; and ring A is selected from a cyclobutane ring; and

preferably, $R_5$ is selected from H,

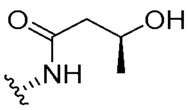 , 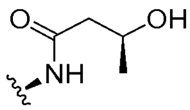 , 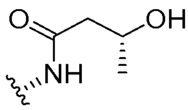 ,

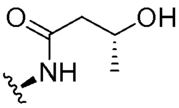 , 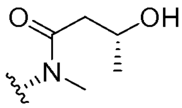 , 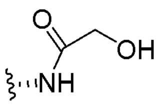 , 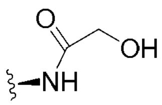 , 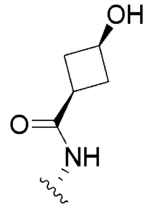 , 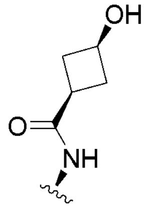 ,

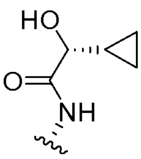 , 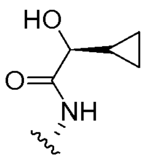 , 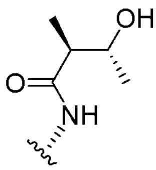 , 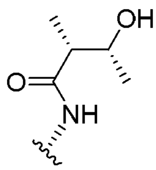 , 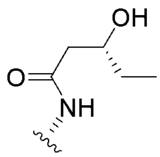 , 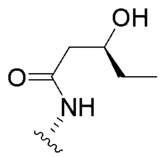 , 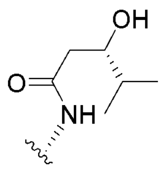 ,

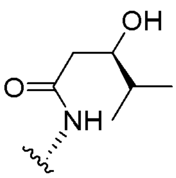 , 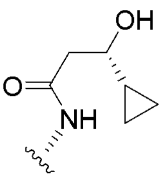 or 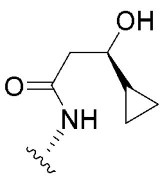 .

According to some embodiments, X is selected from CH or N; and when X is CH, $R_4$ is H; or when X is N, $R_5$ is H; and
preferably, m is selected from 0, 1 or 2; further preferably, m+p=2.

According to some embodiments, the compound represented by formula I' has a structure as shown below:

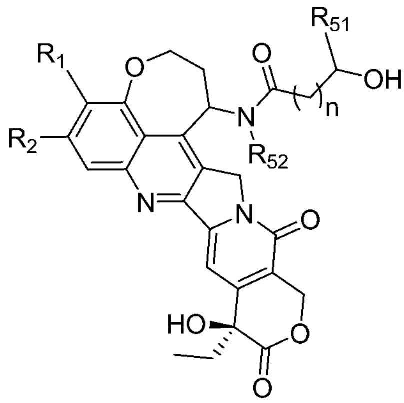 , 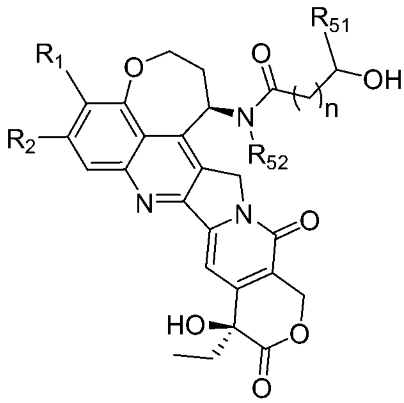 ;

III-d III-d-1

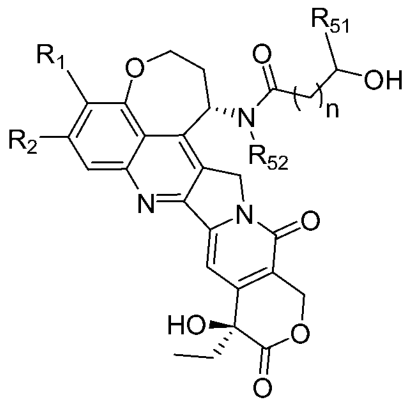,
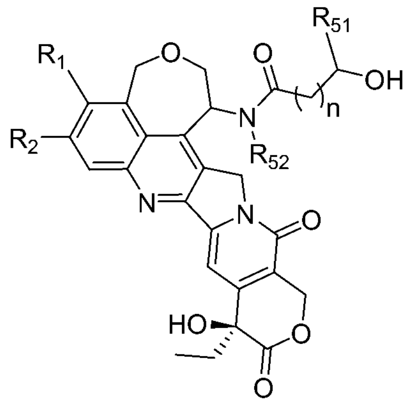;

III-d-2 III-e

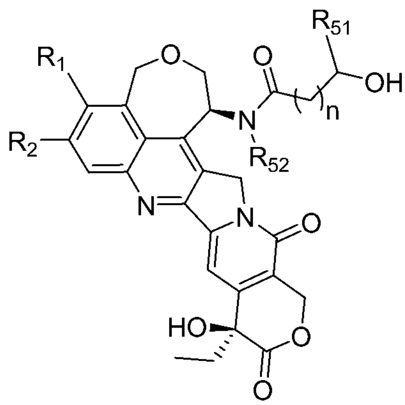,
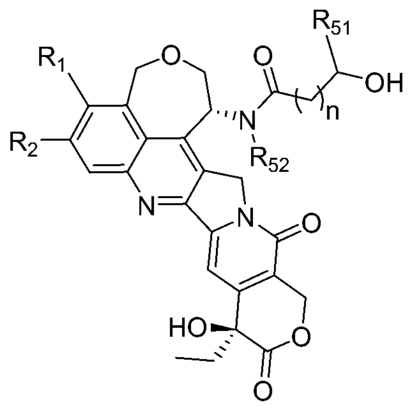;

III-e-1 III-e-2

wherein $R_1$, $R_2$, $R_{51}$, $R_{52}$, and n are each independently as defined herein.

According to some embodiments, the compound represented by formula I' has a structure as shown below:

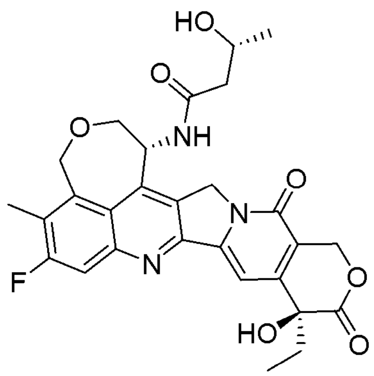,
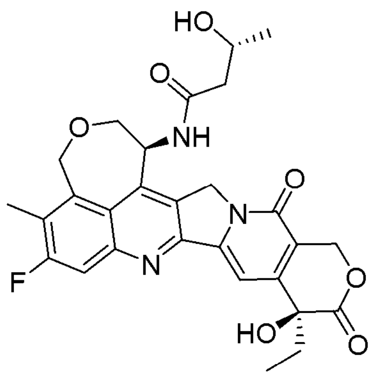,
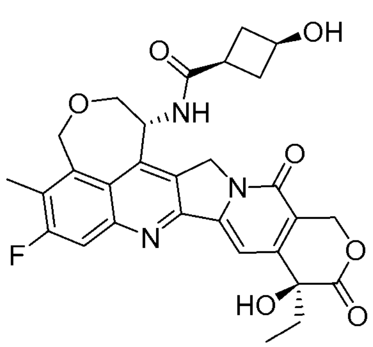,

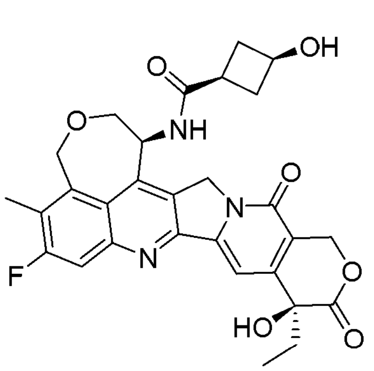,
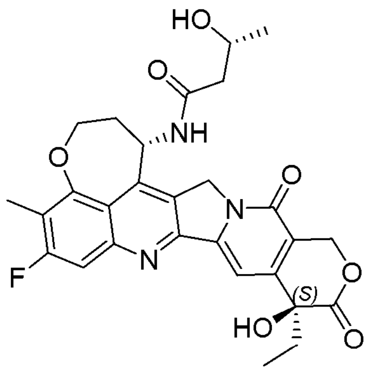,
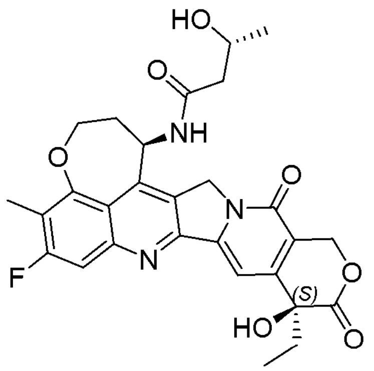,
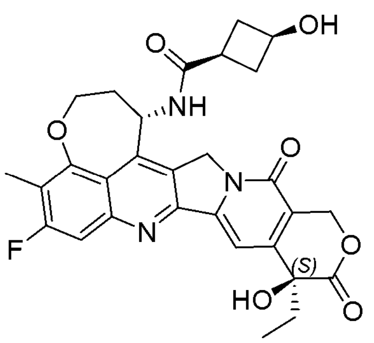,
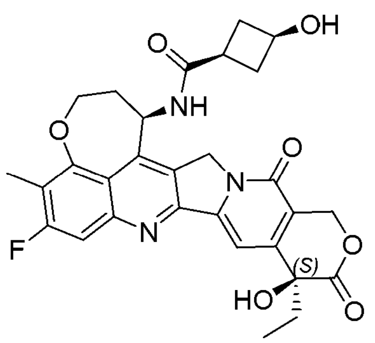,
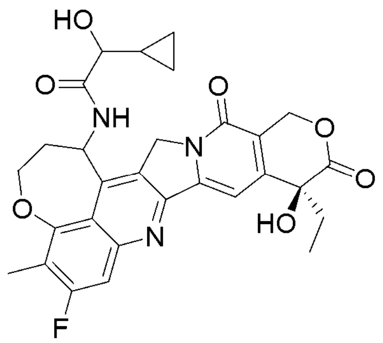,
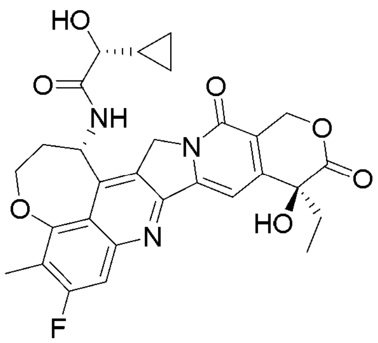,
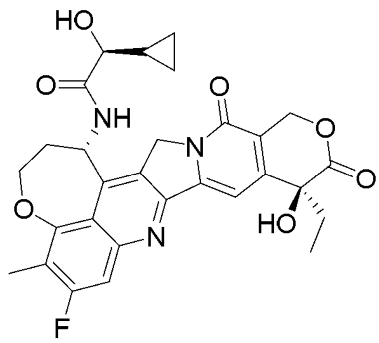,
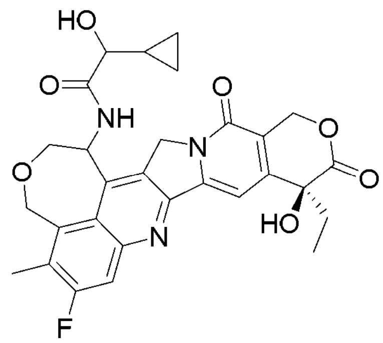,
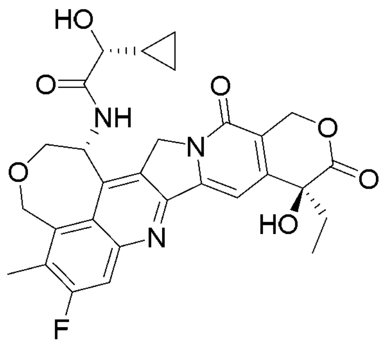, or
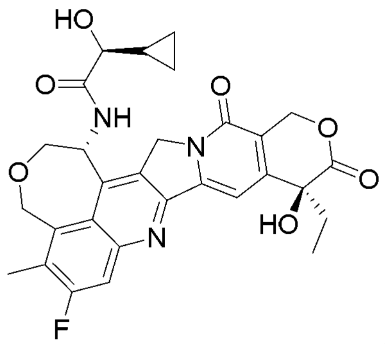.

The present invention further provides a compound represented by formula I", a racemate, a stereoisomer, a tautomer, an isotope-labeled counterpart, a solvate, a polymorph, a pharmaceutically acceptable salt or a prodrug compound thereof:

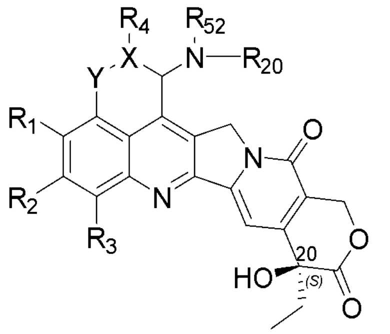

I''

wherein $R_1$, $R_2$, and $R_3$ are the same or different and each independently selected from H, OH, CN, halogen, $C_{1-10}$ alkyl, $C_{2-10}$ alkenyl, $C_{2-10}$ alkynyl, $C_{1-10}$ alkoxy, halo-$C_{1-10}$ alkyl, halo-$C_{1-10}$ alkoxy, cyano-$C_{1-10}$ alkyl, cyano-$C_{1-10}$ alkoxy, or $C_{3-10}$ cycloalkyl;

$R_4$ is selected from H or

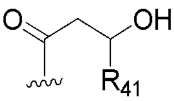 ;

$R_{41}$ is selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl;

$R_{52}$ is selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl;

$R_{20}$ is selected from H or an amino protecting group;

preferably, $R_{20}$ is selected from Fmoc, Boc, Bn, or Cbz;

X is selected from CH or N;

Y is selected from $-(CH_2)_m-O-(CH_2)_p-$;

m is an integer selected from 0-6; and

p is an integer selected from 0-6;

preferably, $R_1$ is selected from H, OH, CN, halogen, $C_{1-6}$ alkyl, $C_{2-6}$ alkenyl, $C_{2-6}$ alkynyl, $C_{3-6}$ cycloalkyl or halo-$C_{1-6}$ alkoxy;

preferably, $R_1$ is selected from H, OH, Br, methyl, difluoromethoxy, 2,2,2-trifluoroethoxy, vinyl, cyclopropyl, or ethynyl;

preferably, $R_2$ is selected from H, halogen, CN, or $C_{1-6}$ alkyl;

preferably, $R_2$ is selected from H or F;

preferably, $R_3$ is selected from H or $C_{1-6}$ alkyl;

preferably, $R_3$ is H;

$R_4$ is selected from H;

preferably, the formula I" is the following compounds:

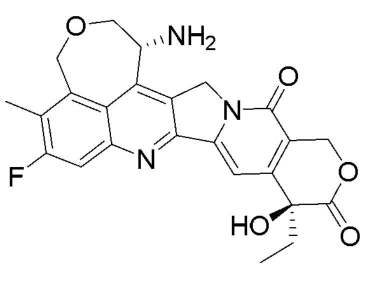 ,

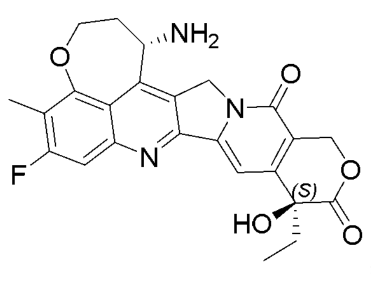 ,

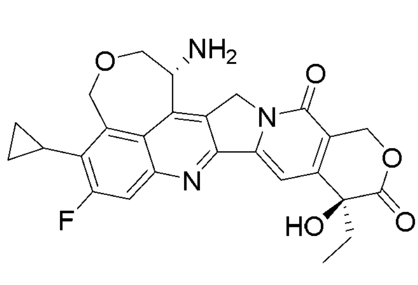 ,

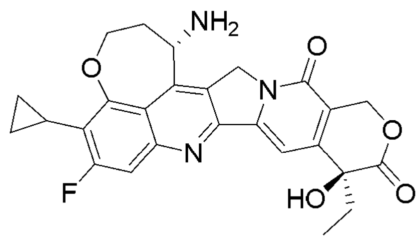 ,

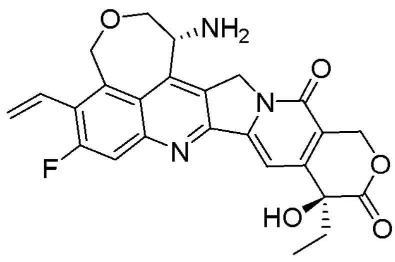 ,

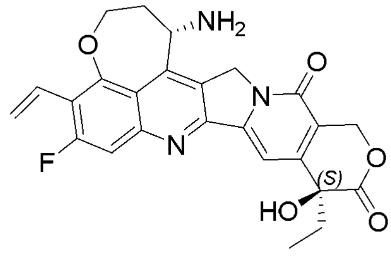 ,

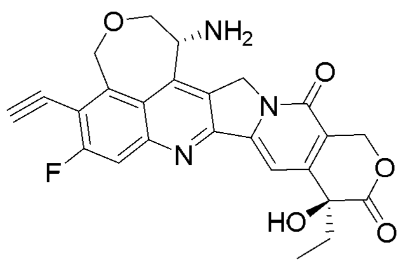 ,

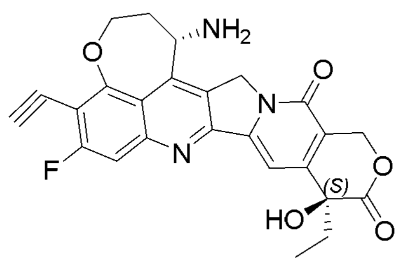 ,

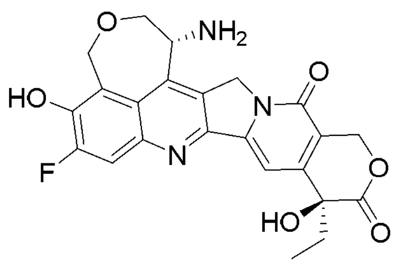 ,

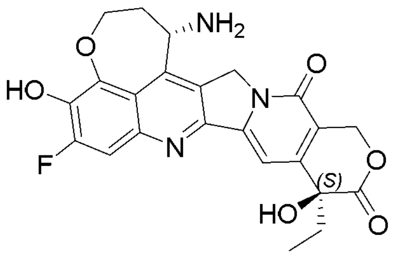 ,

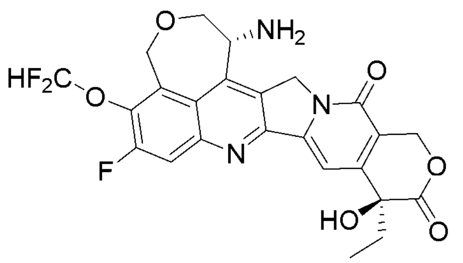 ,

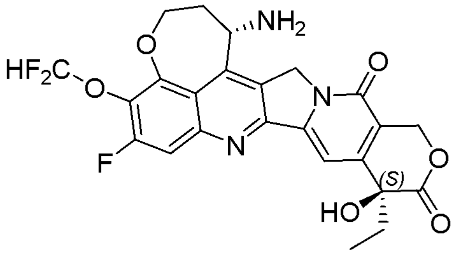 ,

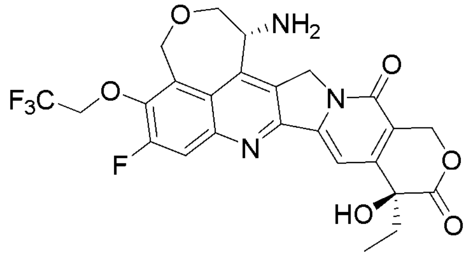 ,

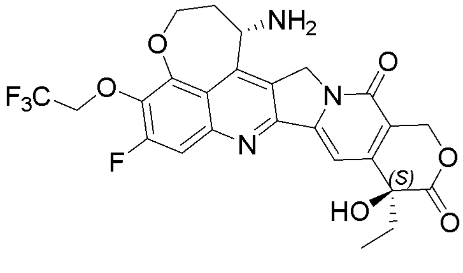 ,

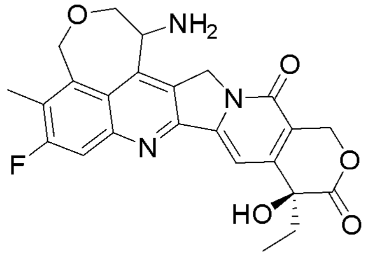 ,

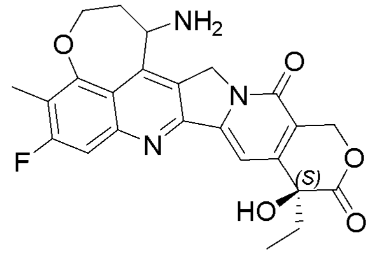 ,

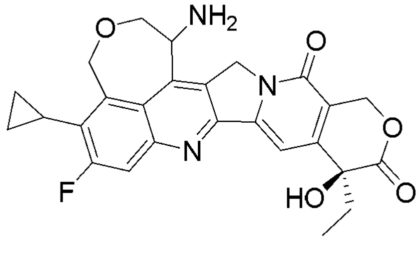 ,

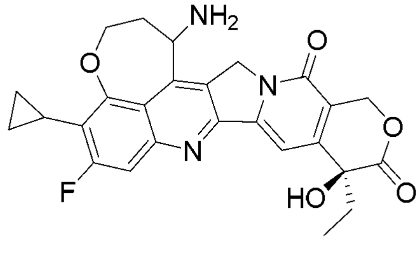 ,

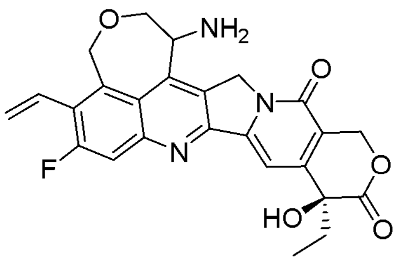,
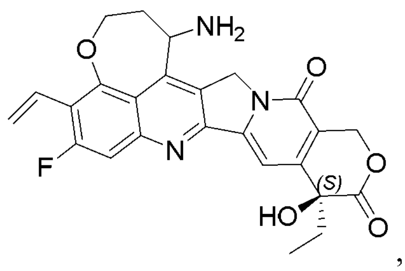,
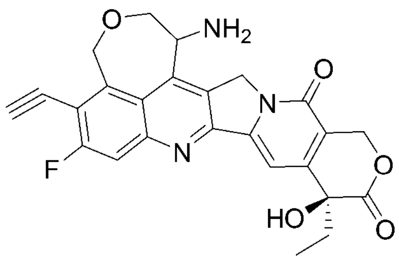,

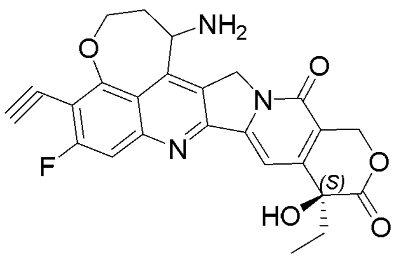,
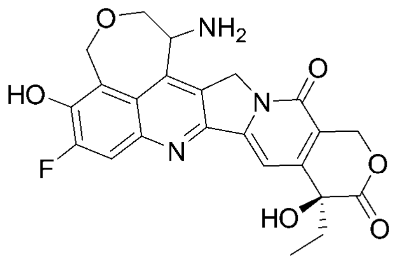,
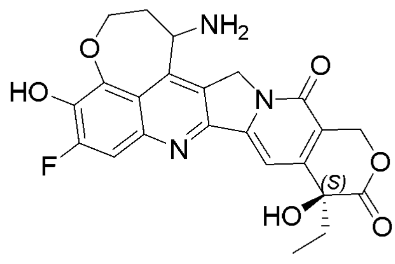,

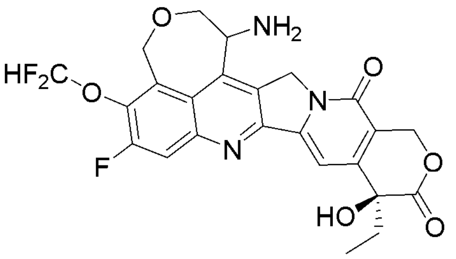,
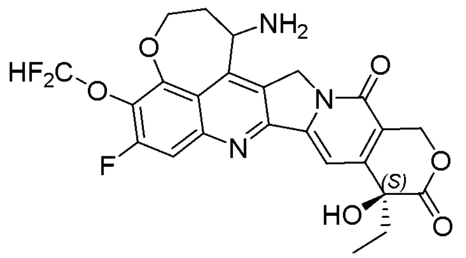,

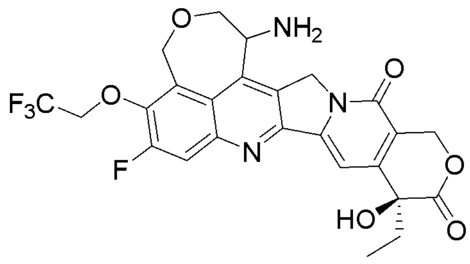, or
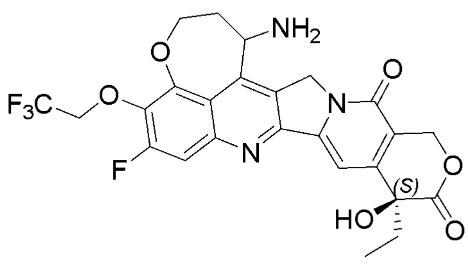.

The present invention further provides a compound represented by formula MII

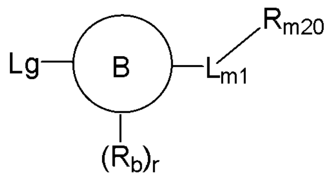

(MII)

wherein Lg is a leaving group, and the leaving group is selected from halogen, sulfone, trifluoromethanesulfonyl or methanesulfonyl; preferably, Lg is methanesulfonyl;

ring B is selected from a 5-6-membered N-containing heteroaromatic ring or a 3-6 membered N-containing heterocyclic ring;

more preferably, ring B is selected from a pyrimidine ring, a pyridine ring or a triazine ring;

each $R_b$ is the same or different and each independently selected from the following groups: halogen, cyano, oxo (=O), $C_{1-6}$ alkyl, halo-$C_{1-6}$ alkyl, hydroxy-$C_{1-6}$ alkyl, $C_{1-6}$ alkoxy or $C_{3-8}$ cycloalkyl, 3-8-membered heterocyclyl, $C_{1-6}$ alkyl-O-

$C_{1-6}$ alkyl-, or $C_{1-6}$ alkyl-(5-6-membered) heteroaryl;
preferably, each $R_b$ is the same or different and each independently selected from cyano, oxo (=O), methoxy, cyclopropyl, trifluoromethyl,

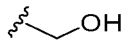, 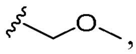, 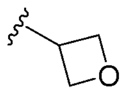 or 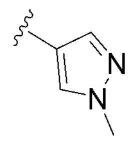 ;

$L_{m1}$ is absent, or is selected from the following groups which are unsubstituted or optionally substituted with one, two or more $R_{m1}$: $C_{6-14}$ aryl, 5-14-membered heteroaryl, or 3-14-membered heterocyclyl; each $R_{m1}$ is the same or different and each independently selected from H, halogen, cyano, $C_{1-6}$ alkyl, or HOOC-$C_{1-3}$ alkylene;
preferably, $L_{m1}$ is absent or is selected from the following groups which are unsubstituted or optionally substituted with one, two or more $R_{m1}$: phenyl, piperidinyl or piperazinyl;
more preferably, $L_{m1}$ is selected from

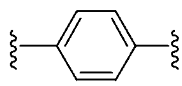, 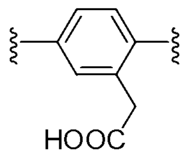, 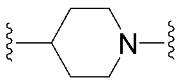

or

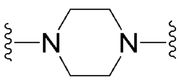;

$R_{m20}$ is selected from the following groups which are unsubstituted or optionally substituted with one, two, or more $R_{m2}$: $-(CH_2)_s-(C=O)-R_Z$, or -ethynyl-$(CH_2)_t-(C=O)-R_Z$;
preferably, $R_{m20}$ is selected from the following groups which are unsubstituted or optionally substituted with one, two, or more $R_{m2}$: $-CH_2-(C=O)-R_Z$, $-(CH_2)_2-(C=O)-R_Z$, $-(CH_2)_5-(C=O)-R_Z$, or -ethynyl-$(CH_2)_3-(C=O)-R_Z$;
each $R_{m2}$ is the same or different and each independently selected from H, halogen, cyano, $C_{1-6}$ alkyl or $-C_{1-6}$ alkylene-COOH, wherein the alkylene is optionally interrupted by one, two or more of the following groups: O, or NH;
preferably, each $R_{m2}$ is the same or different and each independently selected from $-C_{1-3}$ alkylene-COOH, and the alkylene is optionally interrupted by O or NH;
preferably, $R_{m2}$ is selected from

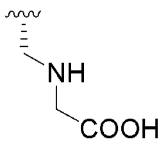, 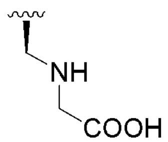, 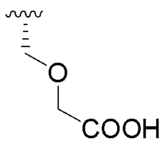, or 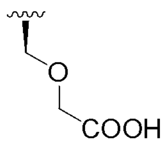;

r is an integer selected from 0-6;
s is an integer selected from 0-6;
t is an integer selected from 0-6;
Rz is selected from hydroxyl, halogen, an active ester, a carboxyl protecting group, an amino acid, a peptide fragment or a hydrophilic fragment;
the amino acid is the N-terminal amino acid of $L_1$, and the peptide fragment is a subfragment of $L_1$ formed from 2, 3 or 4 amino acids at the N-terminus or is $L_1$;
the C-terminus of the peptide fragment is hydroxyl, an active ester, a carboxyl protecting group

or 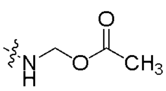;

preferably, the hydrophilic fragment comprises a polyhydroxy group, a polyethylene glycol fragment, a polybetaine fragment or a polycreatine fragment;
preferably, the formula MII is the following formula MII-1:

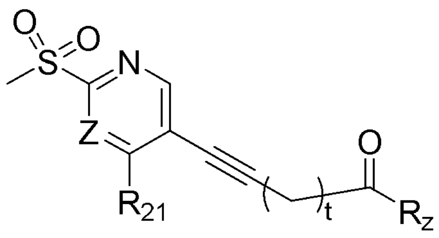

(MII-1)

wherein t and Rz are as defined above; Z is N or $CR_{22}$; $R_{21}$ and $R_{22}$ are each independently selected from H, halogen, cyano, oxo (=O), $C_{1-6}$ alkyl, halo-$C_{1-6}$ alkyl, hydroxy-$C_{1-6}$ alkyl, $C_{1-6}$ alkoxy, $C_{3-8}$ cycloalkyl, 3-8-membered heterocyclyl, or $C_{1-6}$ alkyl-O-$C_{1-6}$ alkyl; provided that when Z is N, $R_{21}$ is not H;
preferably, Rz is OH or halogen; t is an integer of 2-4; Z is N; and $R_{21}$ is selected from cyano, $C_{1-6}$ alkoxy, $C_{3-4}$ cycloalkyl or -$C_{1-6}$ alkyl-O-$C_{1-6}$ alkyl; or $R_{21}$ is H; Z is $CR_{22}$; and $R_{22}$ is cyano or trifluoromethyl;
preferably, the formula MII is the following compounds:

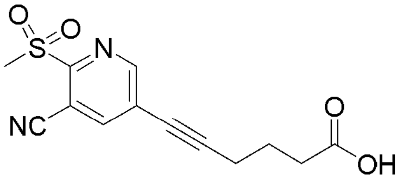
,
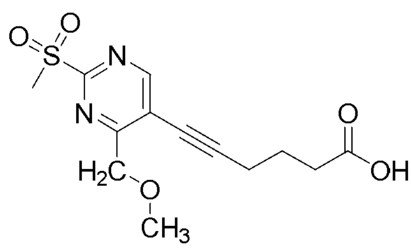
,

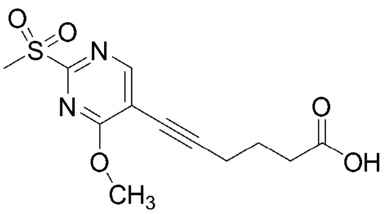
,
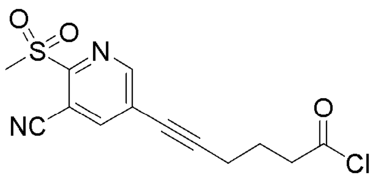
,
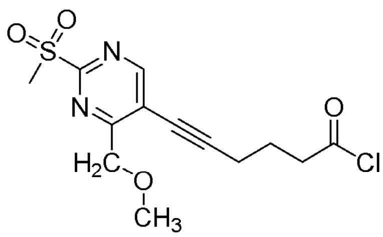
,

or

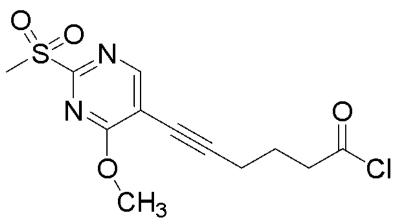
.

The present invention further provides a compound represented by formulas (L'-1) to (L'-3):

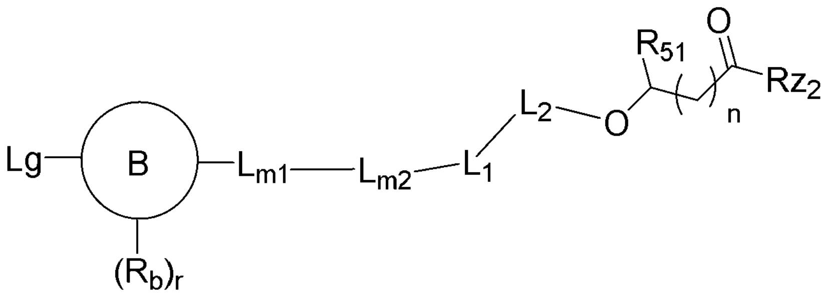

(L'-1)

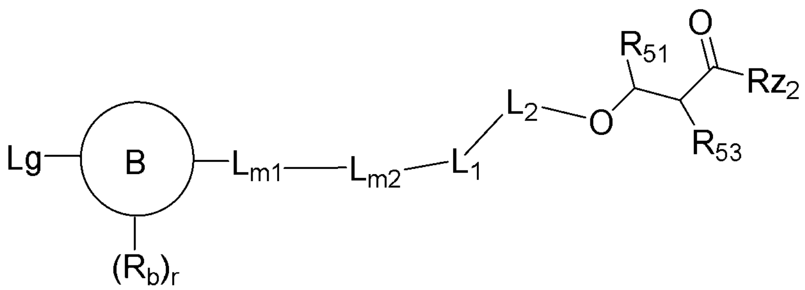

(L'-2)

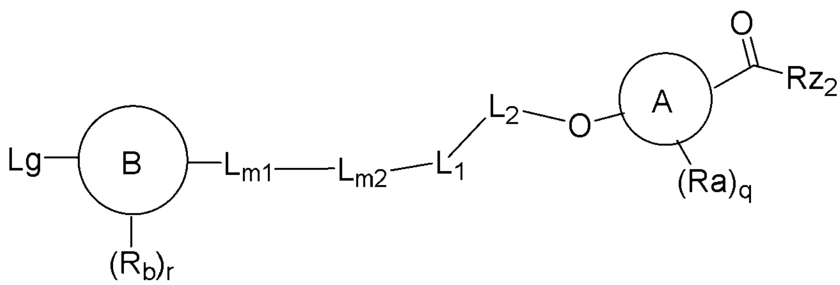

(L'-3)

wherein Lg is a leaving group, and the leaving group is selected from halogen, sulfone, trifluoromethanesulfonyl or methanesulfonyl; preferably, Lg is methanesulfonyl;

ring B is selected from a 5-6-membered N-containing heteroaromatic ring or a 3-6 membered N-containing heterocyclic ring;

more preferably, ring B is selected from a pyrimidine ring, a pyridine ring or a triazine ring;

each $R_b$ is the same or different and each independently selected from the following groups: halogen, cyano, oxo (=O), $C_{1-6}$ alkyl, halo-$C_{1-6}$ alkyl, hydroxy-$C_{1-6}$ alkyl, $C_{1-6}$ alkoxy or $C_{3-8}$ cycloalkyl, 3-8-membered heterocyclyl, $C_{1-6}$ alkyl-O-$C_{1-6}$ alkyl-, or $C_{1-6}$ alkyl-(5-6-membered) heteroaryl;

preferably, each $R_b$ is the same or different and each independently selected from cyano, oxo (=O), methoxy, cyclopropyl, trifluoromethyl,

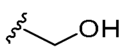 , 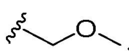 , 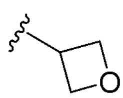 or 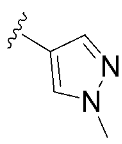 ;

$L_{m1}$ is absent, or is selected from the following groups which are unsubstituted or optionally substituted with one, two or

more $R_{m1}$: $C_{6-14}$ aryl, 5-14-membered heteroaryl, or 3-14-membered heterocyclyl; each $R_{m1}$ is the same or different and each independently selected from H, halogen, cyano, $C_{1-6}$ alkyl, or HOOC-$C_{1-3}$ alkylene;

preferably, $L_{m1}$ is absent or is selected from the following groups which are unsubstituted or optionally substituted with one, two or more $R_{m1}$: phenyl, piperidinyl or piperazinyl;

more preferably, $L_{m1}$ is selected from

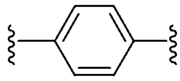,
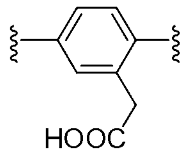,
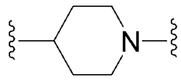

or

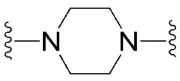;

$L_{m2}$ is selected from the following groups which are unsubstituted or optionally substituted with one, two, or more $R_{m2}$: $-(CH_2)_s$-(C=O)-, or -ethynyl-$(CH_2)_t$-(C=O)-;

preferably, $L_{m2}$ is selected from the following groups which are unsubstituted or optionally substituted with one, two, or more $R_{m2}$: $-CH_2$-(C=O)-, $-(CH_2)_2$-(C=O)-, $-(CH_2)_5$-(C=O)-, or - ethynyl-$(CH_2)_3$-(C=O)-;

each $R_{m2}$ is the same or different and each independently selected from H, halogen, cyano, $C_{1-6}$ alkyl or -$C_{1-6}$ alkylene-COOH, wherein the alkylene is optionally interrupted by one, two or more of the following groups: O, or NH;

preferably, each $R_{m2}$ is the same or different and each independently selected from -$C_{1-3}$ alkylene-COOH, and the alkylene is optionally interrupted by O or NH;

preferably, $R_{m2}$ is selected from

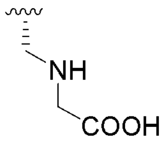,
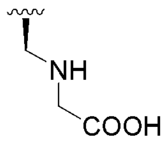,
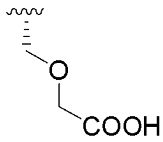, or
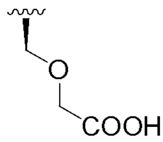;

r is an integer selected from 0-6;

s is an integer selected from 0-6;

t is an integer selected from 0-6;

$Rz_2$ is selected from hydroxyl, halogen, an active ester or a carboxyl protecting group;

preferably, wherein the

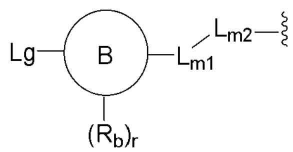

fragment has the structure represented by the following formula:

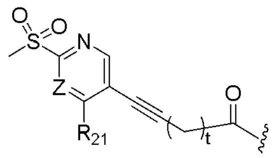

wherein the groups are as defined herein;

$R_{51}$ is independently selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl; $R_{53}$ is selected from $C_{1-6}$ alkyl, $C_{1-6}$ haloalkyl, $C_{1-6}$ alkyl-NH-, ($C_{1-6}$

alkyl)$_2$N-, $C_{1-6}$ alkyl-NH-$C_{1-6}$ alkyl, ($C_{1-6}$ alkyl)$_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl; ring A is selected from $C_{3-8}$ cycloalkyl or 3-8-membered heterocyclyl; Ra is selected from H, hydroxyl, CN, halogen, $C_{1-6}$ alkyl, or $C_{1-6}$ haloalkyl; n is selected from 0, 1, or 2; and q is selected from 0, 1, or 2;

$L_1$ is a peptide residue; preferably, it is selected from glycine-glycine-phenylalanine-glycine (GGFG), glutamic acid-valine-citrulline (EVC), valine-citrulline (VC), aspartic acid-valine-citrulline (DVC), glutamic acid-glycine-glycine-phenylalanine-glycine (EGGFG), or aspartic acid-glycine-glycine-phenylalanine-glycine (DGGFG); preferably, $L_1$ is selected from glycine-glycine-phenylalanine-glycine (GGFG);

$L_2$ is a linking group between the peptide residue and D; preferably, it is selected from a chemical bond, -NH-$C_{1-6}$ alkyl-,

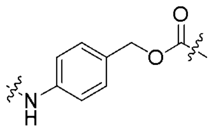
or
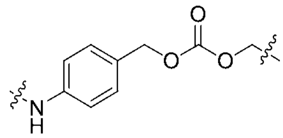
;

preferably, $L_2$ is selected from -NH-$CH_2$-; and

(L'-1) to (L'-3) are preferably:

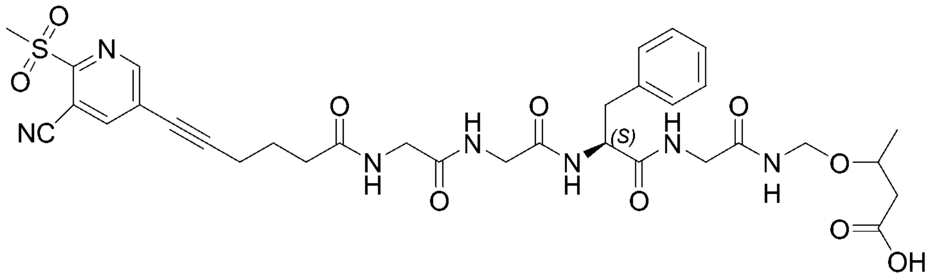

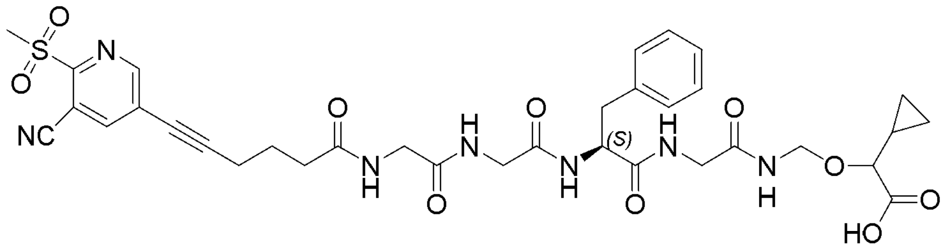

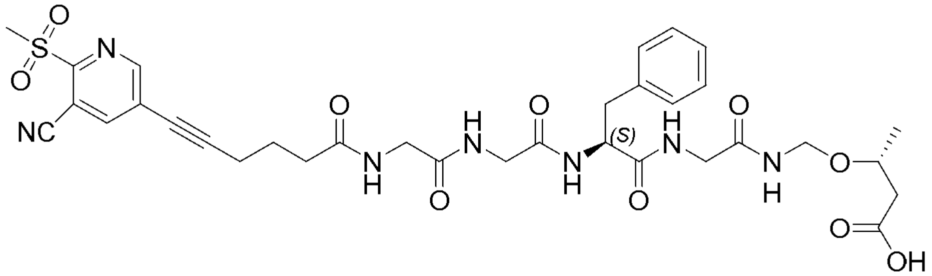

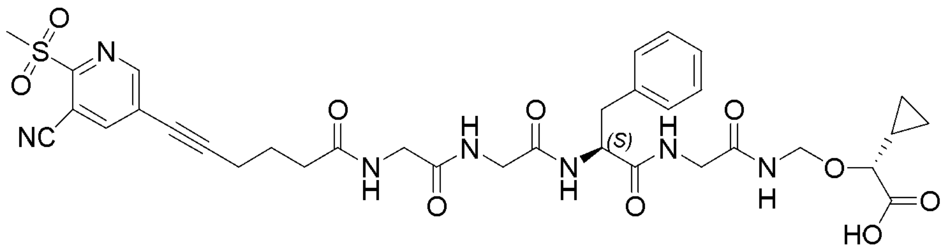

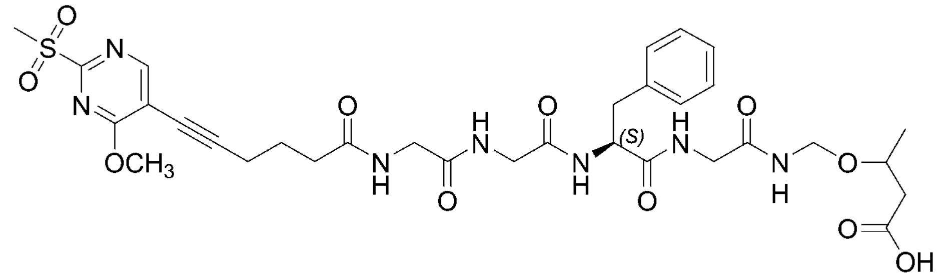

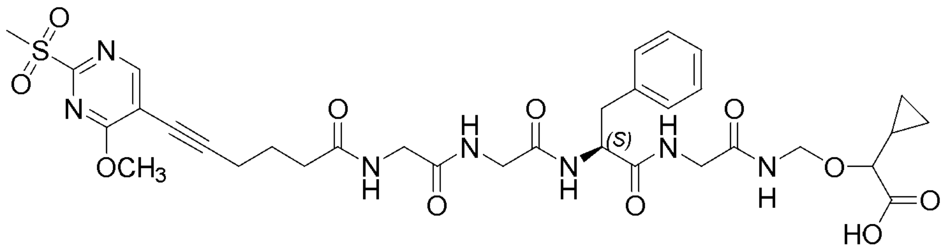

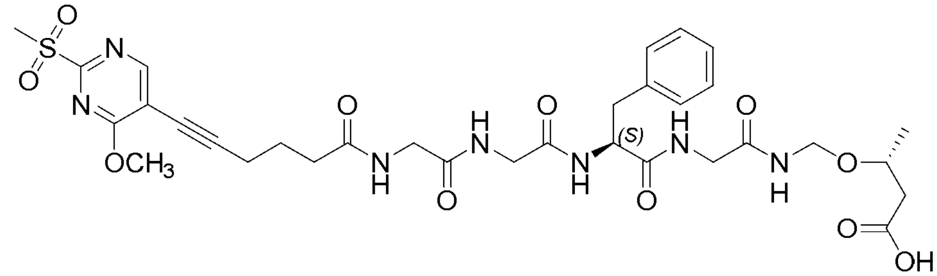

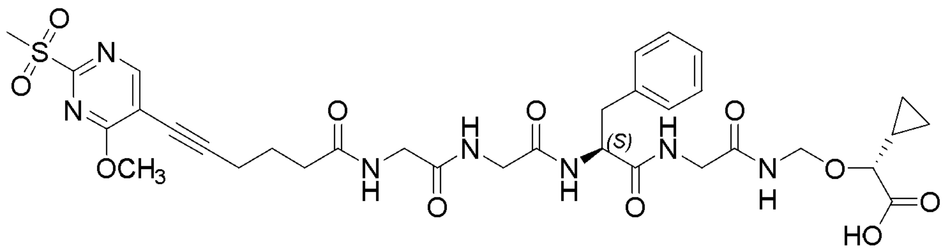

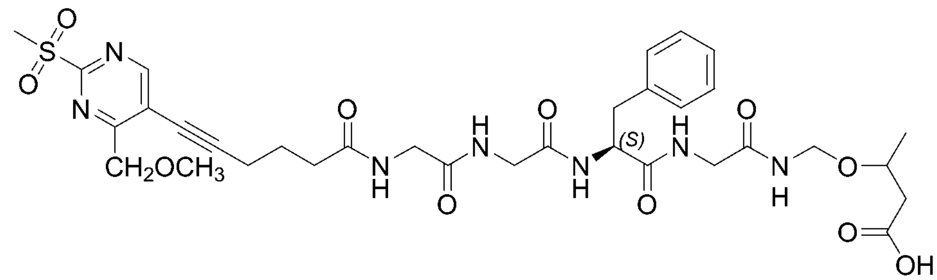

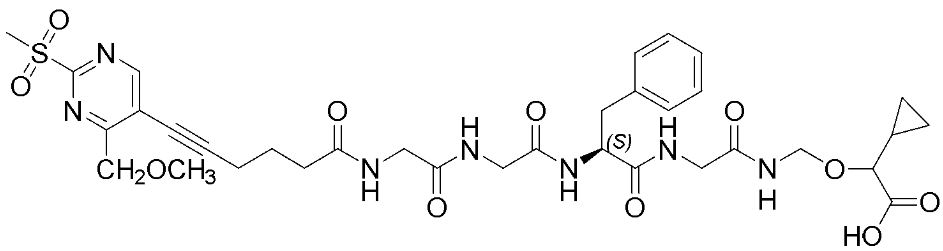

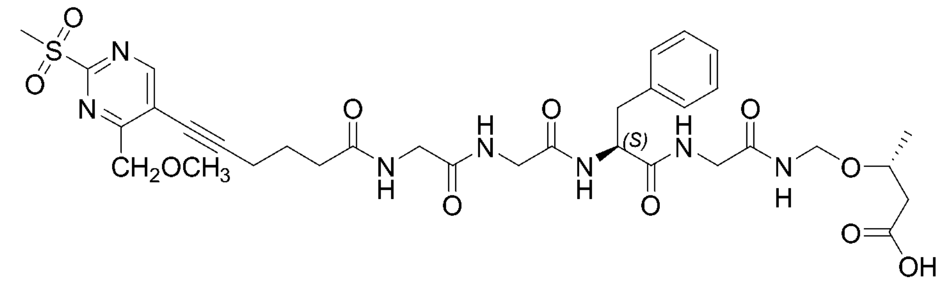

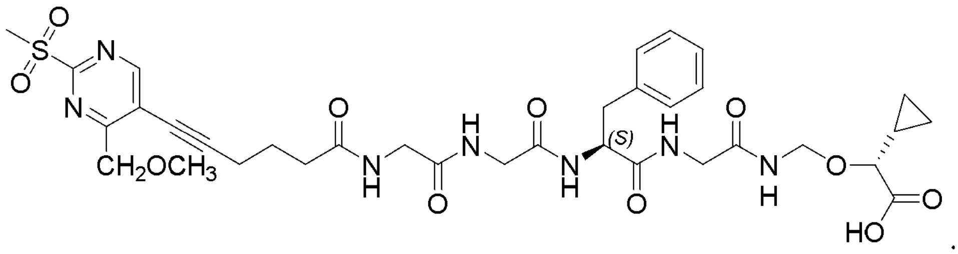

.

The present invention further provides a synthesis method of formula (L'), wherein the $L_2$ is - NH-$CH_{2}$-, comprising the following steps:

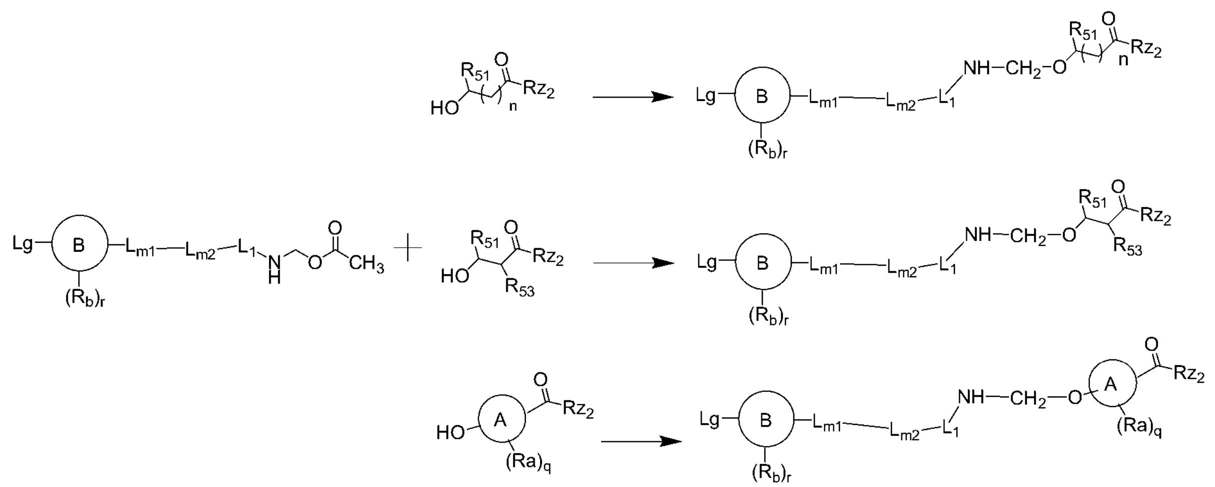

or

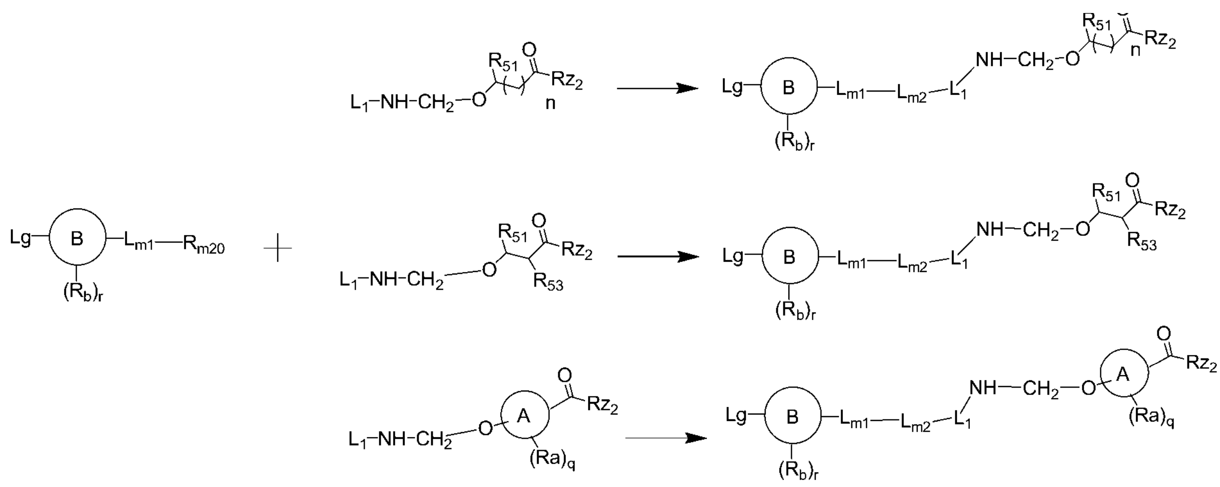

wherein $Rz_2$ is hydroxyl, an active ester or a carboxyl protecting group, and the remaining groups are as defined herein.

The present invention further provides a synthesis method of a conjugated intermediate compound, which comprises a first scheme or a second scheme:

the first scheme is selected from the following steps:

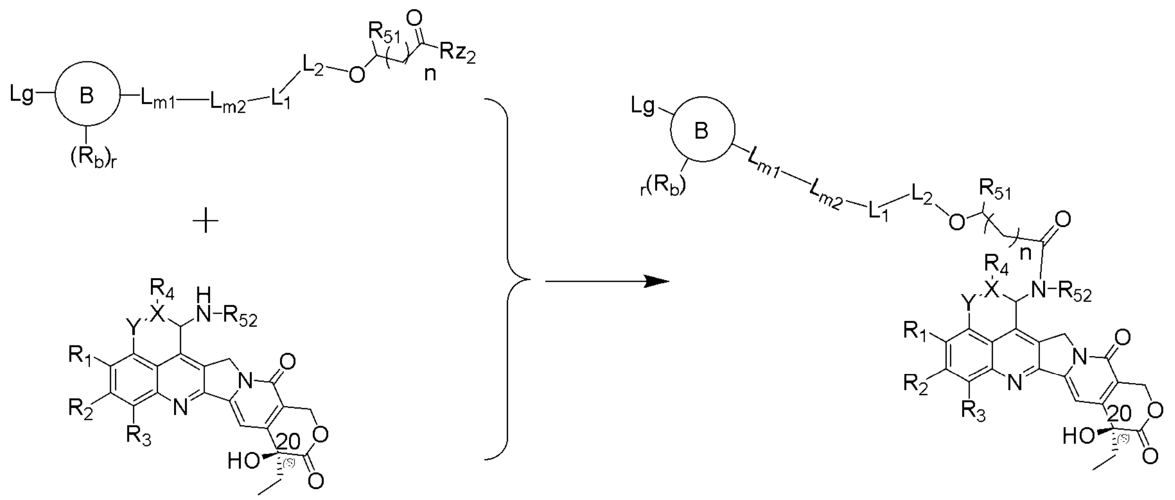

or

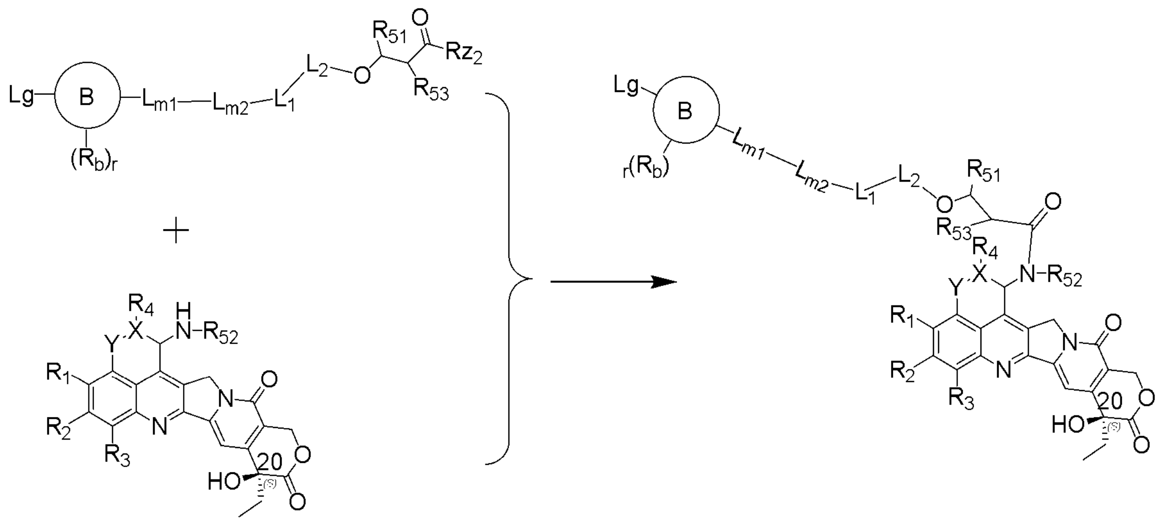

or

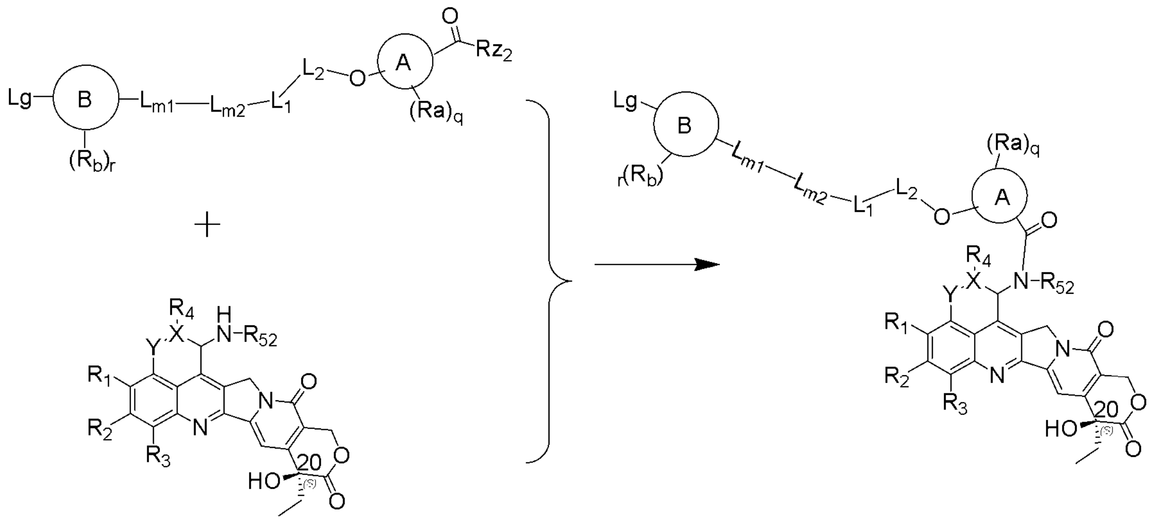

wherein $Rz_2$ is selected from hydroxyl, halogen, an active ester or a carboxyl protecting group, and the remaining groups are as defined herein;

the second scheme comprises the following steps:

allowing

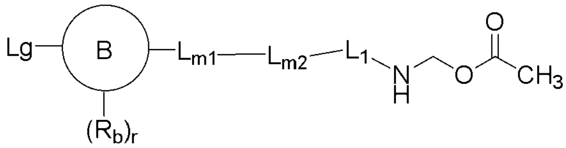

to react with the compound of formula I as described above (wherein $R_5$ is not H).

**Beneficial Effects**

The camptothecin compound provided in the present invention has good tumor inhibiting effects, can be used for treating or preventing cancer (for example, breast cancer or gastric cancer), and can be used for preparing a medicament for treating or preventing such a condition or disease. The cytotoxic drug-linker compound provided by the present invention can be smoothly conjugated with an antibody to give an antibody-drug conjugate. The antibody-drug conjugate provided by the present invention has good tumor inhibition effect and selectivity.

**Definition and Explanation of Terms**

Unless otherwise indicated, the definitions of groups and terms recorded in the specification and claims of the present application, including their definitions as examples, exemplary definitions, preferred definitions, definitions recorded in tables, definitions of specific compounds in examples, etc., may be arbitrarily combined and associated with each other. Such combined and associated group definitions and compound structures should be understood to be within the scope of the specification and/or claims of the present application.

The "linker", "linker structure" or "connector" or "linking unit" mentioned in the present invention refers to a chemical structure fragment or bond that is connected to an antibody at one end and to a drug (pharmaceutical compound) at the other end, and can also be connected to other linkers before being connected to the pharmaceutical compound. The linker structure of the present invention can be synthesized by methods known in the art, or by the method described in the present invention.

The "antibody-drug conjugate" (ADC ) mentioned in the present invention refers to a targeting portion connected to a biologically active drug via a stable linking unit.

The "biologically active molecules" mentioned in the present invention refer to cytotoxic drugs, which are chemical molecules that can strongly destroy the normal growth of tumor cells.

Unless otherwise indicated, the numerical ranges recited in the present specification and claims are equivalent to

at least reciting each specific integer value therein. For example, the numerical range "1-12" is equivalent to reciting each integer value in the numerical range "1-12," namely 1, 2, 3, 4, 5, 6, 7, 8, 9, 10, 11, and 12. In addition, when a numerical range is defined as a "number," it should be understood that both endpoints of the range, each integer within the range, and each decimal within the range are recited.

The term "integer of 0-6" means 0, 1, 2, 3, 4, 5, 6.

The term "halogen" represents fluorine, chlorine, bromine, and iodine.

The "$C_{1-10}$ alkyl" represents a linear and branched alkyl having 1, 2, 3, 4, 5, 6, 7, 8, 9, or 10 carbon atoms, the "$C_{1-8}$ alkyl" represents a linear and branched alkyl having 1, 2, 3, 4, 5, 6, 7, or 8 carbon atoms, and the "$C_{1-6}$ alkyl" represents a linear and branched alkyl having 1, 2, 3, 4, 5, or 6 carbon atoms. The alkyl is, e.g., methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, etc., or isomers thereof.

The "$C_{2-10}$ alkenyl" should be construed to represent preferably a linear or branched monovalent hydrocarbon radical comprising one or more double bonds and having 2, 3, 4, 5, 6, 7, 8, 9, or 10 carbon atoms, and is more preferably "$C_{2-8}$ alkenyl". The "$C_{2-10}$ alkenyl" should be construed to represent preferably a linear or branched monovalent hydrocarbon radical comprising one or more double bonds and having 2, 3, 4, 5, 6, 7, or 8 carbon atoms, for example, having 2, 3, 4, 5, or 6 carbon atoms (namely, $C_{2-6}$ alkenyl) or having 2 or 3 carbon atoms (namely, $C_{2-3}$ alkenyl). It should be understood that in the case where the alkenyl comprises more than one double bond, the double bonds may be separated from each other or conjugated. The alkenyl is, e.g., vinyl, allyl, (E)-2-methylvinyl, (Z)-2-methylvinyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propyvinyl, or 1-isopropylvinyl.

The "$C_{2-10}$ alkynyl" should be construed to represent preferably a linear or branched monovalent hydrocarbon radical comprising one or more triple bonds and having 2, 3, 4, 5, 6, 7, 8, 9, or 10 carbon atoms, for example, having 2, 3, 4, 5, 6, 7, or 8 carbon atoms (namely, "$C_{2-8}$ alkynyl"), having 2, 3, 4, 5, or 6 carbon atoms (namely, "$C_{2-6}$ alkynyl"), or having 2 or 3 carbon atoms (namely, "$C_{2-3}$ alkynyl"). The alkynyl is, e.g., ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl. In particular, the alkynyl is ethynyl, prop-1-ynyl, or prop-2-ynyl.

The term "$C_{3-10}$ cycloalkyl" should be construed to represent saturated monovalent monocyclic, bicyclic (e.g., bridged cyclic or spirocyclic) hydrocarbon ring or tricyclic alkane having 3, 4, 5, 6, 7, 8, 9, or 10 carbon atoms. The $C_{3-10}$ cycloalkyl may be a monocyclic hydrocarbon radical such as cyclopropyl, cyclobutyl, cyclopentyl, cyclohexyl, cycloheptyl, cyclooctyl, cyclononyl, or cyclodecyl, or may be a bicyclic hydrocarbon radical such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonyl, or 2,6-diazaspiro [3,4]octyl, or may be a tricyclic hydrocarbon radical such as adamantyl.

Unless otherwise defined, the term "3-6-membered heterocyclyl" refers to a saturated or unsaturated non-aromatic ring or ring system, for example, it is a 4-, 5-, or 6-membered monocyclic ring, and contains at least one, for example, 1, 2, 3, 4, 5, or more heteroatoms selected from O, S, and N, wherein N and S may further be optionally oxidized to various oxidation states to form nitrogen oxides, -S(O)-, or $-S(O)_2-$ states. The heterocyclyl may comprise a fused or bridged ring and a spirocyclic ring. In particular, the heterocyclyl may include, but is not limited to: a 4-membered ring, such as azetidinyl or oxetanyl; a 5-membered ring, such as tetrahydrofuryl, dioxolyl, pyrrolidyl, imidazolidinyl, pyrazolidyl, or pyrrolinyl; or a 6-membered ring, such as tetrahydropyranyl, piperidyl, morpholinyl, dithianyl, thiomorpholinyl, piperazinyl, or trithianyl.

The term "$C_{6-14}$ aryl" should be construed to represent preferably a monovalent aromatic or partially aromatic monocyclic, bicyclic, or tricyclic hydrocarbon ring having 6, 7, 8, 9, 10, 11, 12, 13, or 14 carbon atoms ("$C_{6-14}$ aryl"), particularly a ring having 6 carbon atoms ("$C_6$ aryl"), such as phenyl; or biphenyl, or a ring having 9 carbon atoms ("$C_9$ aryl"), such as indanyl or indenyl, or a ring having 10 carbon atoms ("$C_{10}$ aryl"), such as tetralyl, dihydronaphthyl, or naphthyl, or a ring having 13 carbon atoms ("$C_{13}$ aryl"), such as fluorenyl, or a ring having 14 carbon atoms ("$C_{14}$ aryl"), such as anthranyl.

When the $C_{6-20}$ aryl is substituted, it can be monosubstituted or polysubstituted. Further, the substitution site is not limited, for example, may be ortho-, para-, or meta-substitution.

The term "5-14-membered heteroaryl" should be construed to comprise such a monovalent monocyclic, bicyclic, or tricyclic aromatic ring system that has 5, 6, 7, 8, 9, 10, 11, 12, 13, or 14 ring atoms, particularly 5 or 6 or 9 or 10 carbon atoms, and comprises from 1 to 5, preferably from 1 to 3, heteroatoms each independently selected from N, O, and S, and, further, may be additionally benzo-fused in each case. "Heteroaryl" also means a group in which a heteroaromatic ring is fused with one or more aryl, alicyclic, or heterocyclic rings, wherein the radical or site of attachment is on the heteroaromatic ring. Non-limiting examples include 1-, 2-, 3-, 5-, 6-, 7- or 8-indolizinyl, 1-, 3-, 4-, 5-, 6-, or 7-isoindolyl, 2-, 3-, 4-, 5-, 6- or 7-indolyl, 2-, 3-, 4-, 5-, 6-, or 7-indazolyl, 2-, 4-, 5-, 6-, 7- or 8-purinyl, 1-, 2-, 3-, 4-, 6-, 7-, 8- or 9-quinolizinyl, 2-, 3- , 4-, 5-, 6-, 7- or 8-quinolinyl, 1-, 3-, 4-, 5-, 6-, 7- or 8-isoquinolinyl, 1-, 4-, 5-, 6-, 7- or 8-phthalazinyl, 2-, 3-, 4-, 5- or 6-naphthyridinyl, 2-, 3-, 5-, 6-, 7- or 8-quinazolinyl, 3-, 4-, 5-, 6-, 7- or 8-cinnolinyl, 2-, 4-, 6- or 7-pteridinyl, 1-, 2-, 3-, 4-, 5-, 6-, 7- or 8-4aH carbazolyl, 1-, 2-, 3-, 4-, 5-, 6-, 7- or 8-carbazolyl, 1-, 3-, 4-, 5-, 6-, 7-, 8- or 9-carbolinyl, 1-, 2-, 3-, 4-, 6-, 7-, 8-, 9- or 10-phenanthridinyl, 1-, 2-, 3-, 4-, 5-, 6-, 7-, 8- or 9-acridinyl, 1-, 2-, 4-, 5-, 6-, 7-, 8- or 9-pyridyl, 2-, 3-, 4-, 5-, 6-, 8-, 9- or 10-phenanthrolinyl, 1-, 2-, 3-, 4-, 6-, 7-, 8- or 9-phenazinyl, 1-, 2-, 3-, 4-, 6-, 7-, 8-, 9- or 10-phenothiazinyl, 1-, 2-, 3-, 4-, 6-, 7-, 8-, 9- or 10-phenazinyl , 2-, 3-, 4-, 5-, 6- or 1-, 3-, 4-, 5-, 6-, 7-, 8-, 9- or 10-benzoisoquinolyl, 2-, 3-, 4- or thieno[2,3-b]furanyl, 2-, 3-, 5-, 6-, 7-, 8-, 9-, 10- or 11-7H-pyrazino[2,3-c]carbazolyl, 2-, 3-, 5-, 6- or 7-2H-furo[3,2-b]pyranyl , 2-, 3-, 4-, 5-, 7-, or 8-5H-pyrido[2,3-d]-o-oxazinyl, 1-, 3-, or 5-1H-pyrazolo[4,3-d]-oxazolyl, 2-, 4-, or 54H-imidazo[4,5-d]thiazolyl, 3-, 5-, or 8-pyrazino[2,3-d]pyridazinyl, 2-, 3-, 5-, or 6-imidazo[2,1-b]thiazolyl, 1-, 3-, 6-, 7-, 8-, or 9-furo[3,4-c]cinnolinyl, 1-, 2-, 3-, 4-, 5-, 6-, 8-, 9-, 10 or 11-4H-pyrido[2,3-c]carbazolyl, 2-, 3-, 6- or 7-imidazo[1,2-b][1,2,4]triazinyl, 7-benzo[b]thienyl, 2-, 4-, 5-, 6- or 7-benzoxazolyl, 2-, 4-, 5-, 6- or 7-benzimidazolyl, 2-, 4- , 4-, 5-, 6- or 7-benzothiazolyl, 1-, 2-, 4-, 5-, 6-, 7-, 8- or 9-benzoxapinyl, 2-, 4-, 5-, 6-, 7- or 8-benzoxazinyl, 1-, 2-, 3-, 5-, 6-, 7-, 8-, 9-, 10- or 11-4H-pyrrolo[1,2-b][2]benzazapinyl. A typical fused heteroaryl includes, but is not limited to, 2-, 3-, 4-, 5-, 6-, 7-, or 8-quinolyl, 1-, 3-, 4-, 5-, 6-, 7-, or 8-isoquinolyl, 2-, 3-, 4-, 5-, 6-, or 7-indolyl, 2-, 3-, 4-, 5-, 6-, or 7-benzo[b]thienyl, 2-, 4-, 5-, 6-, or 7-benzoxazolyl, 2-, 4-, 5-, 6-, or 7-benzimidazolyl, and 2-, 4-, 5-, 6-, or 7-benzothiazolyl. The 5-20-membered heteroaryl may be linked to other groups to form the compound of the present invention by linking a carbon atom on a 5-20-membered heteroaryl ring to the other groups, or by linking a heteroatom on a 5-20-membered heteroaryl ring to the other groups. When the 5-20-membered heteroaryl is substituted, it may be monosubstituted or polysubstituted. Further, the substitution site is not limited, for example, hydrogen attached to a carbon atom on the heteroaryl ring may be substituted, or hydrogen attached to a heteroatom on the heteroaryl ring may be substituted.

The term "spirocyclic ring" refers to a ring system in which two rings share 1 ring-forming atom.

The term "fused ring" refers to a ring system in which two rings share 2 ring atoms.

The term "bridged ring" refers to a ring system in which two rings share more than 3 ring-forming atoms.

Wavy lines (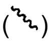) intersecting with chemical bonds are used to indicate positions where a group is linked to other atoms or groups in the general structure.

Unless otherwise specified, the heterocyclyl, heteroaryl, or heteroarylene comprises all possible isomeric forms thereof, such as positional isomers thereof. Therefore, for some illustrative non-limiting examples, the substitution or bonding to other radicals may be comprised at, e.g., 1, 2, or more of its 1-, 2-, 3-, 4-, 5-, 6-, 7-, 8-, 9-, 10-, 11-, 12-C, (if present), comprising pyridin-2-yl, pyridyliden-2-yl, pyridin-3-yl, pyridyliden-3-yl, pyridin-4-yl, and pyridyliden-4-yl; and the thienyl or thienylenyl comprises thien-2-yl, thienylen-2-yl, thien-3-yl, and thienylen-3-yl; pyrazol-1-yl, pyrazol-3-yl, pyrazol-4-yl, and pyrazol-5-yl.

The term "alkoxy" refers to -O-(alkyl), wherein the alkyl is as defined above. Non-limiting examples of alkoxy comprise: methoxy, ethoxy, propoxy, and butoxy. The alkoxy may be optionally substituted or unsubstituted, and may be, when substituted, substituted with a substituent which is preferably one or more groups independently selected from alkyl, alkenyl, alkynyl, alkoxy, alkylamino, halogen, sulfydryl, hydroxyl, nitro, cyano, cycloalkyl, heterocycloalkyl, aryl, heteroaryl, cycloalkoxy, or heterocycloalkoxy.

The term "alkylamino" refers to -NH-(alkyl), wherein the alkyl is as defined above. Non-limiting examples of alkylamino comprise, for example, methylamino, ethylamino, propylamino, isopropylamino, and butylamino.

The term "$(alkyl)_2$amino" refers to -N-$(alkyl)_2$, wherein the alkyl is as defined above. Non-limiting examples of $(alkyl)_2$ amino comprise: for example, dimethylamino, methylethylamino, diethylamino, dipropylamino, methylpropylamino, diisopropylamino, and dibutylamino.

The "haloalkyl" refers to alkyl substituted with one or more halogens, wherein the alkyl is as defined above.

The term "antibody" refers to an immunoglobulin-derived molecule capable of specifically binding to a target antigen, and the immunoglobulin-derived molecule binds to the target antigen through at least one antigen-binding site located in a variable region thereof. When referring to the term "antibody", unless otherwise specified in the context, it includes not only an intact antibody but also an antigen-binding fragment capable of specifically binding to a target antigen. An "intact antibody" typically consists of two pairs of polypeptide chains, each pair having one light chain (LC) and one heavy chain (HC). Light chains of the antibody can be classified as kappa (κ) and lambda (λ) light chains. Heavy chains can

be classified as μ, δ, γ, α or ε, and the isotype of the antibody is respectively defined as IgM, IgD, IgG, IgA, and IgE. Within light and heavy chains, the variable and constant regions are joined by a "J" region of about 12 or more amino acids, and the heavy chain also comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. A constant domain is not directly involved in binding of an antibody to an antigen, but exhibits various effector functions, such as mediating the binding of immunoglobulin to host tissues or factors, including binding of various cells (e.g. effector cells) of the immune system and the first component (C1q) of the classical complement system. VH and VL regions can be further subdivided into regions with high variability (called complementarity determining regions (CDRs)) interspersed with relatively conserved regions called framework regions (FRs). Each of the VH and VL consists of 3 CDRs and 4 FRs arranged from an amino terminal to a carboxyl terminal in the following order: FR1, CDR1, FR2, CDR2, FR3, CDR3, and FR4. The variable regions (VH and VL) of each heavy chain/light chain pair respectively form an antigen-binding site. The distribution of amino acids in each region or domain can be based on the definitions in Kabat, Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883. As used herein, the term "complementarity determining region" or "CDR" refers to amino acid residues responsible for antigen binding in a variable region of an antibody. Each of the variable regions of the heavy and light chains comprises three CDRs, named CDR1, CDR2, and CDR3. The precise boundaries of these CDRs can be defined according to various numbering systems known in the art, such as the definitions in the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given antibody, a person of skill in the art will readily identify the CDRs defined by each numbering system. Furthermore, the corresponding relationship between different numbering systems is well known to a person of skill in the art (for example, see Lefranc et al., Dev. Comparat. Immunol. 27:55-77, 2003). In the present invention, the CDR comprised in the antibody or the antigen-binding fragment thereof of the present invention can be determined according to various numbering systems known in the art. In some embodiments, the CDR comprised in the antibody or antigen-binding fragment thereof of the present invention is preferably determined by the Kabat numbering system. As used herein, the term "framework region" or "FR" residues refer to those amino acid residues in variable regions of an antibody other than the CDR residues as defined above. The term "antibody" is not limited to any particular method of producing the antibody. For example, antibodies include recombinant antibodies, monoclonal antibodies, and polyclonal antibodies. Antibodies can be of different isotypes, for example, IgG (e.g., IgG1, IgG2, IgG3, or IgG4 subtype), IgA1, IgA2, IgD, IgE, or IgM antibodies. As used herein, the term "antigen-binding fragment" of an antibody refers to a polypeptide comprising a fragment of a full-length antibody that retains the ability to specifically bind to the same antigen that the full-length antibody binds to and/or competes with the full-length antibody for specific binding to the antigen, which is also referred to as an "antigen-binding portion". Generally, see Fundamental Immunology, Ch. 7 (Paul, W., ed., 2nd ed., Raven Press, N.Y. (1989), which is incorporated herein by reference in its entirety for all purposes. The antigen-binding fragment of an antibody can be produced by recombinant DNA technologies or by enzymatic or chemical cleavage of an intact antibody. Non-limiting examples of the antigen-binding fragment include Fab, Fab', F(ab')2, Fd, Fv, disulfide-linked Fv, scFv, di-scFv, a diabody, a single-domain antibody, and a polypeptide comprising at least a portion of an antibody sufficient to confer a specific antigen-binding ability to the polypeptide. Engineered antibody variants are reviewed in Holliger et al., 2005; Nat Biotechnol, 23: 1126-1136. As used herein, the term "Fd" means an antibody fragment consisting of VH and CH1 domains; the term "Fab fragment" means an antibody fragment consisting of VL, VH, CL, and CH1 domains; the term "F(ab')2 fragment" means an antibody fragment comprising two Fab fragments linked by a disulfide bridge on a hinge region; and the term "Fab' fragment" means a fragment obtained after reducing disulfide bonds linking two heavy chain fragments in the F(ab')2 fragment, consisting of a complete light chain and the Fd fragment (consisting of VH and CH1 domains) of a heavy chain. As used herein, the term "Fv" means an antibody fragment consisting of the VL and VH domains of a single arm of an antibody. The Fv fragment is generally considered to be the smallest antibody fragment that capable of forming a complete antigen-binding site. It is generally believed that six CDRs confer an antigen-binding specificity to an antibody. However, a single variable region (e.g., the Fd fragment, which comprises only three CDRs specific for an antigen) also has the ability to recognize and bind to an antigen, although the affinity thereof may be lower than that of a complete binding site. As used herein, the term "Fc" means an antibody fragment formed by the second and third constant regions of a first heavy chain of an antibody binding to the second and third constant regions of a second heavy chain of the antibody through disulfide bonds. The Fc fragment of an antibody has a variety of different functions but is not involved in antigen binding. The "effector functions" mediated by the Fc domain comprise Fc receptor binding; Clq binding and complement-dependent cytotoxicity (CDC); antibody-dependent cell-mediated cytotoxicity (ADCC); phagocytosis; downregulation of cell surface receptors (e.g., B cell receptor); and B cell activation, or the like. The Fc domain may comprise both a native Fc region and a variant Fc region. A native Fc region comprises an amino acid sequence that is consistent with the amino acid

sequence of an Fc region found in nature, for example, a native sequence human Fc region comprises a native sequence human IgG1 Fc region; a native sequence human IgG2 Fc region; a native sequence human IgG3 Fc region; and a native sequence human IgG4 Fc region, as well as naturally occurring variants thereof. A variant Fc region comprises an amino acid sequence which differs from the amino acid sequence of a native sequence Fc region by at least one amino acid modification. In some embodiments, a variant Fc region may possess altered effector functions (e.g., Fc receptor binding, antibody glycosylation, number of cysteine residues, effector cell function, or complement function) compared to a native Fc region. As used herein, the term "scFv" refers to a single polypeptide chain comprising VL and VH domains, wherein the VL and VH are linked by a linker. Such scFv molecules may have a general structure: NH2-VL-Linker-VH-COOH or NH2-VH-Linker-VL-COOH. A suitable linker in the prior art consists of repeated GGGGS amino acid sequences or variants thereof. For example, a linker having an amino acid sequence (GGGGS)4 may be used, but a variant thereof may also be used. In some cases, a disulfide bond may also exist between the VH and VL of the scFv. In some embodiments of the present invention, the scFv may form di-scFv, which refers to two or more single scFvs in series to form an antibody. In some embodiments of the present invention, the scFv may form (scFv)2, which refers to two or more single scFvs in parallel to form an antibody. As used herein, the term "diabody" means that the VH and VL domains thereof are expressed on a single polypeptide chain, but it uses a linker that is too short to allow pairing between two domains of the same chain, thereby forcing the domains to pair with the complementary domains of another chain and forming two antigen-binding sites (see, e.g. Holliger P. et al., Proc. Natl. Acad. Sci. USA 90:6444-6448 (1993), and Poljak R.J. et al., Structure 2:1121-1123 (1994)). As used herein, the term "single-domain antibody (sdAb)" has the meaning commonly understood by a person of skill in the art, and refers to an antibody fragment consisting of a single monomeric variable antibody domain (e.g. single heavy chain variable region), which retains the ability to specifically bind to the same antigen as the full-length antibody. As used herein, the term "bispecific antibody" refers to an antibody having a binding specificity for two different antigens (or epitopes). The term "multispecific antibody" refers to an antibody having a binding specificity for at least more than two (e.g. three or four) different antigens (or epitopes). The bispecific antibody or the multispecific antibody comprises multiple antigen-binding domains having a binding specificity for different antigens (or epitopes), and thus is able to bind to at least two different binding sites and/or target molecules. Each antigen-binding domain comprised in the bispecific antibody or the multispecific antibody can be independently selected from a full-length antibody (e.g. IgG antibody) or an antigen-binding fragment thereof (e.g. Fv fragment, Fab fragment, F(ab')2 fragment, or scFv). In some cases, individual antigen-binding domains are linked by peptide linkers. Each of the above antibody fragments retains the ability to specifically bind to the same antigen as the full-length antibody, and/or competes with the full-length antibody for specific binding to the antigen. An antigen-binding fragment of an antibody (e.g., the above-mentioned antibody fragment) can be obtained from a given antibody (e.g., antibody provided in the present invention) by a conventional technology known to those skilled in the art (e.g., recombinant DNA technology or enzymatic or chemical cleavage method), and the antigen-binding fragment of the antibody can be screened for specificity in the same manner as for an intact antibody. As used herein, the term "humanized antibody" refers to a non-human antibody that is genetically engineered, and the amino acid sequence thereof is modified to increase the homology with the sequence of a human antibody. Generally speaking, all or part of CDR regions of a humanized antibody are derived from a non-human antibody (donor antibody), and all or part of non-CDR regions (e.g. variable region FR and/or constant region) are derived from a human immunoglobulin (receptor antibody). In some embodiments, CDR regions of a humanized antibody are derived from a non-human antibody (donor antibody), and all or part of non-CDR regions (e.g. variable region FR and/or constant region) are derived from a human immunoglobulin (receptor antibody). A humanized antibody generally retains the desired properties of a donor antibody, including but not limited to, antigen specificity, affinity, reactivity, etc. In the present application, the donor antibody may be a murine antibody having desired properties (e.g. antigen specificity, affinity, reactivity). To prepare a humanized antibody, CDR regions of a donor antibody can be inserted into human framework sequences by a method known in the art. In some cases, the human framework sequence may comprise amino acid mutations that are substituted with corresponding non-human residues. In addition, a humanized antibody may also comprise residues that are not found in a variable region (e.g. light chain variable region or heavy chain variable region) of an initial donor antibody or in a human framework sequence to further improve or optimize the performance of the humanized antibody. As used herein, the term "chimeric antibody" refers to an antibody in which a portion of the light chain and/or the heavy chain thereof is derived from one antibody (which may be derived from a particular species or belong to a particular antibody type or subtype), and another portion of the light chain and/or the heavy chain is derived from another antibody (which may be derived from the same or different species or belong to the same or different antibody types or subtypes), but in any case, the binding activity to a target antigen is still retained. In some embodiments, the term "chimeric antibody" may include an antibody in which the heavy chain variable region and light chain variable region of the antibody are derived from a first antibody, and the heavy chain constant region and light chain constant region of the antibody are derived from a second antibody.

Those skilled in the art will appreciate that the compound represented by formula (I) may exist in the form of various pharmaceutically acceptable salts. If the compounds have basic centers, they can form acid addition salts; if they have acidic centers, they can form base addition salts; if the compounds contain both acidic centers (e.g. carboxyl) and basic centers (e.g. amino), they can also form inner salts.

The compounds of the present invention may be present in the form of solvates, such as hydrates, wherein the compounds of the present invention comprise polar solvents, such as water, methanol or ethanol, as structural elements of the lattice of the compounds. The polar solvent, especially water, may be present in a stoichiometric or non-stoichiometric amount.

Depending on their molecular structure, the compounds of the present invention may be chiral and may therefore exist in various enantiomeric forms. These compounds may therefore exist in racemic or optically active forms. The compounds of the present invention encompass isomers in which the chiral carbons are each in R or S configuration, or mixtures and racemates thereof. The compounds of the present invention or their intermediates can be separated into enantiomeric compounds by chemical or physical methods known to those skilled in the art, or used in synthesis in this form. In the case of racemic amines, diastereomers are prepared from the mixture by reaction with an optically active resolving reagent. Examples of suitable resolving reagents are optically active acids, such as the R and S forms of tartaric acid, diacetyltartaric acid, dibenzoyltartaric acid, mandelic acid, malic acid, lactic acid, appropriate N-protected amino acids (e.g. N-benzoylproline or N-phenylsulfonylproline) or various optically active camphorsulfonic acids. Chromatographic enantiomeric resolution can also be advantageously carried out by means of optically active resolving reagents, such as dinitrobenzoylphenylglycine, cellulose triacetate or other carbohydrate derivatives or chirally derivatized methacrylate polymers immobilized on silica gel. Suitable eluents for this purpose are aqueous or alcoholic solvent mixtures, for example, hexane/isopropanol/acetonitrile.

The corresponding stable isomers can be separated according to known methods, such as by extraction, filtration or column chromatography.

The term "patient" refers to any animal, including mammals, preferably mice, rats, other rodents, rabbits, dogs, cats, pigs, cows, sheep, horses or primates, and most preferably humans.

The term "therapeutically effective amount" refers to that amount of an active compound or drug which causes a biological or medical response sought by a researcher, veterinarian, physician or other clinician in a tissue, system, animal, individual or human, and which comprises one or more of the following: (1) prevention of disease: for example, prevention of a disease, disorder or condition in an individual who is susceptible to the disease, disorder or condition but has not yet experienced or developed the pathology or symptoms of the disease. (2) Inhibition of disease: for example, inhibition of a disease, disorder or condition (i.e., prevention of further progression of pathology and/or symptoms) in an individual undergoing or exhibiting the pathology or symptoms of the disease, disorder or condition. (3) Alleviation of disease: for example, alleviation of a disease, disorder or condition (i.e., reversion of pathology and/or symptoms) in an individual undergoing or exhibiting the pathology or symptoms of the disease, disorder or condition.

## BRIEF DESCRIPTION OF THE DRAWINGS

Fig. 1: Schematic diagram of free toxin release of ADC-5 in mouse, rat, human and monkey plasma;
Fig. 2: Schematic diagram of free toxin release of ADC-10 in mouse, rat, human and monkey plasma;
Fig. 3: Schematic diagram of free toxin release of DS8201 in mouse, rat, human and monkey plasma;
Fig. 4: Schematic diagram of free toxin release of ADC-5, ADC-10, ADC-14, and ADC-15 in mouse plasma;
Fig. 5: Schematic diagram of free toxin release of ADC-5, ADC-10, ADC-14, and ADC-15 in human plasma;
Fig. 6: Schematic diagram of efficacy evaluation in NCI-N87 tumor-bearing mice;
Fig. 7: Schematic diagram of efficacy evaluation in JIMT-1 tumor-bearing mice.

## DETAILED DESCRIPTION OF THE INVENTION

The technical solution of the present invention will be further described in detail below in conjunction with specific embodiments. It should be understood that the following embodiments are only intended to exemplify and explain the present invention and should not be construed as limiting the scope of protection of the present invention. All technologies realized based on the above contents of the present invention are included in the scope that the present invention intends to protect.

Unless otherwise stated, the raw materials and reagents used in the following examples are commercially available, or may be prepared in accordance with known methods.

The structures of the compounds are determined by nuclear magnetic resonance (NMR) or/and mass spectrum (MS). NMR shifts ($\delta$) are given in $10^{-6}$ (ppm). A nuclear magnetic resonance spectrometer (Bruker AVANCE-400) is used for NMR measurement with a solvent of deuterated dimethyl sulfoxide (DMSO-$d_6$), deuterated chloroform ($CDCl_3$), or deuterated methanol ($CD_3OD$), and with an internal standard of tetramethylsilane (TMS).

Agilent 1200 /1290 DAD-6110/6120 Quadrupole MS/LC/MS (manufacturer: Agilent, MS model: 6110/6120 Quadrupole MS), waters ACQuity UPLC-QD/SQD (manufacturer: waters, MS model: waters ACQuity Qda Detector/-

waters SQ Detector), and THERMO Ultimate 3000-Q Exactive (manufacturer: THERMO, MS model: THERMO Q Exactive) are used for MS measurement.

Agilent 1260II HPLC and Waters Acquity UPLC H-Class high performance liquid chromatograph are used for high performance liquid chromatography (HPLC) analysis.

Waters Acquity UPCC high performance liquid chromatograph is used for chiral HPLC analysis and determination.

High performance liquid chromatography is performed using Waters MS-triggered Prep-LC with SQD2 detector, Waters MS triggered Prep-LC with Acquity QDA detector, Waters MS-triggered Prep-LC with QDA detector and GILSON Prep LC with UV detector.

Combiflash Rf200 (TELEDYNE ISCO) is used for CombiFlash fast preparative chromatograph.

Yantai Huanghai HSGF254 or Qingdao GF254 silica gel plate is used as the silica gel plate for thin layer chromatography, the specification of the silica gel plate for thin layer chromatography (TLC) is from 0.15 mm to 0.2 mm, and the specification for thin layer chromatography for separation and purification of products is from 0.4 mm to 0.5 mm.

Yantai Huanghai silica gel of 200-300 mesh is generally used as the carrier for silica gel column chromatography.

The average inhibition rate and $IC_{50}$ value of kinases are determined using NovoStar microplate reader (German BMG).

Known starting materials of the present invention may be synthesized using or in accordance with methods known in the art, or may be purchased from, e.g., ABCR GmbH & Co. KG, Acros Organics, Aldrich Chemical Company, Accela ChemBio Inc, or Chembee Chemicals.

Unless otherwise specified in the examples, all reactions can be carried out under an argon atmosphere or a nitrogen atmosphere.

The argon atmosphere or the nitrogen atmosphere means that the reaction flask is connected to an argon or nitrogen balloon with a volume of about 1 L.

A hydrogen atmosphere means that the reaction flask is connected to a hydrogen balloon with a volume of about 1 L.

Parr 3916EKX hydrogenator and QL-500 hydrogen generator or HC2-SS hydrogenator are used for a pressurized hydrogenation reaction.

Vacuumization and filling with hydrogen are usually repeatedly operated 3 times prior to the hydrogenation reaction.

CEM Discover-S 908860 microwave reactor is used for a microwave reaction.

Unless otherwise specified in the examples, the solution refers to an aqueous solution.

Unless otherwise specified in the examples, the reaction temperature is room temperature (20°C-30°C).

Thin layer chromatography (TLC) is used for monitoring reaction processes in the examples. A developing agent used in a reaction, an eluent system of column chromatography used for purifying compounds, and a developing agent system of the thin layer chromatography comprise: A: dichloromethane/methanol system, and B: n-hexane/ethyl acetate system, wherein a volume ratio of the solvent is adjusted based on different polarity of the compounds, or adjusted optionally by addition of a small amount of an alkaline or acidic reagent such as triethylamine or acetic acid.

ADC preparation and analysis instruments: for the desktop centrifuge, Eppendoff, 5810R, 5430R, 5418R are used.

The pH meter used is METTLER TOLEDO, Seven Ecellence, and the electronic scale used is METTLER TOLEDO, ME1002E. The constant temperature mixer used is eppendorf, 5382KN644547. The inversion mixer used is IKA LoopSTER. The Nanodrop spectrophotometer used is Thermo Scientific, 2000-C. The microplate reader used is BioTek, EPOCH2. 6224 TOF, 6530 LC/Q-TOF is used for LC-MS, and Agilent Technologies, 1260 Infinity II is used for HPLC.

## Example 1

(*R*)-*N*-((1*S*,9*S*)-9-ethyl-5-fluoro-4,9-dihydroxy-10,13-dioxo-2,3,9,10,13,15-hexahydro-1*H*,12*H*-benzo[*de*]pyrano [3',4',6,7]indolizino[1,2-*b*]quinolin-1-yl)-3-hydroxybutanamide

(*R*)-*N*-((1*R*,9*S*)-9-ethyl-5-fluoro-4,9-dihydroxy-10,13-dioxo-2,3,9,10,13,15-hexahydro-1*H*,12*H*-benzo[*de*]pyrano [3',4',6,7]indolizino[1,2-*b*]quinolin-1-yl)-3-hydroxybutanamide

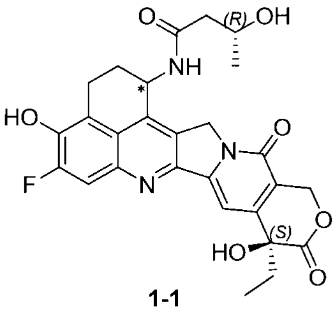

1-1
1-2

The position with * indicates that the atomic chirality there is R or S

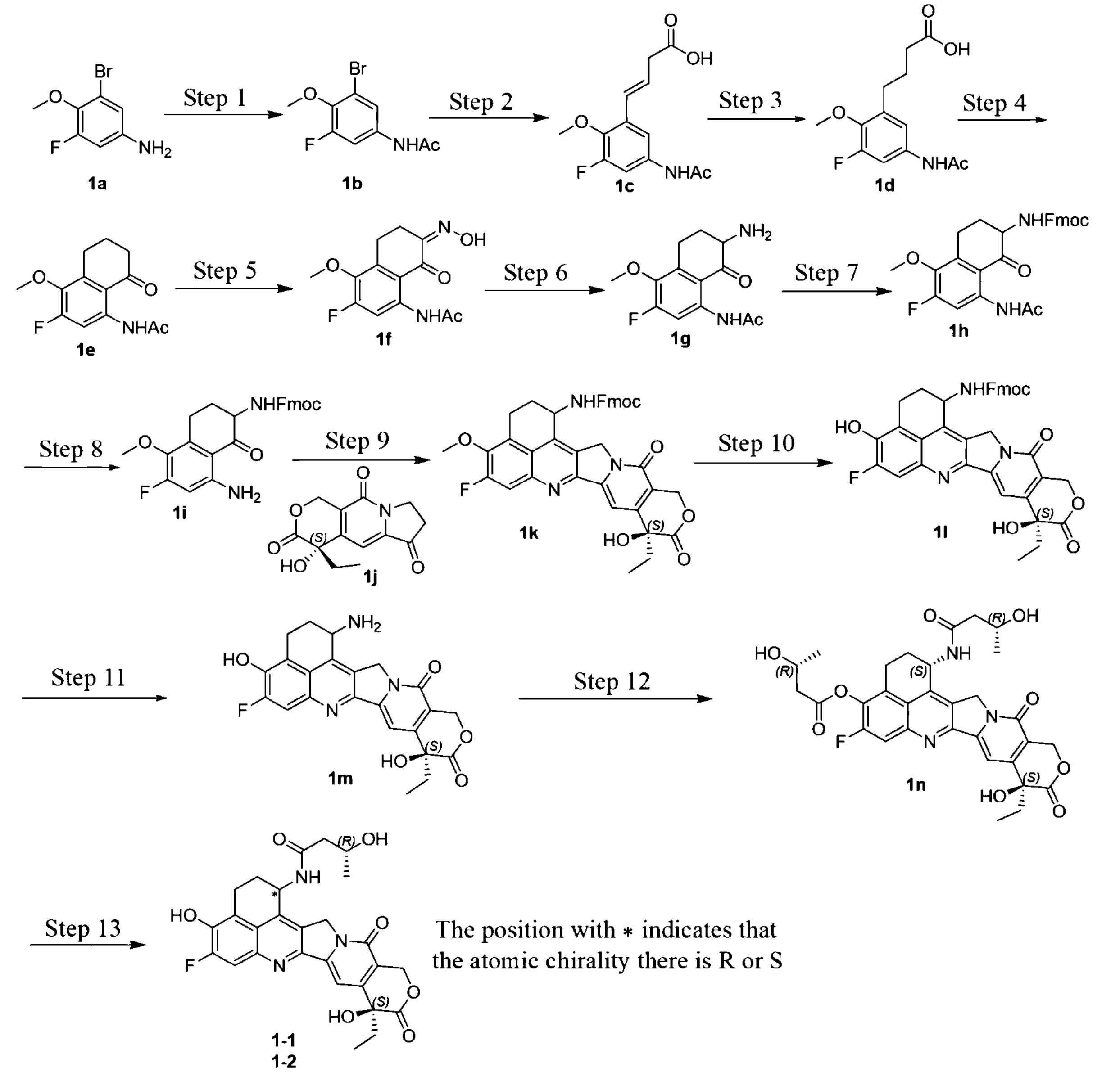


1a 1b 1c 1d 1e 1f 1g 1h 1i 1j 1k 1l 1m 1n

1-1
1-2

The position with * indicates that the atomic chirality there is R or S

Step 1 N-(3-bromo-5-fluoro-4-methoxyphenyl)acetamide **1b**

3-bromo-5-fluoro-4-methoxyaniline **1a** (20 g, 90.8 mmol) was dissolved in dichloromethane (40 mL). After the mixture was cooled to 0°C, acetyl chloride (14.3 g, 181.6 mmol) and triethylamine (27.6 mg, 272.4 mmol) were slowly added. The reaction mixture was stirred at 0°C for 0.5 h. After the reaction was completed, water (30 mL) was added to the reaction mixture. The mixture was extracted with dichloromethane (50 mL×3). The organic phases were combined, washed with saturated brine, dried, and concentrated. The resulting residue was purified by silica gel column chromatography with eluent system B, to give the title compound **1b** (20 g, yield: 84%).
MS m/z (ESI): 262.1 (M+H) $^+$.

Step 2 (E)-4-(5-acetylamino-3-fluoro-2-methoxyphenyl)but-3-enoic acid **1c**

The compound **1b** (20 g, 76.3 mmol) was dissolved in dioxane (30 mL) and water (10 mL), to which but-3-enoic acid (7.23 g, 83.9 mmol), palladium acetate (1.71 g, 7.6 mmol), tris(o-methylphenyl)phosphorus (4.64 g, 15.2 mmol), and N,N-diisopropylethylamine (30 mg, 229 mmol) were added. The reaction mixture was stirred at 100°C for 16 h. After the reaction was completed, the reaction mixture was filtered, and the resulting residue was purified by silica gel column chromatography with eluent system B to give the title compound **1c** (**20** g, yield: 87%).
MS m/z (ESI): 268.1 (M+H) $^+$.

Step 3 4-(5-acetylamino-3-fluoro-2-methoxyphenyl)butyric acid **1d**

The compound **1c** (20 g, 74.8 mmol) was dissolved in tetrahydrofuran (50 mL), and 10% Pd/C (0.8 g, 7.4 mmol) was added. The reaction mixture was stirred in a hydrogen atmosphere at room temperature for 2 h. After the reaction was completed, the reaction mixture was filtered, and the filtrate was concentrated under reduced pressure to give a crude product. The resulting residue was purified by silica gel column chromatography with eluent system A to give the title compound **1d** (20 g, yield: 99%).
MS m/z (ESI): 270.1 (M+1) $^+$.

Step 4 N-(3-fluoro-4-methoxy-8-oxo-5,6,7,8-tetrahydronaphthalen-1-yl)acetamide **1e**

The compound **1d** (20 g, 74.4 mmol) was dissolved in trifluoroacetic acid (60 mL), the mixture was cooled to 0°C, and then trifluoroacetic anhydride (31.24 g, 148.8 mmol) was slowly added. The reaction mixture was stirred at room temperature for 7 h. After the reaction was completed, the reaction mixture was slowly poured into water (50 mL), and extracted with ethyl acetate (50 mL×3). The organic phases were combined, washed with a saturated aqueous solution of sodium bicarbonate until the system became neutral, then washed with saturated brine, dried, and concentrated. The resulting residue was purified by silica gel column chromatography with eluent system B, to give the title compound **1e** (9.2 g, yield: 46%).
MS m/z (ESI): 252.1 (M+1) $^+$.

Step 5 (*Z*)-*N*-(3-fluoro-7-(hydroxyimino)-4-methoxy-8-oxo-5,6,7,8-tetrahydronaphthalen-1-yl)acetamide **1f**

Potassium tert-butoxide (9.83 g, 87.5 mmol) was dissolved in tetrahydrofuran (40 mL) and tert-butanol (10 mL), and the mixture was cooled to 0°C. Then, N-(3-fluoro-4-methoxy-8-oxo-5,6,7,8-tetrahydronaphthalen-1-yl)acetamide **1e** (10 g, 39.8 mmol) was dissolved in tetrahydrofuran (10 mL), the resulting mixture was slowly added to the reaction mixture, and ten minutes later, isoamyl nitrite (7.46 g, 63.6 mmol) was added. The reaction mixture was stirred at 0°C for 1 h. After the reaction was completed, a saturated ammonium chloride solution was added to the reaction mixture to quench the reaction. The mixture was extracted with ethyl acetate (50 mL×3). The organic phases were combined, then washed with saturated brine, dried, and concentrated. The resulting residue was purified by silica gel column chromatography with eluent system B, to give the title compound **1f** (6 g, yield: 51%).
MS m/z (ESI): 281.1 (M+1) $^+$.

Step 6 *N*-(7-amino-3-fluoro-4-methoxy-8-oxo-5,6,7,8-tetrahydronaphthalen-1-yl)acetamide **1g**

(*Z*)-*N*-(3-fluoro-7-(hydroxyimino)-4-methoxy-8-oxo-5,6,7,8-tetrahydronaphthalen-1-yl)acetamide **1f** (6 g, 21.4 mmol) was dissolved in dioxane (60 mL) and 2 *N* hydrochloric acid solution (20 mL), and 10% Pd/C (1.13 g, 10.7 mmol) was added. The reaction mixture was stirred in a hydrogen atmosphere at room temperature for 5 h. After the reaction was completed, the reaction mixture was filtered and concentrated to give a crude product **1g** (5 g), which was directly used in the next step without purification.
MS m/z (ESI): 267.1 (M+1) $^+$.

Step 7 (9*H*-fluoren-9-yl)methyl(8-acetamido-6-fluoro-5-methoxy-1-oxo-1,2,3,4-tetrahydronaphthalen-2-yl)carbamate **1h**

The compound **1g** (5 g, 18.8 mmol) obtained from the previous step was dissolved in dioxane (50 mL), and after the solution was adjusted with sodium bicarbonate to pH=7-8, 9-fluorenylmethyl-*N*-succinimidyl carbonate (6.36 g, 18.8

mmol) was slowly added. The reaction mixture was stirred at room temperature for 1 h. After the reaction was completed, the reaction mixture was slowly poured into water (40 mL), and extracted with ethyl acetate (40 mL×3). The organic phases were combined, washed with saturated brine, dried, and concentrated.

The resulting residue was purified by silica gel column chromatography with eluent system B, to give the title compound **1h** (5.3 g, yield: 54%).

MS m/z (ESI): 489.2 $(M+1)^+$.

Step 8 (9*H*-fluoren-9-yl)methyl(8-amino-6-fluoro-5-methoxy-1-oxo-1,2,3,4-tetrahydronaphthalen-2-yl)carbamate **1i**

The compound **1h** (5 g, 10.25 mmol) was dissolved in dioxane (50 mL) and 12 *N* hydrochloric acid (10 mL). The reaction mixture was stirred at 60°C for 2 h. After the reaction was completed, the reaction mixture was slowly poured into water (50 mL), and extracted with ethyl acetate (40 mL×3). The organic phases were combined, washed with saturated brine, dried, and concentrated. The resulting residue was purified by silica gel column chromatography with eluent system B, to give the title compound **1i** (3.75 g, yield: 78%).

MS m/z (ESI): 447.2 $(M+1)^+$.

Step 9 (9*H*-fluoren-9-yl)methyl((9*S*)-9-ethyl-5-fluoro-9-hydroxy-4-methoxy-10,13-dioxo-2,3,9,10,13,15-hexahydro-1*H*,12*H*-benzo[de]pyrano[3',4',6,7]indolizino[1,2-*b*]quinolin-1-yl)carbamate **1k**

The compound **1i** (3 g, 6.6 mmol) was dissolved in toluene (30 mL), and (*S*)-4-ethyl-4-hydroxy-7,8-dihydro-1*H*-pyranindolizine-3,6,10(4*H*)-trione **1j** (2.61 g, 9.9 mmol) and p-toluenesulfonic acid (2.52 g, 13.2 mmol) were added. The reaction mixture was stirred at 110°C for 5 h. After the reaction was completed, the mixture was extracted with ethyl acetate (30 mL×3). The organic phases were combined, washed with saturated brine, dried, and concentrated. The resulting residue was purified by silica gel column chromatography with eluent system B, to give the title compound **1k** (1.8 g, yield: 36%).

MS m/z (ESI): 674.2 $(M+1)^+$.

Step 10 (9*H*-fluoren-9-yl)methyl((9*S*)-9-ethyl-5-fluoro-4,9-dihydroxy-10,13-dioxo-2,3,9,10,13,15-hexahydro-1*H*,12*H*-benzo[de]pyrano[3',4',6,7]indolizino[1,2-*b*]quinolin-1-yl)carbamate **1l**

The compound **1k** (1.8 g, 2.7 mmol) was dissolved in 40% hydrobromic acid (40 mL). The reaction mixture was stirred at 100°C for 2 h. After the reaction was completed, the reaction mixture was extracted with ethyl acetate (50 mL×3). The organic phases were combined, washed with saturated brine, dried, and concentrated. The resulting residue was purified by silica gel column chromatography with eluent system B, to give the title compound **1l** (1.08 g, yield: 60%).

MS m/z (ESI): 660.2 $(M+1)^+$.

Step 11 (9*S*)-1-amino-9-ethyl-5-fluoro-4,9-dihydroxy-1,2,3,9,12,15-hexahydro-10*H*,13*H*-benzo[*de*]pyrano[3',4',6,7]indolizino[1,2-*b*]quinoline-10,13-dione **1m**

The compound **1l** (500 mg, 0.7 mmol) was dissolved in *N,N*-dimethylformamide (5 mL), and diethylamine (166 mg, 2.3 mmol) was added. The reaction mixture was stirred at room temperature for 1 h. After the reaction was completed, the reaction mixture was spin-dried using an oil pump to remove diethylamine, thus giving the crude product **1m,** which was slurried with ethyl acetate to give a solid. The solid was directly used in the next reaction step.

MS m/z (ESI): 438.1 $(M+1)^+$.

Step 12 (1*S*,9*S*)-9-ethyl-5-fluoro-9-hydroxy-1-((*R*)-3-hydroxybutyrylamino)-10,13-dioxo-2,3,9,10,13,15-hexahydro-1*H*,12*H*-benzo[de]pyrano[3',4',6,7]indolizino[1,2-*b*]quinolin-4-yl-(R)-3-hydroxybutyrate **1n**

The compound **1m** (100 mg, 0.2 mmol) was dissolved in *N,N*-dimethylformamide (3 mL), and (*R*)-3-hydroxybutyric acid (36 mg, 0.34 mmol), 2-(7-azabenzotriazole)-*N,N,N',N'*-tetramethyluronium hexafluorophosphate (174 mg, 0.46 mmol), and *N,N*-diisopropylethylamine (88 mg, 0.68 mmol) were added. The reaction mixture was stirred at room temperature for 1 h. After the reaction was completed, the reaction mixture was extracted with ethyl acetate (50 mL×3). The organic phases were combined, washed with saturated brine, dried, and concentrated. The resulting residue was purified by silica gel column chromatography with eluent system B, to give the title compound **1n** (90 mg, yield: 64%).

MS m/z (ESI): 610.2 $(M+1)^+$.

Step 13

(R)-N-((1S,9S)-9-ethyl-5-fluoro-4,9-dihydroxy-10,13-dioxo-2,3,9,10,13,15-hexahydro-1*H*,12*H*-benzo[*de*]pyrano [3',4',6,7]indolizino[1,2-*b*]quinolin-1-yl)-3-hydroxybutanamide

(*R*)-*N*-((1*R*,9*S*)-9-ethyl-5-fluoro-4,9-dihydroxy-10,13-dioxo-2,3,9,10,13,15-hexahydro-1*H*,12*H*-benzo[*de*]pyrano [3',4',6,7]indolizino[1,2-*b*]quinolin-1-yl)-3-hydroxybutanamide

The compound **1n** (90 mg, 0.15 mmol) was dissolved in methanol (5 mL), and then an aqueous solution of lithium hydroxide (2 mL, 1 M) was added at room temperature. The mixture was stirred at room temperature for reaction for 15 min. After the reaction was completed, methanol was spun away, and the remaining aqueous phase was lyophilized to give a crude product, which was purified by preparative high performance liquid chromatography (Waters MS-triggered Prep-LC with SQD2 detector, chromatographic column: Xbridge C18 150*19 mm, 5 μm; mobile phase 1: water (containing 0.1% TFA); mobile phase 2: acetonitrile; 15-min gradient, gradient proportion: acetonitrile phase 23%-33%, flow rate: 20 mL/min) to give compound **1-1** (35 mg, yield: 30%) and compound **1-2** (28 mg, yield: 24%).

Mono-configurational compound **1-1** (short retention time):

MS m/z (ESI): 524.2 (M+1) $^+$.
$^1$H NMR (400 MHz, DMSO-$d_6$) δ 10.42 (s, 1H), 8.39 (d, J = 8.8 Hz, 1H), 7.84 (d, J = 11.6 Hz, 1H), 7.26 (s, 1H), 6.50 (s, 1H), 5.57 - 5.49 (m, 1H), 5.41 (s, 2H), 5.27 - 5.11 (m, 2H), 4.04 (dd, J = 13.2, 6.0 Hz, 1H), 3.18 - 3.04 (m, 2H), 2.36 - 2.17 (m, 2H), 2.15 - 1.96 (m, 2H), 1.92 - 1.77 (m, 2H), 1.08 (d, J = 6.4 Hz, 3H), 0.93 - 0.81 (m, 3H).

Mono-configurational compound **1-2** (long retention time):

MS m/z (ESI): 524.2 (M+1) $^+$.
$^1$H NMR (400 MHz, DMSO-$d_6$) δ 10.42 (s, 1H), 8.42 (d, J = 8.8 Hz, 1H), 7.84 (d, J = 11.6 Hz, 1H), 7.26 (s, 1H), 5.59 - 5.51 (m, 1H), 5.42 (s, 2H), 5.29 - 5.14 (m, 2H), 4.04 (dd, J = 13.2, 6.4 Hz, 1H), 3.17 (dd, J = 12.0, 4.8 Hz, 1H), 3.10 - 2.92 (m, 1H), 2.28 (dd, J = 13.6, 7.2 Hz, 1H), 2.18 (dd, J = 13.6, 5.6 Hz, 1H), 2.13 - 1.95 (m, 2H), 1.93 - 1.78 (m, 2H), 1.08 (d, J = 6.0 Hz, 3H), 0.91 - 0.82 (m, 3H).

**Example 2**

(*R*)-*N*-((1*S*,9*S*)-9-ethyl-4,9-dihydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-9-ethyl-4,9-dihydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

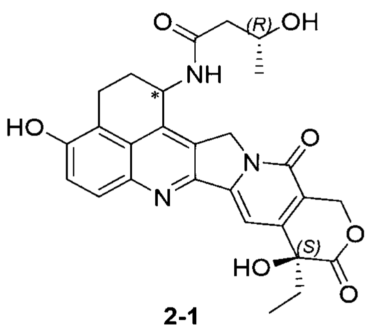

**2-1**
**2-2**

The position with * indicates that the atomic chirality there is R or S

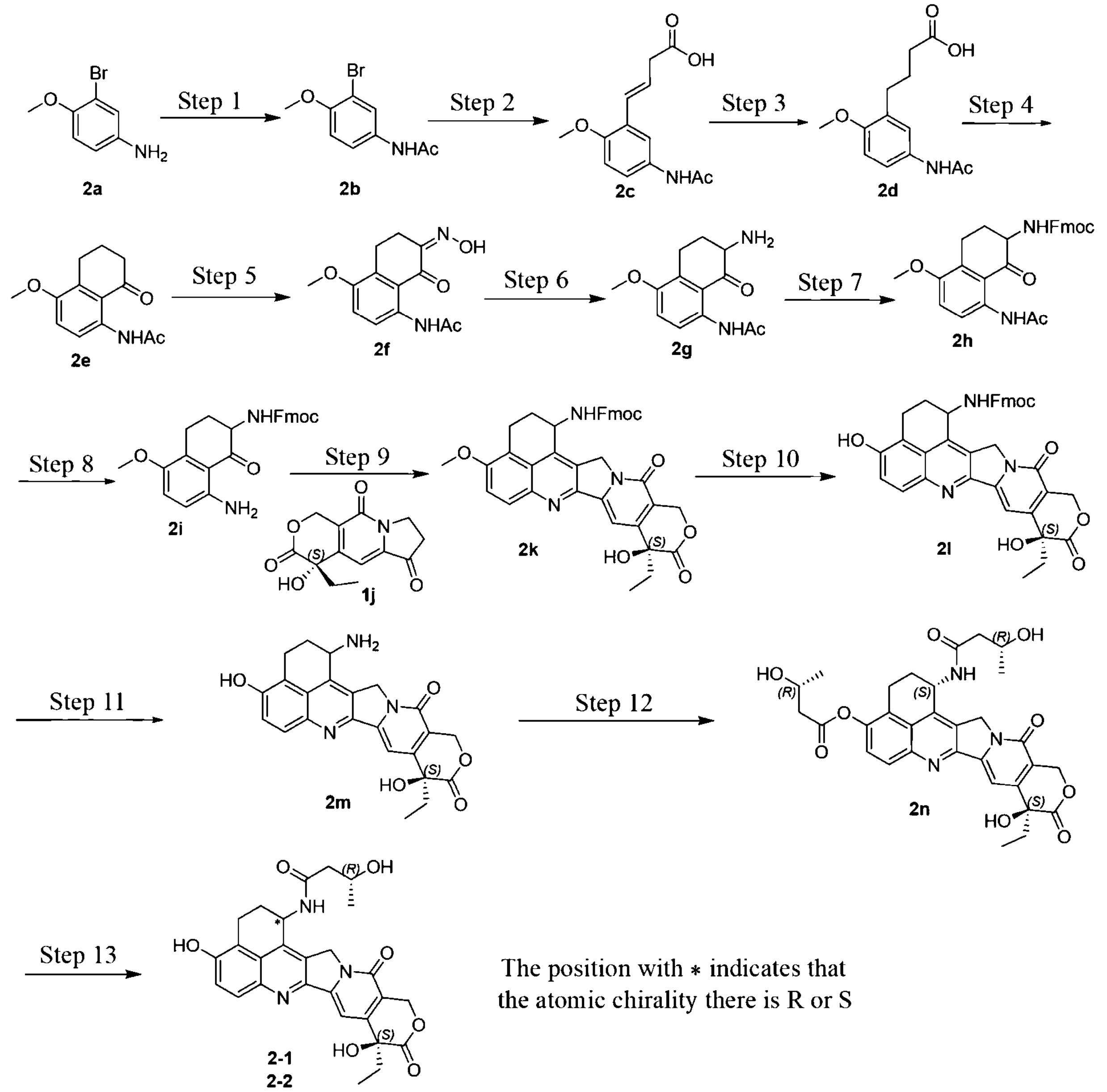


2a 2b 2c 2d 2e 2f 2g 2h 2i 1j 2k 2l 2m 2n 2-1 2-2

In accordance with the synthetic route in Example 1, the raw material from the Step 1 was replaced with 3-bromo-4-methoxyaniline **2a** (25 g, 0.12 mol), and the system was purified by preparative high performance liquid chromatography (Waters MS-triggered Prep-LC with SQD2 detector, chromatographic column: Xbridge C18 150*19 mm, 5 μm; mobile phase 1: water (0.1% TFA); mobile phase 2: acetonitrile; 15-min gradient, gradient proportion: acetonitrile phase 23%-33%, flow rate: 20 mL/min) to give the title products **2-1** (19 mg, yield: 28%) and **2-2** (30 mg, yield: 44%).

Mono-configurational compound **2-1** (short retention time):

MS m/z (ESI): 506 (M+1) $^+$.

$^1$H NMR (400 MHz, DMSO-$d_6$) δ 10.21 (s, 1H), 8.39-8.37 (m, 1H), 7.92-7.90 (m, 1H), 7.51-7.50 (m, 1H), 7.24 (s, 1H), 6.49 (s, 1H), 5.57-5.48 (m, 1H), 5.41(s, 2H), 5.27-5.11 (m, 2H), 4.65 (s, 1H), 4.09-3.98 (m, 1H), 3.09-2.96 (m, 2H), 2.25-2.23 (m, 2H), 2.05-2.00 (m, 2H), 1.95-1.77 (m, 2H), 1.23 (s, 2H),1.08-1.07 (m, 3H), 0.86-0.85(m, 3H).

Mono-configurational compound **2-2** (long retention time):

MS m/z (ESI): 506 (M+1) $^+$.

$^1$H NMR (400 MHz, DMSO-$d_6$) δ 10.22 (s, 1H), 8.42 - 8.40 (d, J = 8.4 Hz, 1H), 7.93 - 7.90 (d, J =8.4 Hz, 1H), 7.51 - 7.50 (m, 1H), 7.25 (s, 1H), 5.58 - 5.46 (m,1H), 5.42 (s, 2H), 5.21 - 5.20 (m, 2H), 4.04 - 4.01 (m, 1H), 2.96 - 2.94 (m, 2H), 2.28

(s, 2H), 2.10 - 1.97 (m, 2H), 1.86 (s, 2H), 1.23 (s, 2H), 1.08 - 1.06 (m, 3H), 0.87- 0.85 (m, 3H).

**Example 3**

(*R*)-*N*-((1*S*,9*S*)-4-bromo-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-4-bromo-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

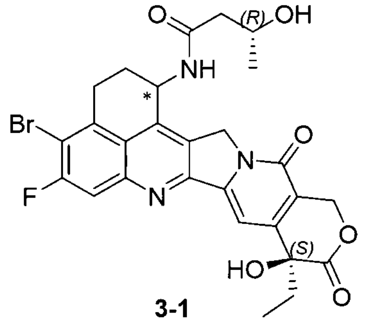

**3-1**
**3-2**

The position with * indicates that the atomic chirality there is R or S

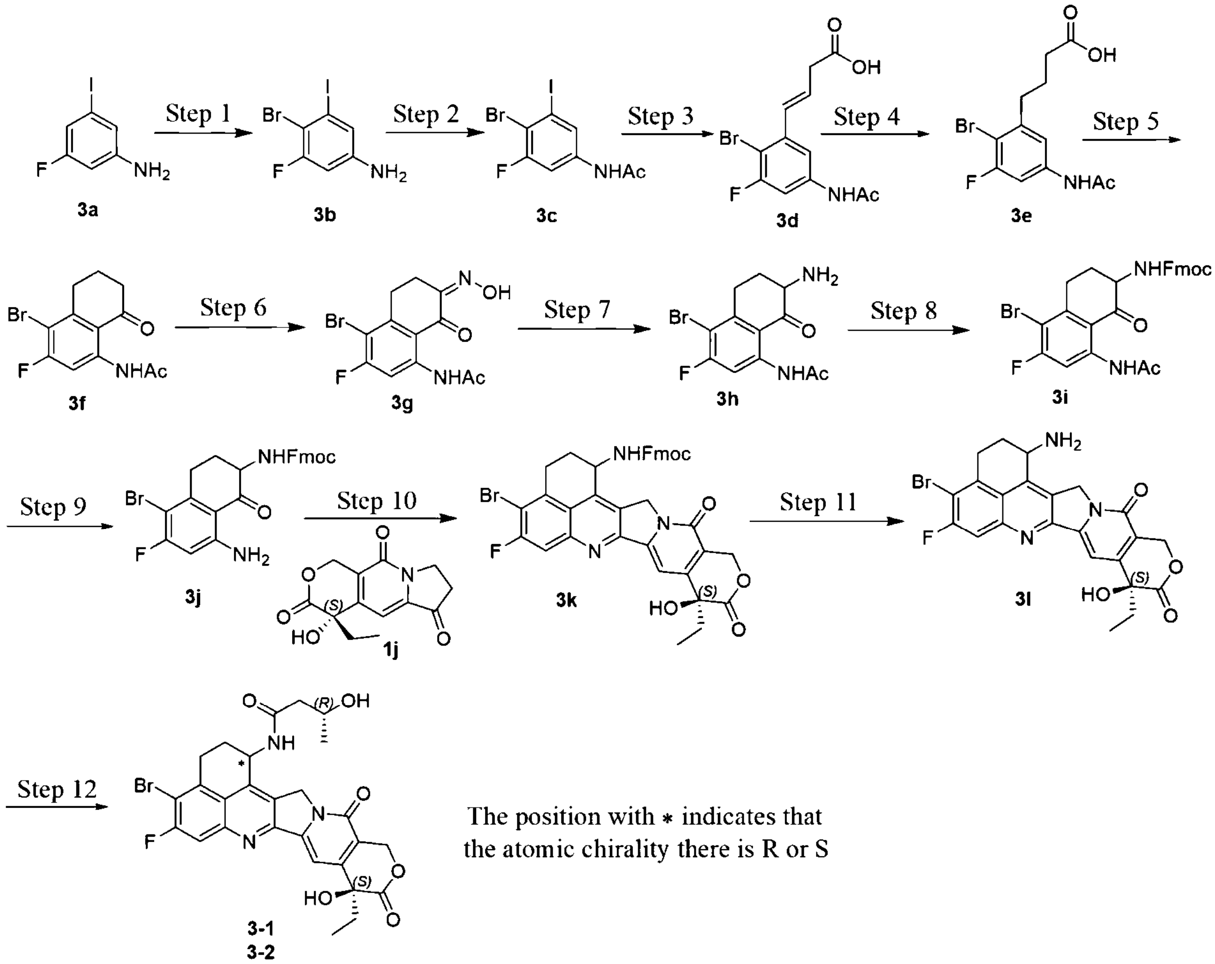


3a, 3b, 3c, 3d, 3e, 3f, 3g, 3h, 3i, 3j, 1j, 3k, 3l, 3-1, 3-2

The position with * indicates that the atomic chirality there is R or S

Step 1 4-bromo-3-fluoro-5-iodoaniline **3b**

3-fluoro-5-iodoaniline **3a** (50 g, 210.95 mmol) was dissolved in DMF (250 mL), and NBS (41.30 g, 232.04 mmol) was slowly added under an ice bath condition. The mixture was stirred for reaction at room temperature for 16 h. After the reaction was completed, water was added, and the system was extracted with dichloromethane. The organic phase was washed with saturated brine, collected, dried over anhydrous sodium sulfate, and spin-dried to remove the solvent, thus giving the crude product **3b** (66 g).
MS m/z (ESI): 315.9, 317.9 $(M+1)^+$.
$^1$H NMR (400 MHz, DMSO-$d_6$) δ 7.01 (d, 1H), 6.47 (dd, 1H), 5.74 (s, 2H).

Step 2 *N*-(4-bromo-3-fluoro-5-iodophenyl)acetamide **3c**

The compound **3b** (66 g, 208.92 mmol) was dissolved in dichloromethane (660 mL), and triethylamine (42.28 g, 417.84 mmol) was added. After the system was cooled to 0°C, acetyl chloride (19.68 g, 250.70 mmol) was slowly added dropwise. After the dropwise addition was complete, the system was stirred at room temperature for 4 h. After the reaction was completed, the mixture was spin-dried to remove the solvent, thus giving a crude product, which was redissolved in ethyl acetate. Then, the system was adjusted with dilute hydrochloric acid to have a pH of 2-3, then extracted with ethyl acetate, and washed with saturated brine. The organic phase was collected and dried over anhydrous sodium sulfate. The system was spin-dried to remove the solvent, thus giving a crude product, which was slurried with a mixed solvent of dichloromethane/methanol (10:1) to give the title compound **3c** (60 g, yield: 80%).
MS m/z (ESI): 357.9, 359.9 $(M+1)^+$.
$^1$H NMR (400 MHz, DMSO-$d_6$) δ 10.27 (s, 1H), 7.96 (s, 1H), 7.66 (dd, 1H), 2.06 (s, 3H).

Step 3 (*E*)-4-(5-acetylamino-2-bromo-3-fluorophenyl)but-3-enoic acid **3d**

The compound **3c** (20 g, 55.87 mmol) was dissolved in a mixed solvent of dioxane (200 mL) and water (40 mL), and but-3-enoic acid (4.81 g, 55.87 mmol), DIPEA (14.45 g, 111.74 mmol), palladium acetate (630 mg, 2.79 mmol), and tris(o-methylphenyl)phosphorus (1.7 g, 5.59 mmol) were added. The mixture was stirred for reaction under the protection of nitrogen at 100°C for 16 h. After the reaction was completed, water and dichloromethane were added, and the system was washed with saturated sodium bicarbonate solution 3-5 times. The aqueous phase was collected, adjusted with hydrochloric acid to a pH of 2-3, and extracted with ethyl acetate 5-7 times. The organic phase was collected, dried over anhydrous sodium sulfate, and spin-dried to remove the solvent, thus giving the crude compound **3d** (17 g).
MS m/z (ESI): 316.0, 317.9 $(M+1)^+$.

Step 4 4-(5-acetylamino-2-bromo-3-fluorophenyl)butyric acid **3e**

The compound **3d** (8 g, 25.3 mmol) was dissolved in methanol (80 mL), and a platinum-carbon catalyst (800 mg) was added. The mixture was stirred for reaction in a hydrogen environment at room temperature for two hours. After the reaction was completed, the mixture was filtered. The filtrate was collected, and spin-dried to remove the solvent, thus giving the crude product **3e** (8 g). The crude product was directly used in the next reaction step without purification.
MS m/z (ESI): 318.0, 320.0 $(M+1)^+$.
$^1$H NMR (400 MHz, DMSO-$d_6$) δ 12.14 (s, 1H), 10.24 (s, 1H), 7.63 (dd, 1H), 7.25 (s, 1H), 2.75-2.67 (m, 2H), 2.29 (t, 2H), 2.05 (s, 3H), 1.79 (dd, 2H).

Step 5 *N*-(4-bromo-3-fluoro-8-oxo-5,6,7,8-tetrahydronaphthalen-1-yl)acetamide **3f**

The compound 3e (8 g, 25.1 mmol) was dissolved in trifluoroacetic acid (80 mL), and trifluoroacetic anhydride (15.82 g, 75.3 mmol) was slowly added under an ice bath condition. The mixture was stirred for reaction at 0°C for 4 h. After the reaction was completed, water was added in an ice bath. The system was adjusted with 15% sodium hydroxide solution to a pH of 9-10, and then extracted with dichloromethane. The organic phase was washed with saturated brine, collected, dried over anhydrous sodium sulfate, and spin-dried to remove the solvent, thus giving the compound **3f** (4.8 g, yield: 53%).
MS m/z (ESI): 329.0, 331.0 $(M+1)^+$.

Step 6 (Z)-N-(4-bromo-3-fluoro-7-(hydroxyimino)-8-oxo-5,6,7,8-tetrahydronaphthalen-1-yl)acetamide **3g**

Potassium tert-butoxide (1.62 g, 14.4 mmol) was dissolved in a mixed solvent of tetrahydrofuran (80 mL) and tert-butanol (20 mL), and a solution of the compound **3f** (2.15 g, 7.2 mmol) in tetrahydrofuran (20 mL) was slowly added in an ice bath. After the mixture was stirred for reaction at 0°C for 10 min, isoamyl nitrite (1.27 g, 10.8 mmol) was added, and the

reaction was continued by stirring at 0°C for 50 min. After the reaction was completed, the system was adjusted with dilute hydrochloric acid to have a pH of 4-5, and extracted with ethyl acetate. The organic phase was washed with saturated brine, collected, dried over anhydrous sodium sulfate, and spin-dried to remove the solvent, thus giving a crude product, which was slurried with methyl tert-butyl ether to give the title compound **3g** (1.1 g, yield: 46%).

MS m/z (ESI): 329.0, 331.0 (M+1) $^+$.

$^1$H NMR (400 MHz, $CD_3OD$) $\delta$ 8.52 (d, 1H), 3.21 (dd, 2H), 3.07 (dd, 2H), 2.24 (s, 3H).

Step 7 *N*-(7-amino-4-bromo-3-fluoro-8-oxo-5,6,7,8-tetrahydronaphthalen-1-yl)acetamide **3h**

The compound **3g** (500 mg, 1.52 mmol) was dissolved in dioxane (10 mL), and 1 mL of 1M hydrochloric acid and a platinum-carbon catalyst (100 mg) were added. The mixture was stirred for reaction in a hydrogen environment at room temperature for 4 h. After the reaction was completed, the mixture was filtered. The filtrate was collected and concentrated to give a crude product **3h** (500 mg), which was directly used in the next reaction step.

MS m/z (ESI): 315.1, 317.1 (M+1) $^+$.

Step 8 (9*H*-fluoren-9-yl)methyl(8-acetylamino-5-bromo-6-fluoro-1-oxo-1,2,3,4-tetrahydronaphthalen-2-yl)carbamate **3i**

The compound **3h** (500 mg) was dissolved in dioxane (10 mL), the filtrate from the step 7 was adjusted with a saturated sodium carbonate solution to a pH of 8-9, and then fluorenylmethoxycarbonyl chloride (432 mg, 1.67 mmol) was added. The mixture was stirred for reaction at room temperature for 1 h. After the reaction was completed, the system was extracted with ethyl acetate. The organic phase was washed with saturated brine, collected, dried over anhydrous sodium sulfate, and spin-dried to remove the solvent, thus giving a crude product. The resulting residue was purified by silica gel column chromatography with eluent system B, to give the title compound **3i** (300 mg, yield: 37%).

MS m/z (ESI): 537.0, 539.0 (M+1) $^+$.

Step 9 (9*H*-fluoren-9-yl)methyl(8-amino-5-bromo-6-fluoro-1-oxo-1,2,3,4-tetrahydronaphthalen-2-yl)carbamate **3j**

The compound **3i** (700 mg, 1.30 mmol) was dissolved in methanol (10 mL), and concentrated hydrochloric acid (12 mol/L, 2 mL) was added. The mixture was stirred for reaction at 60°C for 1 h. After the reaction was completed, the resulting residue was purified by silica gel column chromatography with eluent system B, to give the title compound **3j** (500 mg, yield: 77%).

Step 10 ((9*H*-fluoren-9-yl)methyl((9*S*)-4-bromo-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)carbamate **3k**

The compound **3j** (200 mg, 0.40 mmol) was dissolved in toluene (5 mL), and (S)-4-ethyl-4-hydroxy-7,8-dihydro-1*H*-pyrano[3,4-*f*]indolizine-3,6,10(4*H*)-trione **1j** (117 mg, 0.44 mmol) and p-toluenesulfonic acid monohydrate (77 mg, 0.40 mmol) were added. The mixture was stirred for reaction at 120°C for 2 h. After the reaction was completed, the mixture was spin-dried to remove the solvent, thus giving a crude product. The resulting residue was purified by silica gel column chromatography with eluent system B to give the title compound **3k** (250 mg, yield: 86%).

MS m/z (ESI): 722.0, 724.0 (M+1) $^+$.

$^1$H NMR (400 MHz, $CDCl_3$) $\delta$ 8.19-7.31 (m, 9H), 5.68 (dd, 1H), 5.33-5.18 (m, 2H), 4.66 (s, 2H), 4.34 (d, 1H), 4.21-4.04 (m, 2H), 3.26 (s, 1H), 3.00-2.94 (m, 1H), 2.04 (s, 2H), 1.81 (s, 2H), 1.70-1.50 (m, 2H), 1.27 (dd, 3H).

Step 11 (9*S*)-1-amino-4-bromo-9-ethyl-5-fluoro-9-hydroxy-1,2,3,9,12,15-hexahydro-10*H*,13*H*-benzo[*de*]pyrano[3',4',6,7]indolizino[1,2-*b*]quinoline-10,13-dione **3l**

The compound **3k** (200 mg, 0.28 mmol) was dissolved in DMF (1 mL), and diethylamine (0.1 mL) was added. The mixture was stirred for reaction at room temperature for 30 min. After the reaction was completed, the resulting residue was purified by silica gel column chromatography with eluent system A, to give the title compound **3l** (100 mg, yield: 72%).

MS m/z (ESI): 500.0, 502.1 (M+1) $^+$.

Step 12

(*R*)-*N*-((1*S*,9*S*)-4-bromo-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-4-bromo-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

The compound **31** (100 mg, 0.20 mmol) was dissolved in DMF (2 mL), and (R)-3-hydroxybutyric acid (25 mg, 0.24 mmol), 2-(7-azobenzotriazole)-*N,N,N',N'*-tetramethyluronium hexafluorophosphate (114 mg, 0.30 mmol), and *N,N*-diisopropylethylamine (52 mg, 0.40 mmol) were added. The mixture was stirred for reaction at room temperature for 1 h. After the reaction was completed, the system was purified by preparative high performance liquid chromatography (Waters MS-triggered Prep-LC with QDA detector, chromatographic column: WELCH Xtimate C18 21.2*250mm, 10 μm; mobile phase 1: water (0.1% FA); mobile phase 2: acetonitrile; 15-min gradient, gradient proportion: acetonitrile phase 38%-48%, flow rate: 25 mL/min) to give compound **3-1** (1.62 mg, yield: 6%) and compound **3-2** (1.32 mg, yield: 7.4%).

Mono-configurational compound **3-1** (short retention time):

MS m/z (ESI): 586.0, 588.0 (M+1) $^+$.

$^1$H NMR (400 MHz, $CD_3OD$) δ 8.10 (d, *J* = 10.0 Hz, 1H), 7.90 (s, 1H), 5.96 - 5.88 (m, 1H), 5.83 (d, *J* = 16.4 Hz, 1H), 5.71 - 5.48 (m, 4H), 4.52 - 4.46 (m, 1H), 2.71 - 2.65 (m, 2H), 2.59 - 2.52 (m, 2H), 2.23 - 2.18 (m, 3H), 1.49 (d, *J*= 6.4 Hz, 3H), 1.25 (t, *J*= 7.2 Hz, 3H).

Mono-configurational compound **3-2** (long retention time):

MS m/z (ESI): 586.0, 588.0 (M+1) $^+$.

$^1$H NMR (400 MHz, $CD_3OD$) δ 7.79 (d, *J* = 9.6 Hz, 1H), 7.59 (s, 1H), 5.67 - 5.59 (m, 1H), 5.52 (d, *J* = 16.4 Hz, 1H), 5.41 - 5.33 (m, 2H), 5.32 - 5.23 (m, 2H), 4.24 - 4.18 (m, 1H), 2.34 (d, *J* = 6.8 Hz, 2H), 2.28 - 2.21 (m, 2H), 1.93 - 1.86 (m, 3H), 1.17 (d, *J* = 6.4 Hz, 3H), 0.94 (t, *J*= 7.2 Hz, 3H).

## Example 4

(*R*)-*N*-((1*S*,9*S*)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

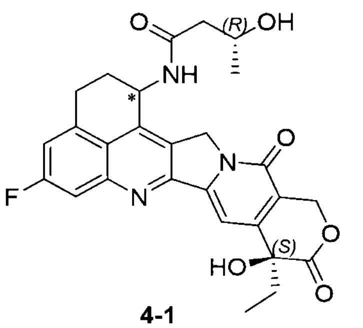

**4-1**
**4-2**

The position with * indicates that the atomic chirality there is R or S

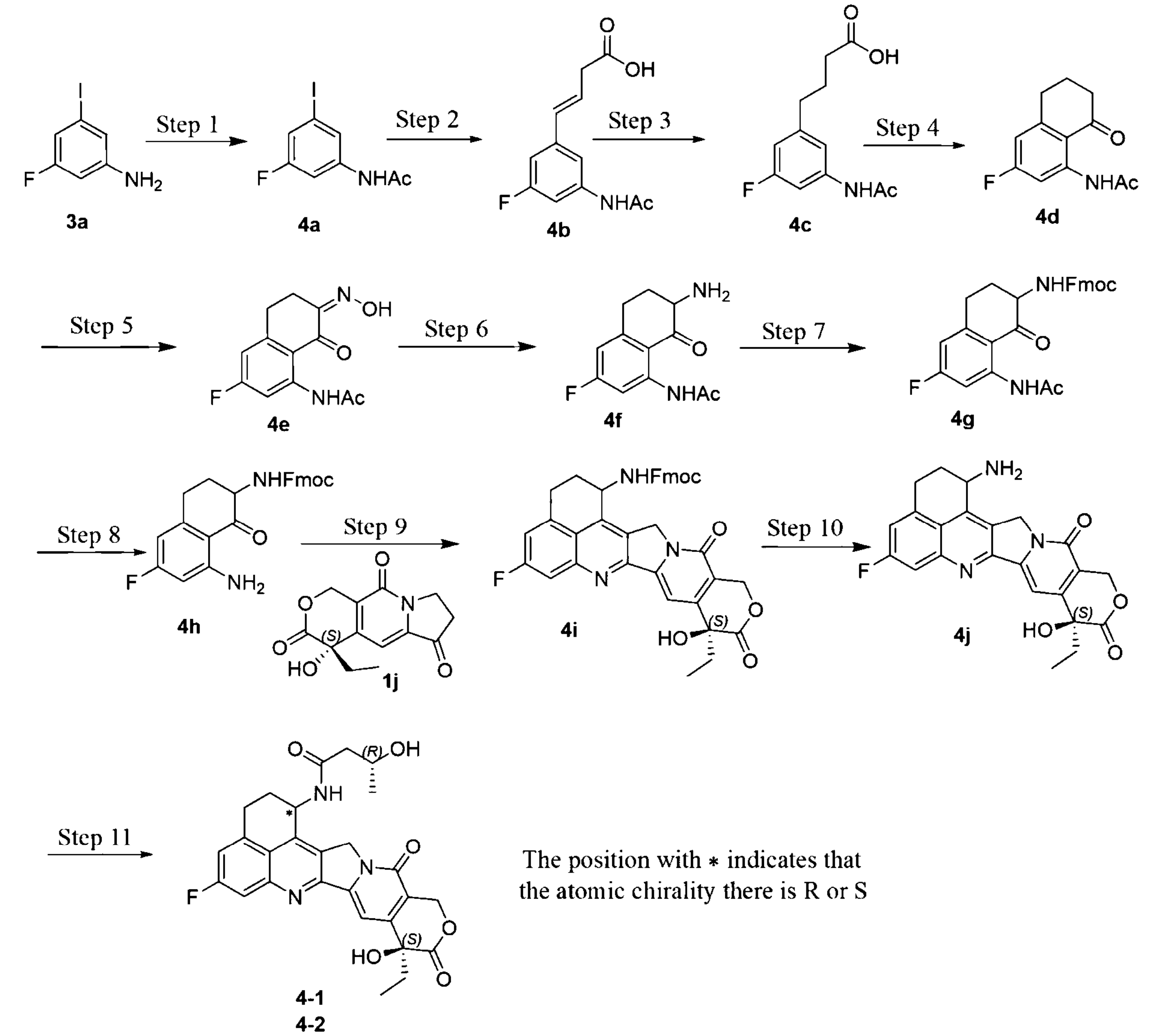


In accordance with the synthetic route in Example 3, 3-fluoro-5-iodoaniline (25 g, 105.5 mmol) was used as the starting material, and the system was purified by preparative high performance liquid chromatography (Waters MS-triggered Prep-LC with QDA detector, chromatographic column: WELCH Xtimate C18 21.2*250 mm, 10 μm; mobile phase 1: water (0.1% FA); mobile phase 2: acetonitrile; 15-min gradient, gradient proportion: acetonitrile phase 38%-48%, flow rate: 25 mL/min) to give the title products **4-1** (2.5 mg, yield: 12%) and **4-2** (3.1 mg, yield: 15%).

Mono-configurational compound **4-1** (short retention time):

MS m/z (ESI): 508.1 (M+1) $^+$.

$^1$H NMR (400 MHz, $CD_3OD$) δ 7.65 (d, *J* = 10.2 Hz, 2H), 7.37 (d, *J* = 8.8 Hz, 1H), 5.66 (s, 1H), 5.56 (d, *J* = 16.4 Hz, 1H), 5.40 - 5.20 (m, 4H), 4.28 - 4.21 (m, 1H), 2.47 - 2.39 (m, 2H), 2.27 (s, 2H), 1.98 - 1.90 (m, 2H), 1.24 (d, *J* = 6.4 Hz, 4H), 0.99 (t, *J* = 7.2 Hz, 3H).

Mono-configurational compound **4-2** (long retention time):

MS m/z (ESI): 508.1 (M+1) $^+$.

$^1$H NMR (400 MHz, $CD_3OD$) δ 7.57 (d, *J* = 6.2 Hz, 2H), 7.30 (d, *J* = 8.0 Hz, 1H), 5.60 (d, *J* = 6.0 Hz, 1H), 5.48 (d, *J* = 16.4 Hz, 1H), 5.36 - 5.15 (m, 5H), 4.22 - 4.15 (m, 1H), 2.36 - 2.30 (m, 2H), 2.18 (t, *J* = 6.4 Hz, 3H), 1.90 - 1.83 (m, 2H), 1.15 (d, *J* = 6.4 Hz, 3H), 0.91 (t, *J* = 7.2 Hz, 3H).

**Example 5**

(*R*)-*N*-((1*S*,9*S*)-4-(difluoromethoxy)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-4-(difluoromethoxy)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

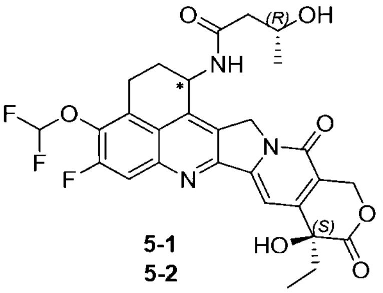

**5-1**
**5-2**

The position with * indicates that the atomic chirality there is R or S

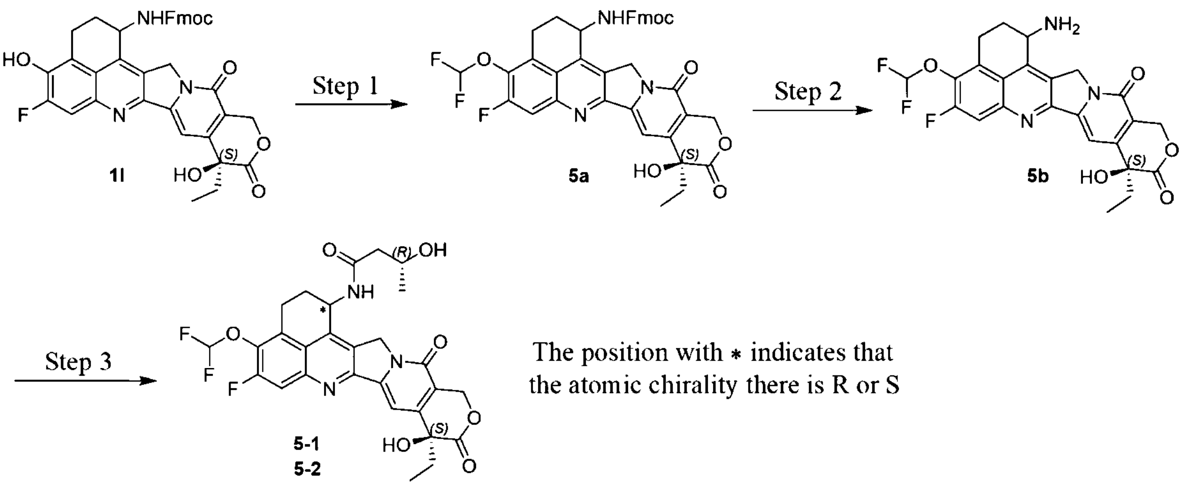


**1l** **5a** **5b** **5-1** **5-2**

Step 1 (9*H*-fluoren-9-yl)methyl((9S)-4-(difluoromethoxy)-9-ethyl-5-fluoro-9-hydroxy-10,13-dioxo-2,3,9,10,13,15-hexahydro-1*H*,12*H*-benzo[de]pyrano[3',4',6,7]indolizino[1,2-*b*]quinolin-1-yl)carbamate **5a**

The compound **11** (200 mg, 0.30 mmol) was dissolved in DMF (5 mL), and cesium carbonate (198 mg, 0.61 mmol) and diethyl (bromodifluoromethyl)phosphonate (121 mg, 0.45 mmol) were added in an ice bath. The mixture was stirred for reaction at 0°C for 1 h. After the reaction was completed, water was added, and the system was extracted with ethyl acetate. The organic phase was washed with saturated brine, collected, dried over anhydrous sodium sulfate, and spin-dried to remove the solvent, thus giving a crude product. The crude product was separated and purified by column chromatography to give the title compound **5a** (200 mg, yield: 93%).

MS m/z (ESI): 711.2 (M+1) $^+$.

Step 2 (9*S*)-1-amino-4-(difluoromethoxy)-9-ethyl-5-fluoro-9-hydroxy-1,2,3,9,12,15-hexahydro-10*H*,13*H*-benzo[*de*]pyrano[3',4',6,7]indolizino[1,2-*b*]quinoline-10,13-dione **5b**

The compound **5a** (140 mg, 0.20 mmol) was dissolved in N,N-dimethylformamide (1 mL), and diethylamine (0.1 mL) was added. The mixture was stirred for reaction at room temperature for 1 h. After the reaction was completed, the reaction mixture was spin-dried under vacuum using an oil pump to remove the solvent, thus giving the crude title compound **5b** (90 mg). The crude product was directly used in the next reaction step without purification.

MS m/z (ESI): 488.1 (M+1) $^+$.

Step 3

(*R*)-*N*-((1*S*,9*S*)-4-(difluoromethoxy)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-4-(difluoromethoxy)-9-ethyl-5-fluoro-9-hydroxy-10,13-dioxo-2,3,9,10,13,15-hexahydro-1*H*,12*H*-benzo[*de*]pyrano[3',4',6,7]indolizino[1,2-*b*]quinolin-1-yl)-3-hydroxybutanamide

The crude product **5b** (90 mg, 0.18 mmol) was dissolved in *N,N*-dimethylformamide (1 mL), and (*R*)-3-hydroxybutyric acid (23 mg, 0.22 mmol), 2-(7-azobenzotriazole)-*N,N,N',N'*-tetramethyluronium hexafluorophosphate (105 mg, 0.28 mmol), and N,N-diisopropylethylamine (48 mg, 0.37 mmol) were added. The mixture was stirred for reaction at room temperature for 1 h. After the reaction was completed, the mixture was spin-dried to remove the solvent, thus giving a crude product, which was purified by preparative high performance liquid chromatography (GILSON Prep LC with UV detector, chromatographic column: Xtimate C18 250*30 mm, 10 μm; mobile phase 1: water (0.1% FA); mobile phase 2: acetonitrile; 15-min gradient, gradient proportion: acetonitrile phase 37%-47%, flow rate: 50 mL/min) to give the title compound **5-1** (8.6 mg, yield: 16%) and the title compound **5-2** (7.8 mg, yield: 14.7%).

Mono-configurational compound **5-1** (short retention time):

MS m/z (ESI): 574.1 $(M+1)^+$.

$^1$H NMR (400 MHz, DMSO-$d_6$) δ 8.49 (d, *J* = 8.4 Hz, 2H), 8.06 (d, *J* = 11.2 Hz, 1H), 7.34 (s, 1H),7.31 (t, *J* = 72.8 Hz, 1H) 6.54 (s, 1H), 5.63 - 5.54 (m, 1H), 5.43 (s, 2H), 5.32 - 5.16 (m, 2H), 4.68 (d, *J* = 4.4 Hz, 1H), 4.05 (s, 1H), 3.23 (d, *J* = 5.6 Hz, 1H), 2.36 - 2.19 (m, 2H), 2.19 - 2.05 (m, 2H), 1.93 - 1.78 (m, 2H), 1.10 (d, *J* = 6.4 Hz, 3H), 0.88 (t, *J* = 7.2 Hz, 3H).

Mono-configurational compound **5-2** (long retention time):

MS m/z (ESI): 574.1 $(M+1)^+$.

$^1$H NMR (400 MHz, DMSO-$d_6$) δ 8.52 (d, *J* = 8.8 Hz, 2H), 8.10 - 8.04 (m, 1H), 7.35 (s, 1H), 7.32 (t, *J* = 72.8 Hz, 1 H) 6.55 (s, 1H), 5.66 - 5.58 (m, 1H), 5.43 (d, *J* = 16.8 Hz, 2H), 5.26 (t, *J* = 12.0 Hz, 2H), 4.66 (d, *J* = 4.4 Hz, 1H), 4.14 - 4.01 (m, 1H), 3.25 (s, 1H), 2.34 - 2.20 (m, 2H), 2.16 - 2.07 (m, 2H), 1.92 - 1.80 (m, 2H), 1.10 (d, *J* = 6.4 Hz, 3H), 0.89 (t, *J* = 7.2 Hz, 3H).

## Example 6

(*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4',6,7]indolizino[1,2-*b*]quinolin-1-yl)-3-hydroxybutanamide

*(R)-N-((1R,10S)-10-ethyl-6-fluoro-10-hydroxy-5-methyl-11,14-dioxo-1,2,3,4,10,11,14,16*-octahydro-13*H*-cyclohepta[3',4',6,7]indolizino[1,2-*b*]quinolin-1-yl)-3-hydroxybutanamide

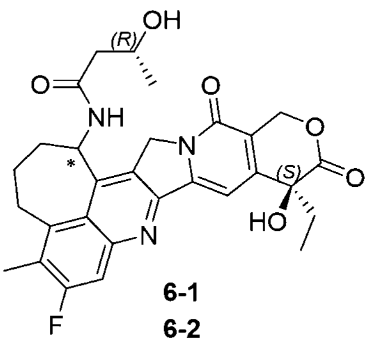

**6-1**
**6-2**

The position with * indicates that the atomic chirality there is R or S

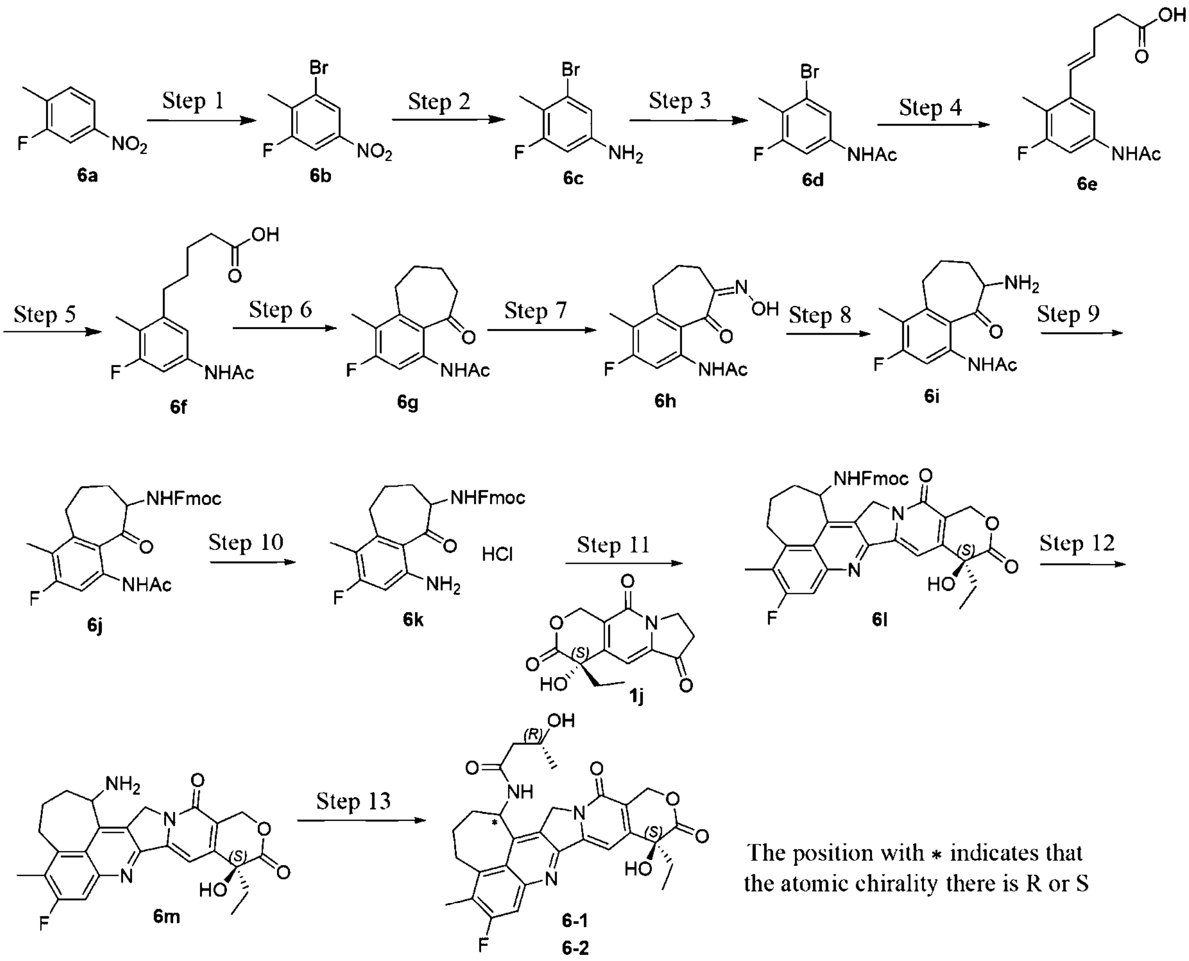


6a 6b 6c 6d 6e 6f 6g 6h 6i 6j 6k 1j 6l 6m 6-1 6-2

Step 1 1-bromo-3-fluoro-2-methyl-5-nitrobenzene **6b**

2-fluoro-1-methyl-4-nitrobenzene **6a** (20.0 g, 0.13 mol) was dissolved in n-heptane (50 mL), concentrated sulfuric acid (50 mL) was added, the mixture was heated to 60°C, *N*-bromosuccinimide (35.6 g, 0.20 mol) was added batchwise at this temperature, and the mixture was kept for reaction at 60°C for 2 h. The reaction mixture cooled to room temperature was added dropwise to ice water, and extracted with toluene. The organic phases were combined, successively washed with a sodium sulfite solution, water, and saturated brine, dried over anhydrous sodium sulfate, and then concentrated under reduced pressure, to give the title compound **6b** (30.0 g), which was directly used in the next reaction step without purification.

MS m/z (ESI): 233.9 $(M+1)^+$.

$^1$H NMR (400 MHz, CDCl3) δ 8.29-8.23 (m, 1H), 7.88 (dd, 1H), 2.44 (d, 3H).

Step 2 3-bromo-5-fluoro-4-methylaniline **6c**

The compound **6b** (30.0 g, 0.13 mol) was dissolved in methanol (200 mL), platinum-carbon (3.0 g, 5% content) was added, and the mixture was, after hydrogen replacement, kept for reaction in a hydrogen atmosphere at room temperature for 16 h. After the reaction was completed, the reaction mixture was filtered, and the filtrate was concentrated to give the title compound **6c** (20.0 g, yellow oil), which was directly used in the next reaction step without purification.

MS m/z (ESI): 204.0 $(M+1)^+$.

Step 3 *N*-(3-bromo-5-fluoro-4-methylphenyl)acetamide **6d**

3-bromo-5-fluoro-4-methylaniline **6c** (20.0 g, 0.10 mol) was dissolved in dichloromethane (100 mL) under an ice bath condition, triethylamine (20.2 g, 0.20 mol) and acetyl chloride (11.8 g, 0.15 mol) were added, and the mixture was kept for reaction under an ice bath condition for 3 h. After the reaction was completed, water was added to the reaction mixture to quench the reaction, and the mixture was extracted with ethyl acetate. The organic phases were combined, dried over

anhydrous sodium sulfate, and concentrated under reduced pressure to give a crude product, which was slurried with a mixed solvent of dichloromethane and petroleum ether (V/V=10:1) to give the title compound **6d** (10.0 g, yield: 32%).
MS m/z (ESI): 246.0 (M+1)$^+$.

Step 4 (*E*)-5-(5-acetylamino-3-fluoro-2-methylphenyl)pent-4-enoic acid **6e**

*N*-(3-bromo-5-fluoro-4-methylphenyl)acetamide **6d** (10.0 g, 40.8 mmol) was dissolved in dioxane (40 mL) and water (10 mL), to which pent-4-enoic acid (6.1 g, 61.20 mmol), palladium acetate (0.7 g, 4.10 mmol), tris(o-methylphenyl) phosphorus (2.5 g, 8.20 mmol), and *N,N*-diisopropylethylamine (15.9 g, 122.01 mmol) were added. The reaction mixture was stirred for reaction at 100°C for 16 h. After the reaction was completed, water and dichloromethane were added, and the system was washed with saturated sodium bicarbonate solution 3 times. The aqueous phase was collected. The aqueous phase was adjusted with hydrochloric acid to have a pH of 2-3, and extracted with ethyl acetate 3 times. The organic phase was collected, dried over anhydrous sodium sulfate, and spin-dried to remove the solvent, thus giving the title compound **6e** (10 g). The product was directly used in the next reaction step without purification.
MS m/z (ESI): 266.1 (M+1)$^+$,
**[0222]** The synthetic route in Example 3 was used as the subsequent synthetic route, except that the intermediate **3d** in Example 3 was replaced with the intermediate **6e** (10 g, 0.04 mol). The final product was purified by preparative high performance liquid chromatography (Waters MS-triggered Prep-LC with SQD2 detector, chromatographic column: Xbridge C18 150*19 mm, 5 μm; mobile phase 1: water (0.1% TFA); mobile phase 2: acetonitrile; 15-min gradient, gradient proportion: acetonitrile phase 37%-47%, flow rate: 20 mL/min) to give the title products **6-1** (12.6 mg, yield: 14%) and **6-2** (17.2 mg, yield: 19%).
Mono-configurational compound **6-1** (short retention time):

MS m/z (ESI): 536 (M+1)$^+$.
$^1$H NMR (400 MHz, DMSO-$d_6$) $\delta$ 8.60 - 8.55 (m, 1H), 7.73 - 7.72 (m, 1H), 7.28 (s, 1H), 6.51 (s, 1H), 5.54 - 5.53 (m, 1H), 5.42 (s, 2H), 5.32 - 5.26 (m, 1H), 5.24 - 5.22 (m, 1H), 4.65 (s, 1H), 4.01 (s, 1H), 3.22 - 3.18 (m, 2H), 2.42 - 2.40 (m, 3H), 2.28 - 2.24 (m, 4H), 2.10 - 1.67 (m, 6H), 1.06 (d, *J* = 6.0Hz, 3H), 0.85 - 0.82 (m, 3H).

Mono-configurational compound **6-2** (long retention time):

MS m/z (ESI): 536 (M+1)$^+$.
$^1$H NMR (400 MHz, DMSO-$d_6$) $\delta$ 8.67 - 8.66 (m, 1H), 7.73 - 7.71 (m, 1H), 7.28 (s, 1H), 6.49 (s, 1H), 5.53 - 5.52 (m, 1H), 5.41 - 5.40 (m, 3H), 5.35 - 5.33 (m, 1H), 4.67 - 4.65 (m, 1H), 4.03 - 3.96 (m, 1H), 3.22 - 3.18 (m, 2H), 2.42 (s, 4H), 2.40 - 2.15 (m, 4H), 2.11 - 1.52 (m, 6H), 1.08 - 1.07 (m, 3H), 0.87 - 0.85 (m, 3H).

**Example 7**

(R)-N-((1S,9S)-9-ethyl-5-fluoro-9-hydroxy-10,13-dioxo-4-vinyl-2,3,9,10,13,15-hexahydro-1*H*,12*H*-benzo[*de*]pyrano [3',4',6,7]indolizino[1,2-*b*]quinolin-1-yl)-3-hydroxybutanamide

(*R*)-*N*-((1*R*,9*S*)-9-ethyl-5-fluoro-9-hydroxy-10,13-dioxo-4-vinyl-2,3,9,10,13,15-hexahydro-1*H*,12*H*-benzo[*de*]pyrano [3',4',6,7]indolizino[1,2-*b*]quinolin-1-yl)-3-hydroxybutanamide

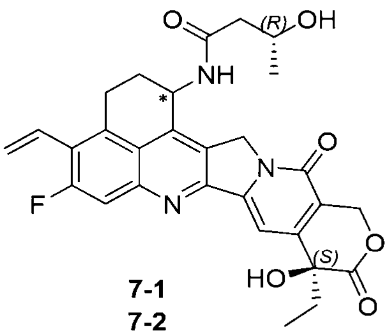

**7-1**
**7-2**

The position with * indicates that the atomic chirality there is R or S

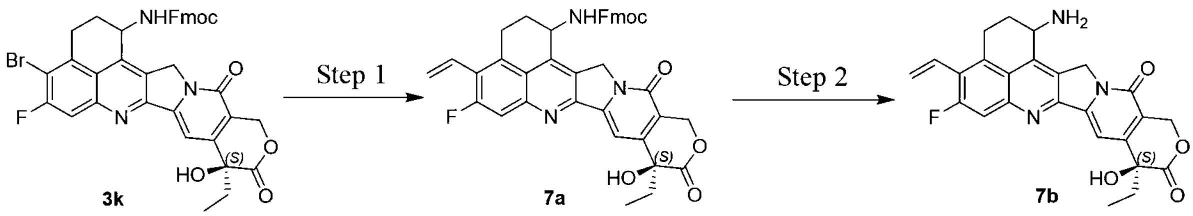


3k 7a 7b

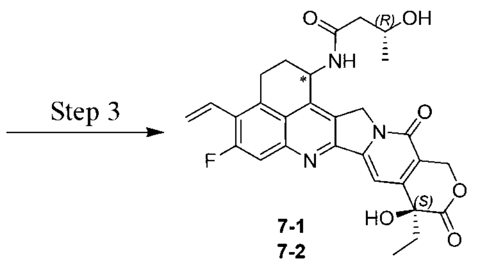


7-1
7-2

The position with * indicates that the atomic chirality there is R or S

Step 1 (9*H*-fluoren-9-yl)methyl((9*S*)-9-ethyl-5-fluoro-9-hydroxy-10,13-dioxo-4-vinyl-2,3,9,10,13,15-hexahydro-1H,12H-benzo[de]pyrano[3',4',6,7]indolizino[1,2-b]quinolin-1-yl)carbamate **7a**

The compound 3k (140 mg, 0.20 mmol) and potassium vinyltrifluoroborate (54 mg, 0.40 mmol) were dissolved in dioxane (16 mL) and water (4 mL), and potassium phosphate (128 mg, 0.60 mmol) and methanesulfonato(2-dicyclohexylphosphino-2',6'-diisopropoxy-1,1'-biphenyl)(2-amino-1,1'-biphenyl-2-yl)palladium (II) (34 mg, 0.04 mmol) were added. Nitrogen replacement was conducted. The reaction mixture was stirred at 100°C for 5 h. After the reaction was completed, water (20 mL) was added into the reaction mixture. The mixture was extracted with dichloromethane (15 mL×3). The organic phases were combined, washed with saturated brine, dried, and concentrated. The resulting residue was purified by silica gel column chromatography with eluent system B, to give the title compound **7a** (80 mg, yield: 60%).
MS m/z (ESI): 670.2 $(M+1)^+$,

Step 2 Preparation of (*S*)-1-amino-9-ethyl-5-fluoro-9-hydroxy-4-vinyl-1,2,3,9,12,15-hexahydro-10*H*,13*H*-benzo[*de*]pyrano[3',4',6,7]indolizino[1,2-*b*]quinoline-10,13-dione

The compound **7a** (80 mg, 0.12 mmol) was dissolved in N,N-dimethylformamide (10 mL), diethylamine (1 mL) was added, and the reaction mixture was stirred at room temperature for 1 h. After the reaction was completed, the mixture was vacuum concentrated, slurried with ethyl acetate, and filtered to give the title compound **7b** (30 mg, yield: 51%).
MS m/z (ESI): 448.2 $(M+1)^+$,

Step 3

(*R*)-*N*-((1*S*,9*S*)-9-ethyl-5-fluoro-9-hydroxy-10,13-dioxo-4-vinyl-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-9-ethyl-5-fluoro-9-hydroxy-10,13-dioxo-4-vinyl-2,3,9,10,13,15-hexahydro-1*H*,12*H*-benzo[*de*]pyrano[3',4',6,7]indolizino[1,2-*b*]quinolin-1-yl)-3-hydroxybutanamide

The compound **7b** (30 mg, 0.07 mmol) and (*R*)-3-hydroxybutyric acid (8 mg, 0.08 mmol) were dissolved in *N,N*-dimethylformamide (5 mL), and *N,N*-diisopropylethylamine (18.2 mg, 0.14 mmol) and 2-(7-azabenzotriazole)-N,N,N',N'-tetramethyluronium hexafluorophosphate (39.9 mg, 0.11 mmol) were added. The reaction mixture was stirred at 25°C for 5 h. After the reaction was completed, the reaction mixture was concentrated, and purified by preparative high performance liquid chromatography (Waters MS-triggered Prep-LC with SQD2 detector, chromatographic column: Xbridge C18 150*19 mm, 5 μm; mobile phase 1: water (containing 0.1% formic acid); mobile phase 2: acetonitrile; 15-min gradient, gradient proportion: acetonitrile phase 43%-53%, flow rate: 20 mL/min) to give the title compound **7-1** (1.76 mg, yield: 10%) and the title compound **7-2** (2.31 mg, yield: 10%).

Mono-configurational compound **7-1** (short retention time):

MS m/z (ESI): 534.2 $(M+1)^+$.
$^1$H NMR (400 MHz, DMSO-$d_6$) δ 8.51 (d, *J* = 8.8 Hz, 1H), 8.48 (s, 1H), 7.84 (d, *J* = 12.0 Hz, 1H), 7.32 (s, 1H), 6.97 (dd, *J*

= 18.0, 12.0 Hz, 1H), 6.58 (s, 1H), 5.91 - 5.77 (m, 2H), 5.61 - 5.50 (m, 1H), 5.43 (s, 2H), 5.22 (q, *J* = 19.2 Hz, 2H), 4.72 (s, 1H), 4.04 (dd, *J* = 12.4, 6.4 Hz, 1H), 3.27 (d, *J* = 4.8 Hz, 1H), 2.30 - 2.20 (m, 2H), 2.11 (t, J = 9.2 Hz, 2H), 1.90 - 1.80 (m, 2H), 1.09 (d, *J* = 6.4 Hz, 3H), 0.87 (t, *J* = 7.2 Hz, 3H).

Mono-configurational compound **7-2** (long retention time):

MS m/z (ESI): 534.2 (M+1) $^+$.

$^1$H NMR (400 MHz, DMSO-$d_6$) δ 8.55 (d, *J* = 8.8 Hz, 1H), 8.47 (s, 1H), 7.85 (d, *J* = 12.0 Hz, 1H), 7.32 (s, 1H), 6.97 (dd, *J* = 18.0, 12.0 Hz, 1H), 6.62 (s, 1H), 5.82 (dd, *J* = 24.8, 14.0 Hz, 2H), 5.57 (dt, *J* = 8.8, 4.4 Hz, 1H), 5.43 (d, *J* = 1.2 Hz, 2H), 5.24 (s, 2H), 4.72 (s, 1H), 4.03 (dt, *J* = 12.8, 6.4 Hz, 1H), 3.27 (d, *J* = 5.4 Hz, 1H), 2.23 (ddd, *J* = 18.4, 13.2, 6.4 Hz, 2H), 2.08 (dd, *J* = 26.4, 10.0 Hz, 2H), 1.87 (tt, *J* = 14.0, 7.2 Hz, 2H), 1.08 (d, *J* = 6.0 Hz, 3H), 0.87 (s, 3H).

**Example 8**

(*R*)-*N*-((1*S*,9S)-9-ethyl-5-fluoro-9-hydroxy-10,13-dioxo-4-(2,2,2-trifluoroethoxy)-2,3,9,10,13,15-hexahydro-1*H*,12*H*-benzo[*de*]pyrano[3',4',6,7]indolizino[1,2-*b*]quinolin-1-yl)-3-hydroxybutanamide

(*R*)-*N*-((1*R*,9*S*)-9-ethyl-5-fluoro-9-hydroxy-10,13-dioxo-4-(2,2,2-trifluoroethoxy)-2,3,9,10,13,15-hexahydro-1*H*,12*H*-benzo[de]pyrano[3',4',6,7]indolizino[1,2-*b*]quinolin-1-yl)-3-hydroxybutanamide

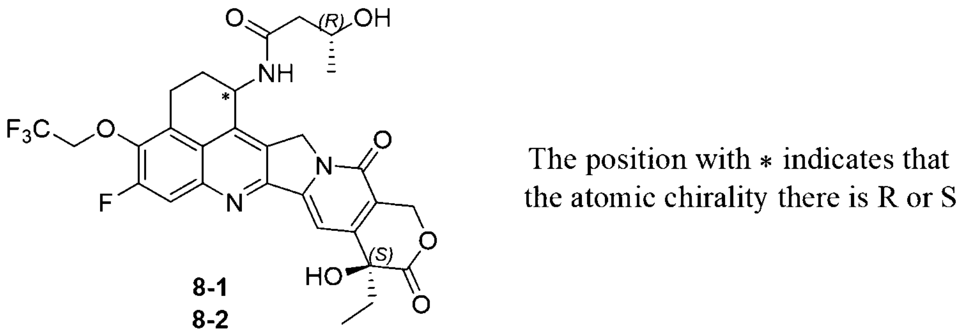


8-1
8-2

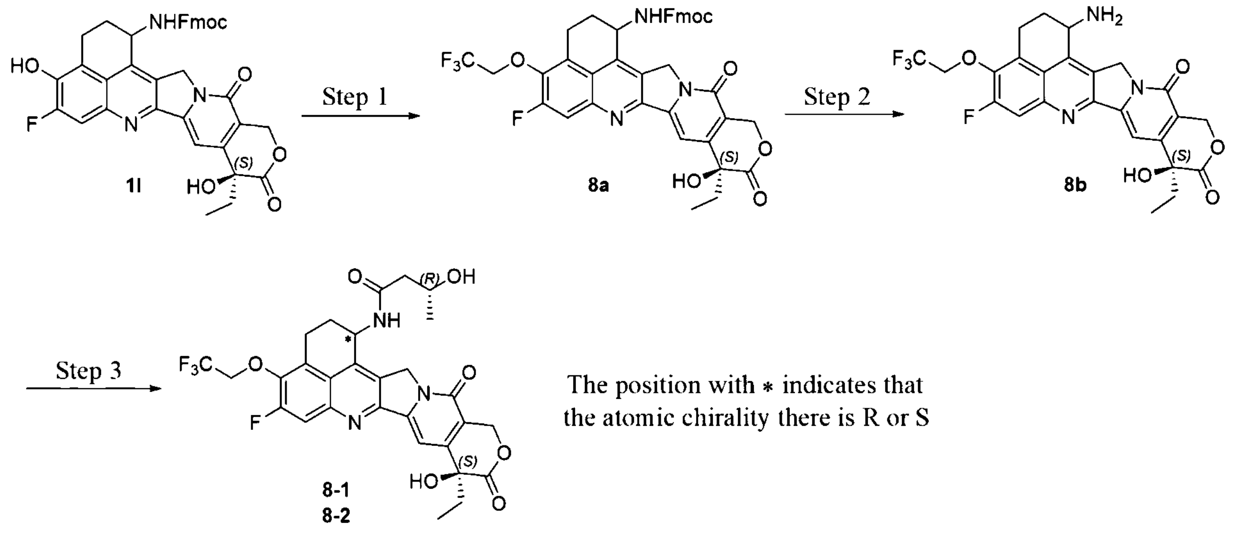


1l
8a
8b
8-1
8-2

Step 1 (9*H*-fluoren-9-yl)methyl((9*S*)-9-ethyl-5-fluoro-9-hydroxy-10,13-dioxo-4-(2,2,2-trifluoroethoxy)-2,3,9,10,13,15-hexahydro-1*H*,12*H*-benzo[de]pyrano[3',4',6,7]indolizino[1,2-*b*]quinolin-1-yl)carbamate **8a**

The compound **11** (200 mg, 0.30 mmol) was dissolved in *N,N*-dimethylformamide (3 mL), and 2,2,2-trifluoroethyl trifluoromethanesulfonate (141 mg, 0.60 mmol) and cesium carbonate (296 mg, 0.90 mmol) were slowly added. The reaction mixture was stirred at room temperature for 10 min. After the reaction was completed, the mixture was diluted with

water (10 mL), and extracted with ethyl acetate (10 mL×3). The organic phases were combined and concentrated. The resulting residue was purified by silica gel column chromatography with eluent system B, to give the title compound **8a** (180 mg, yield: 75%).

MS m/z (ESI): 742.2 (M+H) +.

Step 2 (9S)-1-amino-9-ethyl-5-fluoro-9-hydroxy-4-(2,2,2-trifluoroethoxy)-1,2,3,9,12,15-hexahydro-10*H*,13*H*-benzo[*de*]pyrano[3',4',6,7]indolizino[1,2-*b*]quinoline-10,13-dione **8b**

The compound **8a** (180 mg, 0.24 mmol) was dissolved in N,N-dimethylformamide (3 mL), to which diethylamine (35 mg, 0.48 mmol) was added. The reaction mixture was kept for reaction at room temperature for 1 h. After the reaction was completed, the reaction mixture was concentrated under reduced pressure to give a crude product **8b** (100 mg), which was directly used in the next reaction step without purification.

MS m/z (ESI): 520.1 (M+H) +.

Step 3

(*R*)-*N*-((1*S*,9*S*)-9-ethyl-5-fluoro-9-hydroxy-10,13-dioxo-4-(2,2,2-trifluoroethoxy)-2,3,9,10,13,15-hexahydro-1*H*,12*H*-benzo[de]pyrano[3',4',6,7]indolizino[1,2-*b*]quinolin-1-yl)-3-hydroxybutanamide

(*R*)-*N*-((1*R*,9*S*)-9-ethyl-5-fluoro-9-hydroxy-10,13-dioxo-4-(2,2,2-trifluoroethoxy)-2,3,9,10,13,15-hexahydro-1*H*,12*H*-benzo[de]pyrano[3',4',6,7]indolizino[1,2-*b*]quinolin-1-yl)-3-hydroxybutanamide

The compound **8b** (50 mg, 0.09 mmol) was dissolved in N,Ndimethylformamide (2 mL), and (*R*)-3-hydroxybutyric acid (15 mg, 0.14 mmol), 2-(7-azabenzotriazole)-*N,N,N',N'*-tetramethyluronium hexafluorophosphate (73 mg, 0.19 mmol), and *N,N*-diisopropylethylamine (37 mg, 0.28 mmol) were added. The mixture was stirred for reaction at room temperature for 1 h. After the reaction was completed, the mixture was concentrated under reduced pressure to give a crude product, which was purified by preparative high performance liquid chromatography (GILSON Prep LC with UV detector, chromatographic column: Xtimate C18 150*19 mm, 10 μm; mobile phase 1: water (containing 0.1% FA); mobile phase 2: acetonitrile; 15-min gradient, gradient proportion: acetonitrile phase 38%-48%, flow rate: 50 mL/min) to give the title compound **8-1** (2.5 mg, yield: 4.6%) and the title compound **8-2** (3.5 mg, yield: 6.4%).

Mono-configurational compound **8-1** (short retention time):

MS m/z (ESI): 606.2 (M+1) +.
$^1$H NMR (400 MHz, DMSO-$d_6$) δ 8.43 (d, J = 8.8 Hz, 1H), 7.98 (d, J = 12.0 Hz, 1H), 7.31 (s, 1H), 6.52 (s, 1H), 5.59 - 5.53 (m, 1H), 5.42 (s, 2H), 5.23 (s, 2H), 4.93 - 4.85 (m, 2H), 4.66 (d, J = 4.8 Hz, 1H), 4.08 - 4.03 (m, 1H), 3.21 (d, J = 6.0 Hz, 2H), 2.34 - 2.27 (m, 1H), 2.22 (d, J = 8.4 Hz, 1H), 2.10 (dd, J = 12.4, 5.6 Hz, 2H), 1.89 - 1.81 (m, 2H), 1.10 (d, J = 6.4 Hz, 3H), 0.90 - 0.84 (m, 3H).

Mono-configurational compound **8-2** (long retention time):

MS m/z (ESI): 606.2 (M+1) +.
$^1$H NMR (400 MHz, DMSO-$d_6$) δ 8.45 (d, J = 8.8 Hz, 1H), 7.98 (d, J = 12.4 Hz, 1H), 7.32 (s, 1H), 6.52 (s, 1H), 5.63 - 5.55 (m, 1H), 5.43 (s, 2H), 5.33 - 5.18 (m, 2H), 4.88 (dd, J = 18.0, 9.2 Hz, 2H), 4.65 (d, J = 4.4 Hz, 1H), 4.09 - 4.00 (m, 1H), 3.24 - 3.12 (m, 2H), 2.28 (dd, J = 13.6, 7.6 Hz, 1H), 2.19 (dd, J = 13.6, 5.6 Hz, 1H), 2.09 (dd, J = 16.8, 4.8 Hz, 2H), 1.93 - 1.78 (m, 2H), 1.09 (d, J = 6.4 Hz, 3H), 0.94 - 0.78 (m, 3H).

**Example 9**

*(S)-9-ethyl-4,9-dihydroxy-1,2,3,9,12,15-hexahydro-10H,13H-*benzopyrano[3',4',6,7]indolizino[1,2-*b*]quinoline-10,13-dione **9**

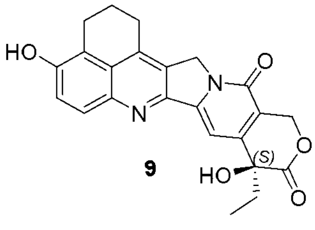

9

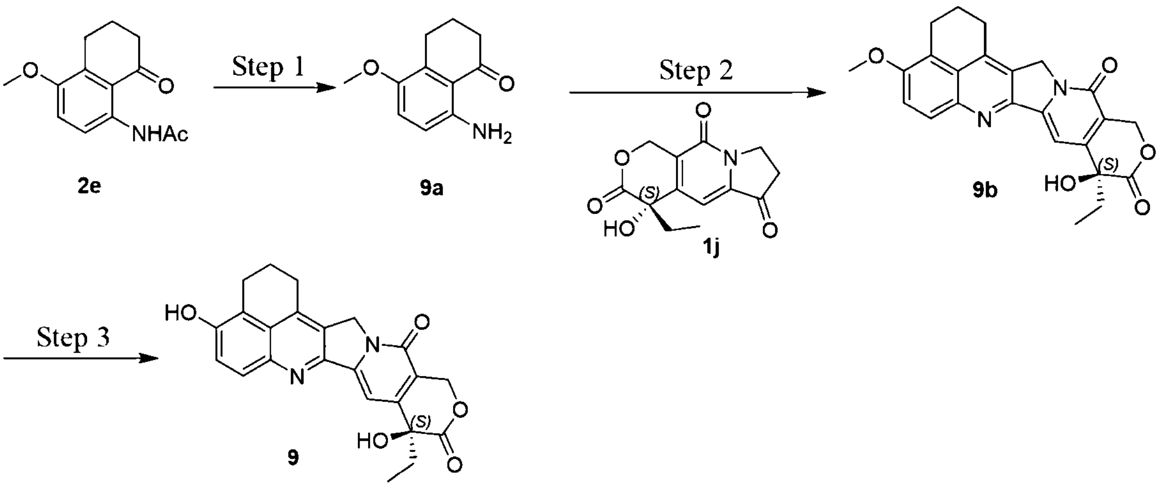


2e 9a 1j 9b 9

Step 1 8-amino-5-methoxy-3,4-dihydronaphthalen-1(2*H*)-one **9a**

The compound 2e (500 mg, 2.14 mmol) was dissolved in a mixed solvent of methanol and concentrated hydrochloric acid (36 mL, V/V=5:1). The reaction mixture was kept for reaction at 60°C for 2 h. After the reaction was completed, the reaction mixture was directly concentrated, to give the title compound **9a** (400 mg, yield: 88%).

MS m/z (ESI): 192.1 $(M+1)^+$.

Step 2 (*S*)-9-ethyl-9-hydroxy-4-methoxy-1,2,3,9,12,15-hexahydro-10*H*,3*H*-benzopyrano[3',4',6,7]indolizino[1,2-*b*]quinoline-10,13-dione **9b**

The compound **9a** (400 mg, 2.09 mmol) was dissolved in toluene (15 mL), and the compound **1j** (661 mg, 2.51 mmol) was added. The reaction mixture was stirred at 110°C for 16 h. After the reaction was completed, water (20 mL) was added into the reaction mixture. The mixture was extracted with dichloromethane (15 mL×3). The organic phases were combined, washed with saturated brine, dried, and concentrated. The purification was performed by silica gel column chromatography with eluent system B to give the title compound **9b** (698 mg, yield: 80%).

MS m/z (ESI): 419.1 $(M+1)^+$.

Step 3 (*S*)-9-ethyl-4,9-dihydroxy-1,2,3,9,12,15-hexahydro-10*H*,13*H*-benzopyrano[3',4',6,7]indolizino[1,2-*b*]quinoline-10,13-dione **9**

The compound **9b** (100 mg, 0.24 mmol) was dissolved in hydrobromic acid (30 mL). The reaction mixture was kept for reaction at 100°C for 2 h. After the reaction was completed, the reaction mixture was directly concentrated, and then purified by preparative high performance liquid chromatography (Waters MS triggered Prep-LC with Acquity QDA detector, chromatographic column: Welch C18 250×21.2 mm, 10 μm; mobile phase 1: water (containing 0.1% aqueous ammonia); mobile phase 2: acetonitrile; 15-min gradient, gradient proportion: acetonitrile phase 10%-30%, flow rate: 25 mL/min) to give the title compound **9** (19.4 mg, yield: 20%).

MS m/z (ESI): 405.1 $(M+1)^+$.

$^1$H NMR (400 MHz, $CD_3OD$) δ 7.79 - 7.71 (m, 2H), 7.29 (d, *J* = 9.0 Hz, 1H), 5.01 (d, *J* = 4.0 Hz, 2H), 4.95 (d, *J* = 13.6 Hz, 2H), 3.01 - 2.86 (m, 4H), 2.43 - 2.35(m, 1H), 2.28 - 2.19 (m, 1H),2.06 - 2.00 (m, 2H), 1.10 - 1.00 (m, 3H).

**Example 10**

(1*S*,3*R*)-N-((1*S*,10*S*)-10-ethyl-6-fluoro-10-hydroxy-5-methyl-11,14-dioxo-1,2,3,4,10,11,14,16-octahydro-13*H*-cyclohepta[*de*]pyrano[3',4':6,7]indolizino[1,2-*b*]quinolin-1-yl)-3-hydroxycyclobutane-1-carboxamide

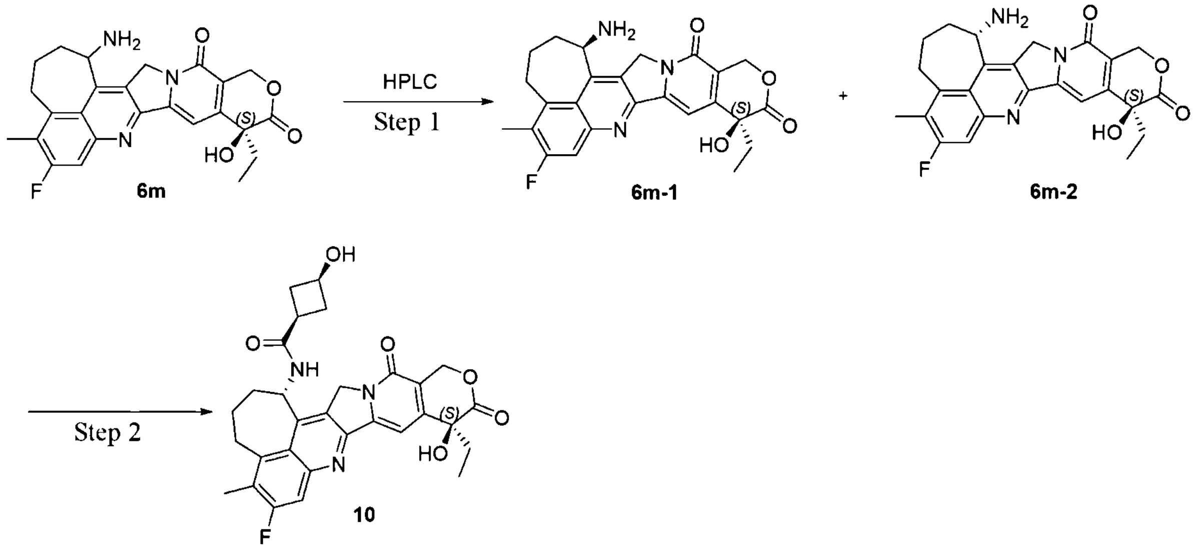


6m 6m-1 6m-2 10

Step 1

(1*R*,10*S*)-1-amino-10-ethyl-6-fluoro-10-hydroxy-5-methyl-2,3,4,10,13,16-hexahydro-14*H*-cyclohepta[3',4':6,7]indolizino[1,2-*b*]quinoline-11,14-(1*H*)-dione

*(1S, 1 0S)-1-amino-1 0-ethyl-6-fluoro-1 0-hydroxy-5-methyl-2,3,4, 1 0, 13, 16-hexahydro-14H*-cyclohepta[3',4':6,7]indolizino[1,2-*b*]quinoline-11,14-(1*R*)-dione

Compound **6m** (700 mg) was purified by preparative high performance liquid chromatography (GILSON Prep LC with UV detector, chromatographic column: Xbridge 5 μm C18 150x30 mm; mobile phase 1: water (0.1% TFA); mobile phase 2: acetonitrile; 15-min gradient, gradient proportion: acetonitrile phase 22%-32%, flow rate: 50 mL/min), to give the compounds **6m-1** (290 mg, short retention time) and **6m-2** (300 mg, long retention time).
MS m/z (ESI): 450.2 $(M+H)^+$

Step 2

(1*S*,3*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta[*de*]pyrano[3',4':6,7]indolizino[1,2-*b*]quinolin-1-yl)-3-hydroxycyclobutane-1-carboxamide **10**

The compound **6m-2** (30 mg, 0.06 mmol) was dissolved in N,N-dimethylformamide (3 mL), and (1*S*,3*S*)-3-hydroxycyclobutane-1-carboxylic acid (8.5 mg, 0.07 mmol), 2-(7-azabenzotriazole)-*N,N,N',N'*-tetramethyluronium hexafluorophosphate (38 mg, 0.10 mmol), and *N,N*-diisopropylethylamine (17.2 mg, 0.13 mmol) were added. The reaction mixture was stirred at room temperature for 1 h. After the reaction was completed, the reaction mixture was diluted with water (10 mL), and extracted with ethyl acetate (10 mL×3). The organic phases were combined, washed with saturated brine, dried, and concentrated to give a crude product. The crude product was purified by preparative high performance liquid chromatography (GILSON Prep LC with UV detector, chromatographic column: Xbridge 5 μm C18 150x30 mm; mobile phase 1: water (containing 0.1% TFA); mobile phase 2: acetonitrile; 15-min gradient, gradient proportion: acetonitrile phase 30%-95%, flow rate: 50 mL/min) to give the compound **10** (2 mg, yield: 5%).
MS m/z (ESI): 548.2 $(M+H)^+$.
$^1H$ NMR (400 MHz, $CD_3OD$) δ 8.71 (d, 1H), 7.57-7.48 (m, 2H), 5.65 (s, 1H), 5.55 (d, 1H), 5.35 (dd, 2H), 5.10 (d, 1H), 4.13 (dd, 1H), 2.73 (dd, 1H), 2.61-2.33 (m, 8H), 2.20-2.08 (m, 4H), 1.96 (dd, 4H), 1.02-0.97 (m, 3H).

**Example 11**

*(R)-2-cyclopropyl-N-((1S,10S)-10-ethyl-6-fluoro-10-hydroxy-5-methyl-11,14-dioxo*-1,2,3,4,10,11,14,16-octahydro-13*H*-cyclohepta[*de*]pyrano[3',4':6,7]indolizino[1,2-*b*]quinolin-1-yl)-2-hydroxyacetamide

(*S*)-2-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[*de*]pyrano[3',4':6,7]indolizino[1,2-b]quinolin-1-yl)-2-hydroxyacetamide

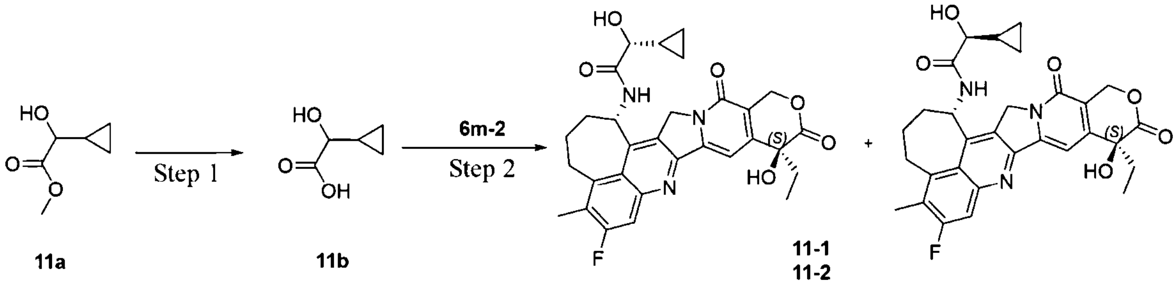


**11a** **11b** **11-1** **11-2**

Step 1 2-cyclopropyl-2-hydroxyacetic acid **11b**

Methyl 2-cyclopropyl-2-hydroxyacetate **11a** (50 mg, 0.38 mmol) was dissolved in tetrahydrofuran (6 mL), and water (0.5 mL) and lithium hydroxide (27 mg, 1.15 mmol) were added. The mixture was stirred for reaction at room temperature for 1 h. After the reaction was completed, the mixture was spin-dried to remove the solvent, thus giving the crude product 2-cyclopropyl-2-hydroxyacetic acid **11b** (50 mg, white solid), which was directly used in the next reaction step without purification.

MS m/z (ESI): 117.1 $(M+H)^+$.

Step 2

(*R*)-2-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-2-hydroxyacetamide

(*S*)-2-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-2-hydroxyacetamide

The compound **6m-2** (50 mg, 0.11 mmol) was dissolved in N,N-dimethylformamide (2 mL), and 2-cyclopropyl-2-hydroxyacetic acid **11b** (44 mg, 0.38 mmol), *N,N*-diisopropylethylamine (29 mg, 0.22 mmol), and 2-(7-azabenzotriazole)-*N,N,N',N'*-tetramethyluronium hexafluorophosphate (63 mg, 0.17 mmol) were added. The mixture was stirred for reaction at room temperature for 15 min. After the reaction was completed, the system was purified by preparative high performance liquid chromatography (Waters MS-triggered Prep-LC with SQD2 detector, chromatographic column: Xbridge 5 μm C18 150x19 mm; mobile phase 1: water (containing 0.1% FA); mobile phase 2: acetonitrile; 10-min gradient, gradient proportion: acetonitrile phase 40%-50%, flow rate: 20 mL/min) to give compound **11-1** (2.3 mg, yield: 4%) and compound **11-2** (2.5 mg, yield: 4%).

Mono-configurational compound (compound with short retention time)

MS m/z (ESI): 548.2 $(M+H)^+$.
$^1$H NMR (400 MHz, DMSO-$d_6$) δ 8.49 (d, 1H), 7.74 (d, 1H), 7.28 (s, 1H), 6.50 (s, 1H), 5.56 (s, 1H), 5.49-5.40 (m, 4H), 5.29 (d, 1H), 3.61 (t, 1H), 3.22 (s, 2H), 2.42 (s, 3H), 2.12-1.63 (m, 6H), 1.11 (d, 1H), 0.87 (t, 3H), 0.47-0.27 (m, 4H).

Mono-configurational compound (compound with long retention time)

MS m/z (ESI): 548.2 $(M+H)^+$.
$^1$H NMR (400 MHz, DMSO-$d_6$) δ 8.43 (d, 1H), 7.74 (d, 1H), 7.28 (s, 1H), 6.51 (s, 1H), 5.54 (s, 1H), 5.42 (t, 4H), 5.31 (d, 1H), 3.68-3.53 (m, 1H), 3.22 (s, 2H), 2.42 (s, 3H), 2.12-1.66 (m, 6H), 1.10 (d, 1H), 0.87 (t, 3H), 0.48-0.28 (m, 4H).

**Example 12**

(1*S*,3*R*)-*N*-((1*S*,9*S*)-9-ethyl-5-fluoro-9-hydroxy-10,13-dioxo-4-vinyl-2,3,9,10,13,15-hexahydro-1*H*,12*H*-benzo[*d*]pyrano[3',4':6,7]indolizino[1,2-*b*]quinolin-1-yl)-3-hydroxycyclobutane-1-carboxamide

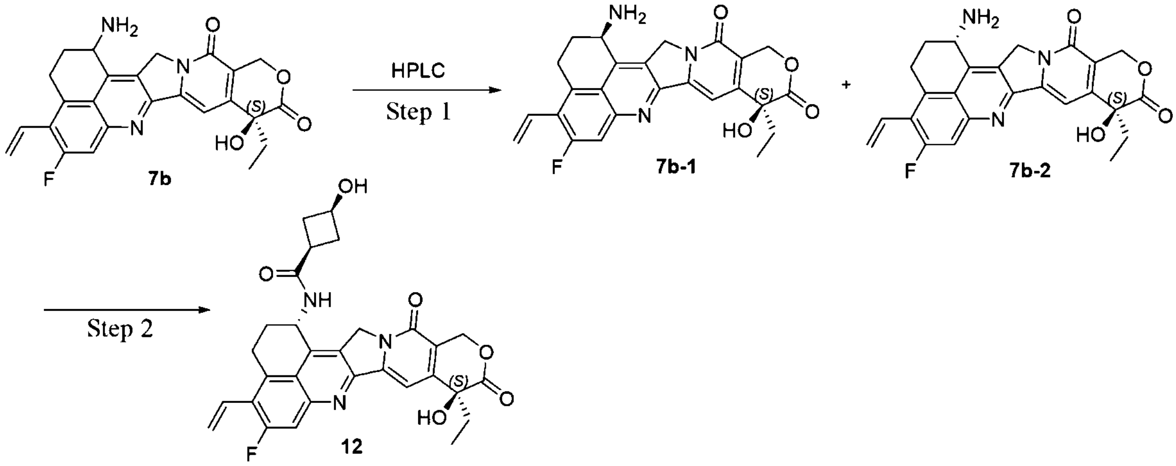


7b, 7b-1, 7b-2, 12

Step 1

(1*R*,9*S*)-1-amino-9-ethyl-5-fluoro-9-hydroxy-4-vinyl-1,2,3,9,12,15-hexahydro-10*H*,13*H*-benzo[*d*]pyrano[3',4':6,7]indolizino[1,2-*b*]quinoline-10,13-dione

(1*S*,9*S*)-1-amino-9-ethyl-5-fluoro-9-hydroxy-4-vinyl-1,2,3,9,12,15-hexahydro-10*H*,13*H*-benzo[*d*]pyrano[3',4':6,7]indolizino[1,2-*b*]quinoline-10,13-dione

The compound **7b** (500 mg) was purified by preparative high performance liquid chromatography (GILSON Prep LC with UV detector, chromatographic column: Xbridge 5 μm C18 150x30 mm; mobile phase 1: water (0.1% TFA); mobile phase 2: acetonitrile; 15-min gradient, gradient proportion: acetonitrile phase 22%-32%, flow rate: 50 mL/min), to give the compounds **7b-1** (180 mg, short retention time) and **7b-2** (195 mg, long retention time).

MS m/z (ESI): 448.2 $(M+H)^+$.

Step 2

(1*S*,3*R*)-*N*-((1*S*,9*S*)-9-ethyl-5-fluoro-9-hydroxy-10,13-dioxo-4-vinyl-2,3,9,10,13,15-hexahydro-1*H*,12*H*-benzo[*d*]pyrano[3',4':6,7]indolizino[1,2-*b*]quinolin-1-yl)-3-hydroxycyclobutane-1-carboxamide **12**

The compound **7b-2** (22 mg, 0.05 mmol) was dissolved in N,N-dimethylformamide (0.5 mL), and (1S,3S)-3-hydroxycyclobutane-1-carboxylic acid (7 mg, 0.06 mmol), 2-(7-azabenzotriazole)-*N,N,N',N'*-tetramethyluronium hexafluorophosphate (30 mg, 0.08 mmol), and *N,N*-diisopropylethylamine (13 mg, 0.10 mmol) were added. The mixture was stirred for reaction at room temperature for 15 min. After the reaction was completed, the reaction mixture was purified by preparative high performance liquid chromatography (Waters MS-triggered Prep-LC with SQD2 detector, chromatographic column: Xbridge 5 μm C18 150x19 mm; mobile phase 1: water (containing 0.1% FA); mobile phase 2: acetonitrile; 10-min gradient, gradient proportion: acetonitrile phase 45%-100%, flow rate: 20 mL/min) to give the compound **12** (3 mg, yield: 11%).

MS m/z (ESI): 546.2 $(M+H)^+$.

$^1H$ NMR (400 MHz, $CD_3OD$) δ 8.50 (s, 1H), 7.71 (d, 1H), 7.64 (s, 1H), 6.95 (dd, 1H), 5.83 (dd, 2H), 5.67-5.61 (m, 1H), 5.57 (d, 1H), 5.39 (d, 1H), 5.24 (d, 2H), 4.16-4.05 (m,1H), 3.45-3.35 (m, 2H), 2.73-2.42 (m, 4H), 2.29-2.17 (m, 4H), 2.01-1.91 (m, 2H), 1.00 (t, 3H).

**Example 13**

*N*-((7*S*,15*R*)-7-benzyl-17-(((1*S*,10*S*)-10-ethyl-6-fluoro-10-hydroxy-5-methyl-11,14-dioxo-1,2,3,4,10,11,14,16-octahydro-13*H*-cyclohepta[*de*]pyrano[3',4':6,7]indolizino[1,2-*b*]quinolin-1-yl)amino)-15-methyl-2,5,8,11,17-pentaoxo-14-oxo-3,6,9,12-tetraazaheptadecyl)-6-(2-(methylsulfonyl)pyrimidin-5-yl)hexy-5-nylamide **13**

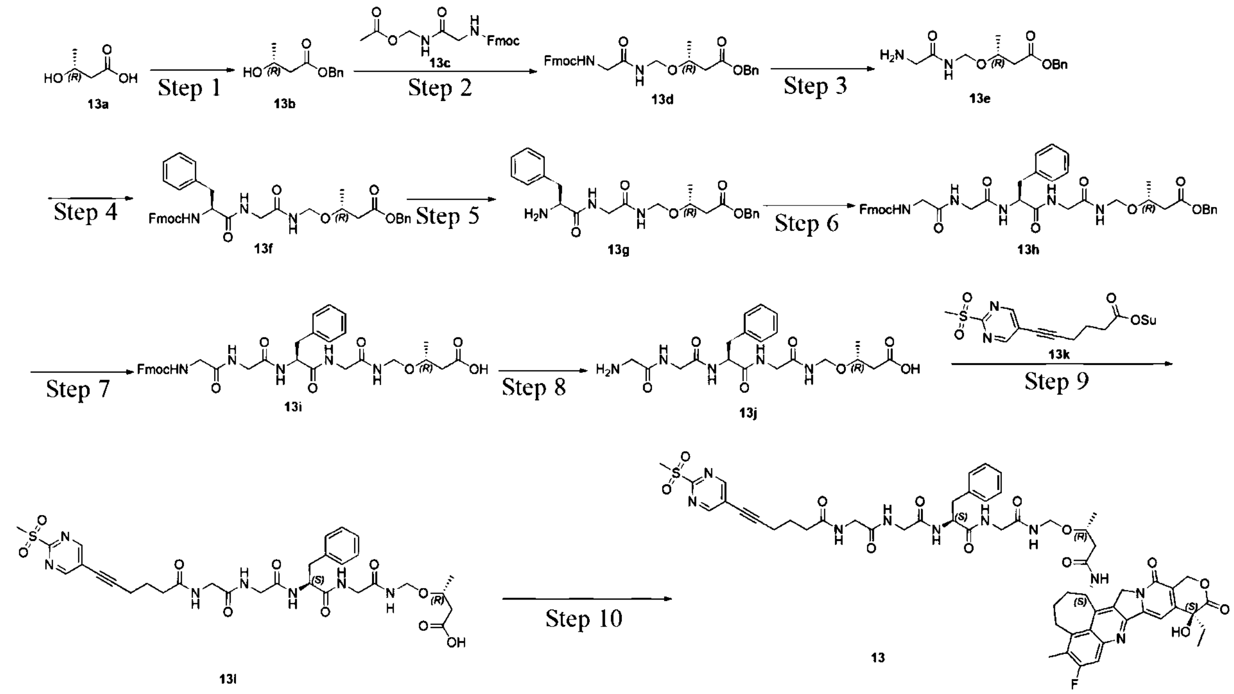


Step 1 Benzyl (*R*)-3-hydroxybutyrate**13b**

(*R*)-3-hydroxybutyric acid **13a** (50 g, 0.48 mol) was dissolved in N,N-dimethylformamide (100 mL), and benzyl bromide (90 g, 0.52 mol) and cesium carbonate (312.8 g, 0.96 mol) were added. The reaction mixture was left to react at room temperature for 12 h. After the reaction was completed, water (1000 mL) was added into the reaction mixture, and the mixture was extracted with ethyl acetate (100 mL x 3). The organic phases were combined, washed with saturated brine, dried, and concentrated. The crude product was purified by silica gel column chromatography with system B to give the title compound **13b** (40 g, yield: 40%).

MS m/z (ESI): 217.1 $(M+Na)^+$.

Step 2 Benzyl (*R*)-1-(9*H*-fluoren-9-yl)-10-methyl-3,6-dioxo-2,9-dioxa-4,7-diazadodecan-12-oate **13d**

Methyl (2-((((9H-fluoren-9-yl)methoxy)carbonyl)amino)acetamide)acetate **13c** (15 g, 40.6 mmol) was dissolved in dichloromethane (150 mL), and the compound **13b** (39 g, 203.2 mmol) and pyridinium 4-methylbenzenesulfonate (2 g, 8.1 mmol) were added. The reaction mixture was left to react at 40°C for 12 h. After the reaction was completed, the reaction mixture was concentrated under reduced pressure, and the resulting concentrate was purified by silica gel column chromatography with system B to give the title compound **13d** (10 g, yield: 50%).

MS m/z (ESI): 525.2 $(M+Na)^+$.

Step 3 Benzyl (*R*)-3-((2- aminoacetamido)methoxy)butyrate **13e**

The compound **13d** (10 g, 19.9 mmol) was dissolved in *N,N*-dimethylformamide (50 mL), diethylamine (3 g, 39.8 mmol) was added, and the reaction mixture was stirred at room temperature for 1 h. After the reaction was completed, the reaction mixture was concentrated to give a crude product **13e** (5 g), which was directly used in the next reaction step without purification.

MS m/z (ESI): 281.1 $(M+H)^+$.

Step 4: Benzyl (5*S*,13*R*)-5-benzyl-1-(9*H*-fluoren-9-yl)-13-methyl-3,6,9-trioxo-2,12-dioxo-4,7,10-triazapentadecan-15-carboxylate **13f**

The compound **13e** (5 g, 17.8 mmol) was dissolved in *N,N*-dimethylformamide (20 mL), and (*S*)-2,5-dioxopyrrolidin-1-yl 2-((((9H-fluoren-9-yl)methoxy)carbonyl)amino)-3-phenylpropanoate (7 g, 19.5 mmol) and *N,N*-diisopropylethylamine (4.5 g, 35.6 mmol) were added. The reaction mixture was stirred for reaction at room temperature for 2 h. After the reaction was completed, the reaction mixture was concentrated to give a crude product **13f** (10 g), which was directly used in the next reaction step without purification.
MS m/z (ESI): 672.3 $(M+Na)^+$.

Step 5 Benzyl (*R*)-3-((2-((*S*)-2-amino-3-phenylpropionamido)acetamido)methoxy)butyrate **13g**

The compound **13f** (10 g, 15.4 mmol) was dissolved in *N,N*-dimethylformamide (50 mL), and diethylamine (2 g, 30.8 mmol) was added. The reaction mixture was stirred for reaction at room temperature for 1 h. After the reaction was completed, the system was diluted with water (100 mL) and extracted with ethyl acetate (100 mL x 3). The organic phases were combined, washed with saturated brine, dried, and concentrated. The resulting concentrate was purified by silica gel column chromatography with system A to give the product **13 g** (4 g, yield: 61%).
MS m/z (ESI): 428.1 $(M+H)^+$.

Step 6 Benzyl (11*S*,19*R*)-11-benzyl-1-(9*H*-fluoren-9-yl)-19-methyl-3,6,9,12,15-pentaoxo-2,18-dioxo-4,7,10,13,16-pentaazabenzofuran-21-oate **13h**

The compound **13g** (4 g, 9.3 mmol) was dissolved in *N,N*-dimethylformamide (30 mL). (((9H-fluoren-9-yl)methoxy)carbonyl)glycylglycine (3.6 g, 10.2 mmol), *N,N,N',N'*-tetramethyl-O-(7-azabenzotriazol-1-yl)uronium hexafluorophosphate (5.3 g, 13.9 mmol) and *N,N*-diisopropylethylamine (2.3 g, 18.6 mmol) were added. The reaction mixture was stirred for reaction at room temperature for 1 h. After the reaction was completed, the reaction mixture was diluted with water (100 mL) and extracted with ethyl acetate (50 mL x 3). The organic phases were combined, washed with saturated brine, dried, and concentrated. The resulting crude product was purified by silica gel column chromatography with system A to give the title compound **13h** (3 g, yield: 42%).
MS m/z (ESI): 786.3 $(M+H)^+$.

Step 7 (11*S*,19*R*)-11-benzyl-1-(9*H*-fluoren-9-yl)-19-methyl-3,6,9,12,15-pentaoxo-2,18-dioxo-4,7,10,13,16-pentaazabenzofuran-21-carboxylic acid **13i**

The compound **13h** (3 g, 3.9 mmol) was dissolved in ethanol (20 mL) and ethyl acetate (10 mL), palladium/carbon (2 g, 19.5 mmol) was added, hydrogen replacement was conducted, and the reaction mixture was stirred in a hydrogen atmosphere at room temperature for 2 h. After the reaction was completed, the reaction mixture was filtered through diatomite, and the filter cake was washed with ethyl acetate. The filtrates were combined and concentrated under reduced pressure to give the crude title compound **13i** (2 g), which was directly used in the next reaction step without purification.
MS m/z (ESI): 696.3 $(M+H)^+$.

Step 8 (7*S*,15*R*)-1-amino-7-benzyl-15-methyl-2,5,8,11-tetraoxo-14-oxo-3,6,9,12-tetraazaheptadecan-17-carboxylic acid **13j**

The compound **13i** (2 g, 3 mmol) was dissolved in N,N-dimethylformamide (20 mL), diethylamine (0.44 g, 6 mmol) was added, and the reaction mixture was stirred to react at room temperature for 1 h. After the reaction was completed, tetrahydrofuran (20 mL) was added to the reaction mixture, the precipitated solid was filtered, and the filter cake was collected to give the title compound **13j** (1 g, yield: 76%).
MS m/z (ESI): 452.2 $(M+H)^+$.

Step 9 (3*R*,11*S*)-11-benzyl-3-methyl-24-(2-(methylsulfonyl)pyrimidin-5-yl)-7,10,13,16,19-pentaoxo-4-oxo-6,9,12,15,18-pentaazatetracarbonyl-23-enoic acid **131**

The compound **13j** (300 mg, 0.66 mmol) was dissolved in *N,N*-dimethylformamide (10 mL), and 2,5-dioxopyrrolidin-1-yl-6-(2-(methylsulfonyl)pyrimidin-5-yl)hex-5-ynoate (267 mg, 0.72 mmol) and *N,N*-diisopropylethylamine (171 mg, 1.32 mmol) were added. The reaction mixture was stirred at room temperature for 1 h. After the reaction was completed, the reaction mixture was diluted with water (50 mL), and extracted with ethyl acetate (30 mLx3). The organic phases were combined, washed with saturated brine, dried, and concentrated. The resulting crude product was purified by

preparative high performance liquid chromatography (GILSON Prep LC with UV detector, chromatographic column: Xtimate 10 μm C18 250 x 30 mm; mobile phase 1: water (containing 0.1% FA); mobile phase 2: acetonitrile; 15-min gradient, gradient proportion: acetonitrile phase 25%-95%, flow rate: 50 mL/min) to give the title compound **131** (100 mg, 21%).

MS m/z (ESI): 724.3 $(M+Na)^+$.

Step 10 *N*-((7S,15R)-7-benzyl-17-(((1S,10S)-10-ethyl-6-fluoro-10-hydroxy-5-methyl-11,14-dioxo-1,2,3,4,10,11,14,16-octahydro-13*H*-cyclohepta[*de*]pyrano[3',4':6,7]indolizino[1,2-*b*]quinolin-1-yl)amino)-15-methyl-2,5,8,11,17-pentaoxo-14-oxo-3,6,9,12-tetraazaheptadecyl)-6-(2-(methylsulfonyl)pyrimidin-5-yl)hexy-5-nylamide **13**

The compound **131** (100 mg, 0.14 mmol) was dissolved in *N,N*-dimethylformamide (6 mL), and the compound **6m-2** (64 mg, 0.14 mmol), 4-(4,6-dimethoxytriazin-2-yl)-4-methylmorpholine hydrochloride (84 mg, 0.28 mmol) and triethylamine (29 mg, 0.28 mmol) were added. The reaction mixture was stirred for reaction at room temperature for 1 h. After the reaction was completed, the reaction mixture was diluted with water (20 mL), and extracted with ethyl acetate (20 mLx3). The organic phases were combined, washed with saturated brine, dried, and concentrated. The resulting crude product was purified by preparative high performance liquid chromatography (Waters MS-triggered Prep-LC with SQD2 detector, chromatographic column: Xbridge 5 μm C18 150 x 19 mm; mobile phase 1: water (containing 0.1% FA); mobile phase 2: acetonitrile; 15-min gradient, gradient proportion: acetonitrile phase 40%-100%, flow rate: 20 mL/min) to give the title compound **13** (10 mg, yield: 6%).

MS m/z (ESI): 1133.3 $(M+H)^+$.

$^1$H NMR (400 MHz, $CD_3OD$) δ 8.92 (s, 1H), 7.60 (d, 2H), 7.16 (dd, 6H), 5.57 (d, 5H), 5.36 (s, 2H), 4.70 (d, 2H), 4.57 (d, 2H), 4.45 (d, 2H), 3.80-3.72 (m, 4H), 3.48 (s, 2H), 3.13 (s, 2H), 2.60-2.51 (m, 4H), 2.47-2.40 (m, 6H), 1.93 (d, 6H), 1.30 (d, 2H), 1.23 (d, 3H), 1.01-0.96 (m, 3H).

## Example 14

*N*-((7S,15R)-7-benzyl-17-((1S,10S)-10-ethyl-6-fluoro-10-hydroxy-5-methyl-11,14-dioxo-1,2,3,4,10,14,16-octahydro-13*H*-cycloheptan[de]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(2,5-dioxo-2,5-dihydro-1*H*-pyrrol-1-yl)hexanamide

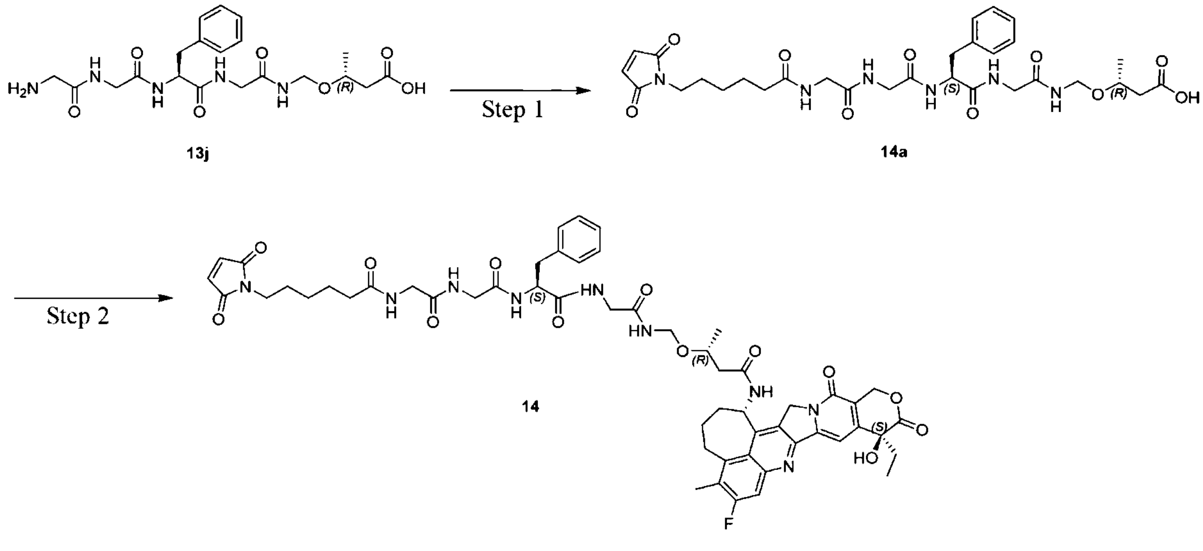


13j

14a

14

Step 1 (3*R*, 11*S*)-11-benzyl-24-(2,5-dioxo-2,5-dihydro-1*H*-pyrrol-1-yl)-3-methyl-7,10,13,16,19-pentaoxo-4-oxa-6,9,12,15,18-pentaazabutanoic acid **14a**

The compound **13j** (200 mg, 0.44 mmol) was dissolved in *N,N*-dimethylformamide (2 mL), 2,5-dioxopyrrolidin-1-yl 6-(2,5-dioxo-2,5-dihydro-1*H*-pyrrol-1-yl)hexanoate (136 mg, 0.44 mmol) and *N,N*-diisopropylethylamine (114 mg, 0.88 mmol) were added, and the reaction mixture was stirred at room temperature for 1 h. After the reaction was completed, the reaction mixture was purified by preparative high performance liquid chromatography (Waters MS-triggered Prep-LC with QDA detector, chromatographic column: Xbridge 5 μm C18 150 x 19 mm; mobile phase 1: water (containing 0.1% FA);

mobile phase 2: acetonitrile; 10-min gradient, gradient proportion: acetonitrile phase 25%-35%, flow rate: 25 mL/min) to give the title compound **14a** (110 mg, yield: 39%).

MS m/z (ESI): 667.1 $(M+Na)^+$.

Step 2 *N*-((7S,15R)-7-benzyl-17-((1S,10S)-10-ethyl-6-fluoro-10-hydroxy-5-methyl-11,14-dioxo-1,2,3,4,10,14,16-octahydro-13*H*-cycloheptan[*de*]pyrano[3',4':6,7]indolizino[1,2-*b*]quinolin-1-yl)amino)-15-methyl-2,5,8,11,17-pentaoxo-14-oxa-3,6,9,12-tetraazeheptadecyl)-6-(2,5-dioxo-2,5-dihydro-1*H*-pyrrol-1-yl)hexanamide **14**

The compound **6m-2** (50 mg, 0.11 mmol) was dissolved in *N,N*-dimethylformamide (0.5 mL), and the compound **13b** (72 mg, 0.11 mmol), 2-(7-azabenzotriazole)-*N,N,N',N'*-tetramethyluronium hexafluorophosphate (62 mg, 0.08 mmol), and N,N-diisopropylethylamine (28 mg, 0.22 mmol) were added. The mixture was stirred for reaction at room temperature for 15 min. After the reaction was completed, the reaction mixture was purified by preparative high performance liquid chromatography (Waters MS-triggered Prep-LC with SQD2 detector, chromatographic column: Xbridge 5μm C18 150 x19 mm; mobile phase 1: water (containing 0.1% FA); mobile phase 2: acetonitrile; 12-min gradient, gradient proportion: acetonitrile phase 41%-51%, flow rate: 20 mL/min) to give the compound **14** (46 mg, yield: 39%).

MS m/z (ESI): 1076.3 $(M+H)^+$.

$^1H$ NMR (400 MHz, DMSO-$d_6$) δ 8.65 (d, 1H), 8.47 (s, 1H), 8.26 (d, 1H), 8.09 (d, 1H), 8.05 (s, 1H), 7.98 (d, 1H), 7.30 (s, 1H), 7.22-7.16 (m, 5H), 6.99 (s, 2H), 5.53 (s, 1H), 5.42 (s, 2H), 5.29 (s, 2H), 4.57 (d, 1H), 4.54-4.44 (m, 2H), 3.99 (d, 1H), 3.73-3.55 (m, 8H), 3.02 (d, 1H), 2.80-2.72 (m, 1H), 2.69-2.54 (m, 4H), 2.45-2.31(m, 4H), 2.09 (t, 3H), 1.94-1.76 (m, 4H), 1.46 (dd, 4H), 1.23-1.15 (m, 2H), 1.13 (d, 3H), 0.86 (t, 3H).

**Example 15**

*N*-((7*S*,15*R*)-7-benzyl-17-((1*S*,9*S*)-9-ethyl-5-fluoro-9-hydroxy-10,13-dioxo-4-vinyl-2,3,9,10,13,15-hexahydro-1*H*,12*H*-benzo[*d*]pyrano[3',4':6,7]indolizino[1,2-*b*]quinolin-1-yl)amino)-15-methyl-2,5,8,11,17-pentaoxo-14-oxa-3,6,9,12-tetraazeheptadecyl)-6-(2,5-dioxo-2,5-dihydro-1*H*-pyrrol-1-yl)hexanamide

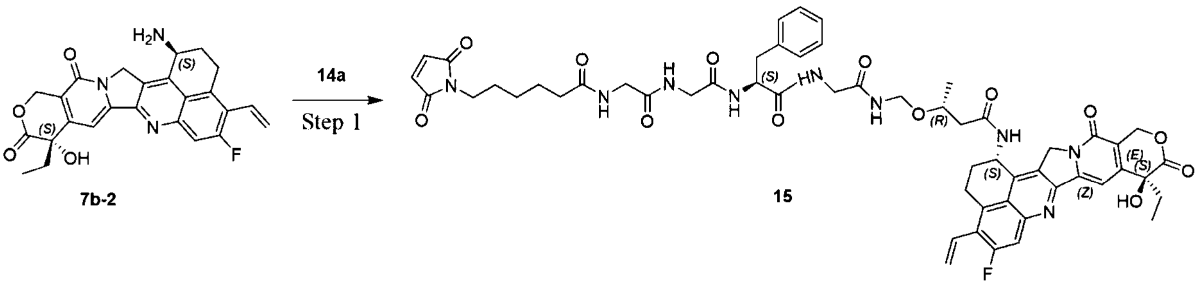


7b-2 15

Step 1 *N*-((7*S*,15*R*)-7-benzyl-17-((1*S*,9*S*)-9-ethyl-5-fluoro-9-hydroxy-10,13-dioxo-4-vinyl-2,3,9,10,13,15-hexahydro-1*H*,12*H*-benzo[*d*]pyrano[3',4':6,7]indolizino[1,2-*b*]quinolin-1-yl)amino)-15-methyl-2,5,8,11,17-pentaoxo-14-oxa-3,6,9,12-tetraazeheptadecyl)-6-(2,5-dioxo-2,5-dihydro-1*H*-pyrrol-1-yl)hexanamide **15**

(1*S*,9*S*)-1-amino-9-ethyl-5-fluoro-9-hydroxy-4-vinyl-1,2,3,9,12,15-hexahydro-10*H*,13*H*-benzo[d]pyrano [3',4':6,7]indolizino[1,2-*b*]quinoline-10,13-dione **7b-2** (20 mg, 0.04 mmol), 2-(7-azabenzotriazole)-*N,N,N',N'*-tetramethyluronium hexafluorophosphate (23 mg, 0.06 mmol) and N,N-diisopropylethylamine (11 mg, 0.09 mmol) were added. The mixture was stirred for reaction at room temperature for 30 min. After the reaction was completed, the crude product was purified by preparative high performance liquid chromatography (Waters MS-triggered Prep-LC with SQD2 detector, chromatographic column: Xbridge 5 μm C18 150 x 19 mm; mobile phase 1: water (containing 0.1% FA); mobile phase 2: acetonitrile; 12-min gradient, gradient proportion: acetonitrile phase 44%-54%, flow rate: 20 mL/min) to give the compound **15** (6 mg, yield: 14%).

MS m/z (ESI): 1074.3 $(M+1)^+$.

$^1H$ NMR (400 MHz, $CD_3OD$) δ 7.57-7.50 (m, 2H), 7.19-7.09 (m, 6H), 6.93 (dd, 1H), 6.76 (s, 2H), 5.81 (dd, 2H), 5.66 (t, 1H), 5.55 (d, 1H), 5.37-5.26 (m, 3H), 4.70 (d, 2H), 4.50 (dd, 1H), 4.27 (d, 1H), 3.77 (q, 4H), 3.45 (dd, 2H), 3.10 (dd, 2H), 2.88-2.81 (m, 1H), 2.51-2.45 (m, 2H), 2.30-2.18 (m, 5H), 1.89 (q, 2H), 1.57 (t, 5H), 1.28 (d, 6H), 0.98 (t, 3H).

**Example 16**

(1*R*,3*R*)-3-(((*S*)-7-benzyl-20-(2,5-dioxo-2,5-dihydro-1*H*-pyrrol-1-yl)-3,6,9,12,15-pentaoxo-2,7,8,11,14-pentaazadicarbonyl)oxy)-*N*-((1*S*,9*S*)-9-ethyl-5-fluoro-9-hydroxy-10,13-dioxo-4-vinyl-2,3,9,10,13,15-hexahydro-1*H*,12*H*-benzo[*d*]pyrano[3',4':6,7]indolizino[1,2-*b*]quinolin-1-yl)cyclobutane-1-carboxamide

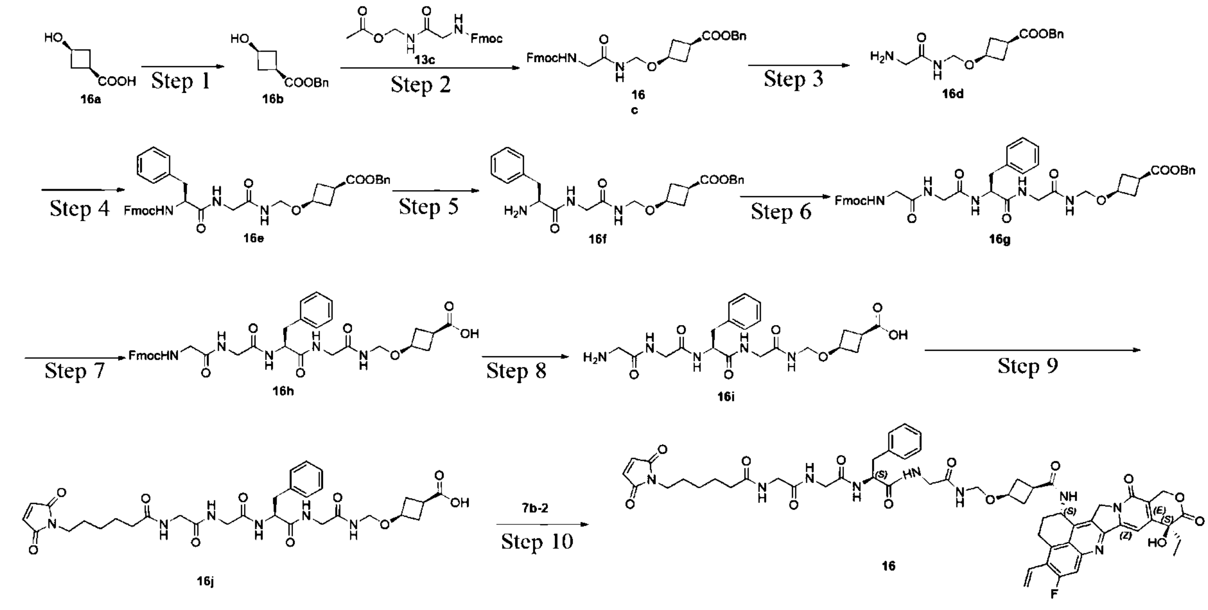


The same synthetic route as Example **13** was adopted, except that the raw material (R)-3-hydroxybutyric acid in Step 1 was replaced with (1S,3S)-3-hydroxycyclobutane-1-carboxylic acid **16a** (10 g, 86.20 mmol). After eight steps of reaction, the compound **16i** (2.8 g, yield: 30%) was obtained.
MS m/z (ESI): 464.2 $(M+1)^+$.

Step 9 (1*R*,3*S*)-3-(((*S*)-7-benzyl-20-(2,5-dioxo-2,5-dihydro-1*H*-pyrrol-1-yl)-3,6,9,12,15-pentaoxo-2,7,8,11,14-pentaazaeicosyl)oxy)cyclobutane-1-carboxylic acid **16j**

The compound **16i** (750 mg, 1.62 mmol) was dissolved in DMF (5 mL), and 2,5-dioxopyrrolidin-1-yl 6-(2,5-dioxo-2,5-dihydro-1*H*-pyrrol-1-yl)hexanoate (499 mg, 1.62 mmol) and *N,N*-diisopropylethylamine (209 mg, 1.62 mmol) were added. The mixture was stirred for reaction at room temperature for 1 h. After the reaction was completed, the reaction mixture was purified by preparative high performance liquid chromatography (GILSON Prep LC with UV detector, chromatographic column: Xtimate 10 μm C18 250 x 30 mm; mobile phase 1: water (containing 0.1% FA); mobile phase 2: acetonitrile; 15-min gradient, gradient proportion: acetonitrile phase 20%-95%, flow rate: 50 mL/min) to give the compound **16j** (150 mg, yield: 13%).
MS m/z (ESI): 679.2 $(M+23)^+$.

Step 10 (1*R*,3*R*)-3-(((*S*)-7-benzyl-20-(2,5-dioxo-2,5-dihydro-1*H*-pyrrol-1-yl)-3,6,9,12,15-pentaoxo-2,7,8,11,14-pentaazadicarbonyl)oxy)-*N*-((1*S*,9*S*)-9-ethyl-5-fluoro-9-hydroxy-10,13-dioxo-4-vinyl-2,3,9,10,13,15-hexahydro-1*H*,12*H*-benzo[d]pyrano[3',4':6,7]indolizino[1,2-*b*]quinolin-1-yl)cyclobutane-1-carboxamide **16**

The Compound **7b-2** (20 mg, 0.04 mmol), the compound **16j** (33 mg, 0.05 mmol), 2-(7-azabenzotriazole)-*N,N,N',N'*-tetramethyluronium hexafluorophosphate (23 mg, 0.06 mmol) and *N,N*-diisopropylethylamine (11 mg, 0.09 mmol) were mixed. The mixture was stirred for reaction at room temperature for 30 min. After the reaction was completed, the crude product was purified by preparative high performance liquid chromatography (Waters MS-triggered Prep-LC with SQD2 detector, chromatographic column: Xbridge 5 μm C18 150 x 19 mm; mobile phase 1: water (containing 0.1% FA); mobile phase 2: acetonitrile; 12-min gradient, gradient proportion: acetonitrile phase 40%-55%, flow rate: 20 mL/min) to give the compound **16** (10 mg, yield: 23%).
MS m/z (ESI): 1086.2 $(M+H)^+$.
$^1$H NMR (400 MHz, CDOD) δ 8.52 (s, 2H), 7.71 (d,1H), 7.63 (s, 1H), 7.29-7.15 (m, 4H), 6.94 (dd, 1H), 6.76 (s, 1H),

5.90-5.78 (m, 2H), 5.64 (s, 1H), 5.54 (d, 1H), 5.35 (d, 1H), 5.24 (d, 1H), 4.72-4.63 (m, 2H), 4.49-4.44 (m, 1H), 4.04 (s, 1H), 3.96-3.85 (m, 2H), 3.83-3.69 (m, 4H), 3.49-3.46 (m, 1H), 3.46-3.41 (m, 2H), 3.15 (s, 1H), 3.14-3.11 (m, 1H), 2.96 (s, 1H), 2.70 (s, 1H), 2.66-2.54 (m, 2H), 2.50-2.45 (m, 1H), 2.34-2.26 (m, 2H), 2.26-2.17 (m, 3H), 1.99-1.91 (m, 2H), 1.63-1.48 (m, 4H), 1.28 (d, 3H), 1.00 (t, 3H).

**Example 17**

(*R*)-*N*-((1*R*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxybutanamide

(*R*)-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxybutanamide

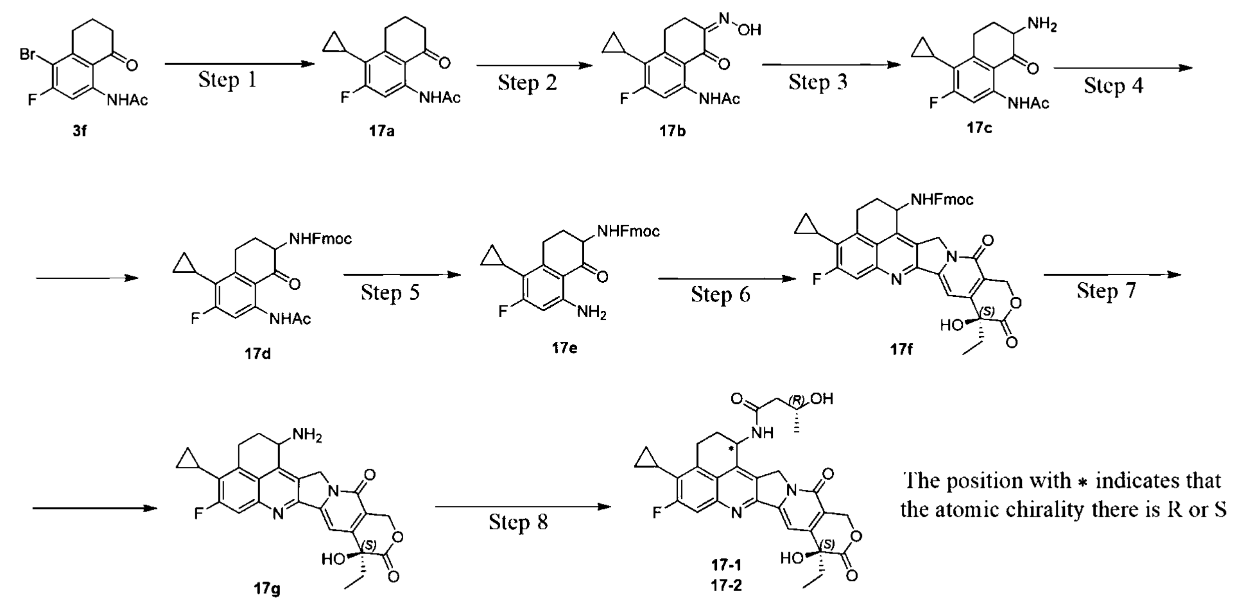


3f 17a 17b 17c 17d 17e 17f 17g 17-1 17-2

Step 1 N-(4-cyclopropyl-3-fluoro-8-oxo-5,6,7,8-tetrahydronaphthalen-1-yl)acetamide **17a**

The compound **3f** (1.5 g, 5.02 mmol), cyclopropylboronic acid (1.3 g, 15.06 mmol), [1,1-bis(diphenylphosphino) ferrocene]dichloropalladium (2.2 g, 3.01 mmol), and cesium carbonate (1.6 g, 15.06 mmol) were dissolved in dioxane (15 mL). The mixture was stirred for reaction in a microwave reactor at 110 °C for 2 h. After the reaction was completed, the reaction mixture was poured into water, extracted with ethyl acetate three times, and dried over anhydrous sodium sulfate. The organic phases were combined, and concentrated. The system was purified by silica gel column chromatography with eluent system B to give the title compound **17a** (1.1 g, yield: 84%).
MS m/z (ESI): 262.1 $(M+1)^+$.

Step 2 (*Z*)-*N*-(4-cyclopropyl-3-fluoro-7-(hydroxyimino)-8-oxo-5,6,7,8-tetrahydronaphthalen-1-yl)acetamide **17b**

Potassium tert-butoxide (0.94 g, 8.4 mmol) was dissolved in a mixed solvent of tetrahydrofuran and tert-butanol (50 mL, V/V=4:1), the compound **17a** (1.1 g, 4.2 mmol) was dissolved in 10 mL of the tetrahydrofuran solution, and the mixture was slowly added to the reaction system at 0°C. After the mixture was stirred for reaction at 0°C for 10 min, isoamyl nitrite (0.74 g, 6.3 mmol) was added, and the reaction was continued by stirring at 0°C for 50 min. After the reaction was completed, the system was adjusted with dilute hydrochloric acid to have a pH of 4-5, and extracted with ethyl acetate 3 times. The organic phase was washed with saturated brine, collected, dried over anhydrous sodium sulfate, and spin-dried to remove the solvent, thus giving the crude product **17b,** which was directly used in the next reaction step without purification.
MS m/z (ESI): 291.0 $(M+1)^+$.

Step 3 *N*-(7-amino-4-cyclopropyl-3-fluoro-8-oxo-5,6,7,8-tetrahydronaphthalen-1-yl)acetamide **17c**

The compound **17b** was dissolved in methanol (50 mL), and a palladium-carbon catalyst (500 mg) was added. The mixture was stirred for reaction in a hydrogen environment at room temperature for two hours. After the reaction was completed, the mixture was filtered. The filtrate was collected to give the crude product **17c,** which was directly used in the next reaction step without purification.
MS m/z (ESI): 277.1 (M+1) $^+$.

Step 4 (9*H*-fluoren-9-yl)methyl(8-acetylamino-5-cyclopropyl-6-fluoro-1-oxo-1,2,3,4-tetrahydronaphthalen-2-yl)carbamate **17d**

The filtrate obtained from the previous step was adjusted with a saturated sodium carbonate solution to a PH of 8-9, and then Fmoc-Cl (1.19 g, 4.6 mmol) was added. The mixture was stirred for reaction at room temperature for 1 h. After the reaction was completed, the system was extracted with ethyl acetate. The organic phase was washed with saturated brine, collected, dried over anhydrous sodium sulfate, and spin-dried to remove the solvent, thus giving a crude product. The crude product was purified by silica gel column chromatography with system A to give the compound **17d** (1 g, yield: 48%).
MS m/z (ESI): 499.1 (M+1)$^+$.

Step 5 (9*H*-fluoren-9-yl)methyl(8-amino-5-cyclopropyl-6-fluoro-1-oxo-1,2,3,4-tetrahydronaphthalen-2-yl)carbamate **17e**

The compound **17e** (1 g, 2.01 mmol) was dissolved in dioxane (20 mL), and concentrated hydrochloric acid (12 mol/L, 5 mL) was added. The mixture was stirred for reaction at 60°C for 1 h. After the reaction was completed, the mixture was spin-dried to remove the solvent, thus giving a crude product, which was purified by silica gel column chromatography with system A to give the compound **17f** (700 mg, yield: 77%).
MS m/z (ESI): 457.1 (M+1)$^+$.

Step 6 (9*H*-fluoren-9-yl)methyl((9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)carbamate **17g**

The compound **17f** (700 mg, 1.54 mmol) was dissolved in toluene (10 mL), and (S)-4-ethyl-4-hydroxy-7,8-dihydro-1*H*-pyrano[3,4-f]indolizine-3,6,10(4*H*)-trione (484 mg, 1.84 mmol), and p-toluenesulfonic acid monohydrate (292 mg, 1.54 mmol) were added. The reaction system was stirred for reaction at 140°C for 4 h. After the reaction was completed, the mixture was spin-dried to remove the solvent, thus giving a crude product, which was purified by silica gel column chromatography with system Achu to give the title compound **17g** (700 mg, yield: 66%).
MS m/z (ESI): 684.2 (M+1) $^+$.

Step 7 (9*S*)-1-amino-4-cyclopropyl-9-ethyl-5-fluoro-9-hydroxy-1,2,3,9,12,15-hexahydro-10*H*,13*H*-benzo[*d*]pyrano [3',4':6,7]indolizino[1,2-*b*]quinoline-10,13-dione **17h**

The compound **17g** (100 mg, 0.15 mmol) was dissolved in *N,N*-dimethylformamide (5 mL), and diethylamine (0.5 mL) was added. The mixture was stirred for reaction at room temperature for 30 min. After the reaction was completed, the mixture was vacuum concentrated to give the crude compound 17h (60 mg), which was directly used in the next reaction step without purification.
MS m/z (ESI): 462.1 (M+1)$^+$.

Step 8 (*R*)-*N*-((1*R*,9*S*)-4-cyclopropyl-9-ethyl-5-fluoro-9-hydroxy-10,13-dioxo-2,3,9,10,13,15-hexahydro-1*H*,12*H*-benzo [*d*]pyrano[3',4':6,7]indolizino[1,2-*b*]quinolin-1-yl)-3-hydroxybutanamide

(*R*)-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxybutanamide

The compound 17h (60 mg, 0.13 mmol) was dissolved in DMF (5 mL), and then (R)-3-hydroxybutyric acid (19 mg, 0.18 mmol), 2-(7-azabenzotriazole)-*N,N,N',N'*-tetramethyluronium hexafluorophosphate (87 mg, 0.23 mmol), and N,N-dimethylacetamide (59 mg, 0.45 mmol) were added. The mixture was stirred for reaction at room temperature for 30 min. After the reaction was completed, the reaction mixture was purified by preparative high performance liquid chromatography (Waters MS-triggered Prep-LC with SQD2 detector, chromatographic column: Xbridge 5 μm C18 150x19 mm;

mobile phase 1: water (containing 0.1% FA); mobile phase 2: acetonitrile; 15-min gradient, gradient proportion: acetonitrile phase 38%-48%, flow rate: 20 mL/min) to give the compounds **17-1** (6 mg, yield: 11%) and **17-2** (4 mg, yield: 8%).

Mono-configurational compound **17-1** (short retention time):

MS m/z (ESI): 548.2 $(M+1)^+$.

$^1$H NMR (400 MHz, DMSO-$d_6$) δ 8.44 (d, 1H), 7.75 (d, 1H), 7.47 (d, 1H), 7.30 (s, 1H), 7.11 (d, 1H), 5.60-5.52 (m, 1H), 5.43 (s, 2H), 5.29-5.13 (m, 2H), 4.09-4.00 (m, 1H), 2.48 (s, 1H), 2.29 (s, 2H), 2.24-2.18 (m, 1H), 2.14 (d, 2H), 1.99-1.92 (m, 1H), 1.91-1.79 (m, 2H), 1.15-1.10 (m, 2H), 1.09 (d, 3H),0.87 (s, 3H), 0.76 (s, 2H).

Mono-configurational compound **17-2** (long retention time):

MS m/z (ESI): 548.2 $(M+1)^+$.

$^1$H NMR (400 MHz, DMSO-$d_6$) δ 8.46 (d, 1H), 7.76 (d, 1H), 7.47 (d, 1H), 7.30 (s, 1H), 7.13-7.06 (m, 1H), 5.59 (dd, 1H), 5.43 (d, 2H), 5.24 (d, 2H), 4.05 (dd, 1H), 2.29 (t, 1H), 2.25 (d, 1H), 2.19 (d, 1H), 2.18-2.06 (m, 3H), 1.95 (d, 1H), 1.85 (dd, 2H), 1.16-1.10 (m, 2H), 1.08 (d, 3H), 0.87 (s, 4H), 0.76 (d, 2H).

**Example 18**

(1*S*,3*S*)-*N*-((1*R*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxycyclobutane-1-carboxamide

(1*S*,3*R*)-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxycyclobutane-1-carboxamide

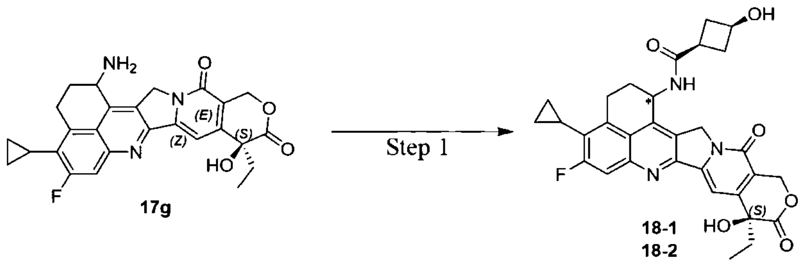


17g

18-1
18-2

The position with * indicates that the atomic chirality there is R or S

Step 1

(1*S*,3*S*)-*N*-((1*R*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4': 6,7]indolizino[1,2-*b*]quinolin-1-yl)-3-hydroxycyclobutane-1-carboxamide

(1*S*,3*R*)-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4': 6,7]indolizino[1,2-*b*]quinolin-1-yl)-3-hydroxycyclobutane-1-carboxamide

The compound **17g** (69 mg, 0.15 mmol) was dissolved in *N*,*N*-dimethylformamide (5 mL), and (1S,3S)-3-hydroxycyclobutane-1-carboxylic acid (21 mg, 0.18 mmol), 2-(7-azabenzotriazole)-*N*,*N*,*N'*,*N'*-tetramethyluronium hexafluorophosphate (87 mg, 0.23 mmol), and *N,N*-dimethylacetamide (59 mg, 0.45 mmol) were added. The mixture was stirred for reaction at room temperature for 30 min. After the reaction was completed, the reaction mixture was purified by preparative high performance liquid chromatography (Waters MS-triggered Prep-LC with QDA detector, chromatographic column: WELCH Xtimate C18 21.2x250 mm 10 μm; mobile phase 1: water (containing 0.1% FA); mobile phase 2: acetonitrile; 18-min gradient, gradient proportion: acetonitrile phase 35%-65%, flow rate: 20 mL/min) to give the compounds **18-1** (4 mg, yield: 6%) and **18-2** (2.4 mg, yield: 4%:)

Mono-configurational compound **18-1** (short retention time):

MS m/z (ESI): 560.2 $(M+1)^+$.

$^1$H NMR (400 MHz, DMSO-$d_6$) δ 8.44 (d, 1H), 7.71 (d, 1H), 7.29 (d, 1H), 6.54 (d, 1H), 5.56 (s, 1H), 5.42 (s, 2H), 5.17-5.09 (m, 3H), 3.94 (d, 1H), 2.44-2.30 (m, 4H), 2.08-2.03 (m, 4H), 1.94-1.84 (m, 4H), 1.11 (d, 2H), 0.87 (t, 3H), 0.76 (s, 2H).

Mono-configurational compound **18-2** (long retention time):

MS m/z (ESI): 560.2 $(M+1)^+$.

$^1$H NMR (400 MHz, DMSO-$d_6$) δ 8.45 (d, 1H), 7.74 (d, 1H), 7.30 (s, 1H), 6.53 (s, 1H), 5.57 (s, 1H), 5.43 (s, 2H), 5.14 (dd, 3H), 3.97-3.91 (m, 1H), 2.40-2.27 (m, 4H), 2.15-2.05 (m, 4H), 1.96-1.84 (m, 4H), 1.11 (d, 2H), 0.87 (t, 3H), 0.75 (d, 2H).

**Example 19**

(*R*)-*N*-((1*S*,10*S*)-5-cyclopropyl-10-ethyl-6-fluoro-10-hydroxy-11,14-dioxo-1,2,3,4,10,14,16-octahydro-13*H*-cyclohepta [de]pyrano[3',4':6,7]indolizino[1,2-*b*]quinolin-1-yl)-3-hydroxybutanamide

(*R*)-*N*-((1*R*,10*S*)-5-cyclopropyl-10-ethyl-6-fluoro-10-hydroxy-11,14-dioxo-1,2,3,4,10,14,16-octahydro-13*H*-cyclohepta [*de*]pyrano[3',4':6,7]indolizino[1,2-*b*]quinolin-1-yl)-3-hydroxybutanamide

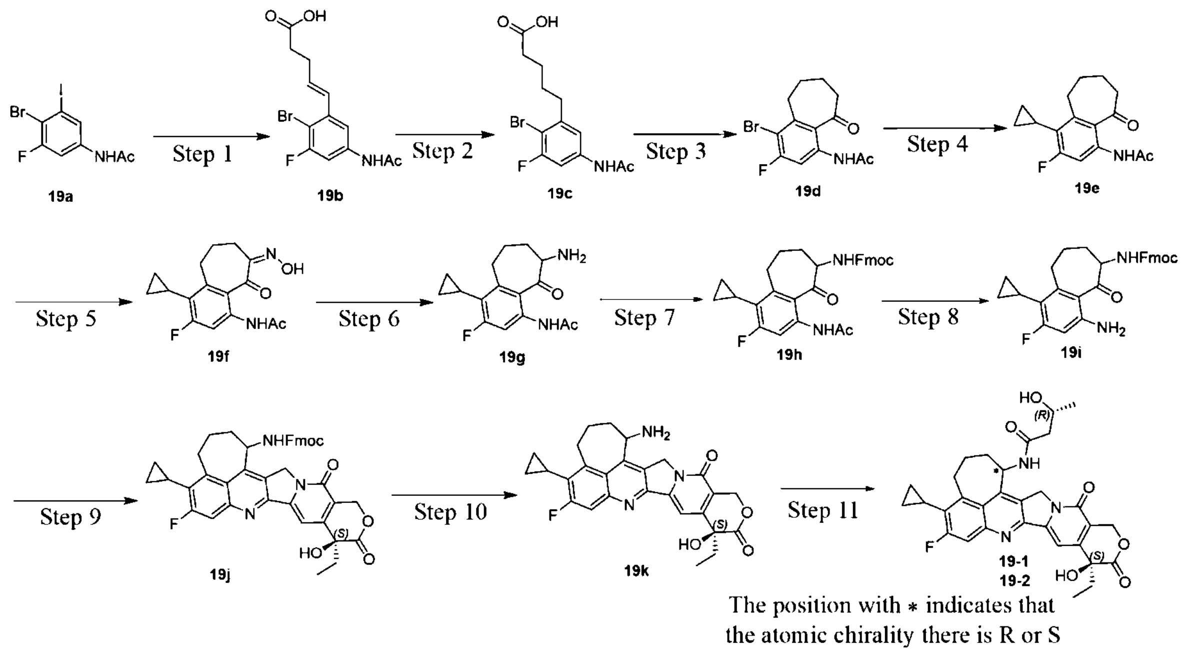


Step 1 5-(5-acetylamino-2-bromo-3-fluorophenyl)pent-4-enoic acid **19b**

The compound **19a** (7.0 g, 19.5 mmol) was dissolved in dioxane (60 mL) and water (15 mL), to which pent-4-enoic acid (2.2 g, 22.0 mol), bis(triphenylphosphine)palladium chloride (700 mg, 1.0 mmol), and sodium carbonate (6.2 g, 58.4 mmol) were added. The reaction mixture was stirred under the protection of nitrogen at 90 °C for 16 h. After the reaction was completed, the reaction mixture was filtered through diatomite, and washed with ethyl acetate. The resulting filtrate was directly concentrated. The crude product was diluted with water (200 mL), and extracted with diethyl ether 3 times (100 mLx3). The aqueous phase was adjusted with dilute hydrochloric acid to have pH=2, and extracted with ethyl acetate (100 mLx3). The organic phases were combined and concentrated to give the crude product **19b** (6.0 g).

MS m/z (ESI): 330.1 $(M+1)^+$.

Step 2 5-(5-acetylamino-2-bromo-3-fluorophenyl)pentanoic acid **19c**

The compound **19b** (6.0 g, 18.2 mmol) was dissolved in anhydrous methanol (100 mL), to which platinum/carbon (5%, 600 mg) was added. The reaction mixture was subjected to hydrogen replacement three times, and stirred for reaction at room temperature for 16 h. After the reaction was completed, the reaction mixture was filtered through diatomite, and washed with ethyl acetate. The resulting filtrate was directly concentrated to give the crude compound **19c** (5.0 g).

MS m/z (ESI): 332.0 $(M+1)^+$.

Step 3 *N*-(4-bromo-3-fluoro-9-oxo-6,7,8,9-tetrahydro-5*H*-benzo[7]cycloalken-1-yl)acetamide **19d**

The compound **19c** (2.5 g, 3.3 mmol) was dissolved in polyphosphoric acid (25 mL). The reaction mixture was stirred for reaction at 140°C for 2 h. After the reaction was completed, water (100 mL) was added to the reaction mixture, and the mixture was stirred for 4 h. The aqueous phase was extracted with ethyl acetate (100 mLx3). The organic phases were combined and concentrated. The resulting crude product was purified by silica gel column chromatography with system B to give the compound **19d** (920 mg, yield: 39%).

MS m/z (ESI): 314.0 $(M+1)^+$.

A synthetic route the same as that in Example **17** was used as the subsequent synthetic route, except that the compound **3f** was replaced with the compound **19d** (720 mg, 2.3 mmol). The final product was purified by preparative high performance liquid chromatography (Waters MS-triggered Prep-LC with QDA detector, chromatographic column: WELCH Xtimate C18 21.2x250 mm 10 μm; mobile phase 1: water (containing 0.1% FA); mobile phase 2: acetonitrile; 15-min gradient, gradient proportion: acetonitrile phase 40%-95%, flow rate: 30 mL/min) to give products **19-1** (6.5 mg, yield: 9%) and **19-2** (11mg, yield: 16%).

Mono-configurational compound **19-1** (short retention time):

MS m/z (ESI): 562.2 $(M+1)^+$.
$^1$H NMR (400 MHz, $CDCl_3$) δ 7.63-7.50 (m, 3H), 7.07 (s, 1H), 5.73-5.69 (m, 2H), 5.43 (d, 1H), 5.36-5.15 (m, 2H), 4.24 (s, 1H), 3.81 (s, 1H),3.64-3.50 (m, 1H), 3.47-3.32 (m, 1H), 2.52-2.41 (m, 2H), 2.36 -2.12 (m, 3H), 1.88-1.83 (m, 4H), 1.25 (d, 3H), 1.19-1.15 (m, 2H), 1.02 (t, 3H), 0.81-0.77 (m, 2H).

Mono-configurational compound **19-2** (long retention time):

MS m/z (ESI): 562.2 $(M+1)^+$.
$^1$H NMR (400 MHz, $CDCl_3$) δ 7.50-7.35 (m, 3H), 5.72-5.55 (m, 1H), 5.47-5.29 (m, 2H), 5.26-5.05 (m, 2H), 4.51-4.31 (m, 1H), 4.05-3.91 (m, 1H), 3.70-3.54 (m, 1H), 3.53-3.37 (m, 1H), 2.66-2.50 (m, 2H), 2.49-2.37 (m, 1H), 2.28-2.14 (m, 1H), 2.11-1.75 (m, 5H), 1.41-1.29 (m, 3H), 1.29-1.10 (m, 2H),1.04-0.95 (m, 3H), 0.88-0.71 (m, 2H).

**Example 20**

(1*S*,3*S*)-*N*-((1*R*,10*S*)-5-cyclopropyl-10-ethyl-6-fluoro-10-hydroxy-11,14-dioxo-1,2,3,4,10,11,14,16-octahydro-13*H*-cyclohepta[*de*]pyrano[3',4':6,7]indolizino[1,2-*b*]quinolin-1-yl)-3-hydroxybutane-1-carboxamide

(1*S*,3*S*)-*N*-((1*S*,10*S*)-5-cyclopropyl-10-ethyl-6-fluoro-10-hydroxy-11,14-dioxo-1,2,3,4,10,11,14,16-octahydro-13*H*-cyclohepta[*de*]pyrano[3',4':6,7]indolizino[1,2-*b*]quinolin-1-yl)-3-hydroxybutane-1-carboxamide

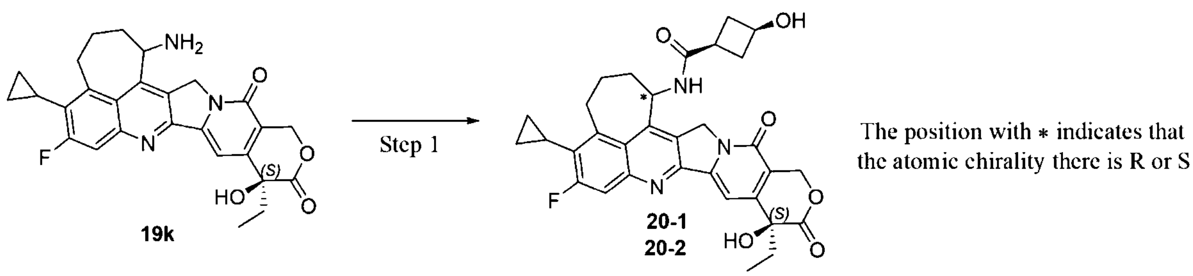


19k

20-1
20-2

Step 1

(1*S*,3*S*)-*N*-((1*R*,10*S*)-5-cyclopropyl-10-ethyl-6-fluoro-10-hydroxy-11,14-dioxo-1,2,3,4,10,11,14,16-octahydro-13*H*-cyclohepta[*de*]pyrano[3',4':6,7]indolizino[1,2-*b*]quinolin-1-yl)-3-hydroxybutane-1-carboxamide

(1*S*,3*S*)-*N*-((1*S*,10*S*)-5-cyclopropyl-10-ethyl-6-fluoro-10-hydroxy-11,14-dioxo-1,2,3,4,10,11,14,16-octahydro-13*H*-cyclohepta[*de*]pyrano[3',4':6,7]indolizino[1,2-*b*]quinolin-1-yl)-3-hydroxybutane-1-carboxamide

The compound **19k** (75 mg, 0.16 mmol) was dissolved in *N*,*N*-dimethylformamide (3 mL), and (1S,3S)-3-hydroxycyclobutane-1-carboxylic acid (22 mg, 0.19 mmol), 2-(7-azabenzotriazole)-*N*,*N*,*N*',*N*'-tetramethyluronium hexafluorophosphate (91 mg, 0.24 mmol), and *N,N*-diisopropylethylamine (171 mg, 1.32 mmol) were added. The reaction mixture was stirred for reaction at room temperature for 1 h. After the reaction was completed, the reaction mixture was diluted with water (50 mL), and extracted with ethyl acetate (30 mLx3). The organic phases were combined, washed with saturated brine, dried, and concentrated. The crude product was purified by preparative high performance liquid chromatography (GILSON Prep LC with UV detector, chromatographic column: WELCH Xtimate C18 21.2x250mm 10 μm; mobile phase 1: water (containing 0.1% NH3.H2O); mobile phase 2: acetonitrile; 15-min gradient, gradient proportion: acetonitrile phase 40%-100%, flow rate: 30 mL/min) to give the compounds **20-1** (2.9 mg, 7%) and **20-2** (1.5 mg, 2%).

Mono-configurational compound **20-1** (short retention time):

MS m/z (ESI): 574.7 $(M+1)^+$.

1H NMR (400 MHz, $CDCl_3$) δ 7.59 (d, 1H), 7.53 (s, 1H), 6.49 (s, 1H), 5.72-5.64 (m, 1H), 5.59 (d, 1H), 5.35 (d, 1H), 5.14 (d, 1H), 4.93 (d, 1H), 4.25-4.19(m, 1H), 3.77 (s, 1H), 3.64-3.52 (m, 1H), 3.46 -3.41 (m, 1H), 2.77-2.68 (m, , 3H), 2.42 -2.10 (m, 5H), 1.94-1.78 (m, 4H), 1.25-1.75 (m, 2H), 1.05 (t, 3H), 0.87-0.72 (m, 2H).

Mono-configurational compound **20-2** (long retention time):

MS m/z (ESI): 574.7 $(M+1)^+$.

$^1H$ NMR (400 MHz, $CDCl_3$) δ 7.53 (d, 1H), 7.46 (s, 1H), 6.91 (br, 1H), 5.59 (t, 1H), 5.30-5.19(m, 2H), 5.11-5.04 (m, 2H), 4.30-4.21 (m, 1H), 3.76 (s, 1H), 3.68 (d, 1H), 3.48-3.34 (m, 1H), 2.88-2.75 (m, 3H), 2.37-2.21 (m, 5H), 1.93 (s, 3H), 1.80-1.72 (m, 2H), 1.23-1.13 (m, 2H), 0.96 (t, 3H), 0.87-0.75 (m, 2H).

**Example 21**

*N*-((7*S*,15*R*)-7-benzyl-17-(((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)amino)-15-methyl-2,5,8,11,17-pentaoxo-14-oxo-3,6,9,12-tetraazeheptadecyl)-6-(2,5-dioxo-2,5-dihydro-1*H*-pyrrol-1-yl)hexanamide

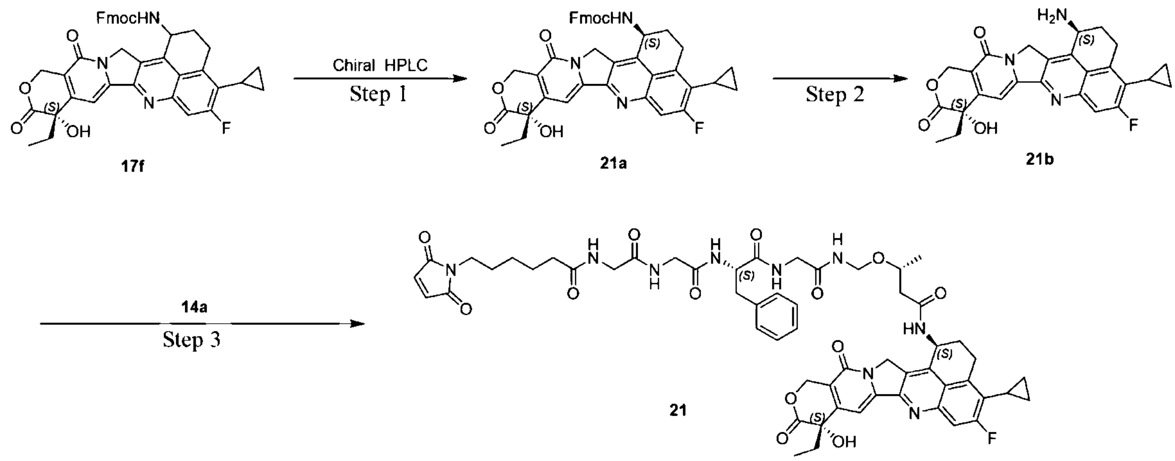


17f 21a 21b 21

Step 1 (9*H*-fluoren-9-yl)methyl((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)carbamate **21a**

The compound **17f** (600 mg, 1.02 mmol) was purified using a Gilson preparative instrument and a Daicel chiral column to separate chiral isomers (chromatographic column: CHIRALPAK IB 3.0 cm I.D.×25 cm,10 μm; mobile phase 1: MeOH; mobile phase 2: DCM; gradient proportion: MeOH/DCM=90/10, flow rate: 25 mL/min) to give the compound **21a** (250 mg, yield: 42%).

Step 2 (1*S*,9*S*)-1-amino-4-cyclopropyl-9-ethyl-5-fluoro-9-hydroxy-1,2,3,9,12,15-hexahydro-10*H*,13*H*-benzo[*d*]pyrano [3',4':6,7]indolizino[1,2-*b*]quinoline-10,13-dione **21b**

The compound **21a** (50 mg, 0.07 mmol) was dissolved in DMF (5 mL), and diethylamine (0.5 mL) was added. The

mixture was stirred for reaction at room temperature for 1 h. After the reaction was completed, the mixture was spin-dried under vacuum to remove the solvent, thus giving the title compound **21b** (34 mg), which was directly used in the next reaction step without purification.

MS m/z (ESI): 462.1 (M+1)$^+$.

Step 3 *N*-((7*S*,15*R*)-7-benzyl-17-(((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)amino)-15-methyl-2,5,8,11,17-pentaoxo-14-oxo-3,6,9,12-tetraazeheptadecyl)-6-(2,5-dioxo-2,5-dihydro-1*H*-pyrrol-1-yl)hexanamide **21**

The compound **21b** (34 mg) was dissolved in DMF (2 mL) and the compound **14a** (54 mg, 0.08 mmol), 2-(7-azabenzotriazole)-*N*,*N*,*N'*,*N'*-tetramethyluronium hexafluorophosphate (40 mg, 0.11 mmol) and *N*,*N*-diisopropylethylamine (18 mg, 0.14 mmol) were added. The mixture was stirred for reaction at room temperature for 30 min. After the reaction was completed, the crude product was purified by preparative high performance liquid chromatography (Waters MS-triggered Prep-LC with QDA detector, chromatographic column: Xbridge 5μm C18 150 x 19 mm; mobile phase 1: water (containing 0.1%FA); mobile phase 2: acetonitrile; 12-min gradient, gradient proportion: acetonitrile phase 41%-51%, flow rate: 25 mL/min) to give the compound 21 (20 mg, yield: 26%).

MS m/z (ESI): 1088.3 (M+H)$^+$.

$^1$H NMR (400 MHz, CD3OD) δ 7.70 (d, 1H), 7.62 (t, 2H), 7.23-7.13 (m, 8H), 6.76 (s, 2H), 5.60 (d, 2H), 5.55 (s, 1H), 5.38 (dd, 3H), 5.23 (d, 2H), 4.41 (dd, 2H), 4.10 (d, 2H), 3.80 (dd, 5H), 3.43 (d, 2H), 3.05 (d, 1H), 3.04-2.96 (m, 2H), 2.56-2.42 (m, 4H), 2.28 (d, 2H), 2.21 (d, 2H), 1.93 (dd, 2H), 1.60 1.52 (m, 4H), 1.26 (d, 4H), 1.14 (d, 3H), 0.99 (t, 3H).

**Example 22**

(1*R*,3*R*)-3-(((*S*)-7-benzyl-(2,5-dioxo-2,5-dihydro-1*H*-pyrrol-1-yl)-3,6,9,12,15-pentaoxo-2,7,8,11,14-pentaazadicarbonyl)oxy)-*N*-((1*S*,9*S*)-4-cyclopropyl-9-fluoro-9-hydroxy-10,9,10,13,15-hexahydro-1*H*,12*H*-benzo[*d*]pyrano[3',4':6,7]indolizino[1,2-*b*]quinolin-1-yl)cyclobutane carboxamide

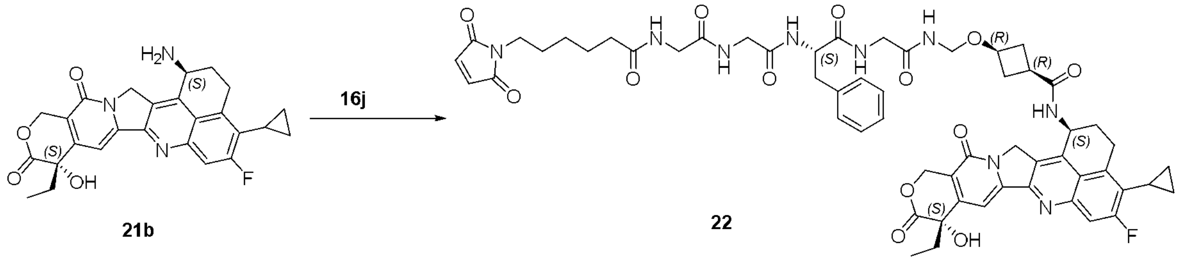


21b 22

Step 1 (1*R*,3*R*)-3-(((*S*)-7-benzyl-(2,5-dioxo-2,5-dihydro-1*H*-pyrrol-1-yl)-3,6,9,12,15-pentaoxo-2,7,8,11,14-pentaazadicarbonyl)oxy)-*N*-((1*S*,9*S*)-4-cyclopropyl-9-fluoro-9-hydroxy-10,9,10,13,15-hexahydro-1*H*,12*H*-benzo[*d*]pyrano [3',4':6,7]indolizino[1,2-*b*]quinolin-1-yl)cyclobutane carboxamide

The compound **21b** (35 mg, 0.08 mmol) was dissolved in N,N-dimethylformamide (3 mL), and the compound **16j** (60 mg, 0.09 mmol), *N,N,N',N'*-tetramethyl-O-(7-azabenzotriazol-1-yl)uronium hexafluorophosphate(43 mg, 0.11 mmol) and N,N-diisopropylethylamine (24 mg, 0.19 mmol) were added. The reaction mixture was stirred for reaction at room temperature for 1 h. After the reaction was completed, the reaction mixture was purified by preparative high performance liquid chromatography (Waters MS-triggered Prep-LC with SQD2 detector, chromatographic column: Xbridge C18 5μm 150 x 19 mm; mobile phase 1: water (containing 0.1% FA); mobile phase 2: acetonitrile; 15-min gradient, gradient proportion: acetonitrile phase 42%-100%, flow rate: 20 mL/min) to give the compound **22** (20 mg, yield: 22%).

MS m/z (ESI): 1100.5 (M+1)$^+$.

$^1$H NMR (400 MHz, $CD_3OD$) δ 8.51 (d, 1H), 8.37-8.20 (m, 2H), 7.59-7.52 (m, 1H), 7.29-7.11 (m, 4H), 6.75 (s, 1H), 5.66 (d, 1H), 5.58-5.48 (m, 1H), 5.37-5.14 (m, 2H), 5.09-4.94 (m, 1H), 4.73-4.61 (m, 2H), 4.48 (dd, 1H), 4.09-3.97 (m, 1H), 3.97-3.68 (m, 5H), 3.57-3.46 (m, 1H), 3.45-3.37 (m, 2H), 3.15 (dd, 1H), 3.04-2.91 (m, 1H), 2.75-2.53 (m, 2H), 2.52-2.41 (m, 1H), 2.38-2.15 (m, 5H), 2.01 (dd, 1H), 1.94 -1.85 (m, 2H), 1.68-1.44 (m, 4H), 1.38-1.20 (m, 6H), 1.15 (d, 2H), 1.02-0.86 (m, 3H), 0.81 (d, 2H).

**Example 23**

*N*-((7*S*,15*R*)-7-benzyl-17-(((1*S*,10*S*)-5-cyclopropyl-10-ethyl-6-fluoro-10-hydroxy-11,14-dioxo-1,2,3,4,10,14,16-octahydro-13*H*-cycloheptan[*de*]pyrano[3',4':6,7]indolizino[1,2-*b*]quinolin-1-yl)amino)-15-methyl-2,5,8,11,17-pentaoxo-14-oxo-3,6,9,12-tetraazeheptadecyl)-6-(2,5-dioxo-2,5-dihydro-1*H*-pyrrol-1-yl)hexanamide

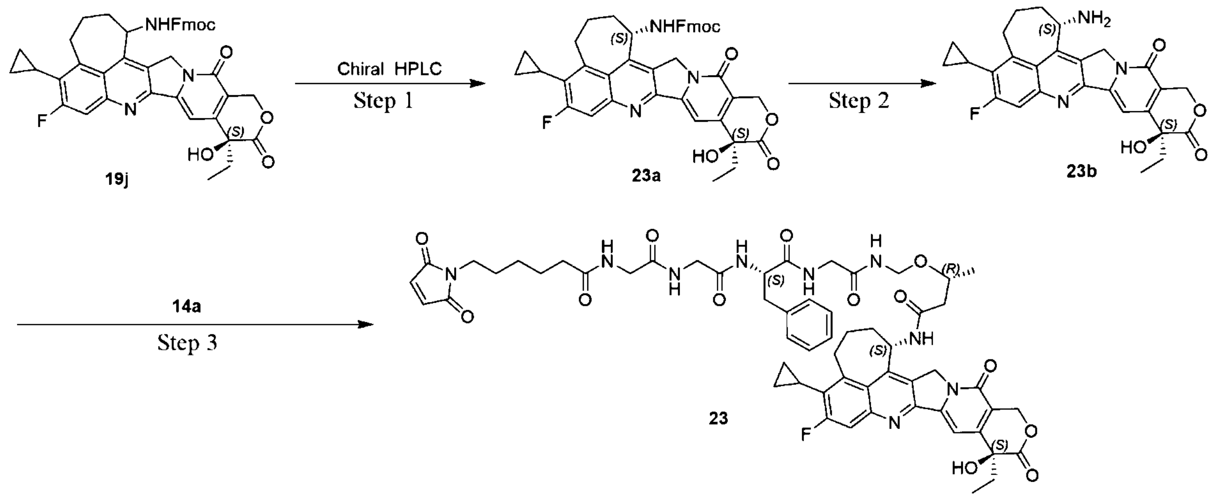


19j 23a 23b 23

Step 1 (9*H*-fluoro-9-yl)methyl((1*S*,10*S*)-5-cyclopropyl-10-ethyl-6-fluoro-10-hydroxy-11,14-dioxo-1,2,3,4,10,11,14,16-octahydro-13*H*-cyclohepta[3',4':6,7]indolizino[1,2-*b*]quinolin-1-yl)carbamate **23a**

The compound **19j** (500 mg, 0.72 mmol) was purified using a Gilson preparative instrument and a Daicel chiral column to separate chiral isomers (chromatographic column: CHIRALPAK IA 3.0 cm I.D.×25 cm,10 μm; mobile phase 1: MeOH; mobile phase 2: DCM; gradient proportion: MeOH/DCM=70/30, flow rate: 25 mL/min) to give the compound **23a** (220 mg, yield: 44%).

Step 2 (1*S*,10S)-1-amino-5-cyclopropyl-10-ethyl-6-fluoro-10-hydroxy-2,3,4,10,13,16-hexahydro-14*H*-cyclohepta [3',4':6,7]indolizino[1,2-*b*]quinoline-11,14-(1*H*)-dione **23b**

The compound **23a** (45 mg, 0.064 mmol) was dissolved in a mixed solvent of *N,N*-dimethylformamide and diethylamine (7 mL, V/V=5:2), and the reaction mixture was stirred at room temperature for 2 h. After the reaction was completed, the reaction mixture was directly concentrated to give the crude compound **23b** (30 mg), which was directly used in the next reaction step without purification.
MS m/z (ESI): 476.2 $(M+1)^+$.

Step 3 *N*-((7*S*,15*R*)-7-benzyl-17-(((1*S*,10*S*)-5-cyclopropyl-10-ethyl-6-fluoro-10-hydroxy-11,14-dioxo-1,2,3,4,10,14,16-octahydro-13*H*-cycloheptan[de]pyrano[3',4':6,7]indolizino[1,2-*b*]quinolin-1-yl)amino)-15-methyl-2,5,8,11,17-pentaoxo-14-oxo-3,6,9,12-tetraazeheptadecyl)-6-(2,5-dioxo-2,5-dihydro-1*H*-pyrrol-1-yl)hexanamide **23**

The compound **23b** (30 mg) was dissolved in *N*,*N*-dimethylformamide (5 mL), and the compound **14a** (43 mg, 0.064 mmol), 2-(7-azabenzotriazole)-*N,N,N',N'*-tetramethyluronium hexafluorophosphate (37 mg, 0.096 mmol), and *N*,*N*-diisopropylethylamine (171 mg, 1.32 mmol) were added. The reaction mixture was stirred at room temperature for 2 h. After the reaction was completed, water (50 mL) was added into the reaction mixture, and the mixture was extracted with ethyl acetate (30 mL x 3). The organic phases were combined, washed with saturated brine, dried, and concentrated to give a crude product. The crude product was purified by preparative high performance liquid chromatography (Waters MS-triggered Prep-LC with QDA detector, chromatographic column: WELCH Xtimate C18 21.2x250 mm 10 μm; mobile phase 1: water (containing 0.1% FA); mobile phase 2: acetonitrile; 15-min gradient, gradient proportion: acetonitrile phase 47%-95%, flow rate: 30 mL/min) to give the title compound 23 (10.03 mg, yield: 13.5%).
MS m/z (ESI): 1102.2$(M+1)^+$.
$^1$H NMR (400 MHz, $CDCl_3$) δ 8.94 (s, 1H), 8.18-8.14 (m, 2H), 7.65-7.53 (m, 2H), 7.43 (s, 1H), 7.25-7.19 (m, 6H), 6.66 (s, 2H), 6.59 (s, 1H), 5.75-5.63 (m, 3H), 5.32-5.13 (m, 3H), 4.51 (d, 2H), 4.08 (s, 4H), 3.89 (s, 3H), 3.75-3.61 (m, 2H), 3.48 (t, 2H), 3.42-3.31 (m, 1H), 3.08 (d, 2H), 2.38-2.11 (m, 7H), 1.89-1.85 (m, 2H), 1.30-1.25 (m, 7H), 1.20 -1.17 (m, 1H), 1.49-1.09 (m, 1H), 1.02 (t, 3H), 0.88-0.75 (m, 6H).

**Example 24**

(*R*)-*N*-((1*R*,10*S*)-10-ethyl-6-fluoro-10-hydroxy-5-methyl-11,14-dioxo-2,3,10,11,14,16-hexahydro-1*H*,13*H*-oxepino [4,3,2-*de*]pyrano[3',4':6,7]indolizino[1,2-*b*]quinolin-1-yl)-3-hydroxybutanamide

(*R*)-*N*-((1*S*,10*S*)-10-ethyl-6-fluoro-10-hydroxy-5-methyl-11,14-dioxo-2,3,10,11,14,16-hexahydro-1*H*,13*H*-oxepino [4,3,2-*de*]pyrano[3',4':6,7]indolizino[1,2-*b*]quinolin-1-yl)-3-hydroxybutanamide

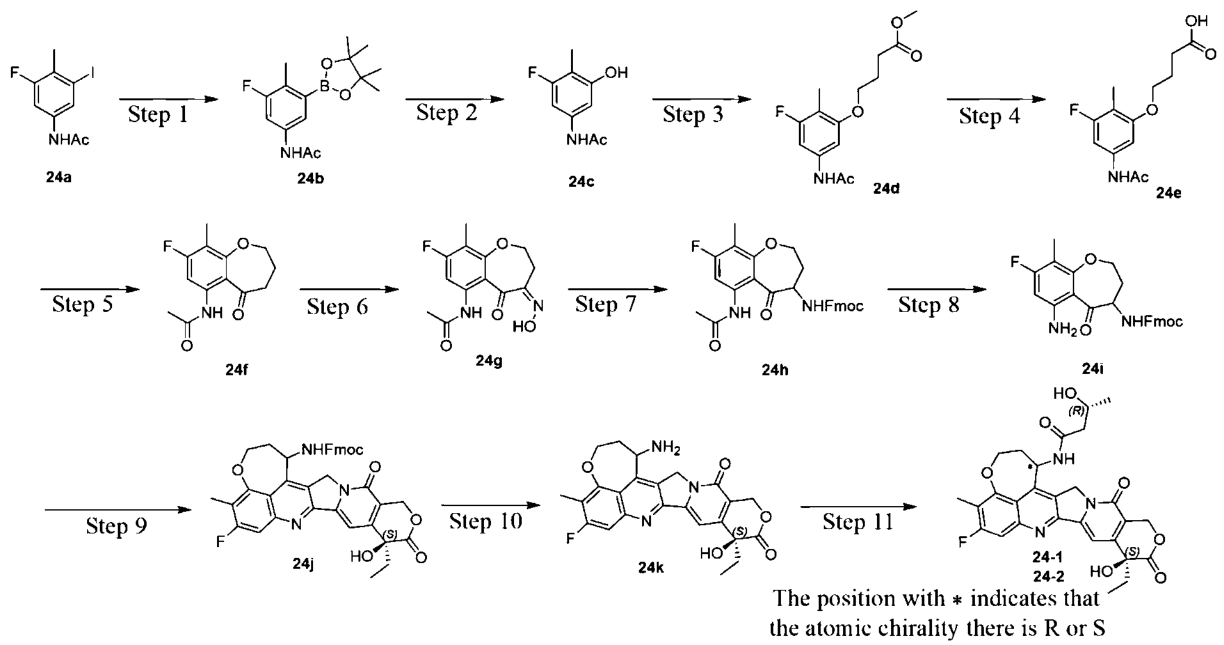


Step 1 *N*-(3-fluoro-4-methyl-5-(4,4,5,5-tetramethyl-1,3,2-dioxaborolan-2-yl)phenyl)acetamide **24b**

*N*-(3-fluoro-5-iodo-4-methylphenyl)acetamide **24a** (7 g, 23.9 mmol) was dissolved in 1,4-dioxane (70 mL), and diboric acid pinacol ester (12.14 g, 47.8 mmol), Pd(dppf)$Cl_2$ (3.47 g, 4.78 mmol) and potassium acetate (7.04 g, 71.7 mmol) were added. The mixture was stirred for reaction under the protection of nitrogen at 110°C for 16 h. After the reaction was completed, the mixture was filtered. The filtrate was collected, and spin-dried to remove the solvent, thus giving a crude product. The crude product was purified by column chromatography with separation system B to give the title compound **24b** (4.1 g, yield: 59%).

MS m/z (ESI): 294.1 $(M+1)^+$.

Step 2 N-(3-fluoro-5-hydroxy-4-methylphenyl)acetamide **24c**

The compound **24b** (4.1 g, 14 mmol) was dissolved in a mixed solvent of tetrahydrofuran and water (40 mL, V/V=1:1), and sodium chlorite (1.52 g, 16.8 mmol) was added. The mixture was stirred for reaction at room temperature for 30 min. After the reaction was completed, ethyl acetate was added, and the organic phase was washed 3 times with an aqueous sodium hydroxide solution (15%wt). The aqueous phase was collected, and the pH was adjusted to 3-5 with hydrochloric acid, followed by extraction with dichloromethane, and the organic phase was collected. The solvent was then removed by rotary evaporation to give a crude product. The crude product was purified by silica gel column chromatography with separation system B to give the title compound **24c** (1.9 g, yield: 74%).

MS m/z (ESI): 184.0 $(M+1)^+$.

Step 3 Methyl 4-(5-acetylamino-3-fluoro-2-methylphenoxy)butyrate **24d**

The compound **24c** (1.9 g, 10.4 mmol) was dissolved in *N*,*N*-dimethylformamide (20 mL), and methyl 4-bromobutyrate (2.82 g, 15.6 mmol), potassium carbonate (2.87 g, 20.8 mmol) and potassium iodide (2.59 g, 15.6 mmol) were added. The mixture was stirred for reaction at 85°C for 16 h. After the reaction was completed, ethyl acetate was

added, the system was washed three times with saturated brine, and the organic phases were collected. The mixture was spin-dried to remove the solvent, thus giving a crude product, which was purified by silica gel column chromatography with system B to give the title compound **24d** (2 g, yield: 68%).
MS m/z (ESI): 284.0 $(M+1)^+$.

Step 4 4-(5-acetylamino-3-fluoro-2-methylphenoxy)butyric acid **24e**

The compound **24d** (2 g, 7.1 mmol) was dissolved in a mixed solvent of tetrahydrofuran and water (20 mL, V/V=1:1), and lithium hydroxide (340 mg, 14.2 mmol) was added to the above system. The mixture was stirred for reaction at room temperature for 2 h. After the reaction was completed, dilute hydrochloric acid was added to adjust the pH of the system to 3-5, ethyl acetate was added for extraction, and the organic phase was collected and dried over anhydrous sodium sulfate. The mixture was spin-dried to remove the solvent, thus giving the crude product **24e** (1.4 g, yield: 73%), and the product was directly used in the next reaction step without purification.
MS m/z (ESI): 270.0 $(M+1)^+$.

Step 5 *N*-(8-fluoro-9-methyl-5-oxo-2,3,4,5-tetrahydrobenzo[*b*]oxan-6-yl)acetamide **24f**

The compound **24e** (1.4 g, 5.2 mmol) was dissolved in trifluoroacetic acid (20 mL), and trifluoroacetic anhydride (3.28 g, 15.6 mmol) was added. The mixture was stirred for reaction at 40°C for 1 h. After the reaction was completed, water (10 mL) was slowly added and stirring was continued at 40°C for 1 h. After the reaction was completed, the system was extracted with ethyl acetate, and the organic phase was collected. The mixture was spin-dried to remove the solvent, thus giving a crude product, which was purified by silica gel column chromatography with separation system B to give the compound **24f** (900 mg, yield: 69%).
MS m/z (ESI): 252.1$(M+1)^+$.
$^1$H NMR (400 MHz, $CDCl_3$) δ 11.24 (s, 1H), 8.10 (d, 1H), 4.15 (t, 2H), 2.84 - 2.77 (m, 2H), 2.17 - 2.10 (m, 5H), 2.07 (d, 3H).

Step 6 (*Z*)-*N*-(8-fluoro-4-(hydroxyimino)-9-methyl-5-oxo-2,3,4,5-tetrahydrobenzo[*b*]oxan-6-yl)acetylamine **24g**

Tetrahydrofuran (20 mL) and tert-butanol (5 mL) were added to a flask, the mixture was cooled to 0°C, and potassium tert-butoxide (806 mg, 6.43 mmol) was added. Then the compound **24f** (900 mg, 3.59 mmol) was added slowly and the reaction mixture was stirred at 0°C for 10 min. Isoamyl nitrite (631 mg, 5.39 mmol) was then added and the reaction was stirred at 0°C for 1 h. After the reaction was completed, hydrochloric acid was added to adjust the pH value to 4-5, and the mixture was extracted with ethyl acetate. The organic phase was washed with brine and dried over anhydrous sodium sulfate, concentrated, and spin-dried to remove the solvent to give the crude product **24 g** (900 mg), which was used directly in the next step without purification.
MS m/z (ESI): 281.0 $(M+1)^+$.

Step 7 (9*H*-fluoren-9-yl)methyl(6-acetamido-8-fluoro-9-methyl-5-oxo-2,3,4,5-tetrahydrobenzo[*b*]oxetan-4-yl)carbamate **24h**

The compound **24g** (900 mg, 3.21 mmol) was dissolved in dioxane (10 mL), and palladium-carbon catalyst (200 mg) and 1 M dilute hydrochloric acid (2 mL) were added. The mixture was stirred for reaction in a hydrogen environment at room temperature for 2 h. After the reaction was completed, the mixture was filtered and the filtrate was collected. A saturated aqueous sodium bicarbonate solution was added to adjust the pH to 10. Then, 9-fluorenylmethyl chloroformate (929 mg, 3.59 mmol) was added and the reaction was stirred at room temperature for 1 hour. After the reaction was completed, the system was extracted by dichloromethane, the organic phase was collected and spin-dried to remove the solvent, thus giving a crude product, which was purified by silica gel column chromatography with system B to give the title compound **24h** (500 mg, yield: 29%).
MS m/z (ESI): 489.1 $(M+1)^+$.

Step 8 (9*H*-fluoren-9-yl)methyl(6-acetamido-8-fluoro-9-methyl-5-oxo-2,3,4,5-tetrahydrobenzo[*b*]oxetan-4-yl)carbamate **24i**

The compound **24h** (500 mg, 1.02 mmol) was dissolved in dichloromethane (5 mL), and a 4 M hydrogen chloride solution in methanol (5 mL) was added. The mixture was stirred for reaction at 60°C for 2 h. After the reaction was completed, the mixture was spin-dried to remove the solvent, thus giving a crude product, which was purified through silica gel column chromatography to give the title compound **24i** (350 mg, yield: 77%).

MS m/z (ESI): 447.1 $(M+1)^+$.

Step 9 (9*H*-fluoro-9-yl)methyl((10*S*)-10-ethyl-6-fluoro-10-hydroxy-5-methyl-11,14-dioxo-2,3,10,11,14,16-hexahydro-1*H*,13*H*-oxepino[4,3,2-*de*]pyrano[3',4':6,7]indolizino[1,2-*b*]quinolin-1-yl)carbamate **24j**

The compound **24i** (350 mg, 0.78 mmol) was dissolved in toluene (5 mL), and (*S*)-4-ethyl-4-hydroxy-7,8-dihydro-1*H*-pyrano[3,4-f]indolizine-3,6,10(4*H*)-trione (248 mg, 0.94 mmol) and p-toluenesulfonic acid monohydrate (223 mg, 1.18 mmol) were added. The mixture was stirred for reaction at 120°C for 2 h. After the reaction was completed, the mixture was spin-dried to remove the solvent, thus giving a crude product, which was purified by silica gel column chromatography with separation system B to give the title compound **24j** (450 mg, yield: 85%).
MS m/z (ESI): 674.2 $(M+1)^+$.

Step 10 (10*S*)-1-amino-10-ethyl-6-fluoro-10-hydroxy-5-methyl-1,2,3,10,13,16-hexahydro-11*H*,14*H*-oxepino[4,3,2-de]pyrano[3',4':6,7]indolizino[1,2-*b*]quinolin-11,14-one **24k**

The compound **24j** (100 mg, 0.15 mmol) was dissolved in *N*,*N*-dimethylformamide, and diethylamine (0.6 mL) was added. The mixture was stirred for reaction at room temperature for 30 min. After the reaction was completed, the mixture was spin-dried to remove the solvent, thus giving the crude product **24k** (65 mg, yield: 97%).
MS m/z (ESI): 452.1 $(M+1)^+$.

Step 11

(*R*)-*N*-((1*R*,10*S*)-10-ethyl-6-fluoro-10-hydroxy-5-methyl-11,14-dioxo-2,3,10,11,14,16-hexahydro-1*H*, 13*H*-oxepino[4,3,2-*de*]pyrano[3',4':6,7]indolizino[1,2-*b*]quinolin-1-yl)-3-hydroxybutanamide

(*R*)-*N*-((1*S*,10*S*)-10-ethyl-6-fluoro-10-hydroxy-5-methyl-11,14-dioxo-2,3,10,11,14,16-hexahydro-1*H*,13*H*-oxepino[4,3,2-*de*]pyrano[3',4':6,7]indolizino[1,2-*b*]quinolin-1-yl)-3-hydroxybutanamide **24**

The compound **24k** (65 mg, 0.14 mmol) was dissolved in *N*,*N*-dimethylformamide (3 mL), and (*R*)-3-hydroxybutyric acid (18 mg, 0.17 mmol), 2-(7-azabenzotriazole)-*N,N,N',N'*-tetramethyluronium hexafluorophosphate (82 mg, 0.22 mmol), and *N*,*N*-diisopropylethylamine (37 mg, 0.29 mmol) were added. The mixture was stirred for reaction at room temperature for 15 min. After the reaction was completed, the crude product was purified by preparative high performance liquid chromatography (Waters MS-triggered Prep-LC with SQD2, chromatographic column: Xbridge C18 150 x 19 mm, 10μm; mobile phase 1: water (containing 0.1% FA); mobile phase 2: acetonitrile; 10-min gradient, gradient proportion: acetonitrile phase 37%-47%, flow rate: 20 mL/min) to give the title compounds **24-1** (7.37 mg, yield: 10%) and **24-2** (4.70 mg, yield: 6%).

**Mono-configurational compound 24-1 (short retention time)**

MS m/z (ESI): 538.2 $(M+1)^+$.
$^1H$ NMR (400 MHz, DMSO-$d_6$) δ 8.48 (d, 1H), 7.68 (d, 1H), 7.47 (d, 1H), 7.29 (s, 1H), 7.11 (d, 1H), 5.61 (dd, 1H), 5.51 - 5.39 (m, 4H), 4.61 - 4.50 (m, 1H), 4.30 - 4.16 (m, 1H), 3.92 - 3.80 (m,1H), 2.35 (d, 3H), 2.29 (s, 1H), 2.20 (dd, 2H), 2.05 - 1.94 (m,1H), 1.92 - 1.77 (m, 2H), 0.87 (t, 3H), 0.82 (d, 3H).

**Mono-configurational compound 24-2 (long retention time)**

MS m/z (ESI): 538.2 $(M+1)^+$.
$^1H$ NMR (400 MHz, DMSO-$d_6$) δ 8.48 (d, 1H), 7.68 (d, 1H), 7.47 (d, 1H), 7.29 (s, 1H), 7.11 (d, 1H), 5.68 (dd, 1H), 5.48 (d, 2H), 5.43 (s, 2H), 4.54 - 4.42 (m,1H), 4.33 - 4.24 (m, 1H), 3.92 - 3.81 (m,1H), 2.35 (d, 3H), 2.29 (s, 1H), 2.26 - 2.16 (m, 2H), 2.03 - 1.93 (m,1H), 1.92 - 1.78 (m, 2H), 1.00 (d, 3H), 0.87 (t, 3H).

**Example 25**

(1*S*,3*S*)-*N*-((1*R*,10*S*)-10-ethyl-6-fluoro-10-hydroxy-5-methyl-11,14-dioxo-2,3,10,11,14,16-hexahydro-1*H*,13*H*-oxepino[4,3,2-*de*]pyrano[3',4':6,7]indolizino[1,2-*b*]quinolin-1-yl)-3-hydroxycyclobutane-1-carboxamide

(1*S*,3*R*)-*N*-((1*S*,10*S*)-10-ethyl-6-fluoro-10-hydroxy-5-methyl-11,14-dioxo-2,3,10,11,14,16-hexahydro-1*H*,13*H*-oxepino [4,3,2-*de*]pyrano[3',4':6,7]indolizino[1,2-*b*]quinolin-1-yl)-3-hydroxycyclobutane-1-carboxamide

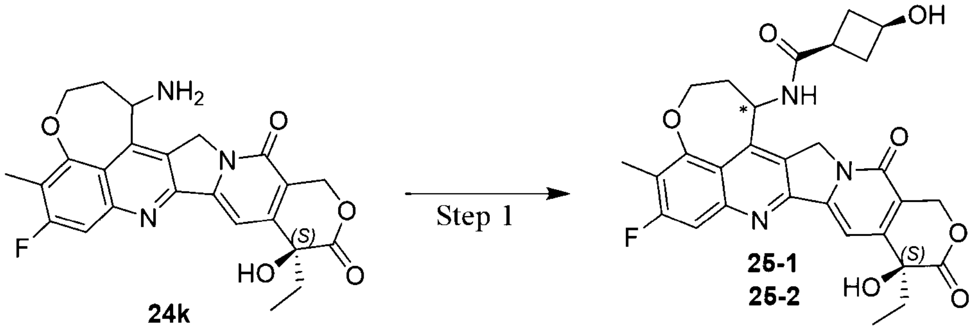


**24k**

**25-1**
**25-2**

The position with * indicates that the atomic chirality there is R or S

Step 1

(1*S*,3*S*)-*N*-((1*R*,10*S*)-10-ethyl-6-fluoro-10-hydroxy-5-methyl-11,14-dioxo-2,3,10,11,14,16-hexahydro-1*H*,13*H*-oxepino [4,3,2-*de*]pyrano[3',4':6,7]indolizino[1,2-*b*]quinolin-1-yl)-3-hydroxycyclobutane-1-carboxamide

(1*S*,3*R*)-*N*-((1*S*,10*S*)-10-ethyl-6-fluoro-10-hydroxy-5-methyl-11,14-dioxo-2,3,10,11,14,16-hexahydro-1*H*,13*H*-oxepino [4,3,2-*de*]pyrano[3',4':6,7]indolizino[1,2-*b*]quinolin-1-yl)-3-hydroxycyclobutane-1-carboxamide

The compound **24k** (65 mg, 0.14 mmol) was dissolved in *N,N*-dimethylformamide (3 mL), and cis-3-hydroxycyclobutanecarboxylic acid (20 mg, 0.17 mmol), 2-(7-azabenzotriazole)-*N,N,N',N'*-tetramethyluronium hexafluorophosphate (82 mg, 0.22 mmol), and *N,N*-diisopropylethylamine (37 mg, 0.29 mmol) were added. The reaction system was stirred at room temperature for 15 min. After the reaction was completed, the crude product was purified by preparative high performance liquid chromatography (Waters MS-triggered Prep-LC with SQD2 detector, chromatographic column: Xbridge C18 150 x 19 mm, 10μm; mobile phase 1: water (containing 0.1% FA); mobile phase 2: acetonitrile; 18-min gradient, gradient proportion: acetonitrile phase 34%-44%, flow rate: 20 mL/min) to give the title compounds **25-1** (6.57 mg yield: 9%) and **25-2** (8.90 mg, yield: 12%).

**Mono-configurational compound 25-1 (short retention time)**

MS m/z (ESI): 550.1 $(M+1)^+$.

$^1H$ NMR (400 MHz, DMSO-$d_6$) δ 8.40 (d, 1H), 7.67 (d, 1H), 7.29 (s, 1H), 5.65 (dd, 1H), 5.50 (d, 1H), 5.44 (s, 2H), 5.29 (d, 1H), 4.45 - 4.37 (m, 1H), 4.36 - 4.25 (m, 1H), 3.95 - 3.82 (m, 1H), 2.57 - 2.53 (m, 1H), 2.48 - 2.44 (m, 2H), 2.35 (d, 3H), 2.30 - 2.24 (m, 1H), 2.22 - 2.15 (m, 1H), 2.06 - 1.69 (m, 6H), 0.86 (dd, 3H).

**Mono-configurational compound 25-2 (long retention time)**

MS m/z (ESI): 550.1 $(M+1)^+$.

$^1H$ NMR (400 MHz, DMSO-$d_6$) δ 8.41 (d, 1H), 7.68 (d, 1H), 7.29 (s, 1H), 5.67 (dd, 1H), 5.48 (d, 1H), 5.41 (d, 2H), 5.28 (d, 1H), 4.44 - 4.26 (m, 2H), 3.97 - 3.85 (m, 1H), 2.59 - 2.53 (m, 1H), 2.47 - 2.42 (m, 2H), 2.34 (t, 3H), 2.30-2.25 (m, 1H), 2.24 - 2.18 (m, 1H), 2.07 - 1.72 (m, 6H), 0.87 (t, 3H).

**Example 26**

*N*-((7*S*,15*R*)-7-benzyl-17-(((1*S*,10*S*)-10-ethyl-6-fluoro-10-hydroxy-5-methyl-11,14-dioxo-1,2,3,4,10,14,16-octahydro-13*H*-cyclohepta[*de*]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(5-cyano-6-(methylsulfonyl)pyridin-3-yl)hexy-5-nylamide

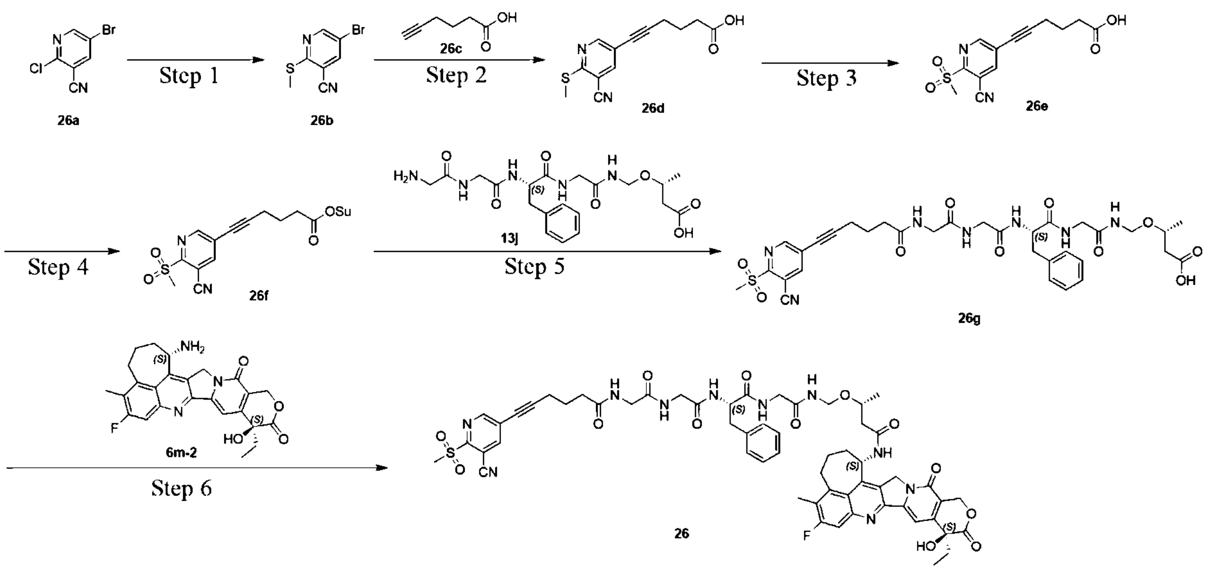


Step 1 5-bromo-2-(methylthio)nicotinonitrile **26b**

5-bromo-2-chloronicotinonitrile **26a** (2 g, 9.2 mmol) was dissolved in ethylene glycol dimethyl ether (20 mL), and sodium thiomethoxide (640 mg, 9.2 mmol) was added in an ice bath. The mixture was stirred for reaction at room temperature for 4 h. After the reaction was completed, the reaction was quenched with an aqueous ammonium chloride solution, the system was extracted with ethyl acetate three times, and dried over anhydrous sodium sulfate. The organic phases were combined and concentrated. The resulting concentrate was purified by silica gel column chromatography with system B to give the title product **26b** (1.5 g, yield: 71%).
MS m/z (ESI): 228.9, 230.9 $(M+1)^+$.

Step 2 6-(5-cyano-6-(methylthio)pyridin-3-yl)hex-5-ynoic acid **26d**

5-bromo-2-(methylthio)nicotinonitrile **26b** (1.5 g, 6.6 mmol) was dissolved in a mixed solvent of tetrahydrofuran (5 mL) and triethylamine (5 mL), and hex-5-ynoic acid **26c** (1.5 g, 13.2 mmol), bistriphenylphosphine palladium dichloride (913 mg, 1.3 mmol) and cuprous iodide (133 mg, 0.7 mmol) were added. The reaction mixture was allowed to react with stirring at 70°C for 2 h. After the reaction was completed, the reaction mixture was filtered and the mother liquor was directly blended. The resulting crude product was purified by silica gel column chromatography with system B to give 6-(5-cyano-6-(methylthio)pyridin-3-yl)hex-5-ynoic acid **26d** (0.7 g, yield: 41%).
MS m/z (ESI): 261.1 $(M+1)^+$。

Step 3 6-(5-cyano-6-(methylsulfonyl)pyridin-3-yl)hexyl-5-ynoic acid **26e**

6-(5-cyano-6-(methylthio)pyridin-3-yl)hexyl-5-ynoic acid **26d** (0.7 g, 2.7 mmol) was dissolved in methanol (10 mL) and water (10 mL) and potassium peroxymonosulfonate (9.3 g, 27 mmol) was added. The mixture was stirred for reaction at room temperature for two hours. After the reaction was completed, the reaction mixture was filtered, the mother liquor was poured into water, extracted with dichloromethane three times, and dried over anhydrous sodium sulfate. The organic phases were combined and concentrated. The filtrate was collected to give the crude product **26e** (815 mg), which was directly used in the next reaction step without purification.
MS m/z (ESI): 293.0 $(M+1)^+$.

Step 4 2,5-dioxopyrrolidin-1-yl-6-(5-cyano-6-(methylsulfonyl)pyridin-3-yl)hexyl-5-ynoate **26f**

The crude product **26e** (815 mg) was dissolved in dichloromethane (10 mL), and *N*-hydroxysuccinimide (345 mg, 3.0 mmol) and 1-ethyl-(3-dimethylaminopropyl)carbodiimide hydrochloride (1.04 g, 5.4 mmol) were added and stirred at room temperature for 2 h. After the reaction was completed, the reaction mixture was poured into water, extracted with dichloromethane three times, dried over anhydrous sodium sulfate, and the organic phases were combined and concentrated. The resulting concentrate was purified by silica gel column chromatography with system B to give the title product **26f** (400 mg, yield: 37%).
MS m/z (ESI): 390.0 $(M+1)^+$.

Step 5 (3*R*,11*S*)-11-benzyl-24-(5-cyano-6-(methylsulfonyl)pyridin-3-yl)-3-methyl-7,10,13,16,19-pentaoxo-4-oxa-6,9,12,15,18-pentaazatetracarbonyl-23-enoic acid **26g**

The compound **26f** (200 mg, 0.5 mmol) was dissolved in *N*,*N*-dimethylformamide (5 mL), and the compound **13j** (249 mg, 0.6 mmol) and *N*,*N*-diisopropylethylamine (130 mg, 1 mmol) were added, and the reaction mixture was stirred at room temperature for 2 h. After the reaction was completed, the reaction mixture was purified by preparative high performance liquid chromatography (Waters MS-triggered Prep-LC with SQD2 detector, chromatographic column: Xbridge C18 150 x 19 mm, 5μm; mobile phase 1: water (containing 0.1% FA); mobile phase 2: acetonitrile; 15-min gradient, gradient proportion: acetonitrile phase 30%-40%, flow rate: 20 mL/min) to give the title compound **26g** (80 mg, yield: 21%).

MS m/z (ESI): 748.3 $(M+1)^+$.

Step 6 *N*-((7*S*,15*R*)-7-benzyl-17-(((1*S*,10*S*)-10-ethyl-6-fluoro-10-hydroxy-5-methyl-11,14-dioxo-1,2,3,4,10,14,16-octahydro-13*H*-cyclohepta[*de*]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(5-cyano-6-(methylsulfonyl)pyridin-3-yl)hexy-5-nylamide **26**

The compound **26g** (25 mg, 0.03 mmol) was dissolved in *N*,*N*-dimethylformamide (5 mL), the compound **6m-2** (15 mg, 0.03 mmol), *O*-(7-azabenzotriazole-1-yl)-*N,N,N',N'*-tetramethyluronium hexafluorophosphate (15 mg, 0.04 mmol) and *N*,*N*-diisopropylethylamine (8 mg, 0.06 mmol) were added, and the reaction was stirred at room temperature for 30 min. After the reaction was completed, the reaction mixture was purified by preparative high performance liquid chromatography (Waters MS-triggered Prep-LC with QDA detector, chromatographic column: WELCH Xtimate C18 21.2 x 250mm 10 μm; mobile phase 1: water (containing 0.1% FA); mobile phase 2: acetonitrile; 15-min gradient, gradient proportion: acetonitrile phase 45%-55%, flow rate: 30 mL/min) to give the title compound **026** (20 mg, yield: 51.7%).

MS m/z (ESI): 1157.3 $(M+1)^+$.

$^1H$ NMR (400 MHz, $CD_3OD$) δ 8.81 (d, 1H), 8.41 (d, 1H), 7.54 (s, 1H), 7.45 (d, 1H), 7.17 (s, 5H), 5.65 (dd, 1H), 5.57 (d, 1H), 5.43 - 5.30 (m, 3H), 4.71 (dd, 2H), 4.50 (dd, 1H), 4.22 (t, 1H), 3.81 (s, 4H), 3.64 (dd, 2H), 3.39 (s, 3H), 3.31 (s, 3H), 3.11 (dd, 1H), 2.90 (dd, 1H), 2.64 - 2.55 (m, 3H), 2.45 (d, 7H), 2.17 (dd, 1H), 2.00 - 1.86 (m, 5H), 1.28 (d, 3H), 1.00 (s, 3H).

Example **27**

*N*-((7*S*,15*R*)-7-benzyl-17-(((1*S*,10*S*)-10-ethyl-6-fluoro-10-hydroxy-5-methyl-11,14-dioxo-1,2,3,4,10,14,16-octahydro-13*H*-cyclohepta[*de*]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(4-cyclopropyl-2-(methylsulfonyl)pyrimidin-5-yl)hexy-5-nylamide

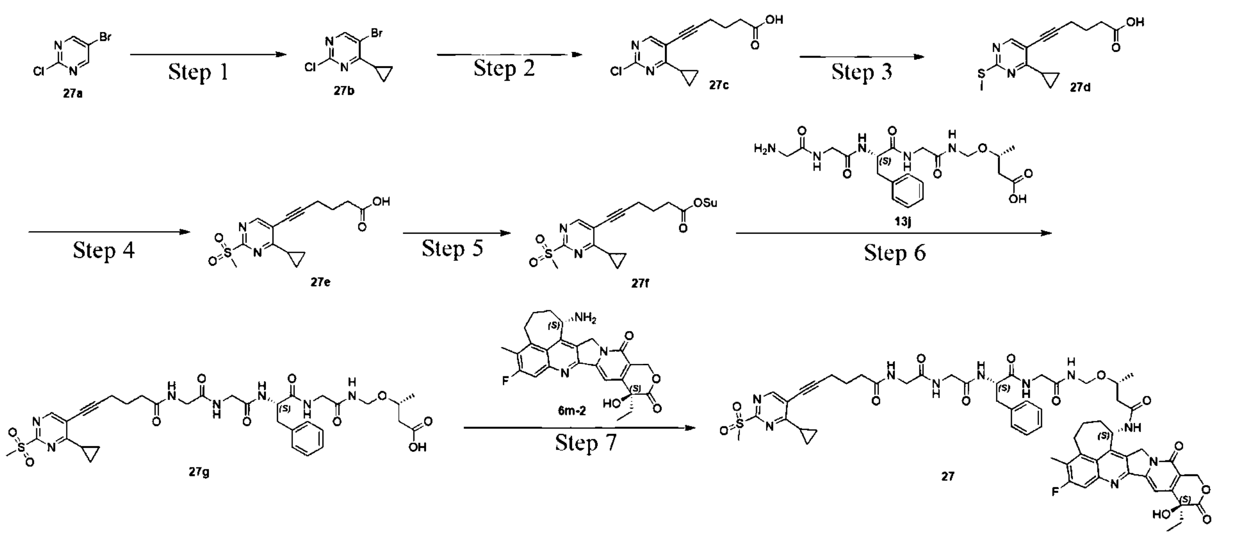


Step 1 5-bromo-2-chloro-4-cyclopropylpyrimidine **27b**

5-bromo-2-chloropyrimidine **27a** (8.50 g, 43.94 mmol) was added to water (50 mL), and cyclopropylcarboxylic acid (4.54 g, 52.73 mmol), silver nitrate (1.49 g, 8.79 mmol) and trifluoroacetic acid (2.51 g, 21.97 mmol) were added. The

reaction was heated to 70°C and stirred. Ammonium persulfate (20.01 g, 87.89 mmol) was slowly added, and the reaction was further stirred for 2 h. After the reaction was completed, the system was extracted with ethyl acetate. The organic phase was collected, washed with saturated brine, dried over anhydrous sodium sulfate, and spin-dried to remove the solvent, thus giving a crude product, which was purified by column chromatography with separation system B to give the title compound **27b** (6 g, yield: 58%).

MS m/z (ESI): 232.9, 235.0, 237.0 $(M+1)^+$.

Step 2 6-(2-Chloro-4-cyclopropylpyrimidin-5-yl)hexyl-5-ynoic acid **27c**

The compound **27b** (3 g, 12.85 mmol) was dissolved in tetrahydrofuran (20 mL), and 5-hexynoic acid (2.16 g, 19.28 mmol), bis(triphenylphosphine)palladium dichloride (1.80 g, 2.57 mmol), cuprous iodide (246 mg, 1.29 mmol) and triethylamine (3.9 g, 38.55 mmol) were added. The reaction was allowed to proceed with stirring under nitrogen protection at 70°C for 2 h. After the reaction was completed, the mixture was filtered. The filtrate was collected, spin-dried to remove the solvent, thus giving a crude product, which was purified by column chromatography with separation system B to give the title compound **27c** (1.9 g, yield: 56%).

MS m/z (ESI): 265.1, 267.1 $(M+1)^+$.

Step 3 6-(4-cyclopropyl-2-(methylthio)pyrimidin-5-yl)hexyl-5-ynoic acid **27d**

The compound **27c** (600 mg, 2.27 mmol) was dissolved in *N*,*N*-dimethylformamide (5 mL), and sodium thiomethoxide (191 mg, 2.72 mmol) and anhydrous magnesium sulfate (644 mg, 4.54 mmol) were added. The reaction was allowed to proceed with stirring at 50°C for 2 h. Subsequently, a saturated aqueous ammonium chloride solution was added into the system, and the mixture was extracted with dichloromethane. The organic phase was collected and the solvent was removed by rotary evaporation to give a crude product **27d** (600 mg). The crude product was directly used in the next reaction step without purification.

MS m/z (ESI): 277.0 $(M+1)^+$.

Step 4 6-(4-cyclopropyl-2-(methylsulfonyl)pyrimidin-5-yl)hexyl-5-ynoic acid **27e**

The compound **27d** (600 mg, 2.17 mmol) was dissolved in a mixed solvent of methanol and water (V/V=1:1, 10 mL), and potassium peroxymonosulfonate (6.67 g, 10.86 mmol) was added. The reaction was allowed to proceed at room temperature for half an hour. After the reaction was completed, the mixture was filtered and the filtrate was collected. The filtrate was extracted with dichloromethane. The organic phase was collected, dried over anhydrous sodium sulfate, washed with saturated brine, and spin-dried to remove the solvent, thus giving a crude product, which was purified by column chromatography with separation system B to give the title compound **27e** (200 mg, yield: 30%).

MS m/z (ESI): 309.1 $(M+1)^+$.

Step 5 2,5-dioxopyrrolidin-1-yl-6-(4-cyclopropyl-2-(methylsulfonyl)pyrimidin-5-yl)hexyl-5-ynoate **27f**

The compound **27e** (200 mg, 0.65 mmol) was dissolved in dichloromethane, and *N*-hydroxysuccinimide (82 mg, 0.71 mmol) and 1-ethyl-(3-dimethylaminopropyl)carbodiimide hydrochloride (249 mg, 1.30 mmol) were added. The reaction was allowed to proceed at room temperature for one hour. After the reaction was completed, the mixture was spin-dried to remove the solvent, thus giving a crude product, which was purified by column chromatography with separation system B to give the title compound **27f** (200 mg, yield: 76%).

MS m/z (ESI): 406.0 $(M+1)^+$.

Step 6 (3*R*,11*S*)-11-benzyl-24-(4-cyclopropyl-2-(methylsulfonyl)pyrimidin-5-yl)-3-methyl-7,10,13,16,19-pentaoxo-4-oxa-6,9,12,15,18-pentaazatetracarbonyl-23-enoic acid **27g**

The compound **27f** (80 mg, 0.20 mmol) was dissolved in *N*,*N*-dimethylformamide (2 mL), and the compound **13j** (107 mg, 0.23 mmol) and *N*,*N*-diisopropylethylamine (51 mg, 0.39 mmol) were added. The mixture was stirred for reaction at room temperature for 1 h. After the reaction was completed, the reaction mixture was purified by preparative high performance liquid chromatography (Waters MS-triggered Prep-LC with SQD2 detector, chromatographic column: Xbridge C18 150 x 19 mm, 5μm; mobile phase 1: water (containing 0.1% FA); mobile phase 2: acetonitrile; 10-min gradient, gradient proportion: acetonitrile phase 40%-50%, flow rate: 20 mL/min) to give the title compound **27g** (50 mg, yield: 34%).

MS m/z (ESI): 764.2 $(M+23)^+$.

Step 7 *N*-((7*S*,15*R*)-7-benzyl-17-(((1*S*,10*S*)-10-ethyl-6-fluoro-10-hydroxy-5-methyl-11,14-dioxo-1,2,3,4,10,14,16-octahydro-13*H*-cyclohepta[de]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(4-cyclopropyl-2-(methylsulfonyl)pyrimidin-5-yl)hexy-5-nylamide **27**

The compound **6m-2** (15 mg, 0.03 mmol) was dissolved in *N*,*N*-dimethylformamide (1 mL), the compound **27g** (25 mg, 0.03 mmol), *O*-(7-azabenzothiazol-1-yl)-*N,N,N',N'*-tetramethyluronium hexafluorophosphate (15 mg, 0.04 mmol) and *N*,*N*-dipropylamine (9 mg, 0.07 mmol) were added. The reaction was stirred at room temperature for 15 min. After the reaction was completed, the reaction mixture was purified by preparative high-performance liquid chromatography (Waters MS-triggered Prep-LC with QDA detector, chromatographic column: WELCH Xtimate C18 21.2*250mm, 10μm; mobile phase 1: water (containing 0.1% FA); mobile phase 2: acetonitrile; 10-min gradient, gradient proportion: acetonitrile phase 40%-70%, flow rate: 30 mL/min) to give the title compound **27** (18 mg, yield: 46%).
MS m/z (ESI): 1173.3 $(M+1)^+$.
$^1H$ NMR (400 MHz, DMSO-$d_6$) δ 8.86 (s, 1H), 8.67 (d, 1H), 8.49 (t, 1H), 8.29 (t, 1H), 8.20 (t, 1H), 8.12 (d, 1H), 8.06 (t, 1H), 7.73 (d, 1H), 7.30 (s, 1H), 7.23 - 7.17 (m, 4H), 7.17 - 7.11 (m,1H), 6.53 (s, 1H), 5.59 - 5.48 (m, 1H), 5.47 - 5.37 (m, 2H), 5.29 (s, 2H), 4.63 - 4.54 (m, 1H), 4.53 - 4.44 (m, 2H), 4.04 - 3.94 (m, 2H), 3.74 (d, 1H), 3.71 - 3.66 (m, 4H), 3.65 (d, 1H), 3.60 (d, 1H), 3.55 (d, 1H), 3.02 (dd, 2H), 2.86 - 2.72 (m, 3H), 2.41 (s, 3H), 2.28 - 2.22 (m, 1H), 2.05 - 1.97 (m, 1H), 1.92 -1.88 (m, 1H), 1.87 - 1.79 (m, 4H), 1.79 - 1.73 (m, 1H), 1.32 - 1.26 (m, 2H), 1.23 (s, 2H), 1.20 - 1.15 (m, 2H), 1.13 (d, 3H), 0.86 (t, 3H).

### Example 28

*N*-((7*S*,15*R*)-7-benzyl-17-(((1*S*, 10*S*)-10-ethyl-6-fluoro-10-hydroxy-5-methyl-11,14-dioxo-1,2,3,4,10,14,16-octahydro-13H-cyclohepta[*de*]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(4-methoxy-2-(methylsulfonyl)pyrimidin-5-yl)hexy-5-nylamide

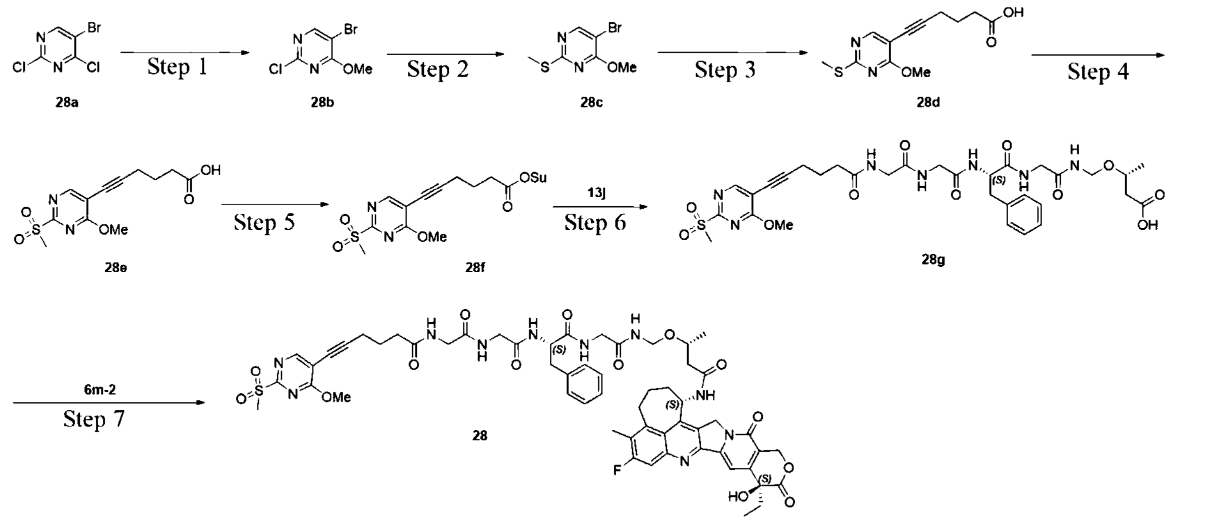


Step 1 5-Bromo-2-chloro-4-methoxypyrimidine **28b**

5-bromo-2,4-dichloropyrimidine **28a** (5.0 g, 22 mmol) was dissolved in methanol (60 mL), and a solution of sodium methoxide in methanol (30%, 4.0 g, 22.0 mol) was added to the solution. The reaction mixture was stirred at room temperature for 2 h. After the reaction was completed, the reaction mixture was concentrated and extracted with ethyl acetate. The organic phase was dried and concentrated to give the title compound **28b** (5.0 g, yield: 100%).
MS m/z (ESI): 223.1 $(M+1)^+$.

Step 2 5-bromo-4-methoxy-2-(methylthio)pyrimidine **28c**

To a solution of the compound **28b** (5.0 g, 22.0 mmol) in *N*,*N*-dimethylformamide (25 mL) was added sodium thiomethoxide (1.52 g, 22 mmol), and the reaction mixture was stirred at 40°C for 1 h. After the reaction was completed, the reaction mixture was poured into water and extracted with ethyl acetate (50 mL x 3). The organic phases were combined and dried. The concentrated crude product was purified by silica gel column chromatography with system B to give the title

compound **28c** (3.0 g, yield: 60%).
MS m/z (ESI): 235.0 $(M+1)^+$.

Step 3 6-(4-methoxy-2-(methylthio)pyrimidin-5-yl)hexyl-5-ynoic acid **28d**

The compound **28c** (3.0 g, 12.8 mmol) was dissolved in isopropanol (20 mL), and hex-5-ynoic acid (1.43 g, 12.8 mmol), cuprous iodide (243 mg, 1.29 mmol), [1,1'-bis(diphenylphosphino)ferrocene]dichloropalladium(II) (300 mg, 0.41 mmol) and a sodium carbonate solution (5M, 8 mL) were added. The reaction mixture was stirred under nitrogen atmosphere at 80°C for 16 h. After the reaction was completed, the reaction mixture was filtered and the filtrate was extracted with ethyl acetate (50 mL x 3). The organic phases were combined, dried and concentrated. The resulting crude product was purified by silica gel column chromatography with system B to give the title compound **28d** (3.0 g, yield: 87%).
MS m/z (ESI): 267.1 $(M+1)^+$.

Step 4: 5-bromo-2,4-dichloropyrimidine-6-(4-methoxy-2-(methylsulfonyl)pyrimidin-5-yl)hexyl-5-ynoic acid **28e**

The compound **28d** (3.0 g, 11.2 mmol) was dissolved in a mixed solvent of methanol and water (40 mL, V/V=1:1), potassium peroxodisulfate (11.6 g, 33.6 mmol) was added, and the reaction mixture was stirred at room temperature for 3 h. After the reaction was completed, the reaction mixture was poured into water and extracted with ethyl acetate (50 mL x 3). The organic phases were combined and dried over anhydrous sodium sulfate to give a crude product. The crude product was purified by silica gel column chromatography with system B to give the title compound **28e** (2.5 g, yield: 75%).
MS m/z (ESI): 299.1 $(M+H)^+$.

Step 5 2,5-dioxopyrrolidin-1-yl-6-(4-methoxy-2-(methylsulfonyl)pyrimidin-5-yl)hexyl-5-ynoate **28f**

To a solution of the compound **28e** (700 mg, 2.3 mmol) and *N*-hydroxysuccinimide (396 mg, 3.45 mmol) in tetrahydrofuran (10 mL) was added *N*,*N*-diisopropylcarbodiimide (434 mg, 3.45 mmol), and the reaction mixture was stirred at room temperature for 3 h. After the reaction was completed, the reaction mixture was concentrated directly to give a crude product, and the crude product was purified by silica gel column chromatography with system B to give the title compound **28f** (600 mg, yield: 60%).
MS m/z (ESI): 395.5 $(M+1)^+$.

Step 6 (3*R*,11*S*)-11-benzyl-24-(4-methoxy-2-(methylsulfonyl)pyrimidin-5-yl)-3-methyl-7,10,13,16,19-pentaoxo-4-oxa-6,9,12,15,18-pentaazatetracarbonyl-23-enoic acid **28g**

The compound **28f** (600 mg, 1.5 mmol) was dissolved in *N*,*N*-dimethylformamide (6 mL), *N,N*-diisopropylethylamine (580 mg, 4.5 mmol) and the compound **13j** (677 mg, 1.5 mmol) were added to the solution in sequence, and the reaction mixture was stirred at room temperature for 3 h. The system was concentrated to give a crude product. The crude product was purified by preparative high performance liquid chromatography (Waters MS-triggered Prep-LC with QDA detector, chromatographic column: Xbridge C18 150 x 19 mm, 5μm; mobile phase 1: water (containing 0.1% FA); mobile phase 2: acetonitrile; 15-min gradient, gradient proportion: acetonitrile phase 29%-100%, flow rate: 25 mL/min) to give the title product **28g** (350 mg, yield: 35%).
MS m/z (ESI): 754.1 $(M+Na)^+$.

Step 7 N-((7*S*,15*R*)-7-benzyl-17-(((1*S*,10*S*)-10-ethyl-6-fluoro-10-hydroxy-5-methyl-11,14-dioxo-1,2,3,4,10,14,16-octahydro-13*H*-cyclohepta[de]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(4-methoxy-2-(methylsulfonyl)pyrimidin-5-yl)hexy-5-nylamide **28**

The compound **28g** (25 mg, 0.034 mmol) was dissolved in *N*,*N*-dimethylformamide (3 mL), and the compound **6m-2** (15 mg, 0.033 mmol), 2-(7-azabenzotriazole)-*N,N,N',N'*-tetramethyluronium hexafluorophosphate (25 mg, 0.065 mmol), and *N*,*N*-diisopropylethylamine (171 mg, 1.32 mmol) were added. The reaction mixture was stirred at room temperature for 3 h. After the reaction was completed, the system was concentrated to give a crude product. The crude product was purified by preparative high performance liquid chromatography (Waters MS-triggered Prep-LC with QDA detector, chromatographic column: WELCH Xtimate C18 21.2 x 250mm 10 μm; mobile phase 1: water (containing 0.1% FA); mobile phase 2: acetonitrile; 15-min gradient, gradient proportion: acetonitrile phase 40%-70%, flow rate: 30 mL/min) to give the title compound **28** (16 mg, yield: 42%).
MS m/z (ESI): 1163.2 $(M+1)^+$.
$^1H$ NMR (400 MHz, $CDCl_3$) δ 8.56 (s, 1H), 8.43 (s, 1H), 7.52 - 7.48 (m, 2H), 7.40 - 7.29 (m, 3H), 7.24 - 7.04 (m, 6H), 6.94 (s, 1H), 5.60 - 5.55 (m, 2H), 5.35 - 5.32 (m, 1H), 5.16 (d, 2H), 5.04 - 4.56 (m, 4H), 4.25 -3.73 (m, 9H), 3.30 (s, 3H), 3.26 -

2.93 (m, 4H), 2.60 - 2.42(m, 7H), 2.26 (s, 3H), 2.17 - 1.74 (m, 8H), 1.31 (d, 3H), 0.97 (t, 3H).

**Example 29**

(*R*)-3-(((*S*)-7-benzyl-17-(4-(2-(methylsulfonyl)pyrimidin-5-yl)piperidin-1-yl)-3,6,9,12,15-pentaoxo-2,5,8,11,14-pentaazaheptadecyl)oxy)-N-((1*S*,10*S*)-10-ethyl-6-fluoro-10-hydroxy-5-methyl-11,14-dioxo-1,3,4,10,11,14,16-octahydro-13*H*-cycloheptane[*de*]pyrano[3',4':6,7]indolizino[1,*b*]quinolin-1-yl)butanamide

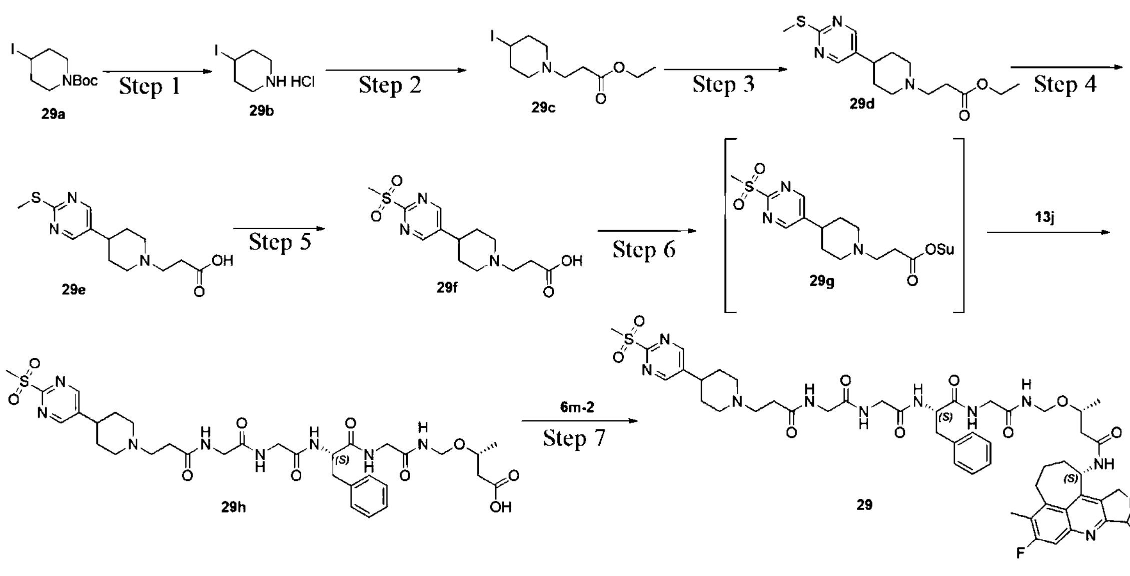


Step 1 4-iodopiperidine hydrochloride **29b**

Tert-butyl 4-iodopiperidine-1-carboxylate **29a** (4 g, 12.86 mmol) was added to a reaction flask, and a solution of 4 M hydrochloric acid in 1,4-dioxane (20 ml) was added at room temperature, and the reaction was carried out at room temperature for one hour. After the reaction was completed, the system was spin-dried to remove the solvent to give crude 4-iodopiperidine hydrochloride **29b** (2.7 g, yield: 100%).

MS m/z (ESI): 212.0 $(M+1)^+$.

Step 2 Ethyl 3-(4-iodopiperidin-1-yl)propanoate **29c**

4-iodopiperidine hydrochloride **29b** (2.7 g, 10.93 mmol), ethyl 3-bromopropionate (2.95 g, 16.40 mmol) and potassium carbonate (7.54 g, 54.65 mmol) were added to a reaction flask, and acetonitrile (50 ml) was added. The reaction was allowed to proceed at 60°C for 16 h. After the reaction was completed, the solvent in the reaction system was removed by spin-drying, methyl tert-butyl ether (50 mL) was added and the mixture was slurried for half an hour. The system was filtered, and the filtrate was spin-dried to give the crude product **29c** (3.1 g), which was directly used in the next reaction step without purification.

MS m/z (ESI): 312.1$(M+1)^+$.

Step 3 Ethyl 3-(4-(2-(methylthio)pyrimidin-5-yl)piperidin-1-yl)propanoate **29d**

Nickel chloride dimethoxyethane (299 mg, 1.37 mmol) and 2-amidinopyridine hydrochloride (215 mg, 1.37 mmol) were added to a reaction flask, dimethylacetamide (10 ml) was added under nitrogen atmosphere, and the mixture was stirred at room temperature for half an hour. In another reaction flask, the compound **29c** (1.7 g, 5.46 mmol), 5-bromo-2-(methylthio)pyrimidine (1.1 g, 5.46 mmol), manganese (75 mg, 1.37 mmol), tetrabutylammonium iodide (2.0 g, 5.46 mmol) were added, and then *N,N*-dimethylacetamide (10 ml) was added, nitrogen replacement was carried out three times. After the replacement was completed, the nickel ligand compound was transferred to the system with a syringe, and the reaction was carried out at 60°C for 3 h. After the reaction was completed, ethyl acetate (200 ml) and water (400 ml) were added to the system for extraction. The organic phase was dried, concentrated, and purified by silica gel column

chromatography with system B to give the title compound **29d** (1.5 g, yield: 89%).

MS m/z (ESI): 310.2(M+1)$^+$.

Step 4 3-(4-(2-(methylthio)pyrimidin-5-yl)piperidin-1-yl)propanoic acid **29e**

The compound **29d** (1.5 g, 4.84 mmol) was added to a mixed solvent of tetrahydrofuran and water (15 mL, V/V = 2:1), lithium hydroxide (348 mg, 14.52 mmol) was added, and the reaction was carried out at room temperature for 16 h. After the reaction was completed, the system was filtered, concentrated and spin-dried to give the crude product **29e** (1.5 g), which was directly used in the next reaction step without purification.

MS m/z (ESI): 282.2(M+1)$^+$.

Step 5 3-(4-(2-(methylsulfonyl)pyrimidin-5-yl)piperidin-1-yl)propanoic acid **29f**

The compound **29e** (1.1 g) was dissolved in trifluoroacetic acid, and excess m-chloroperbenzoic acid was added until the reaction was completed at room temperature. After the reaction was completed, the system was blown dry with nitrogen to remove trifluoroacetic acid, and then water (30 ml) was added for slurrying. The mixture was filtered, and the filtrate was collected, concentrated, and purified by the column chromatography system A to give the compound **29f** (430 mg).

MS m/z (ESI): 314.1(M+1)$^+$.

Step 6 (3*R*,11*S*)-11-benzyl-3-methyl-21-(4-(2-(methylsulfonyl)pyrimidin-5-yl)piperidin-1-yl)-7,10,13,16,19-pentaoxo-4-oxa-6,9,12,15,18-pentaazaeicosanoic acid **29h**

The compound **29f** (430 mg, 1.37 mmol) and *N*-hydroxysuccinimide (189 mg, 1.64 mmol) were dissolved in ultra-dry *N*,*N*-dimethylformamide (10 ml), then *N*,*N*-diisopropylcarbodiimide (206 mg, 1.64 mmol) was added, and the reaction mixture was stirred at room temperature for 2 h. The compound **13j** (300 mg, 0.66 mmol) and *N*,*N*-diisopropylethylamine (213 mg, 1.65 mmol) were then added by the one-pot method. After the reaction was completed, the crude product was purified by preparative high performance liquid chromatography (Waters MS-triggered Prep-LC with QDA detector, chromatographic column: WELCH Xtimate C18 21.2x250 mm 10 μm; mobile phase 1: water (containing 0.1% FA); mobile phase 2: acetonitrile; 15-min gradient, gradient proportion: acetonitrile phase 40%-95%, flow rate: 30 mL/min) to give the title compound **29h** (200 mg).

MS m/z (ESI): 747.3(M+1)$^+$.

Step 7 (*R*)-3-(((*S*)-7-benzyl-17-(4-(2-(methylsulfonyl)pyrimidin-5-yl)piperidin-1-yl)-3,6,9,12,15-pentaoxo-2,5,8,11,14-pentaazaheptadecyl)oxy)-*N*-((1*S*,10*S*)-10-ethyl-6-fluoro-10-hydroxy-5-methyl-11,14-dioxo-1,3,4,10,11,14,16-octahydro-13*H*-cycloheptane[*de*]pyrano[3',4':6,7]indolizino[1,*b*]quinolin-1-yl)butanamide

The compound **29h** (25 mg, 0.033 mmol), the compound **6m-2** (15 mg, 0.033 mmol), 2-(7-azabenzothiazole)-*N*,*N*,*N'*,*N'*-tetramethyluronium hexafluorophosphate (19 mg, 0.0495 mmol) and *N*,*N*-diisopropylethylamine (10.6 mg, 0.0825 mmol) were added to ultra-dry *N,N*-dimethylformamide (5 ml). The reaction was carried out at room temperature for 2 h. After the reaction was completed, the final product was purified by preparative high-performance liquid chromatography (Waters MS-triggered Prep-LC with QDA detector, chromatographic column: WELCH Xtimate C18 21.2*250mm, 10μm; mobile phase 1: water (containing 0.1% FA); mobile phase 2: acetonitrile; 15-min gradient, gradient proportion: acetonitrile phase 40%-95%, flow rate: 30 mL/min) to give the title compound **29** (16.7 mg, yield: 42.3%).

MS m/z (ESI): 1178.4(M+1)$^+$.

$^1$H NMR (400 MHz, DMSO-$d_6$) δ 9.00 (s, 2H), 8.66 (d, 1H), 8.48 (t, 1H), 8.35 (t, 1H), 8.27 (t, 1H), 8.11(d, 1H), 8.02 (t, 1H), 7.74 (d, 1H), 7.30 (s, 1H), 7.21 (d, 4H), 7.18 - 7.12 (m, 1H), 6.51 (s, 1H), 5.53 (s, 1H), 5.42(s, 2H), 5.29 (s, 2H), 4.58 (dd, 1H), 4.49 (dd, 2H), 3.99 (dd, 1H), 3.78 - 3.58 (m, 6H), 3.39 (s, 3H), 3.00 (t, 3H), 2.78 - 2.66 (m, 2H), 2.57 (t, 3H), 2.43 (t, 4H), 2.38 - 2.21 (m, 5H), 2.02 (t, 3H), 1.82 (ddd, 8H), 1.13 (d, 3H), 0.86 (t, 3H).

**Example 30**

*N*-((7*S*,15*R*)-7-benzyl-17-(((1*S*, 9*S*)-4-cyclopropyl-9-ethyl-5-fluoro-9-hydroxy-10,13-dioxo-2,3,9,10,13,15-hexahydro-1*H*,12*H*-benzo[*de*]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(4-methoxy-2-(methylsulfonyl)pyrimidin-5-yl)hexy-5-nylamide

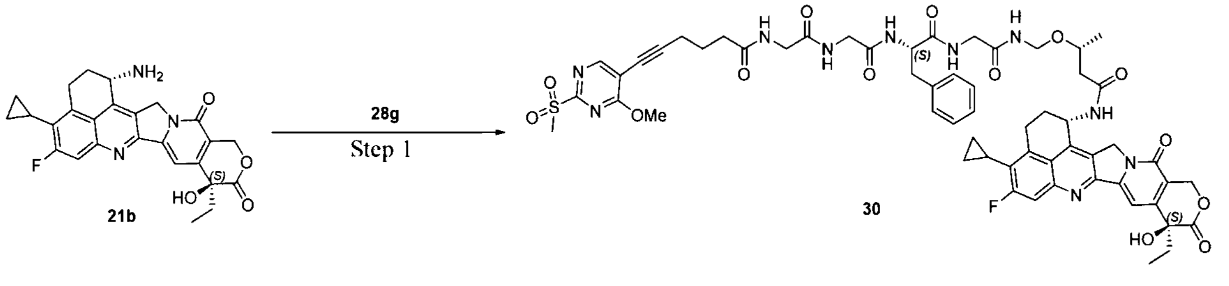


21b 30

Step 1

*N*-((7*S*,15*R*)-7-benzyl-17-(((1*S*, 9*S*)-4-cyclopropyl-9-ethyl-5-fluoro-9-hydroxy-10,13-dioxo-2,3,9,10,13,15-hexahydro-1*H*,12*H*-benzo[*de*]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(4-methoxy-2-(methylsulfonyl)pyrimidin-5-yl)hexy-5-nylamide **30**

The compound **21b** (30 mg , 0.065 mmol) was dissolved in anhydrous *N,N*-dimethylformamide (5 mL), and the compound **28g** (43 mg, 0.064 mmol), 2-(7-azabenzotriazole)-*N,N,N',N'*-tetramethyluronium hexafluorophosphate (37 mg, 0.096 mmol), and N,N-diisopropylethylamine (171 mg, 1.32 mmol) were added. The reaction mixture was stirred at room temperature for 2 h. After the reaction was completed, water (50 mL) was added into the reaction mixture which was then extracted with ethyl acetate (30 mLx3). The organic phases were combined, washed with saturated brine, dried, and concentrated. The crude product was purified by preparative high performance liquid chromatography (Waters MS-triggered Prep-LC with QDA detector, chromatographic column: WELCH Xtimate C18 21.2 x 250mm 10 um; mobile phase 1: water (containing 0.1% FA); mobile phase 2: acetonitrile; 15-min gradient, gradient proportion: acetonitrile phase 42%-52%, flow rate: 30 mL/min) to give the title compound **30** (18.5 mg, 23.5%).

MS m/z (ESI): 1175.3(M+1)$^+$.

$^1$H NMR (400 MHz, DMSO-$d_6$) δ 8.79 (s, 1H), 8.53 - 8.45 (m, 2H), 8.30 (t, 1H), 8.17 (t, 1H), 8.12 (d, 1H), 8.05 (t, 1H), 7.74 (d, 1H), 7.30 (s, 1H), 7.27 - 7.14 (m, 5H), 6.52 (s, 1H), 5.59 - 5.55(m,1H), 5.42 (s, 2H), 5.20 (dd, 2H), 4.55 - 4.51 (m, 3H), 4.09 - 3.97 (m, 4H), 3.78 - 3.54 (m, 6H), 3.39 (s, 3H), 3.31 (s, 3H), 3.04 (dd, 1H), 2.80 - 2.75 (m, 1H), 2.56 - 2.53 (m, 1H), 2.46 - 2.42 (m, 1H), 2.35 - 2.20 (m, 4H), 2.16 - 2.12 (m, 2H), 1.94-1.78 (m, 4H), 1.15-1.12 (m, 5H), 0.87 (t, 3H), 0.79 - 0.72 (m, 2H).

**Example 31**

*N*-((7*S*,15*R*)-7-benzyl-17-(((1*S*,9*S*)-4-cyclopropyl-9-ethyl-5-fluoro-9-hydroxy-10,13-dioxo-2,3,9,10,13,15-hexahydro-1*H*,12*H*-benzo[*de*]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(5-cyano-6-(methylsulfonyl)pyridin-3-yl)hexy-5-nylamide

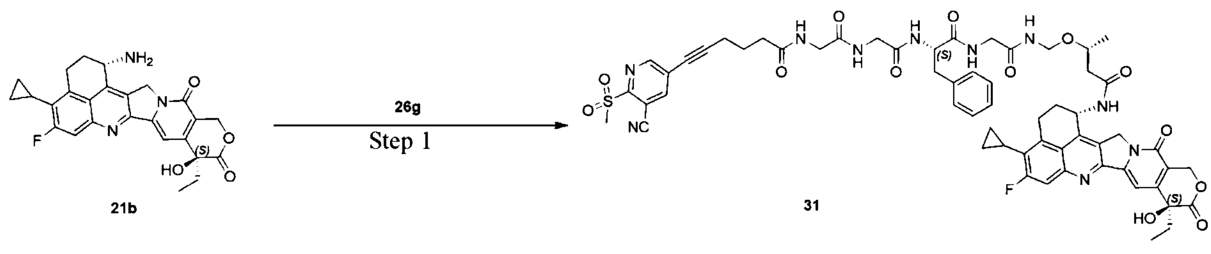


21b 31

Step 1

*N*-((7*S*,15*R*)-7-benzyl-17-(((1*S*,9*S*)-4-cyclopropyl-9-ethyl-5-fluoro-9-hydroxy-10,13-dioxo-2,3,9,10,13,15-hexahydro-1*H*,12*H*-benzo[*de*]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(5-cyano-6-(methylsulfonyl)pyridin-3-yl)hexy-5-nylamide **31**

The compound **26g** (50 mg, 0.06 mmol) was dissolved in *N,N*-dimethylformamide (5 mL), and the compound **21b** (30 mg, 0.06 mmol), 2-(7-azabenzotriazole)-*N,N,N',N'*-tetramethyluronium hexafluorophosphate (30 mg, 0.08 mmol), and N,N-diisopropylethylamine (16 mg, 0.12 mmol) were added. The mixture was stirred for reaction at room temperature

for 30 min. After the reaction was completed, the reaction mixture was purified by preparative high performance liquid chromatography (Waters MS-triggered Prep-LC with SQD2 detector, chromatographic column: WELCH Xtimate C18 21.2 x 250mm 10um; mobile phase 1: water (containing 0.1% FA); mobile phase 2: acetonitrile; 15-min gradient, gradient proportion: acetonitrile phase 42%-52%, flow rate: 20 mL/min) to give the title compound **31** (28 mg, yield: 40%).

MS m/z (ESI): 1169.3 $(M+1)^+$.

$^1H$ NMR (400 MHz, $CD_3OD$) δ 8.77 (d, 1H), 8.37 (d, 1H), 7.57 (s, 1H), 7.51 (d, 1H), 7.23 - 7.19 (m, 2H), 7.15 (s, 3H), 5.58 (dd, 2H), 5.38 - 5.25 (m, 2H), 5.07 (d, 1H), 4.69 (d, 2H), 4.46 - 4.39 (m, 1H), 4.11 (dd, 1H), 3.85 (s, 5H), 3.59 (dd, 1H), 3.51-3.45 (m, 1H), 3.37 (s, 3H), 2.99 (ddd, 2H), 2.57 (t, 2H), 2.46 (dt, 4H), 2.34 - 2.22 (m, 2H), 2.01 - 1.81 (m, 6H), 1.26 (d, 3H), 1.13 (d, 2H), 0.97 (s, 3H), 0.80 (s, 2H).

**Example 32**

*N*-((7*S*,15*R*)-7-benzyl-17-((1*S*,9*S*)-9-ethyl-5-fluoro-9-hydroxy-10,13-dioxo-4-vinyl-2,3,9,10,13,15-hexahydro-1*H*,12*H*-benzo[*d*]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(4-methoxy-2-(methylsulfonyl)pyrimidin-5-yl)hexyl-5-amide

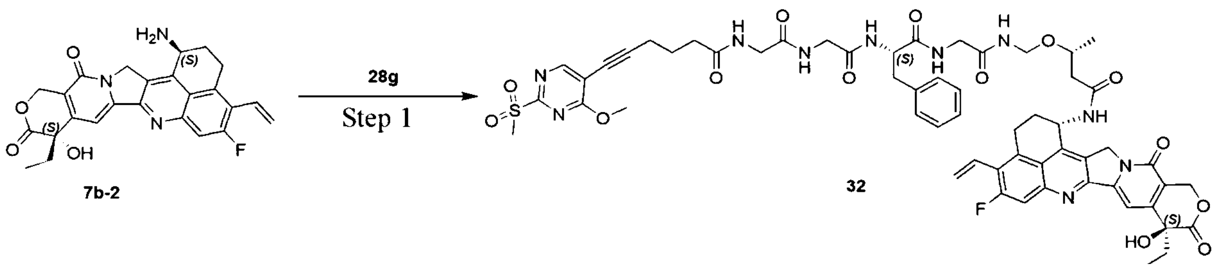


Step 1

*N*-((7*S*,15*R*)-7-benzyl-17-((1*S*,9*S*)-9-ethyl-5-fluoro-9-hydroxy-10,13-dioxo-4-vinyl-2,3,9,10,13,15-hexahydro-1*H*,12*H*-benzo[*d*]pyrano[3',4':6,7]indolizino[1,2-*b*]quinolin-1-yl)amino)-15-methyl-2,5,8,11,17-pentaoxo-14-oxa-3,6,9,12-tetraazaheptadecyl)-6-(4-methoxy-2-(methylsulfonyl)pyrimidin-5-yl)hexyl-5-amide **32**

The compound **7b-2** (20 mg, 0.04 mmol) was dissolved in *N,N*-dimethylformamide (5 mL), and the compound **28g** (32 mg, 0.04 mmol), 2-(7-azabenzotriazole)-*N,N,N',N'*-tetramethyluronium hexafluorophosphate (23 mg, 0.06 mmol), and *N,N*-diisopropylethylamine (11 mg, 0.09 mmol) were added. The mixture was stirred for reaction at room temperature for 30 min. After the reaction was completed, the crude product was purified by preparative high performance liquid chromatography (Waters MS-triggered Prep-LC with SQD2 detector, chromatographic column: Xbridge 5 μm C18 150 x 19 mm; mobile phase 1: water (containing 0.1% FA); mobile phase 2: acetonitrile; 12-min gradient, gradient proportion: acetonitrile phase 42%-52%, flow rate: 20 mL/min) to give the title compound **32** (24 mg, yield: 46%).

MS m/z (ESI): 1161.4 $(M+1)^+$.

$^1H$ NMR (400 MHz, DMSO-$d_6$) δ 8.79 (s, 1H), 8.52 (d, 1H), 8.31 (s, 1H), 8.16 (s, 1H), 8.10 (d, 1H), 8.04 (s, 1H), 7.85 (d, 1H), 7.34 (s, 1H), 7.25 - 7.15 (m, 4H), 6.97 (dd, 2H), 6.54 (s, 1H), 5.87 - 5.80 (m, 2H), 5.59 (s, 1H), 5.43 (s, 1H), 5.32 (d, 2H), 5.18 (d, 2H), 4.65 (s, 2H), 4.52 (d, 4H), 4.06 (s, 3H), 3.69 (d, 3H), 3.03 (d, 2H), 2.71 - 2.65 (m, 4H), 2.32 (dd, 4H), 2.11 (s, 3H), 1.91-1.75 (m, 6H), 1.12 (d, 3H), 0.90 - 0.82 (m, 3H).

**Example 33**

(*R*)-2-cyclopropyl-*N*-((1*S*,10*S*)-5-cyclopropyl-10-ethyl-6-fluoro-10-hydroxy-11,14-dioxo-1,2,3,4,10,11,14,16-octahydro-13*H*-cyclohepta[3',4':6,7]indolizino[1,2-*b*]quinolin-1-yl)-2-hydroxyacetamide

(*S*)-2-cyclopropyl-*N*-((1*S*,10*S*)-5-cyclopropyl-10-ethyl-6-fluoro-10-hydroxy-11,14-dioxo-1,2,3,4,10,11,14,16-octahydro-13*H*-cyclohepta[3',4':6,7]indolizino[1,2-*b*]quinolin-1-yl)-2-hydroxyacetamide

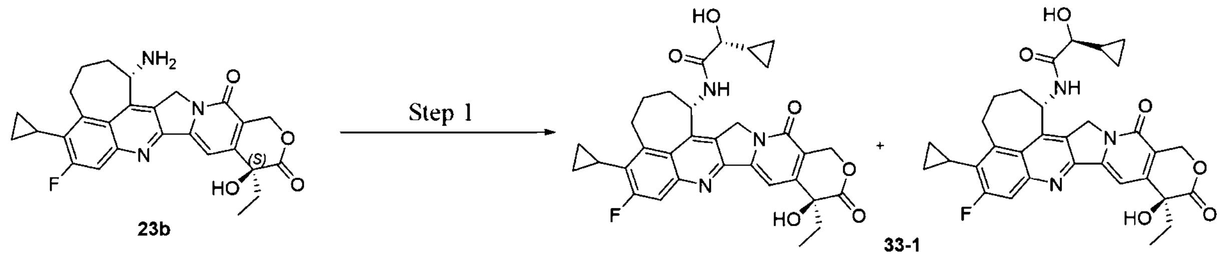


23b

33-1
33-2

Step 1

(*R*)-2-cyclopropyl-*N*-((1*S*,10*S*)-5-cyclopropyl-10-ethyl-6-fluoro-10-hydroxy-11,14-dioxo-1,2,3,4,10,11,14,16-octahydro-13*H*-cyclohepta[3',4':6,7]indolizino[1,2-*b*]quinolin-1-yl)-2-hydroxyacetamide

(S)-2-cyclopropyl-N-((1*S*,10*S*)-5-cyclopropyl-10-ethyl-6-fluoro-10-hydroxy-11,14-dioxo-1,2,3,4,11,14,16-octahydro-13*H*-cyclohepta[3',4':6,7]indolizino[1,2-*b*]quinolin-1-yl)-2-hydroxyacetamide

The compound **23b** (20 mg, 0.042 mmol) was dissolved in *N*,*N*-dimethylacetamide (2 mL), and 2-cyclopropyl-2-hydroxyacetic acid (5.4 mg, 0.046 mmol), benzotriazol-1-yloxytris(dimethylamino)phosphonium hexafluorophosphate (22 mg, 0.051 mmol), and *N,N*-diisopropylethylamine (11 mg, 0.084 mmol) were added. The reaction mixture was stirred at room temperature for 3 h. After the reaction was completed, the reaction mixture was purified by preparative high performance liquid chromatography (Waters MS-triggered Prep-LC with QDA detector, chromatographic column: WELCH Xtimate C18 21.2x250 mm 10 μm; mobile phase 1: water (containing 0.1% FA); mobile phase 2: acetonitrile; 15-min gradient, gradient proportion: acetonitrile phase 46%-100%, flow rate: 30 mL/min) to give products **33-1** (4.1 mg, yield: 17.2%) and **33-2** (1.7 mg, yield: 6.9%).

**33-1** (Mono-configurational compound, short retention time)

MS m/z (ESI): 574.2 $(M+1)^+$.
$^1$H NMR (400 MHz, DMSO-$d_6$) δ 8.62-8.37 (m, 2H), 7.69 (d, 1H), 7.28 (s, 1H), 6.53 (s, 1H), 5.59-5.48 (m, 2H), 5.43 (d, 2H), 5.28 (d, 1H), 3.62 (d, 1H), 3.49-3.46 (m, 1H), 2.19-1.72 (m, 6H), 1.69-1.53 (m, 1H), 1.23 (s, 1H), 1.18-1.00 (m, 3H), 0.85 (t, 3H), 0.83-0.77 (m, 1H), 0.74-0.62 (m, 1H), 0.46-0.28 (m, 4H).

**33-2** (Mono-configurational compound, long retention time)

MS m/z (ESI): 574.2 $(M+1)^+$.
$^1$H NMR (400 MHz, DMSO-$d_6$) δ 8.45 (d, 2H), 7.69 (d, 1H), 7.28 (s, 1H), 6.53 (s, 1H), 5.60-5.52 (m, 1H), 5.48 (s, 1H), 5.43 (d, 2H), 5.30 (d, 1H), 3.59 (d, 1H), 3.49-3.46 (m, 1H), 2.14-1.79 (m, 6H), 1.75-1.61 (m, 1H), 1.24 (s, 1H), 1.17-1.02 (m, 3H), 0.86 (t, *J*=8 Hz, 3H), 0.83-0.77 (m, 1H), 0.74-0.64 (m, 1H), 0.48-0.29 (m, 4H).

**Example 34**

(*S*)-3-cyclopropyl-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxypropanamide

(*R*)-3-cyclopropyl-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxypropanamide

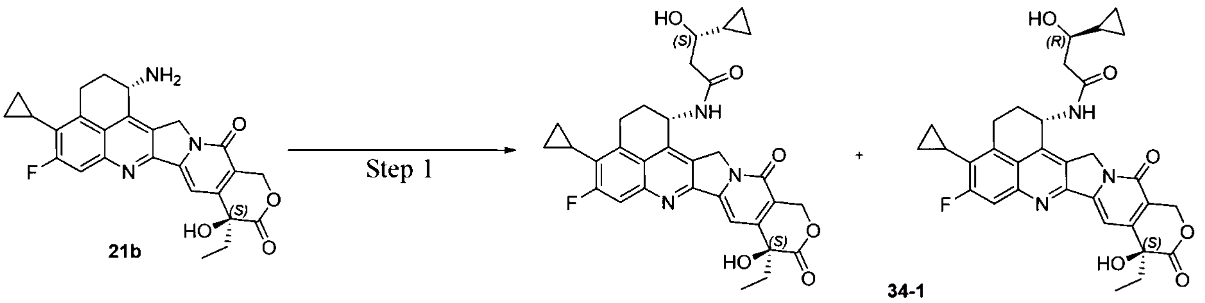


21b

34-1
34-2

Step 1

(*S*)-3-cyclopropyl-*N*-((1*S*,9*S*)-4-cyclopropyl-9-ethyl-5-fluoro-9-hydroxy-10,13-dioxo-2,3,9,10,13,15-hexahydro-1*H*,12*H*-benzo[*d*]pyrano[3',4':6,7]indolizino[1,2-*b*]quinolin-1-yl)-3-hydroxypropanamide

(*R*)-3-cyclopropyl-*N*-((1*S*,9*S*)-4-cyclopropyl-9-ethyl-5-fluoro-9-hydroxy-10,13-dioxo-2,3,9,10,13,15-hexahydro-1*H*,12H-benzo[*d*]pyrano[3',4':6,7]indolizino[1,2-*b*]quinolin-1-yl)-3-hydroxypropanamide

The compound **21b** (30 mg, 0.065 mmol) was dissolved in *N*,*N*-dimethylformamide (3 mL), and 3-cyclopropyl-3-hydroxypropionic acid (15 mg, 0.098 mmol), 2-(7-azabenzotriazole)-*N*,*N*,*N'*,*N'*-tetramethyluronium hexafluorophosphate (37 mg, 0.096 mmol), and *N,N*-diisopropylethylamine (171 mg, 1.32 mmol) were added. The reaction mixture was stirred at room temperature for 2 h. After the reaction was completed, the mixture was purified by preparative high performance liquid chromatography (Waters MS-triggered Prep-LC with QDA detector, chromatographic column: WELCH Xtimate C18 21.2x250 mm 10 μm; mobile phase 1: water (containing 0.1% FA); mobile phase 2: acetonitrile; 15-min gradient, gradient proportion: acetonitrile phase 40%-100%, flow rate: 30 mL/min) to give products **34-1** (4.2 mg, yield: 11%) and **34-2** (3.9 mg, yield: 10%).

**34-1 (Mono-configurational compound, compound with short retention time)**

MS m/z (ESI): 574.2 $(M+1)^+$.

$^1H$ NMR (400 MHz, DMSO-$d_6$) δ 8.53-8.39 (m, 2H), 7.75 (d, 1H), 7.30 (s, 1H), 6.53 (s, 1H), 5.55 (dd, 1H), 5.42 (s, 2H), 5.23 (s, 2H), 4.69 (s, 1H), 2.37 (d, 2H), 2.15 (d, 2H), 2.01-1.77 (m, 4H), 1.17-1.07 (m, 2H), 0.91-0.72 (m, 6H), 0.39-0.12 (m, 5H).

**34-2 (Mono-configurational compound, compound with long retention time)**

MS m/z (ESI): 574.2 $(M+1)^+$.

1H NMR (400 MHz, DMSO-$d_6$) δ 8.45 (d, 2H), 7.75 (d, 1H), 7.31 (s, 1H), 6.53 (s, 1H), 5.64-5.54 (m, 1H), 5.43 (s, 2H), 5.24 (q, 2H), 4.65 (d, 1H), 2.33 (d, 2H), 2.27-1.73 (m, 6H), 1.12 (dd, 2H), 0.91-0.70 (m, 6H), 0.39-0.13 (m, 5H).

**Example 35**

(*R*)-2-cyclopropyl-*N*-((1*S*,10*S*)-10-ethyl-6-fluoro-10-hydroxy-5-methyl-11,14-dioxo-2,3,10,11,14,16-hexahydro-1*H*,13*H*-oxepino[4,3,2-*de*]pyrano[3',4':6,7]indolizino[1,2-b]quinolin-1-yl)-2-hydroxyacetamide

(*S*)-2-cyclopropyl-*N*-((1*S*,10*S*)-10-ethyl-6-fluoro-10-hydroxy-5-methyl-11,14-dioxo-2,3,10,11,14,16-hexahydro-1*H*,13*H*-oxepino[4,3,2-*de*]pyrano[3',4':6,7]indolizino[1,2-*b*]quinolin-1-yl)-2-hydroxyacetamide

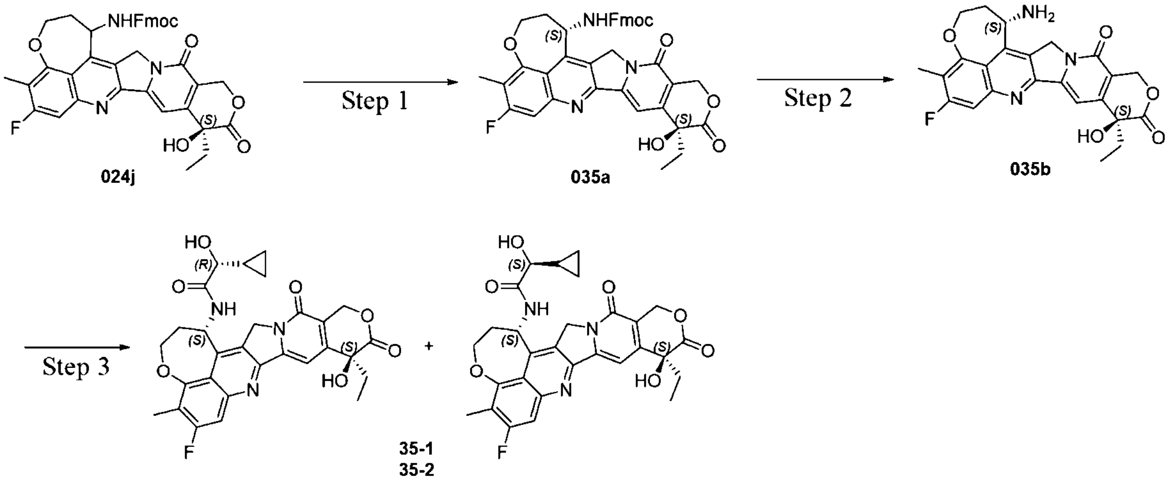


024j 035a 035b

35-1
35-2

Step 1

(9*H*-fluoro-9-yl)methyl((1*S*,10*S*)-10-ethyl-6-fluoro-10-hydroxy-5-methyl-11,14-dioxo-2,3,10,11,14,16-hexahydro-1*H*,13*H*-oxepino[4,3,2-*de*]pyrano[3',4':6,7]indolizino[1,2-*b*]quinolin-1-yl)carbamate **035a**

The compound **024j** (500 mg, 0.74 mmol) was purified using a Gilson preparative instrument and a Daicel chiral column to separate chiral isomers (chromatographic column: CHIRALPAK IE 3.0 cm I.D.×25 cm,10 μm; mobile phase 1: MeOH; mobile phase 2: DCM; gradient proportion: MeOH/DCM=80/20, flow rate: 25 mL/min) to give the compound **035a** (230 mg, yield: 46%).

MS m/z (ESI): 674.1 $(M+1)^+$.

Step 2

(1*S*,10*S*)-1-amino-10-ethyl-6-fluoro-10-hydroxy-5-methyl-1,2,3,10,13,16-hexahydro-11*H*,14*H*-oxepino[4,3,2-*de*]pyrano[3',4':6,7]indolizino[1, 2-*b*]quinolin-11,14-one **035b**

The compound **035a** (50 mg, 0.07 mmol) was dissolved in *N*,*N*-dimethylformamide (5 mL), and diethylamine (0.5 mL) was added. The mixture was stirred for reaction at room temperature for 1 h. After the reaction was completed, the mixture was spin-dried under vacuum to remove the solvent, thus giving the title compound **035b** (34 mg), which was directly used in the next reaction step without purification.

MS m/z (ESI): 452.1 $(M+1)^+$.

Step 3

(*R*)-2-cyclopropyl-*N*-((1*S*,10*S*)-10-ethyl-6-fluoro-10-hydroxy-5-methyl-11,14-dioxo-2,3,10,11,14,16-hexahydro-1*H*,13*H*-oxepino[4,3,2-*de*]pyrano[3',4':6,7]indolizino[1,2-*b*]quinolin-1-yl)-2-hydroxyacetamide

(*S*)-2-cyclopropyl-*N*-((1*S*,10*S*)-10-ethyl-6-fluoro-10-hydroxy-5-methyl-11,14-dioxo-2,3,10,11,14,16-hexahydro-1*H*,13*H*-oxepino[4,3,2-*de*]pyrano[3',4':6,7]indolizino[1,2-*b*]quinolin-1-yl)-2-hydroxyacetamide

The compound **035b** (35 mg, 0.08 mmol) was dissolved in N,N-dimethylformamide (5 mL), and 2-cyclopropyl-2-hydroxyacetic acid (11 mg, 0.09 mmol), 2-(7-azabenzotriazole)-*N,N,N',N'*-tetramethyluronium hexafluorophosphate (46 mg, 0.12 mmol), and *N*,*N*-diisopropylethylamine (21 mg, 0.16 mmol) were added. The mixture was stirred for reaction at room temperature for 30 min. After the reaction was completed, the reaction mixture was purified by preparative high performance liquid chromatography (Waters MS-triggered Prep-LC with SQD2 detector, chromatographic column: Xbridge 5μm C18 150 x 19 mm; mobile phase 1: water (containing 0.1% FA); mobile phase 2: acetonitrile; 15-min gradient, gradient proportion: acetonitrile phase 40%-60%, flow rate: 20 mL/min) to give 2-cyclopropyl-*N*-((1*S*,10*S*)-10-ethyl-6-fluoro-10-hydroxy-5-methyl-11,14-dioxo-2,3,10,11,14,16-hexahydro-1*H*,13*H*-oxepino[4,3,2-*de*]pyrano[3',4':6,7]indolizino[1,2-*b*]quinolin-1-yl)-2-hydroxyacetamide (15 mg). The sample was subjected to chiral resolution (SFC 80 chromatographic column: Daicel CHIRALCEL OD, 250 mm x 30 mm I.D., 10μm; mobile phase: CO2/MeOH[0.2%NH3(7M Solution in MeOH)]= 70/30, flow rate: 70 g/min) to give the compounds **35-1** (5 mg, yield: 12%) and **35-2** (5 mg, yield: 12%).

**35-1 (Mono-configurational compound, compound with short retention time)**

MS m/z (ESI): 550.1 (M+1)$^+$.

$^1$H NMR (400 MHz, DMSO-$d_6$) δ 8.14 (d, 1H), 7.72 (d, 1H), 7.30 (s, 1H), 6.53 (s, 1H), 5.68-5.61 (m, 1H), 5.55-5.52 (m, 2H), 5.44 (s, 2H), 4.67 (dd, 1H), 4.23 (dd, 1H), 3.46 (t, 1H), 2.36 (s, 3H), 2.02-1.97 (m, 2H), 1.92-1.80 (m, 2H), 0.87 (t, 3H), 0.84 (d, 1H), 0.36-0.25 (m, 4H).

**35-2 (Mono-configurational compound, compound with long retention time)**

MS m/z (ESI): 550.1 (M+1)$^+$.

$^1$H NMR (400 MHz, DMSO-$d_6$) δ 8.17 (d, 1H), 7.71 (d, 1H), 7.30 (s, 1H), 6.53 (s, 1H), 5.62 (d, 1H), 5.49 (t, 2H), 5.44 (s, 2H), 4.62-4.53 (m, 1H), 4.29 (dd, 1H), 3.61 (t, 1H), 2.36 (d, 3H), 2.06-1.94 (m, 2H), 1.85 (dd, 2H), 0.87 (t, 3H), 0.85-0.83 (m, 1H), 0.25- 0.19 (m, 2H), 0.08 - 0.02 (m, 2H).

**Example 36**

(*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxypentanamide

(*S*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxypentanamide

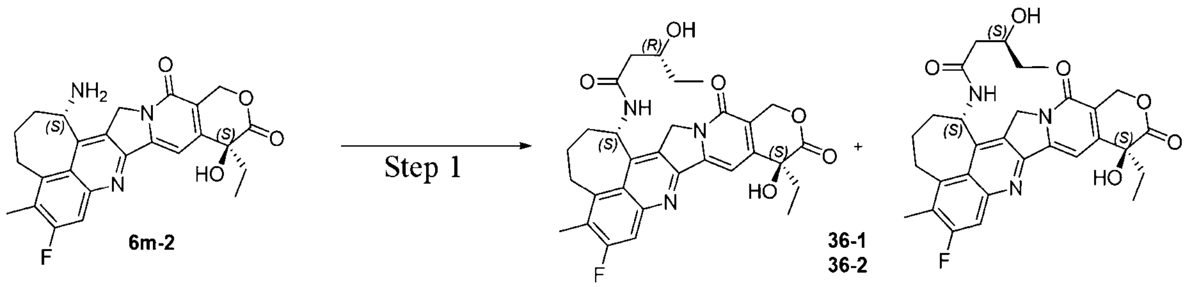


**6m-2** **36-1** **36-2**

Step 1

(*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxypentanamide

(*S*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxypentanamide

The compound **6m-2** (30 mg, 0.07 mmol) was dissolved in *N,N*-dimethylformamide (3 mL), and then 3-hydroxypentanoic acid (9 mg, 0.07 mmol), *N,N*-diisopropylethylamine (17 mg, 0.13 mmol), and 2-(7-azabenzotriazole)-*N,N,N',N'*-tetramethyluronium hexafluorophosphate (38 mg, 0.10 mmol) were added. The reaction system was kept for reaction at normal temperature for 1 h. After the reaction was completed, the system was purified by preparative high performance liquid chromatography (GILSON Prep LC with UV detector, chromatographic column: Xbridge 10 μm C18 250x30 mm, 10 μm; mobile phase 1: water (containing 10 mmol/L FA); mobile phase 2: acetonitrile; 15-min gradient, gradient proportion: acetonitrile phase 5%-57%, flow rate: 50 mL/min) to give the compounds **36-1**(6.98 mg, yield: 18%) and **36-2** (6.70 mg, yield: 18%).

**36-1 (Mono-configurational compound, compound with short retention time)**

MS m/z (ESI): 550.2 (M+1)$^+$.

$^1$H NMR (400 MHz, DMSO-$d_6$) δ 8.61 (d, 1H), 7.74 (d, 1H), 7.29 (s, 1H), 6.52 (s, 1H), 5.53 (s, 1H), 5.43 (s, 2H), 5.39-5.09 (m, 2H), 4.63 (s,1H), 2.43 (s, 3H), 2.32 (d, 3H), 2.10-1.93 (m, 3H), 1.92-1.81 (m, 2H), 1.75 (s, 1H), 1.40-1.32 (m, 2H), 1.24 (s, 2H), 0.91-0.76 (m, 6H).

**36-2 (Mono-configurational compound, compound with long retention time)**

MS m/z (ESI): 550.2 (M+1)+.

$^1$H NMR (400 MHz, DMSO-$d_6$) δ 8.69 (d, 1H), 7.73 (d, 1H), 7.28 (s, 1H), 6.51 (s, 1H), 5.51 (d, 2H), 5.43 (s, 2H), 5.33 (d, 1H),4.64 (s, 1H), 2.42 (s, 4H), 2.34-2.22 (m, 4H), 2.08 (d, 1H), 2.01 (d, 1H), 1.92-1.78 (m, 2H), 1.65 (s, 1H), 1.44-1.31 (m, 2H), 1.24 (s, 1H), 0.95-0.66 (m, 6H).

**Example 37**

(*S*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxy-4-methylpentanamide

(*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-b]quinolin-1-yl)-3-hydroxy-4-methylpentanamide

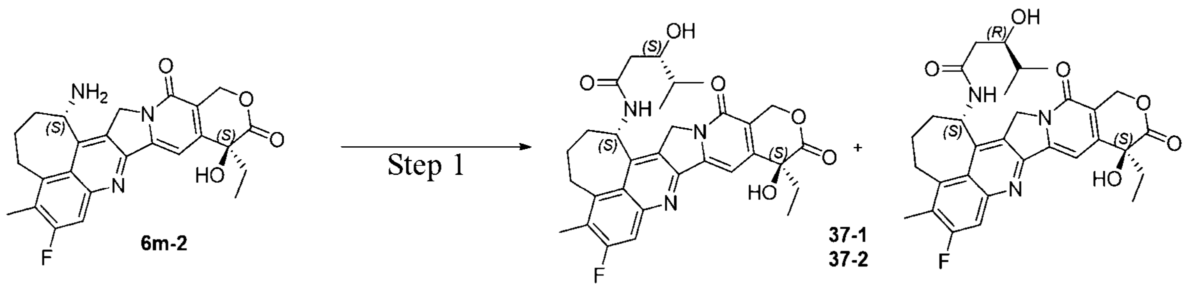


Step 3

(*S*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxy-4-methylpentanamide

(*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta [3',4':6,7]indolizino[1,2-*b*]quinolin-1-yl)-3-hydroxy-4-methylpentanamide

The compound **6m-2** (30 mg, 0.07 mmol) was dissolved in *N*,*N*-dimethylformamide (3 mL), and then 3-hydroxy-4-methylpentanoic acid (9 mg, 0.07 mmol), *N*,*N*-diisopropylethylamine (17 mg, 0.13 mmol), and 2-(7-azabenzotriazole)-*N,N,N',N'*-tetramethyluronium hexafluorophosphate (38 mg, 0.10 mmol) were added. The reaction system was kept for reaction at room temperature for 1 h. After the reaction was completed, the mixture was purified by preparative high performance liquid chromatography (Waters MS-triggered Prep-LC with SQD2 detector, chromatographic column: Xbridge 5 μm C18 150x19 mm; mobile phase 1: water (containing 0.1% FA); mobile phase 2: acetonitrile; 15-min gradient, gradient proportion: acetonitrile phase 42%-100%, flow rate: 20 mL/min) to give compound **37-1** (6.6 mg, yield: 18%) **and** 37-2 (6.9 mg, yield: 18%).

**37-1 (Mono-configurational compound, compound with short retention time)**

MS m/z (ESI): 564.2 (M+1)+.

$^1$H NMR (400 MHz, DMSO-$d_6$) δ 8.68 (d, 1H), 8.48 (br, 1H), 7.73 (d, 1H), 7.28 (s, 1H), 6.56 (s, 1H), 5.60-5.49 (m, 1H), 5.42 (s, 2H), 5.35 (d, 1H), 5.21 (d, 1H), 4.64 (s, 1H), 2.42 (s, 3H), 2.29 (d, 3H), 2.13-1.91 (m, 3H), 1.90-1.80 (m, 2H), 1.79-1.67 (m,1H),1.60-1.47 (m, 1H), 1.23 (s, 1H), 0.87 (d, 3H), 0.82 (d, 6H).

**37-2 (Mono-configurational compound, compound with long retention time)**

MS m/z (ESI): 564.2 (M+1)+.

$^1$H NMR (400 MHz, DMSO-$d_6$) δ 8.78 (s, 1H), 8.50 (s, 1H), 7.73 (s, 1H), 7.28 (s, 1H), 6.56 (s, 1H), 5.43 (s, 3H), 5.34 (d, 2H), 4.62 (s, 2H), 2.29 (s, 3H), 2.07 (s, 2H), 2.02-1.97 (m, 2H), 1.92-1.77 (m, 2H), 1.71-1.49 (m, 1H), 1.23 (s, 1H), 0.99-0.66 (m, 11H).

**Example 38**

(*R*)-3-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-b]quinolin-1-yl)-3-hydroxypropanamide

(*S*)-3-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-b]quinolin-1-yl)-3-hydroxypropanamide

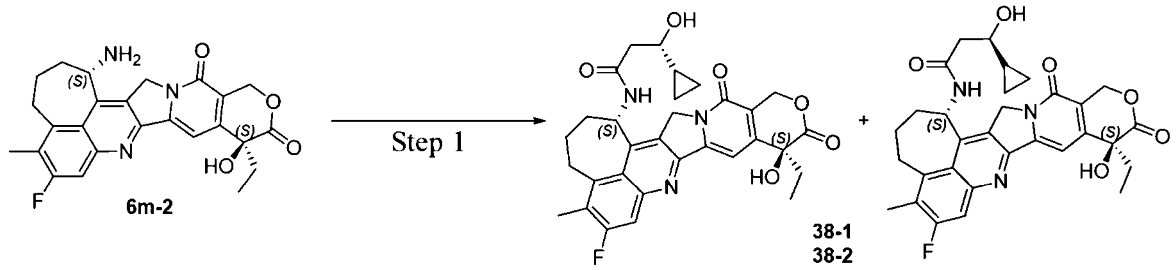


6m-2

38-1
38-2

(*R*)-3-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxypropanamide

(*S*)-3-cyclopropyl-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxypropanamide

The compound **6m-2** (30 mg, 0.07 mmol) was dissolved in *N,N*-dimethylformamide (3 mL), and then 3-cyclopropyl-3-hydroxypropionic acid (18 mg, 0.14 mmol), *N,N*-diisopropylethylamine (17 mg, 0.13 mmol), and 2-(7-azabenzotriazole)-*N,N,N',N'*-tetramethyluronium hexafluorophosphate (38 mg, 0.10 mmol) were added. The reaction system was kept for reaction at normal temperature for 1 h. After the reaction was completed, the system was purified and separated by preparative high performance liquid chromatography (Waters MS-triggered Prep-LC with QDA detector, chromatographic column: Xbridge 5 μm C18 150x19 mm; mobile phase 1: water (containing 0.1% FA); mobile phase 2: acetonitrile; 15-min gradient, gradient proportion: acetonitrile phase 45%-100%, flow rate: 25 mL/min) to give the title compound **38-1** (4 mg, yield: 11%) and the title compound **38-2** (4 mg, yield: 11%).

**38-1 (Mono-configurational compound, compound with short retention time)**

MS m/z (ESI):562.3 $(M+1)^+$.

$^1H$ NMR (400 MHz, DMSO-$d_6$) δ 8.63 (d, 1H), 7.74 (d, 1H), 7.28 (s, 1H), 6.52 (s, 1H), 5.53 (s, 1H), 5.42 (s, 4H), 4.69 (d, 1H), 3.22 (s, 2H), 2.61 (d, 1H), 2.42 (s, 3H), 2.31 (d, 1H), 2.08-1.69 (m, 6H), 0.87 (s, 3H), 0.81 (d, 1H), 0.37-0.20 (m, 3H), 0.16-0.08 (m, 1H).

**38-2 (Mono-configurational compound, compound with long retention time)**

MS m/z (ESI):562.3 $(M+1)^+$.

$^1H$ NMR (400 MHz, DMSO-$d_6$) δ 8.71 (d, 1H), 7.73 (d, 1H), 7.27 (s, 1H), 6.51 (s, 1H), 5.43 (s, 5H), 4.64 (d, 1H), 4.55-4.51(m, 1H), 3.22 (s, 2H), 2.65 (d, 1H), 2.41 (s, 3H), 2.31 (d, 1H), 2.13-1.57 (m, 6H), 0.88 (s, 3H), 0.82 (s, 1H), 0.45 (s, 1H), 0.32-0.13 (m, 3H).

**Example 39**

(2*S*,3*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxy-2-methylbutanamide **39**

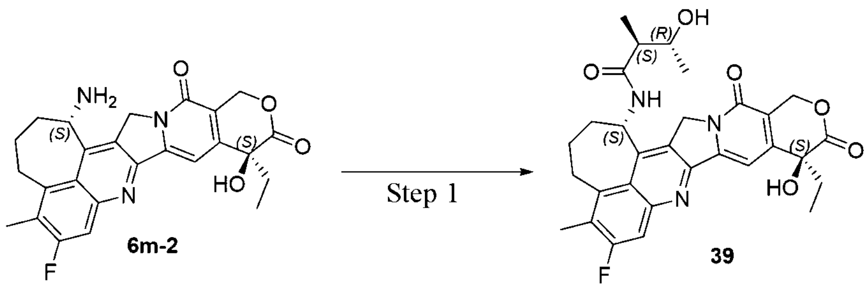


6m-2 39

Step 1

(2*S*,3*R*)-*N*-((1*S*,10*S*)-10-ethyl-6-fluoro-10-hydroxy-5-methyl-11,14-dioxo-1,2,3,4,10,11,14,16-octahydro-13*H*-cyclohepta[3',4':6,7]indolizino[1,2-*b*]quinolin-1-yl)-3-hydroxy-2-methylbutanamide **39**

The compound **6m-2** (30 mg, 0.07 mmol) was dissolved in *N*,*N*-dimethylformamide (2 mL), and (2S,3R)-3-hydroxy-2-methylbutyric acid (12 mg, 0.10 mmol) and 2-(7-azabenzotriazole)-*N*,*N*,*N*',*N*'-tetramethyluronium hexafluorophosphate (38 mg, 0.10 mmol) were added. The mixture was stirred for reaction at room temperature for 15 min. After the reaction was completed, the system was purified by preparative high performance liquid chromatography (Waters MS-triggered Prep-LC with QDA detector, chromatographic column: Xbridge 5 μm C18 150x19 mm, 5 μm; mobile phase 1: water (containing 0.1% FA); mobile phase 2: acetonitrile; 10-min gradient, gradient proportion: acetonitrile phase 42%-52%, flow rate: 25 mL/min) to give the compound **39** (2 mg, yield: 5%).

MS m/z (ESI): 550.0 $(M+1)^+$.

$^1$H NMR (400 MHz, $CDCl_3$) δ 7.73 (s, 1H), 7.42-7.28 (m, 2H), 5.51 (s, 1H), 5.41 (d, 1H), 5.35 (t, 1H), 5.26-5.16 (m, 2H), 5.04 (d, 1H), 4.18-4.00 (m, 2H), 3.33 (s, 2H), 2.65-2.50 (m, 3H), 2.48 (s, 3H), 2.27-2.18 (m, 1H), 2.11-2.04 (m, 2H), 2.03-1.97 (m, 1H), 1.33-1.28 (m, 6H), 0.95 (t, 3H).

**Example 40**

*N*-((2*R*,10*S*)-10-benzyl-2-cyclopropyl-1-((1*S*,10*S*)-10-ethyl-6-fluoro-10-hydroxy-5-methyl-11,14-dioxo-1,2,3,4,10,14,16-octahydro-13*H*-cyclohepta[yl]pyrano[3',4':6,7]indolizino[1,2-*b*]quinolin-1-yl)amino)-1,6,9,12,15-pentaoxo-3-oxo-5,8,11,14-tetraazahexadecan-16-yl)-6-(4-methoxy-2-(methylsulfonyl)pyrimidin-5-yl)hexan-5-amide

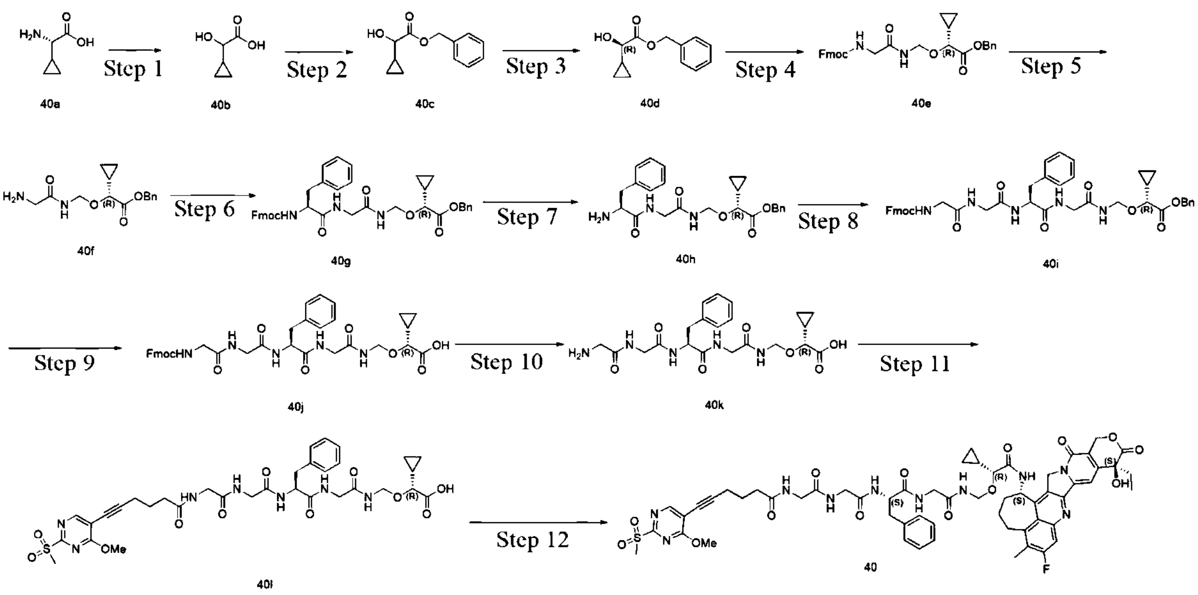


40a 40b 40c 40d 40e 40f 40g 40h 40i 40j 40k 40l 40

Step 1 Preparation of 2-cyclopropyl-2-hydroxyacetic acid **(40b)**

(*S*)-2-amino-2-cyclopropylacetic acid (23 g, 200 mmol) **40a** was dissolved in 2 M sulfuric acid (100 mL). A 4 M aqueous sodium nitrite solution (450 ml) was added in ice-water bath. The reaction mixture was stirred at room temperature for 16h. After the reaction was completed, the reaction mixture was diluted with water, and washed with ethyl acetate. The resulting filtrate was directly concentrated to give the crude compound **40b** (11.5 g).
MS m/z (ESI): 117.1 $(M+H)^+$.

Step 2 Preparation of benzyl 2-cyclopropyl-2-hydroxyacetate **(40c)**

The compound **40b** (11.5 g, 0.099 mol) was dissolved in acetonitrile (50 mL), and potassium carbonate (54.65 g, 0.396 mol), benzyl bromide (17.03 g, 0.099 mol) and tetrabutylammonium iodide (3.66 g, 0.0099 mol) were added in sequence. The reaction mixture was stirred at room temperature for 48h. The reaction mixture was filtered through celite, the filter cake was rinsed with ethyl acetate, and the filtrate was concentrated and purified by silica gel column system B to give the title compound **40c** (5.8 g, yield: 28%).
MS m/z (ESI): 207.1 $(M+H)^+$.

Step 3 Preparation of benzyl (R)-2-cyclopropyl-2-hydroxyacetate **(40d)**

The compound **40c** (12.1 g, 58.4 mmol) was purified using a SFC 150 preparative instrument and a chiral column (chromatographic column: Daicel CHIRALCEL AD, 250 mm x 30 mm I.D. , 10 μm; mobile phase CO2/MeOH [0.2% NH3 (7M Solution in MeOH)] = 90/10, flow rate: 120 g/min) to separate chiral isomers, thereby giving the compound **40d** (5.8 g, yield: 48%).
MS m/z (ESI): 207.1 $(M+H)^+$.

Step 4 Preparation of benzyl (*R*)-10-cyclopropyl-1-(9*H*-fluoren-9-yl)-3,6-dioxo-2,9-dioxo-4,7-diazaundecan-11-oate **(40e)**

The compound **40d** (5.8 g, 28 mmol) was dissolved in dichloromethane (20 mL), methyl (2-((((9*H*-fluoren-9-yl) methoxy)carbonyl)amino)acetamido)acetate (5 g, 14 mmol) and pyridinium 4-methylbenzenesulfonate (1.4 g, 5.6 mmol) were added. The reaction mixture was left to react at 40°C for 12 h. After the reaction was completed, the reaction mixture was concentrated under reduced pressure, and the resulting concentrate was purified by silica gel column chromatography with system B to give the title compound **40e** (1.2 g, yield: 30%).
MS m/z (ESI): 537.2 $(M+Na)^+$.

Step 5 Preparation of benzyl (*R*)-2-((2-aminoacetylamino)methoxy)-2-cyclopropylacetate **(40f)**

The compound **40e** (1.2 g, 2.33 mmol) was dissolved in *N*,*N*-dimethylformamide (10 mL), and diethylamine (0.34 g, 4.66 mmol) was added. The reaction mixture was stirred at room temperature for 1 h. After the reaction was completed, the product was directly used in the next reaction step without purification.
MS m/z (ESI): 293.1 $(M+H)^+$.

Step 6 Preparation of benzyl (5*S*,13*R*)-5-benzyl-13-cyclopropyl-1-(9*H*-fluoren-9-yl)-3,6,9-trioxo-2,12-dioxo-4,7,10-triazatetradecan-14-oate **(40g)**

The compound **40f** (0.6 g, 2.05 mmol) was dissolved in *N*,*N*-dimethylformamide (5 mL), and (((9H-fluoren-9-yl) methoxy)carbonyl)-*L*-phenylalanylglycine (1.09 g, 2.46 mmol) and *N,N*-diisopropylethylamine (0.53 g, 4.1 mmol) were added. The reaction mixture was stirred at room temperature for 2 h. After the reaction was completed, the product was directly used in the next reaction step without purification.
MS m/z (ESI): 684.3 $(M+Na)^+$.

Step 7 Preparation of benzyl (*R*)-2-((2-((*S*)-2-amino-3-phenylpropionamido)acetamido)methoxy)-2-cyclopropylacetate **(40h)**

The compound **40g** (1.2 g, 1.8 mmol) was dissolved in *N*,*N*-dimethylformamide (10 mL), and diethylamine (0.27 g, 3.6 mmol) was added. The reaction mixture was stirred at room temperature for 1 h. After the reaction was completed, water (20 mL) was added into the reaction mixture, and the mixture was extracted with ethyl acetate (10 mL x 3) . The organic phases were combined, washed with saturated brine, dried, and concentrated. The resulting concentrate was

purified by silica gel column chromatography with system B to give the title product **40h** (0.6 g , yield: 70%).
MS m/z (ESI): 440.2 $(M+H)^+$.

Step 8 Preparation of benzyl (11S, 19*R*)-11-benzyl-19-cyclopropyl-1-(9*H*-fluoro-9-yl)-3,6,9,12,15-pentaoxo-2,18-dioxo-4,7,10,13,16-pentaazaeicosanoate **(40i)**

The compound **40h** (0.6 g, 1.36 mmol) was dissolved in *N,N*-dimethylformamide (10 mL). (((9*H*-fluoren-9-yl) methoxy)carbonyl)glycylglycine (0.58 g, 1.63 mmol) , *N,N,N',N'*-tetramethyl-*O*-(7-azabenzotriazol-1-yl)uronium hexafluorophosphate (0.78 g, 2.04 mmol) and *N,N*-diisopropylethylamine (0.38 g, 2.72 mmol) were added. The reaction mixture was stirred at room temperature for 1 h. After the reaction was completed, water (10 mL) was added into the reaction mixture, and the mixture was extracted with ethyl acetate (10 mL x 3) . The organic phases were combined, washed with saturated brine, dried, and concentrated. The resulting concentrate was purified by silica gel column chromatography with system B to give the title product **40i** (0.8 g , yield: 76%).
MS m/z (ESI): 798.2 $(M+H)^+$.

Step 9 Preparation of benzyl (11S,19R)-11-benzyl-19-cyclopropyl-1-(9*H*-fluoren-9-yl)-3,6,9,12,15-pentaoxo-2,18-dioxo-4,7,10,13,16-pentaazaeicosanoate **(40j)**

The compound **40i** (0.8 g, 1.03 mmol) was dissolved in a mixed solvent of ethanol and ethyl acetate (10 mL, V/V = 1:1) and Pd/C (0.2 g, 1.03 mmol) was added. The reaction mixture was stirred at room temperature in a hydrogen atmosphere for 2 h. After the reaction was completed, the product was filtered through celite, the filter cake was washed with ethyl acetate, and the filtrate was combined and concentrated under reduced pressure to give the title compound **40j** (0.55 g, yield: 71%).
MS m/z (ESI): 708.1 $(M+Na)^+$.

Step 10 Preparation of (2*R*,10*S*)-16-amino-10-benzyl-2-cyclopropyl-6,9,12,15-tetraoxo-3-oxo-5,8,11,14-tetraazahexadecanoic acid **(40k)**

The compound **40j** (0.55 g, 0.8 mmol) was dissolved in *N*,*N*-dimethylformamide (10 mL), and diethylamine (117 mg, 1.6 mmol) was added. The reaction mixture was stirred at room temperature for 1 h. After the reaction was completed, tetrahydrofuran (20 mL) was added to the reaction mixture, the precipitated solid was collected by filtration, and the filter cake was collected. This gave the title compound **40k** (0.2 g, yield: 54%).
MS m/z (ESI): 464.2 $(M+H)^+$.

Step 11 Preparation of (2*R*,10*S*)-10-benzyl-2-cyclopropyl-23-(4-methoxy-2-(methylsulfonyl)pyrimidin-5-yl)-6,9,12,15,18-pentaoxo-3-oxo-5,8,11,14,17-pentaazatricarbon-22-ynoic acid **(40l)**

The compound **40k** (200 mg, 0.43 mmol) was dissolved in *N,N*-dimethylformamide (10 mL), and 2,5-dioxopyrrolidin-1-yl-6-(4-methoxy-2-(methylsulfonyl)pyrimidin-5-yl)hex-5-ynoate **28f** (152 mg, 0.39 mmol) and *N,N*-diisopropylethylamine (110 mg, 0.78 mmol) were added. The reaction mixture was stirred at room temperature for 1 h. After the reaction was completed, water (20 mL) was added to the reaction mixture, and the mixture was extracted with ethyl acetate (15 mLx3). The organic phases were combined, washed with saturated brine, dried, and concentrated to give a crude product. The crude product was purified by preparative high performance liquid chromatography (GILSON Prep LC with UV detector, chromatographic column: Xtimate 10u C18 250 x 30 mm; mobile phase 1: water (containing 0.1% FA); mobile phase 2: acetonitrile; 15-min gradient, gradient proportion: acetonitrile phase 25%-95%, flow rate: 50 mL/min) to give the title compound **40l** (100 mg, yield: 35%).
MS m/z (ESI): 766.2 $(M+Na)^+$.

Step 12 Preparation of *N*-((2*R*,10*S*)-10-benzyl-2-cyclopropyl-1-((1*S*,10*S*)-10-ethyl-6-fluoro-10-hydroxy-5-methyl-11,14-dioxo-1,2,3,4,10,14,16-octahydro-13*H*-cyclohepta[yl]pyrano[3',4':6,7]indolizino[1,2-*b*]quinolin-1-yl)amino)-1,6,9,12,15-pentaoxo-3-oxo-5,8,11,14-tetraazahexadecan-16-yl)-6-(4-methoxy-2-(methylsulfonyl)pyrimidin-5-yl) hexan-5-amide **(40)**

The compound **40l** (60 mg, 0.08 mmol) was dissolved in *N,N*-dimethylformamide (6 mL), and the compound **6m-2** (45 mg, 0.08 mmol), 4-(4,6-dimethoxytriazin-2-yl)-4-methylmorpholine hydrochloride (61 mg, 0.16 mmol) and triethylamine (26 mg, 0.16 mmol) were added. The reaction mixture was stirred at room temperature for 1 h. After the reaction was completed, water (20 mL) was added to the reaction mixture, and the mixture was extracted with ethyl acetate (20 mLx3). The organic phases were combined, washed with saturated brine, dried, and concentrated. The crude product

was purified by preparative high performance liquid chromatography (Waters MS-triggered Prep-LC with SQD2 detector, chromatographic column: Xbridge 5um C18 150 x 19 mm; mobile phase 1: water (containing 0.1% FA); mobile phase 2: acetonitrile; 15-min gradient, gradient proportion: acetonitrile phase 40%-100%, flow rate: 20 mL/min) to give the title compounds **40** (10 mg, yield: 6%).

MS m/z (ESI): 1175.3 $(M+H)^+$.

$^1$H NMR (400 MHz, DMSO-$d_6$) δ 8.79 (s, 1H), 8.65 (d, 2H), 8.44 (s, 1H), 8.31 (s, 1H), 8.17 (d, 2H), 8.06 (s, 1H), 7.72 (d, 1H), 7.30 (s, 1H), 7.18 (dt, 5H), 6.52 (s, 1H), 5.60 (s, 1H), 5.44 (t, 3H), 5.30 (d, 1H), 4.82 (dd, 1H), 4.59(dd, 1H), 4.48 (dd, 1H), 4.07 (s, 3H), 3.76 - 3.66 (m, 5H), 3.60 - 3.53 (m, 2H), 3.39 (s, 3H), 3.23 (s, 3H), 3.00 (dd, 1H), 2.78 (d, 1H), 2.40 (d, 3H), 2.34 - 2.22 (m, 3H), 1.86 (qdd, 9H), 1.12 - 1.03 (m, 1H), 0.86 (t, 3H), 0.45 (d, 4H).

## Example 41

N-((2*R*,10*S*)-10-benzyl-2-cyclopropyl-1-((1*S*,10*S*)-10-ethyl-6-fluoro-10-hydroxy-5-methyl-11,14-dioxo-2,3,10,11,14,16-hexahydro-1*H*,13*H*-oxepin[4,3,2-*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(4-methoxy-2-(methylsulfonyl)pyrimidin-5-yl)hexy-5-nylamide

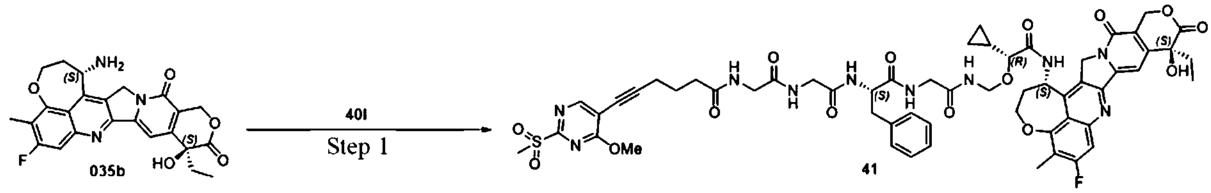


Step 1 *N*-((2*R*,10*S*)-10-benzyl-2-cyclopropyl-1-((1*S*,10*S*)-10-ethyl-6-fluoro-10-hydroxy-5-methyl-11,14-dioxo-2,3,10,11,14,16-hexahydro-1*H*,13*H*-oxepin[4,3,2-*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(4-methoxy-2-(methylsulfonyl)pyrimidin-5-yl) hexy-5-nylamide

The compound **035b** (20 mg, 0,04 mmol) was dissolved in *N*,*N*-dimethylformamide (3 mL), and the compound **40l** (33 mg, 0.04 mmol), *N,N,N',N'*-tetramethyl-*O*-(7-azabenzotriazol-1-yl)uronium hexafluorophosphate (30 mg, 0.08 mmol) and *N*,*N*-dimethylethylenediamine (14 mg, 0.11 mmol) were added. The mixture was stirred for reaction at room temperature for 30 min. After the reaction was completed, the reaction mixture was purified by preparative high performance liquid chromatography (Waters MS-triggered Prep-LC with QDA detector, chromatographic column: Gemini 5u C18 100 x 21.2 mm; mobile phase 1: water (containing 0.1% NH4); mobile phase 2: acetonitrile; 15-min gradient, gradient proportion: acetonitrile phase 45%-75%, flow rate: 30 mL/min) to give the title compound **41** (18 mg, yield: 38%).

MS m/z (ESI): 1177 $(M+1)^+$.

$^1$H NMR (400 MHz, $CD_3OD$) δ 8.61 (s, 1H), 7.68-7.52 (m, 2H), 7.31-7.08 (m, 5H), 5.83-5.69 (m, 2H), 5.55 (dd, 2H), 5.40 (d, 1H), 4.68 (d, 2H), 4.60 (s, 2H), 4.44 - 4.35 (m, 2H), 4.28 (d, 1H), 4.12 (s, 3H), 3.80 - 3.71 (m, 4H), 3.48 (d, 2H), 3.17 - 3.06 (m, 3H), 2.97 - 2.85 (m, 2H), 2.73 (s, 1H), 2.58 (dd, 4H), 2.46 - 2.41 (m, 4H), 2.01 - 1.86 (m, 4H), 1.03 (dd, 3H), 0.94 (d, 1H), 0.49 - 0.40 (m, 2H), 0.34 (s, 2H).

## Example 42

*N*-((2*R*,10*S*)-10-benzyl-2-cyclopropyl-1-((1*S*,10*S*)-5-cyclopropyl-10-ethyl-6-fluoro-10-hydroxy-11,14-dioxo-1,2,3,4,10,14,16-octahydro-13*H*-cyclohepta[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(5-cyano-6-(methylsulfonyl)pyridin-3-yl)hexy-5-nylamide

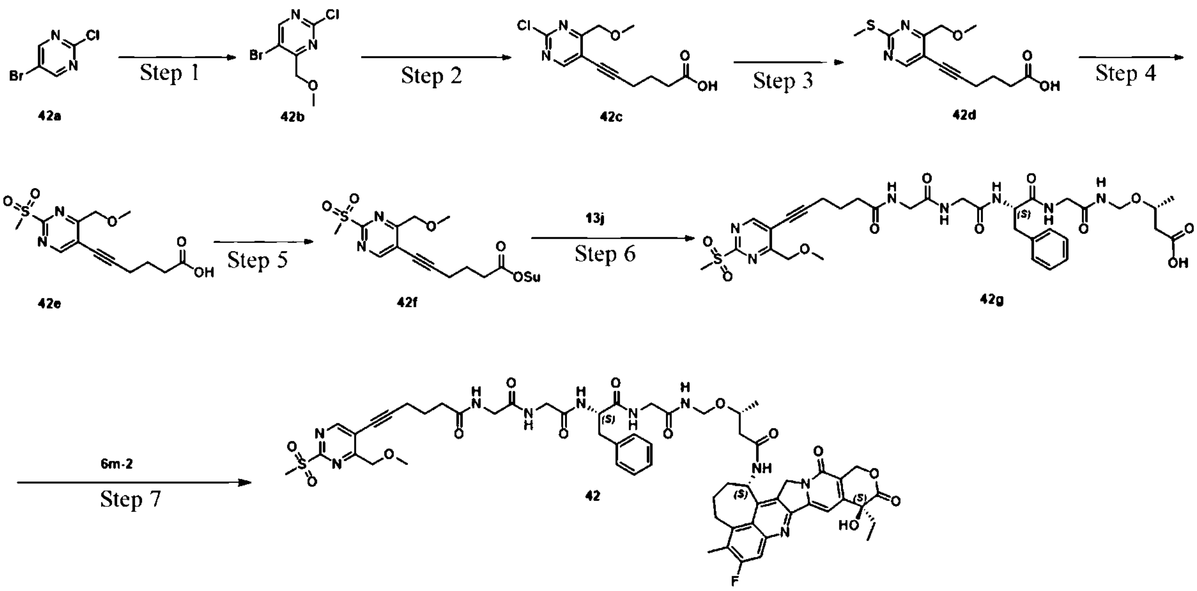


Step 1 Preparation of 5-bromo-2-chloro-4-(methoxymethyl)pyrimidine (**42b**)

5-Bromo-2-chloropyrimidine (10 g, 0.05 mol), silver nitrate (36 g,0.2 mol), ammonium persulfate (57 g, 0.25 mol) and 2-methoxyacetic acid (5.4 g, 0.06 mol) were dissolved in acetonitrile (300 mL) and water (300 mL) and stirred at 60°C for 2 h. The reaction mixture was poured into water and extracted with ethyl acetate three times, washed once with saturated brine, and concentrated under reduced pressure. The crude product obtained was purified by silica gel column chromatography with system B to give the title compound **42b** (1.2 g, yield: 13%).

MS m/z (ESI): 236.9 $(M+1)^+$.

Step 2 Preparation of 6-(2-chloro-4-(methoxymethyl)pyrimidin-5-yl)hexyl-5-ynoic acid **(42c)**

The compound **42b** (1.2 g, 4.5 mmol) was dissolved in tetrahydrofuran (10 mL), and hex-5-ynoic acid (0.76 g, 6.8 mmol), cuprous iodide (86 mg, 0.45 mmol), bistriphenylphosphine palladium dichloride (632 mg, 0.9 mmol) and triethylamine (1.4 g, 13.5 mmol) were added. The reaction mixture was stirred under nitrogen atmosphere at 60°C for 3 h. After the reaction was completed, the reaction mixture was filtered, and the filtrate was separated with ethyl acetate and water. The aqueous phase was extracted with ethyl acetate (20 mLx3). The organic phases were combined, dried and concentrated. The resulting crude product was purified by silica gel column chromatography with system A to give the title compound **42c** (600 mg, yield: 50%).

MS m/z (ESI): 269.1 $(M+1)^+$.

Step 3 6-(4-(methoxymethyl)-2-(methylthio)pyrimidin-5-yl)hexyl-5-ynoic acid **(42d)**

The compound **42c** (600 mg, 2.2 mmol) was dissolved in dimethyl sulfoxide (6 mL), sodium thiomethoxide (154 mg, 2.2 mmol) and anhydrous magnesium sulfate (528 mg, 4.4 mmol) were added, and the reaction mixture was stirred at 50°C for 1 h. After the reaction was completed, the reaction mixture was poured into water and extracted with ethyl acetate (50 mL x 3). The organic phases were combined, dried, and concentrated to give a crude product **42d** (600 mg), which was directly used for the next reaction step without purification.

MS m/z (ESI): 281.1 $(M+1)^+$.

Step 4 Preparation of 6-(4-(methoxymethyl)-2-(methylsulfonyl)pyrimidin-5-yl)hexyl-5-ynoic acid **(42e)**

The compound **42d** (600 mg) obtained in the previous step was dissolved in a mixed solvent of acetone and water (20 mL, V/V = 1:1), potassium peroxymonosulfonate (7.6 g, 22 mmol) was added, and the mixture was stirred at room temperature for 1h. After the reaction was completed, the reaction mixture was poured into water and extracted with ethyl acetate three times, washed once with saturated brine, and concentrated under reduced pressure. The crude product obtained was purified by silica gel column chromatography with system A to give the title compound **42e** (200 mg, yield: 30%).

MS m/z (ESI): 313.0 $(M+1)^+$.

Step 5 Preparation of 2,5-dioxopyrrolidin-1-yl-6-(4-(methoxymethyl)-2-(methylsulfonyl)pyrimidin-5-ylhex-5-ynoate (**42f**)

The compound **42e** (200 mg, 0.64 mmol) and *N*-hydroxysuccinimide (110 mg, 0.96 mmol) were dissolved in dichloromethane (10 mL), 1-(3-dimethylaminopropyl)-3-ethylcarbodiimide hydrochloride (246 mg, 1.28 mmol) was added, and the reaction was stirred at room temperature for 2h. After the reaction was completed, the reaction mixture was concentrated directly to give a crude product, and the crude product was purified by silica gel column chromatography with system B to give the title compound **42f (100** mg, yield: 38%).

MS m/z (ESI): 410.1 $(M+1)^+$.

Step 6 Preparation of (3*R*,11*S*)-11-benzyl-24-(4-(methoxymethyl)-2-(methylsulfonyl)pyrimidin-5-yl)-3-methyl-7,10,13,16,19-pentaoxo-4-oxa-6,9,12,15,18-pentaazatetracarbon-23-enoic acid **(42g)**

The compound **42f** (100 mg, 0.24 mmol) was dissolved in *N,N*-dimethylformamide (3 mL), *N, N*-diisopropylethylamine (62 mg, 0.48 mmol) and the compound **13j** (108 mg, 0.24 mmol) were added to the solution in sequence, and the reaction mixture was stirred at room temperature for 3 h. The reaction mixture was purified by preparative high performance liquid chromatography (Waters MS-triggered Prep-LC with SQD2 detector, chromatographic column: Xbridge 5u C18 150 x 19 mm; mobile phase 1: water (containing 0.1% FA); mobile phase 2: acetonitrile; 15-min gradient, gradient proportion: acetonitrile phase 30%-40%, flow rate: 20 mL/min) to give the title compound **42g** (30 mg, yield: 17%).

MS m/z (ESI): 768.2 $(M+Na)^+$.

Step 7 *N*-((2*R*,10*S*)-10-benzyl-2-cyclopropyl-1-((1*S*,10*S*)-5-cyclopropyl-10-ethyl-6-fluoro-10-hydroxy-11,14-dioxo-1,2,3,4,10,14,16-octahydro-13*H*-cyclohepta[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(5-cyano-6-(methylsulfonyl)pyridin-3-yl) hexy-5-nylamide **(42)**

The compound **42g** (30 mg, 0.04 mmol) was dissolved in *N,N*-dimethylformamide (2 mL), and the compound **6m-2** (18 mg, 0.04 mmol) was added, and 2-(7-azabenzotriazole)-*N,N,N',N'*-tetramethyluronium hexafluorophosphate (23 mg, 0.06 mmol) and *N*, *N*-diisopropylethylamine (10 mg, 0.08 mmol) were added in sequence. The mixture was stirred for reaction at room temperature for 30 min. After the reaction was completed, the reaction mixture was purified by preparative high performance liquid chromatography (Waters MS-triggered Prep-LC with QDA detector, chromatographic column: WELCH Xtimate C18 21.2*250mm 10um; mobile phase 1: water (containing 0.1% NH4HCO3); mobile phase 2: acetonitrile; 18-min gradient, gradient proportion: acetonitrile phase 38%-48%, flow rate: 30 mL/min) to give the title compound **42** (25 mg, yield: 53%).

MS m/z (ESI): 1177.3 $(M+1)^+$.

$^1H$ NMR (400 MHz, $CD_3OD$) $\delta$ 8.88 (s, 1H), 7.55 (s, 1H), 7.48 (d, 1H), 7.18 (dd, 5H), 5.65 (dd, 1H), 5.57 (d, 1H), 5.36 (s, 2H), 5.32 (s, 1H), 4.75 (s, 2H), 4.73 (d, 1H), 4.64 (d, 1H), 4.50 (dd, 1H), 3.81 (t, 4H), 3.71 - 3.66 (m, 1H), 3.58 (d, 1H), 3.50 (s, 3H), 3.39 (s, 3H), 3.29 (d, 2H), 3.10 (dd, 1H), 2.89 (dd, 1H), 2.60 (ddd, 4H), 2.45 (s, 9H), 2.00 - 1.87 (m, 6H), 1.27 (d, 3H), 1.00 (t, 3H).

**Example 43**

*N*-((2*R*,10*S*)-10-benzyl-2-cyclopropyl-1-((1*S*,10*S*)-5-cyclopropyl-10-ethyl-6-fluoro-10-hydroxy-11,14-dioxo-1,2,3,4,10,14,16-octahydro-13*H*-cyclohepta[yl]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(4-(methoxymethyl)-2-(methylsulfonyl)pyrimidin-5-yl)hexan-5-amide

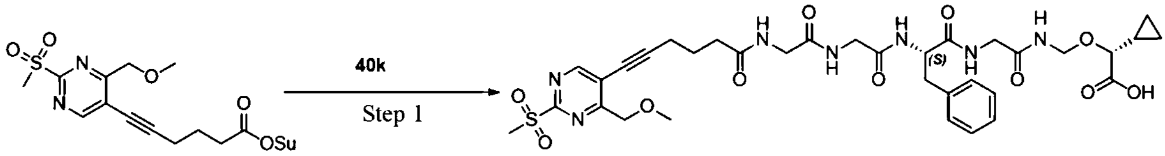


42f 43a

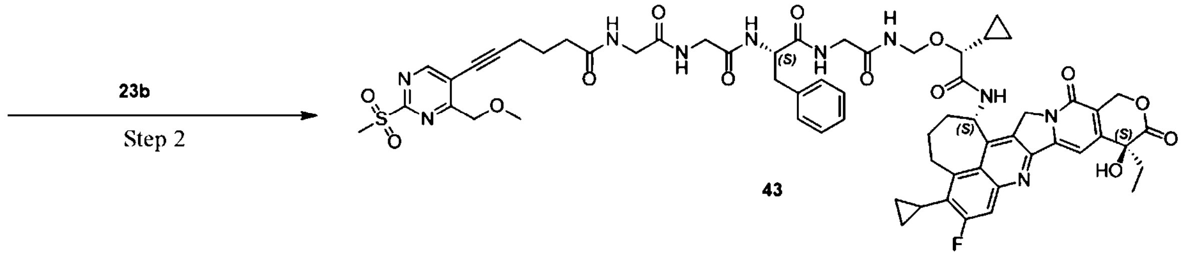


43

Step 1 Preparation of (2*R*,10*S*-10-benzyl-2-cyclopropyl-23-(4-(methoxymethyl)-2-(methylsulfonyl)pyrimidin-5-yl)-6,9,12,15,18-pentaoxo-3-oxa-5,8,11,14,17-pentaazatricarbon-22-ynoic acid **(43a)**

The compound **42f** (25 mg, 0.06 mmol) was dissolved in N,N-dimethylformamide (3 mL), *N,* N-diisopropylethylamine (62 mg, 0.48 mmol) and the compound **40k** (28 mg, 0.06 mmol) were added to the solution in sequence, and the reaction mixture was stirred at room temperature for 3 h. The reaction mixture was purified by preparative high performance liquid chromatography (Waters MS-triggered Prep-LC with SQD2 detector, chromatographic column: Xbridge 5u C18 150 x 19 mm; mobile phase 1: water (containing 0.1% FA); mobile phase 2: acetonitrile; 20-min gradient, gradient proportion: acetonitrile phase 35%-40%, flow rate: 20 mL/min) to give the title compound **43a** (7 mg, , yield: 15%).
MS m/z (ESI): 780.2 $(M+Na)^+$.

Step 2 *N*-((2*R*,10*S*)-10-benzyl-2-cyclopropyl-1-((1*S*,10*S*)-5-cyclopropyl-10-ethyl-6-fluoro-10-hydroxy-11,14-dioxo-1,2,3,4,10,14,16-octahydro-13*H*-cyclohepta[yl]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(4-(methoxymethyl)-2-(methylsulfonyl)pyrimidin-5-yl)hexan-5-amide

The compound **43a** (7 mg, 0.01 mmol) was dissolved in *N,N*-dimethylformamide (2 mL), and the compound **23b** (5 mg, 0.01 mmol), 2-(7-azabenzotriazole)-*N,N,N',N'*-tetramethyluronium hexafluorophosphate (6 mg, 0.02 mmol) and *N, N*-diisopropylethylamine (4 mg, 0.03 mmol) were added. The mixture was stirred for reaction at room temperature for 30 min. After the reaction was completed, the reaction mixture was purified by preparative high performance liquid chromatography (Waters MS-triggered Prep-LC with QDA detector, chromatographic column: WELCH Xtimate C18 21.2*250mm 10um; mobile phase 1: water (containing 0.1% FA); mobile phase 2: acetonitrile; 18-min gradient, gradient proportion: acetonitrile phase 35%-65%, flow rate: 30 mL/min) to give the title compound **43** (5 mg, yield: 45%).
MS m/z (ESI): 1215.3 $(M+1)^+$.
$^1$H NMR (400 MHz, $CD_3OD$) δ 8.85 (s, 1H), 8.52 (s, 1H), 7.60 (s, 1H), 7.18 (dt, 4H), 5.67 (d, 2H), 5.55 (dd, 4H), 5.42 - 5.31 (m, 4H), 4.64 (d, 2H), 3.85 (d, 2H), 3.82 (s, 2H), 3.77 (d, 2H), 3.57 (s, 2H), 3.46 (s, 3H), 3.36 (s, 4H), 2.60 (t, 2H), 2.43 (d, 2H), 2.19 (d, 2H), 1.96 - 1.89 (m, 5H), 1.16 (d, 4H), 0.98 (t, 4H), 0.88 (d, 1H), 0.81 (d, 1H), 0.72 (s, 1H), 0.62 (s, 1H), 0.49 (d, 2H), 0.37 - 0.32 (m, 1H).

**Example 44**

*N*-((2*R*,10*S*)-10-benzyl-2-cyclopropyl-1-((1*S*,10*S*)-5-cyclopropyl-10-ethyl-6-fluoro-10-*hydroxy-11,14-dioxo-1,2,3,4,10,14,16-octahydro-13H*-cyclohepta[yl]pyrano[3',4':6,7]indolizino[1,2-*b*]quinolin-1-yl)amino)-1,6,9,12,15-pentaoxo-3 - oxa-5,8,11,14-tetraazahexadecan-16-yl)-6-(4-methoxy-2-(methylsulfonyl)pyrimidin-5-yl)hexy-5-nylamide

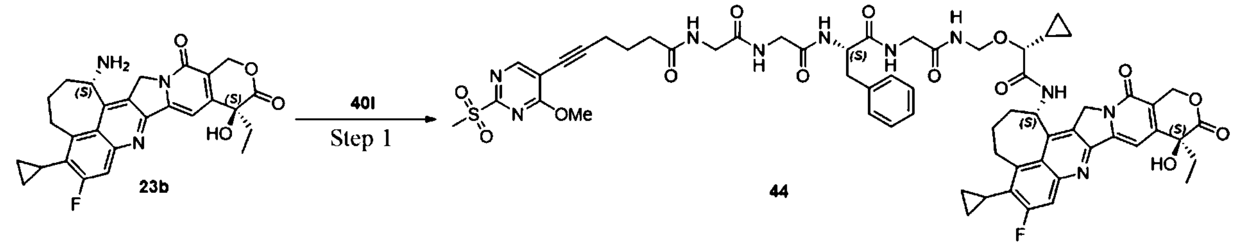


23b

44

Step 1 *N*-((2*R*,10*S*)-10-benzyl-2-cyclopropyl-1-((1*S*,10*S*)-5-cyclopropyl-10-ethyl-6-fluoro-10-hydroxy-11,14-dioxo-1,2,3,4,10,14,16-octahydro-13*H*-cyclohepta[yl]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(4-methoxy-2-(methylsulfonyl)pyrimidin-5-yl)hexyl-5-nylamide **(44)**

The compound **40l** (30 mg, 0.04 mmol) was dissolved in *N,N*-dimethylformamide (2 mL), and the compound **23b** (19.2 mg, 0.040 mmol), Carter condensation agent (21.4 mg, 0.048 mmol) and *N,N*-diisopropylethylamine (10.4 mg, 0.081 mmol) were added. The reaction mixture was stirred at room temperature for 3 h. After the reaction was completed, the reaction mixture was purified by preparative high performance liquid chromatography (Waters MS-triggered Prep-LC with QDA detector, chromatographic column: WELCH Xtimate C18 21.2*250mm 10um; mobile phase 1: water (containing 0.1% NH4HCO3); mobile phase 2: acetonitrile; 18-min gradient, gradient proportion: acetonitrile phase 46%-100%, flow rate: 30 mL/min) to give the title compound **44** (4.2 mg, yield: 9%).

MS m/z (ESI): 1201.4 $(M+1)^+$.

$^1H$ NMR (400 MHz, $CD_3OD$) δ 8.56 (s, 1H), 7.59 (s, 1H), 7.57 (d, 1H), 7.24 - 7.16 (m, 4H), 7.15 - 7.10 (m, 1H), 5.71 - 5.66 (m, 1H), 5.60-5.50 (m, 2H), 5.43-5.31 (m, 2H), 4.96 (d, 2H), 4.68-4.57 (m, 1H), 4.51 (dd, 1H), 4.09 (s, 3H), 3.93 - 3.71 (m, 6H), 3.58 (d, 3H), 3.15-3.10 (m, 1H), 2.97-2.92 (m, 1H), 2.62-2.40 (m, 5H), 2.26 - 2.14 (m, 2H), 2.02-1.74 (m, 6H), 1.37-1.28 (m, 2H), 1.21-1.09 (m, 3H), 0.98 (t, 3H), 0.86- 0.29 (m, 6H).

**Example 45**

*N*-((2*R*,10*S*)-10-benzyl-2-cyclopropyl-1-((1*S*,10*S*)10-ethyl-6-fluoro-10-hydroxy-5-methyl-11,14-dioxo-1,2,3,4,10,14,16-octahydro-13*H*-cyclohepta[yl]pyrano[3',4':6,7]indolizino[1,2-*b*]quinolin-1-yl)amino)-1,6,9,12,15-pentaoxo-3-oxo-5,8,11,14-tetraazahexadecan-16-yl)-6-(5-cyano-6-(methylsulfonyl)pyridin-3-yl)hexy-5-nylamide

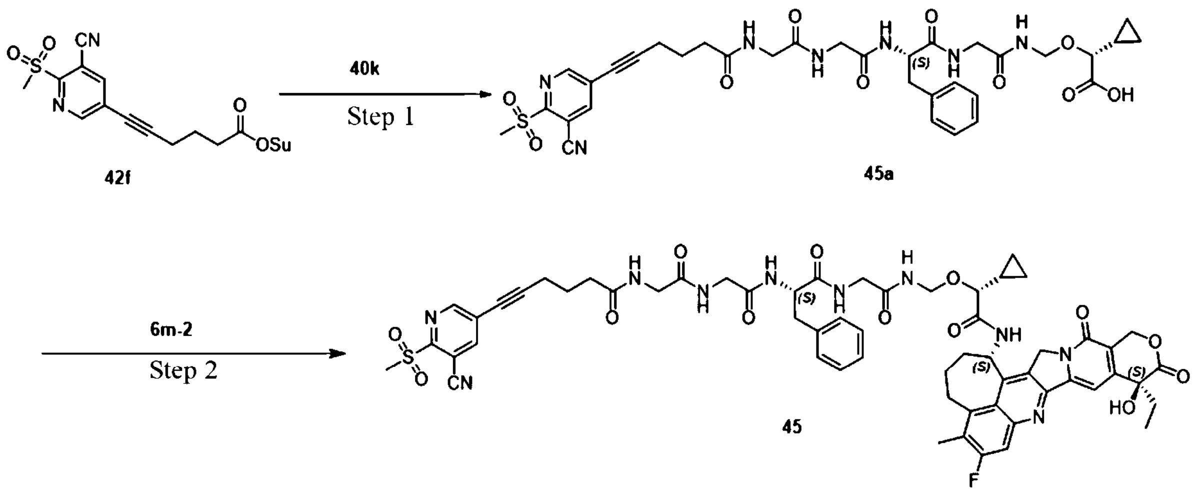


42f

45a

45

Step 1 Preparation of ((2*R*,10*S*)-10-benzyl-23-(5-cyano-6-(methylsulfonyl)pyridin-3-yl)-2-cyclopropyl-6,9,12,15,18-pentaoxo-3-oxa-5,8,11,14,17-pentaazatricarbon-22-ynoic acid **(45a)**

The compound **42f** (100 mg, 0.25 mmol) was dissolved in *N,N*-dimethylformamide (2 mL), and the compound **40k** (130 mg, 0.28 mmol) and *N,N*-diisopropylethylamine (66 mg, 0.51 mmol) were added. The mixture was stirred for reaction at room temperature for 30 min. After the reaction was completed, the reaction mixture was purified by preparative

high performance liquid chromatography (Waters MS-triggered Prep-LC with QDA detector, chromatographic column: Gemini 5u C18 100 x 21.2 mm 10um; mobile phase 1: water (containing 0.1% FA); mobile phase 2: acetonitrile; 15-min gradient, gradient proportion: acetonitrile phase 42%-100%, flow rate: 25 mL/min) to give the title compound **45a** (100 mg, yield: 53%).

MS m/z (ESI): 760.0 $(M+Na)^+$.

Step 2 Preparation of *N*-((2*R*,10*S*)-10-benzyl-2-cyclopropyl-1-((1*S*,10*S*)10-ethyl-6-fluoro-10-*hydroxy-5-methyl-11,14-dioxo-1,2,3,4,10,14,16-octahydro-13H*-cyclohepta[yl]pyrano[3',4':6,7]indolizino[1,2-*b*]quinolin-1-yl)amino)-1,6,9,12,15-pentaoxo-3-oxo-5,8,11,14-tetraazahexadecan-16-yl)-6-(5-cyano-6-(methylsulfonyl)pyridin-3-yl)hexy-5-nylamide **(45)**

The compound **45a** (115 mg, 0.16 mmol) was dissolved in *N,N*-dimethylformamide (2 mL), and the compound **6m-2**(70 mg, 0.16 mmol), 2-(7-azabenzotriazole)-*N,N,N',N'*-tetramethyluronium hexafluorophosphate (89 mg, 0.23 mmol) and *N,N*-dimethylformamide (40 mg, 0.31 mmol) were added. The mixture was stirred for reaction at room temperature for 30 min. After the reaction was completed, the reaction mixture was purified by preparative high performance liquid chromatography (Waters MS-triggered Prep-LC with SQD2 detector, chromatographic column: Xbridge 5 um C18 150 x 19 mm; mobile phase 1: water (containing 0.1% FA); mobile phase 2: acetonitrile; 15-min gradient, gradient proportion: acetonitrile phase 50%-65%, flow rate: 20 mL/min) to give the title compound **45** (60 mg, yield: 33%).

MS m/z (ESI): 1169.2 $(M+1)^+$.

$^1$H NMR (400 MHz, DMSO-$d_6$) δ 8.96 (s, 1H), 8.70 (s, 1H), 8.67 (d, 1H), 8.57 (d, 1H), 8.34-8.22 (m, 1H), 8.21-8.14 (m, 1H), 8.11 (d, 1H), 8.07-8.00 (m, 1H), 7.72 (d, 1H), 7.30 (s, 1H), 7.25-7.17 (m, 4H), 7.17-7.10 (m,1H), 6.52 (s, 1H), 5.60 (s, 1H), 5.44 (t, 2H), 5.30 (d, 1H), 4.86-4.78 (m, 1H), 4.63-4.55 (m, 1H), 4.53-4.45 (m, 1H), 3.71- 3.66 (m, 4H), 3.60 (d, 1H), 3.55 (d, 2H), 3.44 (s, 2H), 3.24-3.20 (m, 2H), 3.04 -2.97 (m, 2H), 2.67 (s, 1H), 2.40 (s, 3H), 2.11-2.04 (m, 2H), 2.03-1.92 (m, 3H), 1.88-1.76 (m, 4H), 1.75 -1.62 (m, 2H), 1.46 (s, 1H), 1.13 -1.02 (m, 1H), 0.86 (t, 4H), 0.50 - 0.39 (m, 4H).

## Example 46

*N*-((2*R*,10*S*)-10-benzyl-2-cyclopropyl-1-((1*S*,10*S*)-5-cyclopropyl-10-ethyl-6-fluoro-10-hydroxy-11,14-dioxo-1,2,3,4,10,14,16-octahydro-13H-cyclohepta[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(5-cyano-6-(methylsulfonyl)pyridin-3-yl)hexy-5-nylamide **(46)**

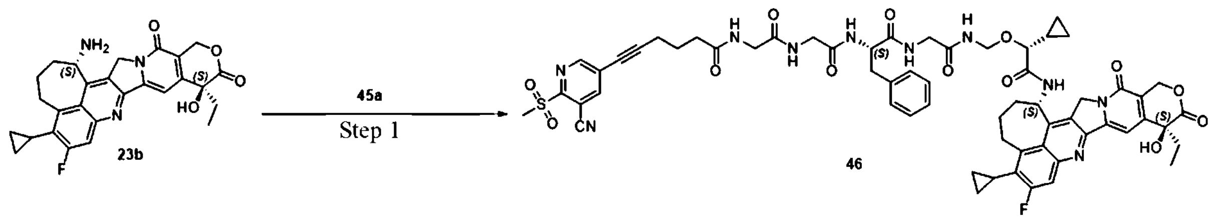


Step 1 N-((2*R*,10*S*)-10-benzyl-2-cyclopropyl-1-((1*S*,10*S*)-5-cyclopropyl-10-ethyl-6-fluoro-10-hydroxy-11,14-dioxo-1,2,3,4,10,14,16-octahydro-13*H*-cyclohepta[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(5-cyano-6-(methylsulfonyl)pyridin-3-yl)hexy-5-nylamide

The compound **45a** (16 mg, 0.02 mmol) was dissolved in *N*,*N*-dimethylformamide (2 mL), and the compound **23b** (10 mg, 0.02 mmol), 2-(7-azabenzotriazole)-*N,N,N',N'*-tetramethyluronium hexafluorophosphate (11 mg, 0.03 mmol) and N,N-diisopropylethylamine (5 mg, 0.04 mmol) were added. The mixture was stirred for reaction at room temperature for 30 min. The reaction mixture was purified by preparative high performance liquid chromatography (Waters MS-triggered Prep-LC with QDA detector, chromatographic column: WELCH Xtimate C18 21.2*250mm 10um; mobile phase 1: water (containing 0.1% $NH_4HCO_3$); mobile phase 2: acetonitrile; 15-min gradient, gradient proportion: acetonitrile phase 45%-55%, flow rate: 25 mL/min) to give the title compound (9 mg, yield: 36%).

MS m/z (ESI): 1195.3 $(M+1)^+$.

$^1$H NMR (400 MHz, $CD_3OD$) δ 8.76 (dd, 1H), 8.35 (dd, 1H), 7.62 - 7.56 (m, 1H), 7.51 (d, 1H), 7.19 (s, 5H), 5.68 (s, 2H), 5.40 (t, 2H), 5.16 - 5.07 (m, 2H), 4.94 (d, 2H), 4.60 (dd, 2H), 4.46 (d, 2H), 3.86 (d, 2H), 3.75 (d, 2H), 3.68 (d, 1H), 3.59 -3.56 (m, 2H), 3.36 (d, 3H), 3.35 (s, 1H), 2.94 (dd, 2H), 2.57 - 2.53 (m, 1H), 2.44 (d, 2H), 2.34 (d, 2H), 2.20 (s, 2H), 1.91 (s, 2H), 1.80 (s, 2H), 1.04 (d, 2H), 0.99 (t, 1H), 0.81 (s, 2H), 0.72 (s, 2H), 0.50 (s, 2H), 0.36 (s, 2H).

**Example 47**

*N*-((2*R*,10*S*)-10-benzyl-2-cyclopropyl-1-((1*S*,10*S*)-10-ethyl-6-fluoro-10-hydroxy-5-methyl-11,14-dioxo-2,3,10,11,14,16-hexahydro-1*H*,13*H*-oxepin[4,3,2-*de*]pyrano[3',4':6,7]indolizino[1,2-b]quinolin-1-yl)amino)-1,6,9,12,15-pentaoxo-3-oxa-5,8,11,14-tetraazahexadecan-16-yl)-6-(4-(methoxymethyl)-2-(methylsulfonyl)pyrimidin-5-yl)hexy-5-nylamide

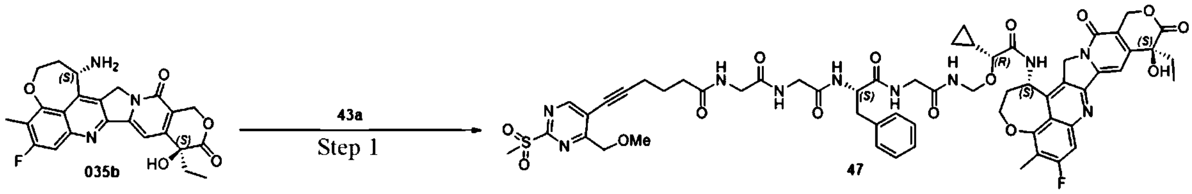


Step 1 *N*-((2*R*,10*S*)-10-benzyl-2-cyclopropyl-1-((1*S*,10*S*)-10-ethyl-6-fluoro-10-hydroxy-5-methyl-11,14-dioxo-2,3,10,11,14,16-hexahydro-1*H*,13*H*-oxepin[4,3,2-*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(4-(methoxymethyl)-2-(methylsulfonyl)pyrimidin-5-yl)hexy-5-nylamide

The compound **035b** (20 mg, 0,04 mmol) was dissolved in *N,N*-dimethylformamide (3 mL), and the compound **43a** (34 mg, 0.04 mmol), 2-(7-azabenzotriazole)-*N,N,N',N'*-tetramethyluronium hexafluorophosphate (30 mg, 0.08 mmol) and N,N-diisopropylethylamine (14 mg, 0.11 mmol) were added. The mixture was stirred for reaction at room temperature for 30 min. After the reaction was completed, the reaction mixture was purified by preparative high performance liquid chromatography (Waters MS-triggered Prep-LC with QDA detector, chromatographic column: Gemini 5u C18 100 x 21.2 mm; mobile phase 1: water (containing 0.1% NH4); mobile phase 2: acetonitrile; 15-min gradient, gradient proportion: acetonitrile phase 30%-60%, flow rate: 30 mL/min) to give the title compound (19 mg, yield: 40%).

MS m/z (ESI): 1191.2 $(M+1)^+$.

$^1H$ NMR (400 MHz, $CD_3OD$) $\delta$ 8.85 (d, 1H), 7.56 (dd, 2H), 7.27-7.10 (m, 5H), 5.78 -5.67 (m, 2H), 5.52 (dd, 2H), 5.37 (d, 1H), 4.74 - 4.56 (m, 7H), 4.42-4.23 (m, 3H), 3.75 (d, 2H), 3.72-3.67 (m, 2H), 3.46 (d, 3H), 3.37 (s, 3H), 3.07 (dd, 1H), 2.94-2.86 (m, 1H), 2.77-2.69 (m, 1H), 2.66-2.55 (m, 3H), 2.45-2.39 (m, 3H), 1.97-1.89 (m, 3H), 1.31 (d, 3H), 1.00 (dd, 3H), 0.94-0.88 (m, 1H), 0.43 (dd, 2H), 0.37-0.27 (m, 2H).

**Example 48**

(*R*)-3-(((*S*)-7-benzyl-17-(4-(2-(methylsulfonyl)-4-(trifluoromethyl)pyrimidin-5-yl)piperidin-1-y1)-3,6,9,12,15-pentaoxo-2,5,8,11,14-pentaazaheptadecyl)oxy)-*N*-((1*S*,10*S*)-10-ethyl-6-fluoro-10-hydroxy-5-methyl-11,14-dioxo-1,2,3,4,10,11,14,16-octahydro-13*H*-cycloheptan[3',4':6,7]indolizino[1,2-*b*]quinolin-1-yl)butanamide

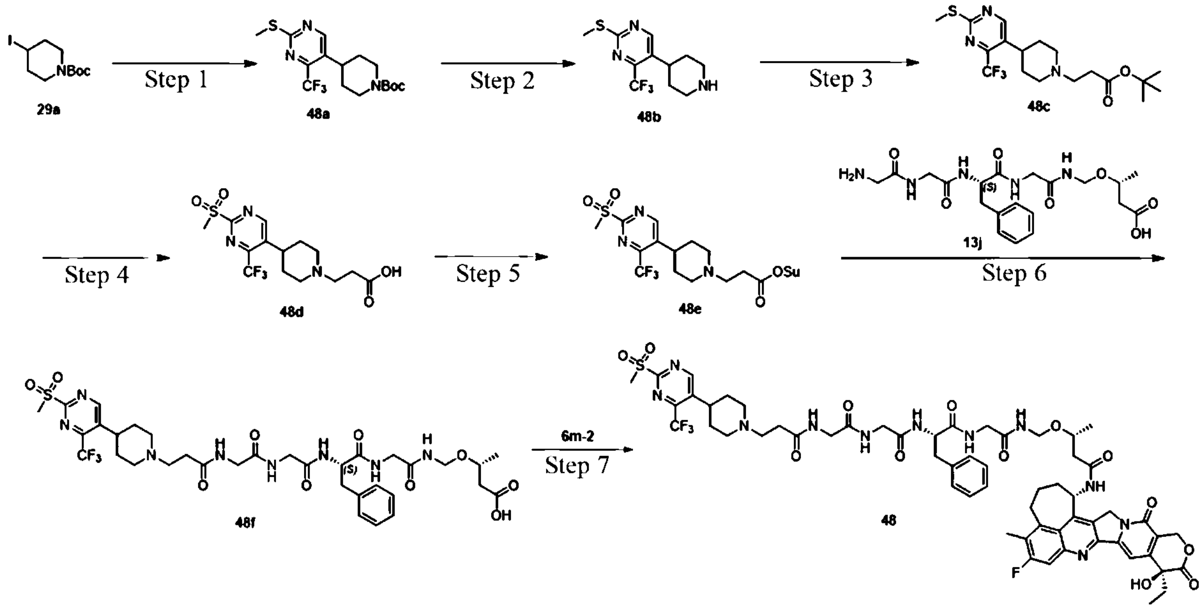


Step 1: Tert-butyl 4-(2-(methylthio)-4-(trifluoromethyl)pyrimidin-5-yl)piperidine-1-carboxylate **(48a)**

Nickel chloride dimethoxyethane (350 mg, 1.6 mmol) and 2-amidinopyridine hydrochloride (250 mg, 1.6 mmol) were added to a reaction flask, *N,N*-dimethylacetamide (10 ml) was added in nitrogen atmosphere, and the mixture was stirred at room temperature for half an hour. In another reaction flask, tert-butyl 4-iodopiperidine-1-carboxylate **29a** (2.0 g, 6.4 mmol), 5-bromo-2-(methylthio)-4-(trifluoromethyl)pyrimidine (1.75 g, 6.4 mmol), manganese (90 mg, 1.6 mmol), tetrabutylammonium iodide (2.36 g, 6.4 mmol) were added, then N,N-dimethylacetamide (10 ml) was added, and nitrogen replacement was carried out three times. After the replacement was completed, the nickel ligand compound was transferred to the system with a syringe, and the reaction was carried out at 60°C for 3 h. After the reaction was completed, ethyl acetate (200 mL) and water (400 mL) were added to the system for extraction. The organic phase was dried, concentrated, and purified by silica gel column chromatography with separation system B to give the title compound **48a** (1.3 g, yield: 48%).

MS m/z (ESI): 322.0 (M-55)$^+$.

Step 2 : 2-(methylthio)-5-(piperidin-4-yl)-4-(trifluoromethyl)pyrimidine **(48b)**

The compound **48a** (1.3 g, 3.4 mmol) was dissolved in a solution of 4N hydrochloric acid in 1,4-dioxane (10 ml). The mixture was stirred for reaction at room temperature for 1 h. After the reaction was completed, the system was directly spin-dried to remove the solvent, thus giving the crude product **48b** (800mg, yield: 85%).

MS m/z (ESI): 278.0 (M+1)$^+$.

Step 3: Tert-butyl 3-(4-(2-(methylthio)-4-(trifluoromethyl)pyrimidin-5-yl)piperidin-1-yl)propionate **(48c)**

The compound **48b** (800 mg, 2.9 mmol) was dissolved in acetonitrile (10 mL), and tert-butyl 3-bromopropionate (910 mg, 4.35 mmol) and potassium carbonate (1.2 g, 8.7 mmol) were added. The mixture was stirred for reaction at 60°C for 16 h. After the reaction was completed, the mixture was spin-dried to remove the solvent, thus giving a crude product, which was purified by silica gel column chromatography with separation system B to give the title compound **48c** (600 mg, yield: 51%).

MS m/z (ESI):406.1 (M+1)$^+$.

Step 4: 3-(4-(2-(methylsulfonyl)-4-(trifluoromethyl)pyrimidin-5-yl)piperidin-1-yl)propanoic acid **(48d)**

The compound **48c** (600 mg, 1.48 mmol) was dissolved in trifluoroacetic acid (10 mL) and m-chloroperbenzoic acid (2.55 g, 14.8 mmol) was added. The reaction was allowed to proceed at 40°C for 4 h. After the reaction was completed, the system was concentrated, and extracted with dichloromethane (80 ml) and water (80 ml). After the aqueous phase was freeze-dried, the reaction mixture was purified by preparative high performance liquid chromatography (Waters MS-triggered Prep-LC with SQD2 detector, chromatographic column: Xbridge 5u C18 150 x 19 mm; mobile phase 1: water

(containing 0.1% FA); mobile phase 2: acetonitrile; 15-min gradient, gradient proportion: acetonitrile phase 10%-20%, flow rate: 20 mL/min) to give the title compound **48d** (230mg, yield: 41%).

MS m/z (ESI): 382.0 $(M+1)^+$.

Step 5 2,5-dioxopyrrolidin-1-yl-3-(4-(2-(methylsulfonyl)-4-(trifluoromethyl)pyrimidin-5-yl)piperidin-1-yl)propanoate **(48e)**

The compound **48d** (50 mg, 0.13 mmol) was dissolved in dimethyl sulfoxide (1 mL), and N-hydroxysuccinimide (17 mg, 0.14 mmol) and EDCI (38 mg, 0.20 mmol) were added. The reaction was allowed to proceed at room temperature for 16 h. After the reaction was completed, the reaction mixture was directly used in the next reaction step.
MS m/z (ESI): 479.1 $(M+1)^+$,

Step 6 (3R,11S)-11-benzyl-3-methyl-21-(4-(2-(methylsulfonyl)-4-(trifluoromethyl)pyrimidin-5-yl)piperidin-1-yl)-7,10,13,16,19-pentaoxo-4-oxa-6,9,12,15,18-pentaazaeicosanoic acid **(48f)**

The compound **13j** (58 mg, 0.13 mmol) was added to the reaction mixture of the previous step, and N,N-diisopropylethylamine (17 mg, 0.26 mmol) was added. The mixture was stirred for reaction at room temperature for 30 min. After the reaction was completed, the reaction mixture was purified by preparative high performance liquid chromatography (Waters MS-triggered Prep-LC with SQD2 detector, chromatographic column: Xbridge 5u C18 150 x 19 mm 5um; mobile phase 1: water (containing 0.1% FA); mobile phase 2: acetonitrile; 9.5-min gradient, gradient proportion: acetonitrile phase 20%-30%, flow rate: 20 mL/min) to give the title compound **48f** (20 mg, yield: 19%).

MS m/z (ESI): 815.2 $(M+1)^+$.

Step 7 (*R*)-3-((((*S*)-7-benzyl-17-(4-(2-(methylsulfonyl)-4-(trifluoromethyl)pyrimidin-5-yl)piperidin-1-yl)-3,6,9,12,15-pentaoxo-2,5,8,11,14-pentaazaheptadecyl)oxy)-*N*-((1*S*,10*S*)-10-ethyl-6-fluoro-10-hydroxy-5-methyl-11,14-dioxo-1,2,3,4,10,11,14,16-octahydro-13*H*-cycloheptan[3',4':6,7]indolizino[1,2-*b*]quinolin-1-yl)butanamide

The compound **48f** (20 mg, 0.025 mmol) was dissolved in *N,N*-dimethylformamide (1 mL), and the compound **6m-2** (11 mg, 0.025 mmol), 2-(7-azabenzotriazole)-*N,N,N',N'*-tetramethyluronium hexafluorophosphate (14 mg, 0.037 mmol) and *N,N*-diisopropylethylamine (6 mg, 0.049 mmol) were added. The mixture was stirred for reaction at room temperature for 30 min. After the reaction was completed, the reaction mixture was purified by preparative high performance liquid chromatography (Waters MS-triggered Pre LC with QDA detector, chromatographic column: Gemini 5u C18 100 x 21.2 mm 5um; mobile phase 1: water (containing 0.1%$NH_4HCO_3$); mobile phase 2: acetonitrile; 9.5-min gradient, gradient proportion: acetonitrile phase 35%-65%, flow rate: 30 mL/min) to give the title compound **48** (3.9 mg, yield: 12%).

MS m/z (ESI): 1246.5 $(M+1)^+$.

$^1$H NMR (400 MHz, $CD_3OD$) δ 9.40 (s, 1H), 7.54 (s, 1H), 7.50 (d, 1H), 7.15-7.07 (m, 5H), 5.64 (dd, 1H), 5.55 (d, 1H), 5.35 (s, 2H), 5.33-5.27 (m, 1H), 4.70 (d, 1H), 4.59 (d, 1H), 4.52 (dd, 1H), 4.2-4.14 (m, 1H), 3.85 (s, 3H), 3.84-3.68 (m, 3H), 3.56 (d, 1H), 3.38 (s, 3H), 3.15-3.12 (m, 2H), 3.09 (d, 1H), 3.05 (d, 1H), 2.85 (dd, 1H), 2.74-2.68 (m, 2H), 2.55 (d, 1H), 2.43 (d, 4H), 2.21-2.10 (m, 4H), 2.06-1.98 (m, 3H), 1.91-1.86 (m, 2H), 1.84-1.78 (m, 2H), 1.60 (s, 1H), 1.34-1.32 (m, 2H), 1.24 (d, 3H), 0.96 (t, 3H), 0.89 (d, 1H).

**Preparation of antibody-drug conjugates**

**Example A-1 Preparation of ADC-1**

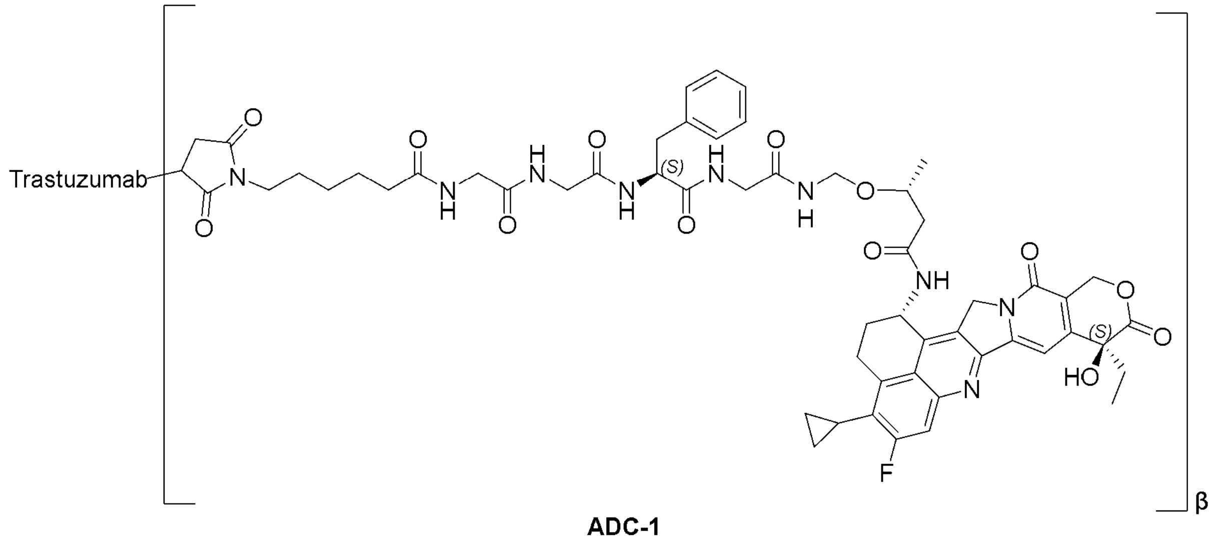

**ADC-1**

Trastuzumab was exchanged into 100 mM Pro-Ac, 20 mM Arg, pH 5.0 using Amicon. Trastuzumab was taken out, 1 M tromethamine, pH 7.5 was added, the pH was adjusted to 7.3. Trastuzumab (67 mg) was placed in a 50 mL centrifuge tube, and 2801 μL of 100 mM acetyl proline, 20 mM arginine, pH 7.3, 96 μL of 0.2 M EDTA, and 893 μL of 5 mM TCEP were added. The system was mixed to react at 37°C on a shaker for 1 h. After the reaction was completed, the reduced product of Trastuzumab was purified using a Zeba desalting column.

The reduced product of Trastuzumab (47.40 mg) was placed in a 50 mL centrifuge tube, and 1103.58 μL of 100 mM Pro-Ac, 20 mM Arg (pH 7.3), 94.80 μL of 0.2 M EDTA, 790 μL of DMA and 632 μL of 10 mM compound **21** were added in an ice bath, and the system was mixed to react on a shaker at 22°C for 3 h. The reaction mixture was purified using a Zeba desalting column, and the sample was concentrated using Amicon and exchanged into a buffer system of 100 mM proline, 20 mM arginine, pH 5.0 to give **ADC-1** (33 mg). The average DAR value of ADC calculated by LC-MS was β=8.14.

Referring to the method of Example A-1, the compound 21 was replaced with a compound represented by other formula V of the present application to prepare the following antibody-drug conjugates, whose structure and DAR value (β) are shown in the following table.

| ADC | Structure | ADC mass (mg) | DAR value (β) |
|---|---|---|---|
| ADC-2 | 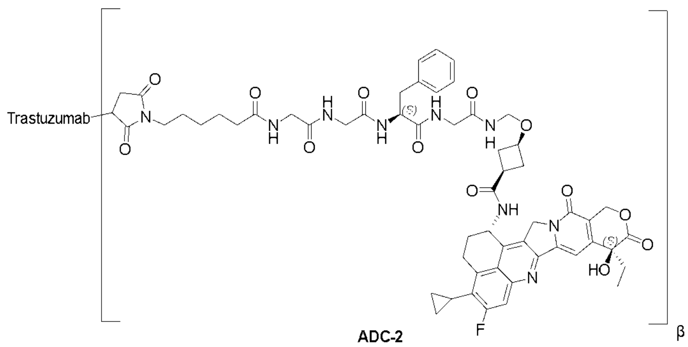 ADC-2 | 35 | 8.06 |
| ADC-3 | 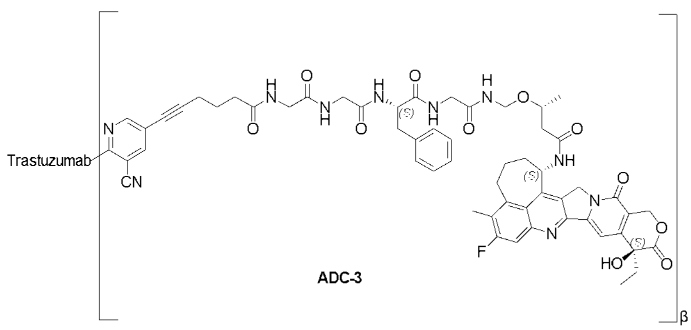 ADC-3 | 36 | 8.00 |

(continued)

| ADC | Structure | ADC mass (mg) | DAR value (β) |
|---|---|---|---|
| ADC-4 | 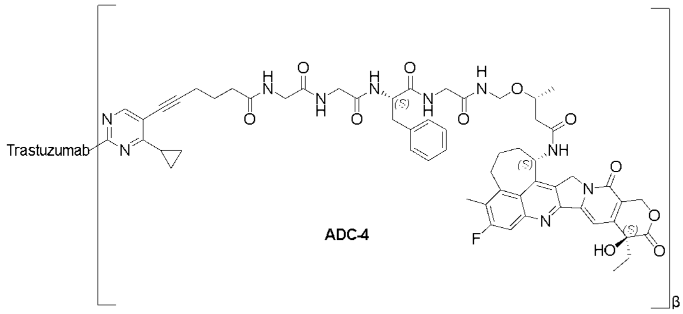 ADC-4 | 35 | 7.99 |
| ADC-5 | 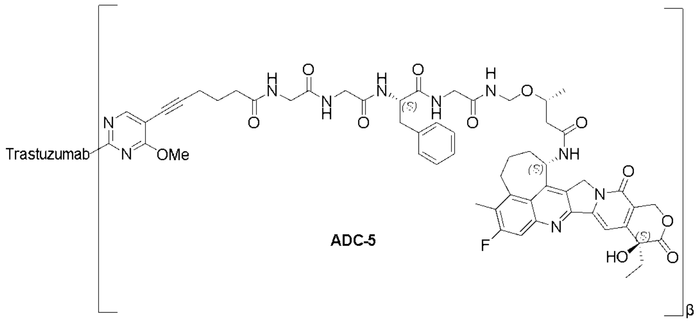 ADC-5 | 34 | 8.02 |
| ADC-6 | 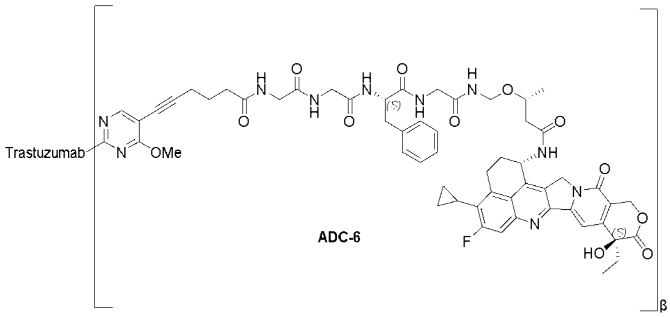 ADC-6 | 35 | 7.98 |
| ADC-7 | 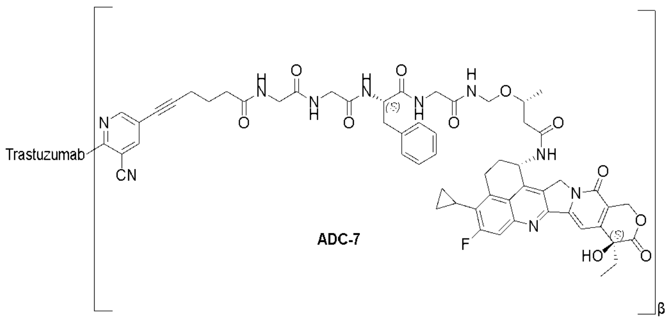 ADC-7 | 36.5 | 8.00 |
| ADC-8 | 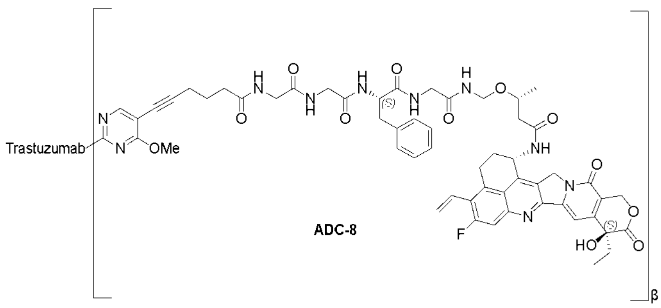 ADC-8 | 26.8 | 7.97 |

(continued)

| ADC | Structure | ADC mass (mg) | DAR value (β) |
|---|---|---|---|
| ADC-9 | 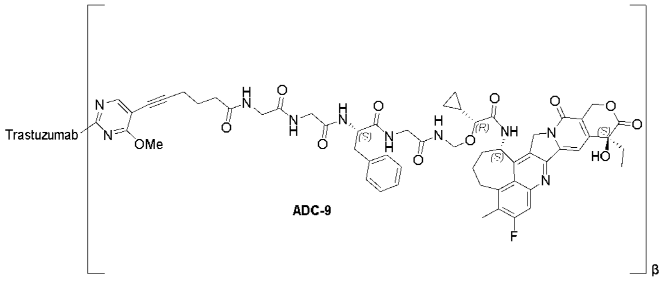 ADC-9 | 31 | 7.92 |
| ADC-10 | 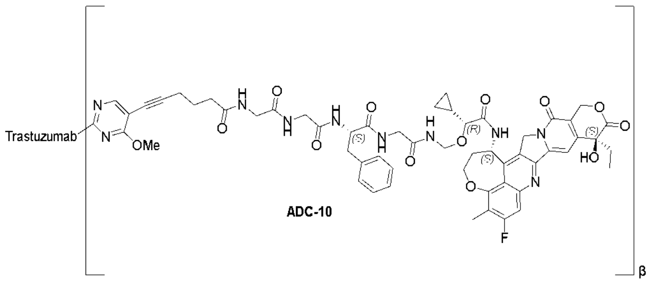 ADC-10 | 29.6 | 7.92 |
| ADC-11 | 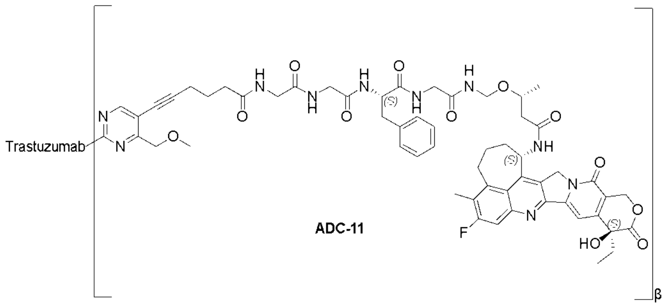 ADC-11 | 33 | 8.00 |
| ADC-12 | 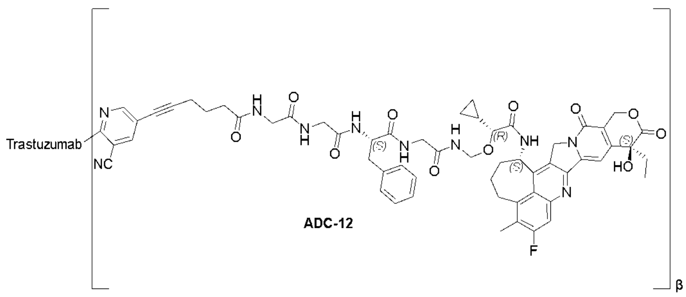 ADC-12 | 32 | 7.71 |
| ADC-13 | 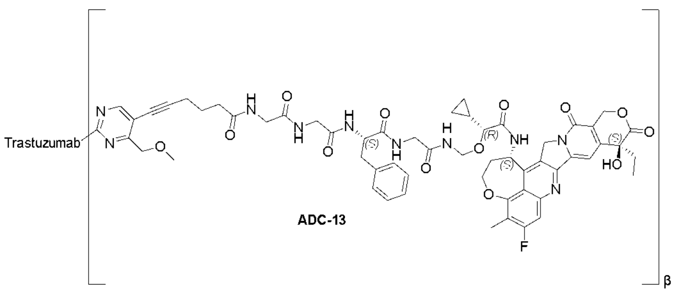 ADC-13 | 29.7 | 7.94 |

(continued)

| ADC | Structure | ADC mass (mg) | DAR value (β) |
|---|---|---|---|
| ADC-14 | 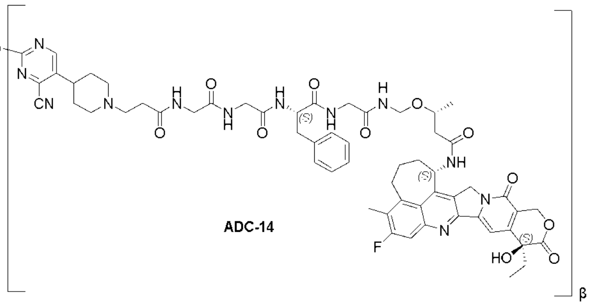 ADC-14 | 20 | 7.84 |
| ADC-15 | 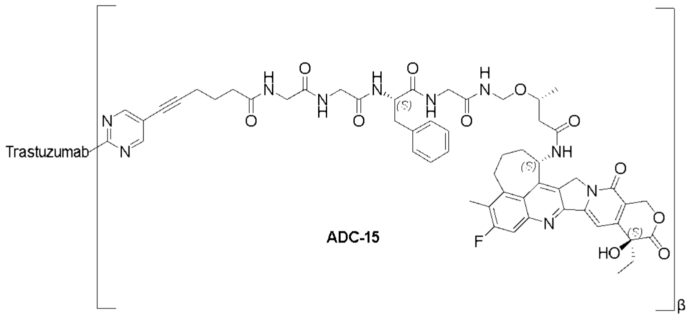 ADC-15 | 21 | 7.78 |

The cytotoxic drug-linker compound represented by formula V of the present invention can be smoothly conjugated with an antibody to give an antibody-drug conjugate.

## Biological Evaluation

The present invention is further described and interpreted below with reference to the test examples, but these test examples are not intended to limit the scope of the present invention.

**Test Example 1** Experiment of inhibition effect of compounds on proliferation of cells SK-BR-3

1: Experimental materials

| Materials and reagents | Supplier | Art. No. |
|---|---|---|
| SK-BR-3 | ATCC | HTB-30 |
| 5A Medium | Gibco | 16600-082 |
| Penicillin-streptomycin | Gibco | 15140-122 |
| Fetal bovine serum | Gibco | 10099-141C |
| Phosphate buffer saline | Gibco | 10010-031 |
| TrypLE | Gibco | 12604-021 |
| DMSO | Sigma | D8418-1L |
| Staurosporine | MCE | HY-15141 |
| CelltiterGlo assay kit (CTG) | Promega | G7573 |

2: Experimental instruments

| Consumables and instruments | Supplier | Art. No. |
|---|---|---|
| White 384-well culture plate | PerkinElmer | 6007680 |
| Plate shaker | QILINBEIER | QB-9002 |
| Centrifuge | Eppendorf | 5810R |
| Carbon dioxide incubator | Thermo Scientific | 371 |
| Microscope | OLYMPUS | CKX41 |
| Echo Qualified 384-Well Polypropylene | LABCYTE | PP-0200 |
| Echo | LABCYTE | 655 |
| CountessII | Gibco | AMQAX1000 |
| Envision | PerkinElmer | 2105 |

3: Test method

(1) Cell plating: first, tumor cells SK-BR-3 were cultured using a corresponding culture medium, trypsinized, centrifuged, resuspended for counting, adjusted to an appropriate concentration, and then plated on a 384-well plate.

(2) Co-incubation of the compounds and tumor cells: after the cells adhered to the wall, 100 nL of a diluted bioactive substance (test compound) was added to the cell culture plate using ECHO, the final concentration of DMSO in wells of the cell plate was 0.33%, and the incubation was performed in an incubator at 37°C with 5% $CO_2$ for 72 h.

(3) After the incubation was complete, 30 μL of a CTG reagent (CelltiterGlo kit) was added to each well. The culture was oscillated on a fast oscillator for 2 min, centrifuged at 1,000 rpm for 1 min, and kept away from light at room temperature for 30 min. The chemiluminescence signal value was read using an Envision instrument.

(4) Cell viability detection: $IC_{50}$ was calculated using GraphPad Prism 8 software, and the $IC_{50}$ of the compounds was obtained using the following nonlinear fitting formula (see Table 1) :

1. Y=Bottom+(Top-Bottom)/(1+10^((Log$IC_{50}$-X)*HillSlope))
2. Y: inhibition rate; X: log value of compound concentration;
3. inhibition rate (%)=100-(compound well reading - low-reading control well reading)/(high-reading control well reading - low-reading control well reading)*100;
4. high-reading control wells: cells with addition of 100 nL DMSO; and
5. low-reading control wells: cell-free wells.

Table 1 Antiproliferative activity of test compounds on SK-BR-3

| **Example and Compound No.** | **$IC_{50}$ (nM)** |
|---|---|
| Control compound (Dxd) | 11 |
| **3-1** | 13 |
| **3-2** | 15 |
| **4-1** | 61 |
| **4-2** | 14 |
| **5-1** | 20 |
| **5-2** | 20 |
| **6-1** | 11 |
| **6-2** | 7.3 |
| **7-1** | 8.6 |
| **7-2** | 7.6 |
| **8-1** | 44 |
| **8-2** | 87 |
| **10** | 10 |
| **11-1** | 2.2 |

(continued)

| Example and Compound No. | $IC_{50}$ (nM) |
|---|---|
| **11-2** | 2.4 |
| **12** | 5.7 |
| **17-1** | 4.6 |
| **17-2** | 6.6 |
| **18-1** | 13.8 |
| **18-2** | 9.6 |
| **19-1** | 8.3 |
| **19-2** | 7.6 |
| **20-1** | 21 |
| **20-2** | 25 |
| **24-1** | 47 |
| **24-2** | 16 |
| **25-1** | 80 |
| **25-2** | 19 |
| **33-1** | 7.8 |
| **33-2** | 8.4 |
| **34-1** | 5.1 |
| **34-2** | 4.8 |
| **35-1** | 16 |
| **35-2** | 9.0 |
| **36-1** | 9.2 |
| **36-2** | 4.4 |
| **37-1** | 19 |
| **37-2** | 6.6 |
| **38-1** | 12 |
| **38-2** | 5.4 |
| **39** | 11 |

The result shows that the compounds of the present invention have strong antiproliferative effects on the proliferation of cells SK-BR-3.

**Test Example 2** Experiment of inhibition by test compounds on proliferation of cells MDA-MB-468

1: Experimental materials

| Materials and reagents | Supplier | Art. No. |
|---|---|---|
| MDA-MB-468 | ATCC | HTB-132 |
| L-15 Medium | Hyclone | SH30525.01 |
| Penicillin-streptomycin | Gibco | 15140-122 |
| Fetal bovine serum | Gibco | 10099-141C |
| Phosphate buffer saline | Gibco | 10010-031 |
| TrypLE | Gibco | 12604-021 |
| DMSO | Sigma | D8418-1L |

(continued)

| Materials and reagents | Supplier | Art. No. |
|---|---|---|
| Staurosporine | MCE | HY-15141 |
| CelltiterGlo assay kit (CTG) | Promega | G7573 |

2: Experimental instruments

| Consumables and instruments | Supplier | Art. No. |
|---|---|---|
| White 384-well culture plate | PerkinElmer | 6007680 |
| Plate shaker | QILINBEIER | QB-9002 |
| Centrifuge | Eppendorf | 5810R |
| Carbon dioxide incubator | Thermo Scientific | 371 |
| Microscope | OLYMPUS | CKX41 |
| Echo Qualified 384-Well Polypropylene | LABCYTE | PP-0200 |
| Echo | LABCYTE | 655 |
| CountessII | Gibco | AMQAX1000 |
| Envision | PerkinElmer | 2105 |

3: Test method

(1) Cell plating: first, tumor cells MDA-MB-468 were cultured using a corresponding culture medium, trypsinized, centrifuged, resuspended for counting, adjusted to an appropriate concentration, and then plated on a 384-well plate.
(2) Co-incubation of the compounds and tumor cells: after the cells adhered to the wall, 100 nL of a diluted bioactive substance (test compound) was added to the cell culture plate using ECHO, the final concentration of DMSO in wells of the cell plate was 0.33%, and the incubation was performed in an incubator at 37°C with 0% $CO_2$ for 72 h.
(3) After the incubation was complete, 30 μL of a CTG reagent (CelltiterGlo kit) was added to each well. The culture was oscillated on a fast oscillator for 2 min, centrifuged at 1,000 rpm for 1 min, and kept away from light at room temperature for 30 min. The chemiluminescence signal value was read using an Envision instrument.
(4) Cell viability detection: $IC_{50}$ was calculated using GraphPad Prism 8 software, and the $IC_{50}$ of the compounds was obtained using the following nonlinear fitting formula (see Table 2):

6. Y=Bottom+(Top-Bottom)/(1+10^((Log$IC_{50}$-X)*HillSlope))
7. Y: inhibition rate; X: log value of compound concentration;
8. inhibition rate (%)=100-(compound well reading - low-reading control well reading)/(high-reading control well reading - low-reading control well reading)* 100;
9. high-reading control wells: cells with addition of 100 nL DMSO; and
10. low-reading control wells: cell-free wells.

Table 2 Antiproliferative activity of test compounds on MDA-MB-468

| **Example and Compound No.** | **$IC_{50}$ (nM)** |
|---|---|
| Control compound (Dxd) | 4.6 |
| **3-1** | 7.5 |
| **3-2** | 12 |
| **4-1** | 34 |
| **4-2** | 13 |
| **5-1** | 6.7 |
| **5-2** | 9.4 |

(continued)

| Example and Compound No. | $IC_{50}$ (nM) |
|---|---|
| **6-1** | 8.8 |
| **6-2** | 6.5 |
| **7-1** | 3.1 |
| **7-2** | 4.4 |
| **8-1** | 13 |
| **8-2** | 38 |
| **10** | 13 |
| **11-1** | 3.9 |
| **11-2** | 4.5 |
| **12** | 9.3 |
| **17-1** | 5.8 |
| **17-2** | 8.1 |
| **18-1** | 12 |
| **18-2** | 12 |
| **19-1** | 7.1 |
| **19-2** | 6.9 |
| **20-1** | 15.6 |
| **20-2** | 19.5 |
| **24-1** | 38 |
| **24-2** | 14 |
| **25-1** | 69 |
| **25-2** | 18 |
| **33-1** | 6 |
| **33-2** | 5 |
| **34-1** | 4 |
| **34-2** | 4 |
| **35-1** | 12 |
| **35-2** | 9 |
| **36-1** | 14 |
| **36-2** | 6.4 |
| **37-1** | 19 |
| **37-2** | 6.6 |
| **38-1** | 13 |
| **38-2** | 5.7 |
| **39** | 15 |

The result shows that the test compounds have strong antiproliferative effects on the proliferation of MDA-MB-468 cells.

**Test Example 3** Experiment of inhibition effect of test compounds on proliferation of NCI-N87 cells

1: Experimental materials

| Materials and reagents | Supplier | Art. No. |
|---|---|---|
| NCI-N87 | ATCC | HTB-30 |
| RPMI-1640 Medium | Gibco | A10491-01 |
| Penicillin-streptomycin | Gibco | 15140-122 |
| Fetal bovine serum | Gibco | 10099-141C |
| Phosphate buffer saline | Gibco | 10010-031 |
| TrypLE | Gibco | 12604-021 |
| DMSO | Sigma | D8418-1L |
| Staurosporine | MCE | HY-15141 |
| CelltiterGlo assay kit (CTG) | Promega | G7573 |

2: Experimental instruments

| Consumables and instruments | Supplier | Art. No. |
|---|---|---|
| White 384-well culture plate | PerkinElmer | 6007680 |
| Plate shaker | QILINBEIER | QB-9002 |
| Centrifuge | Eppendorf | 5810R |
| Carbon dioxide incubator | Thermo Scientific | 371 |
| Microscope | OLYMPUS | CKX41 |
| Echo Qualified 384-Well Polypropylene | LABCYTE | PP-0200 |
| Echo | LABCYTE | 655 |
| CountessII | Gibco | AMQAX1000 |
| Envision | PerkinElmer | 2105 |

3: Test method
(1) Cell plating: first, tumor cells NCI-N87 were cultured using a corresponding culture medium, trypsinized, centrifuged, resuspended for counting, adjusted to an appropriate concentration, and then plated on a 384-well plate.
(2) Co-incubation of the compounds and tumor cells: after the cells adhered to the wall, 100 nL of a diluted bioactive substance (test compound) was added to the cell culture plate using ECHO, the final concentration of DMSO in wells of the cell plate was 0.33%, and the incubation was performed in an incubator at 37°C with 0% $CO_2$ for 72 h.
(3) After the incubation was complete, 30 μL of a CTG reagent (CelltiterGlo kit) was added to each well. The culture was oscillated on a fast oscillator for 2 min, centrifuged at 1,000 rpm for 1 min, and kept away from light at room temperature for 30 min. The chemiluminescence signal value was read using an Envision instrument.
(4) Cell viability detection: $IC_{50}$ was calculated using GraphPad Prism 8 software, and the $IC_{50}$ of the compounds was obtained using the following nonlinear fitting formula (see Table 3) :
11. Y=Bottom+(Top-Bottom)/(1+10^((Log$IC_{50}$-X)*HillSlope))
12. Y: inhibition rate; X: log value of compound concentration;
13. inhibition rate (%)=100-(compound well reading - low-reading control well reading)/(high-reading control well reading - low-reading control well reading)*100;
14. high-reading control wells: cells with addition of 100 nL DMSO; and
15. low-reading control wells: cell-free wells.

Table 3 Antiproliferative activity of test compounds on cells NCI-N87

| **Example and Compound No.** | **$IC_{50}$ (nM)** |
|---|---|
| Control compound (Dxd) | 11 |
| **3-1** | 16 |
| **3-2** | 16 |
| **4-2** | 22 |
| **5-1** | 14 |
| **5-2** | 18 |
| **6-1** | 7.5 |
| **6-2** | 6.8 |
| **7-2** | 4.9 |
| **8-1** | 4.2 |
| **9** | 3 |
| **10** | 23 |
| **11-1** | 2.5 |
| **11-2** | 2.3 |
| **12** | 20 |
| **17-1** | 3.6 |
| **17-2** | 5.3 |
| **18-1** | 11.2 |
| **18-2** | 16.5 |
| **19-1** | 7.4 |
| **19-2** | 7.4 |
| **20-1** | 26 |
| **20-2** | 28 |
| **24-1** | 65 |
| **24-2** | 24 |
| **25-1** | 110 |
| **25-2** | 40 |
| **33-1** | 8 |
| **33-2** | 9 |
| **34-1** | 6 |
| **34-2** | 6 |
| **35-1** | 14 |
| **35-2** | 10 |
| **36-1** | 8.5 |
| **36-2** | 3.9 |
| **37-1** | 8.6 |
| **37-2** | 3.2 |
| **38-1** | 7.5 |
| **38-2** | 2.9 |

(continued)

| Example and Compound No. | $IC_{50}$ (nM) |
|---|---|
| **39** | 9.3 |

The result shows that the test compounds have strong antiproliferative effects on the proliferation of cells NCI-N87.

**Test Example 4** Kinetic solubility test in PBS 7.4

4.1 Experimental steps

1) Preparation of stock solutions

10 mM stock solutions of the test substances **6-2, 11-2, 17-2, Dxd,** and the control drugs progesterone and diclofenac were prepared using DMSO respectively.

2) Kinetic solubility test steps

15 μL of 10 mM stock solution of each of the test substances was added to a corresponding position of a corresponding 96-well plate in a specified sequence. 485 μL of the PBS 7.4 was added to a corresponding vial on a sample plate. The experiment was run in duplicate. Each vial was additionally provided with a stirring rod and was plugged. Then, the sample plate was transferred in a thermostatic mixer, and oscillated at a revolving speed of 1,100 rpm at 25°C for 2 h. 2 h later, the plug was removed, the stirring rod was attracted away with a large magnet, and then the sample was transferred from the sample plate to the filter plate. Negative pressure was generated using a vacuum pump to filter the sample. 5 μL of filtrate and 5 μL of blank DMSO were transferred to another sample plate, and then 490 μL of internal standard water (acetonitrile:water=1:1) containing the internal standard was added. Depending on peak shapes, a certain proportion of internal standard water may be used to dilute the sample diluent to give better peak shapes.

3) Preparation of 3 μM of standard substance solution

6 μL was transferred from the 10 mM DMSO stock solution plate to a blank plate, and was prepared into a 300 μM standard substance solution with addition of 194 μL of DMSO. 5 μL was transferred from the 300 μM standard substance solution plate to still another blank plate, and then 5 μL of blank buffer and 490 μL of the internal standard water containing the internal standard (acetonitrile:water=1:1) were added until a final concentration of the standard substance solution was 3 μM.

4) Sample analysis steps

An injection plate was transferred on an injection tray of an autosampler to evaluate the sample by LC/MS analysis.

4.0. Data analysis

All calculations were performed using Microsoft Excel.

The sample filtrate was analyzed and quantified by qualitative and quantitative LC/MS of standard substance peaks at known concentrations. The calculation formula for the solubility values of the reference drugs and the test substances are as follows:

$$[\text{Sample}] = \frac{AREA_{Sample} \times DF_{Sample} \times [STD]}{AREA_{Std}}$$

[Sample] is the compound concentration, $DF_{Sample}$ refers to the sample dilution factor, [STD] is the standard substance concentration of the compound, $AREA_{Std}$ is the standard substance peak area of the compound, and $AREA_{Sample}$ is the peak area of the compound sample.

4.2 Experimental result

The experimental result is as shown in Table 4.

Table 4 Solubility of representative compounds in PBS 7.4

| Compound | **6-2** | **11-2** | **17-2** | **Dxd** |
|---|---|---|---|---|
| Solubility in PBS 7.4 (μM) | 170 | 34 | 1.7 | 11.3 |

The result shows that the representative compounds **6-2** and **11-2** have significantly better solubility in the PBS 7.4 than Dxd.

**Test Example 5** Pharmacokinetic Test in SD Rats

After a single intravenous injection (dose: 2 mpk) of the compounds **6-2, 11-2, 17-2,** and **Dxd** into male SD rats (blood collection time points: 0.083 h, 0.25 h, 0.5 h, 1 h, 2 h, 4 h, 8 h, and 24 h after IV administration, totaling 8 blood collection time points), average pharmacokinetic parameters in the plasma are as shown in Table 5:

Table 5 Pharmacokinetic test result of representative compounds

| Compound | $C_0$ (ng/mL) | $AUC_{last}$ (h*ng/mL) | $AUC_{inf}$ (h*ng/mL) | $T_{1/2}$ (h) | CL (mL/min/kg) | $V_{ss}$ (L/kg) |
|---|---|---|---|---|---|---|
| **6-2** | 677 | 314 | 318 | 0.39 | 107.9 | 2.93 |
| **11-2** | 848 | 460 | 465 | 0.63 | 72.7 | 3.01 |
| **17-2** | 2693 | 2253 | 2734 | 1.64 | 12.32 | 1.4 |
| **Dxd** | 748 | 301 | 302 | 0.39 | 113 | 2.70 |

The result shows that the representative compounds of the present application are rapidly removed from the rats, have good safety, and have good pharmacokinetic properties.

**Test Example 6** Experiment of inhibition effect of compounds on human hepatomicrosomes CYP1A2, CYP2D6, and CYP3A4

6.1 Positive control inhibitors of each subtype

| CYP | Inhibitor | Final concentration (μM) |
|---|---|---|
| 1A2 | Furafylline | 0, 0.1, 0.3, 1, 3, 10, 30 |
| 2D6 | Quinidine | 0, 0.0015, 0.005, 0.015, 0.05, 0.15, 0.5 |
| 3A4 | Ketoconazole | 0, 0.0015, 0.005, 0.015, 0.05, 0.15, 0.5 |

6.2 Preparation of substrate stock solutions

| CYP | Labeled substrate | Working concentration (μM) | Final substrate concentration (μM) | Incubation time (min) |
|---|---|---|---|---|
| 1A2 | Phenacetin | 800 (100mM PBS) | 40 | 20 |
| 2D6 | Dextromethorphan | 40 (100mM PBS) | 2 | 20 |
| 3A4M | Midazolam | 20 (100mM PBS) | 1 | 5 |
| 3A4T | Testosterone | 80 (100mM PBS) | 40 | 10 |

6.3 Experimental process

The incubation was performed in a 96-well deep well plate. The following volumes were added to each well of the incubation plate: 169 μL of "master solution" and 1 μL of each of the test compounds or positive control compound (DMSO) at various concentrations. The incubation plate was transferred to a water bath for pre-incubation at 37°C for 5 min. Then, 10 μL of diluted substrate solution was added to the incubation plate, and mixed and incubated on a vortex mixer for 15 sec. Then, 20 μL of 10 mM NADPH solution was added, and the reaction started from the final concentration of 1 mM. At a predetermined time point, 300 μL of a quenching solution (cold acetonitrile with addition of 3% formic acid, 200 nM alprazolam, 200 nM labetalol hydrochloride, and 200 nM tolbutamide) was added to quench the reaction. The mixture was centrifuged at 3,220 g for 40 min. 150 μL of the supernatant was transferred to another plate. The supernatant can be diluted with 150 μL of pure water. The mixture was fully mixed, and the content of substrate metabolites was determined by LC/MS/MS.

6.4 Data analysis

Automatic peak integration domains were checked for all samples. The peak areas and internal standard peak areas were analyzed, and then exported to an excel spreadsheet. Inhibition of each enzyme P450 in human hepatomicrosomes was measured as the percentage decrease of marker metabolite-forming activity, as compared to non-inhibited controls (=100% activity). The $IC_{50}$ values were calculated as the logarithm of residual activity (%) and inhibitor concentration.

The percentage of the residual activity was calculated as follows:

$$\text{Area ratio} = \text{analyte peak area/internal standard peak area}$$

$$\text{Residual activity (\%)} = \text{area ratio}_{\text{test drug}} / \text{area ratio}_{\text{blank control}} * 100\%$$

The $IC_{50}$ value was calculated using Excel XLfit 5.5.1.3.

The experimental result is as shown in Table 6.

Table 6 Experimental result of inhibition effect of representative compounds on human hepatomicrosomes

| Compound | 1A2 ($IC_{50}$ μM) | 2D6 ($IC_{50}$ μM) | 3A4T ($IC_{50}$ μM) | 3A4M ($IC_{50}$ μM) |
|---|---|---|---|---|
| **6-2** | >30 | >30 | >30 | >30 |
| **11-2** | >30 | >30 | >30 | >30 |
| **17-2** | >30 | >30 | >30 | >30 |
| **Dxd** | >30 | >30 | >30 | >30 |

The result shows that the representative compounds of the present application have weak inhibitory effect on CYP enzymes and have good safety.

**Test Example 7** Test of stability of compounds in human plasma

7.1 Experimental process

(1) 199 μL of human plasma was added to the culture plate of each cell. The culture plate was preheated to 37°C, and kept at this temperature for 15 min.
(2) After preincubation, 1 μL of 1 mmol/L each test compound and 1 μL of 1 mmol/L control compound were added to 199 μL of plasma, so that a final concentration of each test compound was 5 μmol/L and that of the control compound was 5 μmol/L. The final concentration of the organic solvent was 0.5%. The experiment would be duplicated twice.
(3) Reaction samples were incubated at 37°C.
(4) The reaction was stopped by adding 600 μL of cold methanol containing the internal standard at 0, 1, 2, 6, and 24 h.

Each sample was vortex mixed for 10 min, and then centrifuged at 3,220 g for 30 min to precipitate the protein. 100 μL of the supernatant was transferred to another plate. Based on the signal response and peak shapes of liquid chromatography-mass spectrometry (LC-MS), the supernatant would be diluted with ultrapure water.

7.2 Data analysis

All calculations were performed using Microsoft Excel. The peak area ratio was determined from the chromatogram of extracted ions. The percentage of compounds remaining at each time point was calculated as per the following formula:

$$\text{Residual percentage}_{\text{tmin}}\ (\%) = \text{peak area ratio}_{\text{tmin}} / \text{peak area}_{\text{t0}} \times 100$$

The experimental result is as shown in Table 7.

Table 7 Test result of stability of representative compounds in human plasma

| Compound | Residual percentage (%) | | | | | |
|---|---|---|---|---|---|---|
| | 0 h | 1 h | 2 h | 4h | 6 h | 24 h |
| 6-2 | 100.00 | 96.62 | 86.16 | 93.65 | 88.99 | 87.01 |
| 11-2 | 100.00 | 96.80 | 92.06 | 93.40 | 94.45 | 88.81 |
| 17-2 | 100.00 | 90.07 | 89.34 | 93.75 | 97.06 | 102.21 |
| Dxd | 100.00 | 89.43 | 86.80 237 | 83.58 | 86.42 | 82.33 |

The result shows that the representative compounds 6-2, 11-2, and 17-2 of the present application have good stability in human plasma.

**Test Example 8** ADC biological activity assay

1. Test purpose

The purpose of this experiment is to detect the inhibitory activity of ADC compounds on the proliferation of HER2-expressing Calu-3 cells, SK-BR-3 cells and HER2-negative MDA-MB-468 cells in vitro. Cells were treated with different concentrations of compounds in vitro. After 5 days of culture, the cell proliferation was detected using CTG CellTiter-Glo® Luminescent Cell Viability Assay, and the in vitro activity of the compound was evaluated based on the $IC_{50}$ value.

2. Test method:

(1) On the first day, tumor cells were plated in a 96-well plate, with 5000 cells/100 μL culture medium in each well, and 100 μL DPBS was seeded in each empty well at the edge. The cells were incubated overnight in a 37°C incubator.
(2) On the second day, the old culture medium (50 μL/well) was removed by pipetting; ADC of different concentration gradients was added, with the starting concentration of ADC being 200 nM, and 5-fold dilutions for 9 concentrations. The dosing volume was 50 μL/well.
(3) On the sixth day, CellTiter-Glo Buffer and CellTiter-Glo Substrate reagents were thawed at 4°C. Before use, 10 ml of buffer was taken by pipetting and added to the substrate, the system was mixed well, and equilibrated to room temperature.
(4) On the seventh day, the 96-well plate was equilibrated at room temperature for 30 min and 100 μL of Cell-Titer-Glo was added to each well. After shaking at room temperature in the dark for 5 min, the system was incubated for 10 min, 100 μL of the liquid in the well was transferred to the blank plate, and then detected for chemiluminescence with a microplate reader;

3. Data analysis

The data were processed and analyzed using Microsoft Excel and Graphpad Prism 5. The inhibitory activity of the

tested ADC compounds on the proliferation of Calu-3 cells, SK-BR-3 cells, and MDA-MB-468 cells in vitro is shown in Table 8 below.

Table 8

| Compound No. | $IC_{50}$ (nM) | | |
|---|---|---|---|
| | Calu-3 | SK-BR-3 | MDA-MB-468 |
| ADC-1 | 0.43 | 0.09 | >50 |
| ADC-2 | 0.42 | 0.07 | >50 |
| ADC-3 | 0.39 | 0.085 | >50 |
| ADC-5 | 0.47 | 0.066 | >50 |
| ADC-6 | 0.38 | 0.083 | >50 |
| ADC-7 | 0.43 | 0.099 | >50 |
| ADC-10 | - | 0.11 | >50 |
| ADC-11 | 0.40 | 0.06 | >50 |
| ADC-12 | 0.81 | 0.07 | >50 |
| ADC-13 | 0.73 | 0.10 | >50 |

Conclusion: The antibody-drug conjugates targeting HER2 of the present invention have obvious proliferation inhibitory activity on HER2-positive cells SK-BR-3 and Calu-3; at the same time, they have weak proliferation inhibitory activity on HER2-negative cells MDA-MB-468; therefore, they have good selectivity.

**Test Example 9: Her2-ADC plasma stability experiment**

The mice used in this experiment were CD-1 mice, the rats used were SD rats, and the monkeys used were cynomolgus monkeys.

(1) Free toxin release test and results

The DS8201 sample, ADC-5, and ADC-10 were respectively added to the above-mentioned sterile mouse plasma, sterile rat plasma, sterile human plasma, and sterile monkey plasma at a final concentration of 200 μg/mL. ADC-14 and ADC-15 were added to the above-mentioned sterile mouse plasma and sterile human plasma at a final concentration of 200 μg/mL. The cells were incubated in a 37°C cell culture incubator. The day when incubation started was recorded as day 0. Samples were then taken out on day 1, day 4, day 7, day 14, and day 21, respectively, to detect the content of free toxins.

The results of free toxin release showed that ADC-5 and ADC-10 were quite stable in the plasma of mice, rats, humans and monkeys. The maximum free toxin release rate did not exceed 0.3%, and was significantly better than the reference DS8201, as shown in Figs. 1-3.

The results of free toxin release showed that the free toxin release rates of ADC-5 and ADC-10 in mouse and human plasma were better than those of ADC-14 and ADC-15, as shown in Figs. 4-5. This indicates that the introduction of a suitable substituent (such as methoxy) on the pyrimidine ring can improve the stability of ADC in plasma, thereby reducing the safety issues of ADC caused by toxin shedding.

(2) ADC DAR value test and results

The DS8201 sample, ADC-5, ADC-10, and ADC-13 were added to the above-mentioned sterile human plasma at a final concentration of 200 ug/mL and incubated in a 37°C cell culture incubator. The day when incubation started was recorded as day 0, and then samples were taken out on day 1, day 4, day 7, day 14, and day 21, respectively, to detect changes in DAR values.

Table 9 shows the experimental results of ADC DAR value change: the change of DAR value of the conjugate formed by the small molecule linker of the present invention in human plasma is significantly smaller than that of DS8201, showing better plasma stability, which further confirms the stability of the small molecule linker of the present invention.

Table 9: ADC plasma stability (DAR value change)

| ADC | 0 (Dav) | 1 (Dav) | 4 (Dav) | 7 (Dav) | 14 (Dav) | 21 (Day) |
|---|---|---|---|---|---|---|
| DS8201 | 7.62 | 5.96 | 4.75 | 4.31 | 3.74 | 3.58 |
| ADC-5 | 7.93 | 7.90 | 7.90 | 7.90 | 7.89 | 7.88 |
| ADC-10 | 7.89 | 7.87 | 7.86 | 7.87 | 7.80 | 7.78 |
| ADC-13 | 7.79 | 7.77 | 7.76 | 7.75 | 7.75 | 7.73 |

**Test Example 10: Her2-ADC Mouse PK Experiment**

Experimental objective

C57BL/6J mice were used as test animals to evaluate the pharmacokinetic properties of Her2-ADC in the present application.

1. Test drugs

ADC-5: 10 mg/kg
ADC-9: 10 mg/kg
ADC-10: 10 mg/kg
ADC-13: 10 mg/kg

2. Preparation method: All of them were diluted with PBS.
3. Experimental method

Fifteen mice were given the drug via tail vein, and plasma samples were collected at 10 time points at 0.083, 2, 8, 24, 48, 96, 168, 336, 504, and 672 h from the mice. The concentrations of total antibody and ADC in the samples were determined by ELISA. The experimental results are shown in Table 10, and the ADC molecules of the present application have good pharmacokinetic properties.

Table 10: ADC pharmacokinetic data

| Sample name | $AUC_{0\text{-last}}$ (ng.h/mL) | $T_{1/2}$ (h) | $Vd_{ss}$ (L/kg) | Cl (mL/min/kg) |
|---|---|---|---|---|
| ADC-5 | 24186610 | 168 | 0.117 | 0.00869 |
| ADC-9 | 18654000 | 187 | 0.155 | 0.0113 |
| ADC-10 | 22053380 | 231 | 0.159 | 0.00849 |
| ADC-13 | 26037020 | 213 | 0.131 | 0.00777 |
| **Test Example 11: Efficacy evaluation in NCI-N87 tumor-bearing mice** | | | | |

Experimental objective

Balb/c nude mice were used as test animals to evaluate the efficacy of Her2-ADC in the present application.

**1. Test drugs**

ADC-3: 1.5 mg/kg
ADC-5: 1.5 mg/kg
ADC-10: 1.5 mg/kg
Reference ADC (DS8201): 1.5 mg/kg
Blank: PBS

2. Preparation method: All of them were diluted with PBS.
3. Experimental method

NCI-N87 cells were seeded subcutaneously in the right rib of mice. After the tumors grew for 7 days, the animals were randomly divided into groups, 6 animals per group, for a total of 5 groups (4 experimental groups + 1 blank group).

The drug was administered once by tail vein injection. Tumor volume and body weight were measured twice a week for four weeks, and the data were recorded. Data statistics were calculated using Excel 2023 statistical software: the mean value was calculated using avg; the SD value was calculated using STDEV; the SEM value was calculated using STDEV/SQRT; and the P value of the difference between groups was calculated using TTEST. The experimental results are shown in Table 11 and Fig. 6. The ADC molecules of the present application can significantly reduce the tumor volume and have a comparable tumor inhibition effect compared to the reference ADC.

Table 11: In vivo tumor inhibition effect of ADC on NCI-N87 transplant model

| Sample No. | TGI(%) |
|---|---|
| Reference ADC (DS8201) | 77 |
| ADC-3 | 83 |
| ADC-5 | 64 |
| ADC-10 | 66 |

**Test Example 12: Efficacy evaluation in JIMT-1 tumor-bearing mice**

Experimental objective

SCID Beige mice were used as test animals to evaluate the efficacy of Her2 -ADC in the present application.

**1. Test drugs**

ADC-3: 3 mg/kg
ADC-5: 3 mg/kg
ADC-9: 3 mg/kg
ADC-10: 3 mg/kg
ADC-13: 3 mg/kg
ADC-14: 3 mg/kg
Reference ADC (DS8201): 3 mg/kg
Blank: PBS

2. Preparation method: All of them were diluted with PBS.
3. Experimental method

JIMT-1 cells were seeded subcutaneously in the right rib of mice. After the tumors grew for 8 days, the animals were randomly divided into groups, 6 animals per group, for a total of 8 groups (7 experimental groups + 1 blank group).

The drug was administered once by tail vein injection. Tumor volume and body weight were measured twice a week for four weeks, and the data were recorded. Data statistics were performed using Excel 2023 statistical software: the mean value was calculated using avg; the SD value was calculated using STDEV; the SEM value was calculated using STDEV/SQRT; and the P value of the difference between groups was calculated using TTEST. The experimental results are shown in Table 12 and Fig. 7. The ADC molecules of the present application can significantly reduce the tumor volume and have better tumor inhibition effect than the reference ADC.

Table 12: In vivo tumor inhibition effect of ADC on JIMT-1 transplant model

| Sample No. | TGI(%) |
|---|---|
| Reference ADC (DS8201) | 103 |
| ADC-3 | 120 |
| ADC-5 | 114 |
| ADC-9 | 115 |
| ADC-10 | 115 |

(continued)

| Sample No. | TGI(%) |
|---|---|
| ADC-13 | 121 |
| ADC-14 | 110 |

The embodiments of the technical solutions of the present invention are described in an illustrative manner above. It is to be understood that the scope of protection of the present invention is not limited to the above-mentioned embodiments. Any modifications, equivalent substitutions, improvements, etc. made by those skilled in the art within the spirit and principles of the present invention should be included in the scope of protection of the claims of the present application.

## Claims

1. A compound represented by formula I', a racemate, a stereoisomer, a tautomer, an isotope-labeled counterpart, a solvate, a polymorph, a pharmaceutically acceptable salt or a prodrug compound thereof:

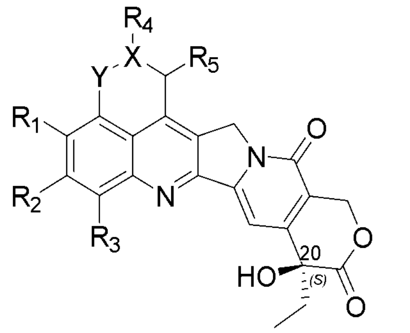

I'

wherein $R_1$, $R_2$, and $R_3$ are the same or different and each independently selected from H, OH, CN, halogen, $C_{1-10}$ alkyl, $C_{2-10}$ alkenyl, $C_{2-10}$ alkynyl, $C_{1-10}$ alkoxy, halo-$C_{1-10}$ alkyl, halo-$C_{1-10}$ alkoxy, cyano-$C_{1-10}$ alkyl, cyano-$C_{1-10}$ alkoxy, or $C_{3-10}$ cycloalkyl;

$R_4$ is selected from H or

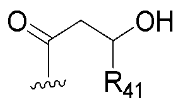 ;

$R_{41}$ is selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl;

$R_5$ is selected from H,

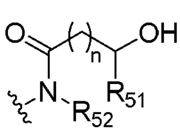 , 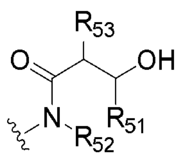 , or 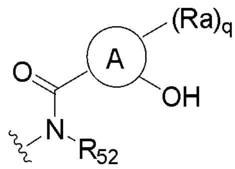 ;

$R_{51}$ and $R_{52}$ are the same or different and each independently selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl; $R_{53}$ is selected

from $C_{1-6}$ alkyl, $C_{1-6}$ haloalkyl, $C_{1-6}$ alkyl-NH-, ($C_{1-6}$ alkyl)$_2$N-, $C_{1-6}$ alkyl-NH-$C_{1-6}$ alkyl, ($C_{1-6}$ alkyl)$_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl; ring A is selected from $C_{3-8}$ cycloalkyl or $C_{3-8}$ heterocyclyl; Ra is selected from H, hydroxyl, CN, halogen, $C_{1-6}$ alkyl, or $C_{1-6}$ haloalkyl; n is selected from 0, 1, or 2; and q is selected from 0, 1, or 2;
X is selected from CH or N;
Y is selected from $-(CH_2)_m-O-(CH_2)_p-$;
m is an integer selected from 0-6; and
p is an integer selected from 0-6.

2. The compound according to claim 1, wherein $R_1$ is selected from H, OH, CN, halogen, $C_{1-6}$ alkyl, $C_{2-6}$ alkenyl, $C_{2-6}$ alkynyl, $C_{3-6}$ cycloalkyl, or halo-$C_{1-6}$ alkoxy;

preferably, $R_1$ is selected from H, OH, Br, methyl, difluoromethoxy, 2,2,2-trifluoroethoxy, vinyl, cyclopropyl, or ethynyl;
preferably, $R_2$ is selected from H, halogen, CN, or $C_{1-6}$ alkyl;
preferably, $R_2$ is selected from H or F;
preferably, $R_3$ is selected from H or $C_{1-6}$ alkyl; and
preferably, $R_3$ is H.

3. The compound according to claim 1 or 2, wherein $R_4$ is selected from H or

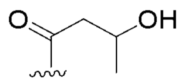
;

for example,

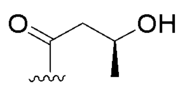
or
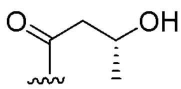
;

preferably, $R_4$ is selected from H or

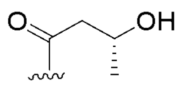
;

preferably, X-$R_4$ is

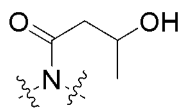
,

and is preferably

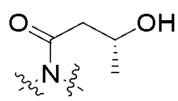
;

preferably, X-$R_4$ is $-CH_2-$; and
preferably, Y is selected from $-CH_2-O-$.

4. The compound according to any one of claims 1 to 3, wherein $R_5$ is selected from H,

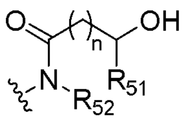 , 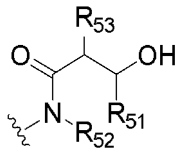 , or 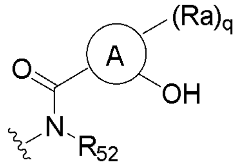 ;

wherein $R_{51}$ is selected from H, methyl, ethyl, isopropyl, or cyclopropyl; $R_{52}$ is selected from H or methyl; $R_{53}$ is selected from methyl; the ring A is selected from $C_{3-6}$ cycloalkyl; Ra is selected from H, hydroxyl, CN, halogen, $C_{1-6}$ alkyl, or $C_{1-6}$ haloalkyl; n is selected from 0 or 1; and q is selected from 0 or 1;

preferably, ring A is selected from a cyclobutane ring;
preferably, $R_5$ is selected from H,

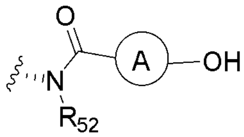 , 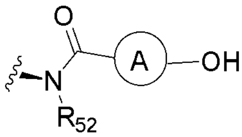 , 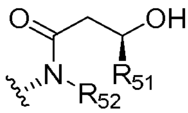 ,

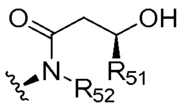 , 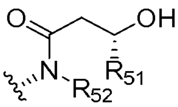 , 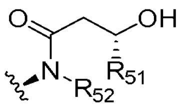 , 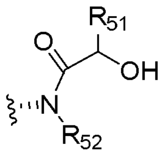 , 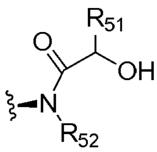 , 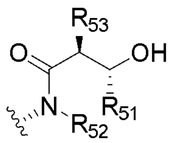 ,

or

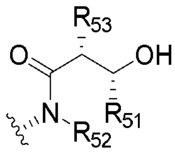 ;

preferably, $R_{51}$ is selected from H, methyl, ethyl, isopropyl, or cyclopropyl; $R_{52}$ is selected from H or methyl; $R_{53}$ is selected from methyl; and ring A is selected from a cyclobutane ring; and
preferably, $R_5$ is selected from H,

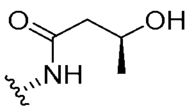 , 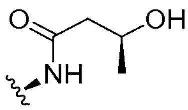 , 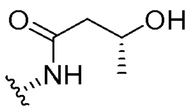 ,

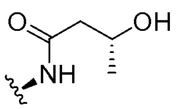 , 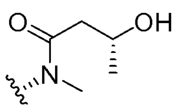 , 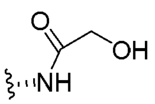 , 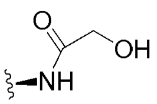 , 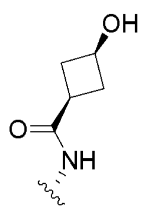 , 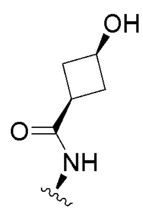 ,

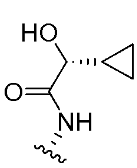 , 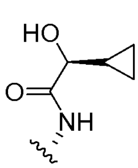 , 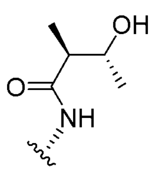 , 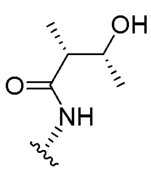 , 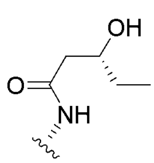 , 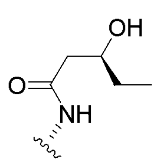 , 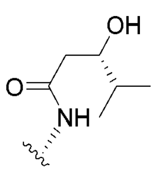 ,

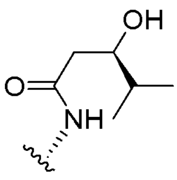, 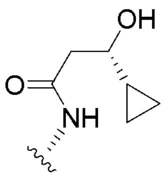 or 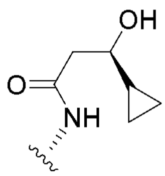 .

5. The compound according to any one of claims 1 to 4, wherein X is selected from CH or N; and when X is CH, $R_4$ is H; or when X is N, $R_5$ is H; and
preferably, m is selected from 0, 1 or 2; further preferably, m+p is 2.

6. The compound according to any one of claims 1 to 5, wherein the compound represented by formula I' has a structure as shown below:

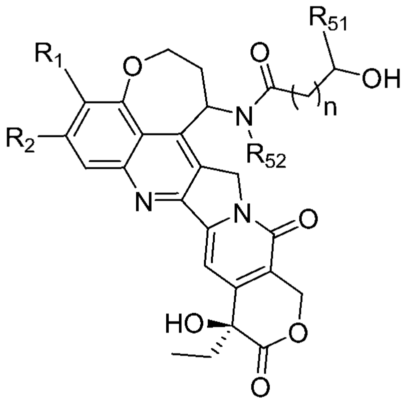 , 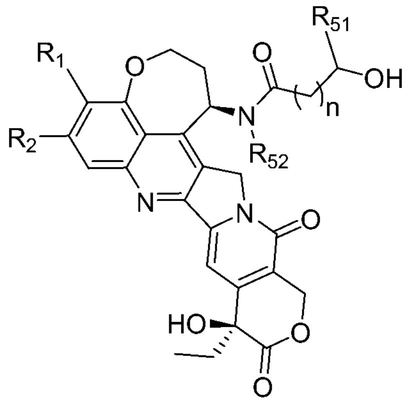 ;

III-d III-d-1

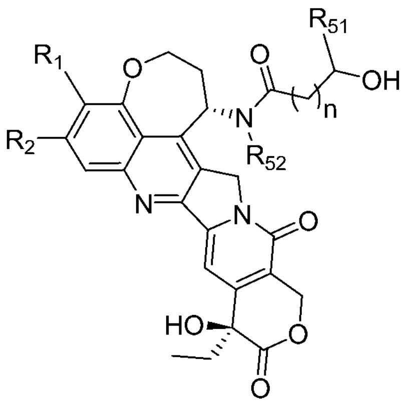 , 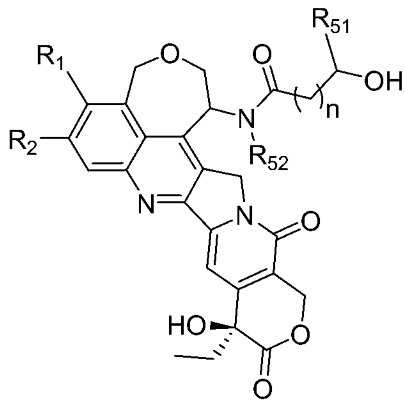 ;

III-d-2 III-e

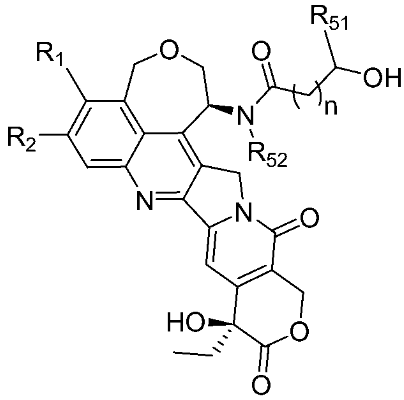 , 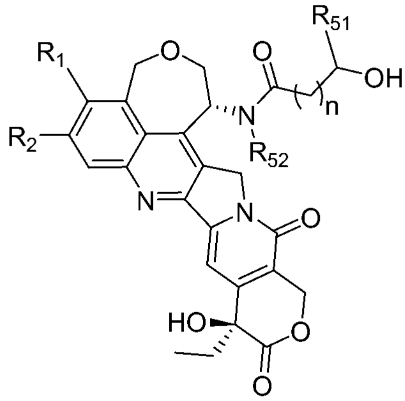 ;

III-e-1 III-e-2

wherein $R_1$, $R_2$, $R_{51}$, $R_{52}$, and n are each independently as defined in any one of claims 1 to 5.

**7.** The compound according to any one of claims 1 to 6, wherein the compound of formula I' has a structure as shown below:

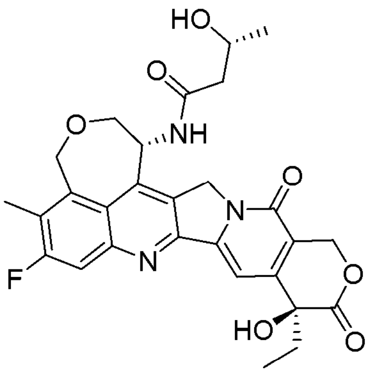 , 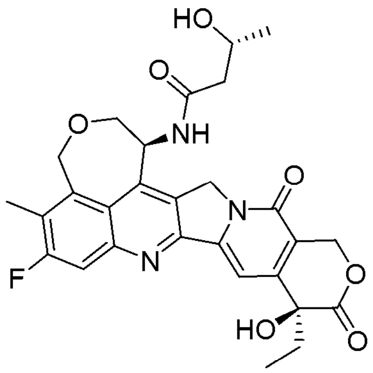 , 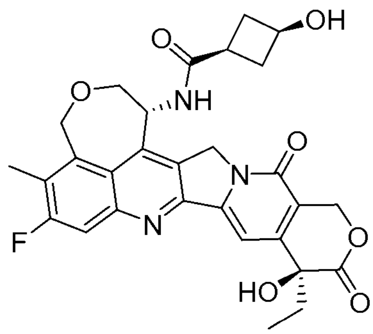 ,

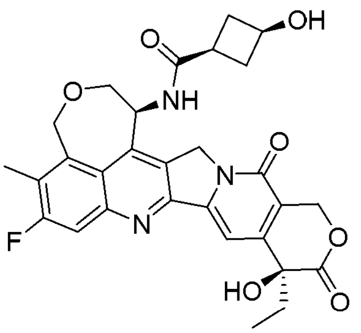 , 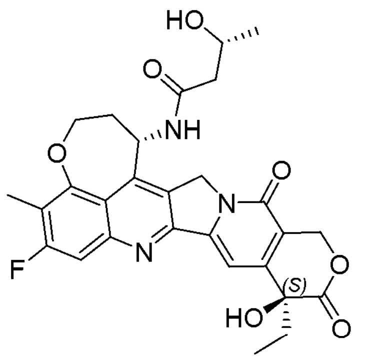 , 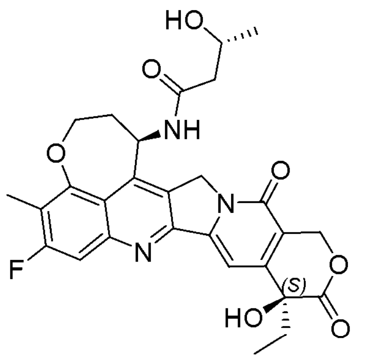 ,

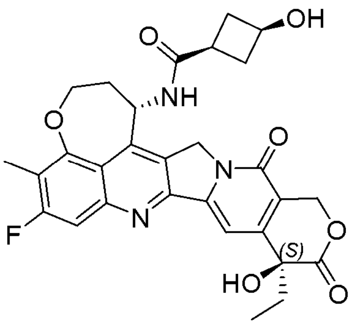,
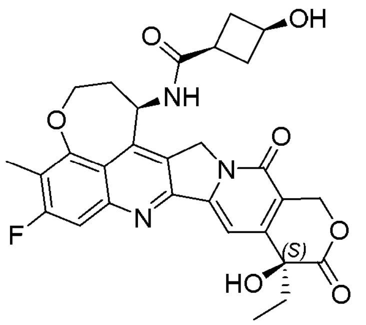,
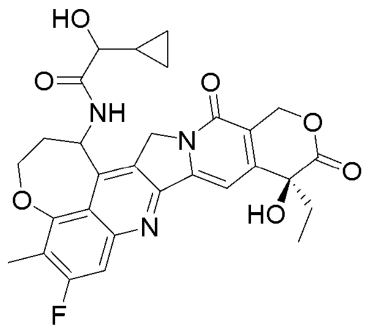,

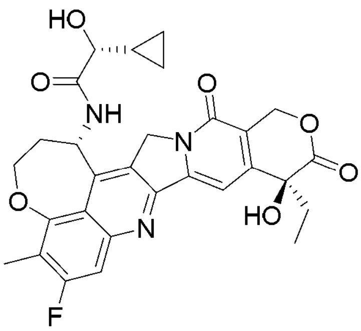,
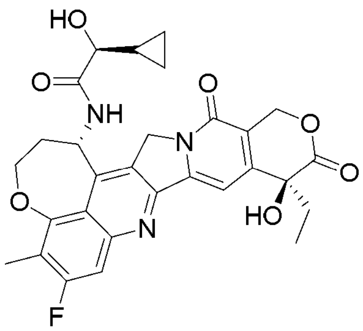,
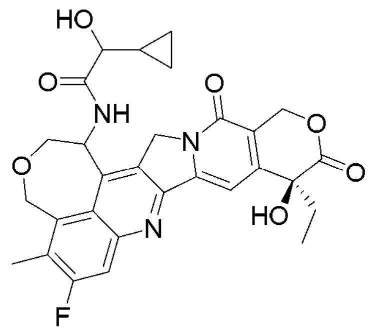,

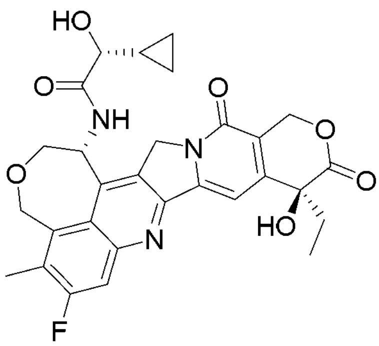, or
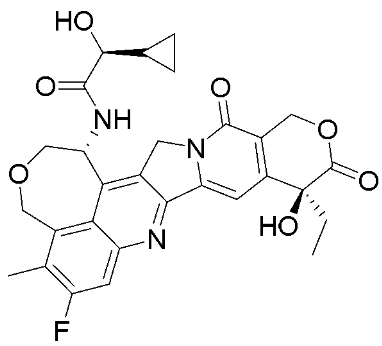.

**8.** A compound represented by formula V, a racemate, a stereoisomer, a tautomer, an isotope-labeled counterpart, a solvate, a polymorph, a pharmaceutically acceptable salt, or a prodrug compound thereof:

$M-L_1-L_2-D$ (Formula V)

wherein M is a linker site linking to an antibody or an antigen-binding fragment thereof;

$L_1$ is a peptide residue; preferably, it is selected from glycine-glycine-phenylalanine-glycine (GGFG), glutamic acid-valine-citrulline (EVC), valine-citrulline (VC), aspartic acid-valine-citrulline (DVC), glutamic acid-glycine-glycine-phenylalanine-glycine (EGGFG), or aspartic acid-glycine-glycine-phenylalanine-glycine (DGGFG); preferably, $L_1$ is selected from glycine-glycine-phenylalanine-glycine (GGFG);

$L_2$ is a linking group between the peptide residue and D; preferably, it is selected from a chemical bond, $-NH-C_{1-6}$ alkyl-,

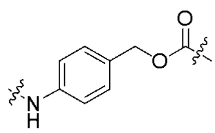 or 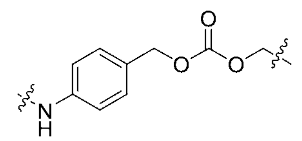

; preferably, $L_2$ is selected from $-NH-CH_2-$;

D is a structural fragment of a biologically active molecule; preferably, D is selected from a dehydrogenated structure of a compound represented by formula I;

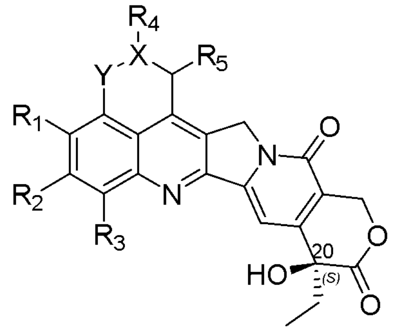

I

wherein $R_1$, $R_2$, and $R_3$ are the same or different and each independently selected from H, OH, CN, halogen, $C_{1-10}$ alkyl, $C_{2-10}$ alkenyl, $C_{2-10}$ alkynyl, $C_{1-10}$ alkoxy, halo-$C_{1-10}$ alkyl, halo-$C_{1-10}$ alkoxy, cyano-$C_{1-10}$ alkyl, cyano-$C_{1-10}$ alkoxy, or $C_{3-10}$ cycloalkyl;

$R_4$ is selected from H or

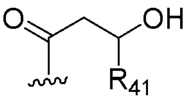 ;

$R_{41}$ is selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl;

$R_5$ is selected from H,

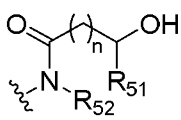 , 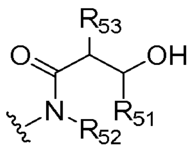 , or 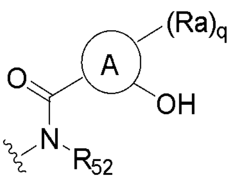 ;

$R_{51}$ and $R_{52}$ are the same or different and each independently selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl; $R_{53}$ is selected from $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl; ring A is selected from $C_{3-8}$ cycloalkyl or 3-8-membered heterocyclyl; Ra is selected from H, hydroxyl, CN, halogen, $C_{1-6}$ alkyl, or $C_{1-6}$ haloalkyl; n is selected from 0, 1, or 2; and q is selected from 0, 1, or 2;

X is selected from CH or N;

Y is selected from $-(CH_2)_m-$, or $-(CH_2)_m-O-(CH_2)_p-$;

m is an integer selected from 0-6;

p is an integer selected from 0-6;

more preferably, D has a structure as shown below:

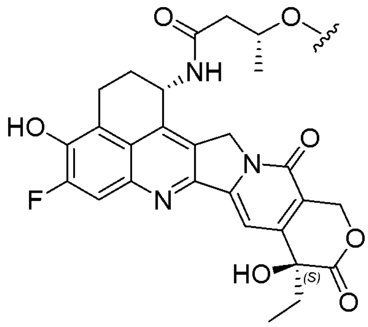,
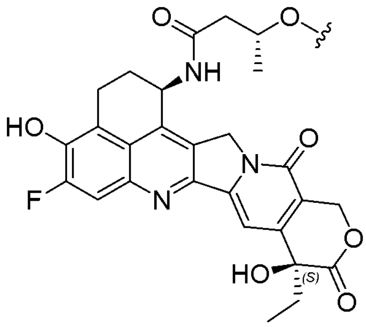,
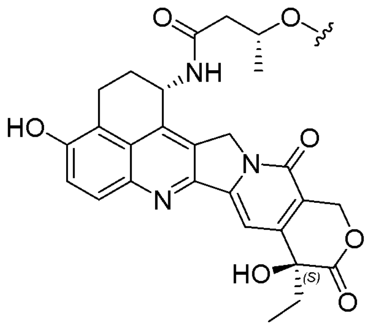,

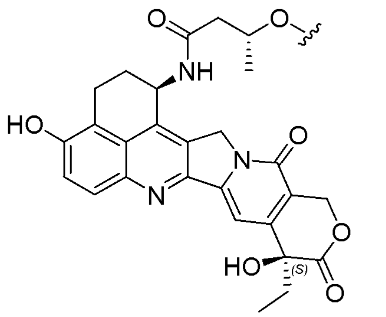,
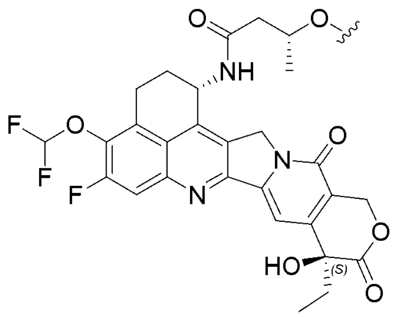,

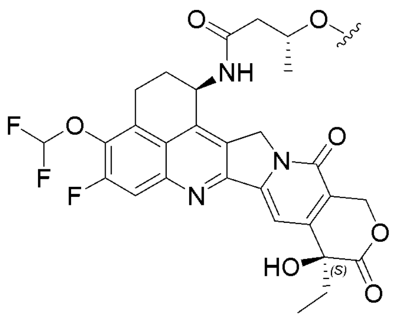,
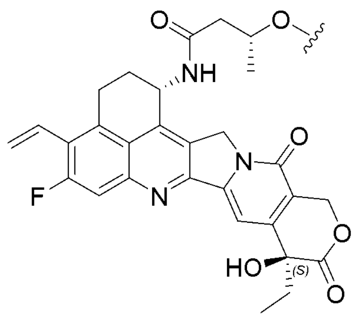,
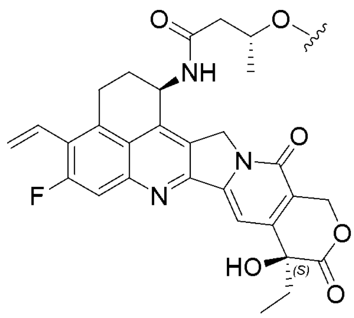,

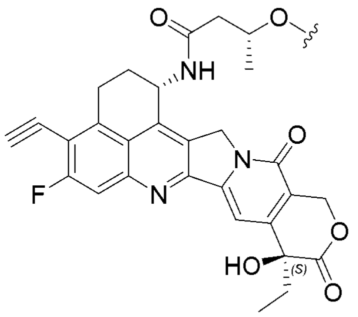,
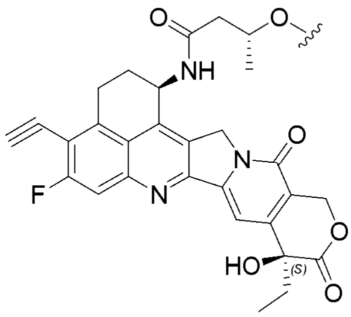,
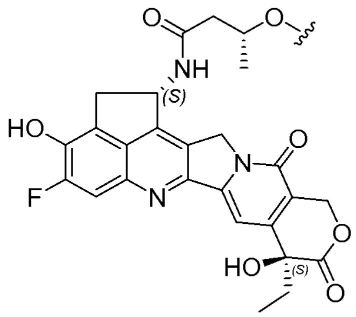,

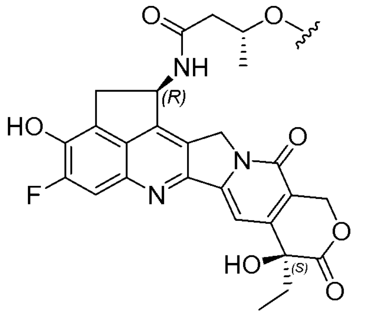,
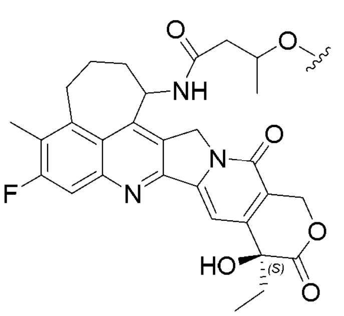,
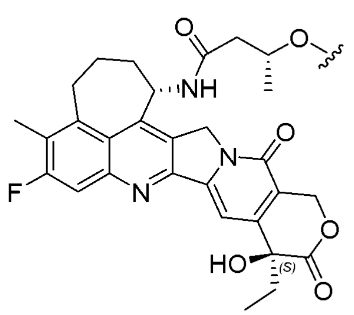,

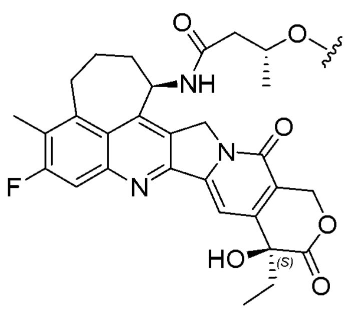
,
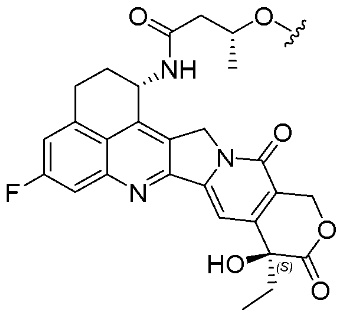
,
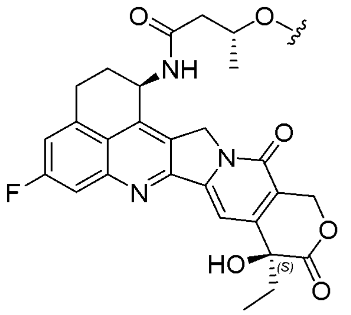
,
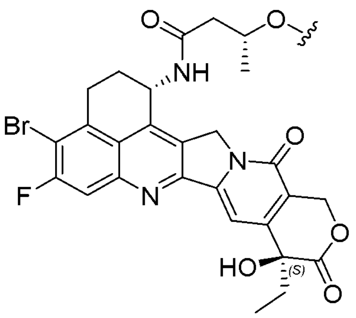
,
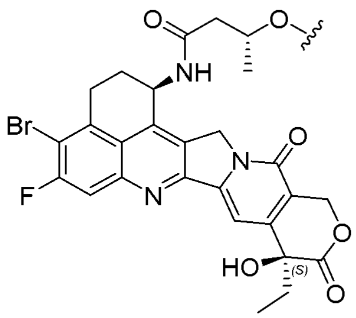
,
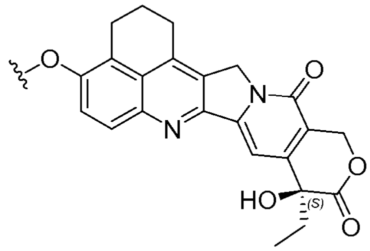
,
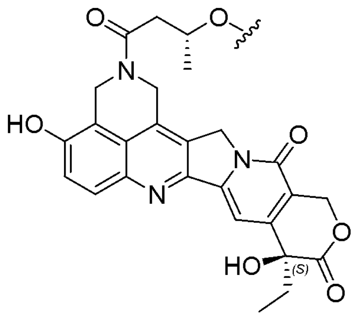
,
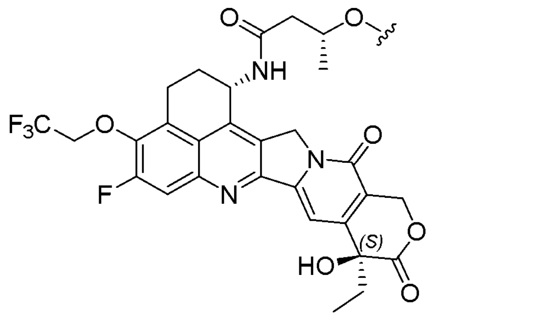
,
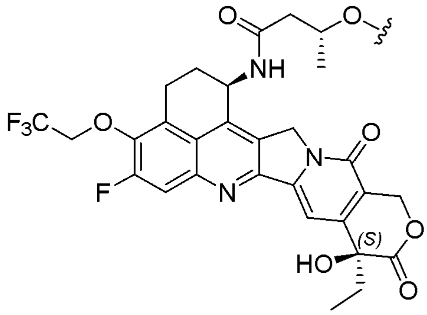
,
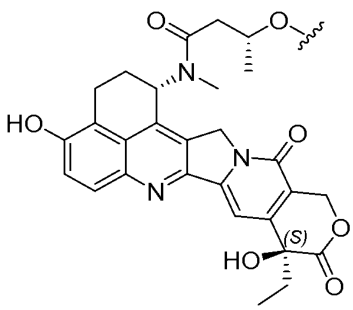
,
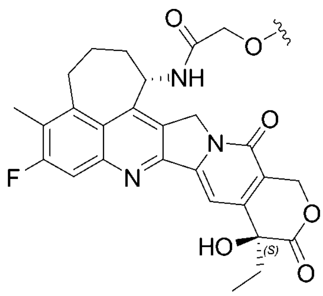
,

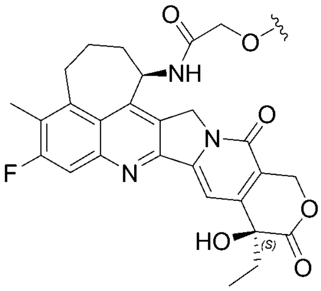 , 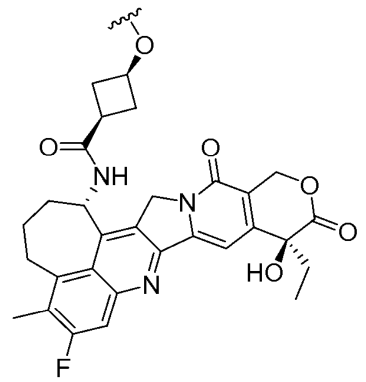 , 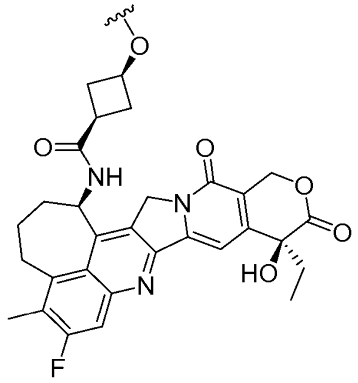 ,

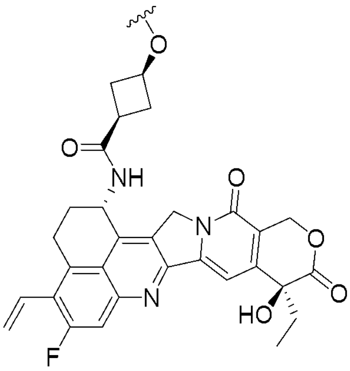 , 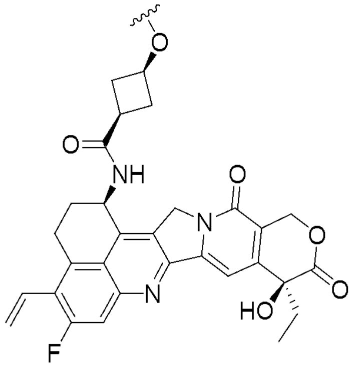 , 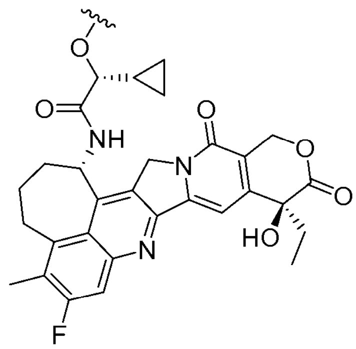 ,

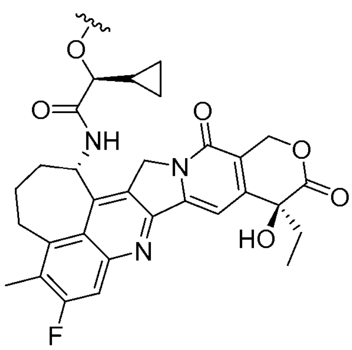 , 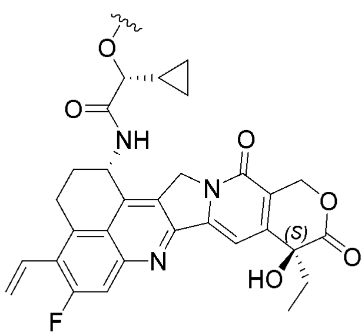 , 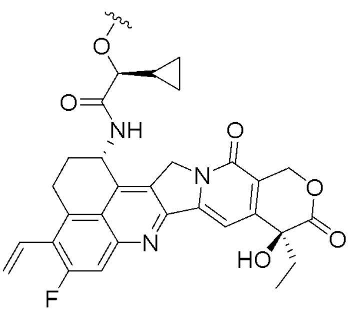 ,

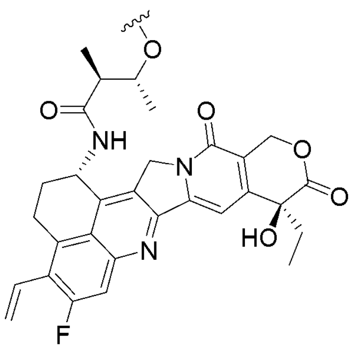 , 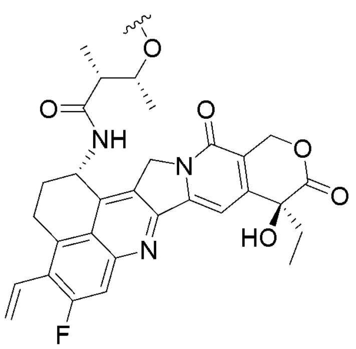 , 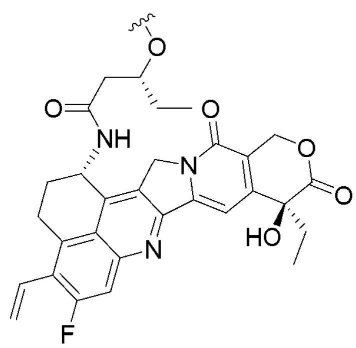 ,

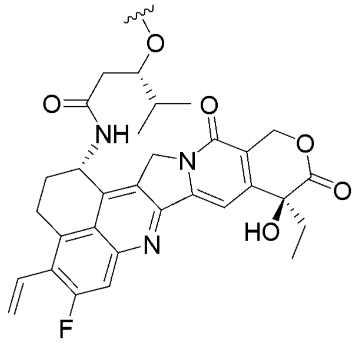
,
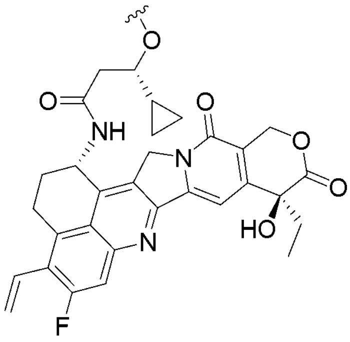
,
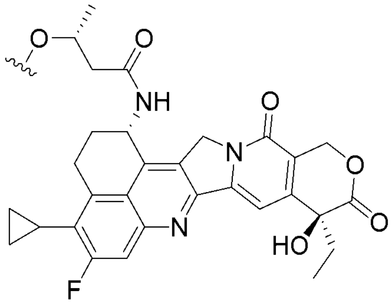
,

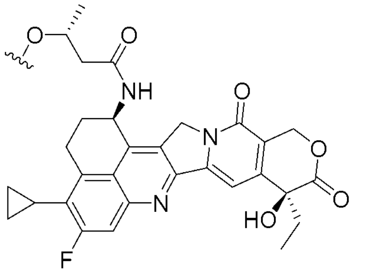
,
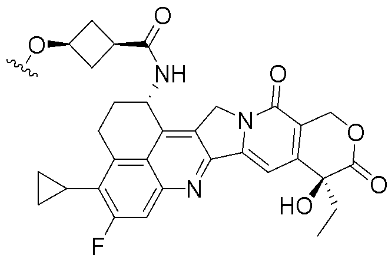
,

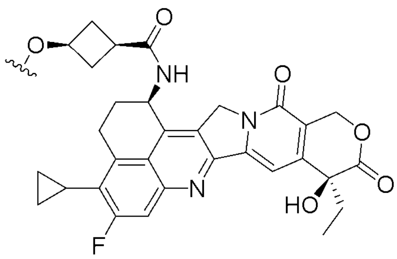
,
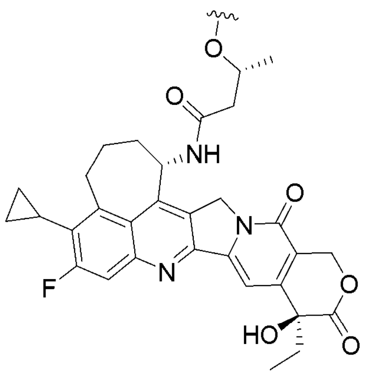
,

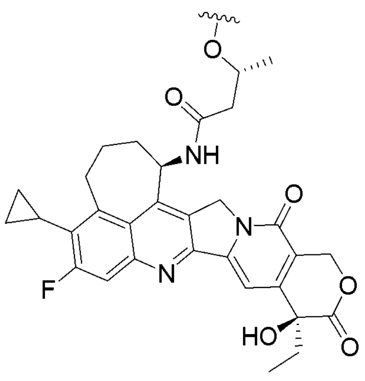
,
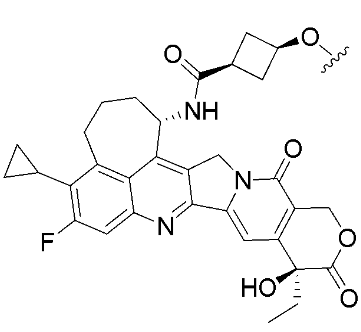
,
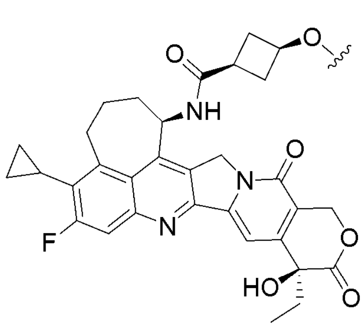
,

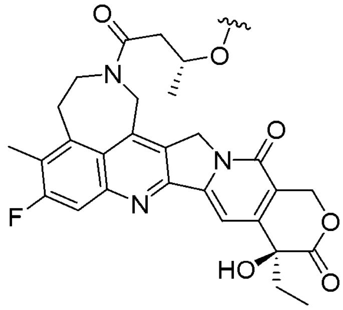
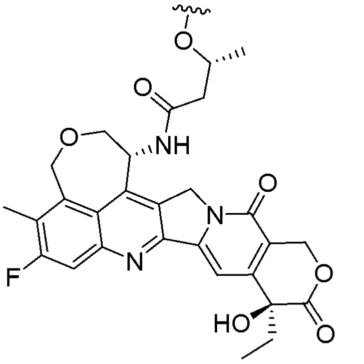
,
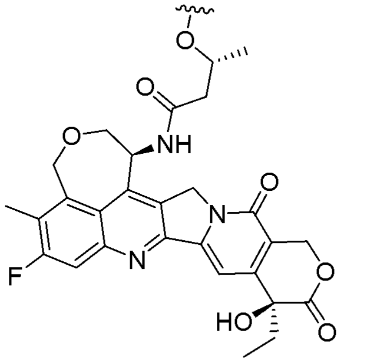
,

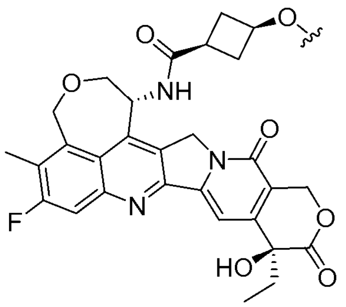
,
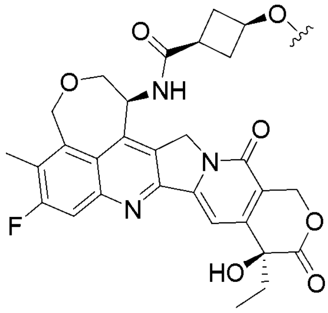
,
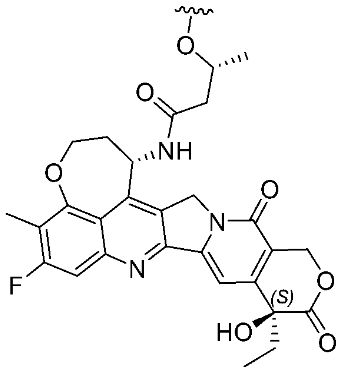
,

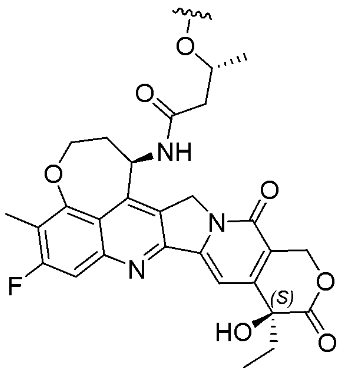
,
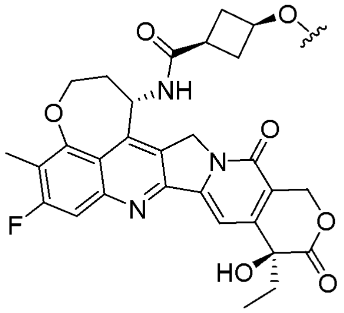
,
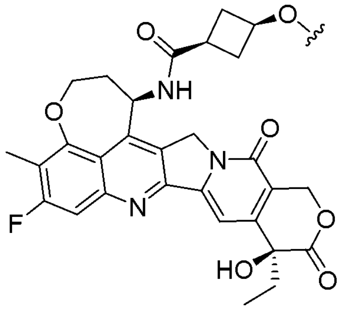
,

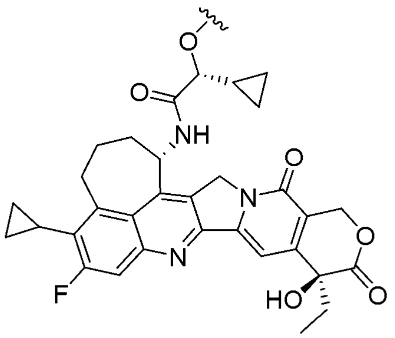
,
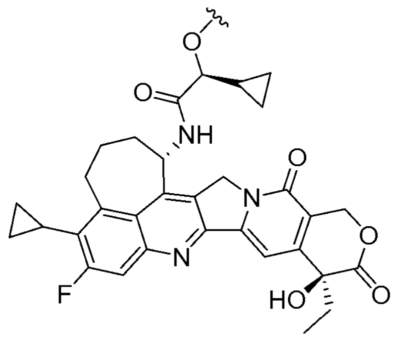
,

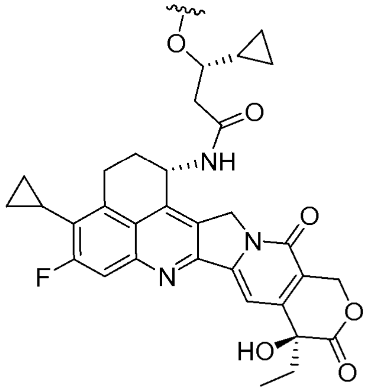, 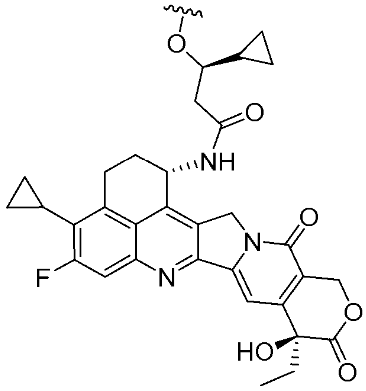, 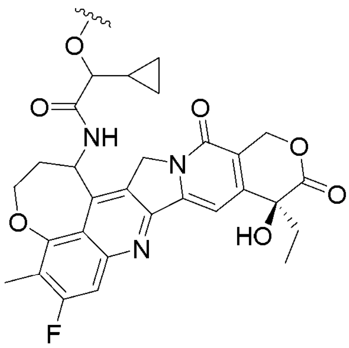,

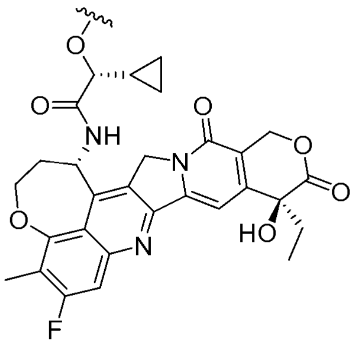, 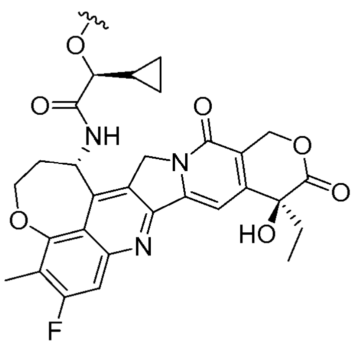, 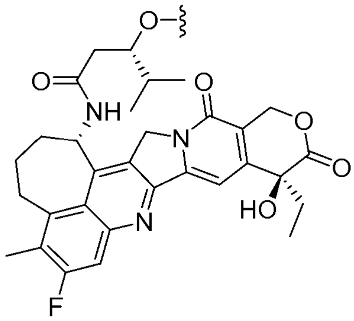,

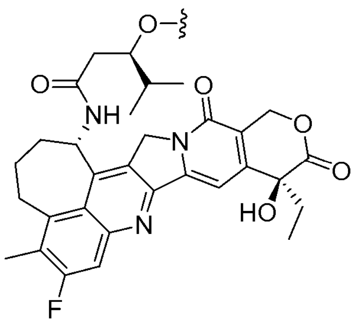, 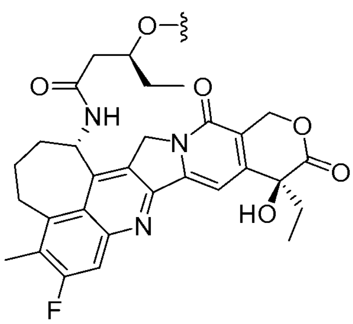, 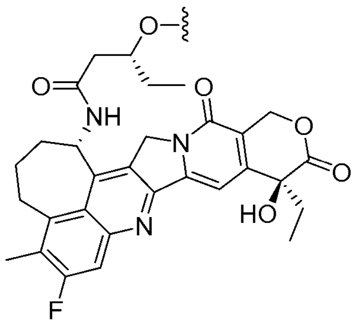,

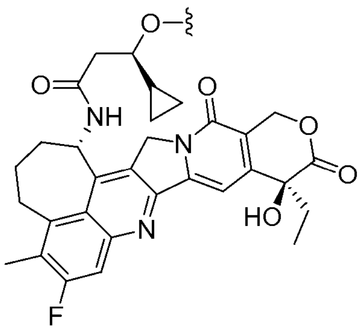, 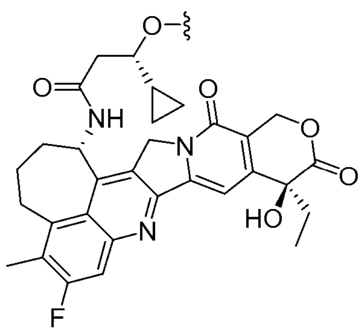, or 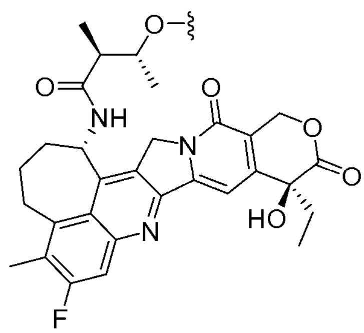;

preferably, M is selected from the following structure

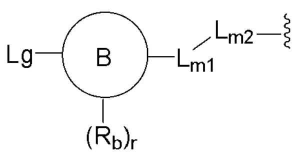

Lg is absent or is a leaving group, and the leaving group is selected from halogen, sulfone, trifluoromethanesulfonyl or methanesulfonyl; preferably, Lg is methanesulfonyl;

ring B is selected from a 5-14-membered heteroaromatic ring, or a 3-14-membered heterocyclic ring; preferably, ring B is selected from a 5-6-membered N-containing heteroaromatic ring or a 3-6 membered N-containing heterocyclic ring;

more preferably, ring B is selected from a pyrimidine ring, a pyridine ring, a triazine ring or

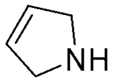;

each $R_b$ is the same or different and each independently selected from the following groups: halogen, cyano, oxo (=O), $C_{1-6}$ alkyl, halo-$C_{1-6}$ alkyl, hydroxy-$C_{1-6}$ alkyl, $C_{1-6}$ alkoxy or $C_{3-8}$ cycloalkyl, 3-8-membered heterocyclyl, $C_{1-6}$ alkyl-O-$C_{1-6}$ alkyl-, or $C_{1-6}$ alkyl-(5-6-membered) heteroaryl;

preferably, each $R_b$ is the same or different and each independently selected from cyano, oxo (=O), methoxy, cyclopropyl, trifluoromethyl,

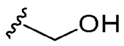, 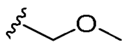, 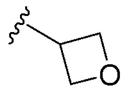 or 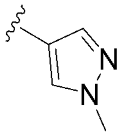;

$L_{ml}$ is absent, or is selected from the following groups which are unsubstituted or optionally substituted with one, two or more $R_{ml}$: $C_{6-14}$ aryl, 5-14-membered heteroaryl, or 3-14-membered heterocyclyl; each $R_{ml}$ is the same or different and each independently selected from H, halogen, cyano, $C_{1-6}$ alkyl, or HOOC-$C_{1-3}$ alkylene;

preferably, $L_{ml}$ is absent or is selected from the following groups which are unsubstituted or optionally substituted with one, two or more $R_{ml}$: phenyl, piperidinyl or piperazinyl;

more preferably, $L_{ml}$ is selected from

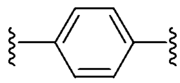, 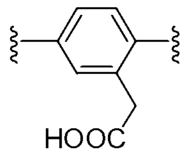, 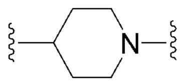

or

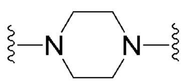;

$L_{m2}$ is selected from the following groups which are unsubstituted or optionally substituted with one, two, or more $R_{m2}$: $-(CH_2)_s-(C=O)-$, or -ethynyl-$(CH_2)_t-(C=O)-$;

preferably, $L_{m2}$ is selected from the following groups which are unsubstituted or optionally substituted with one, two, or more $R_{m2}$: $-CH_2-(C=O)-$, $-(CH_2)_2-(C=O)-$, $-(CH_2)_5-(C=O)-$, or - ethynyl-$(CH_2)_3-(C=O)-$;

each $R_{m2}$ is the same or different and each independently selected from H, halogen, cyano, $C_{1-6}$ alkyl or -$C_{1-6}$ alkylene-COOH, wherein the alkylene is optionally interrupted by one, two or more of the following groups: O, or NH;

preferably, each $R_{m2}$ is the same or different and each independently selected from -$C_{1-3}$ alkylene-COOH, and the alkylene is optionally interrupted by O or NH;

more preferably, $R_{m2}$ is selected from

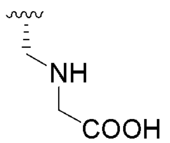, 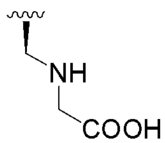, 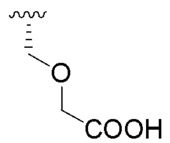,

or

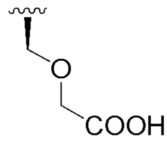;

r is an integer selected from 0-6;
s is an integer selected from 0-6;
t is an integer selected from 0-6;
preferably, M is selected from the following groups:

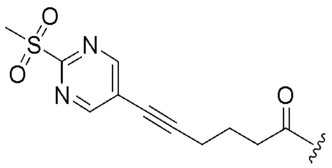,

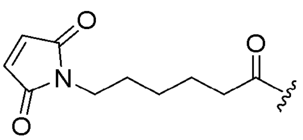, 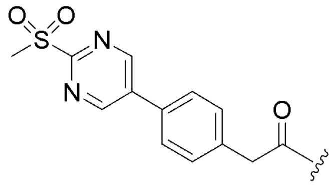, 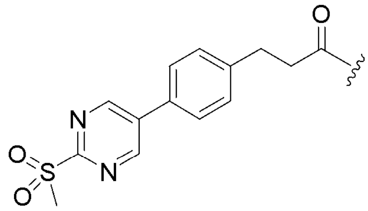,

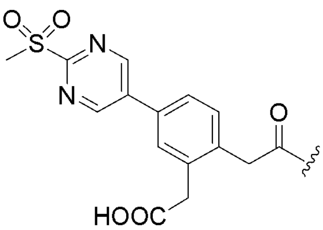, 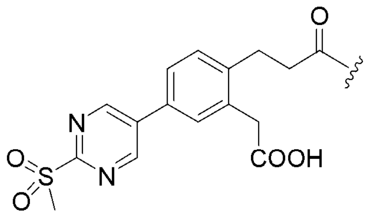, 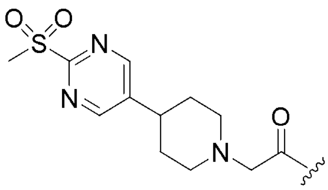,

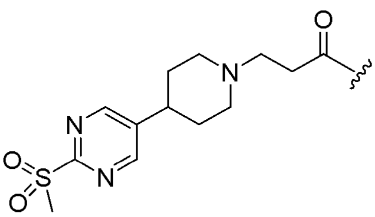, 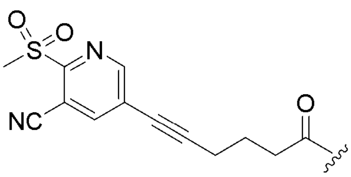, 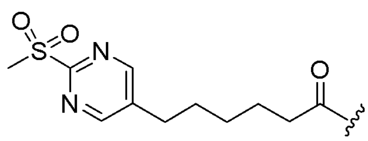,

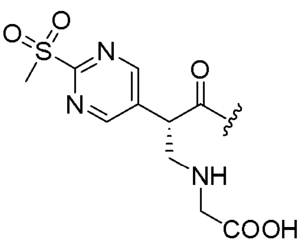, 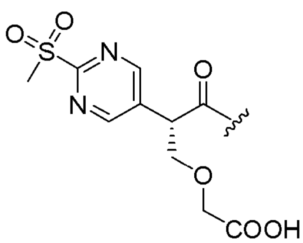, 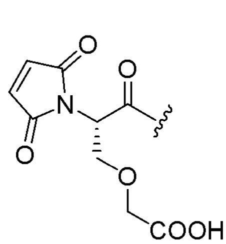,

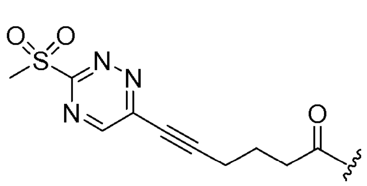,
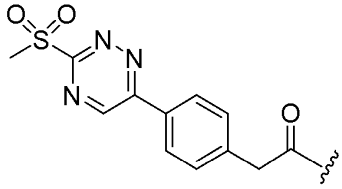,
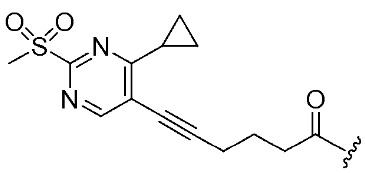,

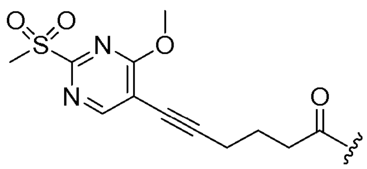,
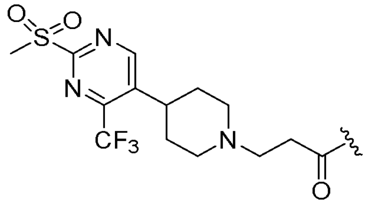,
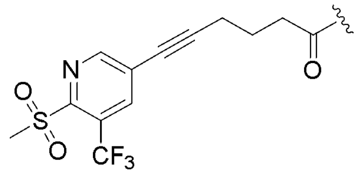,

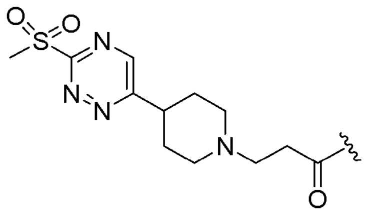,
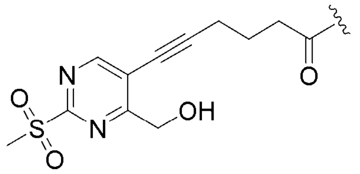,
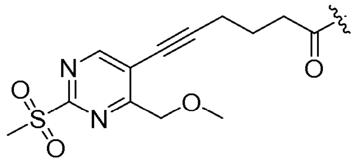,

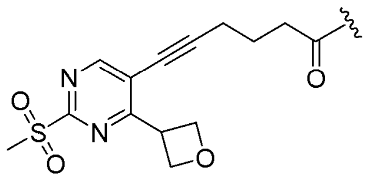,
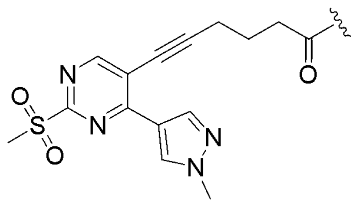,
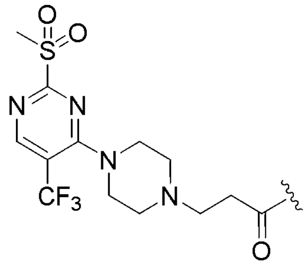,

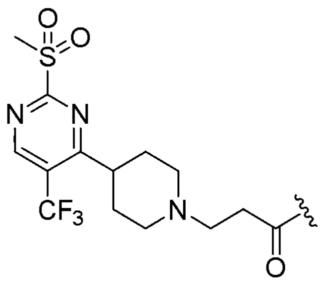,
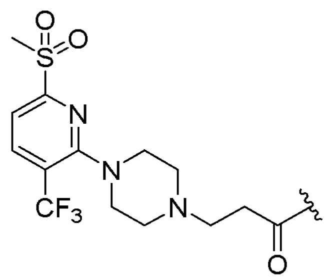,
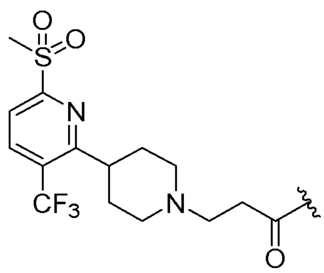,

or

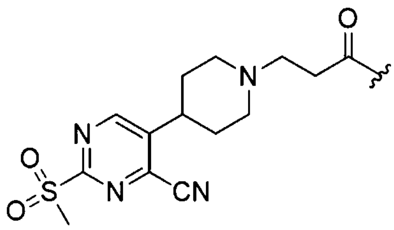;

and

preferably, M-$L_1$- $L_2$ is selected from the following groups:

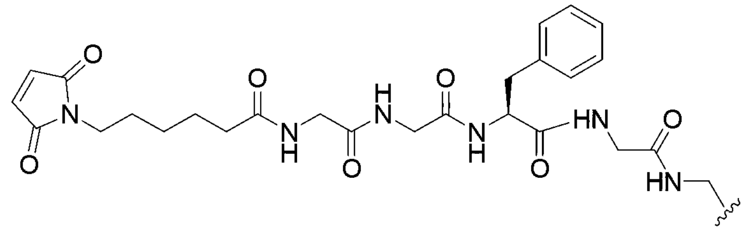

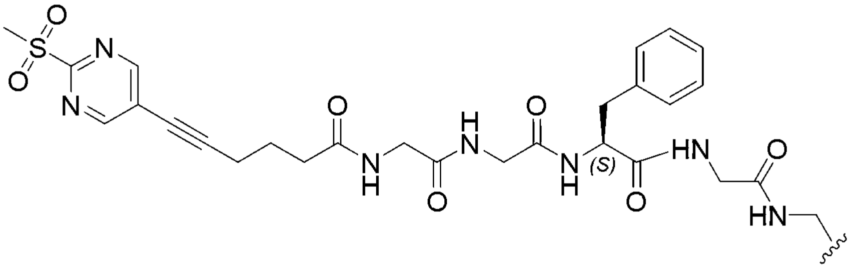

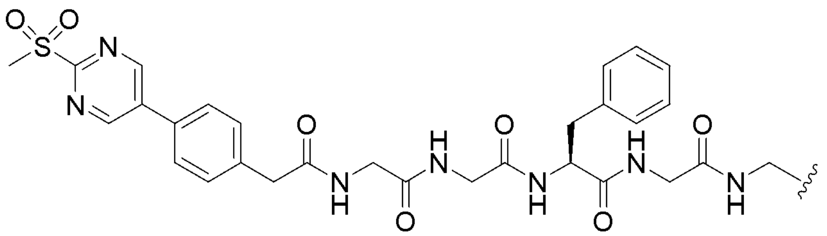

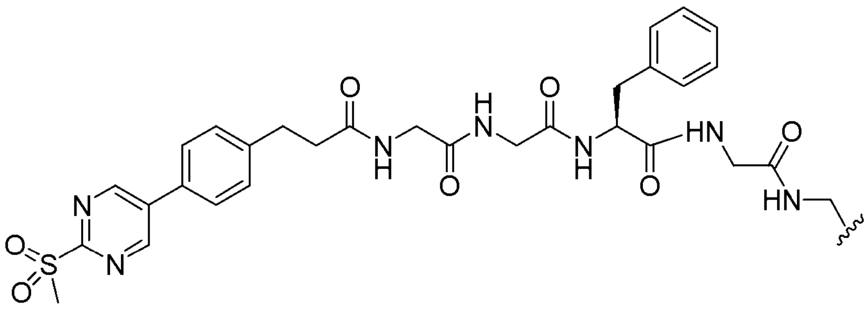

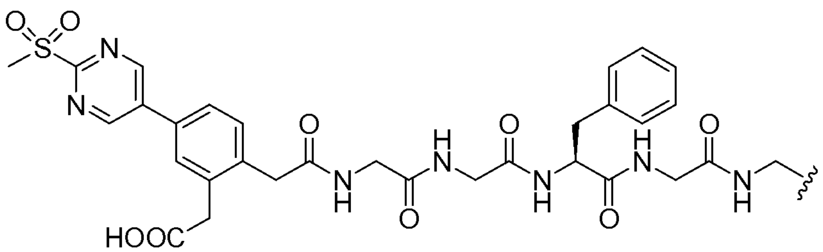

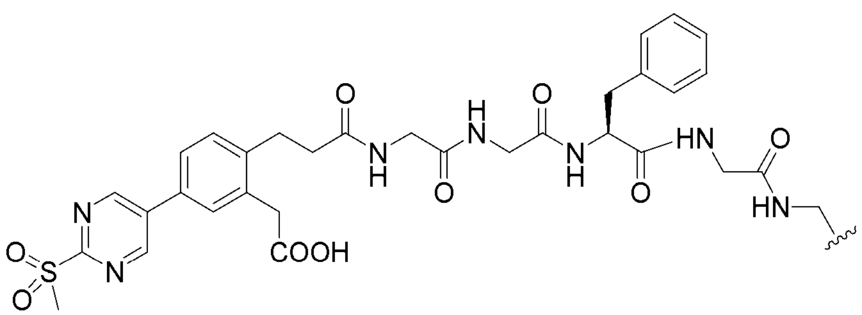

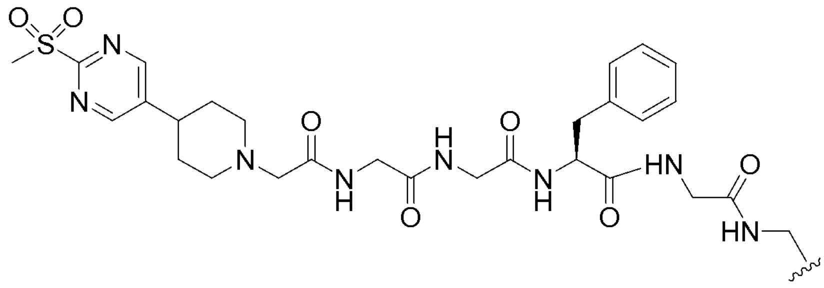

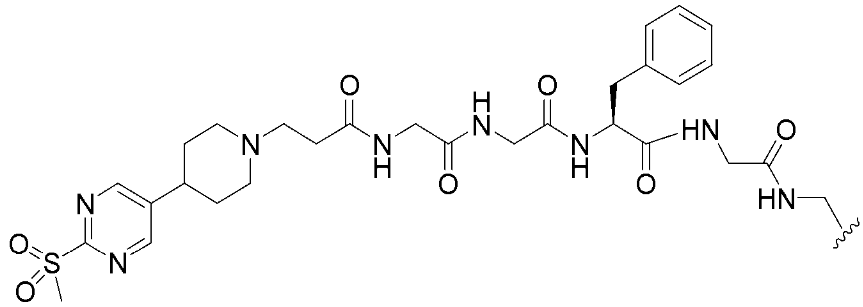

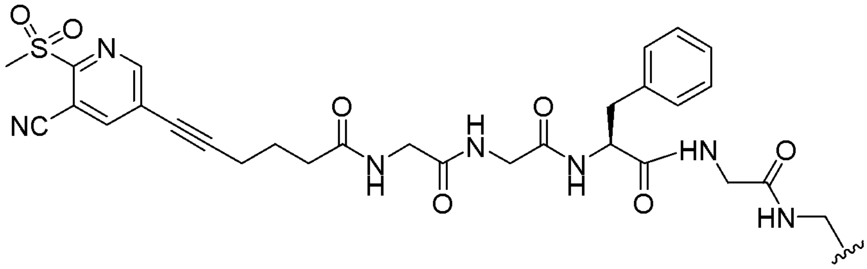

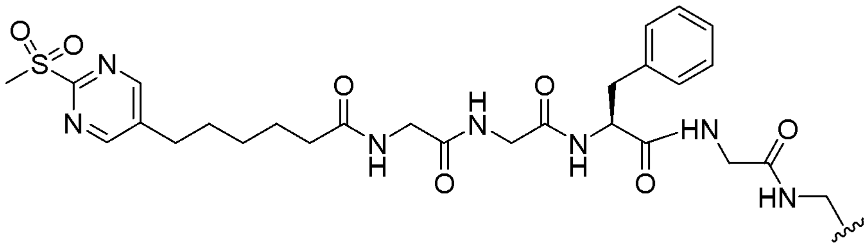

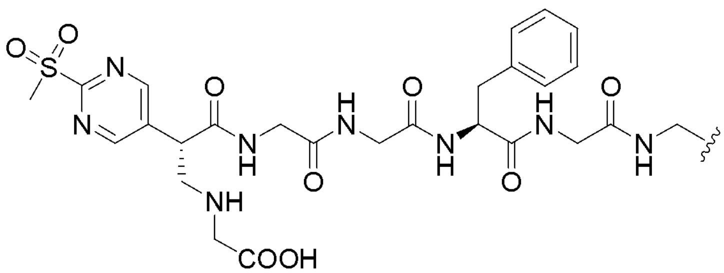

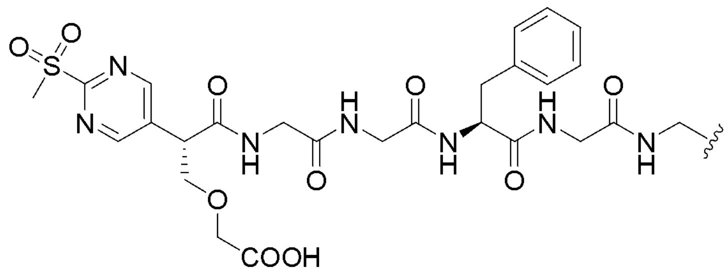

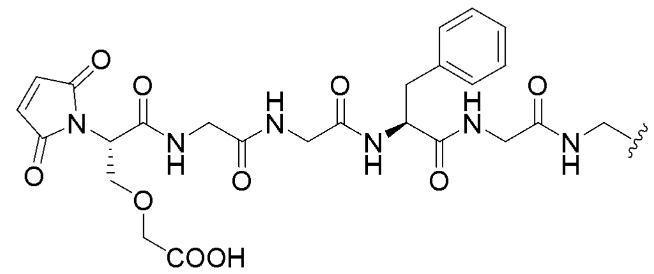

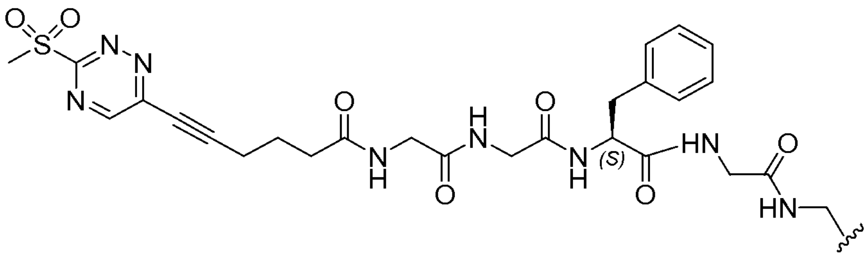

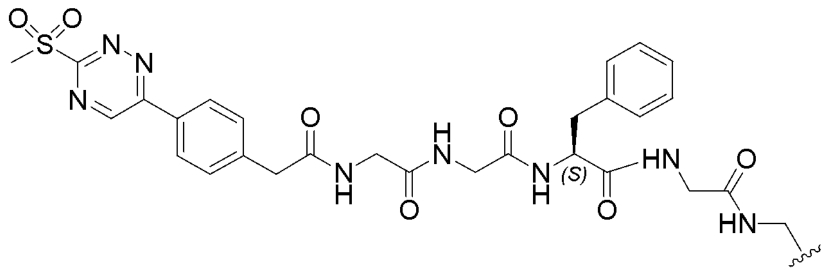

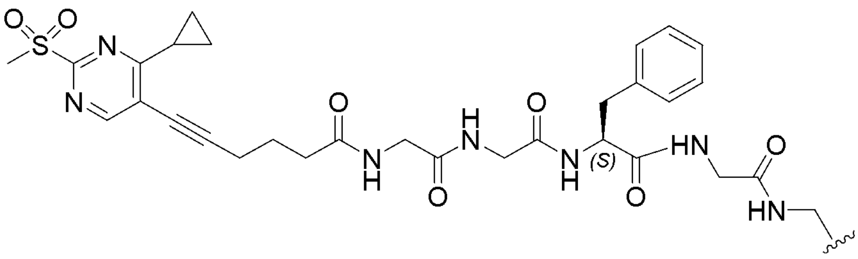

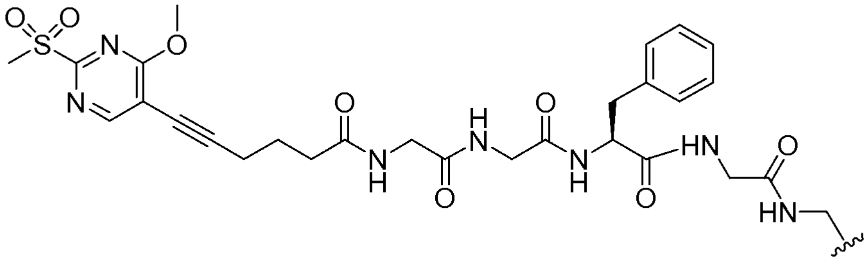

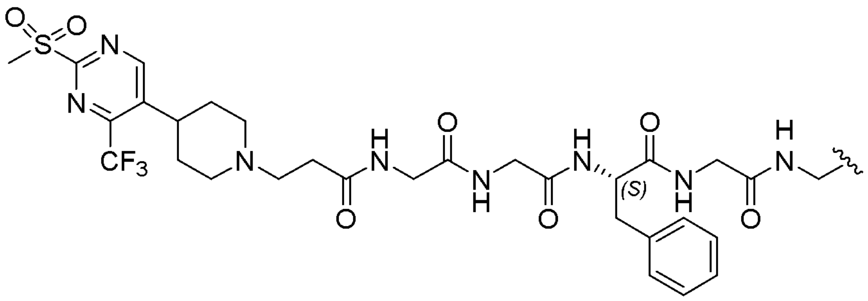

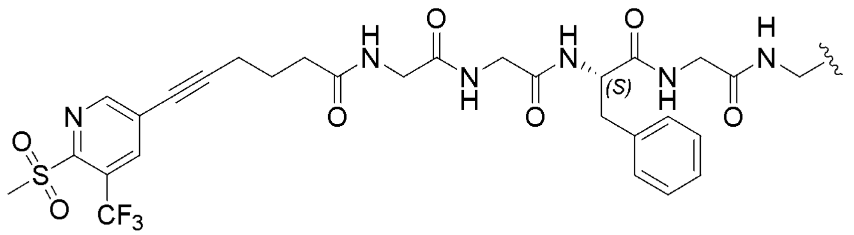

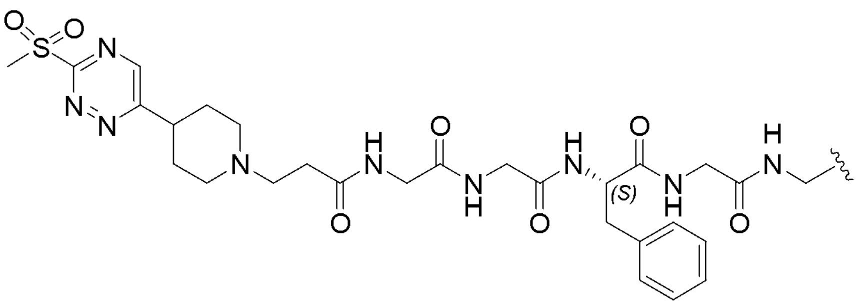

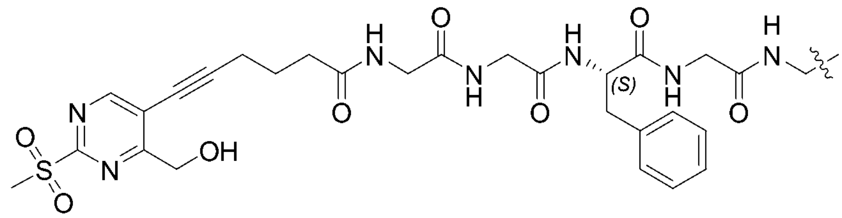

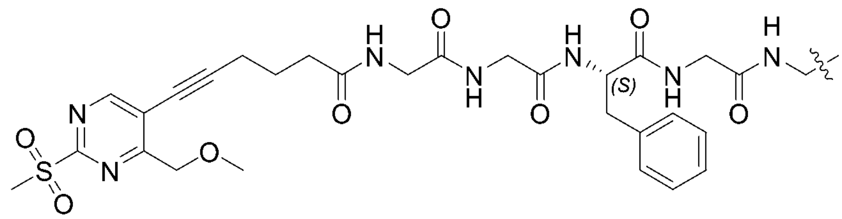

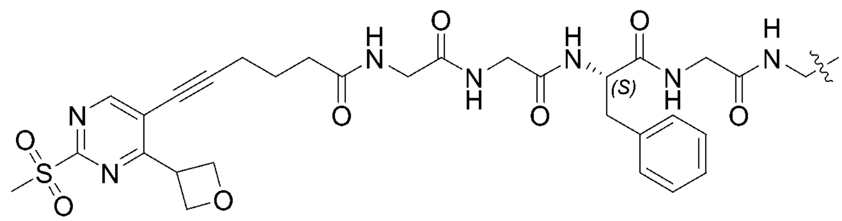

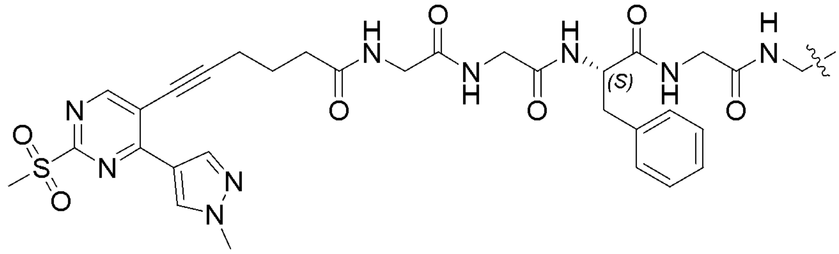

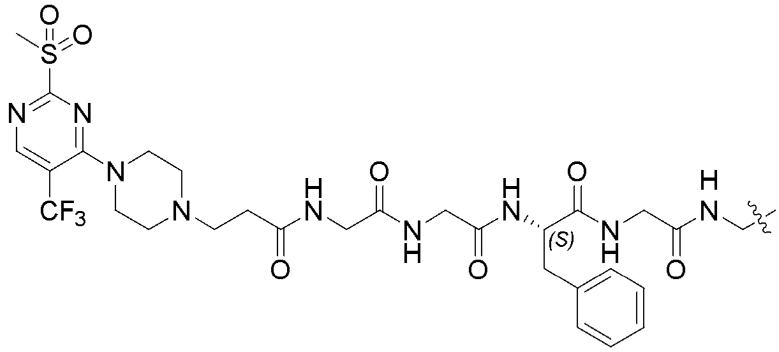

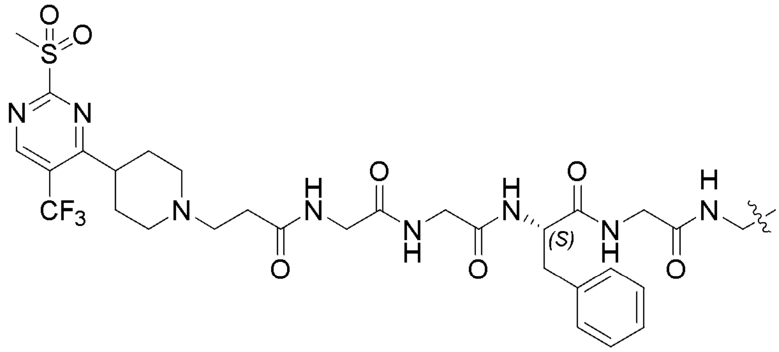

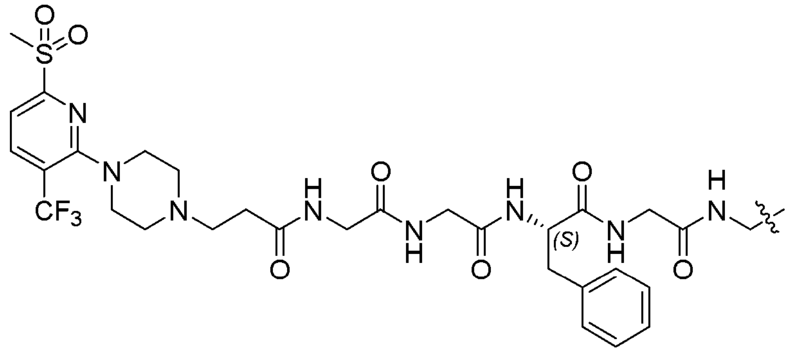

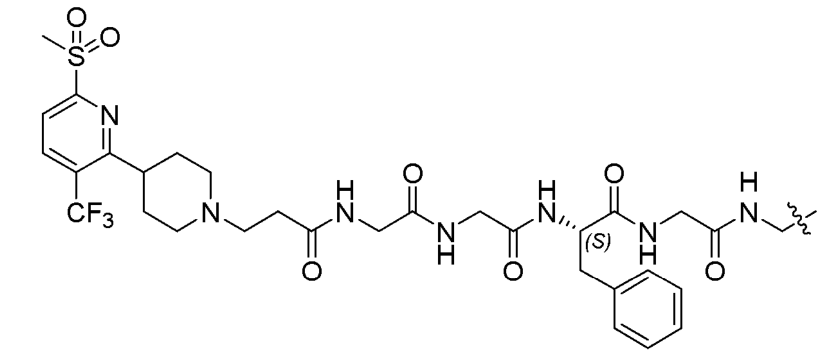

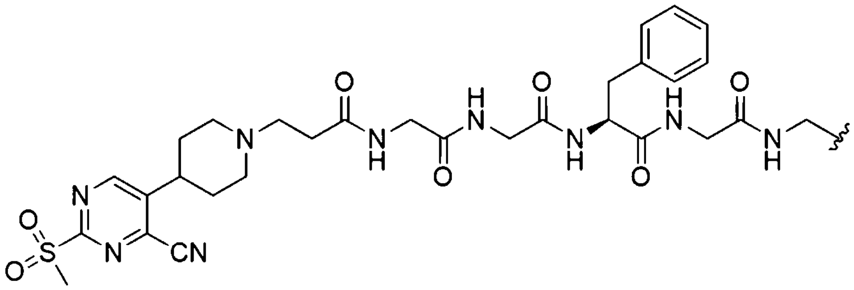

.

9. The compound according to claim 8, wherein the compound represented by formula V is selected from the following structures:

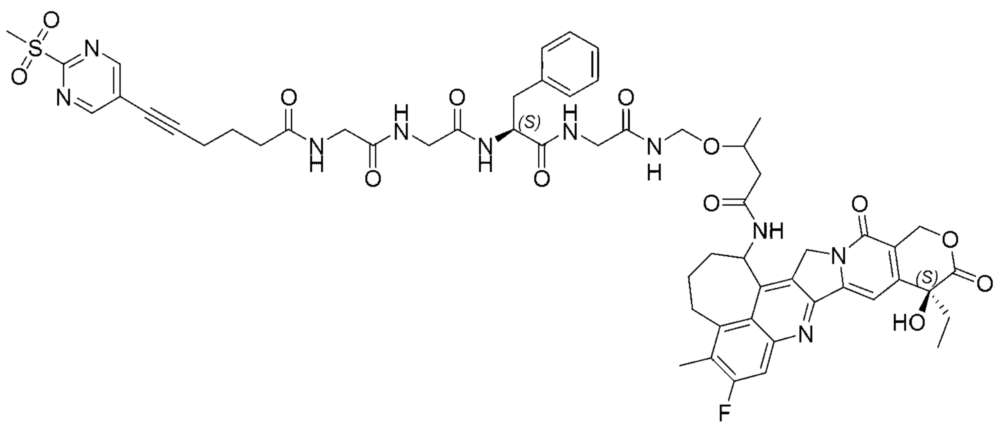

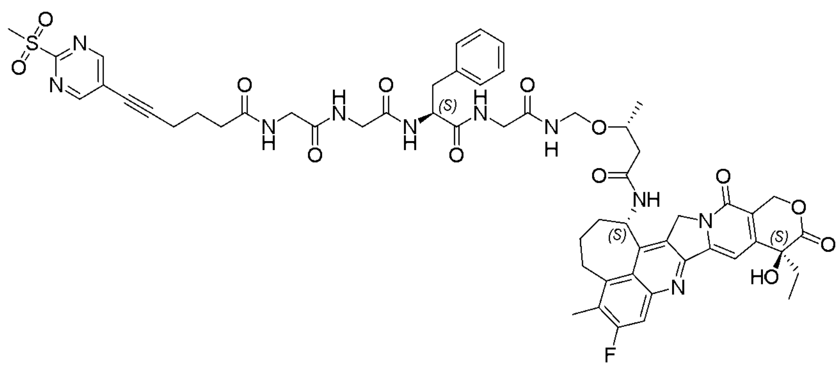

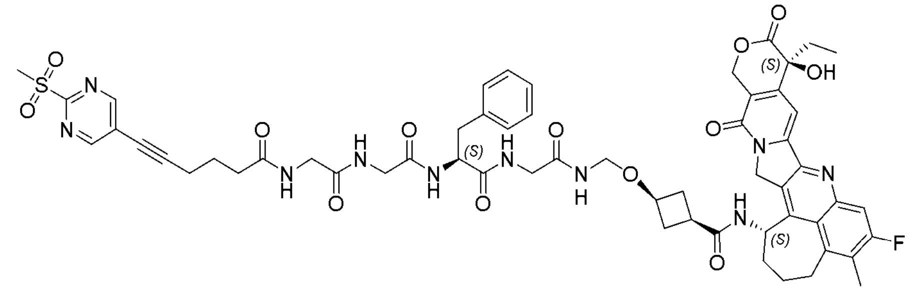

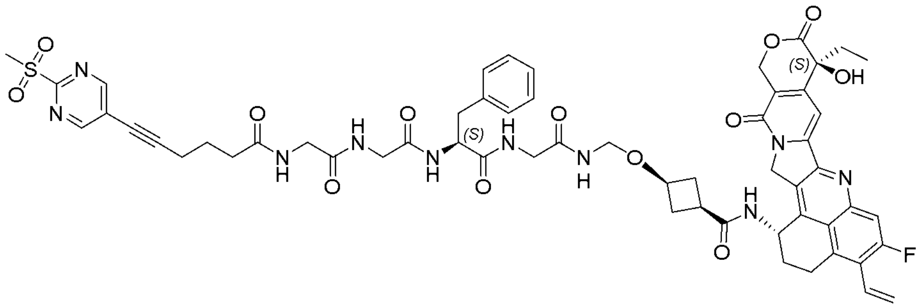

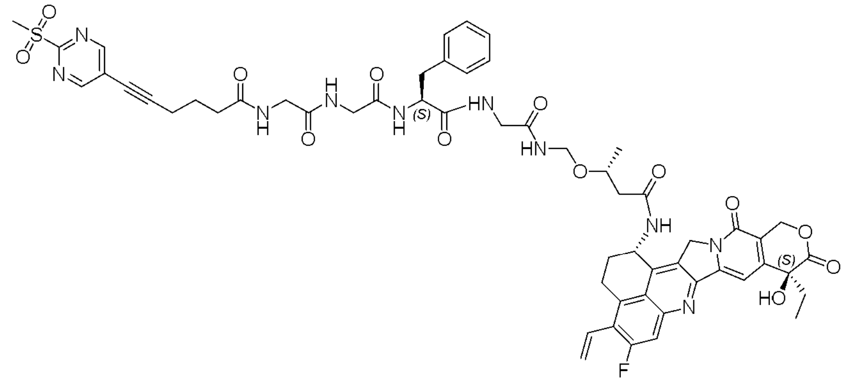

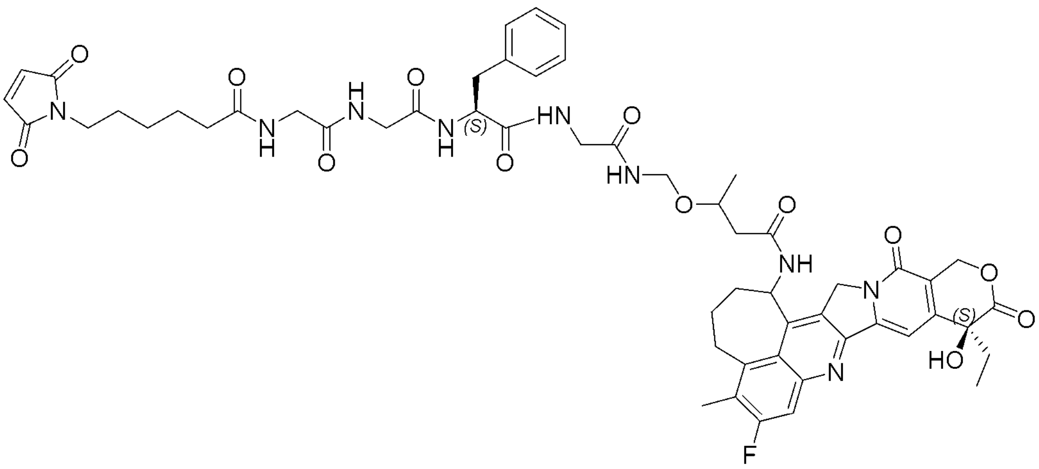

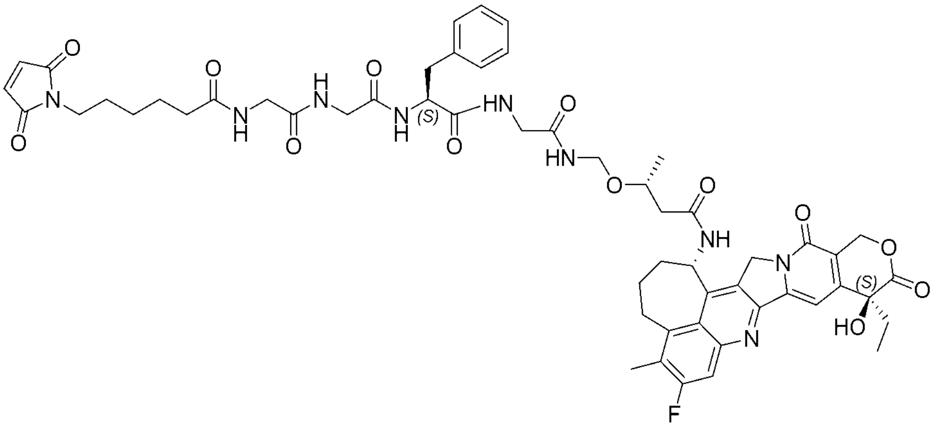

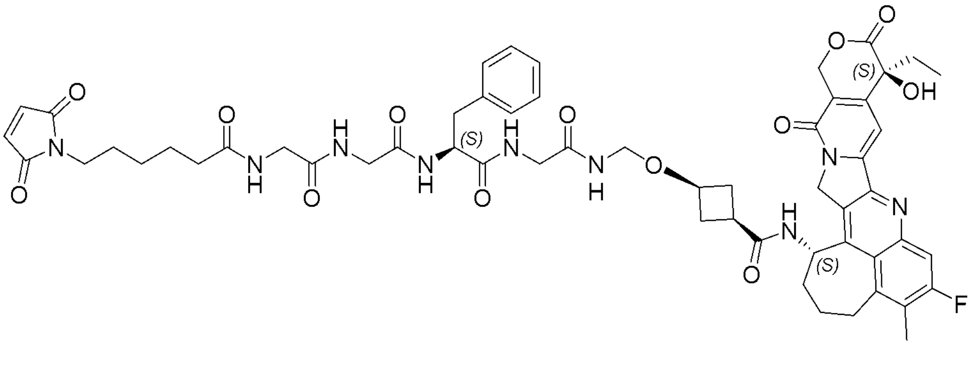

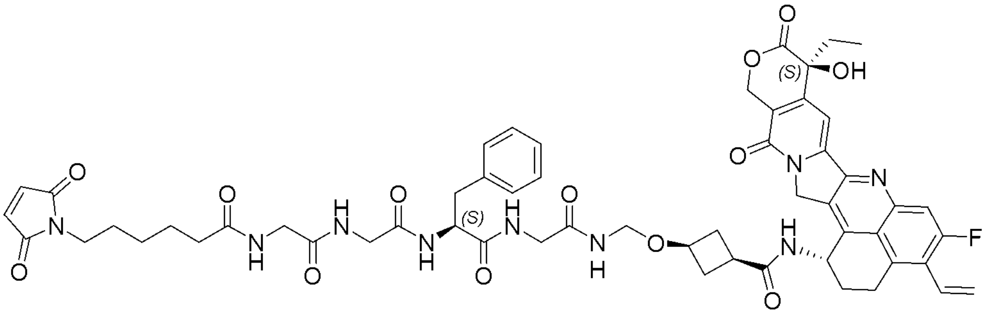

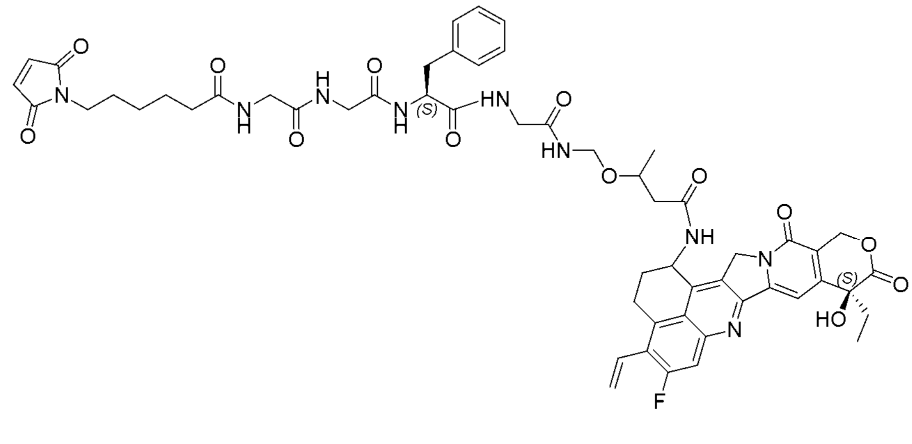

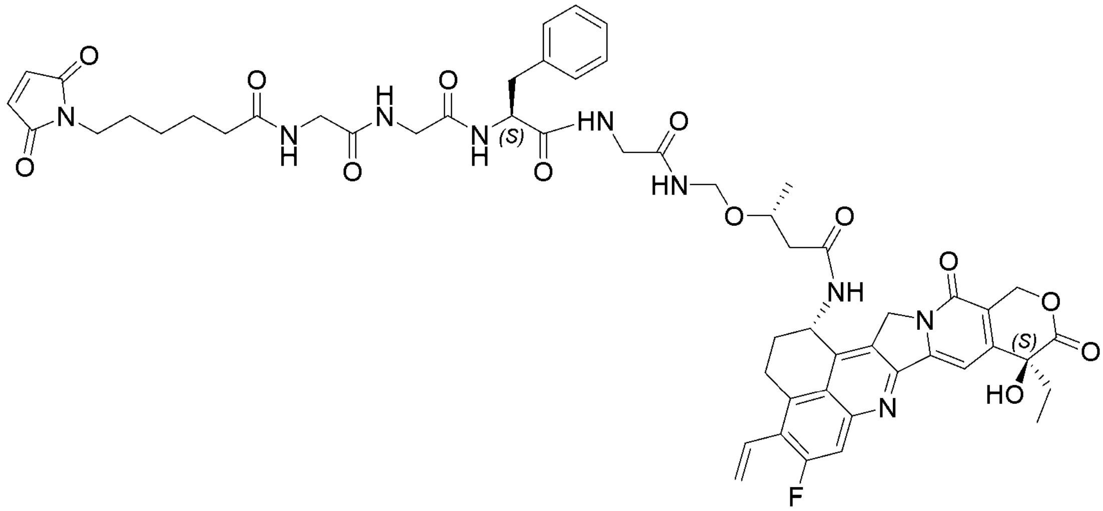

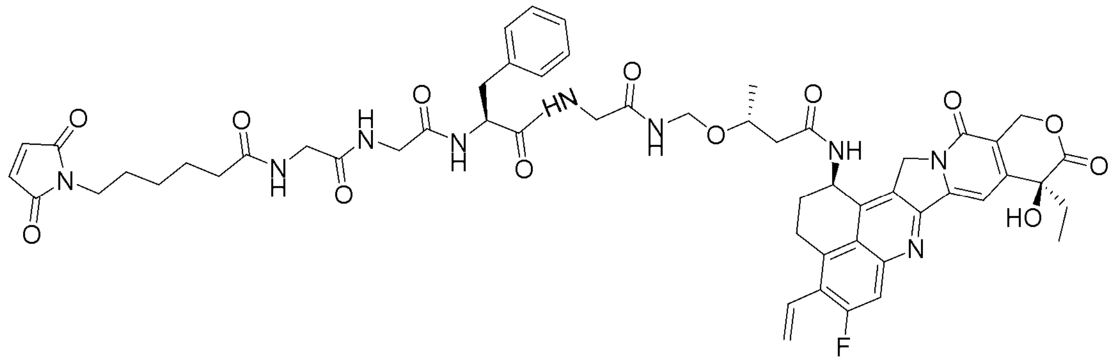

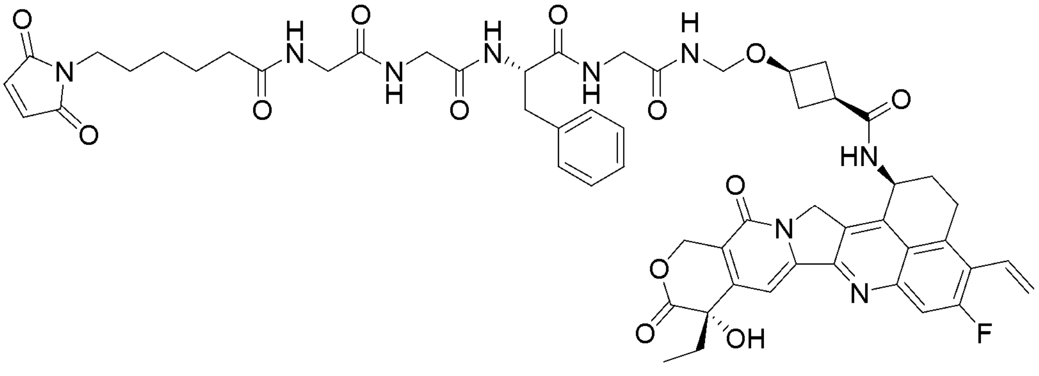

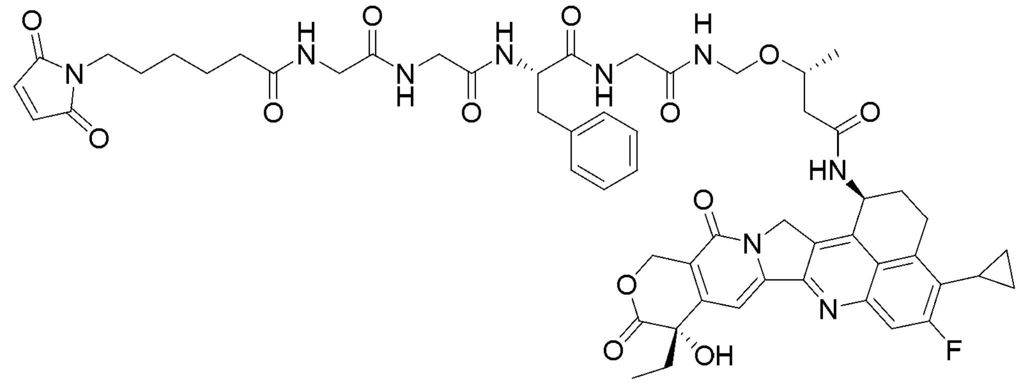

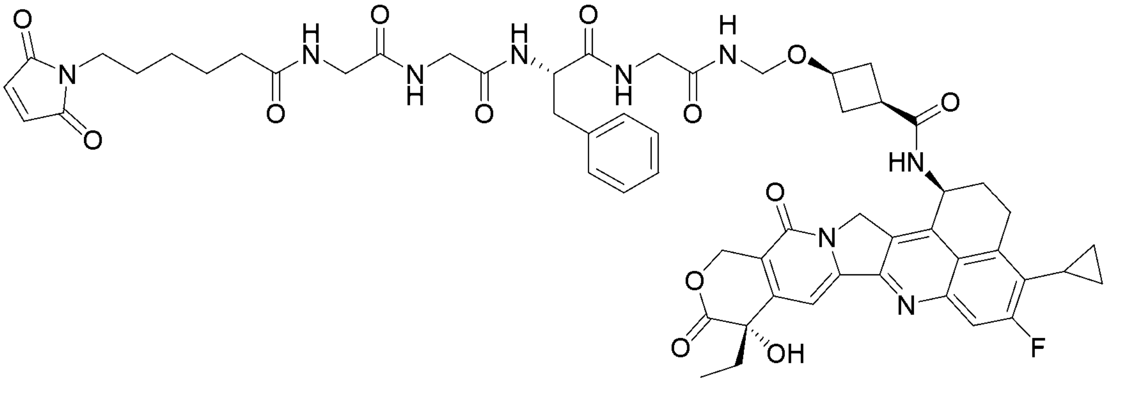

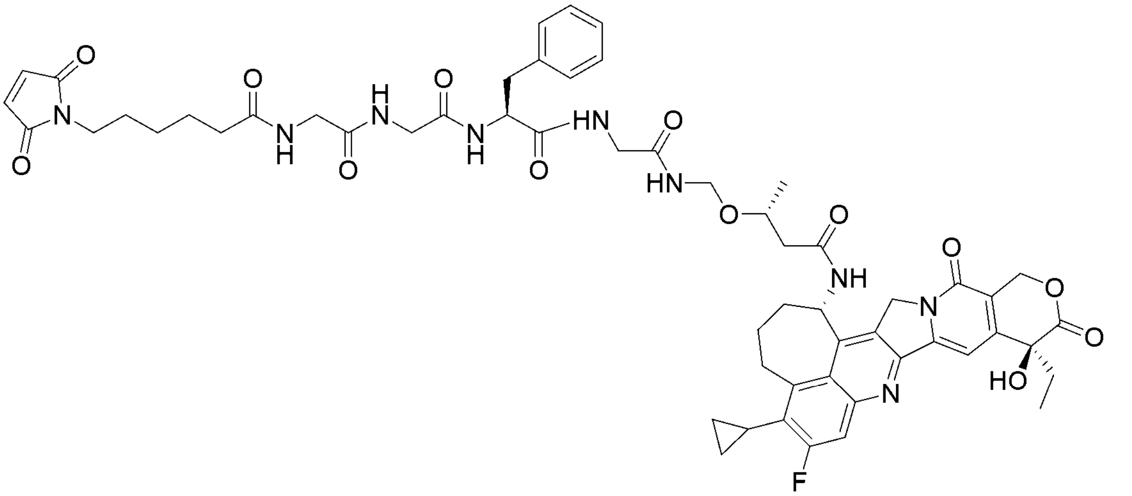

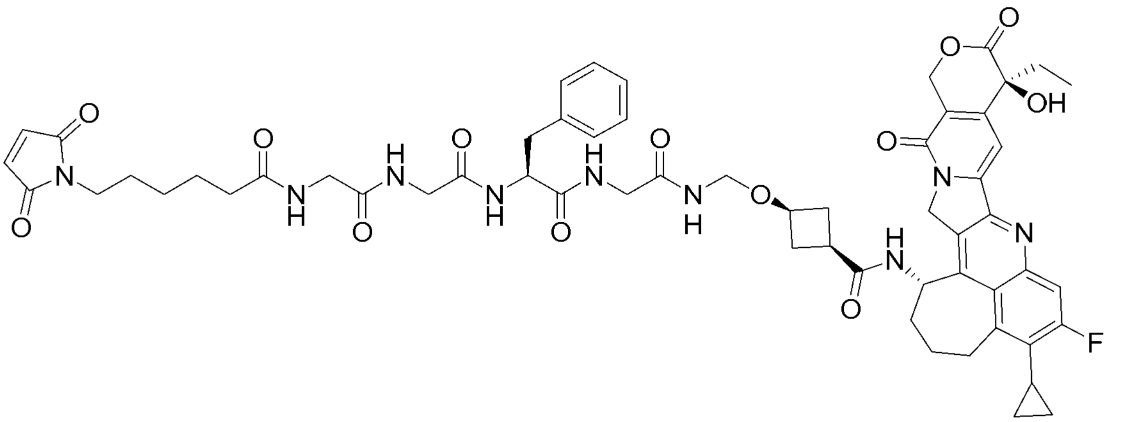

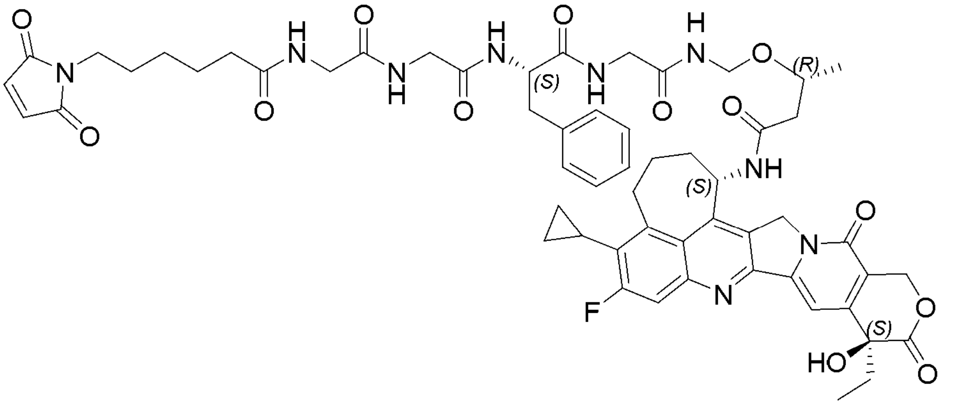

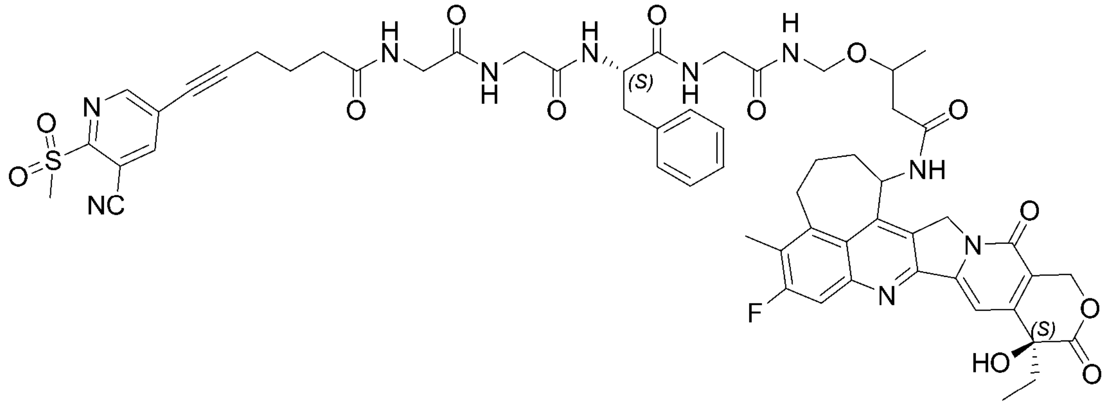

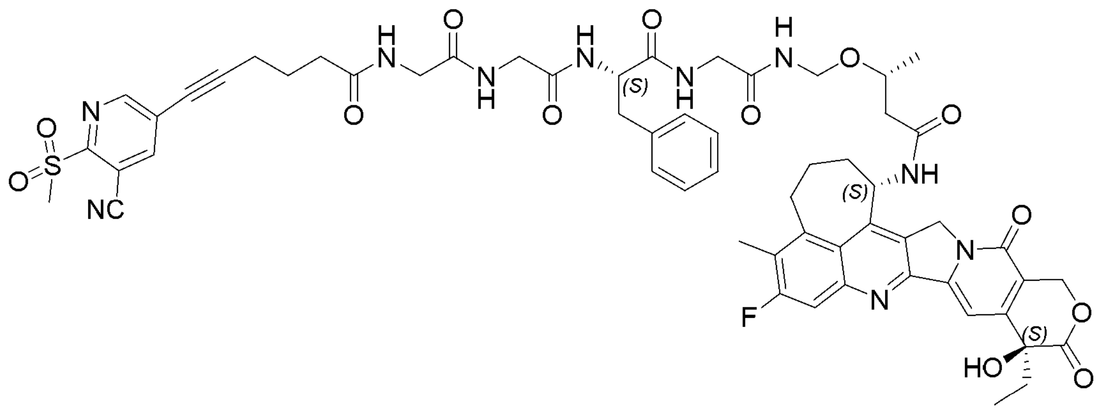

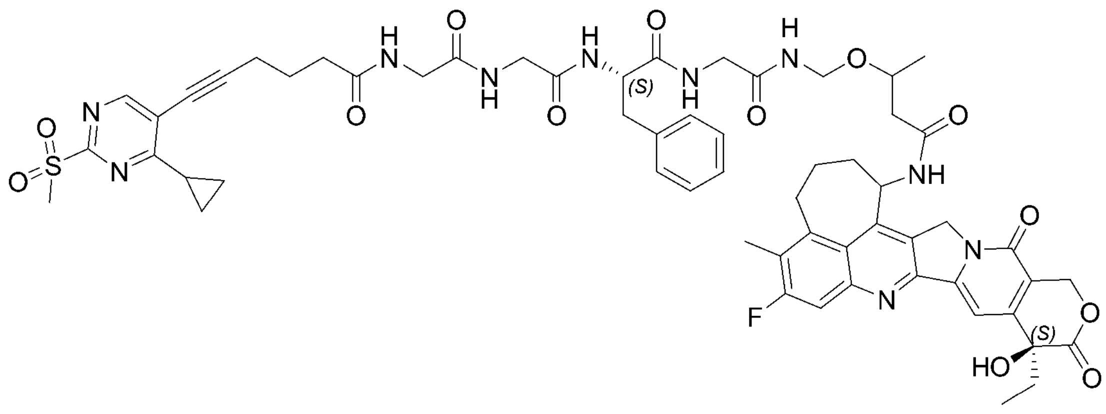

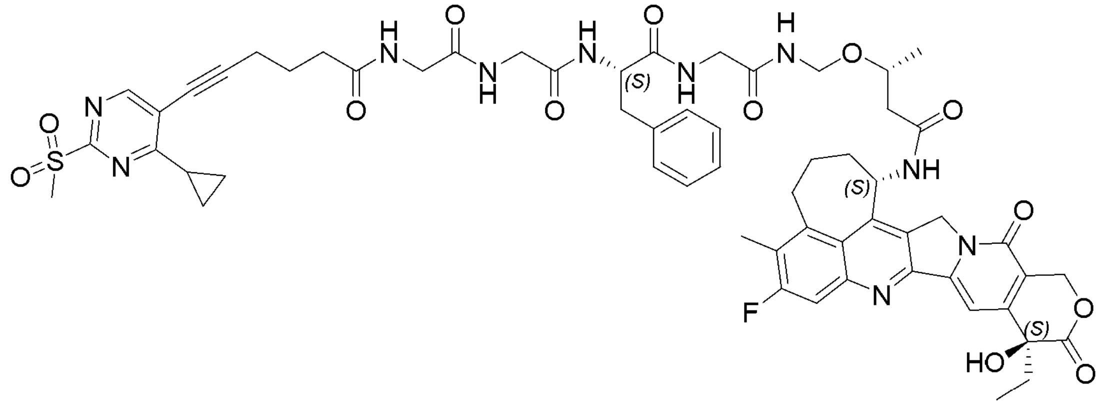

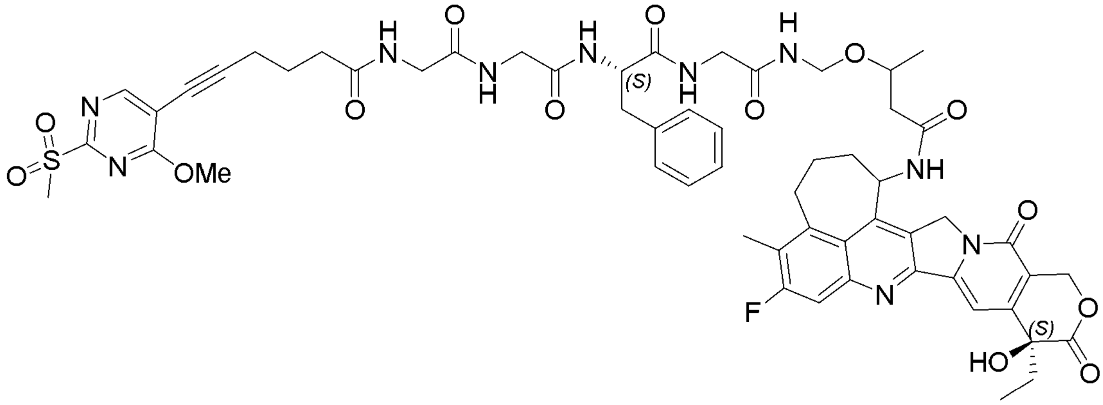

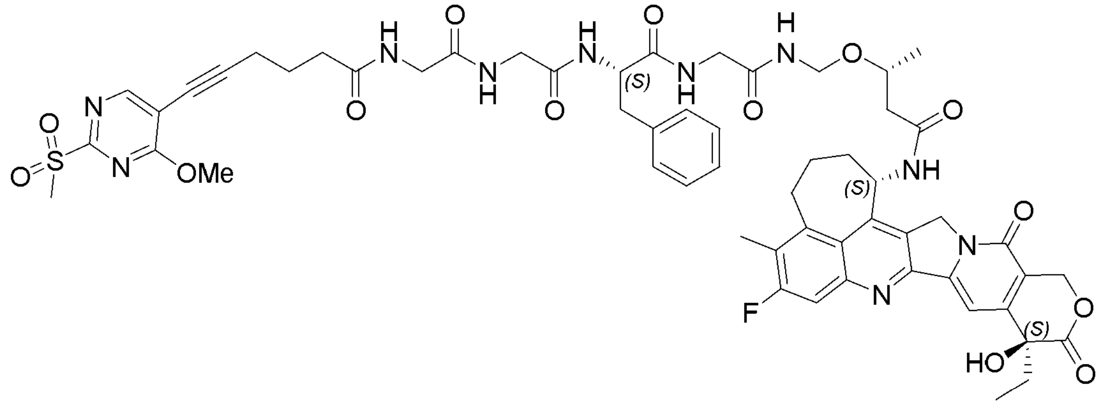

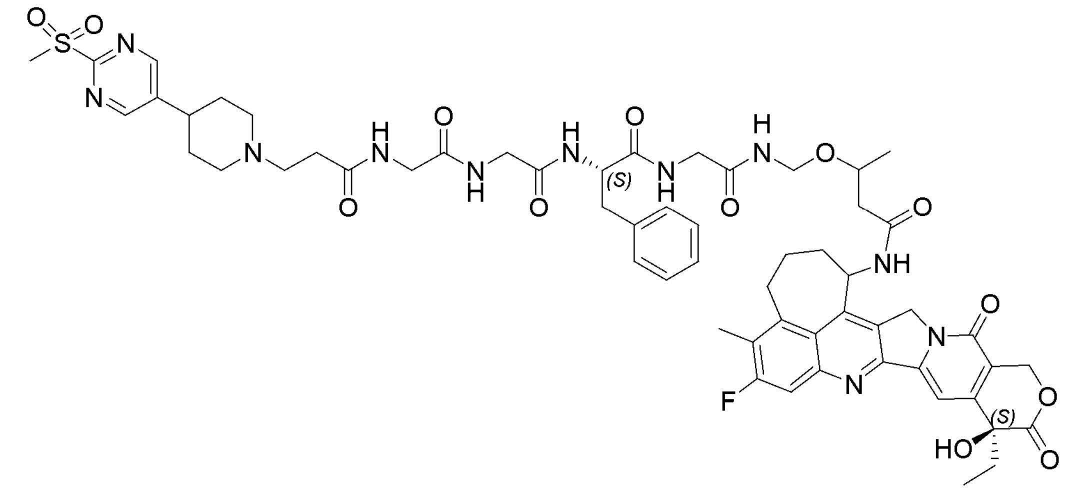

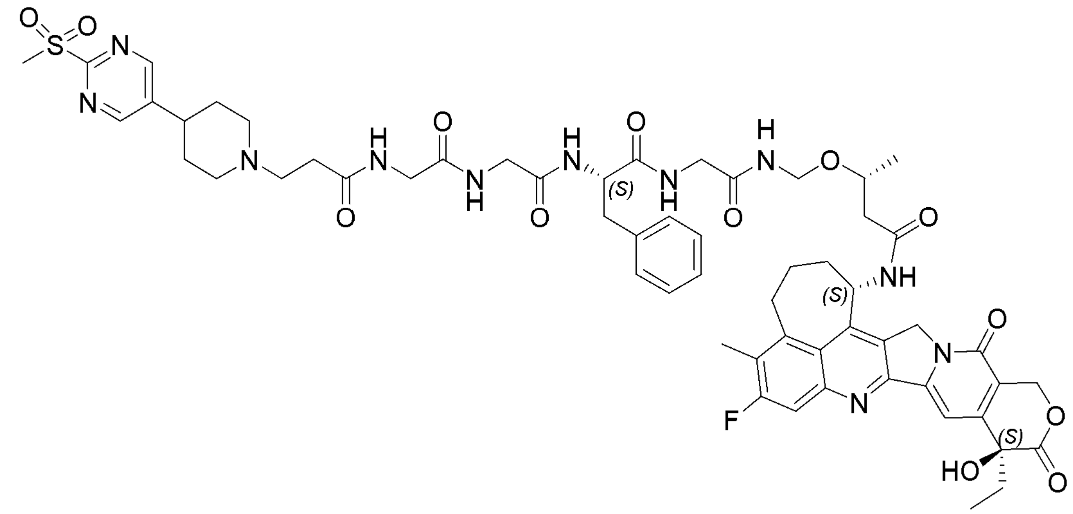

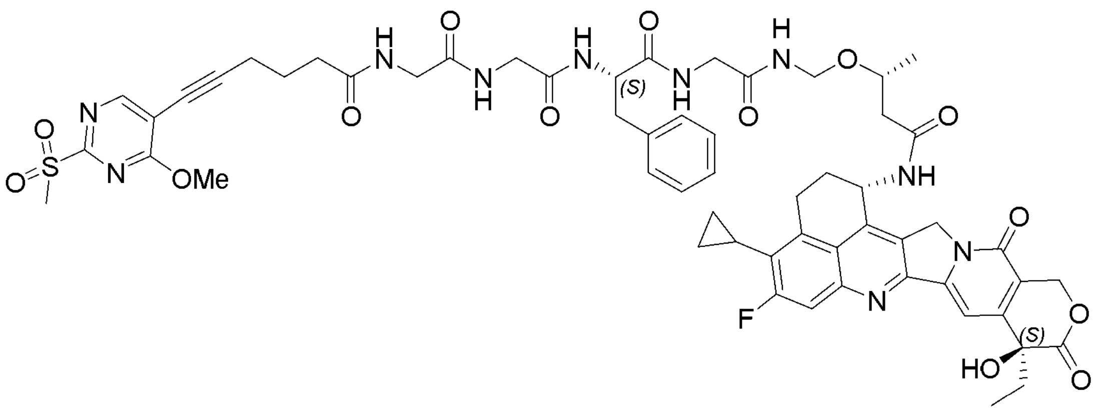

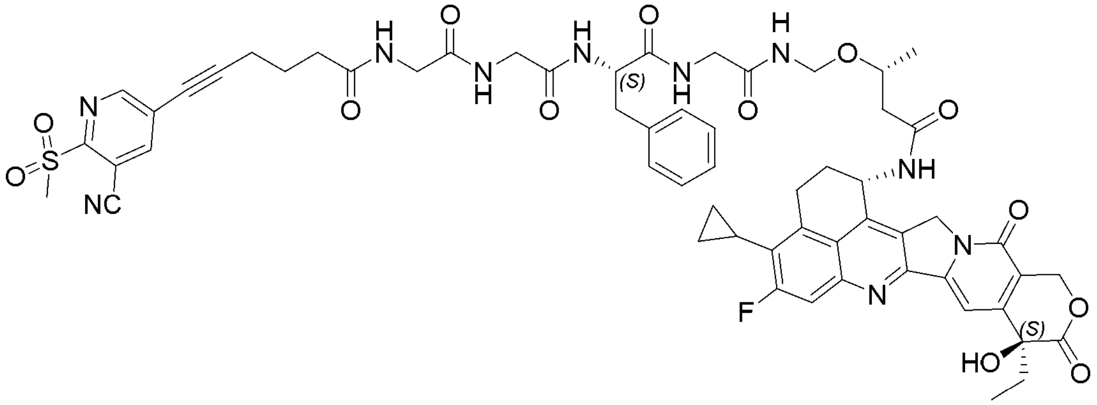

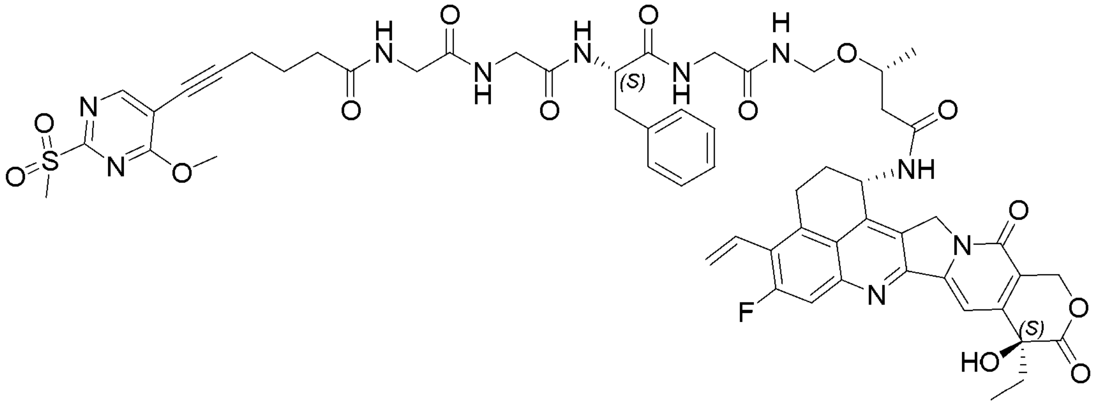

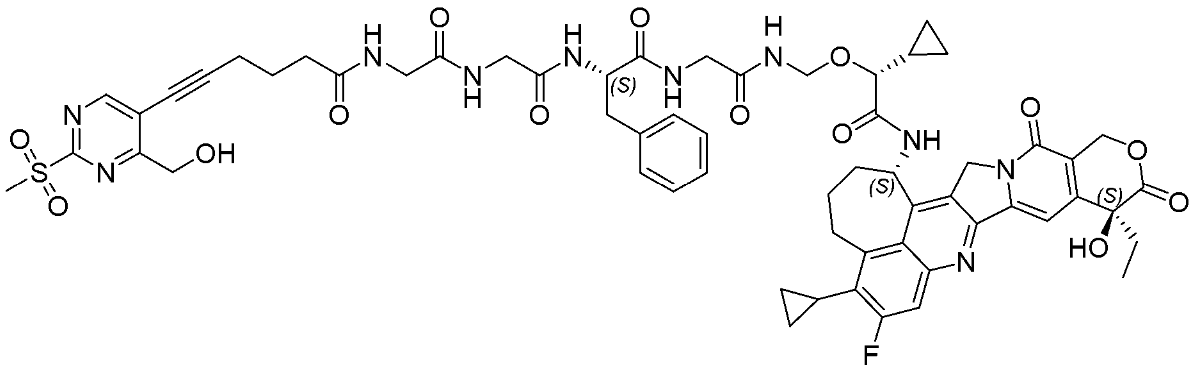

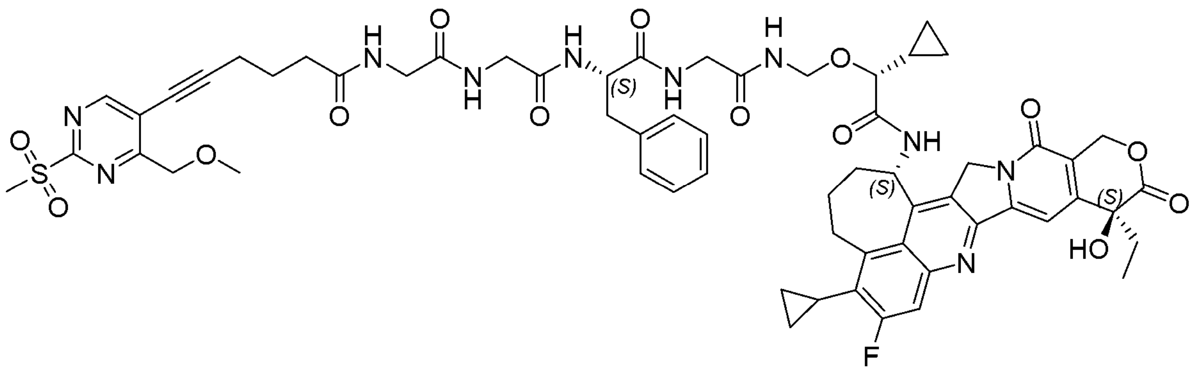

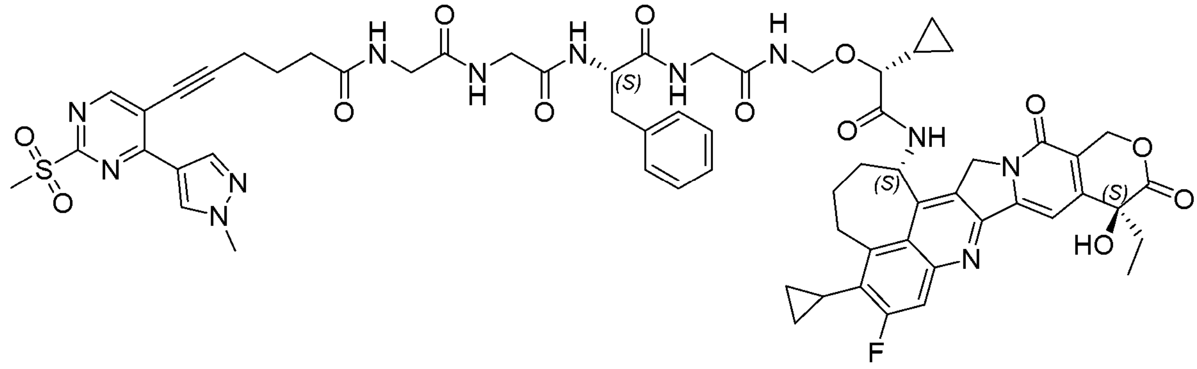

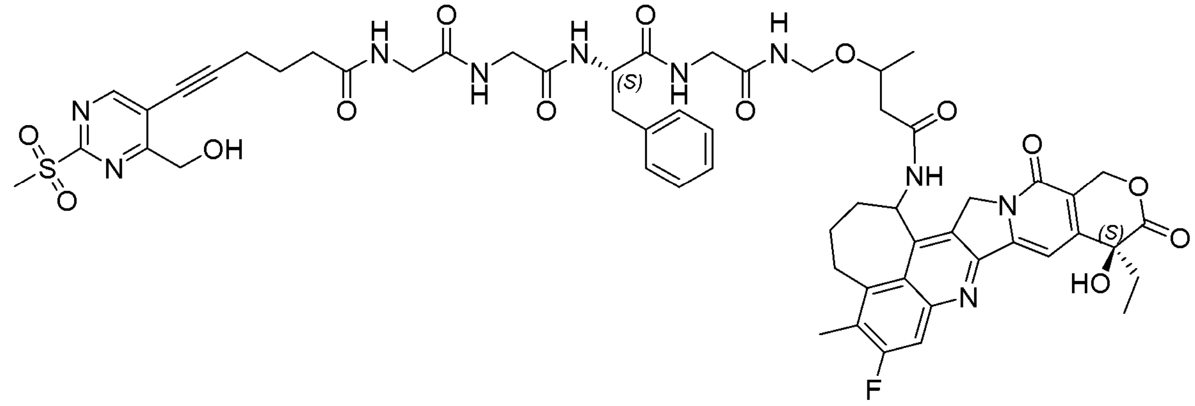

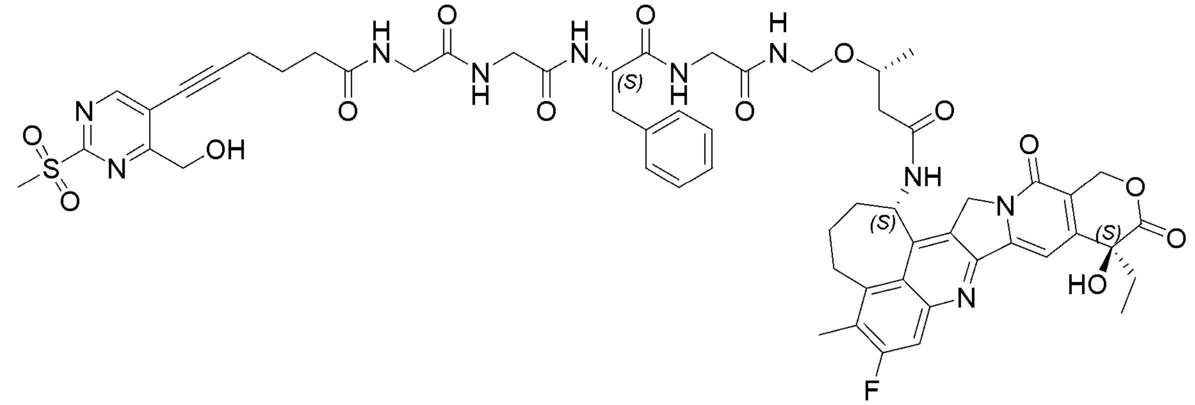

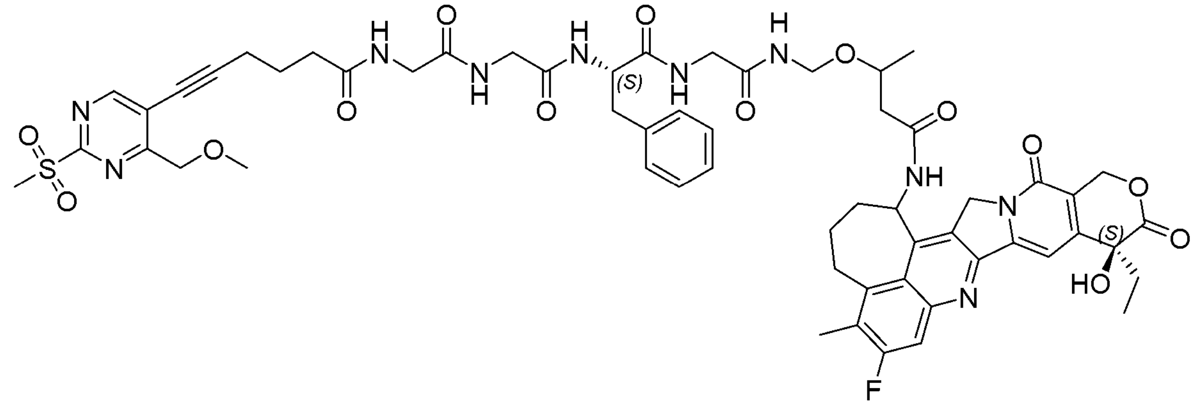

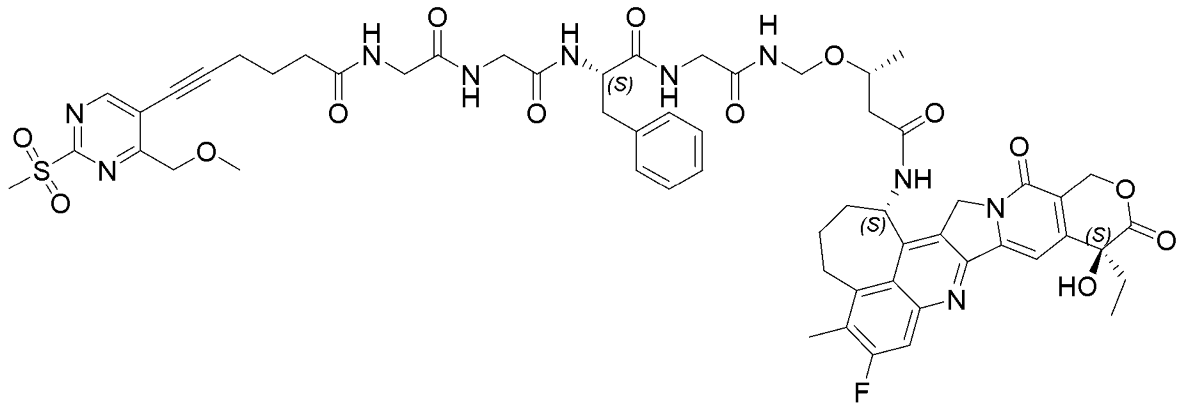

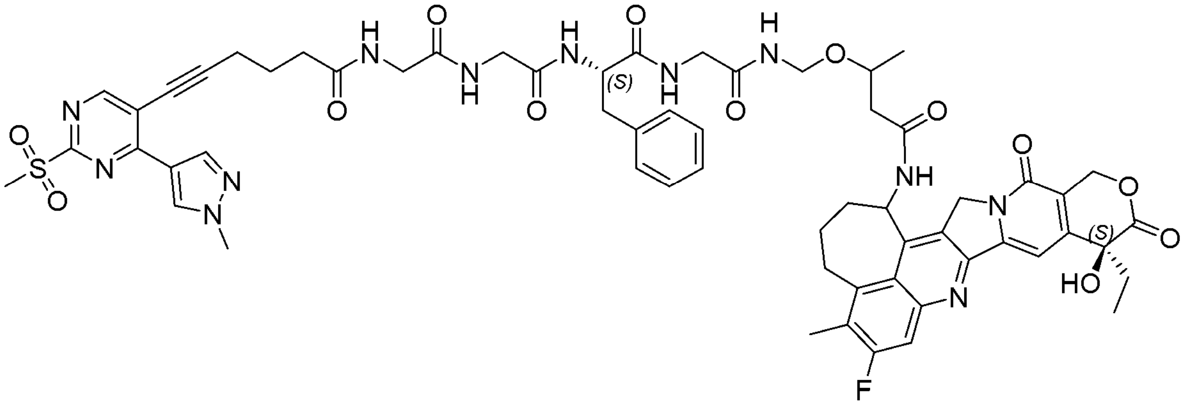

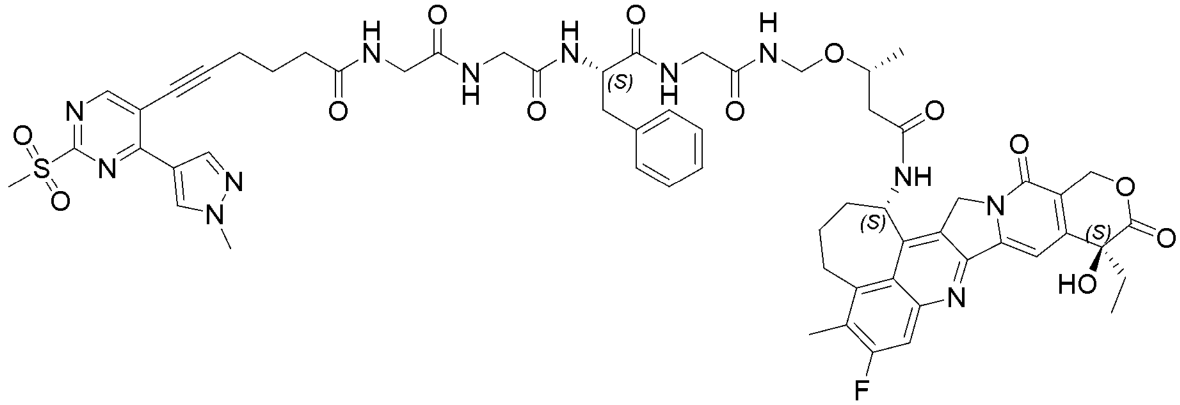

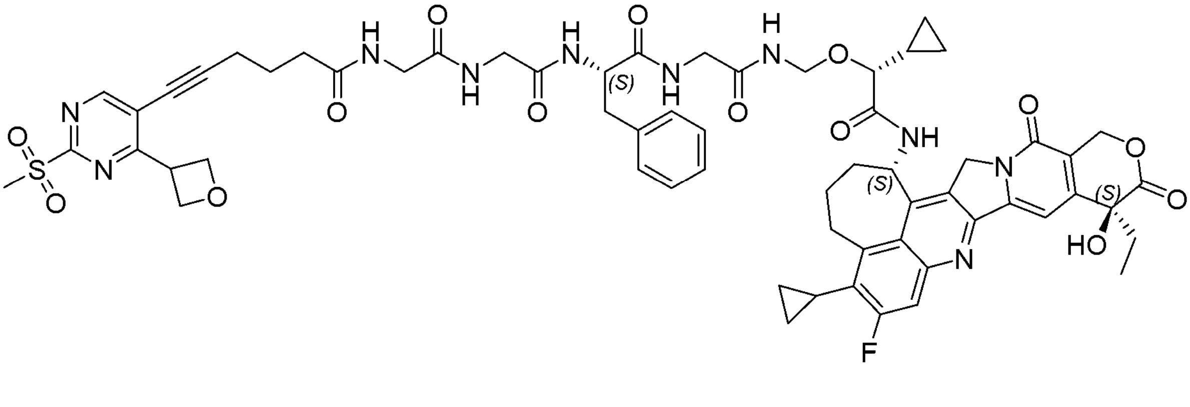

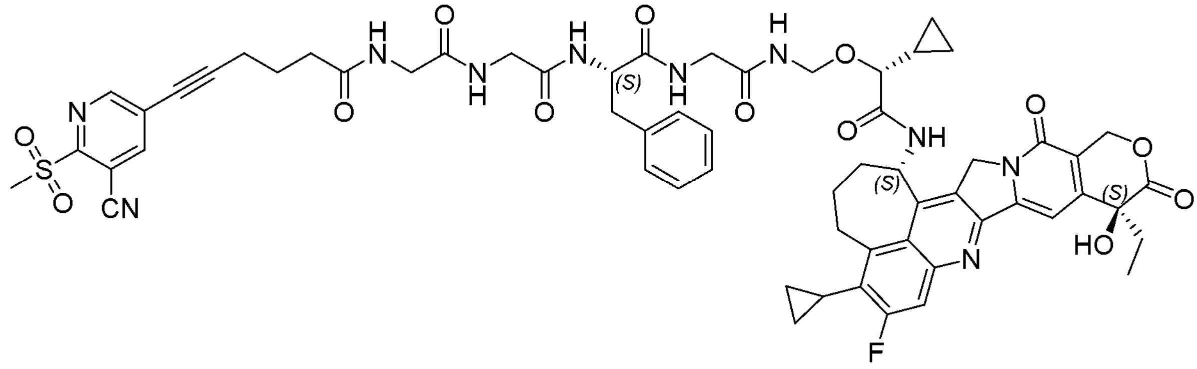

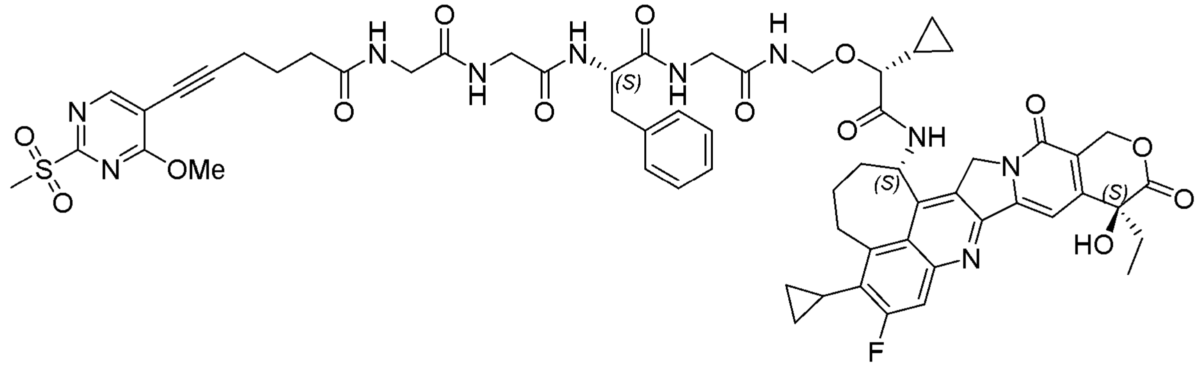

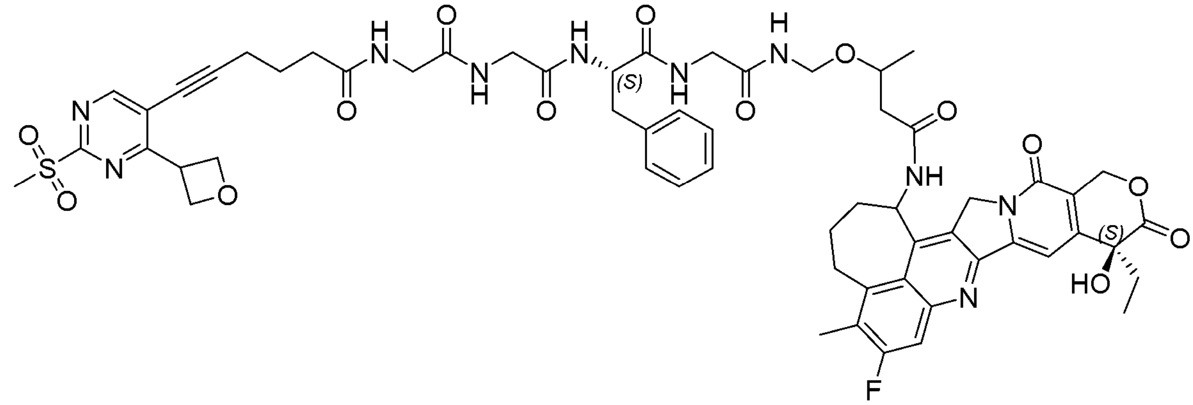

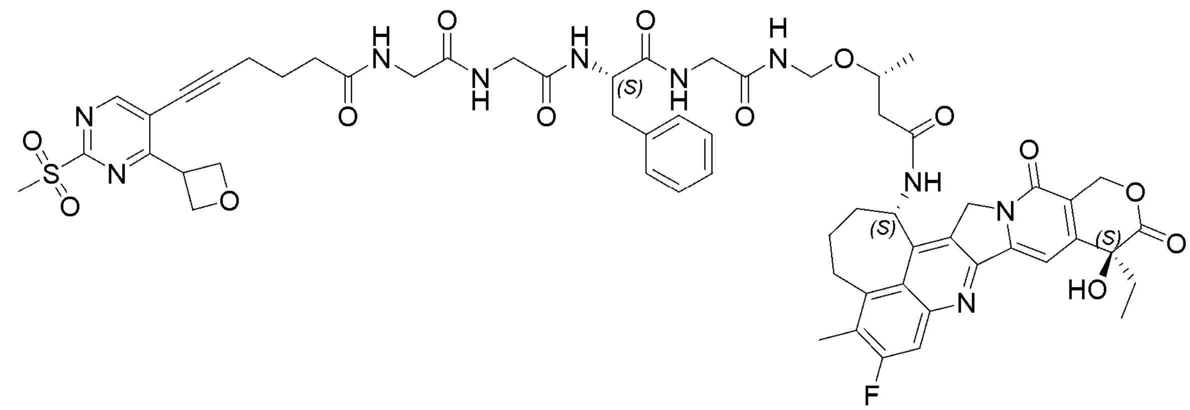

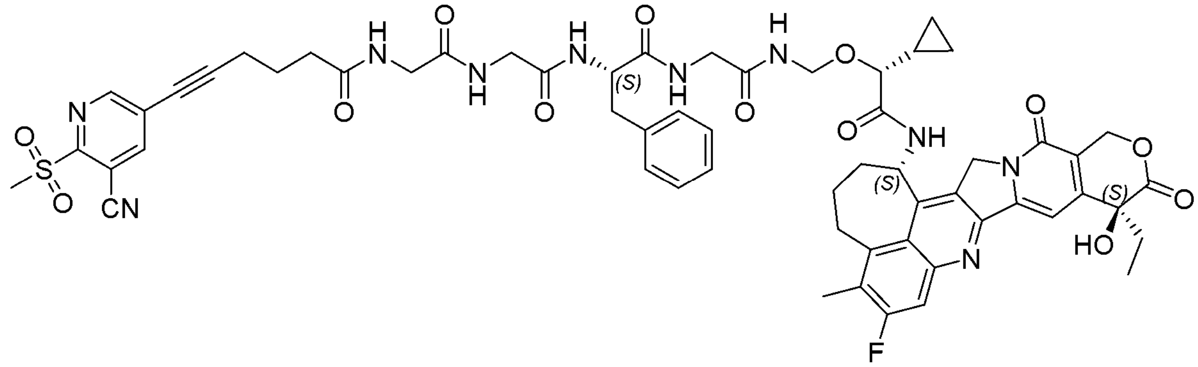

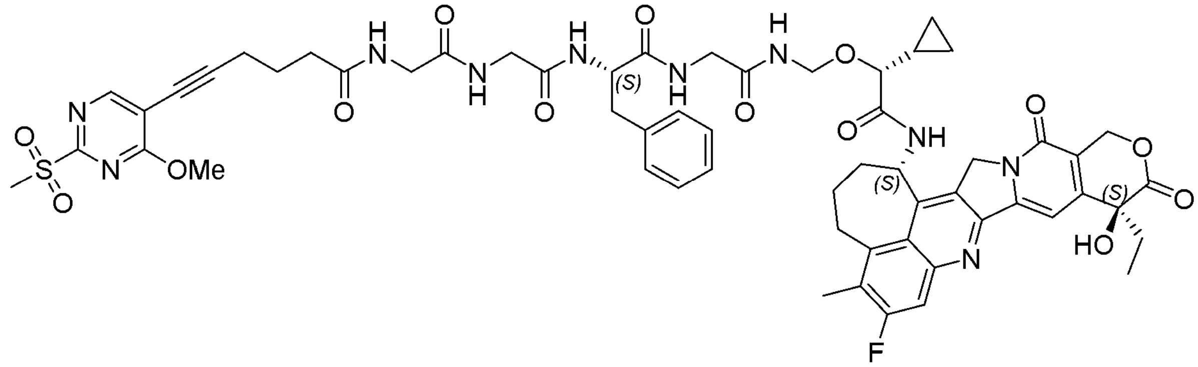

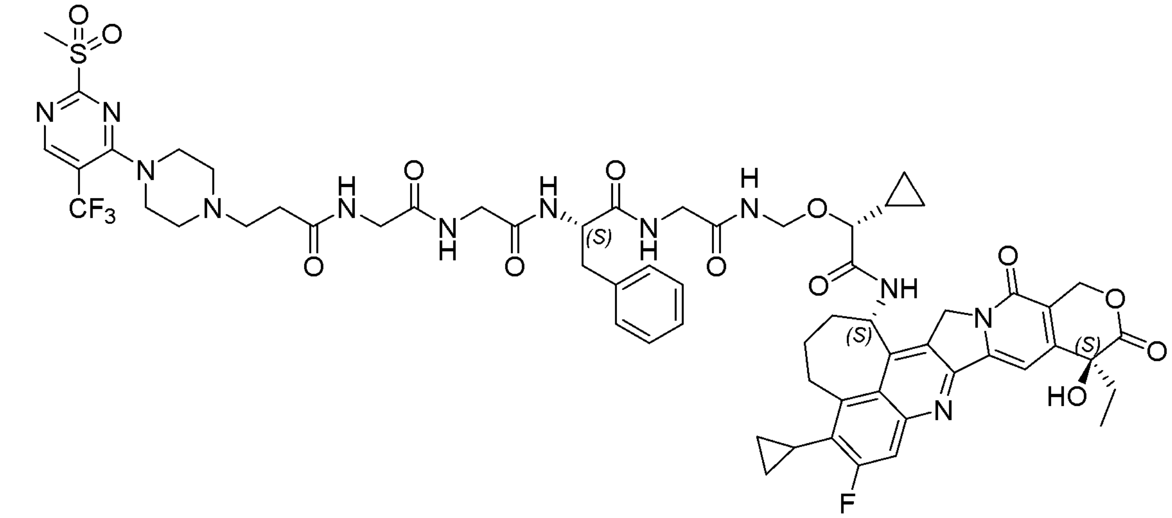

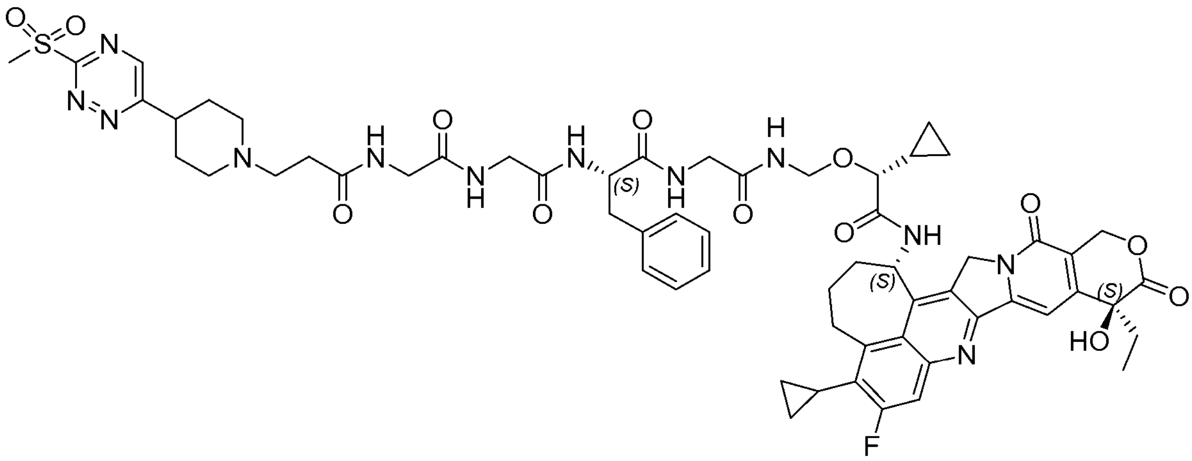

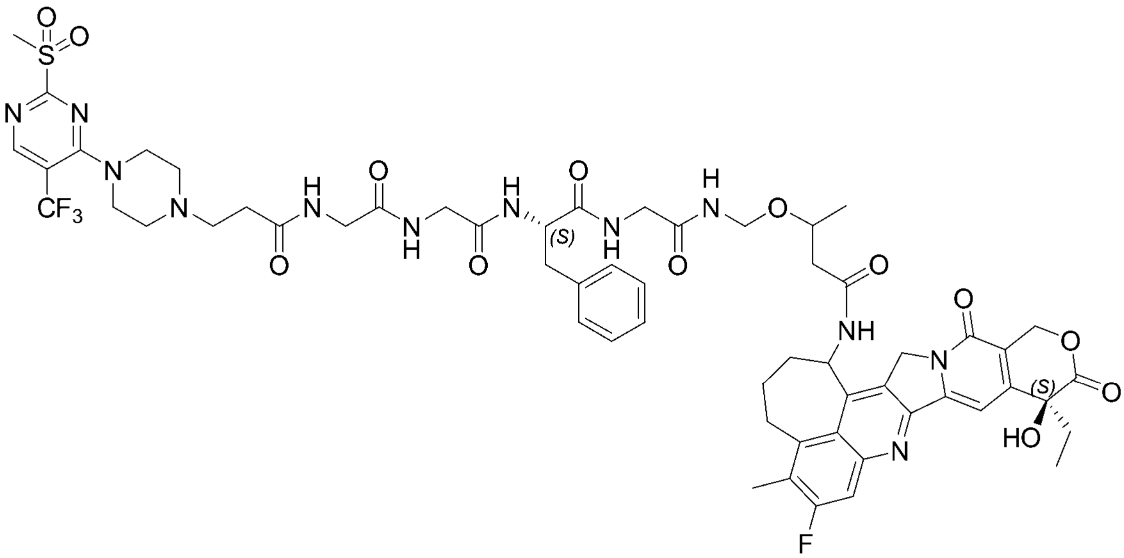

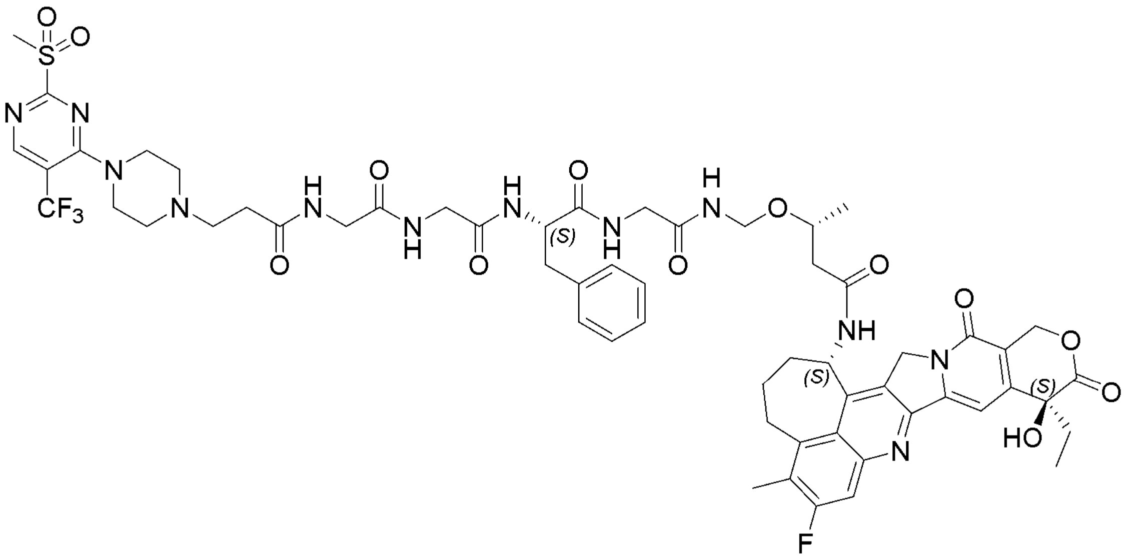

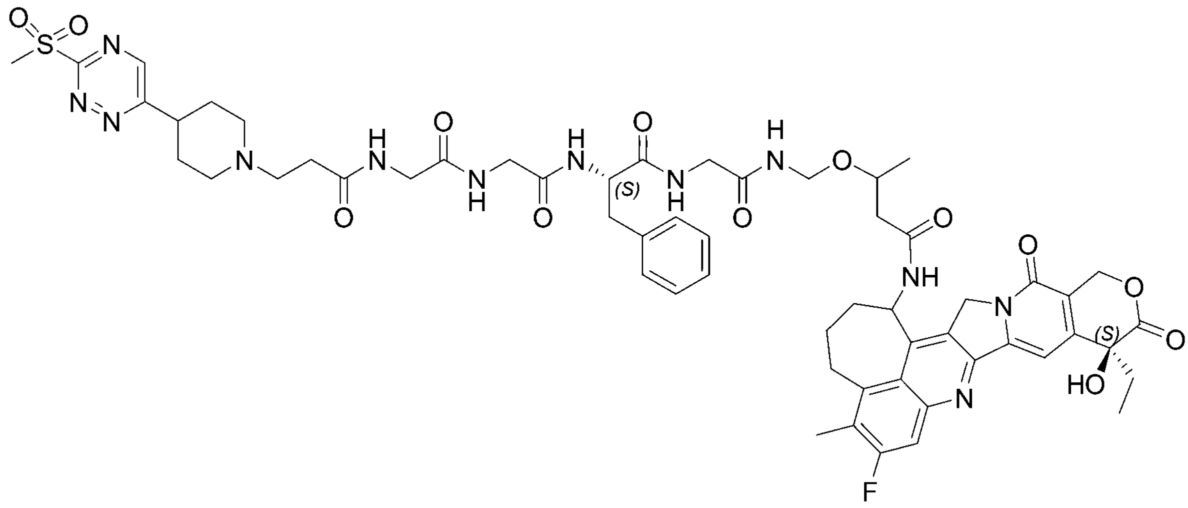

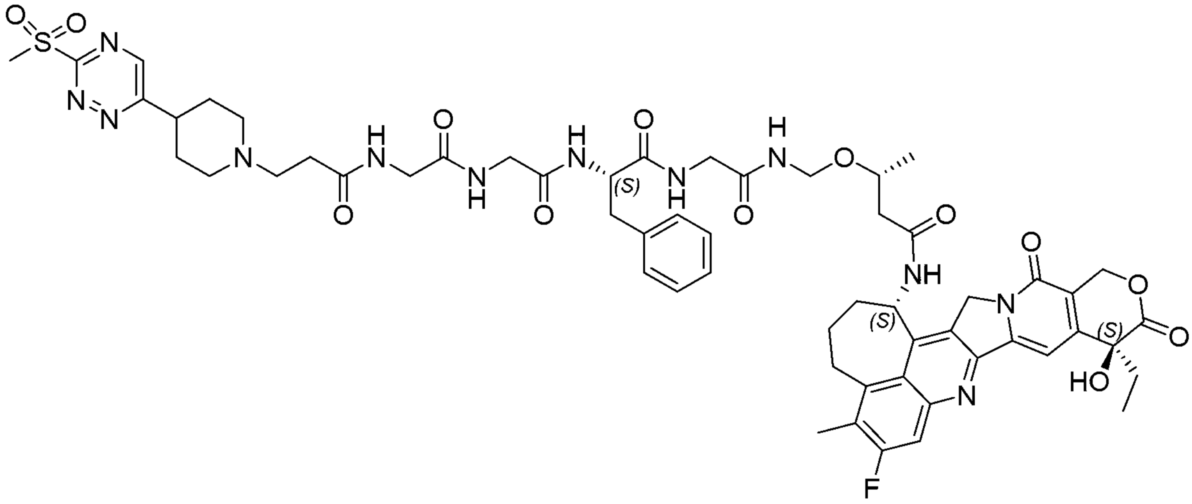

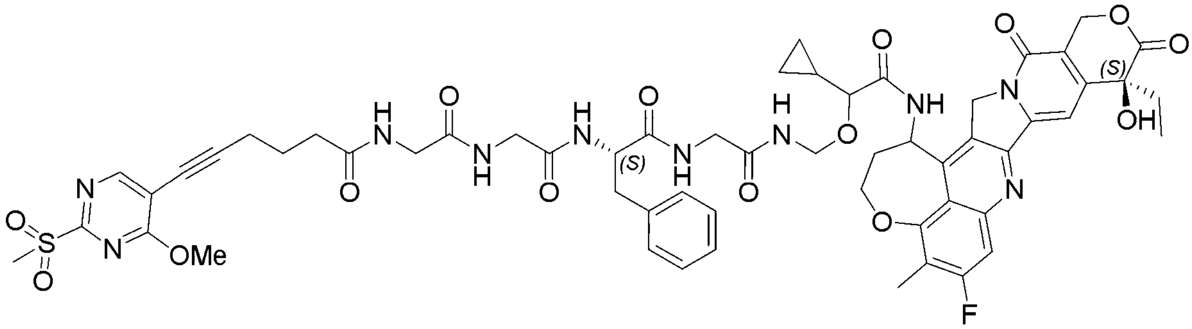

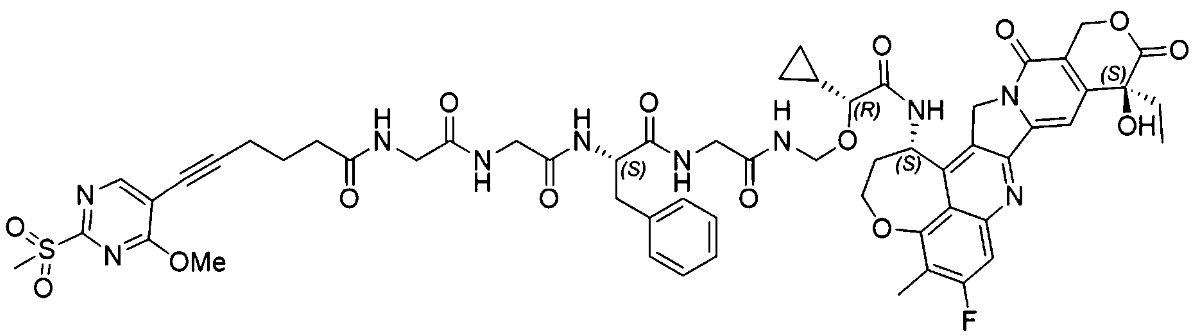

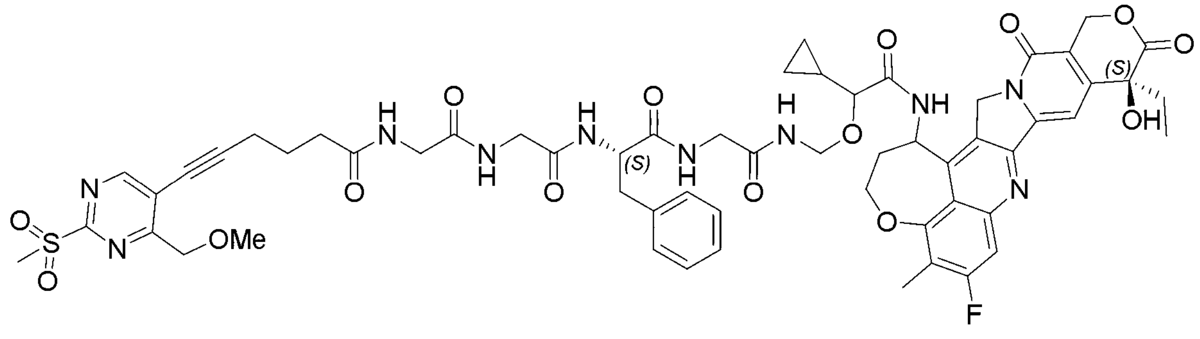

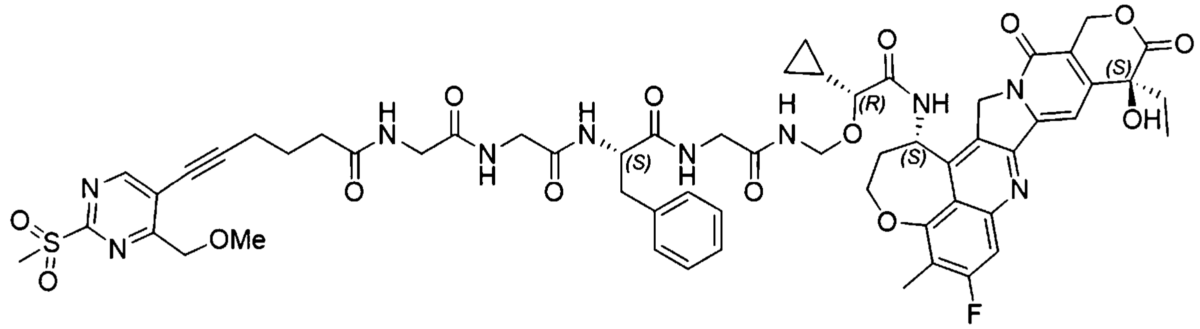

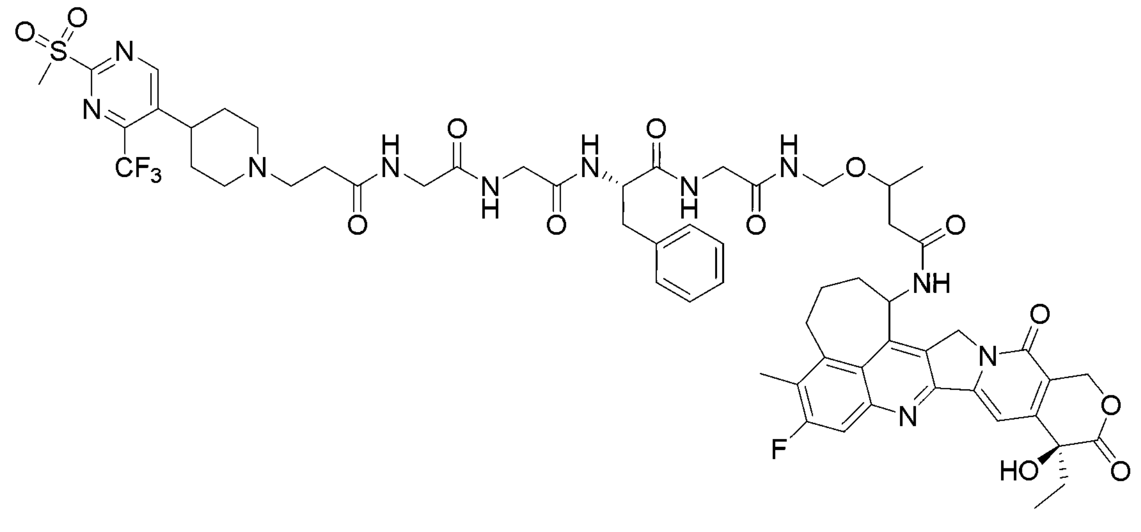

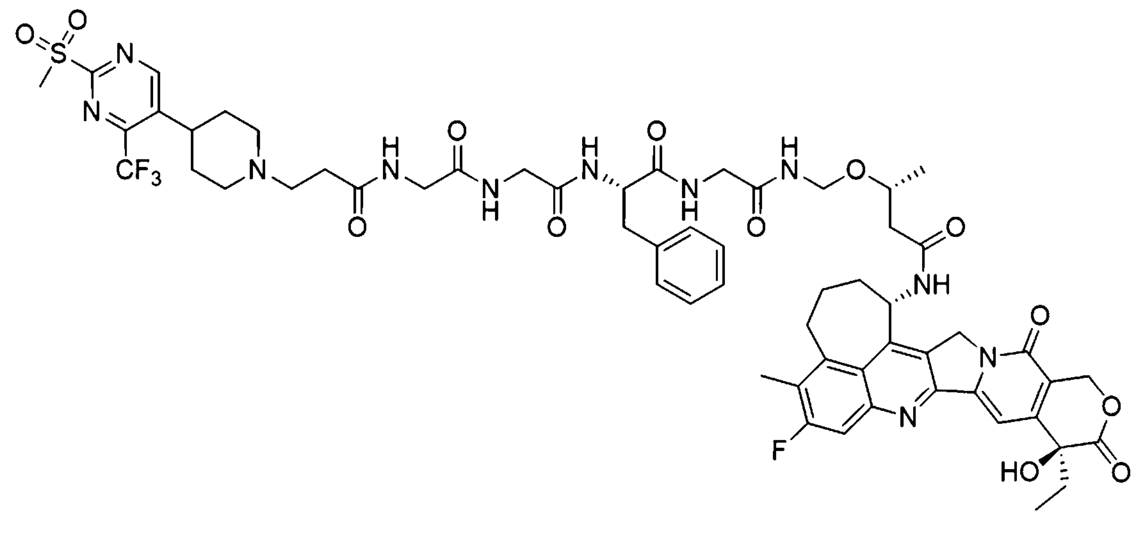

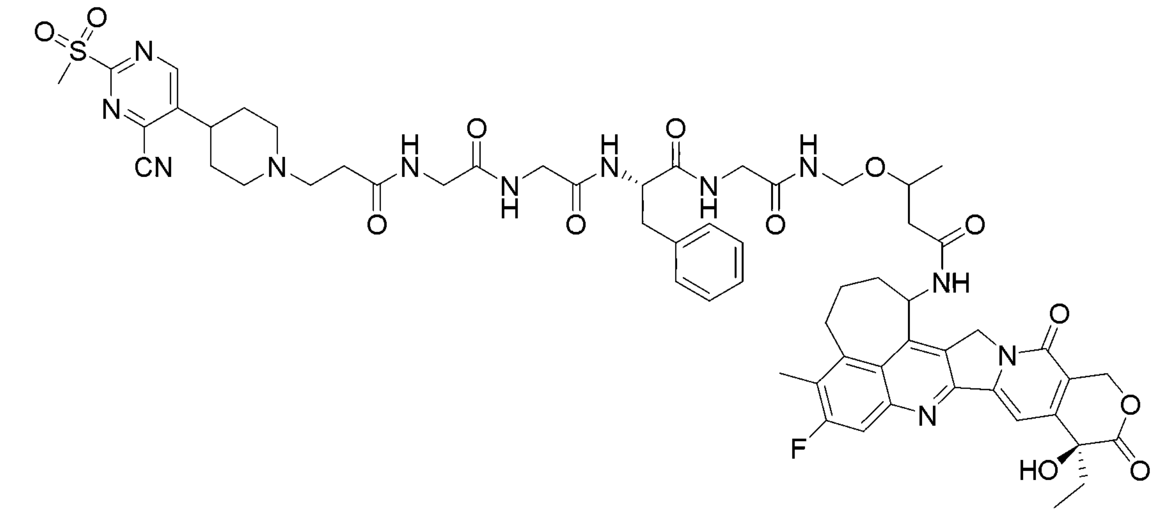

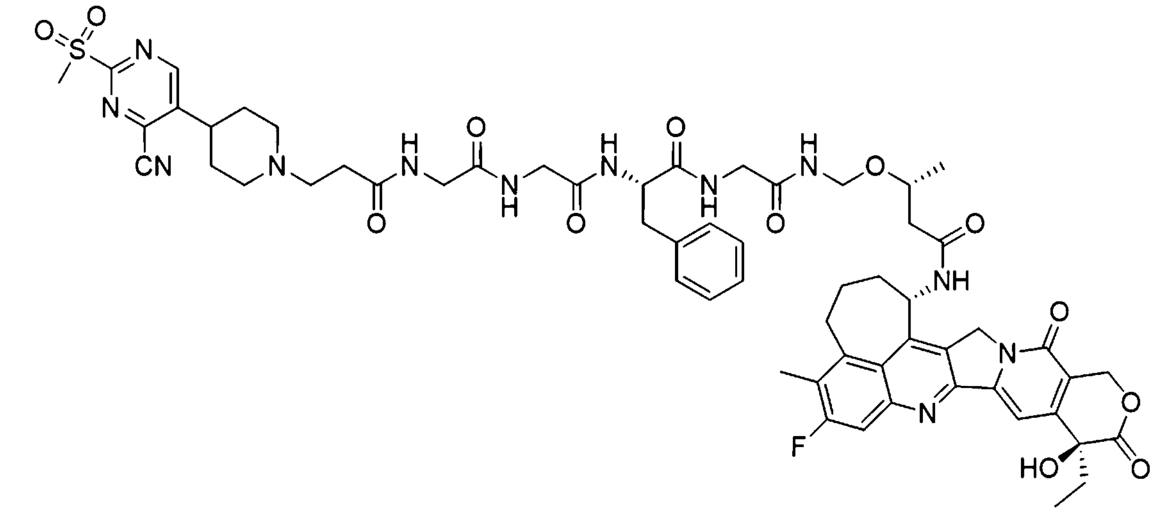

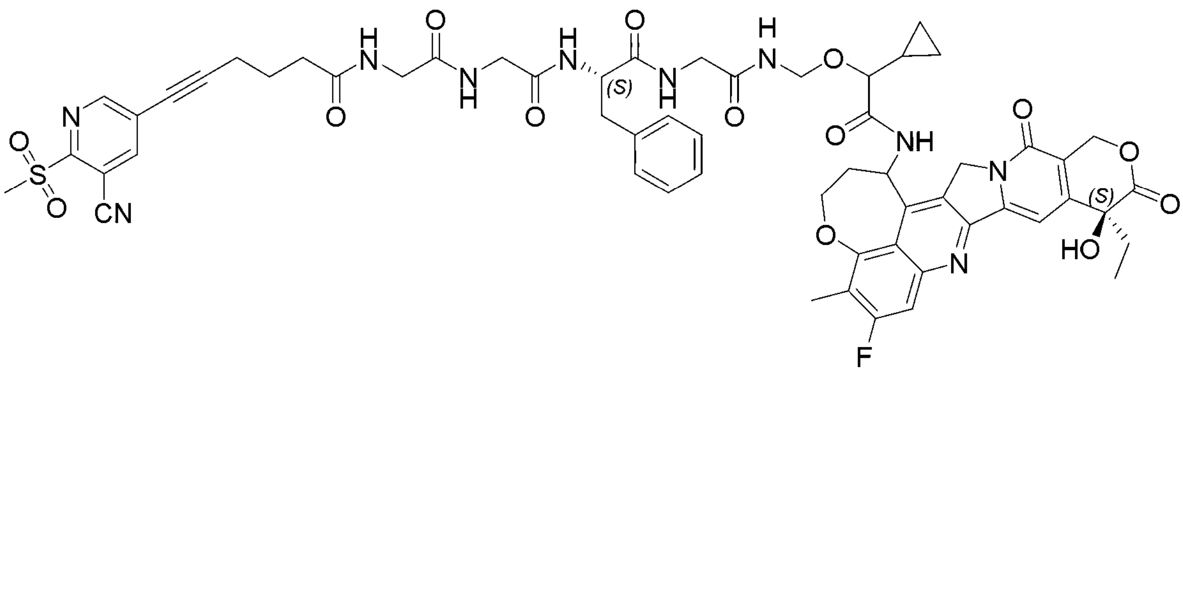

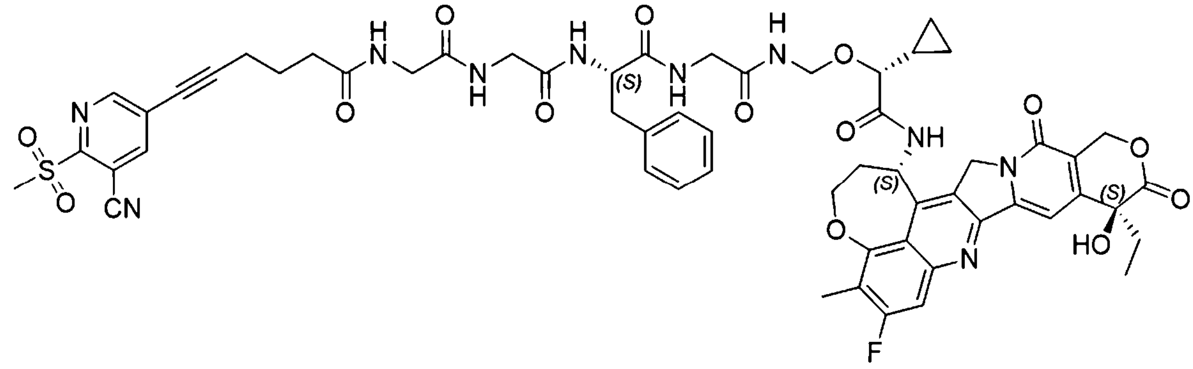

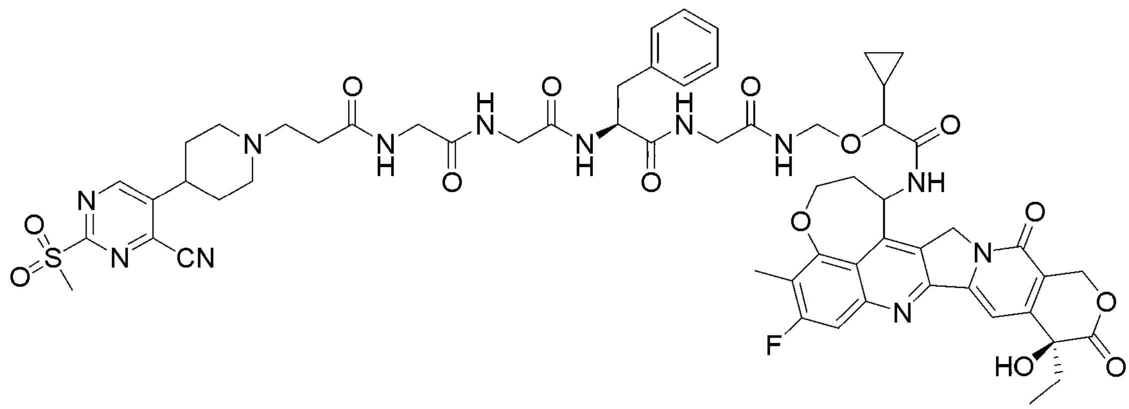

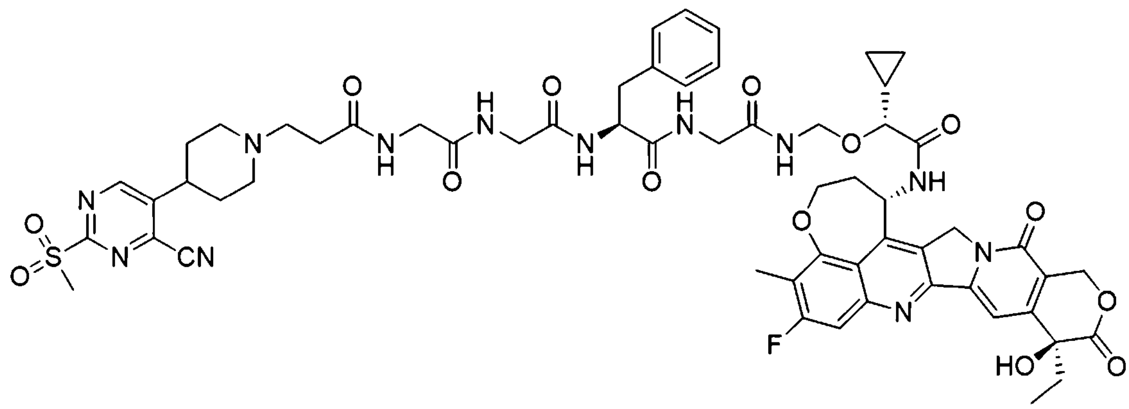

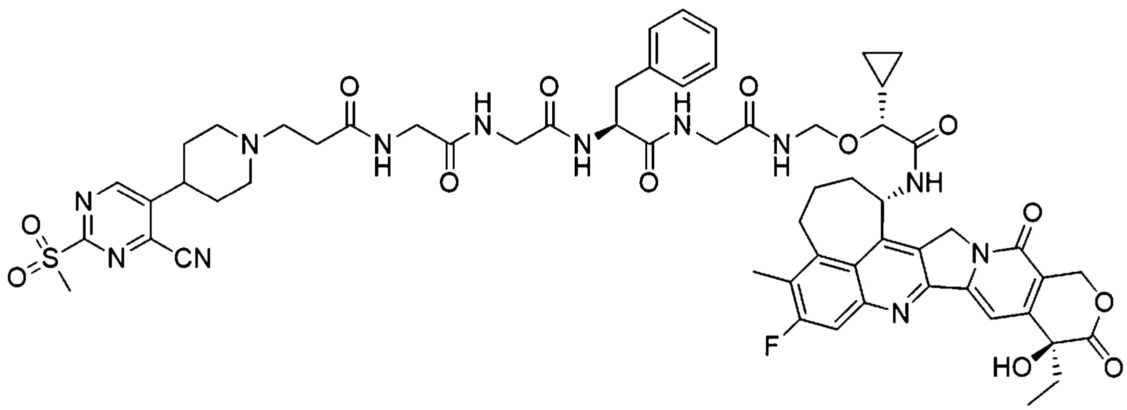

.

**10.** An antibody-drug conjugate, having a structure represented by formula VI,

Ab-[L-D]$_\beta$ (formula VI)

wherein Ab is an antibody or an antigen-binding fragment thereof, D is as defined in claim 8 or 9, L is a linker linking the Ab to the D, and β is an integer or a decimal selected from 1-10;
preferably, Ab is an antibody or an antigen-binding fragment, the antigen-binding fragment is selected from Fab, Fab', (Fab')$_2$, Fd, Fv, disulfide-linked Fv, scFv, di-scFv, (scFv)$_2$, a diabody, and a single domain antibody (sdAb); and/or the antibody is a murine antibody, a humanized antibody, a chimeric antibody, a bispecific antibody, or a multispecific antibody;
preferably, Ab is an anti-HER2 antibody or an antigen-binding fragment thereof; more preferably, Ab is trastuzumab or an antigen-binding fragment thereof; and
preferably, β is selected from an integer or decimal between 4 and 9 (e.g., 7, 7.71, 7.84, 7.92, 7.94, 7.97, 7.98, 7.99, 8, 8.02, 8.06, or 8.14).

**11.** The antibody-drug conjugate according to claim 10, wherein L is selected from M'-$L_1$- $L_2$, wherein M' is a linker site linking to an antibody or an antigen-binding fragment thereof, and is formed by conjugating M defined in any one of claims 8 to 9 with an antibody or an antigen-binding fragment thereof, and $L_1$ and $L_2$ are as defined in any one of claims 8 to 9,

preferably, M' is selected from

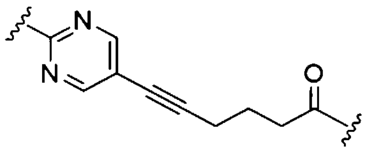,
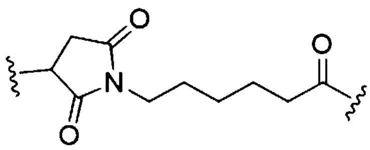,

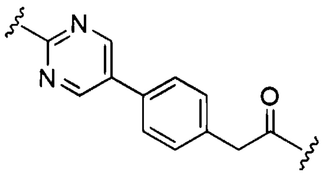,
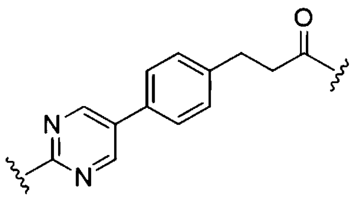,
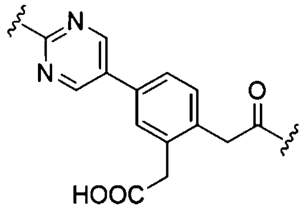,

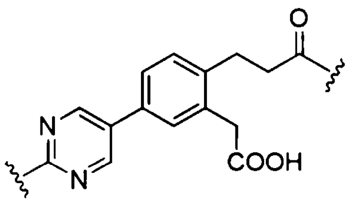,
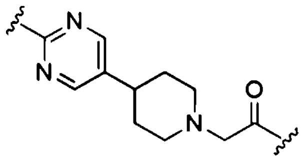,
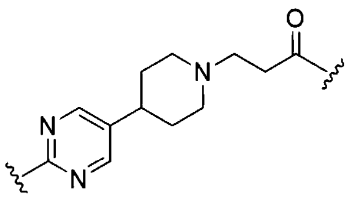,

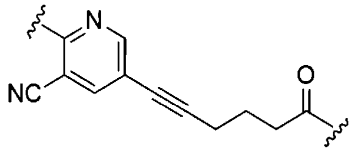, 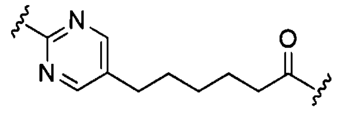, 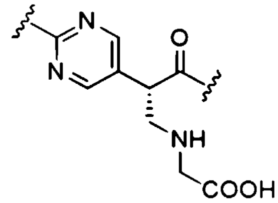,

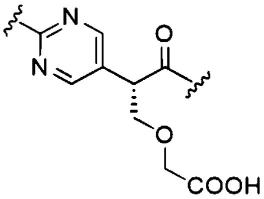, 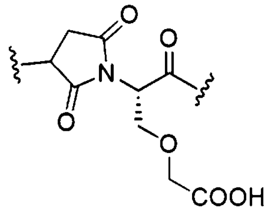, 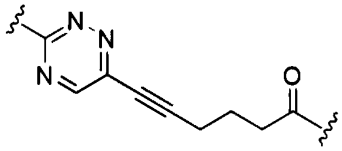,

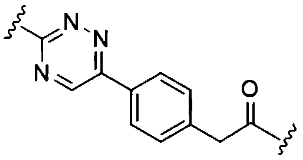, 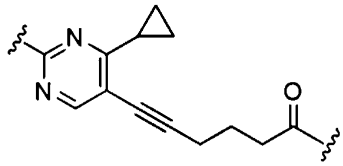, 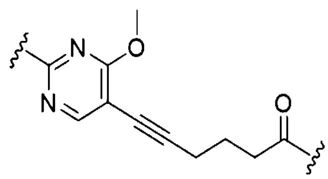,

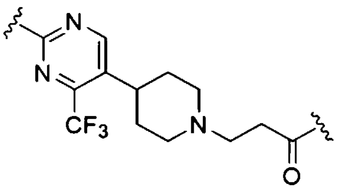, 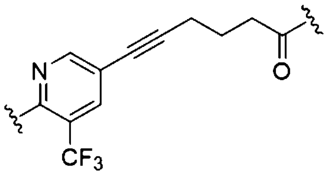, 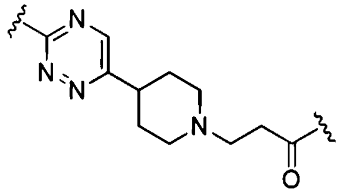,

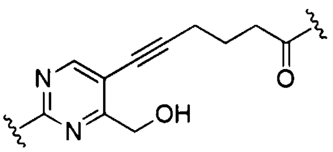, 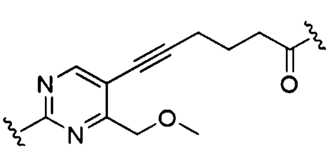, 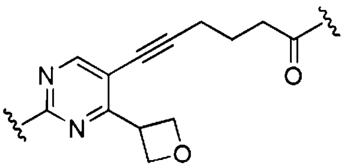,

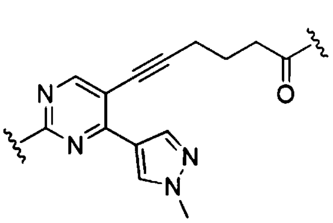, 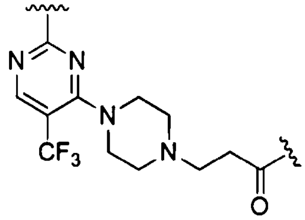, 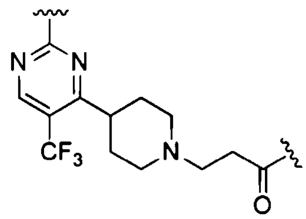,

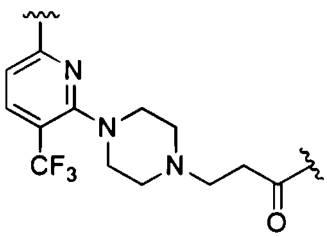, 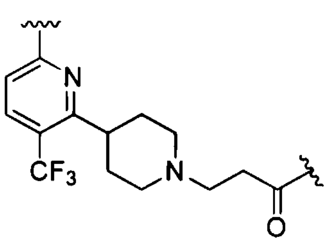, or 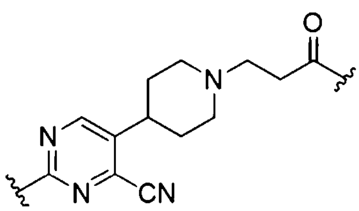;

preferably, the carbonyl linking position in M' is linked to $L_1$, and the linking position on the heterocyclic or

heteroaromatic ring is linked to Ab;
preferably, L is selected from:

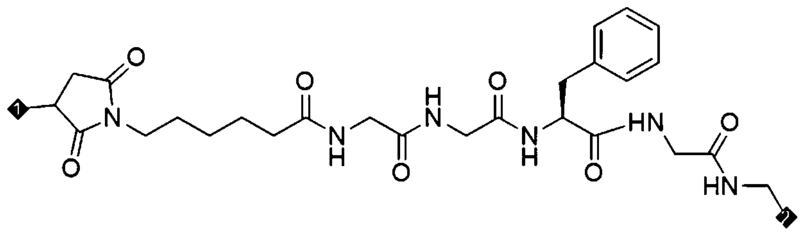

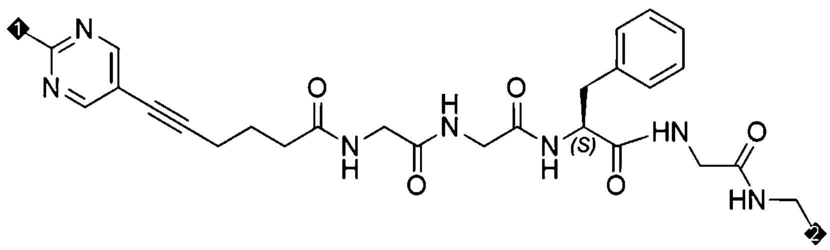

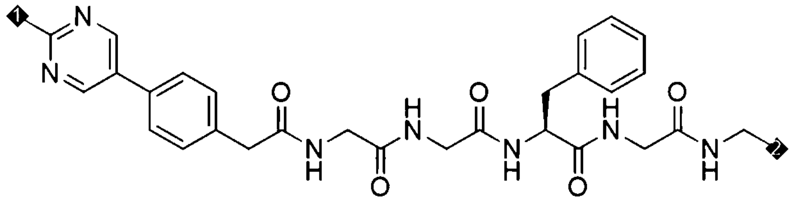

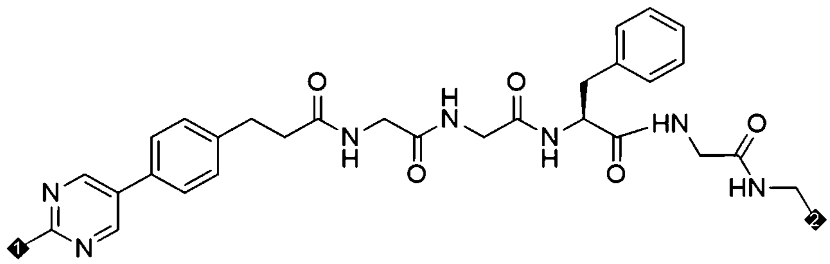

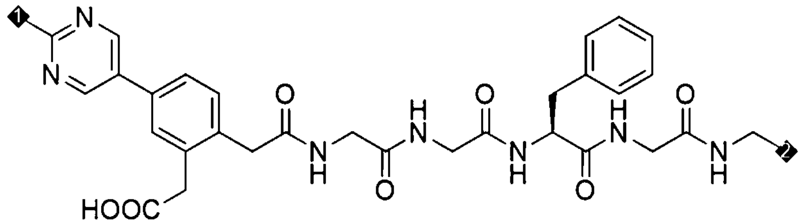

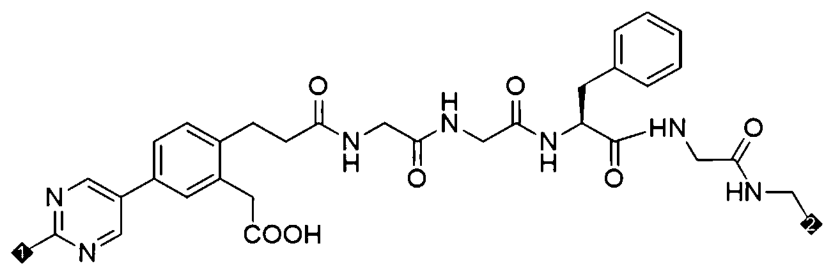

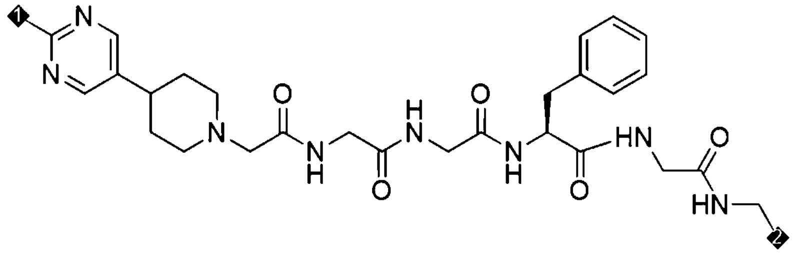

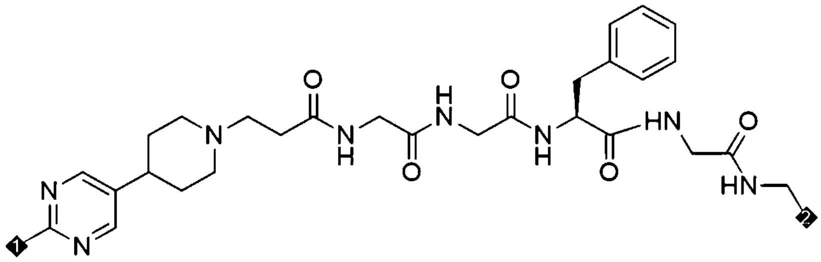

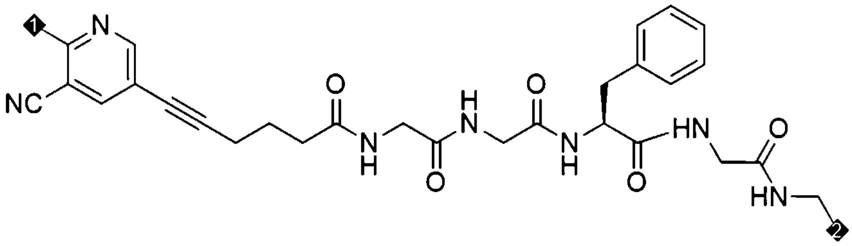

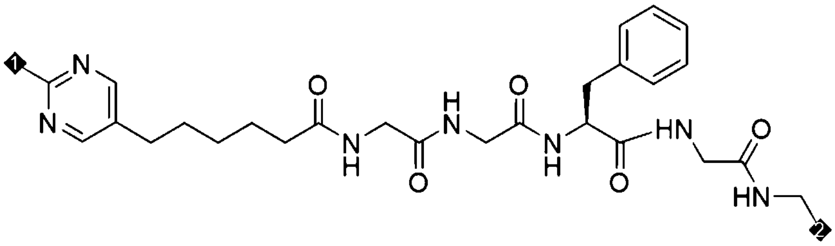

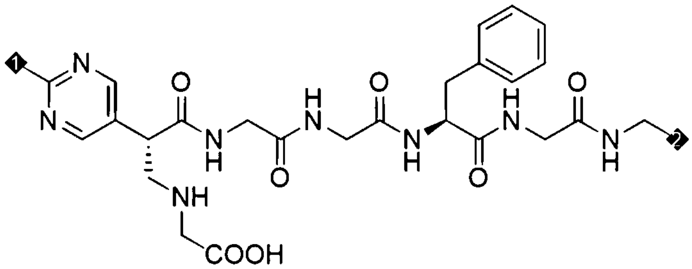

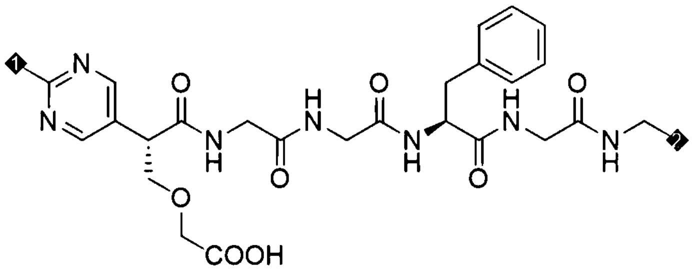

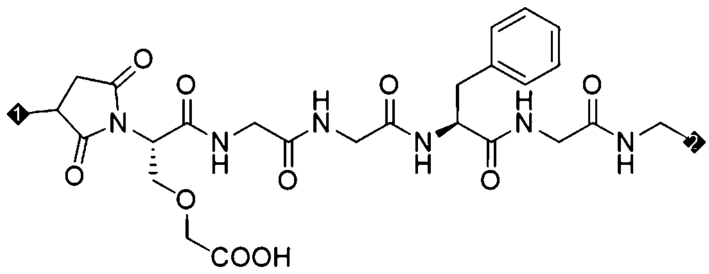

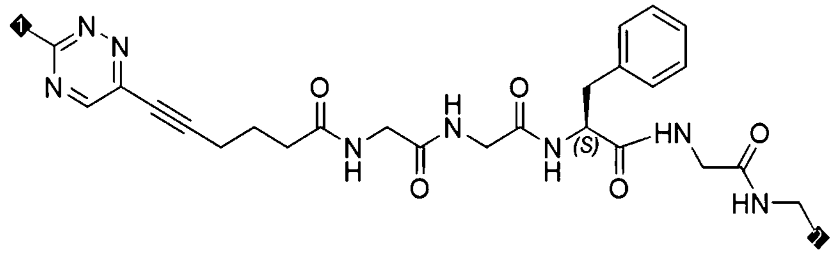

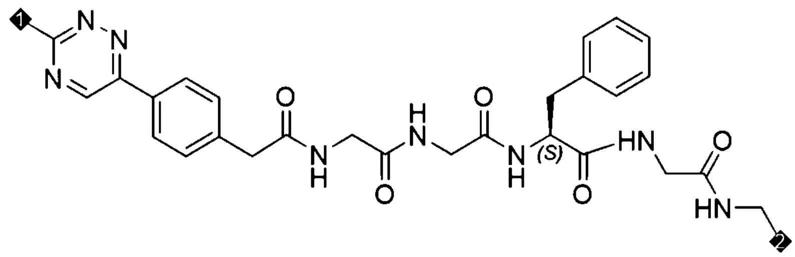

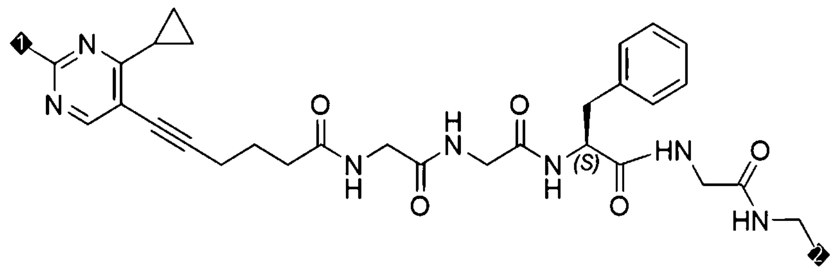

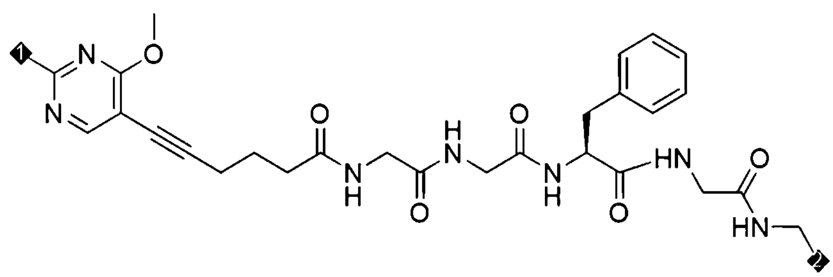

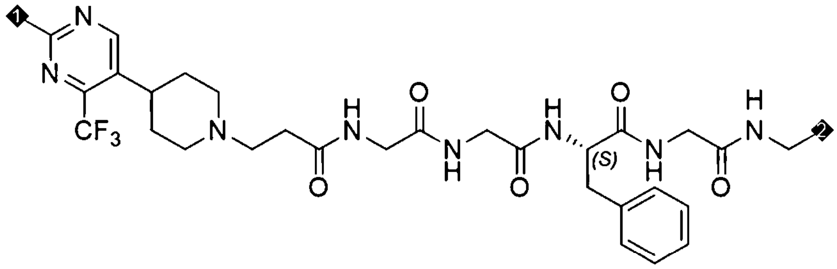

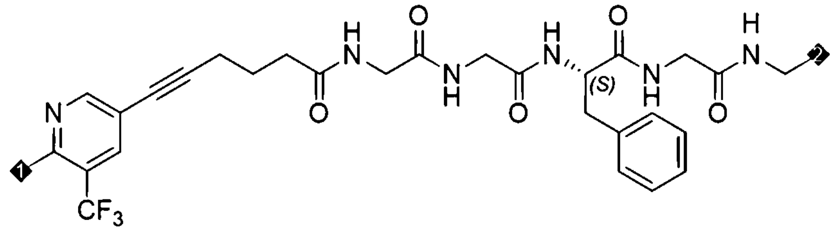

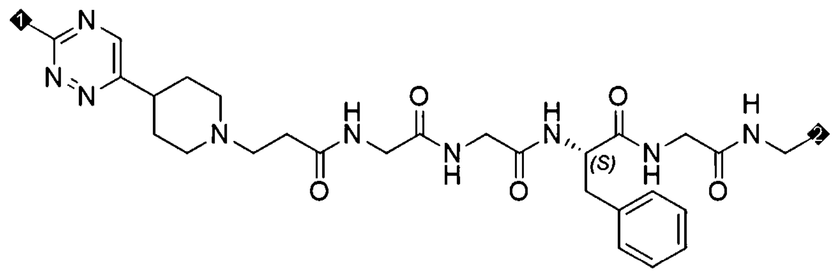

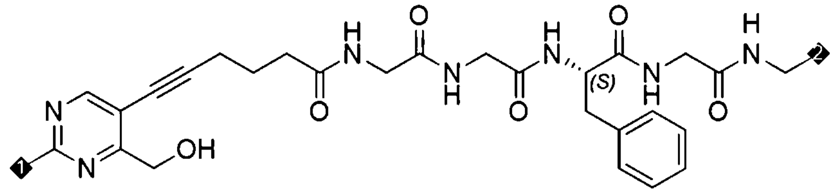

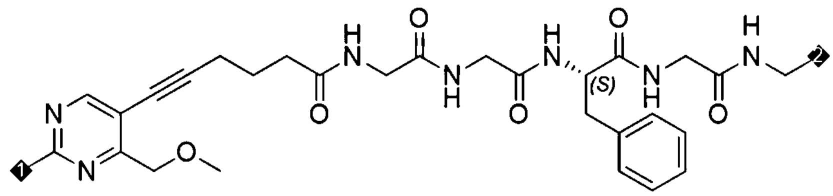

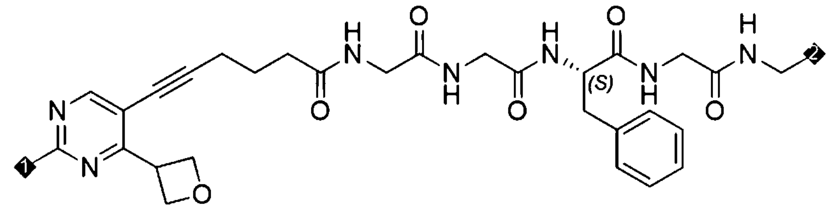

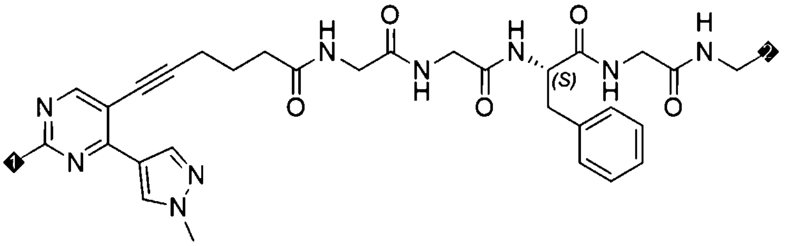

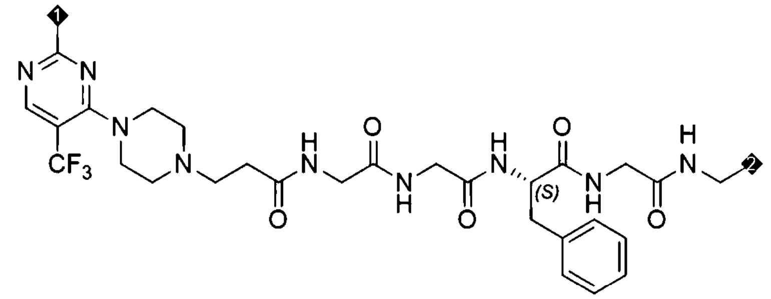

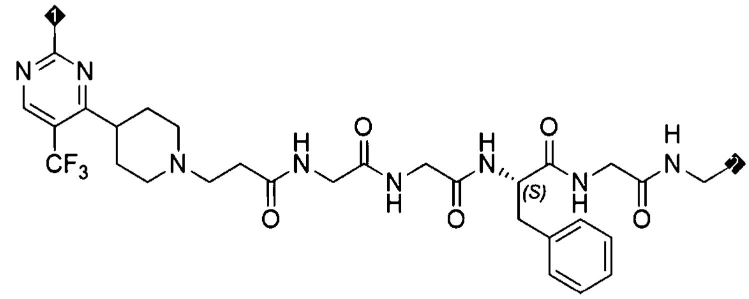

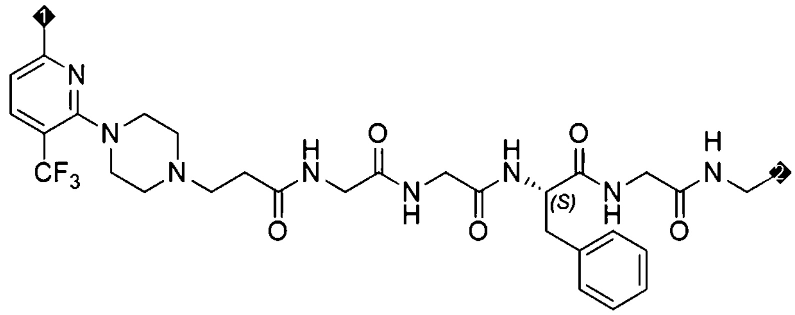

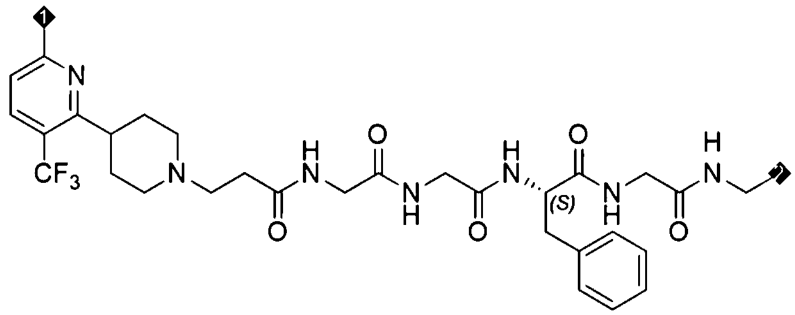

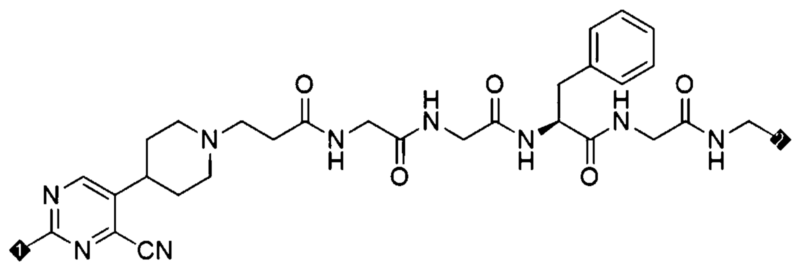

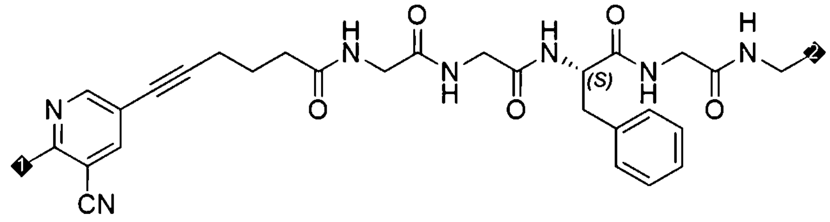

;

wherein position 1 is linked to Ab and position 2 is linked to D;
preferably, L-D is selected from:

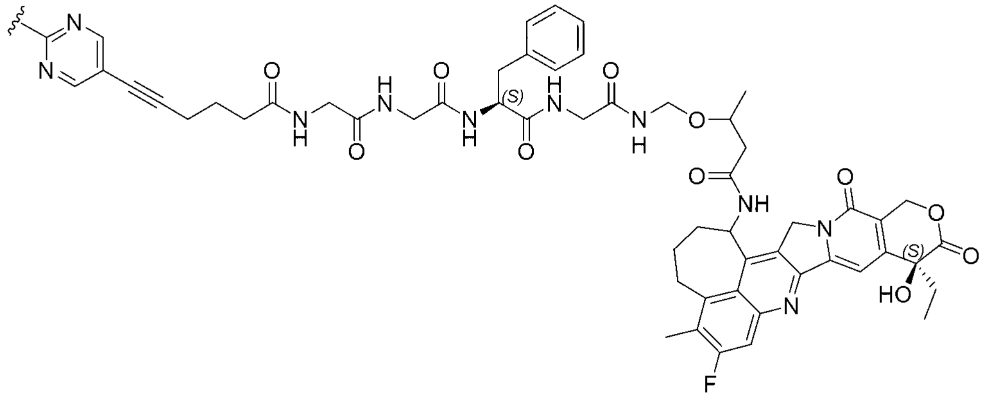

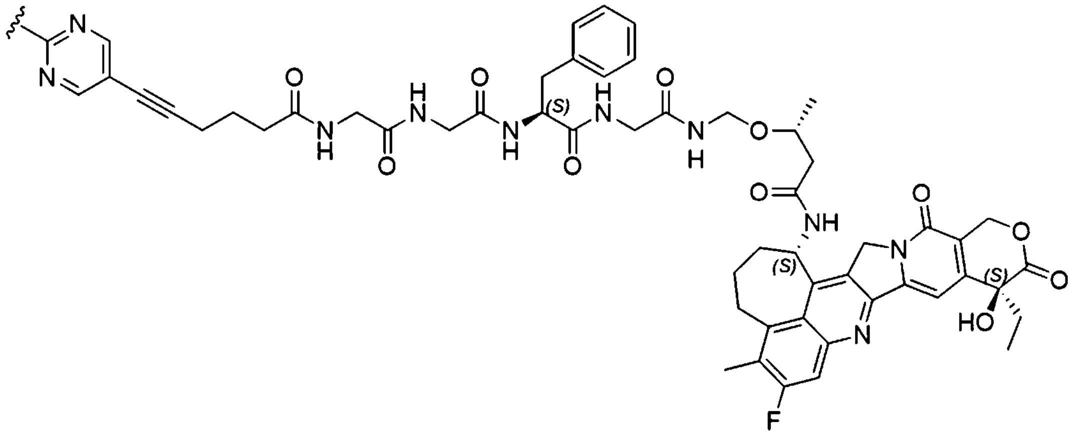

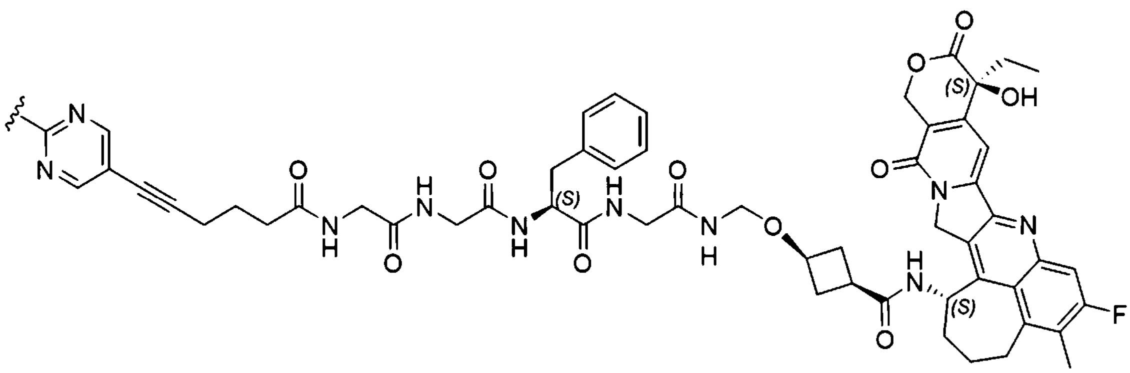

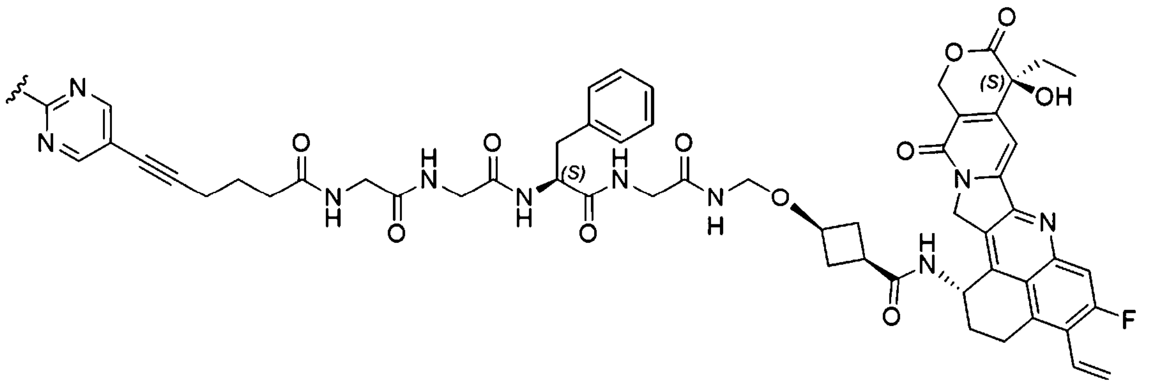

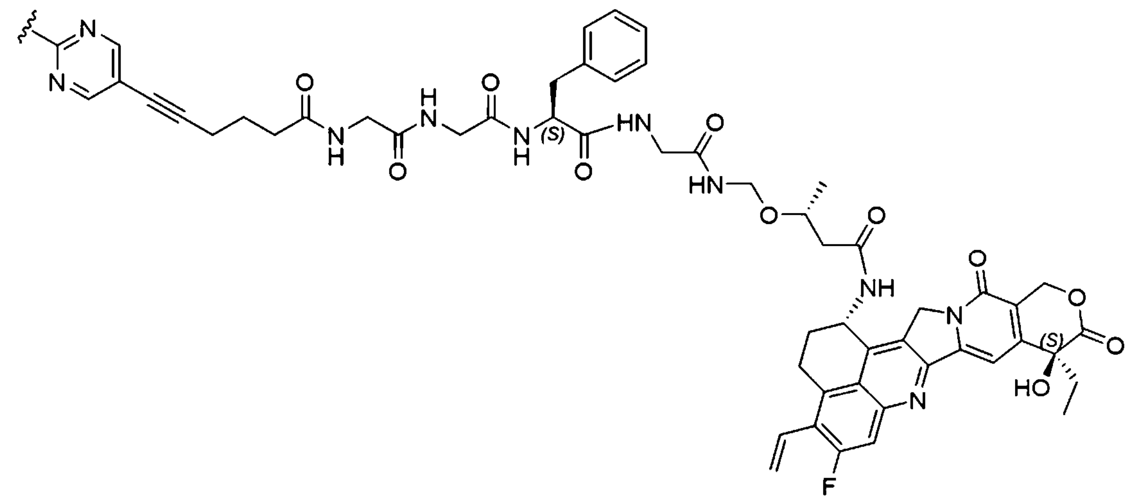

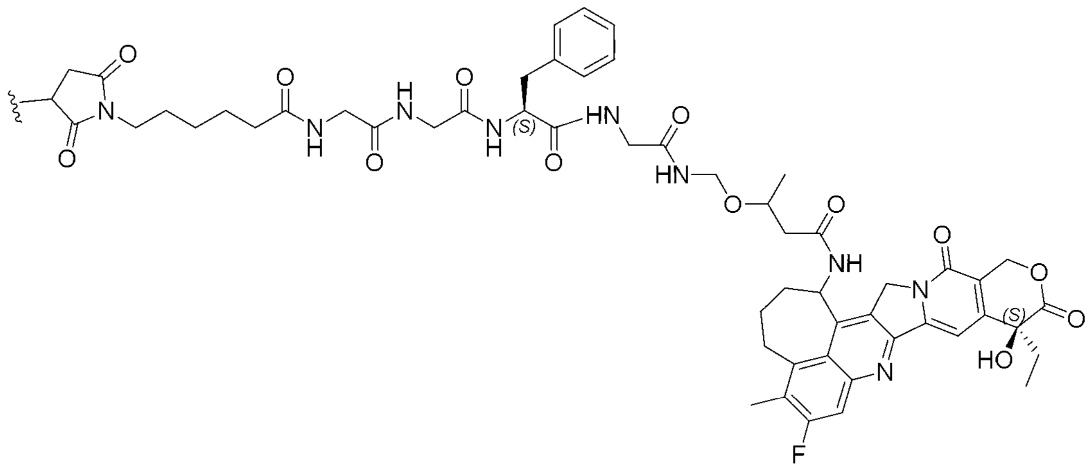

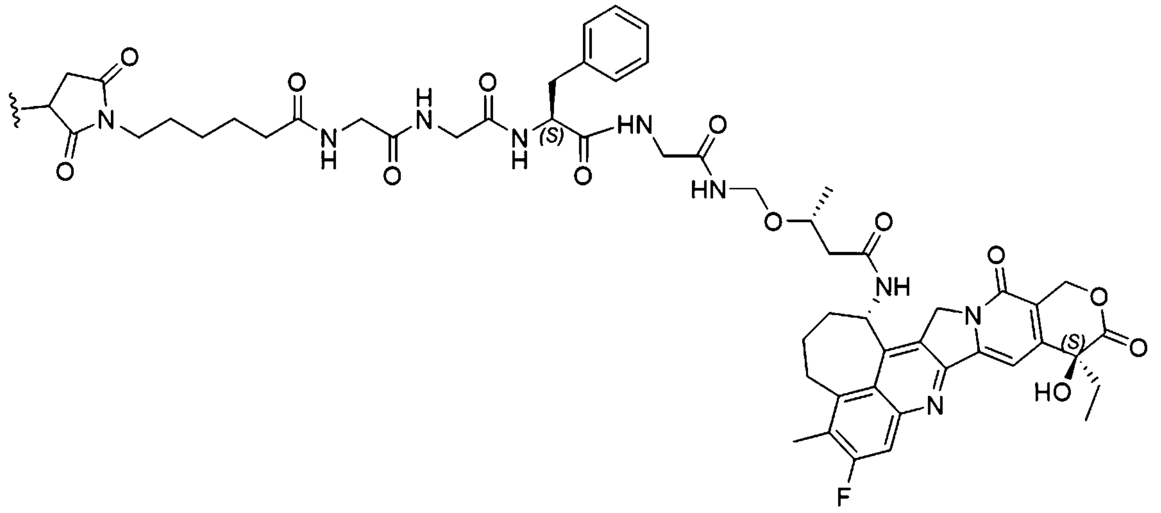

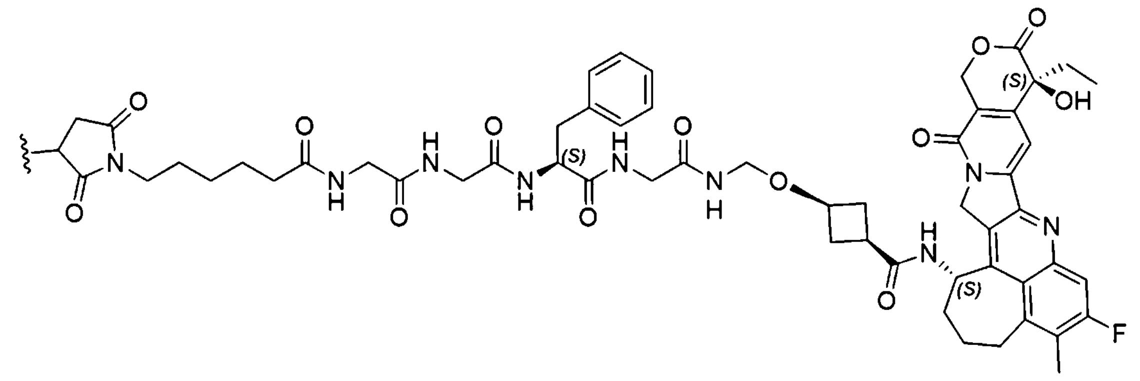

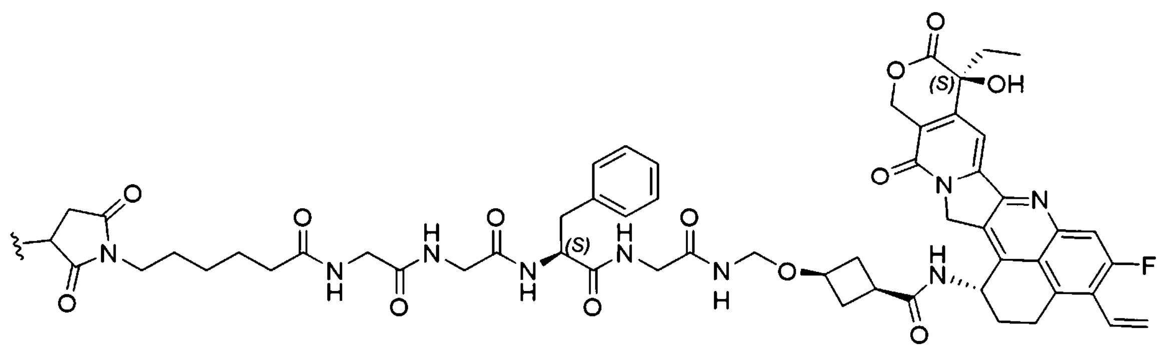

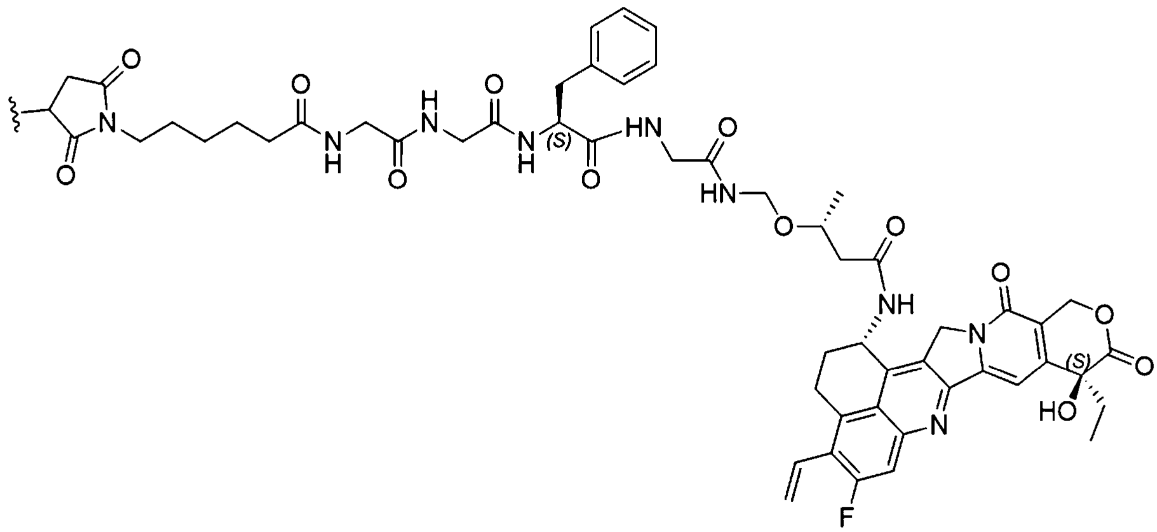

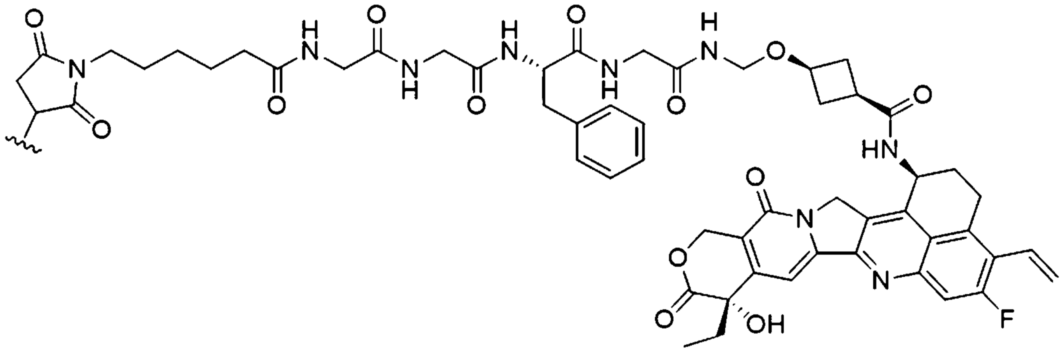

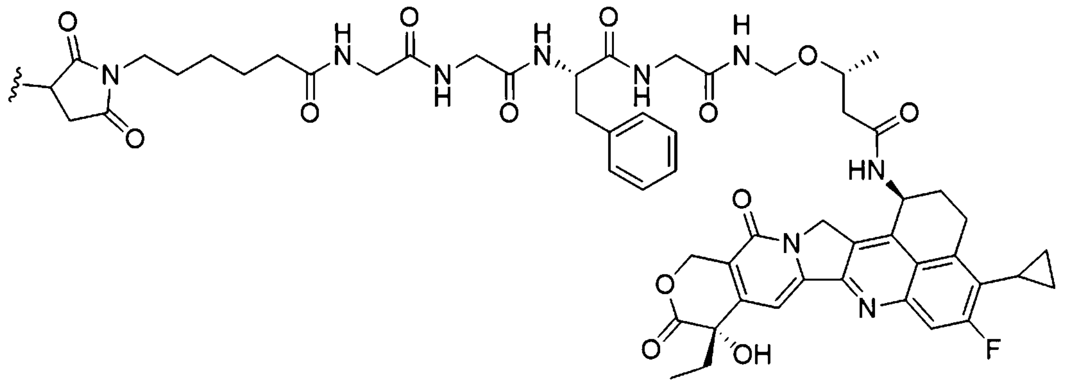

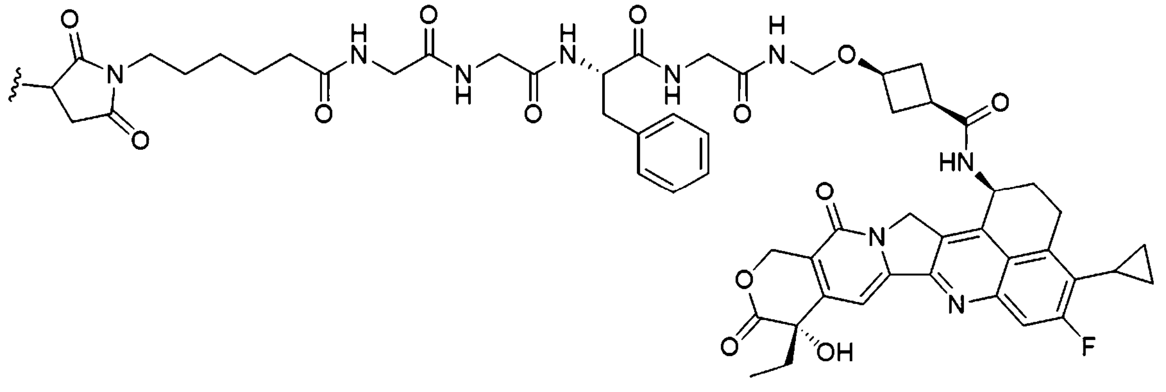

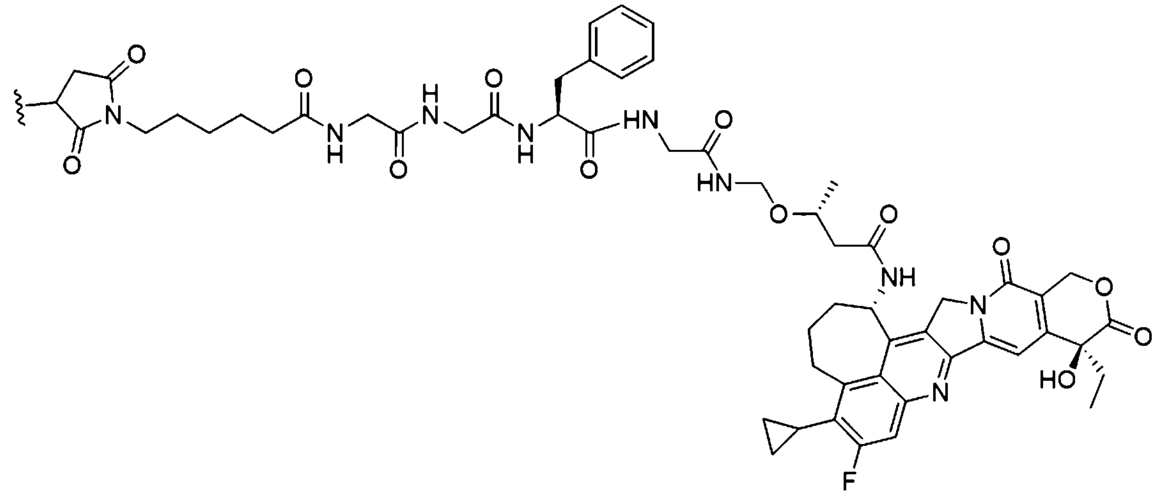

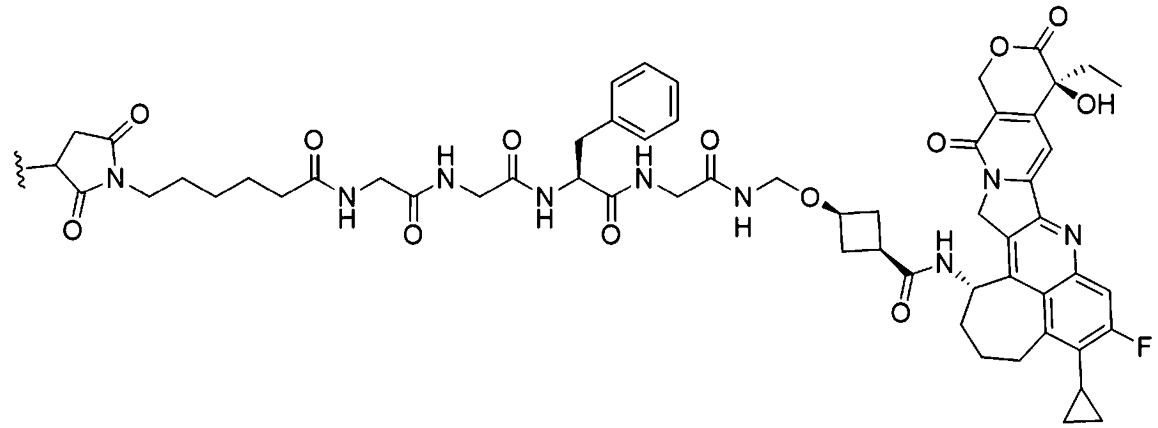

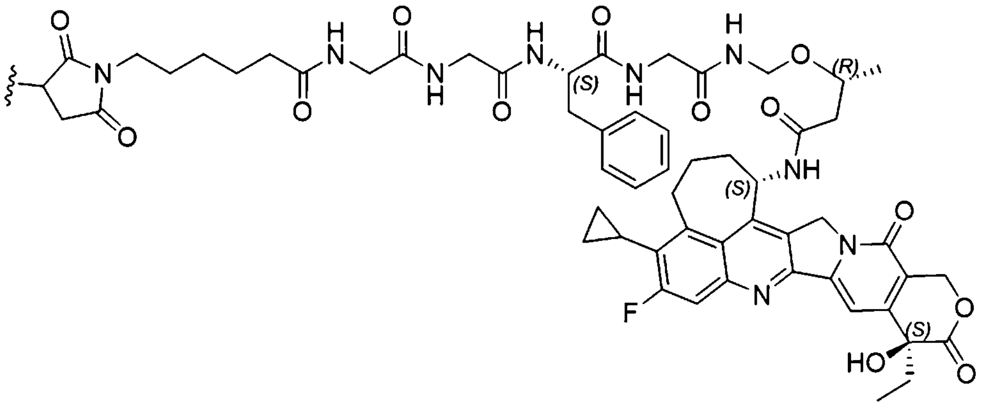

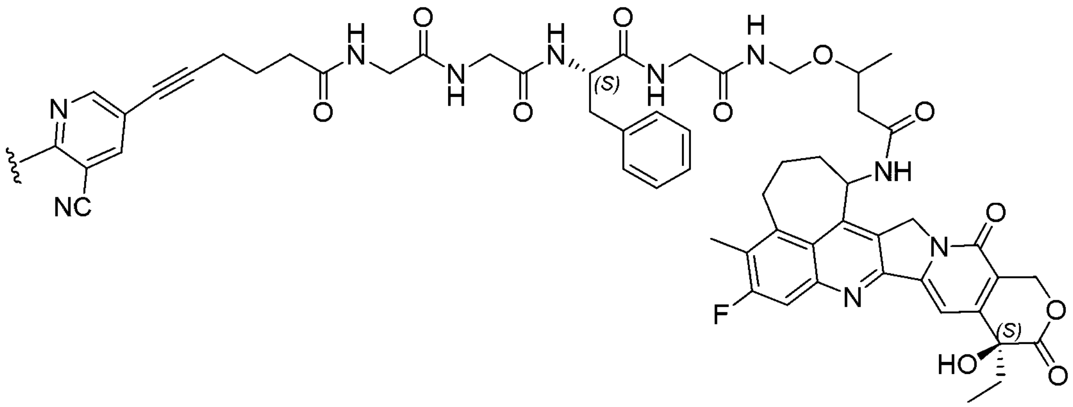

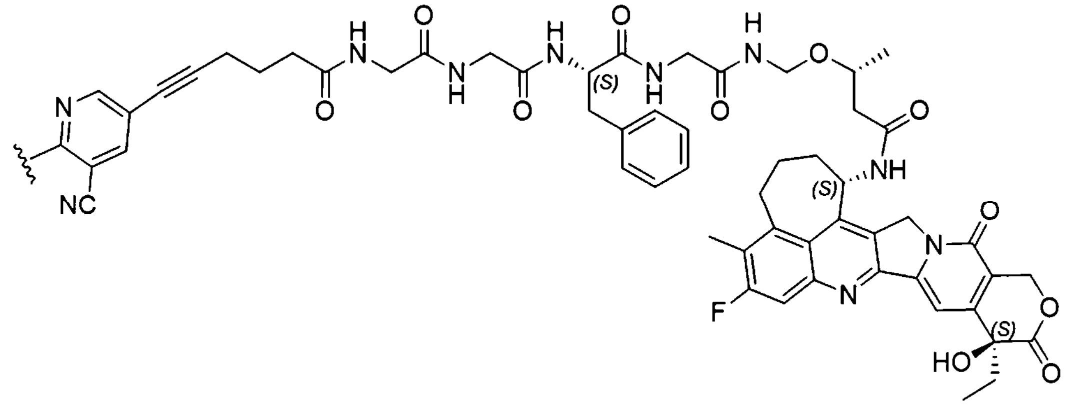

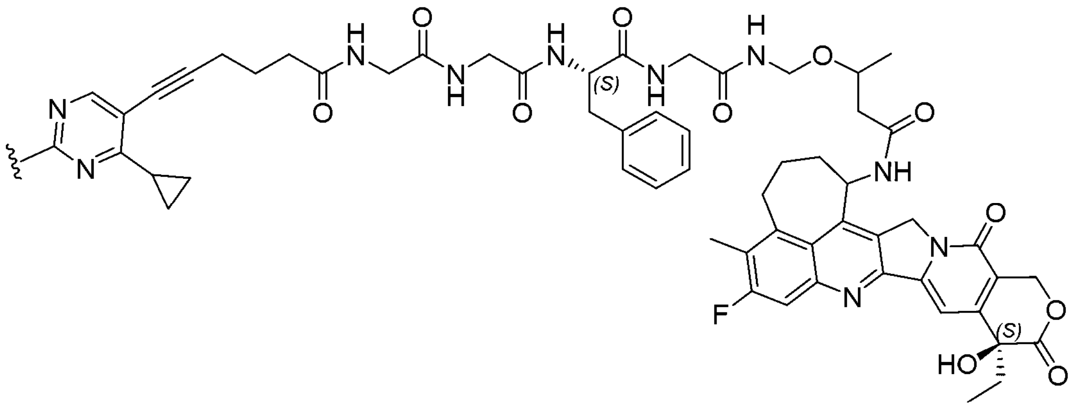

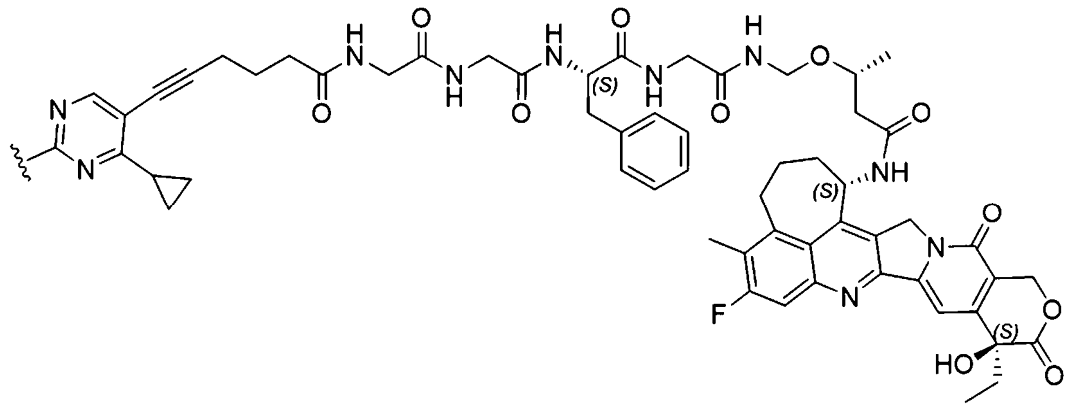

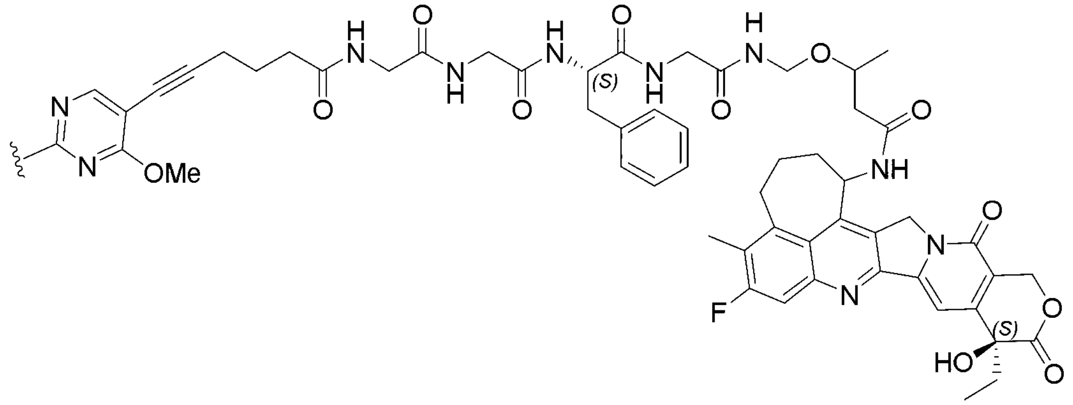

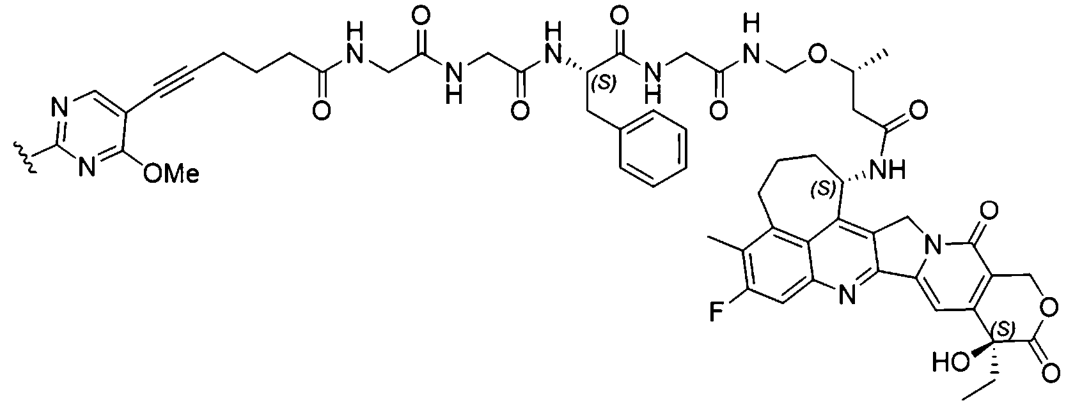

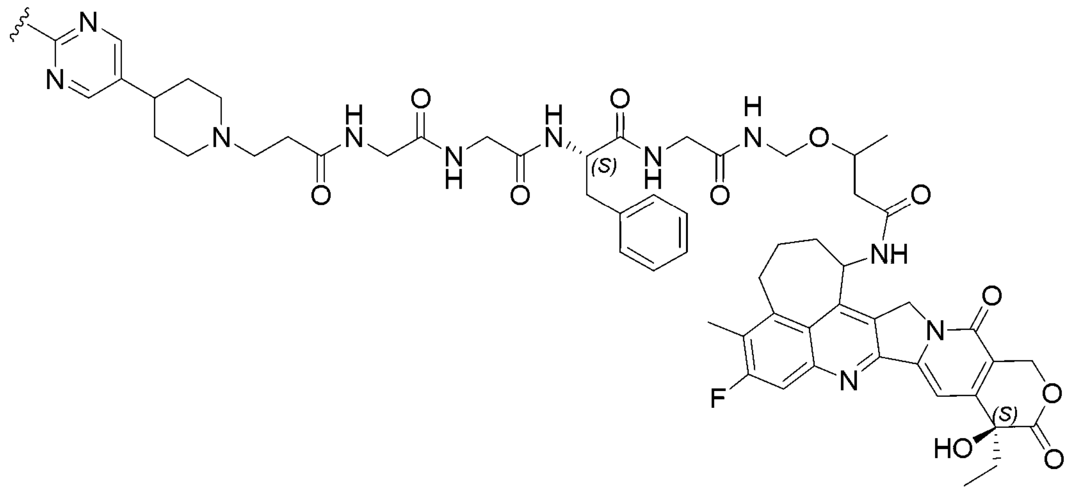

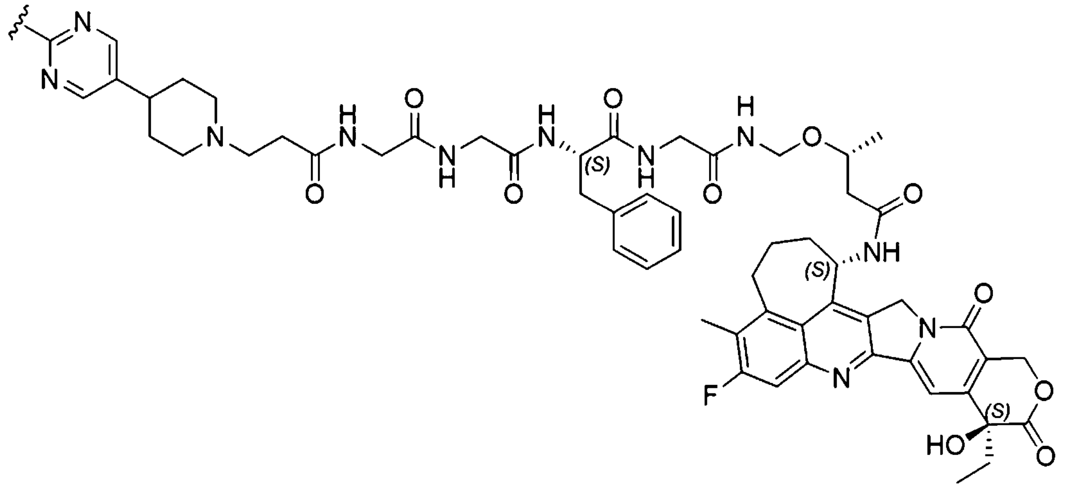

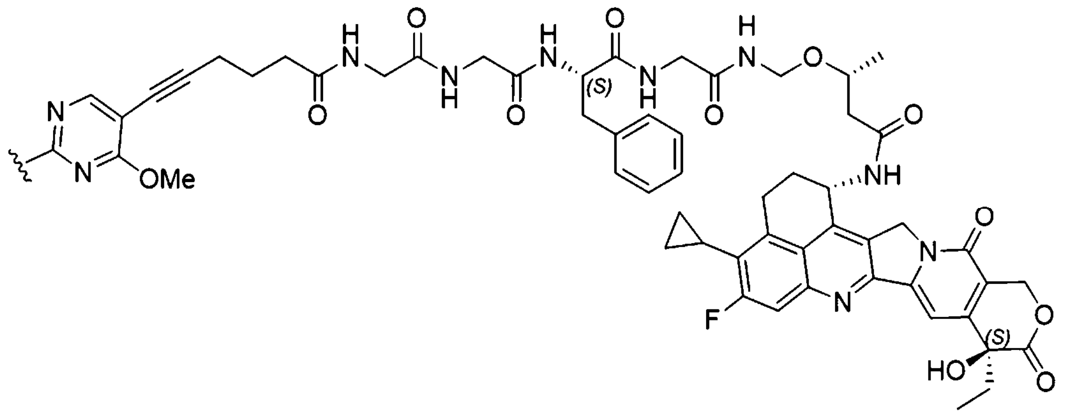

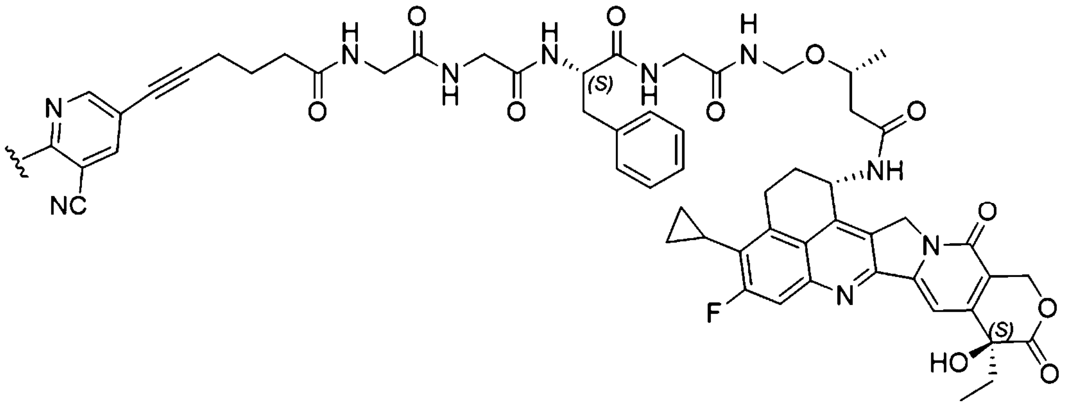

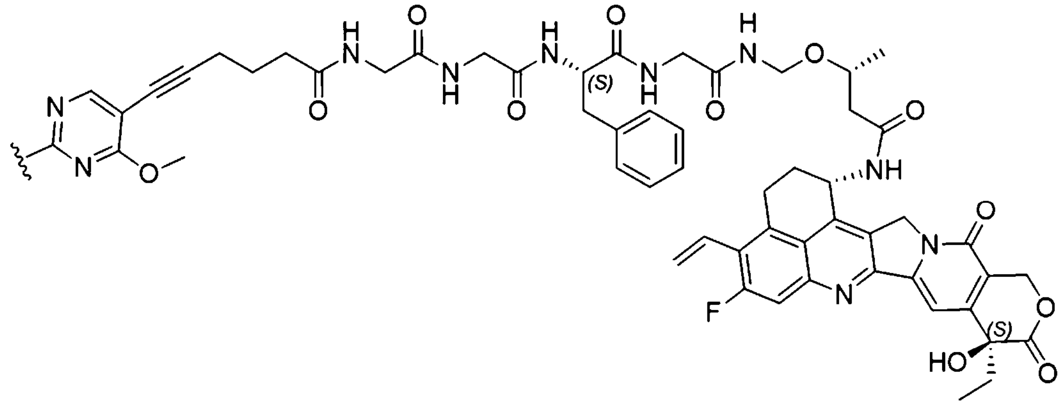

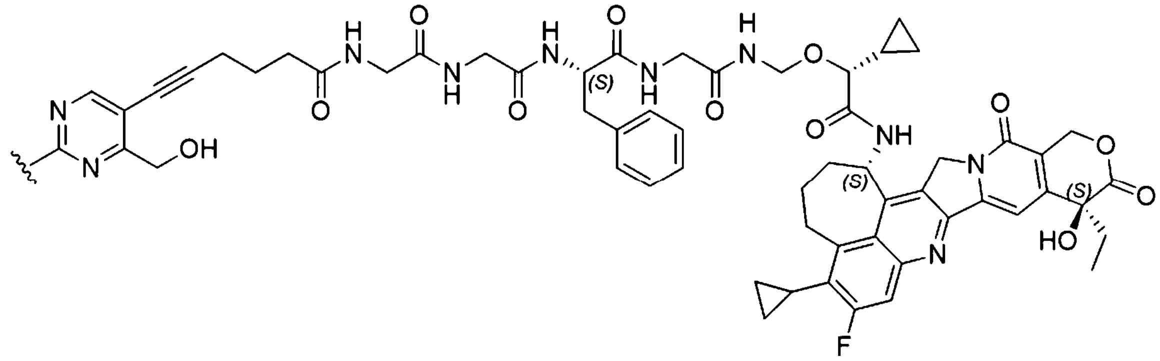

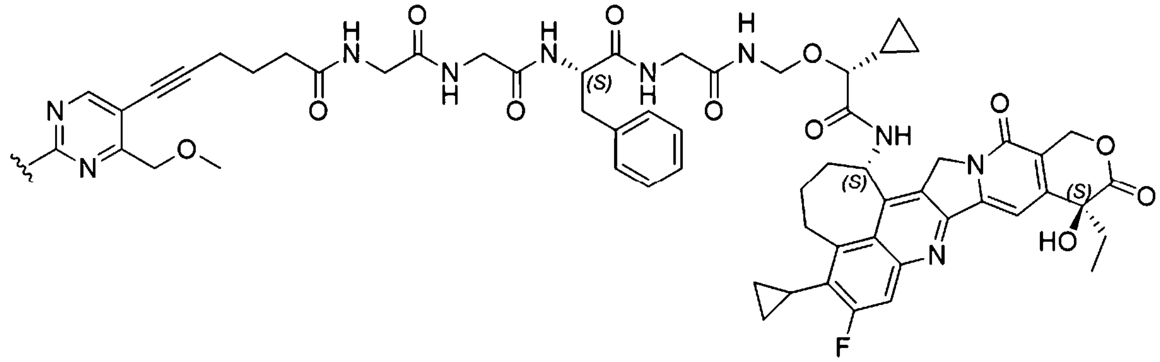

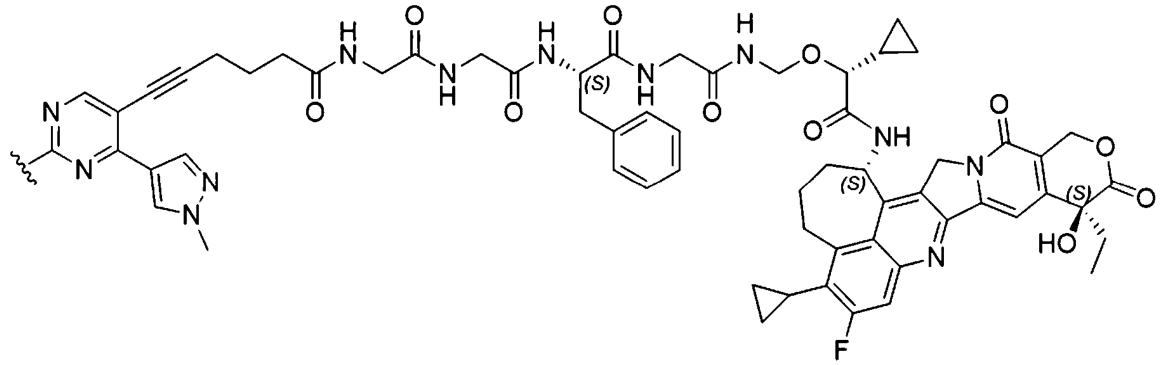

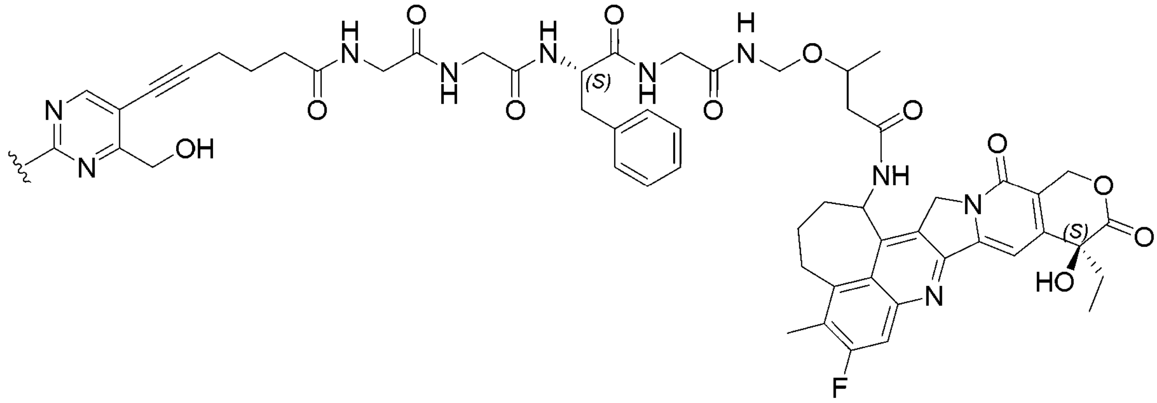

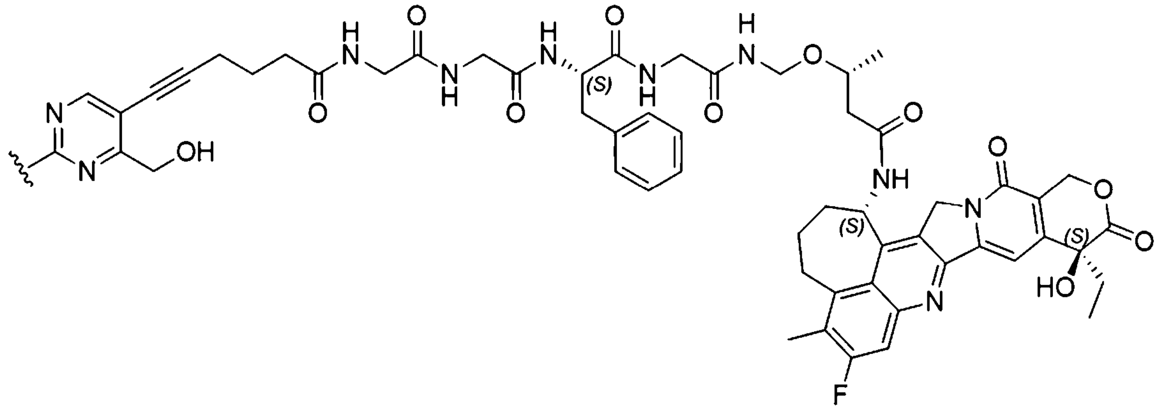

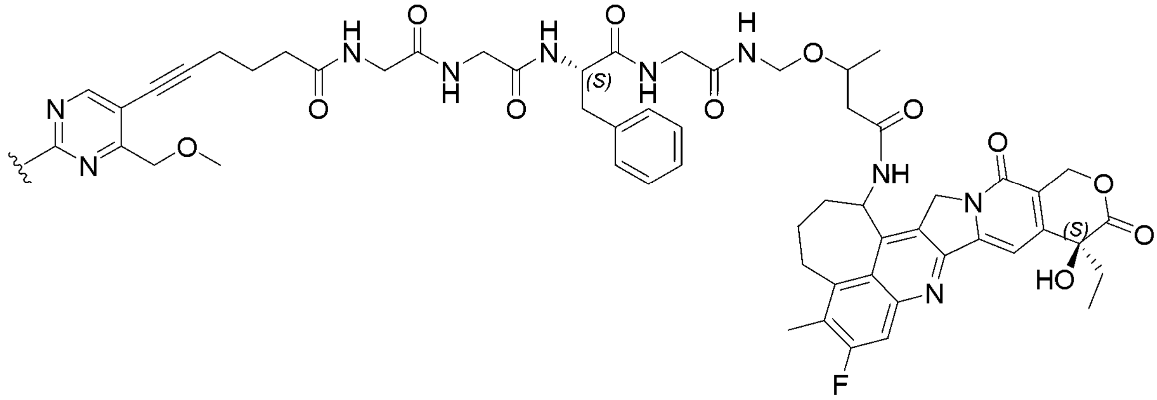

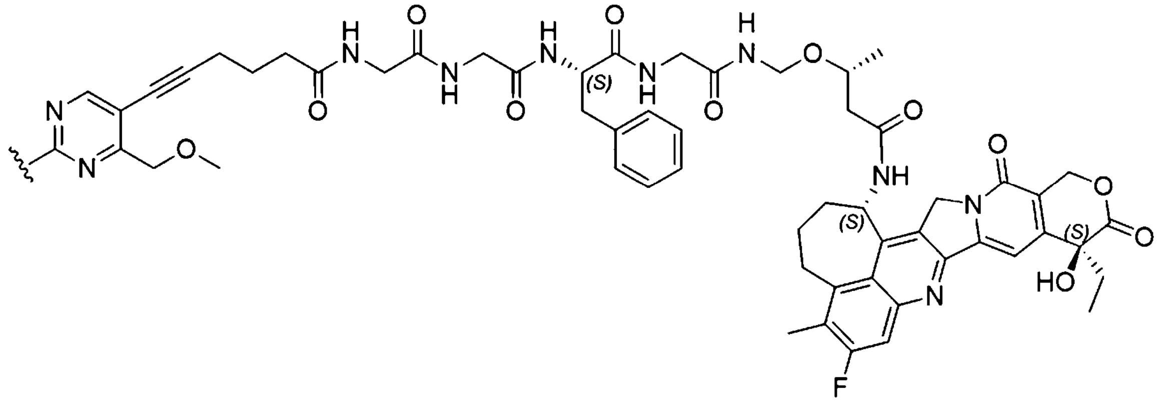

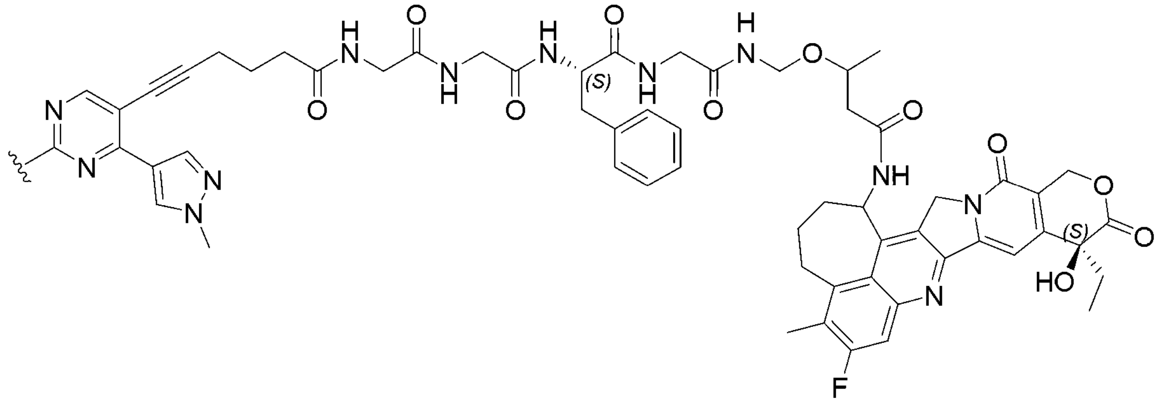

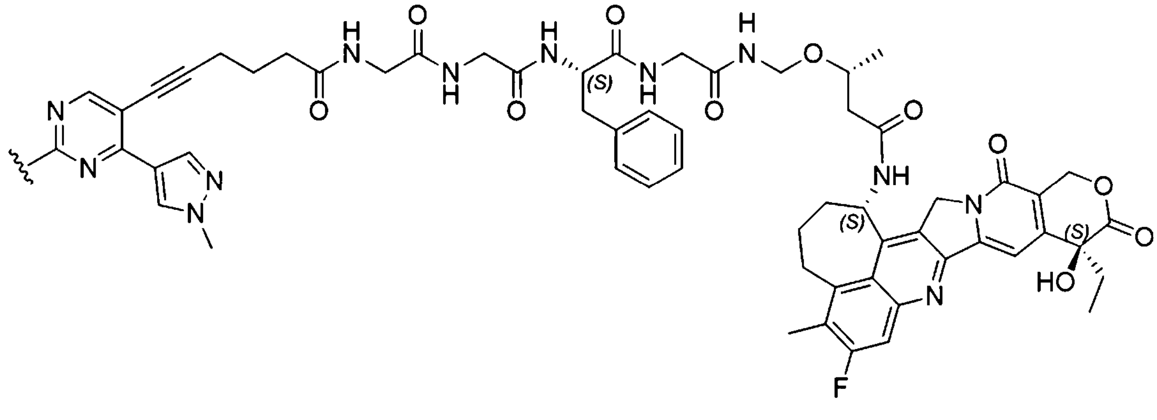

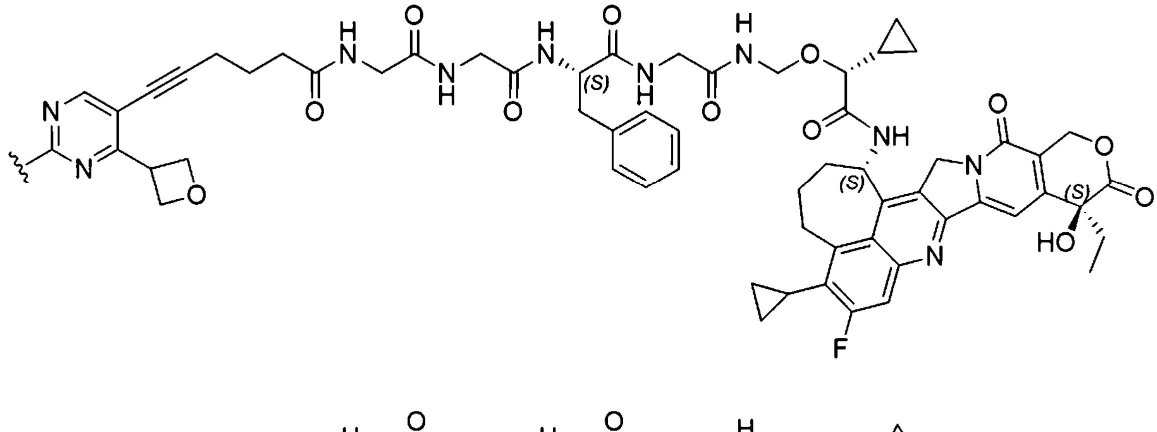

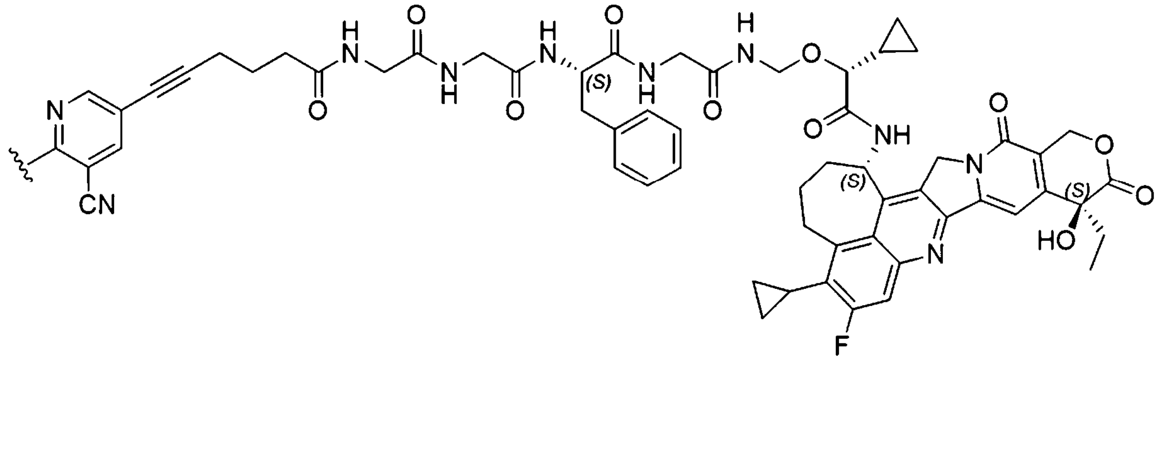

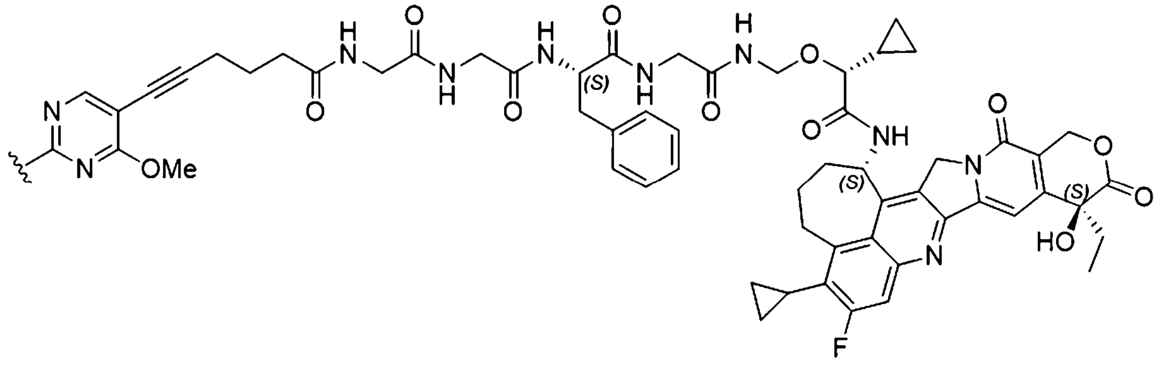

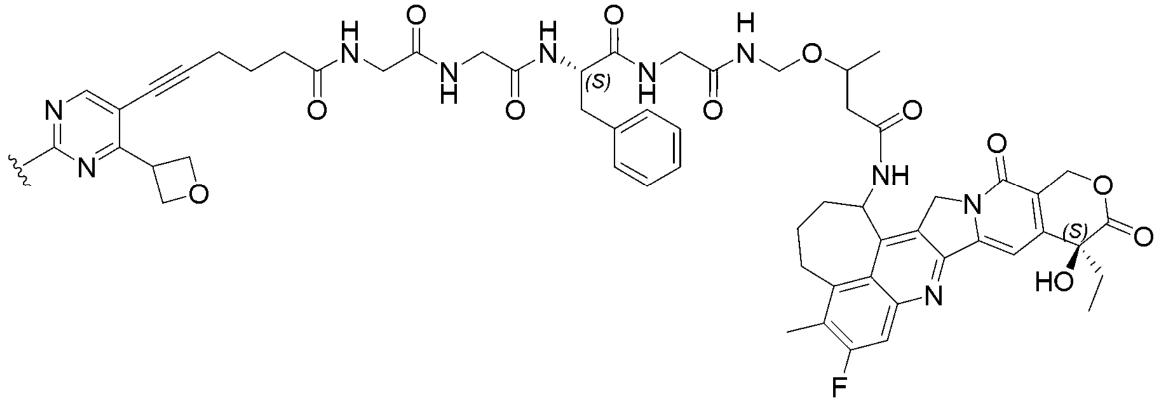

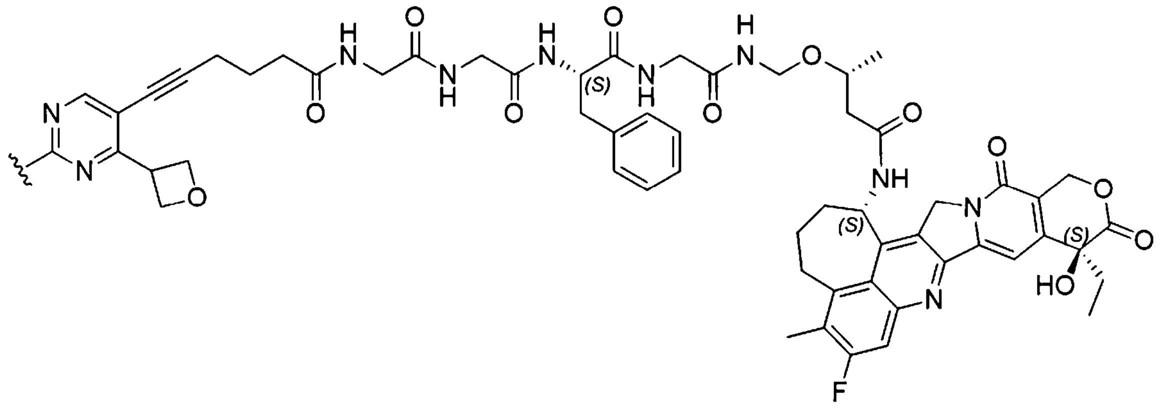

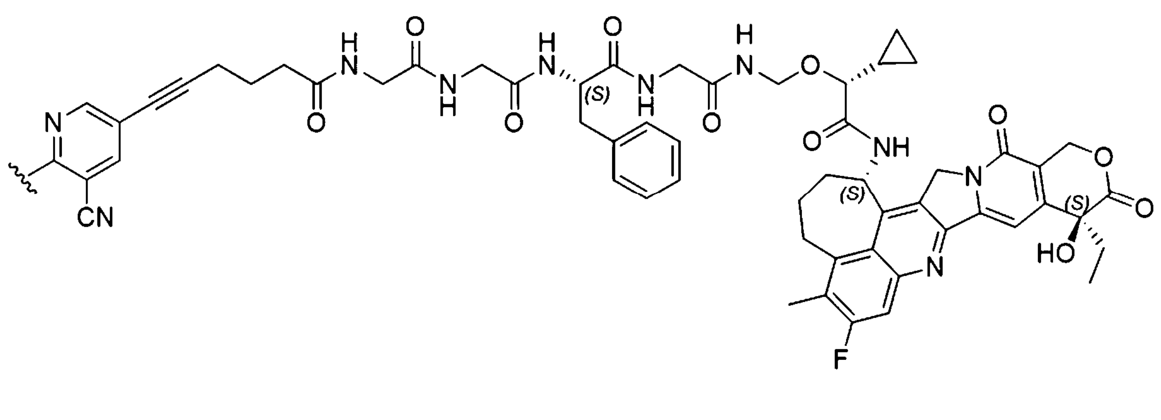

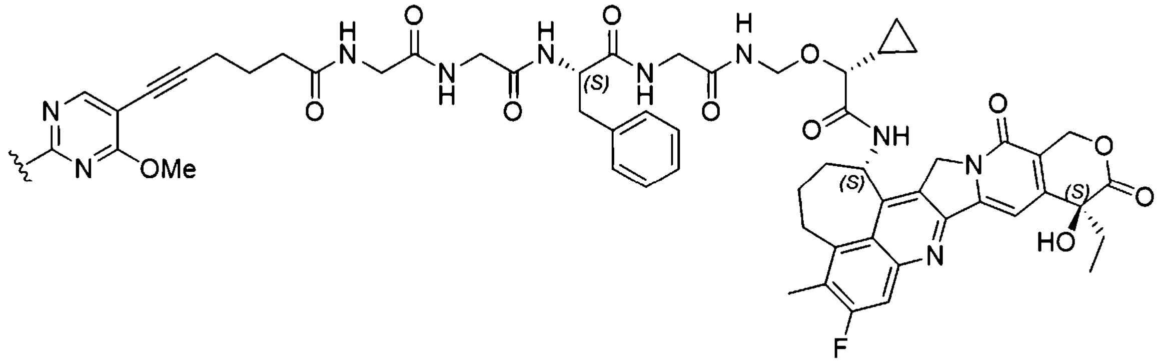

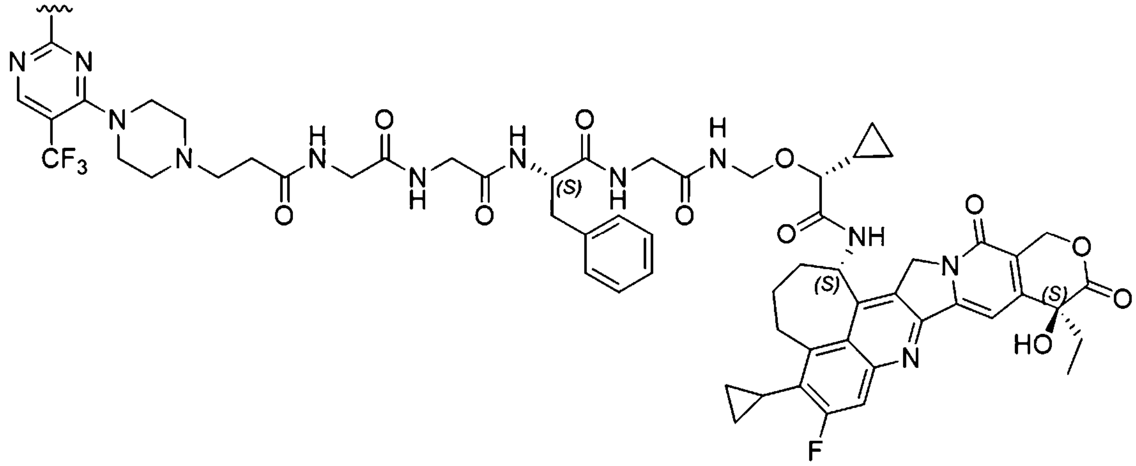

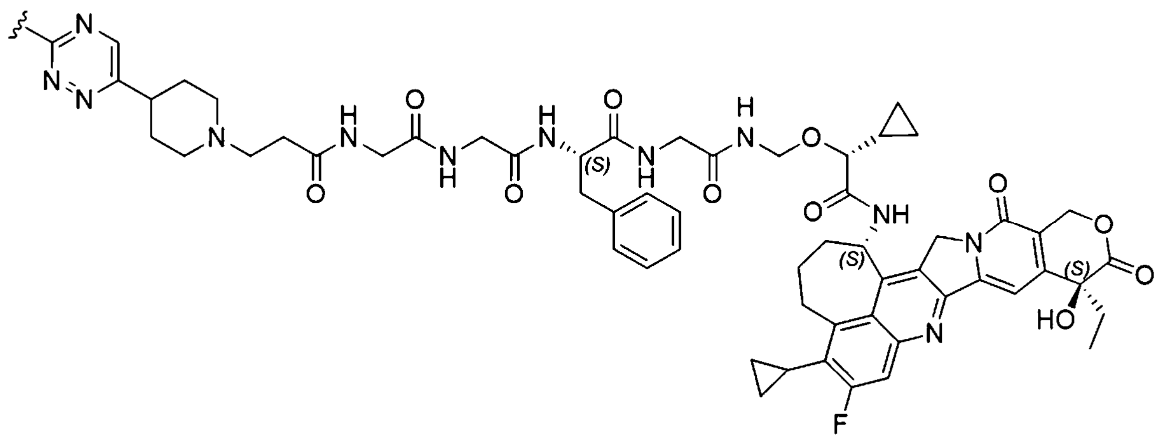

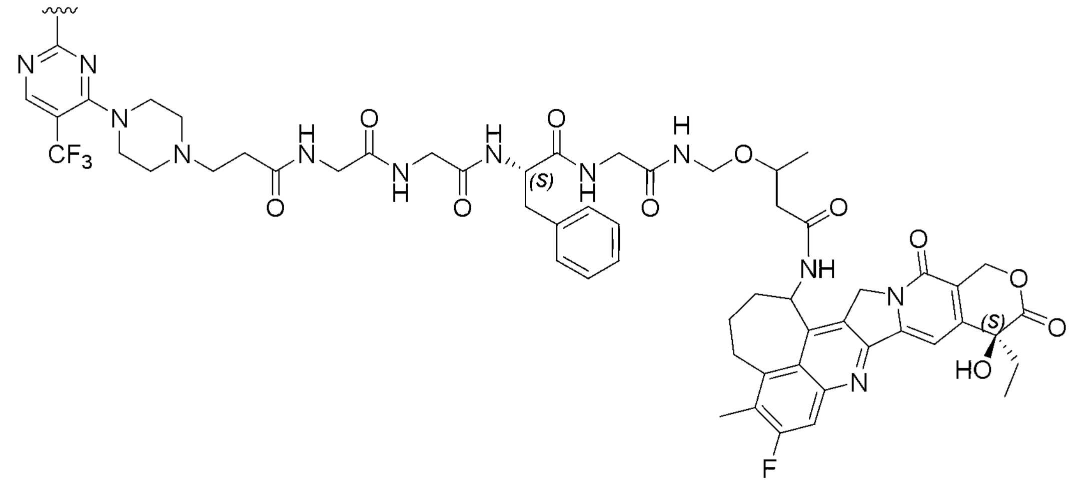

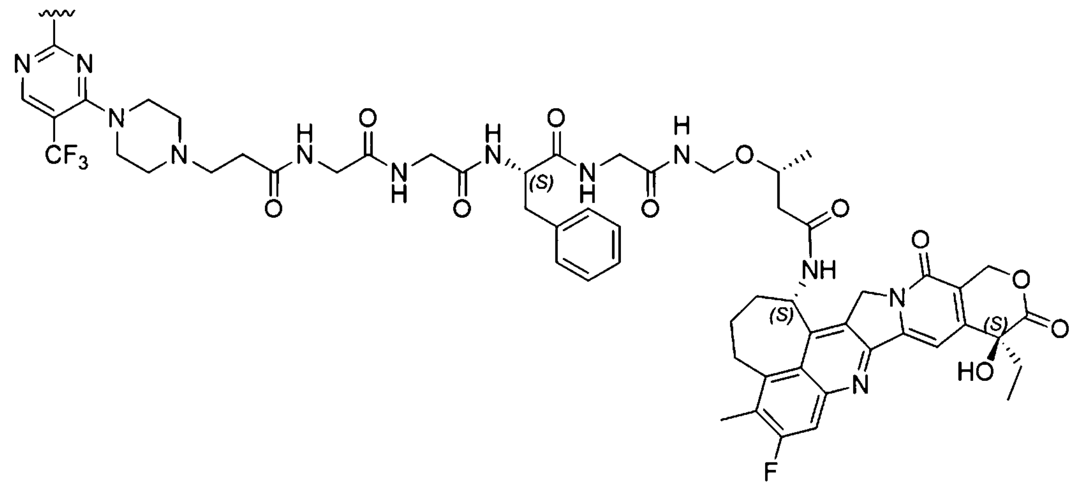

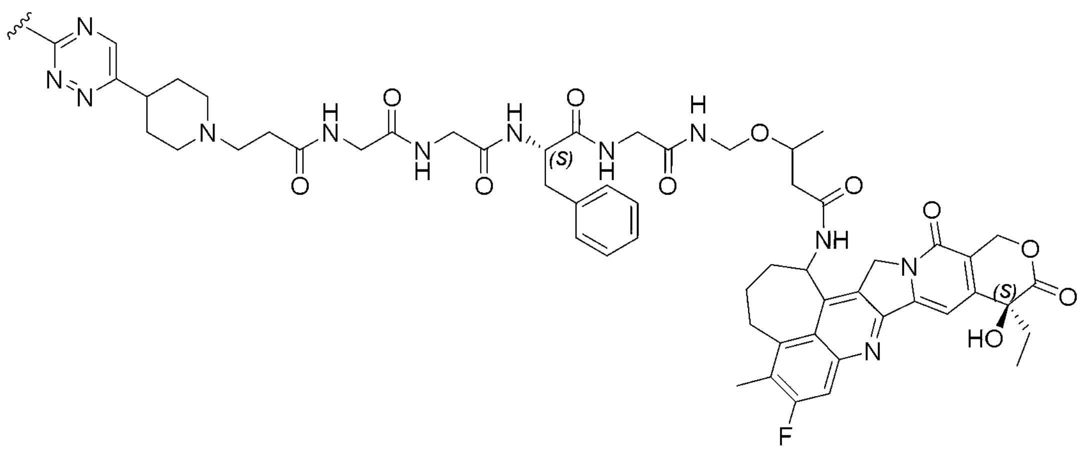

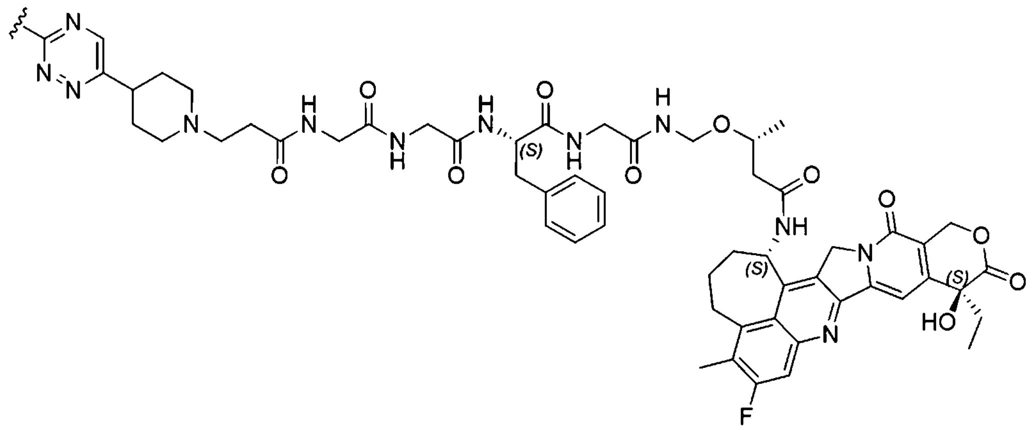

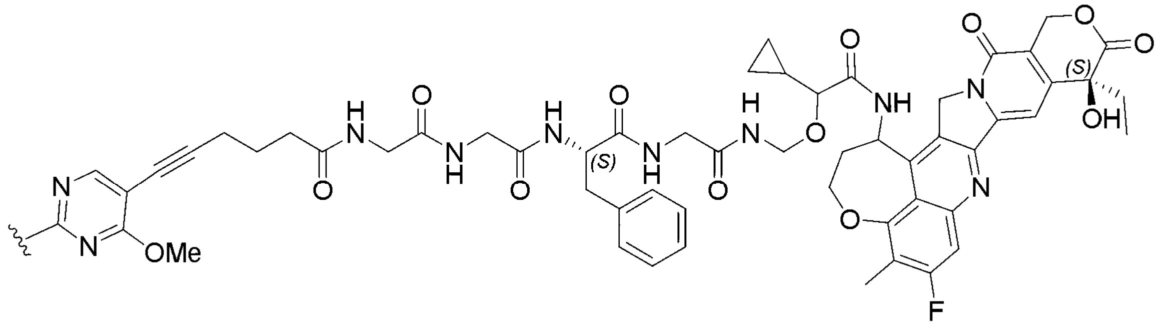

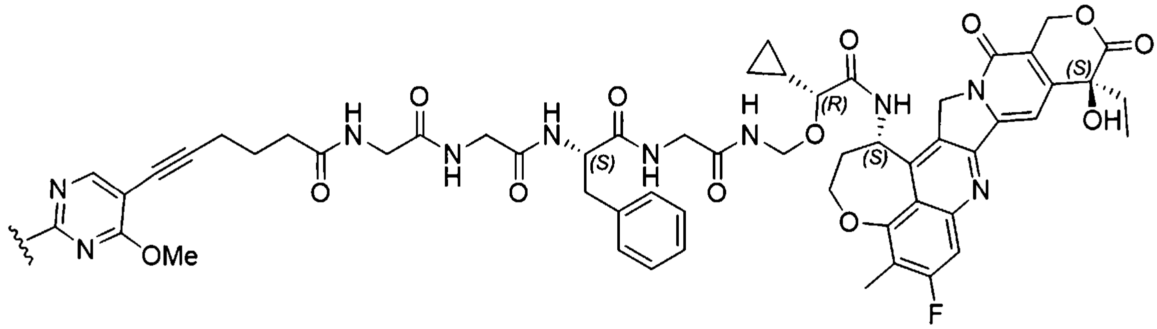

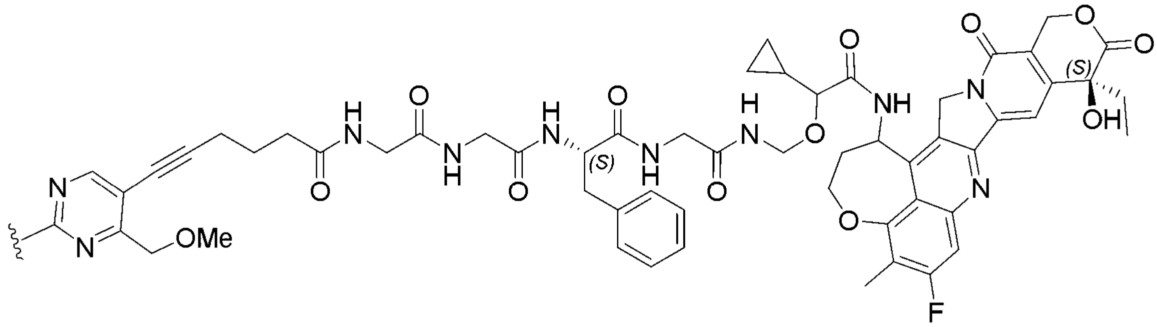

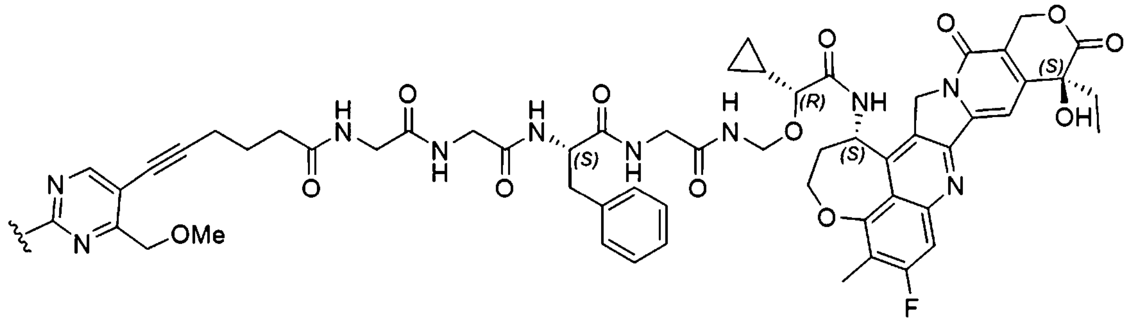

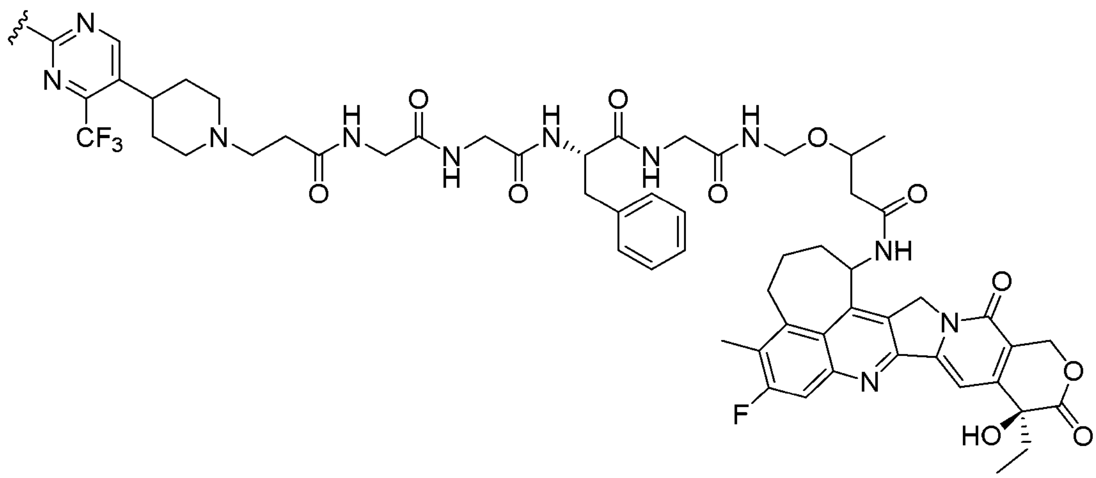

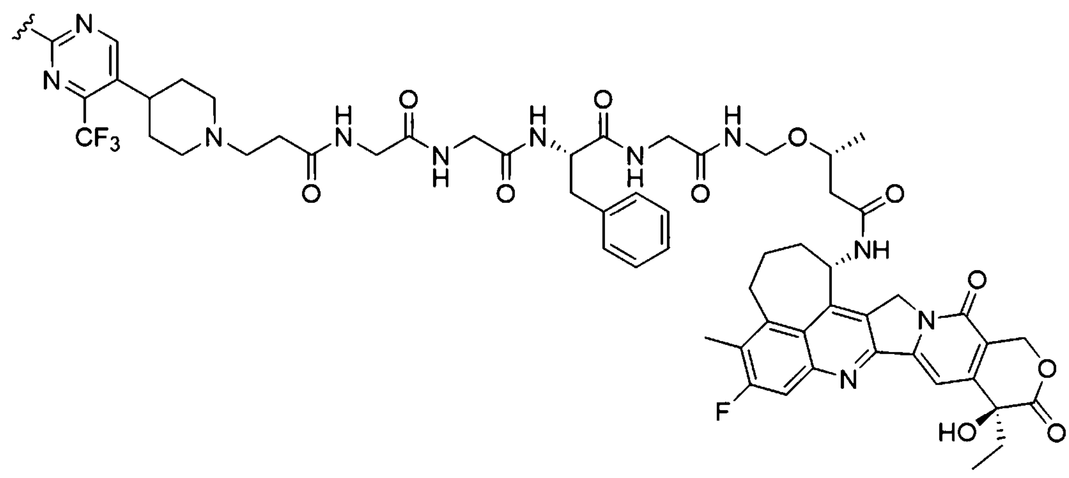

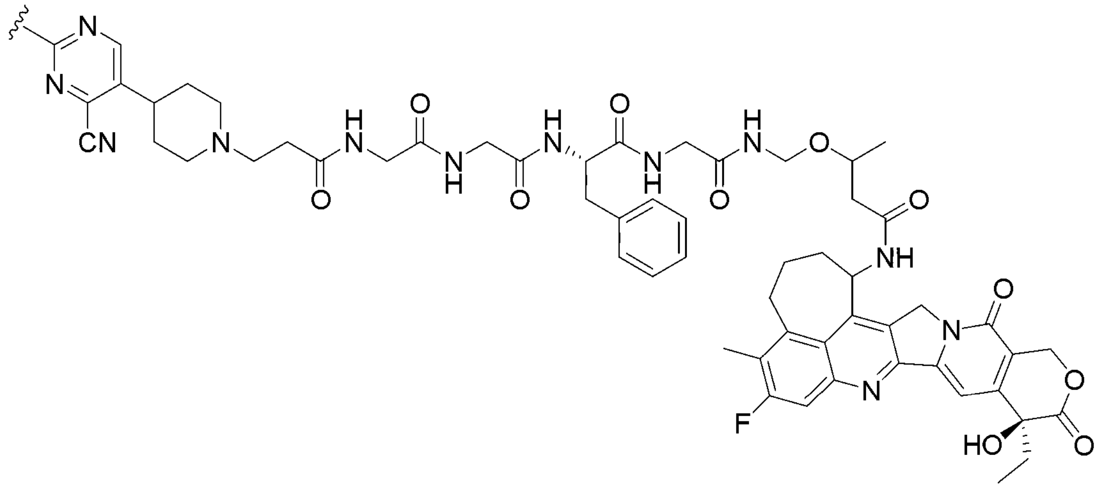

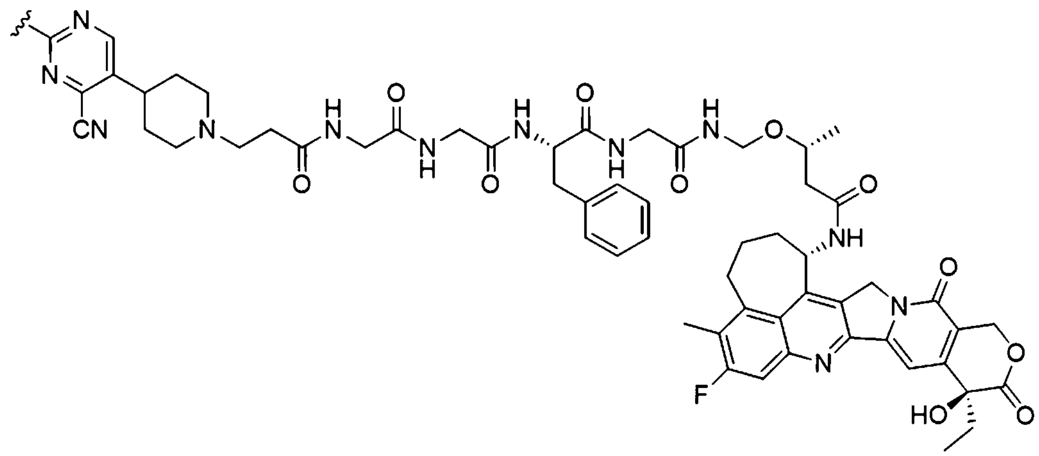

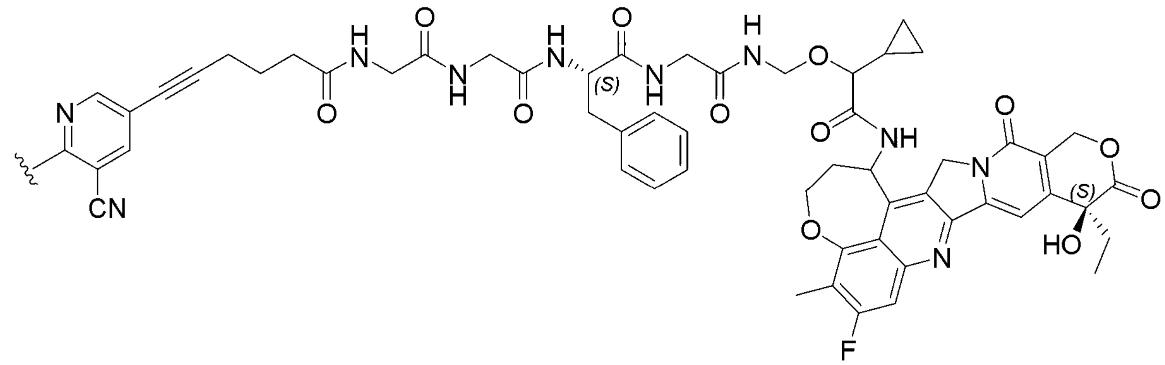

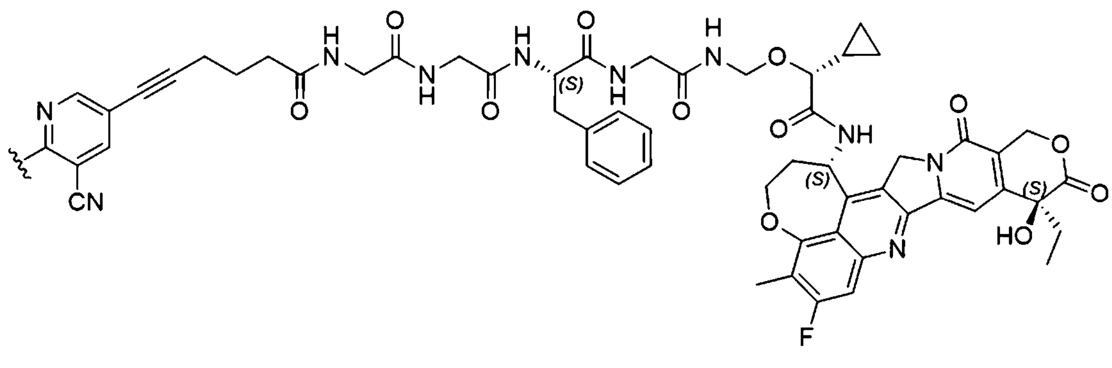

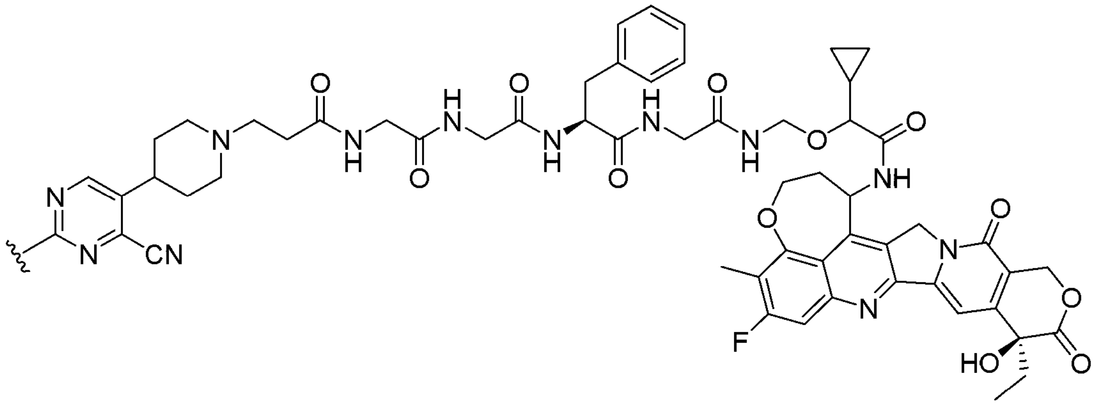

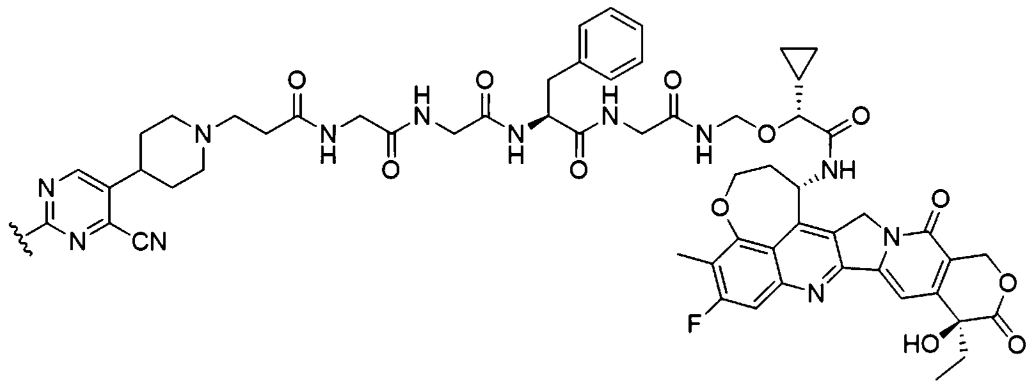

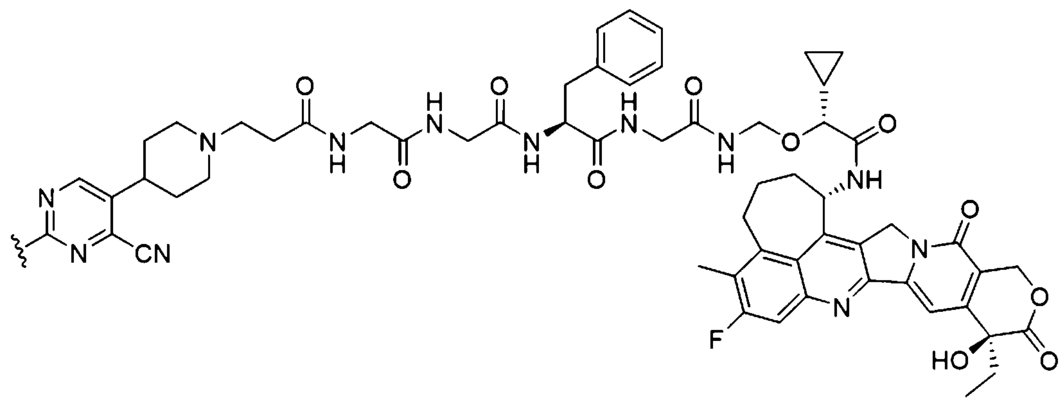

.

12. The antibody-drug conjugate according to claim 10 or 11, wherein the antibody-drug conjugate represented by formula VI is selected from the following structures:

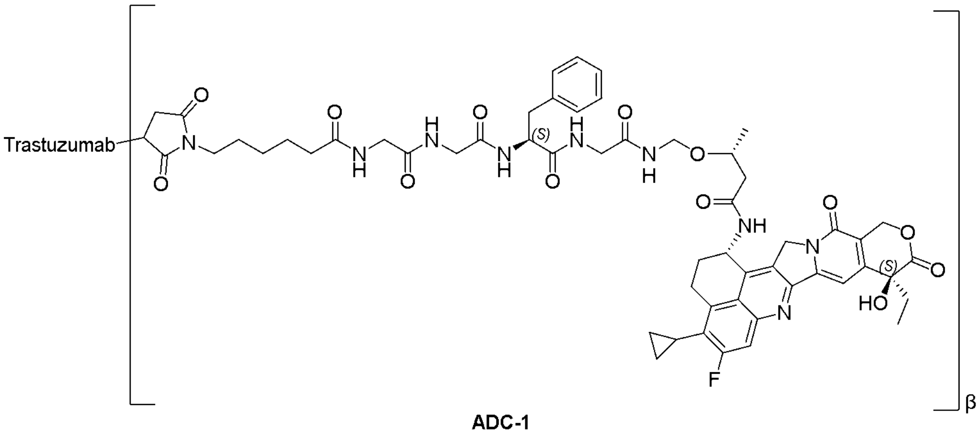

ADC-1

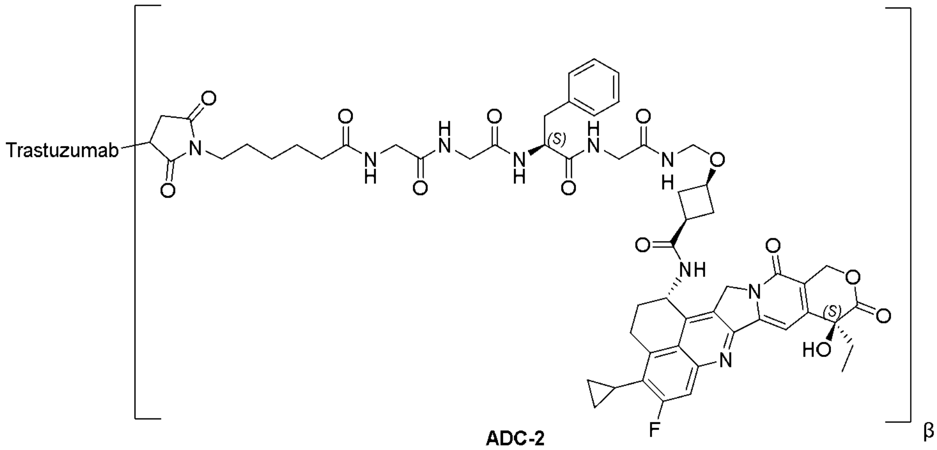

ADC-2

,

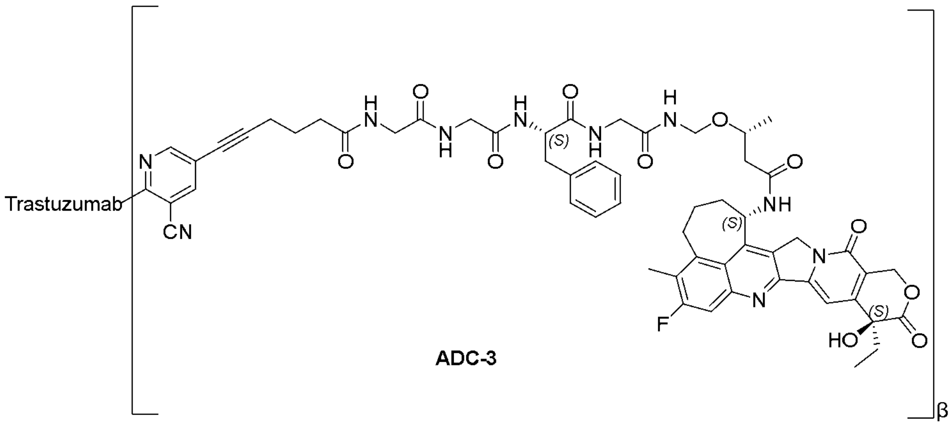

ADC-3

,

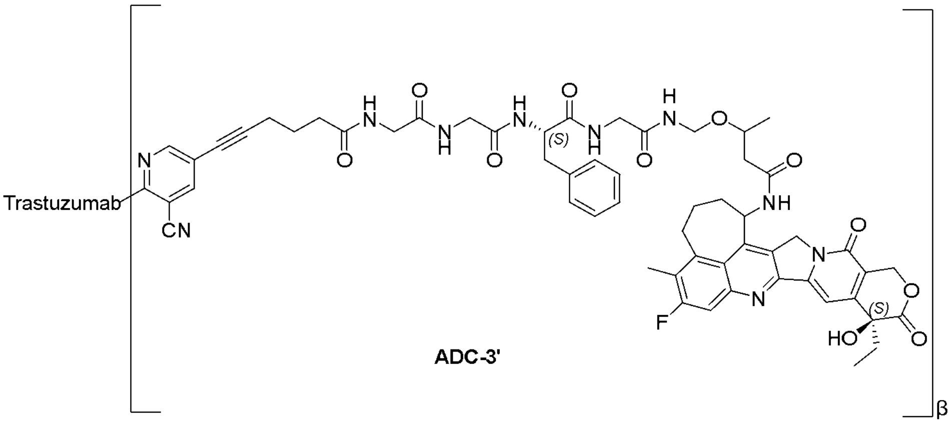

ADC-3',

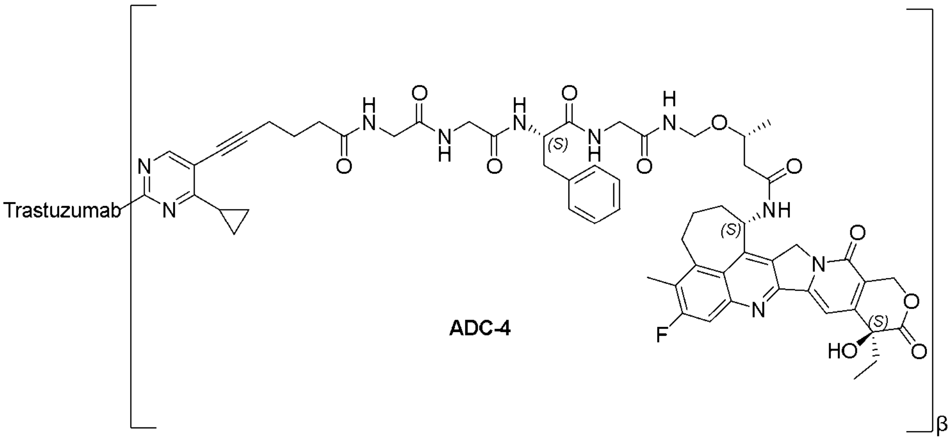

ADC-4,

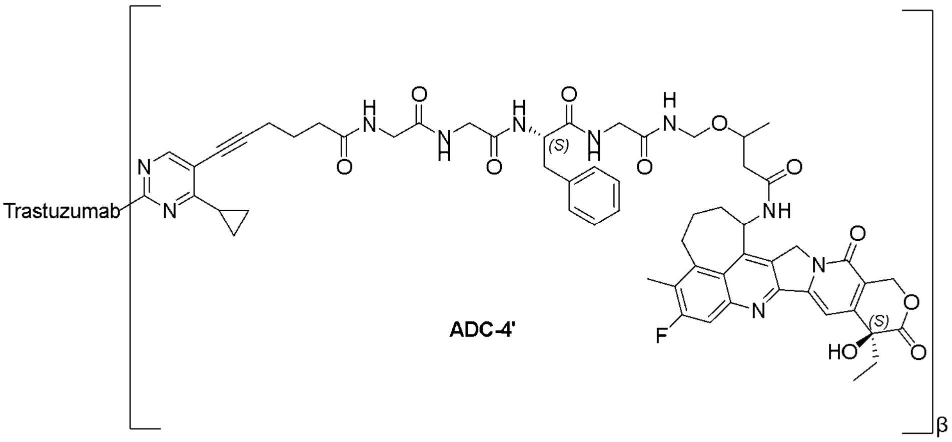

ADC-4',

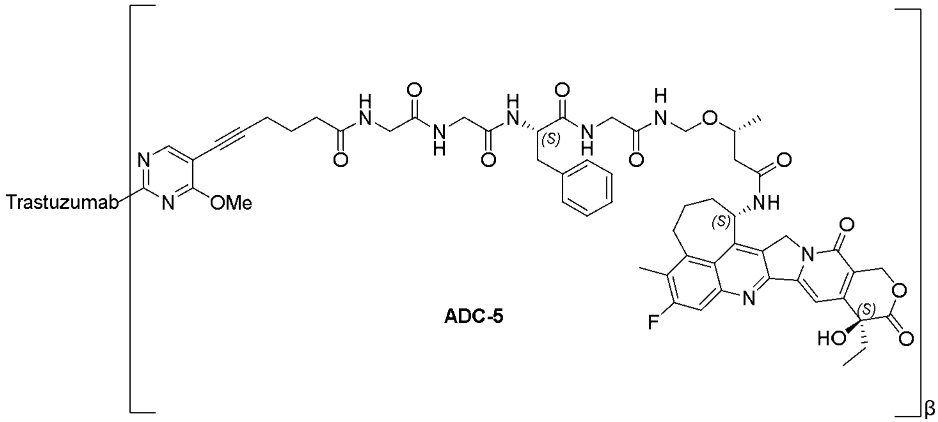

ADC-5

β ,

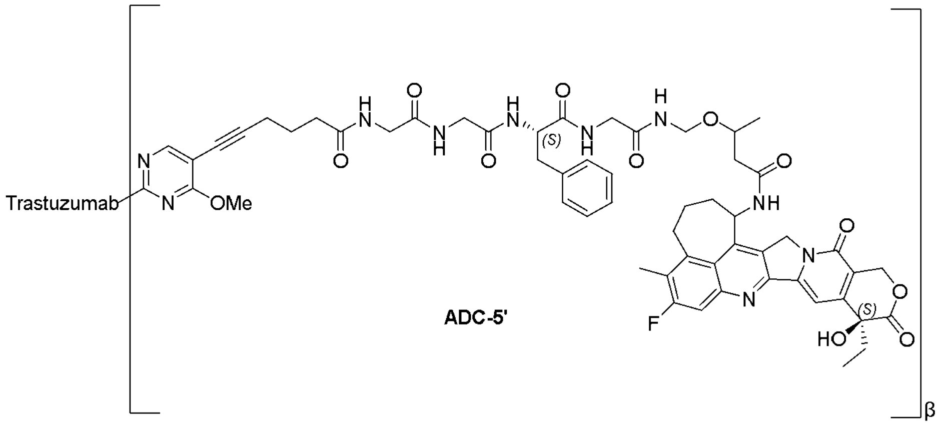

ADC-5'

β ,

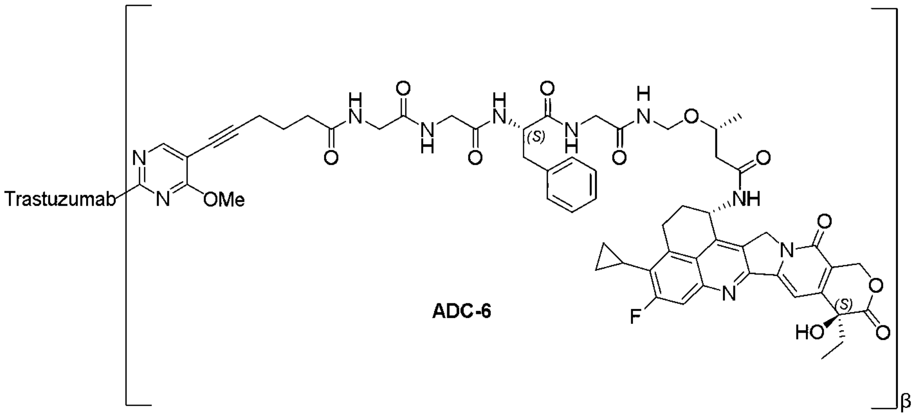

ADC-6

β ,

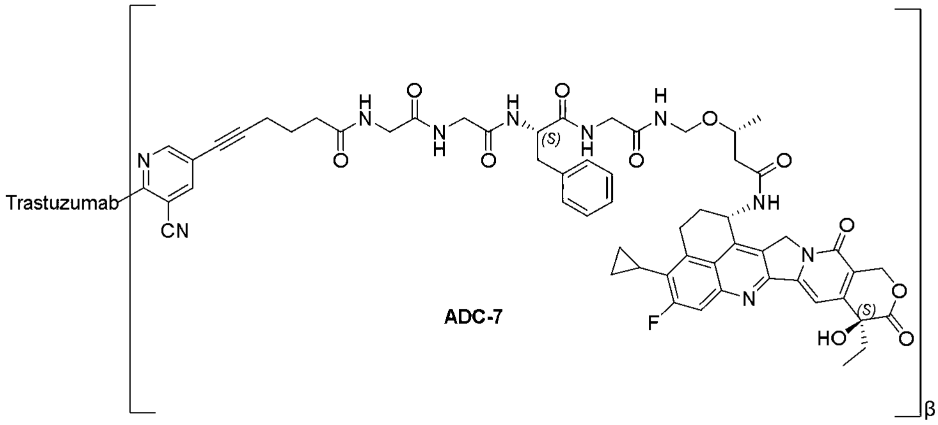

ADC-7

,

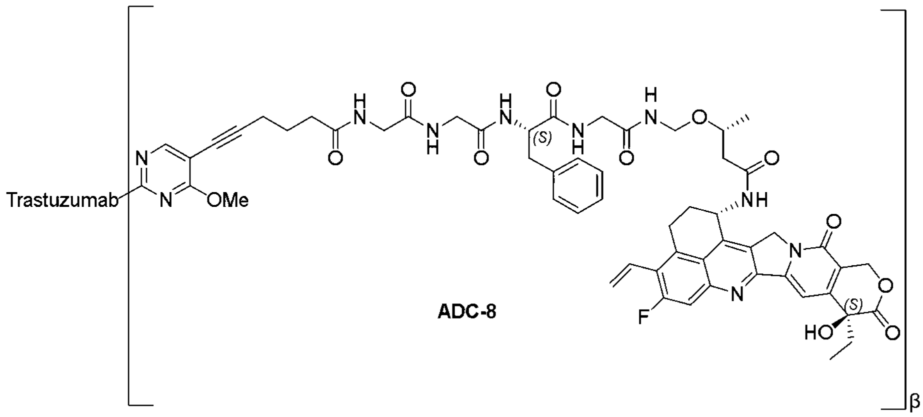

ADC-8

,

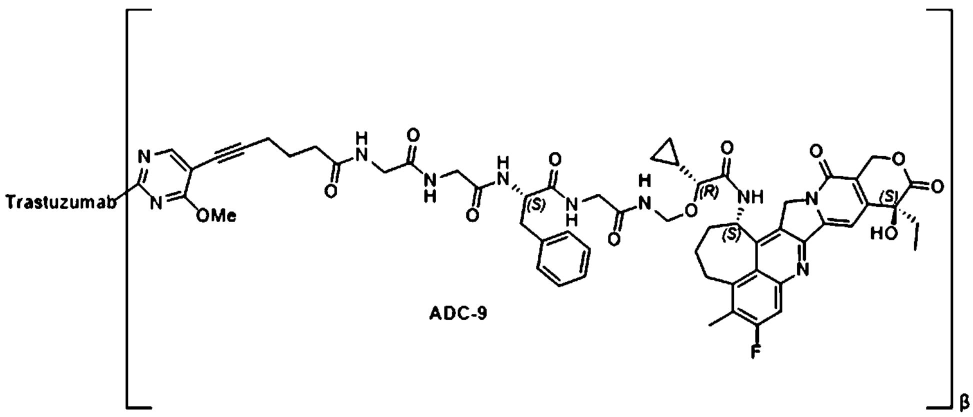

ADC-9

,

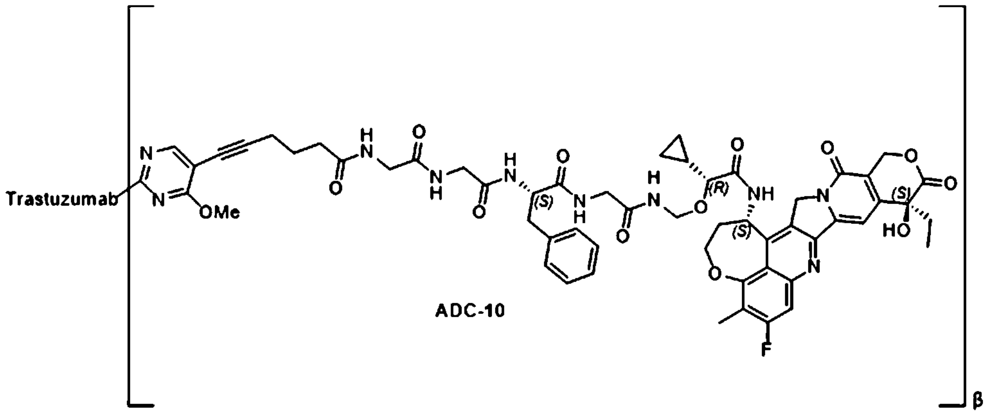

ADC-10

,

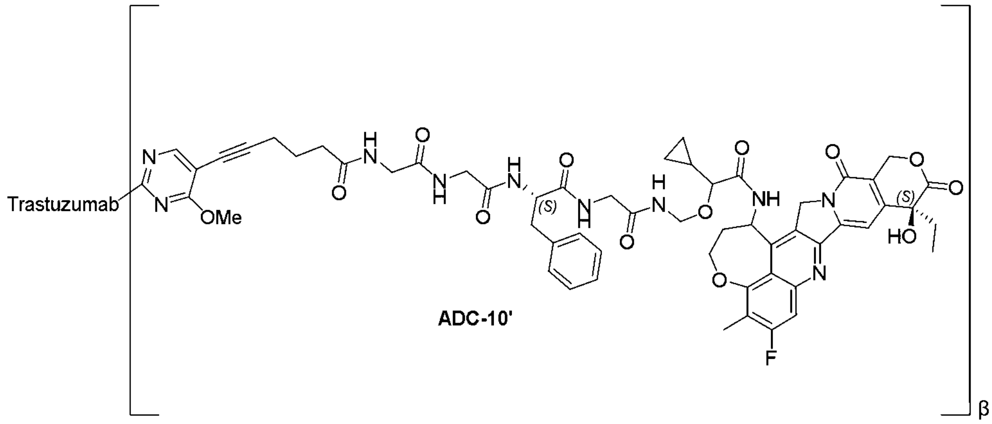

ADC-10'

,

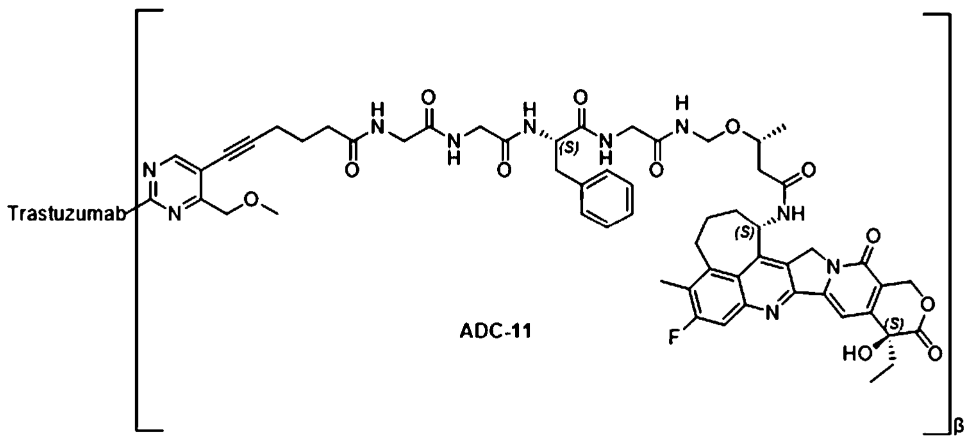

ADC-11

,

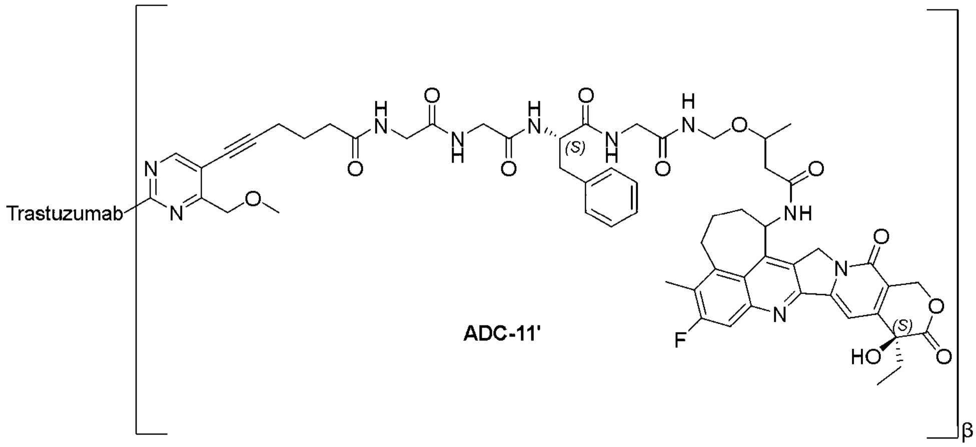

ADC-11' ,

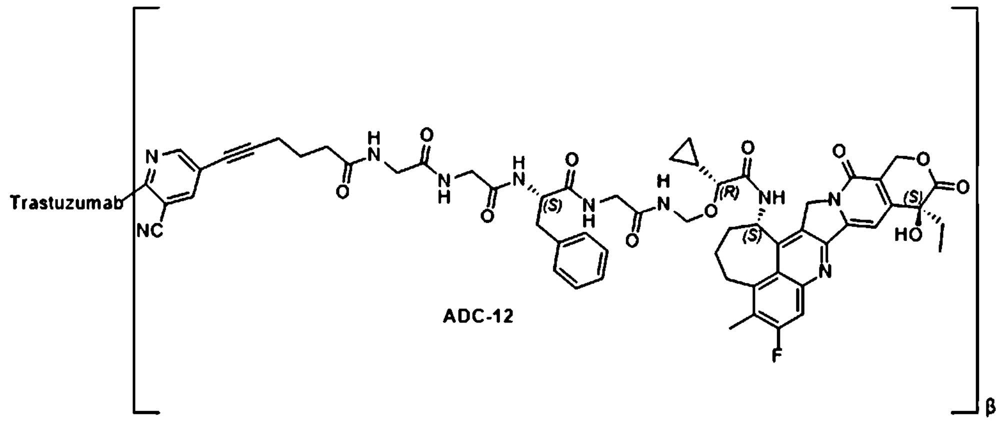

ADC-12 ,

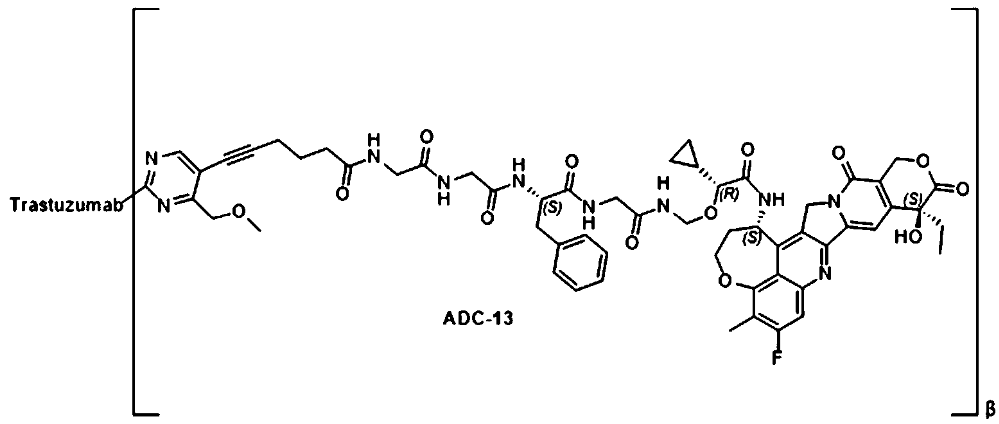

ADC-13 ,

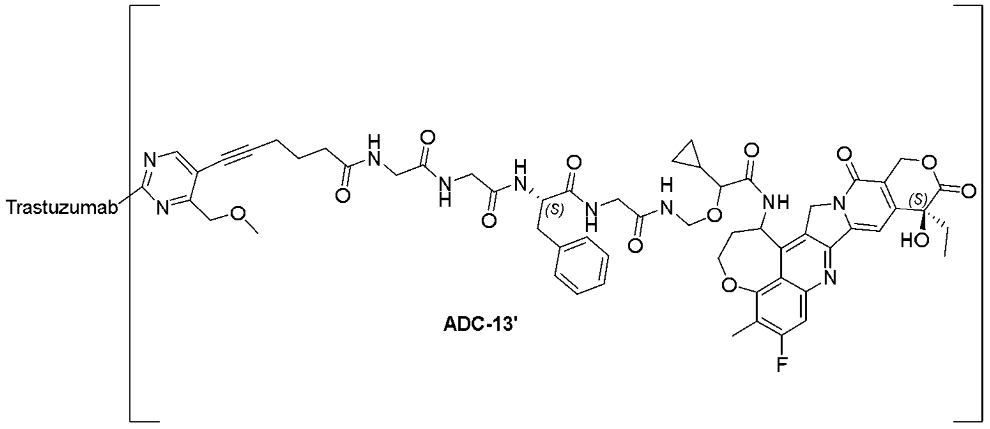

ADC-13' ,

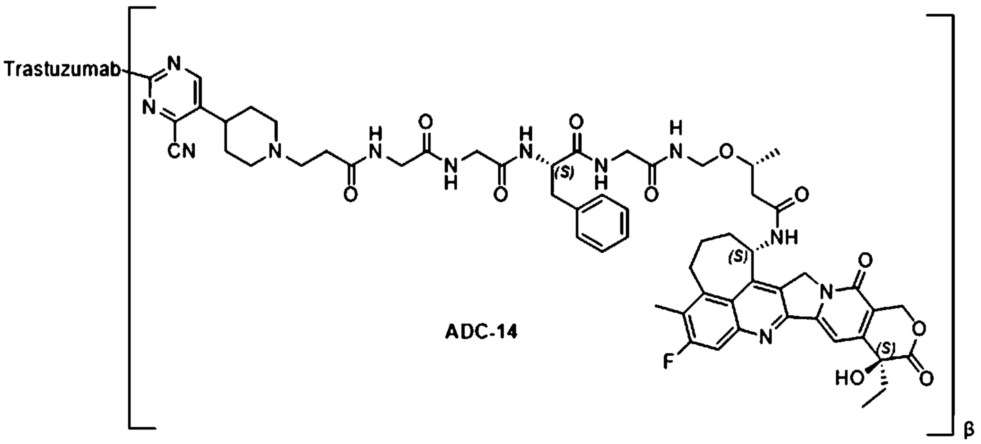

ADC-14 ,

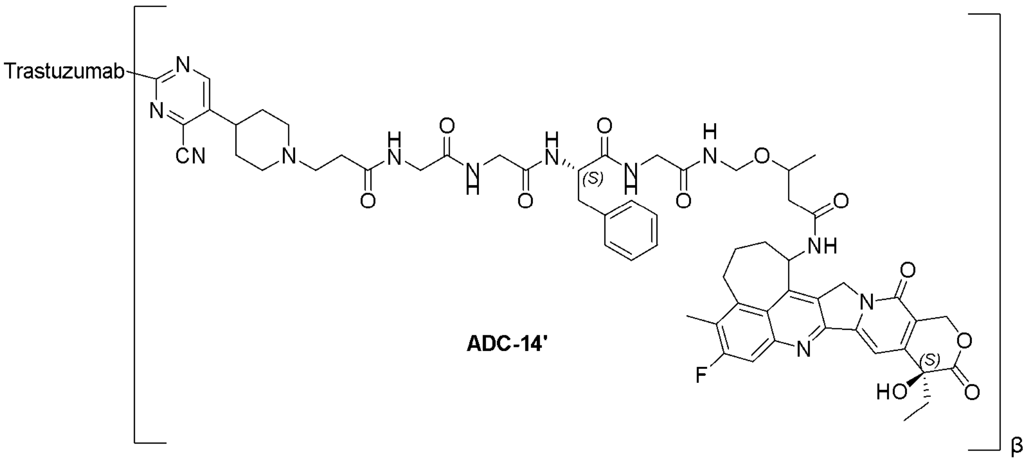

ADC-14' ,

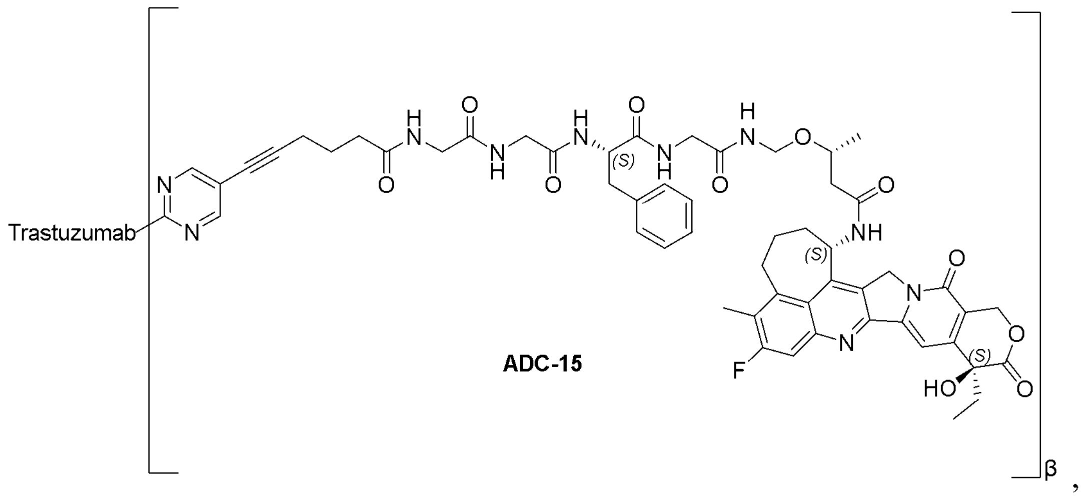

ADC-15

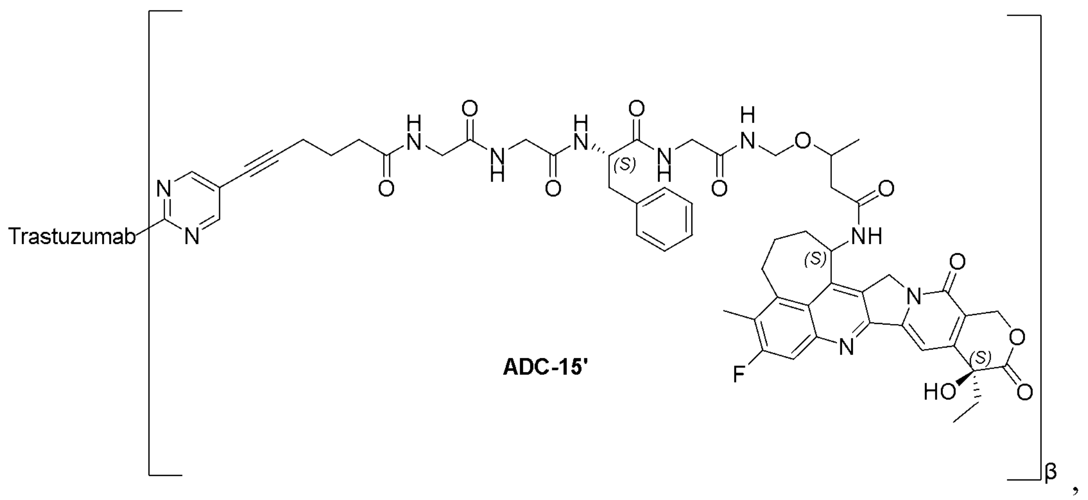

ADC-15'

wherein β is an integer or decimal between 7 and 9.

**13.** A pharmaceutical composition, comprising a therapeutically effective amount of at least one of the compound or antibody-drug conjugate, the racemate, the stereoisomer, the tautomer, the isotope-labeled counterpart, the solvate, the polymorph, the pharmaceutically acceptable salt or the prodrug compound thereof according to any one of claims 1 to 12.

**14.** Use of at least one of the compound or antibody-drug conjugate, the racemate, the stereoisomer, the tautomer, the isotope-labeled counterpart, the solvate, the polymorph, the pharmaceutically acceptable salt or the prodrug compound thereof according to any one of claims 1 to 12, or the pharmaceutical composition according to claim 14 in the manufacture of a topoisomerase I inhibitor and/or in the manufacture of a medicament for preventing or treating a disease or condition associated with topoisomerase I;
preferably, the disease or condition is tumor, comprising breast cancer, gastric cancer, lung cancer, colorectal cancer, large intestine cancer, ovarian cancer, liver cancer, kidney cancer, esophageal cancer, cervical cancer, bladder cancer, pancreatic cancer, prostate cancer, nasopharyngeal carcinoma, melanoma, or leukemia.

**15.** A compound represented by formula I'', a racemate, a stereoisomer, a tautomer, an isotope-labeled counterpart, a solvate, a polymorph, a pharmaceutically acceptable salt or a prodrug compound thereof:

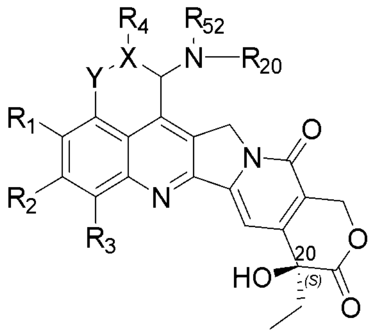

I''

wherein $R_1$, $R_2$, and $R_3$ are the same or different and each independently selected from H, OH, CN, halogen, $C_{1-10}$ alkyl, $C_{2-10}$ alkenyl, $C_{2-10}$ alkynyl, $C_{1-10}$ alkoxy, halo-$C_{1-10}$ alkyl, halo-$C_{1-10}$ alkoxy, cyano-$C_{1-10}$ alkyl, cyano-$C_{1-10}$ alkoxy, or $C_{3-10}$ cycloalkyl;

$R_4$ is selected from H or

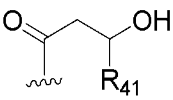 ;

; $R_{41}$ is selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl;

$R_{52}$ is selected from H, $C_{1-6}$ alkyl, $C_{1-6}$ haloalkyl, $C_{1-6}$ alkyl-NH-, $(C_{1-6}$ alkyl$)_2$N-, $C_{1-6}$ alkyl-NH-$C_{1-6}$ alkyl, $(C_{1-6}$ alkyl$)_2$N-$C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl;

$R_{20}$ is selected from H or an amino protecting group;

preferably, $R_{20}$ is selected from Fmoc, Boc, Bn, or Cbz;

X is selected from CH or N;

Y is selected from $-(CH_2)_m-O-(CH_2)_p-$;

m is an integer selected from 0-6;

p is an integer selected from 0-6;

preferably, $R_1$ is selected from H, OH, CN, halogen, $C_{1-6}$ alkyl, $C_{2-6}$ alkenyl, $C_{2-6}$ alkynyl, $C_{3-6}$ cycloalkyl or halo-$C_{1-6}$ alkoxy;

preferably, $R_1$ is selected from H, OH, Br, methyl, difluoromethoxy, 2,2,2-trifluoroethoxy, vinyl, cyclopropyl, or ethynyl;

preferably, $R_2$ is selected from H, halogen, CN, or $C_{1-6}$ alkyl;

preferably, $R_2$ is selected from H or F;

preferably, $R_3$ is selected from H or $C_{1-6}$ alkyl;

preferably, $R_3$ is H;

$R_4$ is selected from H;

preferably, the formula I" is the following compounds:

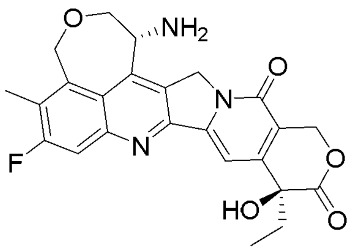 , 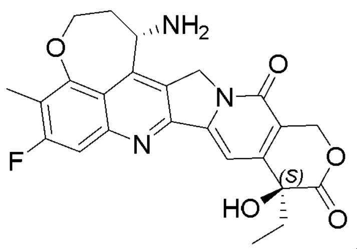 , 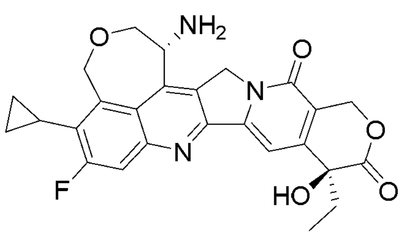 ,

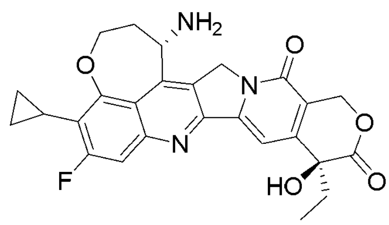
,
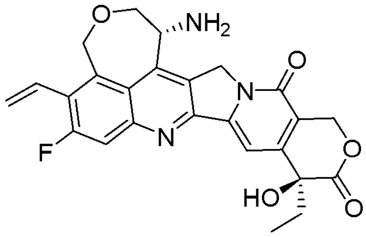
,
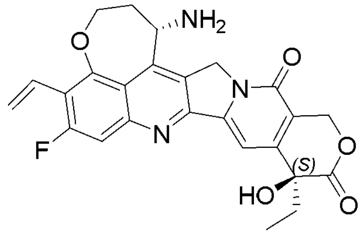
,
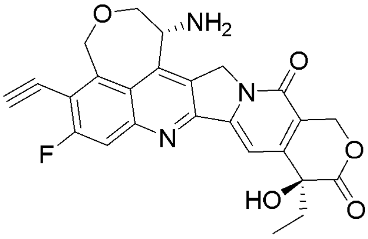
,
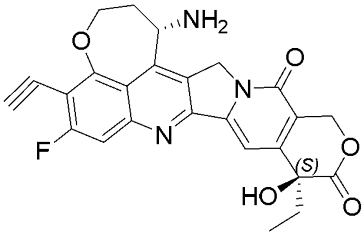
,
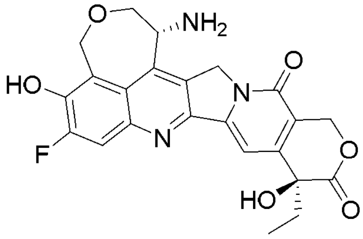
,
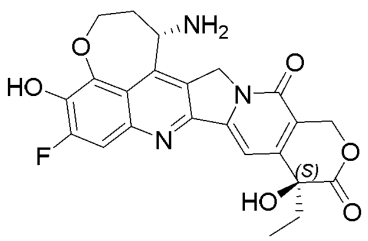
,
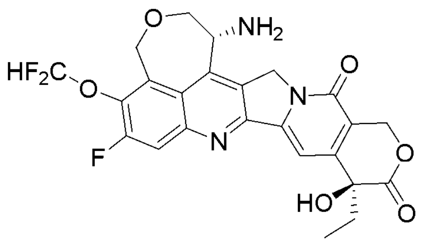
,
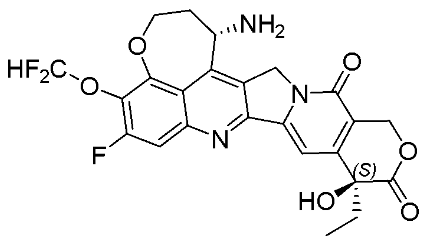
,
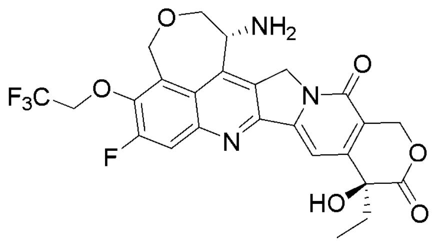
,
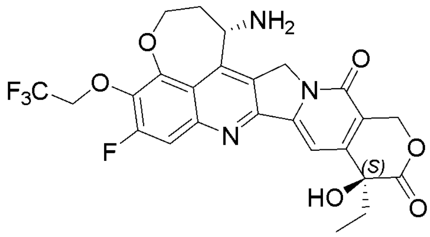
,
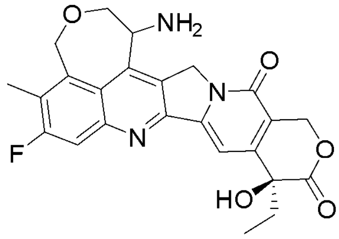
,
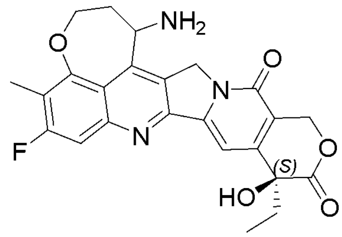
,
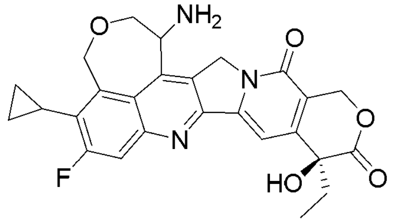
,
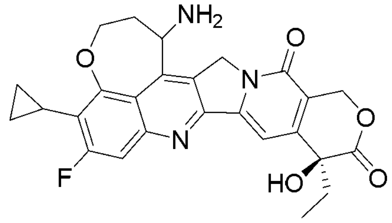
,

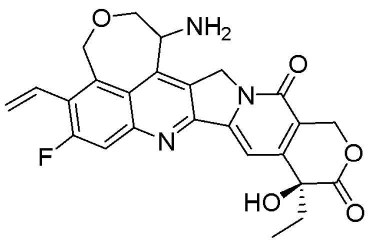,
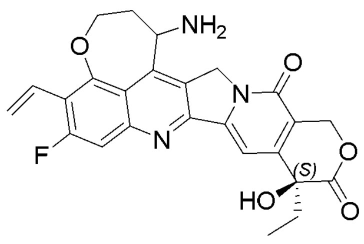,
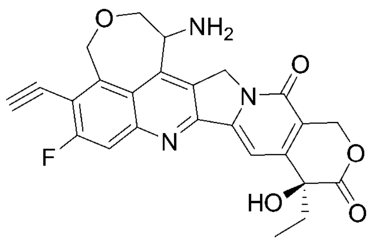,

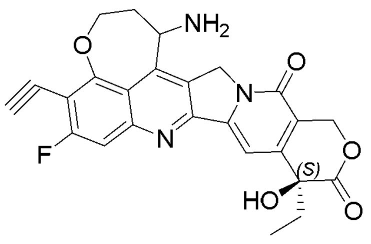,
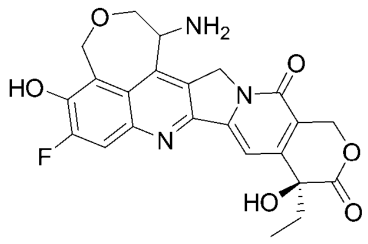,
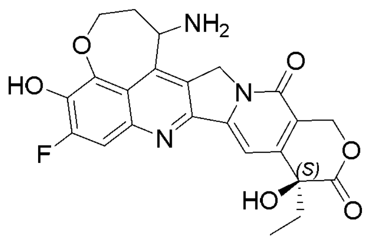,

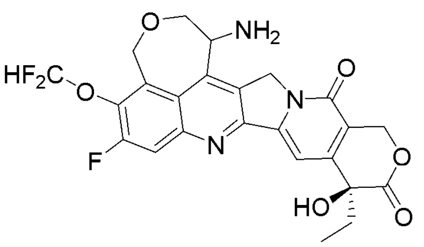,
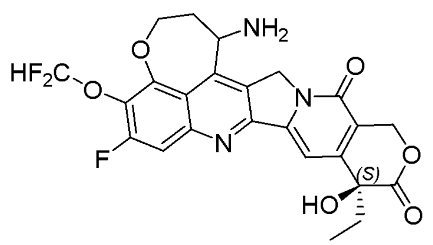,

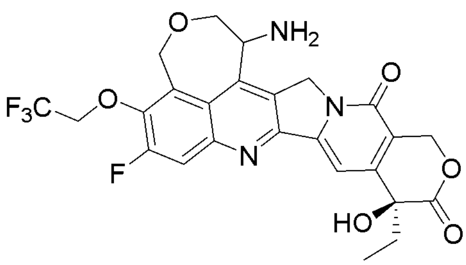, or
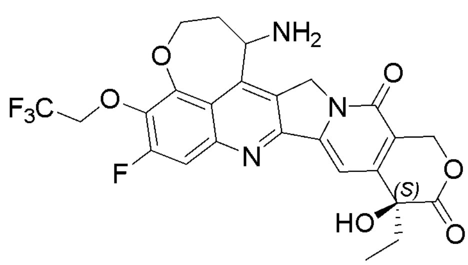.

**16.** A compound represented by formula MII

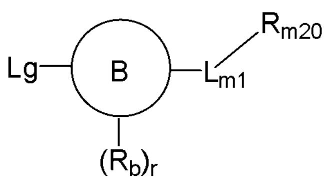

MII

wherein Lg is a leaving group, and the leaving group is selected from halogen, sulfone, trifluoromethanesulfonyl or methanesulfonyl; preferably, Lg is methanesulfonyl;

ring B is selected from a 5-6-membered N-containing heteroaromatic ring or a 3-6 membered N-containing heterocyclic ring;

more preferably, ring B is selected from a pyrimidine ring, a pyridine ring or a triazine ring;

each $R_b$ is the same or different and each independently selected from the following groups: halogen, cyano, oxo (=O), $C_{1-6}$ alkyl, halo-$C_{1-6}$ alkyl, hydroxy-$C_{1-6}$ alkyl, $C_{1-6}$ alkoxy or $C_{3-8}$ cycloalkyl, 3-8-membered heterocyclyl, $C_{1-6}$ alkyl-O-$C_{1-6}$ alkyl-, or $C_{1-6}$ alkyl-(5-6-membered) heteroaryl;

preferably, each $R_b$ is the same or different and each independently selected from cyano, oxo (=O), methoxy,

cyclopropyl, trifluoromethyl,

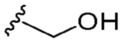, 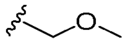, 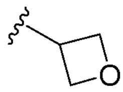 or 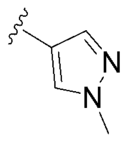;

$L_{m1}$ is absent, or is selected from the following groups which are unsubstituted or optionally substituted with one, two or more $R_{m1}$: $C_{6-14}$ aryl, 5-14-membered heteroaryl, or 3-14-membered heterocyclyl; each $R_{m1}$ is the same or different and each independently selected from H, halogen, cyano, $C_{1-6}$ alkyl, or HOOC-$C_{1-3}$ alkylene;
preferably, $L_{m1}$ is absent or is selected from the following groups which are unsubstituted or optionally substituted with one, two or more $R_{m1}$: phenyl, piperidinyl or piperazinyl;
more preferably, $L_{m1}$ is selected from

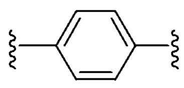, 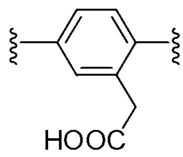, 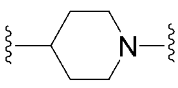 or

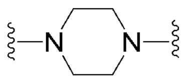;

$R_{m20}$ is selected from the following groups which are unsubstituted or optionally substituted with one, two, or more $R_{m2}$: $-(CH_2)_s-(C=O)-R_Z$, or -ethynyl-$(CH_2)_t-(C=O)-R_Z$;
preferably, is selected from the following groups which are unsubstituted or optionally substituted with one, two, or more $R_{m2}$: $-CH_2-(C=O)-R_Z$, $-(CH_2)_2-(C=O)-R_Z$, $-(CH_2)_5-(C=O)-R_Z$, or -ethynyl-$(CH_2)_3-(C=O)-R_Z$;
each $R_{m2}$ is the same or different and each independently selected from H, halogen, cyano, $C_{1-6}$ alkyl or $-C_{1-6}$ alkylene-COOH, wherein the alkylene is optionally interrupted by one, two or more of the following groups: O, or NH;
preferably, each $R_{m2}$ is the same or different and each independently selected from $-C_{1-3}$ alkylene-COOH, and the alkylene is optionally interrupted by O or NH;
preferably, $R_{m2}$ is selected from

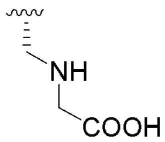, 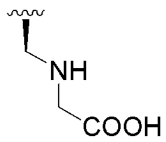, 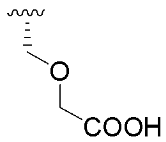, or 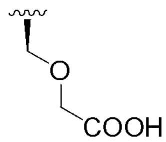;

r is an integer selected from 0-6;
s is an integer selected from 0-6;
t is an integer selected from 0-6;
Rz is selected from hydroxyl, halogen, an active ester, a carboxyl protecting group, an amino acid, a peptide fragment or a hydrophilic fragment;
the amino acid is the N-terminal amino acid of $L_1$, and the peptide fragment is a sub fragment of $L_1$ formed from 2, 3 or 4 amino acids at the N-terminus or is $L_1$;
the C-terminus of the peptide fragment is hydroxyl, an active ester, a carboxyl protecting group or

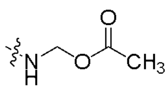;

preferably, the hydrophilic fragment comprises a polyhydroxy group, a polyethylene glycol fragment, a polybetaine fragment or a polycreatine fragment;
preferably, the formula MII is the following formula MII-1:

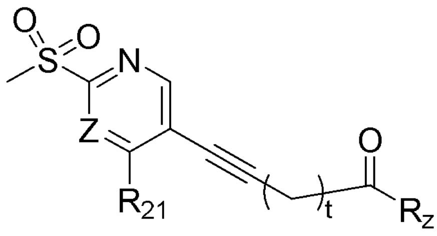

(MII-1)

wherein t and Rz are as defined above; Z is N or $CR_{22}$; $R_{21}$ and $R_{22}$ are each independently selected from H, halogen, cyano, oxo (=O), $C_{1-6}$ alkyl, halo-$C_{1-6}$ alkyl, hydroxy-$C_{1-6}$ alkyl, $C_{1-6}$ alkoxy, $C_{3-8}$ cycloalkyl, 3-8-membered heterocyclyl, or $C_{1-6}$ alkyl-O-$C_{1-6}$ alkyl; provided that when Z is N, $R_{21}$ is not H;
preferably, Rz is OH or halogen; t is an integer of 2-4; Z is N; and $R_{21}$ is selected from cyano, $C_{1-6}$ alkoxy, $C_{3-4}$ cycloalkyl or -$C_{1-6}$ alkyl-O-$C_{1-6}$ alkyl; or $R_{21}$ is H; Z is $CR_{22}$; and $R_{22}$ is cyano or trifluoromethyl;
preferably, the formula MII is the following compounds:

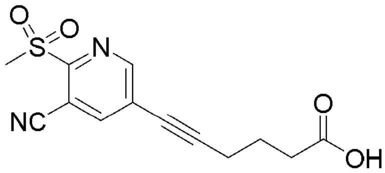
,
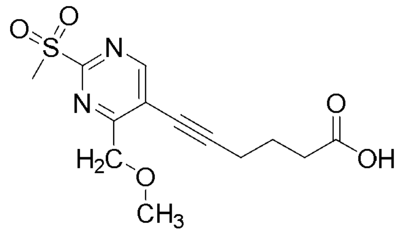
,
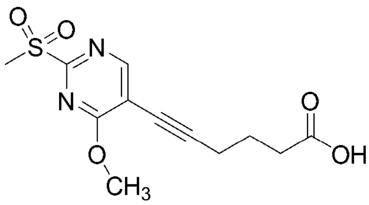
,
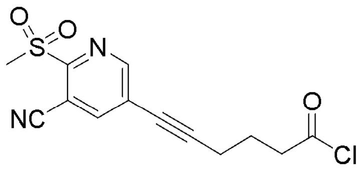
,
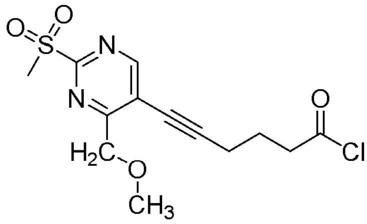
,

or

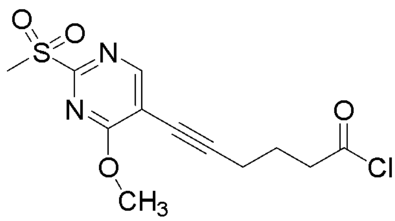
.

**17.** A compound represented by formulas (L'-1) to (L'-3):

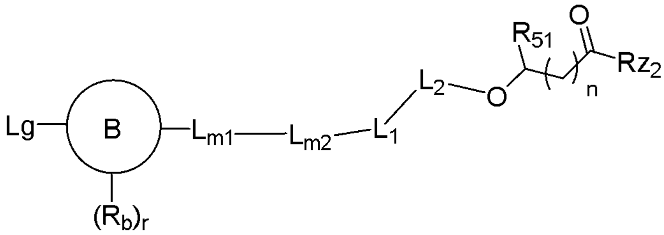

(L'-1)

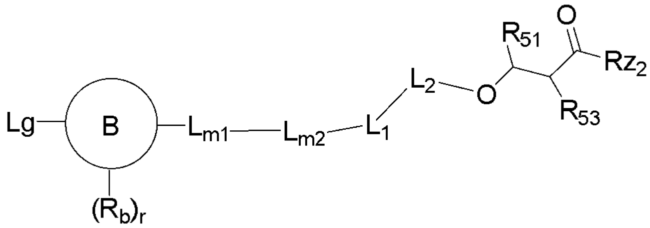

(L'-2)

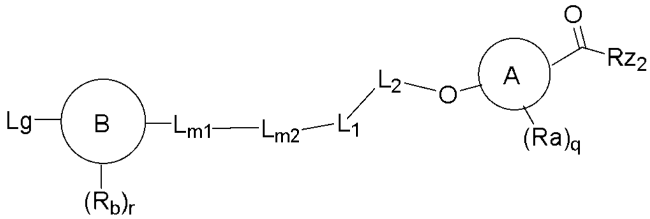

(L'-3)

wherein Lg is a leaving group, and the leaving group is selected from halogen, sulfone, trifluoromethanesulfonyl or methanesulfonyl; preferably, Lg is methanesulfonyl;
ring B is selected from a 5-6-membered N-containing heteroaromatic ring or a 3-6 membered N-containing heterocyclic ring;
more preferably, ring B is selected from a pyrimidine ring, a pyridine ring or a triazine ring;
each $R_b$ is the same or different and each independently selected from the following groups: halogen, cyano, oxo (=O), $C_{1-6}$ alkyl, halo-$C_{1-6}$ alkyl, hydroxy-$C_{1-6}$ alkyl, $C_{1-6}$ alkoxy or $C_{3-8}$ cycloalkyl, 3-8-membered heterocyclyl, $C_{1-6}$ alkyl-O-$C_{1-6}$ alkyl-, or $C_{1-6}$ alkyl-(5-6-membered) heteroaryl;
preferably, each $R_b$ is the same or different and each independently selected from cyano, oxo (=O), methoxy, cyclopropyl, trifluoromethyl,

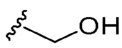 , 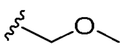 , 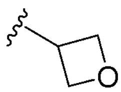 or 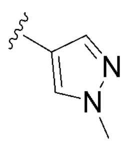 ;

$L_{m1}$ is absent, or is selected from the following groups which are unsubstituted or optionally substituted with one, two or more $R_{m1}$: $C_{6-14}$ aryl, 5-14-membered heteroaryl, or 3-14-membered heterocyclyl; each $R_{m1}$ is the same or different and each independently selected from H, halogen, cyano, $C_{1-6}$ alkyl, or HOOC-$C_{1-3}$ alkylene;
preferably, $L_{m1}$ is absent or is selected from the following groups which are unsubstituted or optionally substituted with one, two or more $R_{m1}$: phenyl, piperidinyl or piperazinyl;
more preferably, $L_{m1}$ is selected from

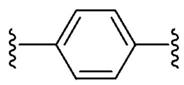, 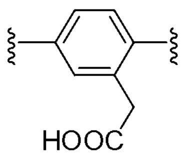, 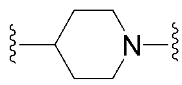 or

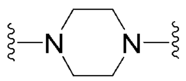;

$L_{m2}$ is selected from the following groups which are unsubstituted or optionally substituted with one, two, or more $R_{m2}$: $-(CH_2)_s-(C=O)-$, or -ethynyl-$(CH_2)_t-(C=O)-$;

preferably, $L_{m2}$ is selected from the following groups which are unsubstituted or optionally substituted with one, two, or more $R_{m2}$: $-CH_2-(C=O)-$, $-(CH_2)_2-(C=O)-$, $-(CH_2)_5-(C=O)-$, or - ethynyl-$(CH_2)_3-(C=O)-$;

each $R_{m2}$ is the same or different and each independently selected from H, halogen, cyano, $C_{1-6}$ alkyl or $-C_{1-6}$ alkylene-COOH, wherein the alkylene is optionally interrupted by one, two or more of the following groups: O, or NH;

preferably, each $R_{m2}$ is the same or different and each independently selected from $-C_{1-3}$ alkylene-COOH, and the alkylene is optionally interrupted by O or NH;

preferably, $R_{m2}$ is selected from

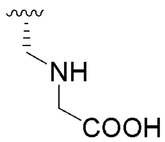, 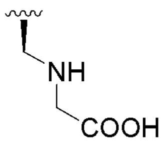, 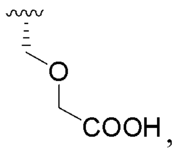, or 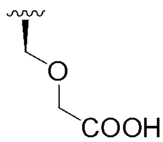;

r is an integer selected from 0-6;

s is an integer selected from 0-6;

t is an integer selected from 0-6;

$Rz_2$ is selected from hydroxyl, halogen, an active ester or a carboxyl protecting group;

preferably, the

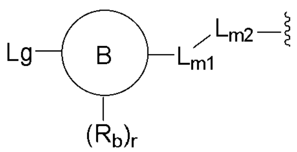

fragment has the structure represented by the following formula:

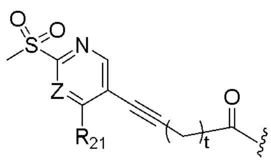

wherein the groups are as defined in claim 16;

$R_{51}$ is independently selected from H, $C_{1-6}$ alkyl, $C_{1-6}$ haloalkyl, $C_{1-6}$ alkyl-NH-, $(C_{1-6}\ alkyl)_{2+}N-$, $C_{1-6}$ alkyl-NH-$C_{1-6}$ alkyl, $(C_{1-6}\ alkyl)_2N-C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and a 5-14-membered heteroaryl; $R_{53}$ is selected from $C_{1-6}$ alkyl, $C_{1-6}$ haloalkyl, $C_{1-6}$ alkyl-NH-, $(C_{1-6}\ alkyl)_2N-$, $C_{1-6}$ alkyl-NH-$C_{1-6}$ alkyl, $(C_{1-6}\ alkyl)_2N-C_{1-6}$ alkyl, $C_{1-6}$ alkoxyalkyl, $C_{2-6}$ alkenyl, $C_{2-6}$ alkynyl, $C_{3-6}$ cycloalkyl, 3-6-membered heterocyclyl, $C_{6-14}$ aryl, and 5-14-membered heteroaryl; ring A is selected from $C_{3-8}$ cycloalkyl or 3-8-membered heterocyclyl; Ra is selected from H, hydroxyl, CN, halogen, $C_{1-6}$ alkyl, or $C_{1-6}$ haloalkyl; n is selected from 0, 1, or 2; and q is selected from 0, 1, or 2;

$L_1$ is a peptide residue; preferably, it is selected from glycine-glycine-phenylalanine-glycine (GGFG), glutamic acid-valine-citrulline (EVC), valine-citrulline (VC), aspartic acid-valine-citrulline (DVC), glutamic acid-glycine-glycine-phenylalanine-glycine (EGGFG), or aspartic acid-glycine-glycine-phenylalanine-glycine (DGGFG); pre-

ferably, $L_1$ is selected from glycine-glycine-phenylalanine-glycine (GGFG); $L_2$ is a linking group between the peptide residue and D; preferably, it is selected from a chemical bond, -NH-$C_{1-6}$ alkyl-,

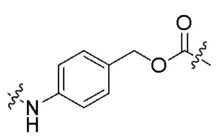

or

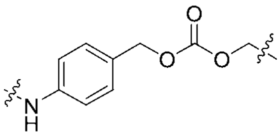

; preferably, $L_2$ is selected from -NH-$CH_2$-;

(L'-1) to (L'-3) are preferably:

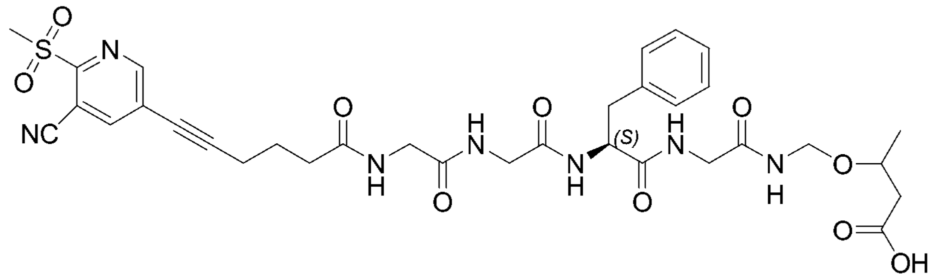

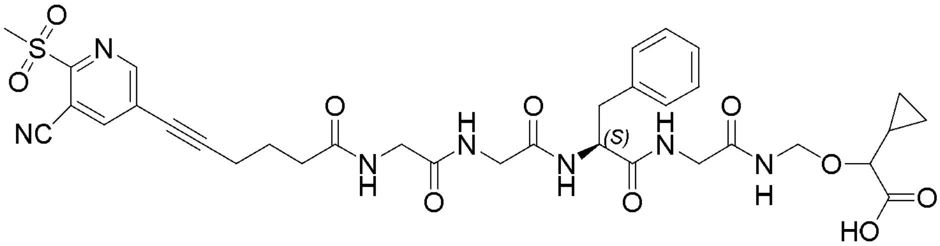

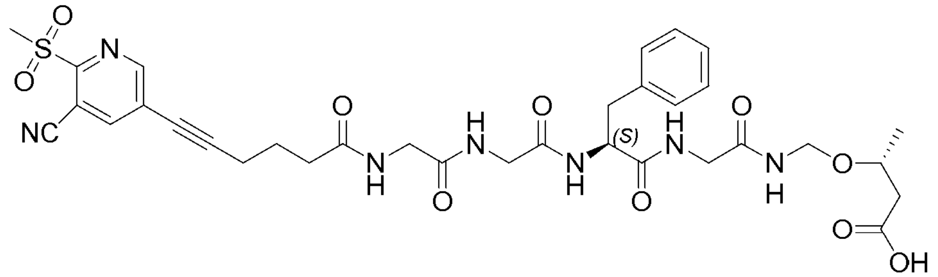

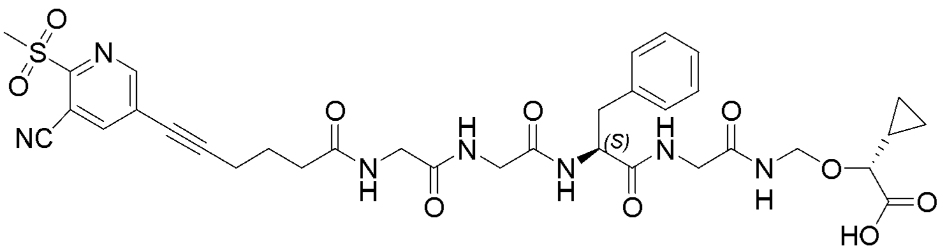

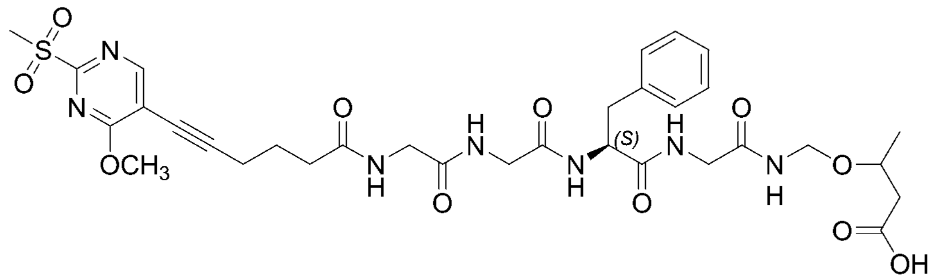

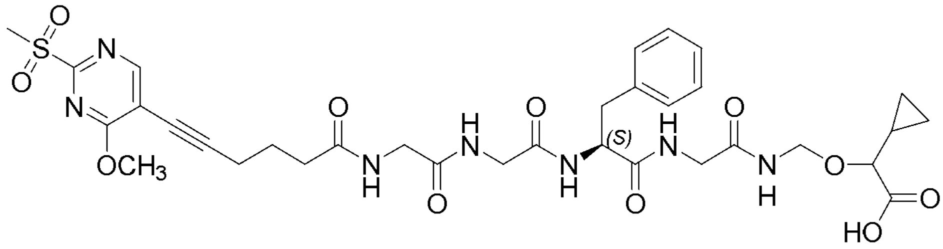

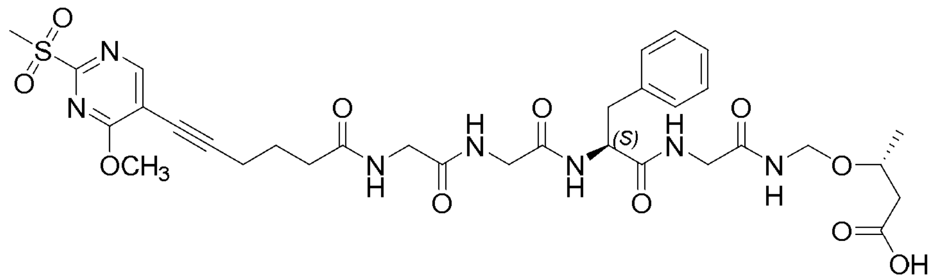

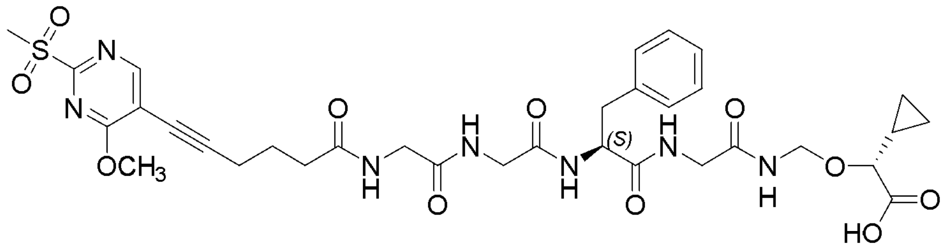

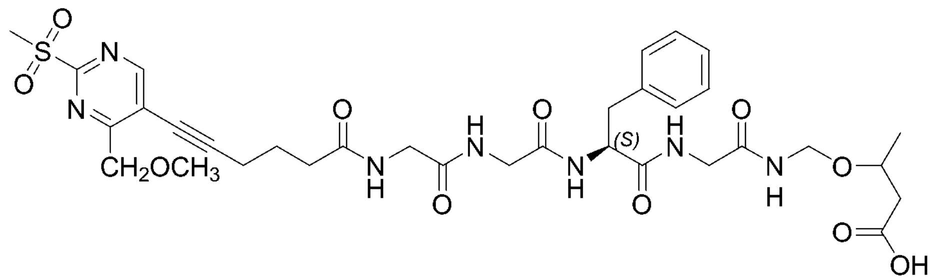

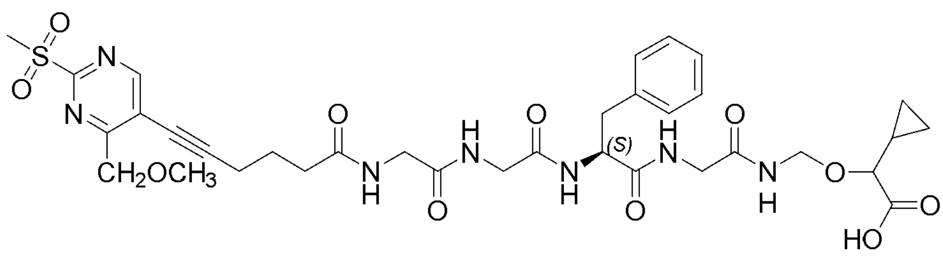

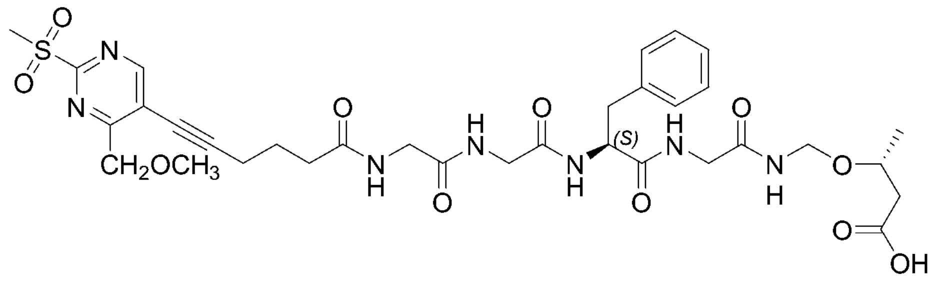

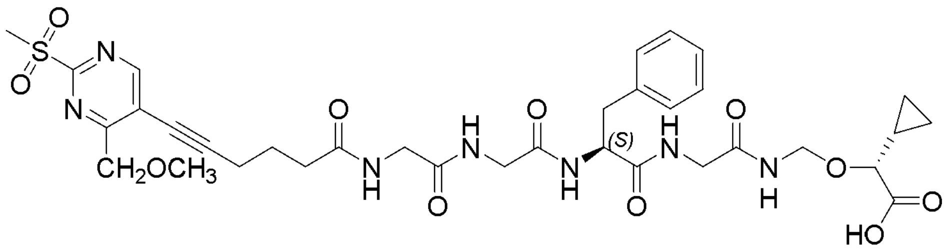

.

**18.** A method for synthesizing the formula (L') according to claim 17, wherein $L_2$ is $-NH-CH_2-$, comprising the following steps:

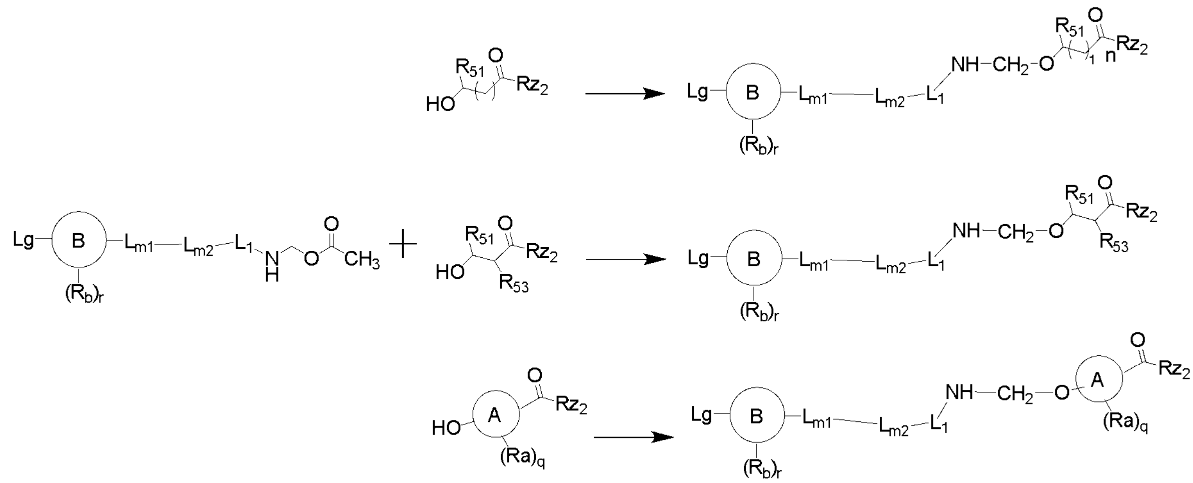

or

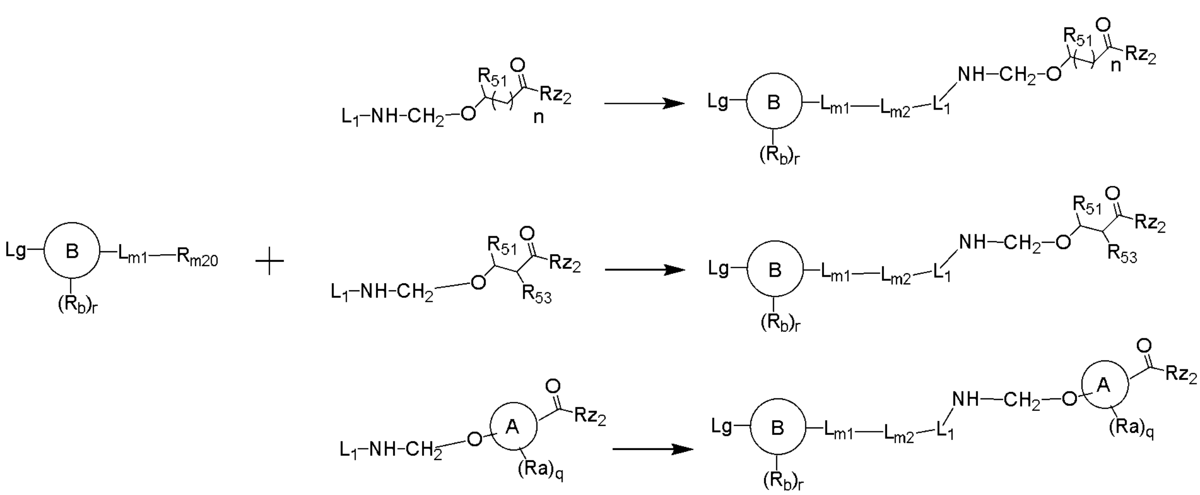

wherein is as defined in claim 16, and $Rz_2$ is hydroxyl, an active ester or a carboxyl protecting group; and the remaining groups are as defined in claim 17.

**19.** A method for synthesizing the compound according to claim 8, comprising a first scheme or a second scheme:

the first scheme is selected from the following steps:

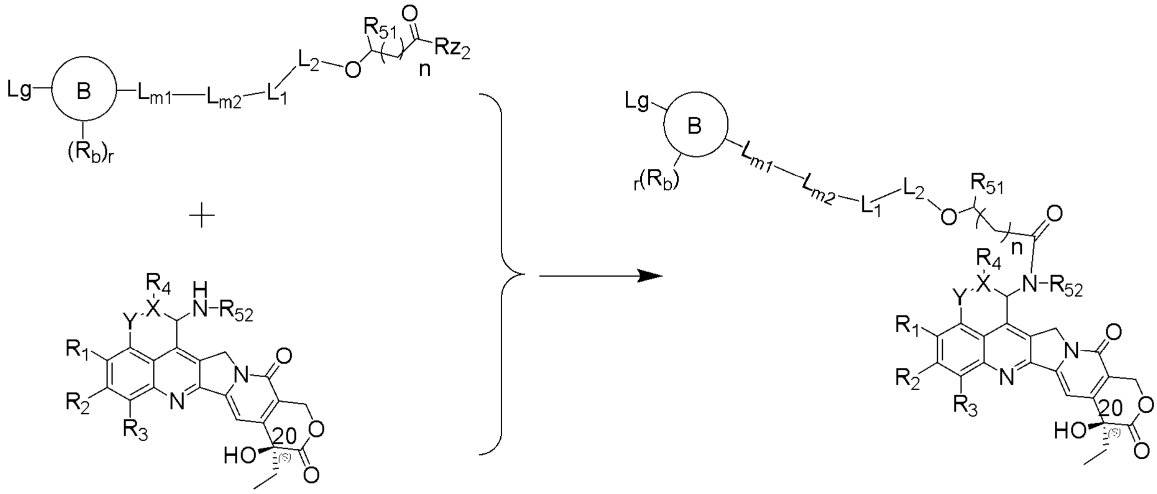

or

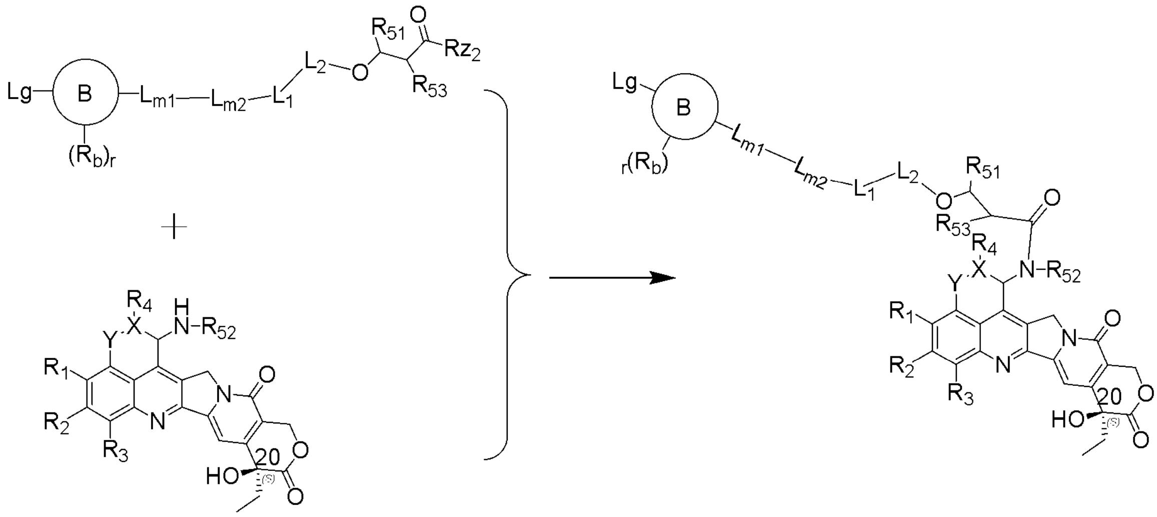

or

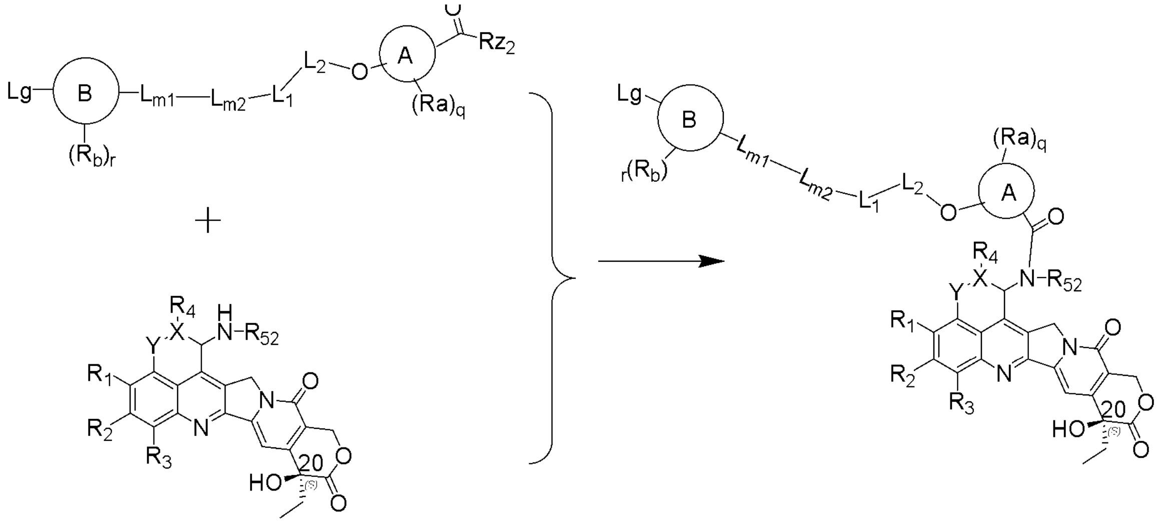

$Rz_2$ is selected from hydroxyl, halogen, an active ester or a carboxyl protecting group, and the remaining groups are as defined in claim 8; and

The second scheme comprises the following steps:

allowing

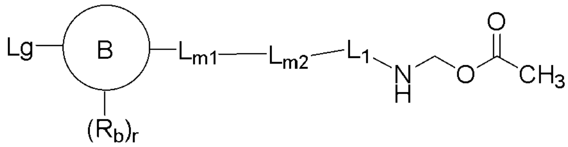

to react with a compound of formula I, wherein the compound of formula I is as described in claim 8, and the remaining groups are as defined in claim 8.

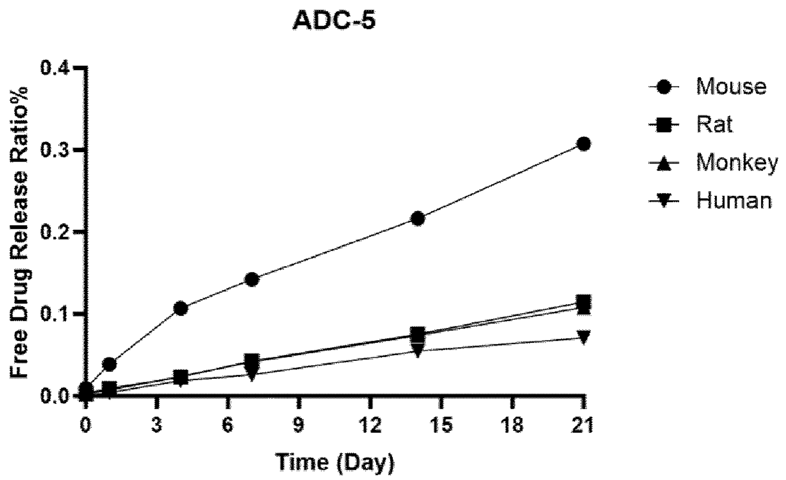


FIG. 1

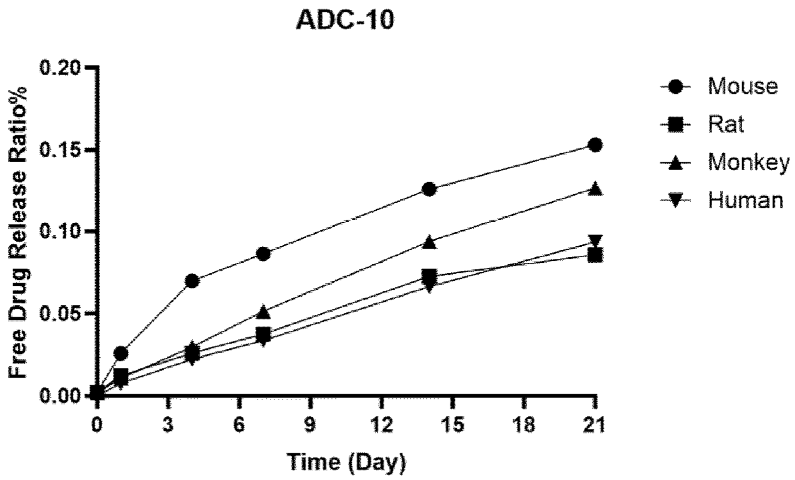


FIG. 2

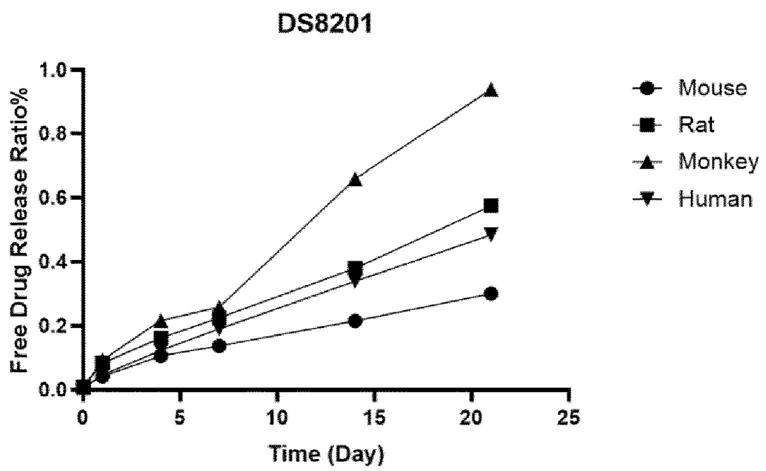


FIG. 3

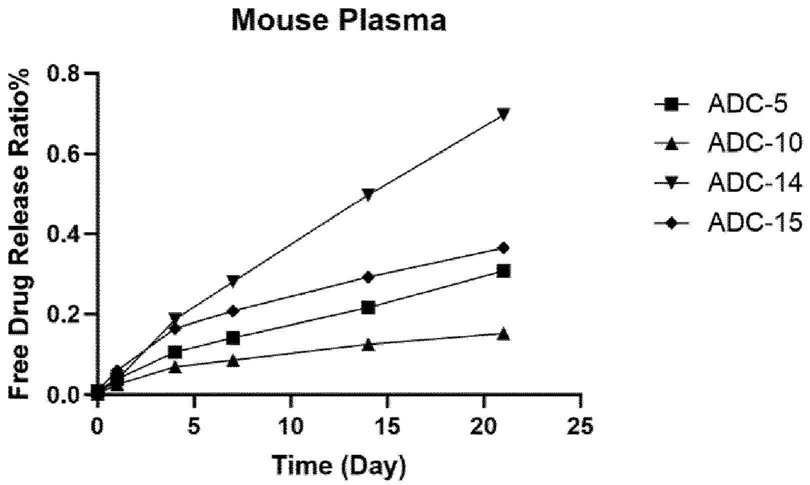


FIG. 4

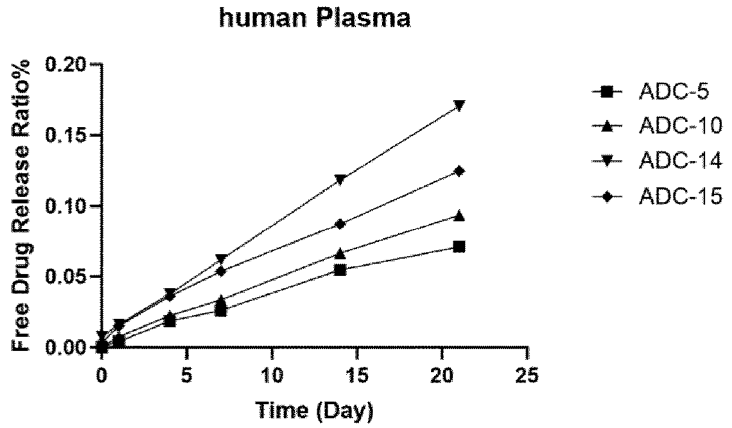


FIG. 5

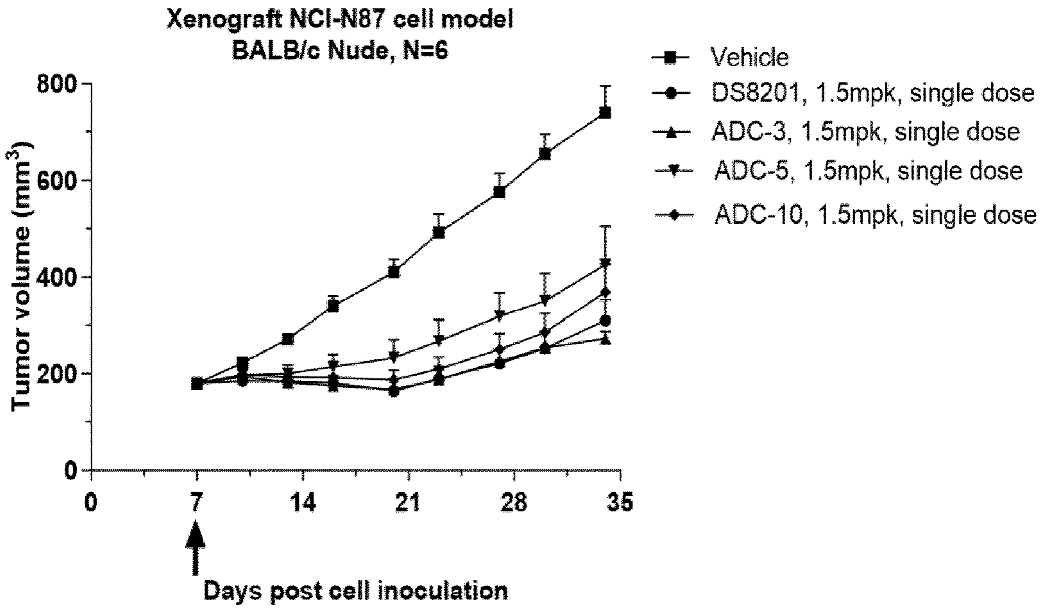


FIG. 6

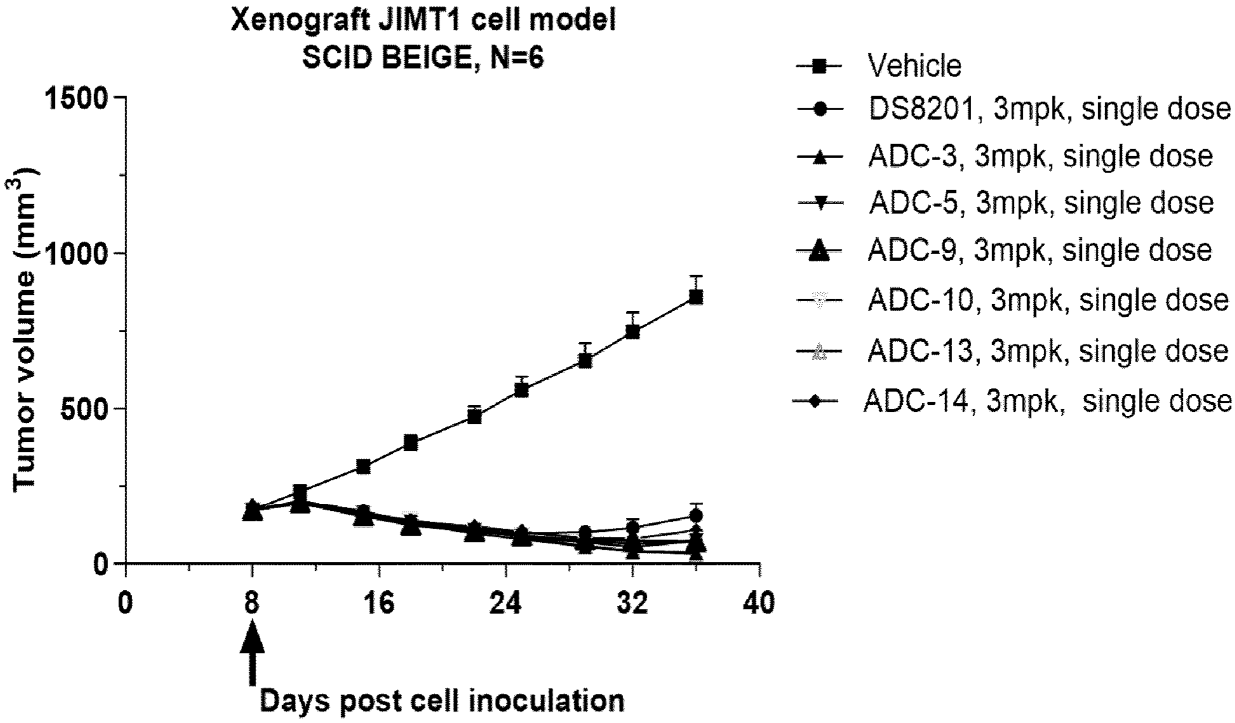
Xenograft JIMT1 cell model
SCID BEIGE, N=6
Tumor volume (mm3)
1500
1000
500
0
0
8
16
24
32
40
Days post cell inoculation
Vehicle
DS8201, 3mpk, single dose
ADC-3, 3mpk, single dose
ADC-5, 3mpk, single dose
ADC-9, 3mpk, single dose
ADC-10, 3mpk, single dose
ADC-13, 3mpk, single dose
ADC-14, 3mpk, single dose

FIG. 7

## INTERNATIONAL SEARCH REPORT

International application No.
**PCT/CN2024/085983**

**A. CLASSIFICATION OF SUBJECT MATTER**

C07D491/22(2006.01)i; C07D495/22(2006.01)i; C07K16/28(2006.01)i; A61P35/00(2006.01)i; A61K31/4745(2006.01)i; A61K47/68(2017.01)i; A61K31/48(2006.01)i; A61K39/395(2006.01)i

According to International Patent Classification (IPC) or to both national classification and IPC

**B. FIELDS SEARCHED**

Minimum documentation searched (classification system followed by classification symbols)

C07D,C07K,A61P,A61K

Documentation searched other than minimum documentation to the extent that such documents are included in the fields searched

Electronic data base consulted during the international search (name of data base and, where practicable, search terms used)

CNKI, CNTXT, ENTXTC, ENTXT, VCN, VEN, STN (REG, CAPLUS): 喜树碱, 抗体, 偶联, camptothecin, antibody, conjugate, 结构式

**C. DOCUMENTS CONSIDERED TO BE RELEVANT**

| Category* | Citation of document, with indication, where appropriate, of the relevant passages | Relevant to claim No. |
|---|---|---|
| X | EP 0495432 A1 (DAIICHI SEIYAKU CO. et al.) 22 July 1992 (1992-07-22) claims 1-9 | 15 |
| X | JP H0687746 A (DAIICHI SEIYAKU CO. et al.) 29 March 1994 (1994-03-29) claims 1-2 | 15 |
| X | WO 2022121981 A1 (WIGEN BIOMEDICINE TECHNOLOGY (SHANGHAI) CO., LTD.) 16 June 2022 (2022-06-16) claims 1, 10, 12, and 15-16, and description, page 24 | 1-19 |
| X | WO 2022166762 A1 (SICHUAN KELUN BIOTECH BIOPHARMACEUTICAL CO., LTD.) 11 August 2022 (2022-08-11) claims 9-12, description, pages 30-31 and 34, and embodiment 25 | 1-19 |
| X | WO 2022253035 A1 (SICHUAN KELUN BIOTECH BIOPHARMACEUTICAL CO., LTD. et al.) 08 December 2022 (2022-12-08) claims 1-22 | 1-19 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 July 2024** | **26 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2024/085983**

**C. DOCUMENTS CONSIDERED TO BE RELEVANT**

| Category* | Citation of document, with indication, where appropriate, of the relevant passages | Relevant to claim No. |
|---|---|---|
| PX | WO 2023143263 A1 (MEDILINK THERAPEUTICS SUZHOU CO., LTD.) 03 August 2023 (2023-08-03)<br>claims 1-28 | 1-19 |
| PX | WO 2023208216 A1 (SICHUAN KELUN BIOTECH BIOPHARMACEUTICAL CO., LTD.) 02 November 2023 (2023-11-02)<br>claims 1-46 | 1-19 |
| E | WO 2024078586 A1 (SICHUAN KELUN BIOTECH BIOPHARMACEUTICAL CO., LTD.) 18 April 2024 (2024-04-18)<br>description, pages 53-55 | 8 |
| X | WO 2020063673 A1 (JIANGSU HENGRUI MEDICINE CO. et al.) 02 April 2020 (2020-04-02)<br>claims 1-28 | 1-19 |
| X | CAS. "RN 791752-85-7 etc."<br>*STN (REG)*, 03 December 2004 (2004-12-03),<br>pages 1-2 | 15 |
| X | WO 2021190602 A1 (JIANGSU HENGRUI MEDICINE CO. et al.) 30 September 2021 (2021-09-30)<br>claims 1-15 | 1-15, 18-19 |
| PX | WO 2023143365 A1 (DUALITY BIOLOGICS SUZHOU CO., LTD.) 03 August 2023 (2023-08-03)<br>claims 1-29 | 1-15, 18-19 |

Form PCT/ISA/210 (second sheet) (July 2022)

**INTERNATIONAL SEARCH REPORT**
**Information on patent family members**

International application No.
**PCT/CN2024/085983**

| Patent document cited in search report | | | Publication date (day/month/year) | Patent family member(s) | | | Publication date (day/month/year) |
|---|---|---|---|---|---|---|---|
| EP | 0495432 | A1 | 22 July 1992 | DE | 69211023 | D1 | 04 July 1996 |
| | | | | DE | 69211023 | T2 | 09 January 1997 |
| | | | | NO | 920201 | D0 | 15 January 1992 |
| | | | | NO | 920201 | L | 17 July 1992 |
| | | | | NO | 180448 | B | 13 January 1997 |
| | | | | NO | 180448 | C | 23 April 1997 |
| | | | | CA | 2059305 | A1 | 17 July 1992 |
| | | | | CA | 2059305 | C | 31 July 2001 |
| | | | | EP | 0495432 | B1 | 29 May 1996 |
| | | | | ES | 2090366 | T3 | 16 October 1996 |
| | | | | HK | 1001561 | A1 | 26 June 1998 |
| | | | | AU | 1018592 | A | 23 July 1992 |
| | | | | AU | 640549 | B2 | 26 August 1993 |
| | | | | GR | 3020395 | T3 | 30 September 1996 |
| | | | | KR | 920014816 | A | 25 August 1992 |
| | | | | KR | 100191193 | B1 | 15 June 1999 |
| | | | | ATE | 138661 | T1 | 15 June 1996 |
| | | | | FI | 920194 | A0 | 16 January 1992 |
| | | | | FI | 920194 | A | 17 July 1992 |
| | | | | FI | 103047 | B | 15 April 1999 |
| | | | | IE | 920079 | A1 | 29 July 1992 |
| | | | | JPH | 0559061 | A | 09 March 1993 |
| | | | | JP | 3008226 | B2 | 14 February 2000 |
| | | | | RU | 2071476 | C1 | 10 January 1997 |
| | | | | DK | 0495432 | T3 | 21 October 1996 |
| JP | H0687746 | A | 29 March 1994 | JP | 3359955 | B2 | 24 December 2002 |
| WO | 2022121981 | A1 | 16 June 2022 | CA | 3200649 | A1 | 16 June 2022 |
| | | | | KR | 20230118891 | A | 14 August 2023 |
| | | | | MX | 2023006751 | A | 19 June 2023 |
| | | | | JP | 2023552610 | A | 18 December 2023 |
| | | | | EP | 4265620 | A1 | 25 October 2023 |
| | | | | AU | 2021394411 | A1 | 06 July 2023 |
| | | | | US | 2023416270 | A1 | 28 December 2023 |
| WO | 2022166762 | A1 | 11 August 2022 | MX | 2023008716 | A | 02 August 2023 |
| | | | | KR | 20230142710 | A | 11 October 2023 |
| | | | | CA | 3209426 | A1 | 11 August 2022 |
| | | | | AU | 2022216696 | A1 | 17 August 2023 |
| | | | | US | 2024158410 | A1 | 16 May 2024 |
| | | | | EP | 4289851 | A1 | 13 December 2023 |
| | | | | JP | 2024506819 | A | 15 February 2024 |
| WO | 2022253035 | A1 | 08 December 2022 | AU | 2022287001 | A1 | 26 October 2023 |
| | | | | EP | 4349372 | A1 | 10 April 2024 |
| | | | | JP | 2024521629 | A | 04 June 2024 |
| | | | | BR | 112023022788 | A2 | 02 January 2024 |
| | | | | CA | 3218527 | A1 | 08 December 2022 |
| | | | | MX | 2023012920 | A | 13 November 2023 |
| | | | | KR | 20240016249 | A | 06 February 2024 |
| WO | 2023143263 | A1 | 03 August 2023 | TW | 202334206 | A | 01 September 2023 |
| WO | 2023208216 | A1 | 02 November 2023 | TW | 202400245 | A | 01 January 2024 |

Form PCT/ISA/210 (patent family annex) (July 2022)

**INTERNATIONAL SEARCH REPORT**
**Information on patent family members**

International application No.
**PCT/CN2024/085983**

| Patent document cited in search report | Publication date (day/month/year) | Patent family member(s) | Publication date (day/month/year) |
|---|---|---|---|
| WO 2024078586 A1 | 18 April 2024 | None | |
| WO 2020063673 A1 | 02 April 2020 | None | |
| WO 2021190602 A1 | 30 September 2021 | CA 3175048 A1 | 30 September 2021 |
| | | TW 202144014 A | 01 December 2021 |
| | | AU 2021243080 A1 | 22 September 2022 |
| | | JP 2023521956 A | 26 May 2023 |
| | | US 2023241242 A1 | 03 August 2023 |
| | | BR 112022019042 A2 | 01 November 2022 |
| | | MX 2022011769 A | 18 October 2022 |
| | | EP 4130045 A1 | 08 February 2023 |
| | | EP 4130045 A4 | 27 December 2023 |
| | | KR 20220157425 A | 29 November 2022 |
| WO 2023143365 A1 | 03 August 2023 | TW 202341984 A | 01 November 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202310367781 A
- WO 202310743291 A
- WO 202310941713 A
- WO 202311173712 A
- WO 202311558824 A
- WO 202311802330 A


### Non-patent literature cited in the description


- *Cancer Res.*, 1989, vol. 49, 6365
- *Nature Review Cancer.*, 2006, vol. 6, 789
- *Med Res. Rev.*, 2015, vol. 35, 753
- **KABAT**. Sequences of Proteins of Immunological Interest. National Institutes of Health, 1987
- **CHOTHIA** ; **LESK**. *J. Mol. Biol.*, 1987, vol. 196, 901-917
- **CHOTHIA et al.** *Nature*, 1989, vol. 342, 878-883
- **KABAT et al.** Sequences of Proteins of Immunological Interest. Public Health Service, National Institutes of Health, 1991
- **LEFRANC et al.** *Dev. Comparat. Immunol.*, 2003, vol. 27, 55-77
- Raven Press. 1989
- **HOLLIGER et al.** *Nat Biotechnol*, 2005, vol. 23, 1126-1136
- **HOLLIGER P. et al.** *Proc. Natl. Acad. Sci. USA*, 1993, vol. 90, 6444-6448
- **POLJAK R.J. et al.** *Structure*, 1994, vol. 2, 1121-1123